# EUROPEAN PATENT APPLICATION

(11) **EP 4 530 282 A1**
(43) Date of publication of application: **02.04.2025**
(21) Application number: 23796363.2
(22) Date of filing: 25.04.2023
(51) Int. Cl.: C07D 205/12, A01M 21/04, A01N 43/42, A01N 47/02, A01N 47/06, A01N 47/16, A01N 47/24, A01N 47/34, A01N 53/12, A01N 55/10, A01N 57/08, A01P 13/00, C07D 209/46, C07D 209/48, C07D 217/22

(54) **HALOALKYL SULFONE ANILIDE COMPOUND AND HERBICIDE CONTAINING SAME**

(30) Priority: 28.04.2022 JP 2022074581
(71) Applicant: SDS BIOTECH K.K., Tokyo 101-0022 (JP)
(72) Inventor: ICHIKAWA, Nobumasa, Tsukuba-shi, Ibaraki 300-2646 (JP); HIRAMA, Taku, Tsukuba-shi, Ibaraki 300-2646 (JP); UNNO, Yuta, Tsukuba-shi, Ibaraki 300-2646 (JP); KANEYASU, Yousuke, Tsukuba-shi, Ibaraki 300-2646 (JP); ISHII, Mahana, Tsukuba-shi, Ibaraki 300-2646 (JP)
(74) Representative: Brann AB
(86) International application number: PCT/JP2023/016225
(87) International publication number: WO 2023/210623

(57) **Abstract**

The present invention relates to a novel haloalkyl sulfonanilide compound or a salt thereof, a herbicide composition containing the compound or salt thereof, a method of weed control, including the step of applying the herbicide composition, and a method of producing the compound.

## Description

### TECHNICAL FIELD

The present invention relates to a novel haloalkyl sulfonanilide compound or a salt thereof, and a herbicide containing the compound or salt as an active ingredient and use thereof.

### BACKGROUND ART

Compounds having a haloalkylsulfonylamino group at position 2 of the benzyl group have been reported to exert herbicidal activity (PTLs 1 to 13).

### CITATION LIST

### PATENT LITERATURE

PTL 1: WO 2004/011429
PTL 2: WO 2006/090792
PTL 3: WO 2008/059948
PTL 4: WO 2008/102908
PTL 5: WO 2010/026989
PTL 6: WO 2010/119906
PTL 7: WO 2014/175206
PTL 8: WO 2015/004282
PTL 9: WO 2015/097071
PTL 10: WO 2016/056565
PTL 11: WO 2016/207081
PTL 12: WO 2016/207082

### SUMMARY OF INVENTION

### TECHNICAL PROBLEM

Currently, a number of herbicides have been developed and are in use. However, there are many kinds of weeds to be controlled. It has been desired to develop highly active and safe herbicidal compounds with a broader spectrum of herbicidal activity.

### SOLUTION TO PROBLEM

As a result of intensive research to discover a new useful compound with higher efficacy and safety as a herbicide, the present inventors have found a new haloalkyl sulfonanilide compound with excellent biological activity. Based on the findings, the present invention has been completed.

The present invention encompasses the disclosure of the following compounds.
(1) A compound or a salt thereof, the compound represented by General Formula [1]: 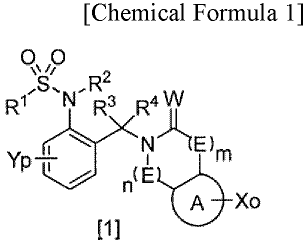
   wherein A is an optionally substituted benzene ring or heteroaromatic ring,
   E is -C(R⁵)(R⁶)-, an oxygen atom, a sulfur atom, SO, SO₂, or N(R⁷),
   W is an oxygen or sulfur atom,
   o is from 0 to 4,
   p is from 0 to 4,
   m is from 0 to 3,
   n is from 0 to 3,
   R¹ is halo C₁-C₆ alkyl,
   R² is a hydrogen atom, C₁-C₆ alkyl optionally substituted by one or more substituents selected from Z¹, C₂-C₆ alkenyl, halo C₂-C₆ alkenyl, C₂-C₆ alkynyl, halo C₂-C₆ alkynyl, (C₁-C₁₂ alkyl)carbonyl, (halo C₁-C₁₂ alkyl)carbonyl, (C₃-C₆ cycloalkyl)carbonyl, (halo C₃-C₆ cycloalkyl)carbonyl, (C₁-C₆ alkyl C₃-C₆ cycloalkyl)carbonyl, C₁-C₆ alkyl(halo C₃-C₆ cycloalkyl)carbonyl, (C₃-C₆ cycloalkoxy)carbonyl, (C₂-C₆ alkenyl)carbonyl, (halo C₂-C₆ alkenyl)carbonyl, phenyl(C₂-C₆ alkenyl)carbonyl optionally substituted by one or more substituents selected from Z², C₁-C₆ alkoxy(C₂-C₆ alkenyl)carbonyl, (C₂-C₆ alkynyl)carbonyl, phenylcarbonyl optionally substituted by one or more substituents selected from Z², heterocyclyl carbonyl optionally substituted by one or more substituents selected from Z², C₃-C₆ cycloalkyl(C₁-C₆ alkyl)carbonyl, (C₁-C₆ alkyl)carbonyl substituted by one substituent selected from Z⁴, (hydroxy C₁-C₆ alkyl)carbonyl, C₁-C₆ alkoxy(C₁-C₆ alkyl)carbonyl, halo C₁-C₆ alkoxy(C₁-C₆ alkyl)carbonyl, (C₁-C₆ alkyl)carbonyl substituted by one substituent selected from Z⁵, phenoxy(halo C₁-C₆ alkyl)carbonyl, (halo C₁-C₆ alkyl)carbonyl substituted by Z⁵, tri(C₁-C₆ alkyl)silyloxy(C₁-C₆ alkyl)carbonyl, C₁-C₆ alkoxy C₁-C₆ alkoxy(C₁-C₆ alkyl)carbonyl, (C₁-C₆ alkyl)carbonyl(C₁-C₆ alkyl)carbonyl, (C₁-C₆ alkyl)carbonyloxy(C₁-C₆ alkyl)carbonyl, (C₁-C₆ alkoxy)carbonyl(C₁-C₆ alkyl)carbonyl, C₁-C₆ alkylthio(C₁-C₆ alkyl)carbonyl, halo C₁-C₆ alkylthio(C₁-C₆ alkyl)carbonyl, (C₁-C₁₂ alkoxy)carbonyl, (halo C₁-C₆ alkoxy)carbonyl, (C₃-C₆ cycloalkyloxy)carbonyl, (C₂-C₆ alkenyloxy)carbonyl, (halo C₂-C₆ alkenyloxy)carbonyl, (C₂-C₆ alkynyloxy)carbonyl, phenoxycarbonyl optionally substituted by one or more substituents selected from Z², heterocyclyloxycarbonyl optionally substituted by one or more substituents selected from Z², C₃-C₆ cycloalkyl(C₁-C₆ alkoxy)carbonyl, (C₁-C₆ alkoxy)carbonyl substituted by one substituent selected from Z⁴, (cyano C₁-C₆ alkoxy)carbonyl, (hydroxy C₁-C₆ alkoxy)carbonyl, C₁-C₆ alkoxy(C₁-C₆ alkoxy)carbonyl, (C₁-C₆ alkoxy)carbonyl substituted by one or more substituents selected from C₁-C₆ alkoxy, halo C₁-C₆ alkoxy(C₁-C₆ alkoxy)carbonyl, tri-C₁-C₆ alkylsilyloxy(C₁-C₆ alkoxy)carbonyl, C₁-C₆ alkoxy C₁-C₆ alkoxy(C₁-C₆ alkoxy)carbonyl, (C₁-C₆ alkyl)carbonyloxy(C₁-C₆ alkoxy)carbonyl, C₁-C₆ alkylsulfonyloxy(C₁-C₆ alkoxy)carbonyl, (C₁-C₆ alkyl)carbonyl(C₁-C₆ alkoxy)carbonyl, (C₁-C₆ alkoxy)carbonyl(C₁-C₆ alkoxy)carbonyl, C₁-C₆ alkylthio(C₁-C₆ alkoxy)carbonyl, halo C₁-C₆ alkylthio(C₁-C₆ alkoxy)carbonyl, C₁-C₆ alkylsulfinyl(C₁-C₆ alkoxy)carbonyl, C₁-C₆ alkylsulfonyl(C₁-C₆ alkoxy)carbonyl, (C₁-C₆ alkylthio)carbonyl, (halo C₁-C₆ alkylthio)carbonyl, mono(C₁-C₆ alkyl)aminocarbonyl, mono(halo C₁-C₆ alkyl)aminocarbonyl, same or different di(C₁-C₆ alkyl)aminocarbonyl, same or different halo di(C₁-C₆ alkyl)aminocarbonyl, C₁-C₆ alkoxyoxalyl, C₁-C₆ alkylsulfonyl, halo C₁-C₆ alkylsulfonyl, C₃-C₆ cycloalkylsulfonyl, C₂-C₆ alkenylsulfonyl, halo C₁-C₆ alkenylsulfonyl, phenylsulfonyl optionally substituted by one or more substituents selected from Z², heterocyclylsulfonyl optionally substituted by one or more substituents selected from Z², C₃-C₆ cycloalkyl C₁-C₆ alkyl sulfonyl, C₁-C₆ alkyl sulfonyl substituted by one substituent selected from Z⁴, C₁-C₆ alkoxy C₁-C₆ alkylsulfonyl, aminosulfonyl, mono C₁-C₆ alkylaminosulfonyl, same or different di C₁-C₆ alkylaminosulfonyl, C₁-C₆ alkylthio, halo C₁-C₆ alkylthio, or cyano,
   Z¹ is, each independently, a halogen atom, C₃-C₆ cycloalkyl, phenyl optionally substituted by one or more substituents selected from Z², heterocyclyl optionally substituted by one or more substituents selected from Z², cyano, C₁-C₆ alkoxy optionally substituted by one or more substituents selected from Z³, C₃-C₆ cycloalkyloxy, phenoxy optionally substituted by one or more substituents selected from Z², phenylcarbonyloxy optionally substituted by one or more substituents selected from Z², heterocyclyl carbonyloxy optionally substituted by one or more substituents selected from Z², (C₁-C₆ alkyl)carbonyloxy optionally substituted by one or more halogen atoms, (C₃-C₆ cycloalkyl)carbonyloxy, (C₁-C₆ alkoxy)carbonyloxy, phenoxycarbonyloxy optionally substituted by one or more substituents selected from Z², - CONR¹¹R¹², C₁-C₆ alkylthio optionally substituted by one or more halogen atoms, C₁-C₆ alkylsulfinyl optionally substituted by one or more halogen atoms, C₁-C₆ alkylsulfonyl optionally substituted by one or more halogen atoms, phenylthio optionally substituted by one or more substituents selected from Z², phenylsulfinyl optionally substituted by one or more substituents selected from Z², C₁-C₆ alkylthio optionally substituted by one substituent selected from Z⁴, C₁-C₆ alkylsulfinyl optionally substituted by one substituent selected from Z⁴, C₁-C₆ alkylsulfinyl optionally substituted by one substituent selected from Z⁴, thiocyanato, (C₁-C₆ alkyl)carbonyl optionally substituted by one or more halogen atoms, (C₁-C₆ alkoxy)carbonyl optionally substituted by one or more halogen atoms, or phenylcarbonyl optionally substituted by one or more substituents selected from Z²,
   Z² each independently represents halogen, C₁-C₆ alkyl, C₂-C₆ alkenyl, C₂-C₆ alkynyl, C₃-C₆ cycloalkyl, halo C₁-C₆ alkyl, halo C₃-C₆ cycloalkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, phenyl, (C₁-C₆ alkyl)carbonyl, (halo C₁-C₆ alkyl)carbonyl, (C₁-C₆ alkoxy)carbonyl, carboxyl, mono(C₁-C₆ alkyl)aminocarbonyl, phenyl substituted by one or more substituents selected from Z⁶,. heterocyclyl optionally substituted by one or more substituents selected from Z⁷, phenoxy optionally substituted by one or more substituents selected from Z⁷, phenylthio optionally substituted by one or more substituents selected from Z⁷, phenylsulfinyl optionally substituted by one or more substituents selected from Z⁷, phenylsulfonyl optionally substituted by one or more substituents selected from Z⁷, phenylcarbonyl optionally substituted by one or more substituents selected from Z⁷, di(C₁-C₆ alkyl)aminocarbonyl, -NR¹³R¹⁴, hydroxyl, cyano, or nitro,
   or two Z² are optionally bonded together with an adjacent carbon or nitrogen atom on a benzene or heterocyclic ring to form a 5- or 6-membered carbocycle or a 5- or 6-membered heterocycle,
      provided that the heterocycle contains one or two heteroatoms selected from the group consisting of an oxygen atom, a sulfur atom, and a nitrogen atom where the nitrogen atom is optionally substituted by C₁-C₆ alkyl, C₂-C₆ alkenyl, C₂-C₆ alkynyl, or C₃-C₆ cycloalkyl, and the carbocycle or heterocycle is optionally substituted by one or more halogen atoms,
      the heteroaromatic ring is thiophene, furan, pyrrole, oxazole, isoxazoline, thiazole, isothiazole, pyrazole, imidazole, 1,3,4-oxadiazole, 1,2,4-oxadiazole, 1,3,4-thiadiazole, 1,2,4-thiadiazole, 1,2,4-triazole, 1,2,3-thiadiazole, 1,2,3-triazole, 1,2,3,4-tetrazole, pyridine, pyrimidine, pyrazine, pyridazine, 1,3,5-triazine, 1,2,4-triazine, benzothiophene, benzofuran, indole, benzothiazole, benzoimidazole, benzoisoxazole, benzoisothiazole, indazole, benzoxazole, quinoline, isoquinoline, quinoxaline, phthalazine, cinnoline, or quinazoline,
      the heterocyclyl is thienyl, furyl, pyrrolyl, oxazolyl, isoxazolyl, isoxazolinyl, thiazolyl, isothiazolyl, pyrazolyl, imidazolyl, 1,3,4-oxadiazolyl, 1,2,4-oxadiazolyl, 1,3,4-thiadiazolyl, 1,2,4-thiadiazolyl, 1,2,4-triazolyl, 1,2,3-thiadiazolyl, 1,2,3-triazolyl, 1,2,3,4-tetrazolyl, pyridyl, pyrimidinyl, pyrazinyl, pyridazinyl, 1,3,5-triazinyl, 1,2,4-triazinyl, benzothienyl, benzofuryl, indolyl, benzothiazolyl, benzoimidazolyl, benzoisoxazolyl, benzoisothiazolyl, indazolyl, benzoxazolyl, quinolyl, isoquinolyl, quinoxalinyl, phthalazinyl, cinnolinyl, quinazolinyl, oxiranyl, oxetanyl, tetrahydrofuranyl, tetrahydrothienyl, 1,1-dioxytetrahydrothienyl, tetrahydropyranyl, tetrahydrothiopyranyl, 1,3-dioxanyl, 2-oxo-1,3-dioxolanyl, 2-oxo-1,3-dioxolyl, or 2,5-dioxopyrrolidinyl,
   Z³ is a halogen atom, phenyl optionally substituted by one or more substituents selected from Z², tri-C₁-C₆ alkylsilyl, C₁-C₆ alkyl diphenylsilyl, C₁-C₆ alkoxy optionally substituted by one or more halogen atoms, phenylcarbonyloxy optionally substituted by one or more substituents selected from Z², or phenylsulfonyl optionally substituted by one or more substituents selected from Z²,
   Z⁴ is phenyl optionally substituted by one or more substituents selected from Z² or heterocyclyl optionally substituted by one or more substituents selected from Z²,
   Z⁵ is phenoxy optionally substituted by one or more substituents selected from Z²,
   Z⁶ is selected from the group consisting of di(C₁-C₆ alkyl)aminocarbonyl, hydroxy, amino, cyano, and nitro,
   Z⁷ is selected from the group consisting of halogen, C₁-C₆ alkyl, C₂-C₆ alkenyl, C₂-C₆ alkynyl, C₃-C₆ cycloalkyl, halo C₁-C₆ alkyl, halo C₃-C₆ cycloalkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, (C₁-C₆ alkyl)carbonyl, (halo C₁-C₆ alkyl)carbonyl, (C₁-C₆ alkoxy)carbonyl, carboxyl, mono(C₁-C₆ alkyl)aminocarbonyl, same or different di(C₁-C₆ alkyl)aminocarbonyl, hydroxyl, amino, cyano, and nitro,
   Z⁸ is selected from the group consisting of phenylthio optionally substituted by one or more substituents selected from Z², phenylsulfinyl optionally substituted by one or more substituents selected from Z², and phenylsulfonyl optionally substituted by one or more substituents selected from Z²,
   R¹¹ and R¹² are each independently selected from the group consisting of a hydrogen atom, C₁-C₆ alkyl, and phenyl optionally substituted by one or more substituents selected from Z²,
   R¹³ is selected from the group consisting of a hydrogen atom, and C₁-C₆ alkyl,
   R¹⁴ is a hydrogen atom or phenyl optionally substituted by one or more substituents selected from Z⁷,
   R³ and R⁴ are each independently a hydrogen atom, C₁-C₆ alkyl, halo C₁-C₆ alkyl, C₁-C₆ cycloalkyl, halo C₁-C₆ cycloalkyl, C₁-C₆ alkoxy, halo C₁-C₆ alkoxy, halogen, or cyano,
   or R³ and R⁴ are optionally bonded together to form a 3- to 7-membered ring,
   R⁵ and R⁶ are each independently a hydrogen atom, halogen, C₁-C₆ alkyl, halo C₁-C₆ alkyl, C₂-C₆ alkenyl, C₂-C₆ alkynyl, C₃-C₆ cycloalkyl, halo C₃-C₆ cycloalkyl, hydroxy C₁-C₆ alkyl, C₁-C₆ alkoxy C₁-C₆ alkyl, halo C₁-C₆ alkoxy C₁-C₆ alkyl, C₁-C₆ alkylthio C₁-C₆ alkyl, halo C₁-C₆ alkylthio C₁-C₆ alkyl, mono(C₁-C₆ alkyl)amino C₁-C₆ alkyl, same or different di(C₁-C₆ alkyl)amino C₁-C₆ alkyl, phenyl optionally substituted by one or more substituents selected from Z², heterocyclyl optionally substituted by one or more substituents selected from Z², C₁-C₆ alkyl substituted by one substituent selected from Z⁴, C₁-C₆ alkyl substituted by one substituent selected from Z⁵*,* (C₁-C₆ alkyl)carbonyl, (halo C₁-C₆ alkyl)carbonyl, phenylcarbonyl optionally substituted by one or more substituents selected from Z², (C₁-C₆ alkoxy)carbonyl, (halo C₁-C₆ alkoxy)carbonyl, carboxyl, -NR¹¹R¹², C₁-C₆ alkoxy, halo C₁-C₆ alkoxy, phenoxy optionally substituted by one or more substituents selected from Z², C₁-C₆ alkylthio, halo C₁-C₆ alkylthio, phenylthio optionally substituted by one or more substituents selected from Z², C₁-C₆ alkylsulfonyl, halo C₁-C₆ alkylsulfonyl, mono(C₁-C₆ alkyl)amino, di(C₁-C₆ alkyl)amino, (C₁-C₆ alkyl)carbonyloxy, hydroxyl, amino, cyano, or nitro,
   or R⁵ and R⁶ on the same carbon are optionally bonded together to form a 3- to 7-membered ring optionally interrupted by one or two heteroatoms selected from the group consisting of an oxygen atom, a sulfur atom, and a nitrogen atom where the nitrogen atom is optionally substituted by C₁-C₆ alkyl, C₂-C₆ alkenyl, C₂-C₆ alkynyl, or C₃-C₆ cycloalkyl or optionally substituted, or to form an olefin,
   or R⁵ and R⁶ are optionally bonded to R⁵ or R⁶ on different carbon to form a 3- to 8-membered ring optionally interrupted by one or two heteroatoms selected from the group consisting of an oxygen atom, a sulfur atom, and a nitrogen atom where the nitrogen atom is optionally substituted by C₁-C₆ alkyl, C₂-C₆ alkenyl, C₂-C₆ alkynyl, or C₃-C₆ cycloalkyl or using optionally substituted C₁-C₄ alkylene, halo C₁-C₄ alkylene, C₂-C₄ alkenylene, or halo C₂-C₄ alkenylene,
   or R⁵ and R⁶ are optionally bonded together with R⁵ or R⁶ on adjacent carbon to form a bond,
   R⁷ is a hydrogen atom, C₁-C₆ alkyl, halo C₁-C₆ alkyl, C₃-C₆ cycloalkyl, halo C₃-C₆ cycloalkyl, C₂-C₆ alkenyl, halo C₂-C₆ alkenyl, C₂-C₆ alkynyl, hydroxy C₁-C₆ alkyl, C₁-C₆ alkoxy C₁-C₆ alkyl, halo C₁-C₆ alkoxy C₁-C₆ alkyl, C₃-C₆ cycloalkyl C₁-C₆ alkyl, C₁-C₆ alkyl substituted by one substituent selected from Z⁴, phenyl optionally substituted by one or more substituents selected from Z², C₁-C₆ alkyl substituted by one substituent selected from Z⁴, (C₁-C₆ alkyl)carbonyl C₁-C₆ alkyl, (halo C₁-C₆ alkyl)carbonyl C₁-C₆ alkyl, (C₁-C₆ alkoxy)carbonyl C₁-C₆ alkyl, (halo C₁-C₆ alkoxy)carbonyl C₁-C₆ alkyl, (C₁-C₆ alkyl)carbonyl, (halo C₁-C₆ alkyl)carbonyl, (C₃-C₆ cycloalkyl)carbonyl, C₃-C₆ cycloalkyl(C₁-C₆ alkyl)carbonyl, (C₂-C₆ alkenyl)carbonyl, phenylcarbonyl optionally substituted by one or more substituents selected from Z², heterocyclyl carbonyl optionally substituted by one or more substituents selected from Z², (C₁-C₆ alkoxy)carbonyl, (halo C₁-C₆ alkoxy)carbonyl, phenoxycarbonyl optionally substituted by one or more substituents selected from Z², aminocarbonyl, mono(C₁-C₆ alkyl)aminocarbonyl, mono halo(C₁-C₆ alkyl)aminocarbonyl, same or different di(C₁-C₆ alkyl)aminocarbonyl, same of different halo di(C₁-C₆ alkyl)aminocarbonyl, (C₁-C₆ alkylthio)carbonyl, (halo C₁-C₆ alkylthio)carbonyl, C₁-C₆ alkylsulfonyl, halo C₁-C₆ alkylsulfonyl, phenylsulfonyl optionally substituted by one or more substituents selected from Z², C₁-C₆ alkylthio C₁-C₆ alkyl, halo C₁-C₆ alkylthio C₁-C₆ alkyl, C₁-C₆ alkyl substituted by one substituent selected from Z⁸, C₁-C₆ alkylsulfinyl C₁-C₆ alkyl, halo C₁-C₆ alkylsulfinyl C₁-C₆ alkyl, C₁-C₆ alkylsulfonyl C₁-C₆ alkyl, halo C₁-C₆ alkylsulfonyl C₁-C₆ alkyl, cyano, amino, or hydroxy,
   X is each independently a hydrogen atom, halogen, C₁-C₆ alkyl, C₂-C₆ alkenyl, C₂-C₆ alkynyl, C₃-C₆ cycloalkyl, halo C₁-C₆ alkyl, halo C₂-C₆ alkenyl, halo C₂-C₆ alkynyl, halo C₃-C₆ cycloalkyl, C₁-C₆ alkoxy, halo C₁-C₆ alkoxy, C₂-C₆ alkenyloxy, C₂-C₆ alkynyloxy, C₃-C₆ cycloalkyl C₁-C₆ alkyloxy, phenyl C₁-C₆ alkyloxy, (C₁-C₆ alkyl)carbonyloxy, phenylcarbonyloxy optionally substituted by one or more substituents selected from Z², heterocyclyl carbonyloxy optionally substituted by one or more substituents selected from Z², (C₁-C₆ alkoxy)carbonyloxy, (C₁-C₆ alkylthio)carbonyloxy, C₁-C₆ alkylsulfonyloxy, halo C₁-C₆ alkylsulfonyloxy, phenylsulfonyloxy, di C₁-C₆ alkylphosphorooxy, tri C₁-C₆ alkylsilyloxy, C₁-C₆ alkoxyC₁-C₆ alkyl, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, phenyl optionally substituted by one or more substituents selected from Z², heterocyclyl optionally substituted by one or more substituents selected from Z², phenoxy optionally substituted by one or more substituents selected from Z², phenylthio optionally substituted by one or more substituents selected from Z², phenylsulfinyl optionally substituted by one or more substituents selected from Z², phenylsulfonyl optionally substituted by one or more substituents selected from Z², di(C₁-C₆ alkyl)amino, (C₁-C₆ alkyl)carbonylamino, bis((C₁-C₆ alkyl)carbonyl)amino, halo C₁-C₆ alkylsulfonylamino, morpholinyl, isoindoline-1,3-dione-2-yl, (C₁-C₆ alkyl)carbonyl, (halo C₁-C₆ alkyl)carbonyl, phenylcarbonyl optionally substituted by one or more substituents selected from Z², (C₁-C₆ alkoxy)carbonyl, carboxyl, mono(C₁-C₆ alkyl)aminocarbonyl, same or different di(C₁-C₆ alkyl)aminocarbonyl, phenylaminocarbonyl optionally substituted by one or more substituents selected from Z², phenyl(C₁-C₆ alkylamino)carbonyl, (C₁-C₆ alkylamino)carbonyl substituted by one substituent selected from Z⁴, hydroxy, amino, cyano, or nitro,
   or two X attached to an adjacent carbon or nitrogen atom on a benzene or heterocyclic ring are bonded together with a ring atom to which they are attached to form a 4- to 6-membered carbocycle or heterocycle where the heterocycle contains one or two heteroatoms selected from the group consisting of an oxygen atom, a sulfur atom, and a nitrogen atom,
      provided that the nitrogen atom is optionally substituted by C₁-C₆ alkyl, C₂-C₆ alkenyl, C₂-C₆ alkynyl, or C₃-C₆ cycloalkyl, and
   Y is each independently a hydrogen atom, halogen, C₁-C₆ alkyl, C₂-C₆ alkenyl, C₂-C₆ alkynyl, C₃-C₆ cycloalkyl, halo C₁-C₆ alkyl, halo C₂-C₆ alkenyl, halo C₂-C₆ alkynyl, halo C₃-C₆ cycloalkyl, C₁-C₆ alkoxy, halo C₁-C₆ alkoxy, C₂-C₆ alkenyloxy, C₂-C₆ alkynyloxy, C₃-C₆ cycloalkyl C₁-C₆ alkyloxy, phenyl C₁-C₆ alkyloxy, (C₁-C₆ alkyl)carbonyloxy, phenylcarbonyloxy optionally substituted by one or more substituents selected from Z², heterocyclyl carbonyloxy optionally substituted by one or more substituents selected from Z², (C₁-C₆ alkoxy)carbonyloxy, (C₁-C₆ alkylthio)carbonyloxy, C₁-C₆ alkylsulfonyloxy, halo C₁-C₆ alkylsulfonyloxy, phenylsulfonyloxy, di C₁-C₆ alkylphosphorooxy, tri C₁-C₆ alkylsilyloxy, C₁-C₆ alkoxy C₁-C₆ alkyl, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, phenyl optionally substituted by one or more substituents selected from Z², heterocyclyl optionally substituted by one or more substituents selected from Z², phenoxy optionally substituted by one or more substituents selected from Z², phenylthio optionally substituted by one or more substituents selected from Z², phenylsulfinyl optionally substituted by one or more substituents selected from Z², phenylsulfonyl optionally substituted by one or more substituents selected from Z², di(C₁-C₆ alkyl)amino, (C₁-C₆ alkyl)carbonylamino, bis((C₁-C₆ alkyl)carbonyl)amino, halo C₁-C₆ alkylsulfonylamino, morpholinyl, isoindoline-1,3-dione-2-yl, (C₁-C₆ alkyl)carbonyl, (halo C₁-C₆ alkyl)carbonyl, phenylcarbonyl optionally substituted by one or more substituents selected from Z², (C₁-C₆ alkoxy)carbonyl, carboxyl, -NR¹¹R¹², hydroxy, amino, cyano, or nitro,
   or two Y are optionally bonded together with an adjacent carbon or nitrogen atom on a benzene ring to form a 5- or 6-membered carbocycle or a 5- or 6-membered heterocycle,
      provided that the heterocycle contains one or two heteroatoms selected from the group consisting of an oxygen atom, a sulfur atom, and a nitrogen atom where the nitrogen atom is optionally substituted by C₁-C₆ alkyl, C₂-C₆ alkenyl, C₂-C₆ alkynyl, or C₃-C₆ cycloalkyl, and the carbocycle or heterocycle is optionally substituted by one or more halogen atoms.
(2) The compound or salt thereof according to (1), the compound represented by General Formula [1a]: 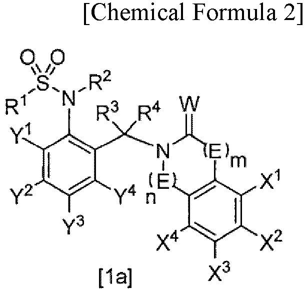
   wherein E is -CH₂-,
   W is an oxygen or sulfur atom,
   m is 0,
   n is 0, 1, or 3,
   R¹ is fluoro C₁-C₆ alkyl,
   R² is a hydrogen atom, C₁-C₆ alkoxy C₁-C₆ alkyl, or (C₁-C₁₂ alkoxy)carbonyl,
   R³ and R⁴ are each a hydrogen atom,
   X¹, X², X³, and X⁴ are each independently a hydrogen atom, halogen, C₁-C₆ alkyl, halo C₁-C₆ alkyl, C₁-C₆ alkoxy, C₂-C₆ alkenyloxy, C₂-C₆ alkynyloxy, C₃-C₆ cycloalkyl C₁-C₆ alkyloxy, phenyl C₁-C₆ alkyloxy, (C₁-C₆ alkyl)carbonyloxy, phenylcarbonyloxy optionally substituted by one or more substituents selected from Z², heterocyclylcarbonyloxy, (C₁-C₆ alkoxy)carbonyloxy, (C₁-C₆ alkylthio)carbonyloxy, C₁-C₆ alkylsulfonyloxy, halo C₁-C₆ alkylsulfonyloxy, phenylsulfonyloxy, di C₁-C₆ alkylphosphorooxy, tri C₁-C₆ alkylsilyloxy, C₁-C₆ alkoxy C₁-C₆ alkyl, phenyl, hydroxyl, amino, cyano, or nitro,
   Y¹, Y², Y³, and Y⁴ are each independently a hydrogen atom or halo C₁-C₆ alkyl, and
   Z² is nitro.
(3) The compound or salt thereof according to (1), the compound represented by General Formula [1a]: 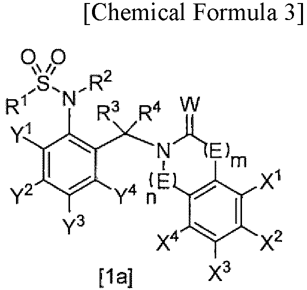
   wherein E is -CH₂-,
   W is an oxygen or sulfur atom,
   m is 0,
   n is 2,
   R¹ is fluoro C₁-C₆ alkyl,
   R² is a hydrogen atom, C₂-C₆ alkynyl, C₁-C₆ alkyl substituted by one substituent selected from Z⁴, cyano C₁-C₆ alkyl, C₁-C₆ alkoxy C₁-C₆ alkyl, (C₁-C₆ alkoxy)carbonyloxy C₁-C₆ alkyl, (C₁-C₁₂ alkyl)carbonyl, (halo C₁-C₁₂ alkyl)carbonyl, (C₃-C₆ cycloalkyl)carbonyl, C₁-C₆ alkyl(C₃-C₆ cycloalkyl)carbonyl, C₁-C₆ alkyl halo(C₃-C₆ cycloalkyl)carbonyl, (C₂-C₆ alkenyl)carbonyl, C₁-C₆ alkoxy(C₂-C₆ alkenyl)carbonyl, phenylcarbonyl, heterocyclyl carbonyl optionally substituted by one or more substituents selected from Z², C₃-C₆ cycloalkyl(C₁-C₆ alkyl)carbonyl, hydroxy(C₁-C₆ alkyl)carbonyl, C₁-C₆ alkoxy(C₁-C₆ alkyl)carbonyl, tri(C₁-C₆ alkyl)silyloxy (C₁-C₆ alkyl)carbonyl, (C₁-C₆ alkyl) carbonyloxy (C₁-C₆ alkyl)carbonyl, C₁-C₆ alkylthio(C₁-C₆ alkyl)carbonyl, (C₁-C₁₂ alkoxy)carbonyl, (halo C₁-C₆ alkoxy)carbonyl, (C₃-C₆ cycloalkyloxy)carbonyl, (C₂-C₆ alkenyloxy)carbonyl, (C₂-C₆ alkynyloxy)carbonyl, phenoxycarbonyl optionally substituted by one or more substituents selected from Z², heterocyclyloxycarbonyl optionally substituted by one or more substituents selected from Z², C₃-C₆ cycloalkyl(C₁-C₆ alkoxy)carbonyl, (C₁-C₆ alkoxy)carbonyl substituted by one substituent selected from Z⁴, (cyano C₁-C₆ alkoxy)carbonyl, (hydroxy C₁-C₆ alkoxy)carbonyl, C₁-C₆ alkoxy(C₁-C₆ alkoxy)carbonyl, (C₁-C₆ alkoxy)carbonyl substituted by one or more substituents selected from C₁-C₆ alkoxy, (C₁-C₆ alkyl)diphenylsilyloxy(C₁-C₆ alkoxy)carbonyl, (C₁-C₆ alkyl)carbonyloxy(C₁-C₆ alkoxy)carbonyl, C₁-C₆ alkylsulfonyloxy(C₁-C₆ alkoxy)carbonyl, (C₁-C₆ alkyl)carbonyl(C₁-C₆ alkoxy)carbonyl, (C₁-C₆ alkoxy)carbonyl(C₁-C₆ alkoxy)carbonyl, C₁-C₆ alkylthio(C₁-C₆ alkoxy)carbonyl, C₁-C₆ alkylsulfonyl(C₁-C₆ alkoxy)carbonyl, (C₁-C₆ alkylthio)carbonyl, mono(C₁-C₆ alkyl)aminocarbonyl, same or different di(C₁-C₆ alkyl)aminocarbonyl, C₁-C₆ alkylsulfonyl, halo C₁-C₆ alkylsulfonyl, or C₃-C₆ cycloalkylsulfonyl,
   R³ and R⁴ are each a hydrogen atom,
   X¹, X², X³, and X⁴ are each independently a hydrogen atom, halogen, C₁-C₆ alkyl, C₃-C₆ cycloalkyl, halo C₁-C₆ alkyl, C₁-C₆ alkoxy, halo C₁-C₆ alkoxy, C₂-C₆ alkynyloxy, (C₁-C₆ alkyl)carbonyloxy, phenylcarbonyloxy, (C₁-C₆ alkoxy)carbonyloxy, C₁-C₆ alkylsulfonyloxy, halo C₁-C₆ alkylsulfonyloxy, C₁-C₆ alkylthio, C₁-C₆ alkylsulfonyl, phenyl, same or different di(C₁-C₆ alkyl)amino, (C₁-C₆ alkyl)carbonylamino, bis((C₁-C₆ alkyl)carbonyl)amino, halo C₁-C₆ alkylsulfonylamino, morpholinyl, isoindoline-1,3-dione-2-yl, (C₁-C₆ alkoxy)carbonyl, hydroxyl, cyano, or nitro,
   or X¹, X², X³, or X⁴ is optionally bonded together with an adjacent carbon atom on a benzene ring to form a 5- or 6-membered carbocycle or a 5- or 6-membered heterocycle, where the heterocycle contains one or two heteroatoms selected from a sulfur atom and a nitrogen atom,
   Y¹, Y², Y³, and Y⁴ are each independently a hydrogen atom, halogen, C₁-C₆ alkyl, halo C₁-C₆ alkyl, C₁-C₆ alkoxy, or hydroxyl,
   or Y¹ and Y², Y² and Y³, or Y³ and Y⁴ are optionally bonded together with a carbon atom to which they are attached to form a 4- to 6-membered carbocycle,
   Z² is C₁-C₆ alkyl, C₁-C₆ alkoxy, or nitro, and
   the heterocyclyl is thienyl, furyl, pyrrolyl, oxiranyl, oxetanyl, tetrahydrofuranyl, tetrahydrothienyl, 1,1-dioxytetrahydrothienyl, tetrahydropyranyl, tetrahydrothiopyranyl, 1,3-dioxanyl, 2-oxo-1,3-dioxolanyl, 2-oxo-1,3-dioxolyl, or 2,5-dioxopyrrolidinyl.
(4) The compound or salt thereof according to (3), wherein n is 2,
   R¹ is trifluoromethyl,
   R² is a hydrogen atom, (C₃-C₆ cycloalkyl)carbonyl, (C₂-C₄ alkenyl)carbonyl, C₁-C₆ alkoxy(C₁-C₆ alkyl)carbonyl, 2-furylcarbonyl, C₁-C₆ alkylthio(C₁-C₆ alkyl)carbonyl, (C₁-C₅ alkoxy)carbonyl, (halo C₁-C₃ alkoxy)carbonyl, (C₃-C₄ cycloalkyloxy)carbonyl, (C₂-C₃ alkynyloxy)carbonyl, heterocyclyloxycarbonyl, C₃-C₄ cycloalkyl(C₁-C₂ alkoxy)carbonyl, heterocyclyl(C₁-C₂ alkoxy)carbonyl, (cyano C₁-C₆ alkoxy)carbonyl, (hydroxy C₁-C₆ alkoxy)carbonyl, C₁-C₆ alkoxy(C₁-C₆ alkoxy)carbonyl, C₁-C₂ alkylsulfonyl, or (C₁-C₄ alkylthio)carbonyl,
   X¹ is a fluorine atom, a chloro atom, or methyl,
   X² and X³ are each a hydrogen atom,
   X⁴ is a hydrogen atom, a fluorine atom, a chloro atom, methyl, trifluoromethyl, or methoxy,
   Y¹, Y², Y³, and Y⁴ are each a hydrogen atom, and
   the heterocyclyl is oxiranyl, oxetanyl, tetrahydrofuranyl, or tetrahydropyranyl.
(5) The compound or salt thereof according to (1), the compound represented by General Formula [1b]: 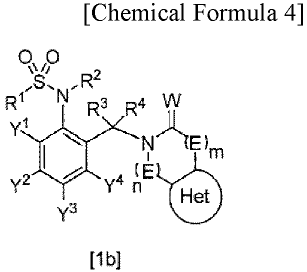
   wherein Het is selected from 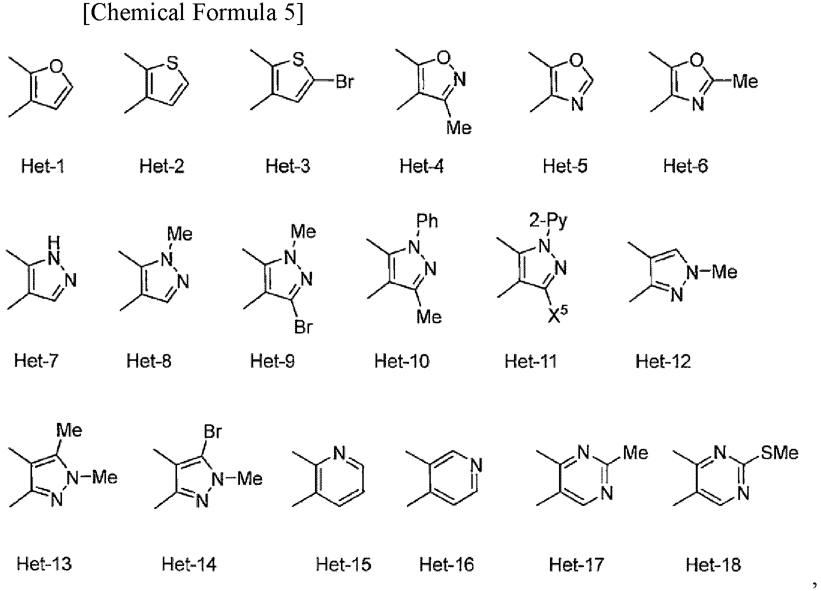
   E is -CH₂-,
   W is an oxygen atom,
   m is 0,
   n is from 1 to 2,
   R¹ is fluoro C₁-C₆ alkyl,
   R² is a hydrogen atom or (C₁-C₁₂ alkoxy)carbonyl,
   R³ and R⁴ are each a hydrogen atom, and
   Y¹, Y², Y³, and Y⁴ are each a hydrogen atom.
(6) The compound or salt thereof according to (5), wherein
   Het is Het-1 or Het-2,
   n is 2,
   R¹ is trifluoromethyl, and
   R² is a hydrogen atom or (C₂-C₆ alkoxy)carbonyl.
(7) The compound or salt thereof according to (1), which is selected from the group consisting of
   N-[2-(N-trifluoromethanesulfonyl)aminobenzyl]-7-azabicyclo[4.2.0]octa-1(6),2,4-trien-8-one,
   N-[2-(N-trifluoromethanesulfonyl)aminobenzyl]-5-fluoro-7-azabicyclo[4.2.0]octa-1(6),2,4-trien-8-one,
   N-[2-(N-trifluoromethanesulfonyl)aminobenzyl]-4-fluoro-7-azabicyclo[4.2.0]octa-1(6),2,4-trien-8-one,
   N-[2-(N-trifluoromethanesulfonyl)aminobenzyl]-3-fluoro-7-azabicyclo[4.2.0]octa-1(6),2,4-trien-8-one,
   N-[2-(N-trifluoromethanesulfonyl)aminobenzyl]-2-fluoro-7-azabicyclo[4.2.0]octa-1(6),2,4-trien-8-one,
   N-[2-(N-trifluoromethanesulfonyl)aminobenzyl]-2-trifluoromethyl-7-azabicyclo[4.2.0]octa-1(6),2,4-trien-8-one,
   N-[2-(N-trifluoromethanesulfonyl)aminobenzyl]-2,5-dichloro-7-azabicyclo[4.2.0]octa-1(6),2,4-trien-8-one,
   N-[2-(N-trifluoromethanesulfonyl)aminobenzyl]-3,4-difluoro-7-azabicyclo[4.2.0]octa-1(6),2,4-trien-8-one,
   N-[2-(N-ethoxycarbonyl-N-trifluoromethanesulfonyl)aminobenzyl]-5-fluoro-7-azabicyclo[4.2.0]octa-1(6),2,4-trien-8-one,
   N-[2-(N-ethoxycarbonyl-N-trifluoromethanesulfonyl)aminobenzyl]-4-fluoro-7-azabicyclo[4.2.0]octa-1(6),2,4-trien-8-one,
   N-[2-(N-ethoxycarbonyl-N-trifluoromethanesulfonyl)aminobenzyl]-3-fluoro-7-azabicyclo[4.2.0]octa-1(6),2,4-trien-8-one,
   N-[2-(N-trifluoromethanesulfonyl)aminobenzyl]-1,3-dihydroisoindol-1-one,
   N-[2-(N-trifluoromethanesulfonyl)aminobenzyl]-7-fluoro-1,3-dihydroisoindol-1-one,
   N-[2-(N-trifluoromethanesulfonyl)aminobenzyl]-7-chloro-1,3-dihydroisoindol-1-one,
   N-[2-(N-trifluoromethanesulfonyl)aminobenzyl]-7-bromo-1,3-dihydroisoindol-1-one,
   N-[2-(N-trifluoromethanesulfonyl)aminobenzyl]-7-methyl-1,3-dihydroisoindol-1-one,
   N-[2-(N-trifluoromethanesulfonyl)aminobenzyl]-7-phenyl-1,3-dihydroisoindol-1-one,
   N-[2-(N-trifluoromethanesulfonyl)aminobenzyl]-7-cyano-1,3-dihydroisoindol-1-one,
   N-[2-(N-trifluoromethanesulfonyl)aminobenzyl]-7-hydroxy-1,3-dihydroisoindol-1-one,
   N-[2-(N-trifluoromethanesulfonyl)aminobenzyl]-7-methoxy-1,3-dihydroisoindol-1-one,
   N-[2-(N-trifluoromethanesulfonyl)aminobenzyl]-7-acetoxy-1,3-dihydroisoindol-1-one,
   N-[2-(N-trifluoromethanesulfonyl)aminobenzyl]-7-benzoyloxy-1,3-dihydroisoindol-1-one,
   N-[2-(N-trifluoromethanesulfonyl)aminobenzyl]-7-(4-nitro-benzoyl)oxy-1,3-dihydroisoindol-1-one,
   N-[2-(N-trifluoromethanesulfonyl)aminobenzyl]-7-methoxycarbonyloxy-1,3-dihydroisoindol-1-one,
   N-[2-(N-trifluoromethanesulfonyl)aminobenzyl]-7-(2-propylthio)carbonyloxy-1,3-dihydroisoindol-1-one,
   N-[2-(N-trifluoromethanesulfonyl)aminobenzyl]-7-(1H-imidazol-1-yl-carbonyloxy)-1,3-dihydroisoindol-1-one,
   N-[2-(N-trifluoromethanesulfonyl)aminobenzyl]-7-(pyrrolidin-1-yl-carbonyloxy)-1,3-dihydroisoindol-1-one,
   N-[2-(N-trifluoromethanesulfonyl)aminobenzyl]-7-methanesulfonyloxy-1,3-dihydroisoindol-1-one,
   N-[2-(N-trifluoromethanesulfonyl)aminobenzyl]-7-trifluoromethanesulfonyloxy-1,3-dihydroisoindol-1-one,
   N-[2-(N-trifluoromethanesulfonyl)aminobenzyl]-7-phenylsulfonyloxy-1,3-dihydroisoindol-1-one,
   N-[2-(N-trifluoromethanesulfonyl)aminobenzyl]-7-diethylphosphorooxy-1,3-dihydroisoindol-1-one,
   N-[2-(N-trifluoromethanesulfonyl)aminobenzyl]-7-tert-butyldimethylsilyloxy-1,3-dihydroisoindol-1-one,
   N-[2-(N-trifluoromethanesulfonyl)aminobenzyl]-7-propargyloxy-1,3-dihydroisoindol-1-one,
   N-[2-(N-trifluoromethanesulfonyl)aminobenzyl]-7-allyloxy-1,3-dihydroisoindol-1-one,
   N-[2-(N-trifluoromethanesulfonyl)aminobenzyl]-7-benzyloxy-1,3-dihydroisoindol-1-one,
   N-[2-(N-trifluoromethanesulfonyl)aminobenzyl]-7-cyclopropylmethyloxy-1,3-dihydroisoindol-1-one,
   N-[2-(N-trifluoromethanesulfonyl)aminobenzyl]-7-amino-1,3-dihydroisoindol-1-one,
   N-[2-(N-trifluoromethanesulfonyl)aminobenzyl]-7-acetylamino-1,3-dihydroisoindol-1-one,
   N-[2-(N-trifluoromethanesulfonyl)aminobenzyl]-7-bisacetylamino-1,3-dihydroisoindol-1-one,
   N-[2-(N-trifluoromethanesulfonyl)aminobenzyl]-7-trifluoromethanesulfonylamino-1,3-dihydroisoindol-1-one,
   N-[2-(N-trifluoromethanesulfonyl)aminobenzyl]-7-(isoindolin-1,3-dione-2-yl)-1,3-dihydroisoindol-1-one,
   N-[2-(N-trifluoromethanesulfonyl)aminobenzyl]-6-fluoro-1,3-dihydroisoindol-1-one,
   N-[2-(N-trifluoromethanesulfonyl)aminobenzyl]-6-methyl-1,3-dihydroisoindol-1-one,
   N-[2-(N-trifluoromethanesulfonyl)aminobenzyl]-6-hydroxy-1,3-dihydroisoindol-1-one,
   N-[2-(N-trifluoromethanesulfonyl)aminobenzyl]-6-methoxy-1,3-dihydroisoindol-1-one,
   N-[2-(N-trifluoromethanesulfonyl)aminobenzyl]-6-acetoxy-1,3-dihydroisoindol-1-one,
   N-[2-(N-trifluoromethanesulfonyl)aminobenzyl]-6-trifluoromethanesulfonyloxy-1,3-dihydroisoindol-1-one,
   N-[2-(N-trifluoromethanesulfonyl)aminobenzyl]-6-propargyloxy-1,3-dihydroisoindol-1-one,
   N-[2-(N-trifluoromethanesulfonyl)aminobenzyl]-6-nitro-1,3-dihydroisoindol-1-one,
   N-[2-(N-trifluoromethanesulfonyl)aminobenzyl]-6-(isoindolin-1,3-dione-2-yl)-1,3-dihydroisoindol-1-one,
   N-[2-(N-trifluoromethanesulfonyl)aminobenzyl]-5-bromo-1,3-dihydroisoindol-1-one,
   N-[2-(N-trifluoromethanesulfonyl)aminobenzyl]-5-methyl-1,3-dihydroisoindol-1-one,
   N-[2-(N-trifluoromethanesulfonyl)aminobenzyl]-5-hydroxy-1,3-dihydroisoindol-1-one,
   N-[2-(N-trifluoromethanesulfonyl)aminobenzyl]-5-methoxy-1,3-dihydroisoindol-1-one,
   N-[2-(N-trifluoromethanesulfonyl)aminobenzyl]-5-acetoxy-1,3-dihydroisoindol-1-one,
   N-[2-(N-trifluoromethanesulfonyl)aminobenzyl]-5-propargyloxy-1,3-dihydroisoindol-1-one,
   N-[2-(N-trifluoromethanesulfonyl)aminobenzyl]-4-hydroxy-1,3-dihydroisoindol-1-one,
   N-[2-(N-trifluoromethanesulfonyl)aminobenzyl]-4-methoxy-1,3-dihydroisoindol-1-one,
   N-[2-(N-trifluoromethanesulfonyl)aminobenzyl]-4-acetoxy-1,3-dihydroisoindol-1-one,
   N-[2-(N-trifluoromethanesulfonyl)aminobenzyl]-4-trifluoromethanesulfonyloxy-1,3-dihydroisoindol-1-one,
   N-[2-(N-trifluoromethanesulfonyl)aminobenzyl]-4-propargyloxy-1,3-dihydroisoindol-1-one,
   N-[2-(N-trifluoromethanesulfonyl)aminobenzyl]-4-nitro-1,3-dihydroisoindol-1-one,
   N-[2-(N-trifluoromethanesulfonyl)aminobenzyl]-5-fluoro-6-nitro-1,3-dihydroisoindol-1-one,
   N-[2-(N-trifluoromethanesulfonyl)aminobenzyl]-5,6-dibromo-4-methoxy-1,3-dihydroisoindol-1-one,
   N-[2-(N-trifluoromethanesulfonyl)amino-5-trifluoromethyl-benzyl]-1,3-dihydroisoindol-1-one,
   N-[2-(N-trifluoromethanesulfonyl)amino-5-trifluoromethyl-benzyl]-5-bromo-1,3-dihydroisoindol-1-one,
   N-[2-(N-ethoxycarbonyl-N-trifluoromethanesulfonyl)aminobenzyl]-1,3-dihydroisoindol-1-one,
   N-[2-(N-ethoxycarbonyl-N-trifluoromethanesulfonyl)aminobenzyl]-7-amino-1,3-dihydroisoindol-1-one,
   N-[2-(N-ethoxycarbonyl-N-trifluoromethanesulfonyl)aminobenzyl]-5-bromo-1,3-dihydroisoindol-1-one,
   N-[2-(N-ethoxycarbonyl-N-trifluoromethanesulfonyl)amino-5-trifluoromethylbenzyl]-1,3-dihydroisoindol-1-one,
   N-[2-(N-ethoxycarbonyl-N-trifluoromethanesulfonyl)amino-5-trifluoromethylbenzyl]-5-bromo-1,3-dihydroisoindol-1-one,
   N-[2-(N-trifluoromethanesulfonyl)aminobenzyl]-7-methoxymethyloxy-1,3-dihydroisoindol-1-one,
   N-[2-(N-trifluoromethanesulfonyl)aminobenzyl]-3,4-dihydroisoquinolin-1(2H)-one,
   N-[2-(N-trifluoromethanesulfonyl)aminobenzyl]-8-fluoro-3,4-dihydroisoquinolin-1(2H)-one,
   N-[2-(N-trifluoromethanesulfonyl)aminobenzyl]-8-chloro-3,4-dihydroisoquinolin-1(2H)-one,
   N-[2-(N-trifluoromethanesulfonyl)aminobenzyl]-8-bromo-3,4-dihydroisoquinolin-1(2H)-one,
   N-[2-(N-trifluoromethanesulfonyl)aminobenzyl]-8-iodo-3,4-dihydroisoquinolin-1(2H)-one,
   N-[2-(N-trifluoromethanesulfonyl)aminobenzyl]-8-methyl-3,4-dihydroisoquinolin-1(2H)-one,
   N-[2-(N-trifluoromethanesulfonyl)aminobenzyl]-8-cyclopropyl-3,4-dihydroisoquinolin-1(2H)-one,
   N-[2-(N-trifluoromethanesulfonyl)aminobenzyl]-8-phenyl-3,4-dihydroisoquinolin-1(2H)-one,
   N-[2-(N-trifluoromethanesulfonyl)aminobenzyl]-8-trifluoromethyl-3,4-dihydroisoquinolin-1(2H)-one,
   N-[2-(N-trifluoromethanesulfonyl)aminobenzyl]-8-cyano-3,4-dihydroisoquinolin-1(2H)-one,
   N-[2-(N-trifluoromethanesulfonyl)aminobenzyl]-8-hydroxy-3,4-dihydroisoquinolin-1(2H)-one,
   N-[2-(N-trifluoromethanesulfonyl)aminobenzyl]-8-methoxy-3,4-dihydroisoquinolin-1(2H)-one,
   N-[2-(N-trifluoromethanesulfonyl)aminobenzyl]-8-acetoxy-3,4-dihydroisoquinolin-1(2H)-one,
   N-[2-(N-trifluoromethanesulfonyl)aminobenzyl]-8-difluoromethoxy-3,4-dihydroisoquinolin-1(2H)-one,
   N-[2-(N-trifluoromethanesulfonyl)aminobenzyl]-8-trifluoromethoxy-3,4-dihydroisoquinolin-1(2H)-one,
   N-[2-(N-trifluoromethanesulfonyl)aminobenzyl]-8-propargyloxy-3,4-dihydroisoquinolin-1(2H)-one,
   N-[2-(N-trifluoromethanesulfonyl)aminobenzyl]-8-dimethylamino-3,4-dihydroisoquinolin-1(2H)-one,
   N-[2-(N-trifluoromethanesulfonyl)aminobenzyl]-8-methoxycarbonyl-3,4-dihydroisoquinolin-1(2H)-one,
   N-[2-(N-trifluoromethanesulfonyl)aminobenzyl]-7-fluoro-3,4-dihydroisoquinolin-1(2H)-one,
   N-[2-(N-trifluoromethanesulfonyl)aminobenzyl]-7-chloro-3,4-dihydroisoquinolin-1(2H)-one,
   N-[2-(N-trifluoromethanesulfonyl)aminobenzyl]-7-bromo-3,4-dihydroisoquinolin-1(2H)-one,
   N-[2-(N-trifluoromethanesulfonyl)aminobenzyl]-7-iodo-3,4-dihydroisoquinolin-1(2H)-one,
   N-[2-(N-trifluoromethanesulfonyl)aminobenzyl]-7-methyl-3,4-dihydroisoquinolin-1(2H)-one,
   N-[2-(N-trifluoromethanesulfonyl)aminobenzyl]-7-ethyl-3,4-dihydroisoquinolin-1(2H)-one,
   N-[2-(N-trifluoromethanesulfonyl)aminobenzyl]-7-tert-butyl-3,4-dihydroisoquinolin-1(2H)-one,
   N-[2-(N-trifluoromethanesulfonyl)aminobenzyl]-7-phenyl-3,4-dihydroisoquinolin-1(2H)-one,
   N-[2-(N-trifluoromethanesulfonyl)aminobenzyl]-7-trifluoromethyl-3,4-dihydroisoquinolin-1(2H)-one,
   N-[2-(N-trifluoromethanesulfonyl)aminobenzyl]-7-nitro-3,4-dihydroisoquinolin-1(2H)-one,
   N-[2-(N-trifluoromethanesulfonyl)aminobenzyl]-7-hydroxy-3,4-dihydroisoquinolin-1(2H)-one,
   N-[2-(N-trifluoromethanesulfonyl)aminobenzyl]-7-methoxy-3,4-dihydroisoquinolin-1(2H)-one,
   N-[2-(N-trifluoromethanesulfonyl)aminobenzyl]-7-ethoxycarbonyloxy-3,4-dihydroisoquinolin-1(2H)-one,
   N-[2-(N-trifluoromethanesulfonyl)aminobenzyl]-7-benzoyloxy-3,4-dihydroisoquinolin-1(2H)-one,
   N-[2-(N-trifluoromethanesulfonyl)aminobenzyl]-7-propargyloxy-3,4-dihydroisoquinolin-1(2H)-one,
   N-[2-(N-trifluoromethanesulfonyl)aminobenzyl]-7-trifluoromethoxy-3,4-dihydroisoquinolin-1(2H)-one,
   N-[2-(N-trifluoromethanesulfonyl)aminobenzyl]-6-fluoro-3,4-dihydroisoquinolin-1(2H)-one,
   N-[2-(N-trifluoromethanesulfonyl)aminobenzyl]-6-chloro-3,4-dihydroisoquinolin-1(2H)-one,
   N-[2-(N-trifluoromethanesulfonyl)aminobenzyl]-6-bromo-3,4-dihydroisoquinolin-1(2H)-one,
   N-[2-(N-trifluoromethanesulfonyl)aminobenzyl]-6-iodo-3,4-dihydroisoquinolin-1(2H)-one,
   N-[2-(N-trifluoromethanesulfonyl)aminobenzyl]-6-methyl-3,4-dihydroisoquinolin-1(2H)-one,
   N-[2-(N-trifluoromethanesulfonyl)aminobenzyl]-6-trifluoromethyl-3,4-dihydroisoquinolin-1(2H)-one,
   N-[2-(N-trifluoromethanesulfonyl)aminobenzyl]-6-methoxy-3,4-dihydroisoquinolin-1(2H)-one,
   N-[2-(N-trifluoromethanesulfonyl)aminobenzyl]-5-fluoro-3,4-dihydroisoquinolin-1(2H)-one,
   N-[2-(N-trifluoromethanesulfonyl)aminobenzyl]-5-chloro-3,4-dihydroisoquinolin-1(2H)-one,
   N-[2-(N-trifluoromethanesulfonyl)aminobenzyl]-5-methyl-3,4-dihydroisoquinolin-1(2H)-one,
   N-[2-(N-trifluoromethanesulfonyl)aminobenzyl]-5-nitro-3,4-dihydroisoquinolin-1(2H)-one,
   N-[2-(N-trifluoromethanesulfonyl)aminobenzyl]-5-methoxy-3,4-dihydroisoquinolin-1(2H)-one,
   N-[2-(N-trifluoromethanesulfonyl)aminobenzyl]-5-acetoxy-3,4-dihydroisoquinolin-1(2H)-one,
   N-[2-(N-trifluoromethanesulfonyl)aminobenzyl]-5-methanesulfonyloxy-3,4-dihydroisoquinolin-1(2H)-one,
   N-[2-(N-trifluoromethanesulfonyl)aminobenzyl]-5-trifluoromethoxy-3,4-dihydroisoquinolin-1(2H)-one,
   N-[2-(N-trifluoromethanesulfonyl)aminobenzyl]-5-ethoxycarbonyloxy-3,4-dihydroisoquinolin-1(2H)-one,
   N-[2-(N-trifluoromethanesulfonyl)aminobenzyl]-5-benzoyloxy-3,4-dihydroisoquinolin-1(2H)-one,
   N-[2-(N-trifluoromethanesulfonyl)aminobenzyl]-5-propargyloxy-3,4-dihydroisoquinolin-1(2H)-one,
   N-[2-(N-trifluoromethanesulfonyl)aminobenzyl]-8-fluoro-7-methyl-3,4-dihydroisoquinolin-1(2H)-one,
   N-[2-(N-trifluoromethanesulfonyl)aminobenzyl]-8-fluoro-7-nitro-3,4-dihydroisoquinolin-1(2H)-one,
   N-[2-(N-trifluoromethanesulfonyl)aminobenzyl]-7,8-dichloro-3,4-dihydroisoquinolin-1(2H)-one,
   N-[2-(N-trifluoromethanesulfonyl)aminobenzyl]-8-chloro-7-methyl-3,4-dihydroisoquinolin-1(2H)-one,
   N-[2-(N-trifluoromethanesulfonyl)aminobenzyl]-6,8-difluoro-3,4-dihydroisoquinolin-1(2H)-one,
   N-[2-(N-trifluoromethanesulfonyl)aminobenzyl]-8-fluoro-6-methyl-3,4-dihydroisoquinolin-1(2H)-one,
   N-[2-(N-trifluoromethanesulfonyl)aminobenzyl]-6-cyclopropyl-8-fluoro-3,4-dihydroisoquinolin-1(2H)-one,
   N-[2-(N-trifluoromethanesulfonyl)aminobenzyl]-6-cyano-8-fluoro-3,4-dihydroisoquinolin-1(2H)-one,
   N-[2-(N-trifluoromethanesulfonyl)aminobenzyl]-6,8-dichloro-3,4-dihydroisoquinolin-1(2H)-one,
   N-[2-(N-trifluoromethanesulfonyl)aminobenzyl]-6,8-dimethyl-3,4-dihydroisoquinolin-1(2H)-one,
   N-[2-(N-trifluoromethanesulfonyl)aminobenzyl]-5,8-difluoro-3,4-dihydroisoquinolin-1(2H)-one,
   N-[2-(N-trifluoromethanesulfonyl)aminobenzyl]-5-chloro-8-fluoro-3,4-dihydroisoquinolin-1(2H)-one,
   N-[2-(N-trifluoromethanesulfonyl)aminobenzyl]-5-bromo-8-fluoro-3,4-dihydroisoquinolin-1(2H)-one,
   N-[2-(N-trifluoromethanesulfonyl)aminobenzyl]-8-fluoro-5-methyl-3,4-dihydroisoquinolin-1(2H)-one,
   N-[2-(N-trifluoromethanesulfonyl)aminobenzyl]-8-fluoro-5-trifluoromethyl-3,4-dihydroisoquinolin-1(2H)-one,
   N-[2-(N-trifluoromethanesulfonyl)aminobenzyl]-8-fluoro-5-methoxy-3,4-dihydroisoquinolin-1(2H)-one,
   N-[2-(N-trifluoromethanesulfonyl)aminobenzyl]-5-chloro-8-fluoro-3,4-dihydroisoquinolin-1(2H)-one,
   N-[2-(N-trifluoromethanesulfonyl)aminobenzyl]-5,8-dichloro-3,4-dihydroisoquinolin-1(2H)-one,
   N-[2-(N-trifluoromethanesulfonyl)aminobenzyl]-5-fluoro-8-hydroxy-3,4-dihydroisoquinolin-1(2H)-one,
   N-[2-(N-trifluoromethanesulfonyl)aminobenzyl]-5-fluoro-8-methoxy-3,4-dihydroisoquinolin-1(2H)-one,
   N-[2-(N-trifluoromethanesulfonyl)aminobenzyl]-5-fluoro-8-methanesulfonyloxy-3,4-dihydroisoquinolin-1(2H)-one,
   N-[2-(N-trifluoromethanesulfonyl)aminobenzyl]-5-fluoro-8-dimethylamino-3,4-dihydroisoquinolin-1(2H)-one,
   N-[2-(N-trifluoromethanesulfonyl)aminobenzyl]-5-fluoro-8-(N-morpholinyl)-3,4-dihydroisoquinolin-1(2H)-one,
   N-[2-(N-trifluoromethanesulfonyl)aminobenzyl]-5-fluoro-8-methylthio-3,4-dihydroisoquinolin-1(2H)-one,
   N-[2-(N-trifluoromethanesulfonyl)aminobenzyl]-5-fluoro-8-methanesulfonyl-3,4-dihydroisoquinolin-1(2H)-one,
   N-[2-(N-trifluoromethanesulfonyl)aminobenzyl]-5,8-bis(methanesulfonyl)-3,4-dihydroisoquinolin-1(2H)-one,
   N-[2-(N-trifluoromethanesulfonyl)aminobenzyl]-6,7-dichloro-3,4-dihydroisoquinolin-1(2H)-one,
   N-[2-(N-trifluoromethanesulfonyl)aminobenzyl]-6-fluoro-7-methyl-3,4-dihydroisoquinolin-1(2H)-one,
   N-[2-(N-trifluoromethanesulfonyl)aminobenzyl]-6-chloro-7-methyl-3,4-dihydroisoquinolin-1(2H)-one,
   N-[2-(N-trifluoromethanesulfonyl)aminobenzyl]-6-fluoro-7-nitro-3,4-dihydroisoquinolin-1(2H)-one,
   N-[2-(N-trifluoromethanesulfonyl)aminobenzyl]-6-chloro-7-nitro-3,4-dihydroisoquinolin-1(2H)-one,
   N-[2-(N-trifluoromethanesulfonyl)aminobenzyl]-5,6-difluoro-3,4-dihydroisoquinolin-1(2H)-one,
   N-[2-(N-trifluoromethanesulfonyl)aminobenzyl]-6,8-difluoro-7-nitro-3,4-dihydroisoquinolin-1(2H)-one,
   N-[2-(N-trifluoromethanesulfonyl)aminobenzyl]-6,8-dichloro-7-nitro-3,4-dihydroisoquinolin-1(2H)-one,
   N-[2-(N-trifluoromethanesulfonyl)aminobenzyl]-3,4-dihydrobenzo[h]isoquinolin-1(2H)-one,
   N-[2-(N-trifluoromethanesulfonyl)aminobenzyl]-3,4-dihydrobenzo[g]isoquinolin-1(2H)-one,
   N-[2-(N-trifluoromethanesulfonyl)aminobenzyl]-7,8-dihydrothiazolo[4,5-h]isoquinoline-9(6H)-one,
   N-[2-(N-methyl-N-trifluoromethanesulfonyl)aminobenzyl]-5,8-difluoro-3,4-dihydroisoquinolin-1(2H)-one,
   N-[2-(N-propargyl-N-trifluoromethanesulfonyl)aminobenzyl]-5,8-difluoro-3,4-dihydroisoquinolin-1(2H)-one,
   N-[2-(N-(4-methoxybenzyl)-N-trifluoromethanesulfonyl)aminobenzyl]-5,8-difluoro-3,4-dihydroisoquinolin-1(2H)-one,
   N-[2-[N-[3-(1,3-dioxan-2-yl)propanyl]-N-trifluoromethanesulfonyl]aminobenzyl]-5,8-difluoro-3,4-dihydroisoquinolin-1(2H)-one,
   N-[2-(N-cyanomethyl-N-trifluoromethanesulfonyl)aminobenzyl]-5,8-difluoro-3,4-dihydroisoquinolin-1(2H)-one,
   N-[2-(N-ethoxymethyl-N-trifluoromethanesulfonyl)aminobenzyl]-5,8-difluoro-3,4-dihydroisoquinolin-1(2H)-one,
   N-[2-[N-(isopropyloxycarbonyloxymethyl)-N-trifluoromethanesulfonyl]aminobenzyl]-5,8-difluoro-3,4-dihydroisoquinolin-1(2H)-one,
   N-[2-(N-acethyl-N-trifluoromethanesulfonyl)aminobenzyl]-5,8-difluoro-3,4-dihydroisoquinolin-1(2H)-one,
   N-[2-(N-ethylcarbonyl-N-trifluoromethanesulfonyl)aminobenzyl]-5,8-difluoro-3,4-dihydroisoquinolin-1(2H)-one,
   N-[2-[N-(1-propylcarbonyl)-N-trifluoromethanesulfonyl]aminobenzyl]-5,8-difluoro-3,4-dihydroisoquinolin-1(2H)-one,
   N-[2-[N-(2-propylcarbonyl)-N-trifluoromethanesulfonyl]aminobenzyl]-5,8-difluoro-3,4-dihydroisoquinolin-1(2H)-one,
   N-[2-[N-(2-methyl-1-propylcarbonyl)-N-trifluoromethanesulfonyl]aminobenzyl]-5,8-difluoro-3,4-dihydroisoquinolin-1 (2H)-one,
   N-[2-[N-(2,2-dimethyl-1-propylcarbonyl)-N-trifluoromethanesulfonyl] aminobenzyl]-5, 8-difluoro-3,4-dihydroisoquinolin-1 (2H)-one,
   N-[2-(N-chloromethylcarbonyl-N-trifluoromethanesulfonyl)aminobenzyl]-5,8-difluoro-3,4-dihydroisoquinolin-1(2H)-one,
   N-[2-(N-cyclopropylcarbonyl-N-trifluoromethanesulfonyl)aminobenzyl]-8-fluoro-3,4-dihydroisoquinolin-1(2H)-one,
   N-[2-(N-cyclopropylcarbonyl-N-trifluoromethanesulfonyl)aminobenzyl]-8-chloro-3,4-dihydroisoquinolin-1(2H)-one,
   N-[2-(N-cyclopropylcarbonyl-N-trifluoromethanesulfonyl)aminobenzyl]-6-chloro-3,4-dihydroisoquinolin-1(2H)-one,
   N-[2-(N-cyclopropylcarbonyl-N-trifluoromethanesulfonyl)aminobenzyl]-5, 8-difluoro-3,4-dihydroisoquinolin-1(2H)-one,
   N-[2-(N-cyclopropylcarbonyl-N-trifluoromethanesulfonyl)aminobenzyl]-8-fluoro-5-methyl-3,4-dihydroisoquinolin-1(2H)-one,
   N-[2-[N-(2-methyl-cyclopropyl)carbonyl-N-trifluoromethanesulfonyl]aminobenzyl]-5,8-difluoro-3,4-dihydroisoquinolin-1(2H)-one,
   N-[2-[N-(2,2-dimethyl-cyclopropyl)carbonyl-N-trifluoromethanesulfonyl]aminobenzyl]-5,8-difluoro-3,4-dihydroisoquinolin-1(2H)-one,
   N-[2-[N-(2,2-dichloro-1-methyl-cyclopropyl)carbonyl-N-trifluoromethanesulfonyl]aminobenzyl]-5,8-difluoro-3,4-dihydroisoquinolin-1(2H)-one,
   N-[2-(N-cyclobutylcarbonyl-N-trifluoromethanesulfonyl)aminobenzyl]-5,8-difluoro-3,4-dihydroisoquinolin-1(2H)-one,
   N-[2-(N-cyclopentylcarbonyl-N-trifluoromethanesulfonyl)aminobenzyl]-5,8-difluoro-3,4-dihydroisoquinolin-1(2H)-one,
   N-[2-(N-cyclohexylcarbonyl-N-trifluoromethanesulfonyl)aminobenzyl]-5,8-difluoro-3,4-dihydroisoquinolin-1(2H)-one,
   N-[2-(N-acryloyl-N-trifluoromethanesulfonyl)aminobenzyl]-5,8-difluoro-3,4-dihydroisoquinolin-1(2H)-one,
   N-[2-[N-(prop-1-en-1-yl)carbonyl-N-trifluoromethanesulfonyl]aminobenzyl]-5,8-difluoro-3,4-dihydroisoquinolin-1(2H)-one,
   N-[2-[N-(2-methyl-prop-1-en-1-yl)carbonyl-N-trifluoromethanesulfonyl]aminobenzyl]-5,8-difluoro-3,4-dihydroisoquinolin-1(2H)-one,
   N-[2-[N-(prop-1-en-2-yl)carbonyl-N-trifluoromethanesulfonyl)aminobenzyl]-5,8-difluoro-3,4-dihydroisoquinolin-1(2H)-one,
   N-[2-[N-(3-ethoxyacryloyl)-N-trifluoromethanesulfonyl]aminobenzyl]-5,8-difluoro-3,4-dihydroisoquinolin-1(2H)-one,
   N-[2-(N-benzoyl-N-trifluoromethanesulfonyl)aminobenzyl]-5,8-difluoro-3,4-dihydroisoquinolin-1(2H)-one,
   N-[2-[N-(furan-2-yl)carbonyl-N-trifluoromethanesulfonyl]aminobenzyl]-5,8-difluoro-3,4-dihydroisoquinolin-1(2H)-one,
   N-[2-[N-(thiophen-2-yl)carbonyl-N-trifluoromethanesulfonyl] aminobenzyl]-5, 8-difluoro-3,4-dihydroisoquinolin-1(2H)-one,
   N-[2-[N-(1-methyl-1H-pyrrol-2-yl)carbonyl-N-trifluoromethanesulfonyl]aminobenzyl]-5,8-difluoro-3,4-dihydroisoquinolin-1(2H)-one,
   N-[2-[N-(furan-3-yl)carbonyl-N-trifluoromethanesulfonyl]aminobenzyl]-5,8-difluoro-3,4-dihydroisoquinolin-1(2H)-one,
   N-[2-[N-(tetrahydrofuran-2-yl)carbonyl-N-trifluoromethanesulfonyl]aminobenzyl]-5,8-difluoro-3,4-dihydroisoquinolin-1(2H)-one,
   N-[2-[N-(tetrahydrofuran-3-yl)carbonyl-N-trifluoromethanesulfonyl]aminobenzyl]-5,8-difluoro-3,4-dihydroisoquinolin-1(2H)-one,
   N-[2-[N-(tetrahydropyran-4-yl)carbonyl-N-trifluoromethanesulfonyl]aminobenzyl]-5,8-difluoro-3,4-dihydroisoquinolin-1(2H)-one,
   N-[2-[N-(2-cyclopropylacetyl)-N-trifluoromethanesulfonyl]aminobenzyl]-5,8-difluoro-3,4-dihydroisoquinolin-1(2H)-one,
   N-[2-[N-(2-hydroxyethyl)carbonyl-N-trifluoromethanesulfonyl]aminobenzyl]-5,8-difluoro-3,4-dihydroisoquinolin-1(2H)-one,
   N-[2-(N-methoxyacetyl-N-trifluoromethanesulfonyl)aminobenzyl]-5,8-difluoro-3,4-dihydroisoquinolin-1(2H)-one,
   N-[2-(N-ethoxyacetyl-N-trifluoromethanesulfonyl)aminobenzyl]-5,8-difluoro-3,4-dihydroisoquinolin-1(2H)-one,
   N-[2-[N-(2-methoxyethyl)carbonyl-N-trifluoromethanesulfonyl]aminobenzyl]-5,8-difluoro-3,4-dihydroisoquinolin-1(2H)-one,
   N-[2-[N-(2-tert-butyldimethylsilyloxyethyl)carbonyl-N-trifluoromethanesulfonyl]aminobenzyl]-5,8-difluoro-3,4-dihydroisoquinolin-1(2H)-one,
   N-[2-(N-acetoxyacetyl-N-trifluoromethanesulfonyl)aminobenzyl]-5,8-difluoro-3,4-dihydroisoquinolin-1(2H)-one,
   N-[2-(N-methylsulfylacetyl-N-trifluoromethanesulfonyl)aminobenzyl]-5,8-difluoro-3,4-dihydroisoquinolin-1(2H)-one,
   N-[2-(N-ethylsulfylacetyl-N-trifluoromethanesulfonyl)aminobenzyl]-5,8-difluoro-3,4-dihydroisoquinolin-1(2H)-one,
   N-[2-[N-(1-methylsulfylethyl)carbonyl-N-trifluoromethanesulfonyl]aminobenzyl]-5,8-difluoro-3,4-dihydroisoquinolin-1(2H)-one,
   N-[2-(N-methoxycarbonyl-N-trifluoromethanesulfonyl)aminobenzyl]-5,8-difluoro-3,4-dihydroisoquinolin-1(2H)-one,
   N-[2-(N-ethoxycarbonyl-N-trifluoromethanesulfonyl)aminobenzyl]-3,4-dihydroisoquinolin-1(2H)-one,
   N-[2-(N-ethoxycarbonyl-N-trifluoromethanesulfonyl)aminobenzyl]-8-fluoro-3,4-dihydroisoquinolin-1(2H)-one,
   N-[2-(N-ethoxycarbonyl-N-trifluoromethanesulfonyl)aminobenzyl]-8-cyclopropyl-3,4-dihydroisoquinolin-1(2H)-one,
   N-[2-(N-ethoxycarbonyl-N-trifluoromethanesulfonyl)aminobenzyl]-8-cyano-3,4-dihydroisoquinolin-1(2H)-one,
   N-[2-(N-ethoxycarbonyl-N-trifluoromethanesulfonyl)aminobenzyl]-8-hydroxy-3,4-dihydroisoquinolin-1(2H)-one,
   N-[2-(N-ethoxycarbonyl-N-trifluoromethanesulfonyl)aminobenzyl]-8-phenyl-3,4-dihydroisoquinolin-1(2H)-one,
   N-[2-(N-ethoxycarbonyl-N-trifluoromethanesulfonyl)aminobenzyl]-5-methyl-3,4-dihydroisoquinolin-1(2H)-one,
   N-[2-(N-ethoxycarbonyl-N-trifluoromethanesulfonyl)aminobenzyl]-8-fluoro-6-methyl-3,4-dihydroisoquinolin-1(2H)-one,
   N-[2-(N-ethoxycarbonyl-N-trifluoromethanesulfonyl)aminobenzyl]-6-cyclopropyl-8-fluoro-3,4-dihydroisoquinolin-1(2H)-one,
   N-[2-(N-ethoxycarbonyl-N-trifluoromethanesulfonyl)aminobenzyl]-6-cyano-8-fluoro-3,4-dihydroisoquinolin-1(2H)-one,
   N-[2-(N-ethoxycarbonyl-N-trifluoromethanesulfonyl)aminobenzyl]-5,8-difluoro-3,4-dihydroisoquinolin-1(2H)-one,
   N-[2-(N-ethoxycarbonyl-N-trifluoromethanesulfonyl)aminobenzyl]-5-bromo-8-fluoro-3,4-dihydroisoquinolin-1(2H)-one,
   N-[2-(N-ethoxycarbonyl-N-trifluoromethanesulfonyl)aminobenzyl]-8-fluoro-5-methyl-3,4-dihydroisoquinolin-1(2H)-one,
   N-[2-(N-ethoxycarbonyl-N-trifluoromethanesulfonyl)aminobenzyl]-8-fluoro-5-trifluoromethyl-3,4-dihydroisoquinolin-1(2H)-one,
   N-[2-(N-ethoxycarbonyl-N-trifluoromethanesulfonyl)aminobenzyl]-8-fluoro-5-methoxy-3,4-dihydroisoquinolin-1(2H)-one,
   N-[2-(N-ethoxycarbonyl-N-trifluoromethanesulfonyl)aminobenzyl]-8-chloro-5-fluoro-3,4-dihydroisoquinolin-1(2H)-one,
   N-[2-(N-ethoxycarbonyl-N-trifluoromethanesulfonyl)aminobenzyl]-8-dimethylamino-5-fluoro-3,4-dihydroisoquinolin-1(2H)-one,
   N-[2-(N-ethoxycarbonyl-N-trifluoromethanesulfonyl)aminobenzyl]-5-fluoro-8-(N-morpholinyl)-3,4-dihydroisoquinolin-1(2H)-one,
   N-[2-(N-ethoxycarbonyl-N-trifluoromethanesulfonyl)aminobenzyl]-5-fluoro-8-methylthio-3,4-dihydroisoquinolin-1(2H)-one,
   N-[2-(N-ethoxycarbonyl-N-trifluoromethanesulfonyl)aminobenzyl]-5,8-bis(methylthio)-3,4-dihydroisoquinolin-1(2H)-one,
   N-[2-(N-ethoxycarbonyl-N-trifluoromethanesulfonyl)aminobenzyl]-5-fluoro-8-methanesulfonyl-3,4-dihydroisoquinolin-1(2H)-one,
   N-[2-(N-ethoxycarbonyl-N-trifluoromethanesulfonyl)aminobenzyl]-5,6-difluoro-3,4-dihydroisoquinolin-1(2H)-one,
   N-[2-[N-(1-propyl)oxycarbonyl-N-trifluoromethanesulfonyl]aminobenzyl]-5,8-difluoro-3,4-dihydroisoquinolin-1(2H)-one,
   N-[2-[N-(2-propyl)oxycarbonyl-N-trifluoromethanesulfonyl]aminobenzyl]-5,8-difluoro-3,4-dihydroisoquinolin-1(2H)-one,
   N-[2-[N-(1-butyl)oxycarbonyl-N-trifluoromethanesulfonyl]aminobenzyl]-5,8-difluoro-3,4-dihydroisoquinolin-1(2H)-one,
   N-[2-[N-(2-methyl-1-propyl)oxycarbonyl-N-trifluoromethanesulfonyl]aminobenzyl]-5,8-difluoro-3,4-dihydroisoquinolin-1(2H)-one,
   N-[2-[N-(2-butyl)oxycarbonyl-N-trifluoromethanesulfonyl]aminobenzyl]-5,8-difluoro-3,4-dihydroisoquinolin-1(2H)-one,
   N-[2-[N-(1,1-dimethyl-ethyl)oxycarbonyl-N-trifluoromethanesulfonyl]aminobenzyl]-5,8-difluoro-3,4-dihydroisoquinolin-1(2H)-one,
   N-[2-[N-(1-pentyl)oxycarbonyl-N-trifluoromethanesulfonyl]aminobenzyl]-5,8-difluoro-3,4-dihydroisoquinolin-1(2H)-one,
   N-[2-[N-(1-hexyl)oxycarbonyl-N-trifluoromethanesulfonyl]aminobenzyl]-5,8-difluoro-3,4-dihydroisoquinolin-1(2H)-one,
   N-[2-[N-(1-heptyl)oxycarbonyl-N-trifluoromethanesulfonyl]aminobenzyl]-5,8-difluoro-3,4-dihydroisoquinolin-1(2H)-one,
   N-[2-[N-(2,2-dimethyl-1-propyl)oxycarbonyl-N-trifluoromethanesulfonyl]aminobenzyl]-5,8-difluoro-3,4-dihydroisoquinolin-1(2H)-one,
   N-[2-[N-(chloromethyl)oxycarbonyl-N-trifluoromethanesulfonyl]aminobenzyl]-5,8-difluoro-3,4-dihydroisoquinolin-1(2H)-one,
   N-[2-[N-(trichloromethyl)oxycarbonyl-N-trifluoromethanesulfonyl]aminobenzyl]-5,8-difluoro-3,4-dihydroisoquinolin-1(2H)-one,
   N-[2-[N-(1-chloroethyl)oxycarbonyl-N-trifluoromethanesulfonyl]aminobenzyl]-5,8-difluoro-3,4-dihydroisoquinolin-1(2H)-one,
   N-[2-[N-(2-fluoroethyl)oxycarbonyl-N-trifluoromethanesulfonyl]aminobenzyl]-8-fluoro-3,4-dihydroisoquinolin-1(2H)-one,
   N-[2-[N-(2-fluoroethyl)oxycarbonyl-N-trifluoromethanesulfonyl]aminobenzyl]-5,8-difluoro-3,4-dihydroisoquinolin-1(2H)-one,
   N-[2-[N-(2,2-difluoroethyl)oxycarbonyl-N-trifluoromethanesulfonyl]aminobenzyl]-3,4-dihydroisoquinolin-1(2H)-one,
   N-[2-[N-(2,2-difluoroethyl)oxycarbonyl-N-trifluoromethanesulfonyl]aminobenzyl]-8-fluoro-3,4-dihydroisoquinolin-1(2H)-one,
   N-[2-[N-(2,2-difluoroethyl)oxycarbonyl-N-trifluoromethanesulfonyl]aminobenzyl]-5,8-difluoro-3,4-dihydroisoquinolin-1(2H)-one,
   N-[2-[N-(2,2,2-trifluoroethyl)oxycarbonyl-N-trifluoromethanesulfonyl] aminobenzyl]-5, 8-difluoro-3,4-dihydroisoquinolin-1 (2H)-one,
   N-[2-[N-(2-chloroethyl)oxycarbonyl-N-trifluoromethanesulfonyl]aminobenzyl]-5,8-difluoro-3,4-dihydroisoquinolin-1(2H)-one,
   N-[2-[N-(2,2-difluoro-1-methyl-ethyl)oxycarbonyl-N-trifluoromethanesulfonyl]aminobenzyl]-5,8-difluoro-3,4-dihydroisoquinolin-1(2H)-one,
   N-[2-[N-(2,2,2-trifluoro-1-methyl-ethyl)oxycarbonyl-N-trifluoromethanesulfonyl] aminobenzyl]-5, 8-difluoro-3,4-dihydroisoquinolin-1 (2H)-one,
   N-[2-[N-(1,3-difluoropropan-2-yl)oxycarbonyl-N-trifluoromethanesulfonyl]aminobenzyl]-5,8-difluoro-3,4-dihydroisoquinolin-1(2H)-one,
   N-[2-(N-cyclopropyloxycarbonyl-N-trifluoromethanesulfonyl)aminobenzyl]-5,8-difluoro-3,4-dihydroisoquinolin-1(2H)-one,
   N-[2-(N-cyclobutyloxycarbonyl-N-trifluoromethanesulfonyl)aminobenzyl]-5,8-difluoro-3,4-dihydroisoquinolin-1(2H)-one,
   N-[2-(N-cyclopentyloxycarbonyl-N-trifluoromethanesulfonyl)aminobenzyl]-5,8-difluoro-3,4-dihydroisoquinolin-1(2H)-one,
   N-[2-(N-cyclohexyloxycarbonyl-N-trifluoromethanesulfonyl)aminobenzyl]-5,8-difluoro-3,4-dihydroisoquinolin-1(2H)-one,
   N-[2-(N-allyloxycarbonyl-N-trifluoromethanesulfonyl)aminobenzyl]-5,8-difluoro-3,4-dihydroisoquinolin-1(2H)-one,
   N-[2-[N-(but-3-en-2-yl)oxycarbonyl-N-trifluoromethanesulfonyl]aminobenzyl]-5,8-difluoro-3,4-dihydroisoquinolin-1(2H)-one,
   N-[2-[N-(2-methylallyl)oxycarbonyl-N-trifluoromethanesulfonyl]aminobenzyl]-5,8-difluoro-3,4-dihydroisoquinolin-1(2H)-one,
   N-[2-[N-(3-methylbut-2-en-1-yl)oxycarbonyl-N-trifluoromethanesulfonyl]aminobenzyl]-5,8-difluoro-3,4 dihydroisoquinolin-1(2H)-one,
   N-[2-(N-propargyloxycarbonyl-N-trifluoromethanesulfonyl)aminobenzyl]-5,8-difluoro-3,4-dihydroisoquinolin-1(2H)-one,
   N-[2-[N-(but-3-yn-2-yl)oxycarbonyl-N-trifluoromethanesulfonyl]aminobenzyl]-5,8-difluoro-3,4-dihydroisoquinolin-1(2H)-one,
   N-[2-(N-phenoxycarbonyl-N-trifluoromethanesulfonyl)aminobenzyl]-5,8-difluoro-3,4-dihydroisoquinolin-1(2H)-one,
   N-[2-[N-(4-nitro-phenoxy)carbonyl-N-trifluoromethanesulfonyl]aminobenzyl]-5,8-difluoro-3,4-dihydroisoquinolin-1(2H)-one,
   N-[2-[N-(oxetan-3-yl)oxycarbonyl-N-trifluoromethanesulfonyl]aminobenzyl]-3,4-dihydroisoquinolin-1(2H)-one,
   N-[2-[N-(oxetan-3-yl)oxycarbonyl-N-trifluoromethanesulfonyl]aminobenzyl]-8-fluoro-3,4-dihydroisoquinolin-1(2H)-one,
   N-[2-[N-(oxetan-3-yl)oxycarbonyl-N-trifluoromethanesulfonyl]aminobenzyl]-5,8-difluoro-3,4-dihydroisoquinolin-1(2H)-one,
   N-[2-[N-(tetrahydrofuran-3-yl)oxycarbonyl-N-trifluoromethanesulfonyl]aminobenzyl]-3,4-dihydroisoquinolin-1(2H)-one,
   N-[2-[N-(tetrahydrofuran-3-yl)oxycarbonyl-N-trifluoromethanesulfonyl]aminobenzyl]-5,8-difluoro-3,4-dihydroisoquinolin-1(2H)-one,
   N-[2-[N-((S)-tetrahydrofuran-3-yl)oxycarbonyl-N-trifluoromethanesulfonyl]aminobenzyl]-5,8-difluoro-3,4-dihydroisoquinolin-1(2H)-one,
   N-[2-[N-((R)-tetrahydrofuran-3-yl)oxycarbonyl-N-trifluoromethanesulfonyl] aminobenzyl]-5, 8-difluoro-3,4-dihydroisoquinolin-1 (2H)-one,
   N-[2-[N-(tetrahydrothiophen-3-yl)oxycarbonyl-N-trifluoromethanesulfonyl] aminobenzyl]-5, 8-difluoro-3,4-dihydroisoquinolin-1 (2H)-one,
   N-[2-[N-(1,1-dioxydotetrahydrothiophen-3-yl)oxycarbonyl-N-trifluoromethanesulfonyl]aminobenzyl]-5,8-difluoro-3,4-dihydroisoquinolin-1(2H)-one,
   N-[2-[N-(tetrahydro-2H-pyran-3-yl)oxycarbonyl-N-trifluoromethanesulfonyl] aminobenzyl]-5, 8-difluoro-3,4-dihydroisoquinolin-1 (2H)-one,
   N-[2-[N-(tetrahydro-2H-pyran-4-yl)oxycarbonyl-N-trifluoromethanesulfonyl]aminobenzyl]-5,8-difluoro-3,4-dihydroisoquinolin-1(2H)-one,
   N-[2-[N-(1,3-dioxan-5-yl)oxycarbonyl-N-trifluoromethanesulfonyl]aminobenzyl]-5,8-difluoro-3,4-dihydroisoquinolin-1(2H)-one,
   N-[2-[N-(2,2-dimethyl-1,3-dioxan-5-yl)oxycarbonyl-N-trifluoromethanesulfonyl]aminobenzyl]-5,8-difluoro-3,4 dihydroisoquinolin-1(2H)-one,
   N-[2-[N-(tetrahydro-2H-thiopyran-4-yl)oxycarbonyl-N-trifluoromethanesulfonyl]aminobenzyl]-5,8-difluoro-3,4-dihydroisoquinolin-1(2H)-one,
   N-[2-[N-(1,1-dioxydotetrahydro-2H-thiopyran-4-yl)oxycarbonyl-N-trifluoromethanesulfonyl]aminobenzyl]-5,8-difluoro-3,4-dihydroisoquinolin-1(2H)-one,
   N-[2-[N-(2,5-dioxopyrrolidin-1-yl)oxycarbonyl-N-trifluoromethanesulfonyl]aminobenzyl]-5,8-difluoro-3,4-dihydroisoquinolin-1(2H)-one,
   N-[2-[N-(cyclopropylmethyloxy)carbonyl-N-trifluoromethanesulfonyl]aminobenzyl]-5,8-difluoro-3,4-dihydroisoquinolin-1(2H)-one,
   N-[2-(N-benzyloxycarbonyl-N-trifluoromethanesulfonyl)aminobenzyl]-5,8-difluoro-3,4-dihydroisoquinolin-1(2H)-one,
   N-[2-[N-(oxirane-2-ylmethyl)oxycarbonyl-N-trifluoromethanesulfonyl]aminobenzyl]-5,8-difluoro-3,4-dihydroisoquinolin-1(2H)-one,
   N-[2-[N-[(2-methyloxiran-2-yl)methyl]oxycarbonyl-N-trifluoromethanesulfonyl] aminobenzyl]-5, 8-difluoro-3,4-dihydroisoquinolin-1 (2H)-one,
   N-[2-[N-[(3,3-dimethyloxiran-2-yl)methyl]oxycarbonyl-N-trifluoromethanesulfonyl] aminobenzyl]-5, 8-difluoro-3,4-dihydroisoquinolin-1 (2H)-one,
   N-[2-[N-[1-(oxiran-2-yl)ethyl]oxycarbonyl-N-trifluoromethanesulfonyl] aminobenzyl]-5, 8-difluoro-3,4-dihydroisoquinolin-1 (2H)-one,
   N-[2-[N-(oxetan-2-ylmethyl)oxycarbonyl-N-trifluoromethanesulfonyl] aminobenzyl]-5, 8-difluoro-3,4-dihydroisoquinolin-1 (2H)-one,
   N-[2-[N-(oxetan-3-ylmethyl)oxycarbonyl-N-trifluoromethanesulfonyl] aminobenzyl]-5, 8-difluoro-3,4-dihydroisoquinolin-1 (2H)-one,
   N-[2-[N-[(tetrahydrofuran-2-yl)methyl]oxycarbonyl-N-trifluoromethanesulfonyl] aminobenzyl]-5, 8-difluoro-3,4-dihydroisoquinolin-1 (2H)-one,
   N-[2-[N-[(tetrahydrofuran-3-yl)methyl]oxycarbonyl-N-trifluoromethanesulfonyl] aminobenzyl]-5, 8-difluoro-3,4-dihydroisoquinolin-1 (2H)-one,
   N-[2-[N-[(tetrahydro-2H-pyran-2-yl)methyl]oxycarbonyl-N-trifluoromethanesulfonyl] aminobenzyl]-5, 8-difluoro-3,4-dihydroisoquinolin-1 (2H)-one,
   N-[2-[N-[(tetrahydro-2H-pyran-4-yl)methyl]oxycarbonyl-N-trifluoromethanesulfonyl] aminobenzyl]-5, 8-difluoro-3,4-dihydroisoquinolin-1 (2H)-one,
   N-[2-[N-[(2-oxo-1,3-dioxolan-4-yl)methyl]oxycarbonyl-N-trifluoromethanesulfonyl] aminobenzyl]-5, 8-difluoro-3,4-dihydroisoquinolin-1 (2H)-one,
   N-[2-[N-[(5-methyl-2-oxo-1,3-dioxol-4-yl)methyl]oxycarbonyl-N-trifluoromethanesulfonyl] aminobenzyl]-5, 8-difluoro-3,4-dihydroisoquinolin-1 (2H)-one,
   N-[2-[N-(2-cyano-ethoxy)carbonyl-N-trifluoromethanesulfonyl]aminobenzyl]-5,8-difluoro-3,4-dihydroisoquinolin-1(2H)-one,
   N-[2-[N-(2-hydroxy-ethoxy)carbonyl-N-trifluoromethanesulfonyl]aminobenzyl]-5,8-difluoro-3,4-dihydroisoquinolin-1 (2H)-one,
   N-[2-[N-(2-methoxy-ethoxy)carbonyl-N-trifluoromethanesulfonyl]aminobenzyl]-5,8-difluoro-3,4-dihydroisoquinolin-1 (2H)-one,
   N-[2-[N-(1-methyl-2-methoxy-ethoxy)carbonyl-N-trifluoromethanesulfonyl]aminobenzyl]-5,8-difluoro-3,4-dihydroisoquinolin-1(2H)-one,
   N-[2-[N-(1,3-dimethoxypropan-2-yl)oxycarbonyl-N-trifluoromethanesulfonyl]aminobenzyl]-5,8-difluoro-3,4-dihydroisoquinolin-1(2H)-one,
   N-[2-[N-[2-(tert-butyldiphenylsilyloxy)ethyl]oxycarbonyl-N-trifluoromethanesulfonyl] aminobenzyl]-5, 8-difluoro-3,4-dihydroisoquinolin-1 (2H)-one,
   N-[2-[N-[2-(acetyloxy)ethoxy]carbonyl-N-trifluoromethanesulfonyl]aminobenzyl]-5,8-difluoro-3,4-dihydroisoquinolin-1 (2H)-one,
   N-[2-[N-[2-(methanesulfonyloxy)ethoxy]carbonyl-N-trifluoromethanesulfonyl]aminobenzyl]-5,8-difluoro-3,4-dihydroisoquinolin-1(2H)-one,
   N-[2-[N-(2-oxopropyl)oxycarbonyl-N-trifluoromethanesulfonyl]aminobenzyl]-5,8-difluoro-3,4-dihydroisoquinolin-1(2H)-one,
   N-[2-[N-(methoxycarbonylmethoxycarbonyl)oxycarbonyl-N-trifluoromethanesulfonyl]aminobenzyl]-5,8-difluoro-3,4-dihydroisoquinolin-1(2H)-one,
   N-[2-[N-(2-methylthio-ethoxy)carbonyl-N-trifluoromethanesulfonyl]aminobenzyl]-5,8-difluoro-3,4-dihydroisoquinolin-1 (2H)-one,
   N-[2-[N-(2-methanesulfonyl-ethoxy)carbonyl-N-trifluoromethanesulfonyl]aminobenzyl]-5,8-difluoro-3,4-dihydroisoquinolin-1(2H)-one,
   N-[2-[N-(2-propylthio)carbonyl-N-trifluoromethanesulfonyl]aminobenzyl]-3,4-dihydroisoquinolin-1(2H)-one,
   N-[2-[N-(2-propylthio)carbonyl-N-trifluoromethanesulfonyl]aminobenzyl]-8-fluoro-3,4-dihydroisoquinolin-1(2H)-one,
   N-[2-(N-ethylthio-carbonyl-N-trifluoromethanesulfonyl)aminobenzyl]-5,8-difluoro-3,4-dihydroisoquinolin-1(2H)-one,
   N-[2-[N-(2-propylthio)carbonyl-N-trifluoromethanesulfonyl]aminobenzyl]-5,8-difluoro-3,4-dihydroisoquinolin-1(2H)-one,
   N-[2-[N-(2-butyl)thio-carbonyl-N-trifluoromethanesulfonyl]aminobenzyl]-5,8-difluoro-3,4-dihydroisoquinolin-1(2H)-one,
   N-[2-(N-methylaminocarbonyl-N-trifluoromethanesulfonyl)aminobenzyl]-5,8-difluoro-3,4-dihydroisoquinolin-1(2H)-one,
   N-[2-[N-(2-propylamino)carbonyl-N-trifluoromethanesulfonyl]aminobenzyl]-5,8-difluoro-3,4-dihydroisoquinolin-1(2H)-one,
   N-[2-(N-dimethylaminocarbonyl-N-trifluoromethanesulfonyl)aminobenzyl]-5,8-difluoro-3,4-dihydroisoquinolin-1(2H)-one,
   N-[2-(N-methanesulfonyl-N-trifluoromethanesulfonyl)aminobenzyl]-3,4-dihydroisoquinolin-1(2H)-one,
   N-[2-(N-methanesulfonyl-N-trifluoromethanesulfonyl)aminobenzyl]-5,8-difluoro-3,4-dihydroisoquinolin-1(2H)-one,
   N-[2-(N-methanesulfonyl-N-trifluoromethanesulfonyl)aminobenzyl]-5-fluoro-8-hydroxy-3,4-dihydroisoquinolin-1(2H)-one,
   N-[2-(N-ethanesulfonyl-N-trifluoromethanesulfonyl)aminobenzyl]-5,8-difluoro-3,4-dihydroisoquinolin-1(2H)-one,
   N-[2-[N,N-bis(trifluoromethanesulfonyl)]aminobenzyl]-8-hydroxy-3,4-dihydroisoquinolin-1(2H)-one,
   N-[2-[N,N-bis(trifluoromethanesulfonyl)]aminobenzyl]-8-propargyloxy-3,4-dihydroisoquinolin-1(2H)-one,
   N-[2-[N,N-bis(trifluoromethanesulfonyl)]aminobenzyl]-5,8-difluoro-3,4-dihydroisoquinolin-1(2H)-one,
   N-[2-(N-cyclopropanesulfonyl-N-trifluoromethanesulfonyl)aminobenzyl]-5,8-difluoro-3,4-dihydroisoquinolin-1(2H)-one,
   sodium[2-[(5,8-difluoro-1-oxo-3,4-dihydroisoquinolin-2(1H)-yl)methyl]phenyl] [(trifluoromethyl)sulfonyl]amide,
   potassium[2-[(5,8-difluoro-1-oxo-3,4-dihydroisoquinolin-2(1H)-yl)methyl]phenyl] [(trifluoromethyl)sulfonyl]amide,
   N-[2-(N-trifluoromethanesulfonyl)amino-3-chloro-benzyl]-3,4-dihydroisoquinolin-1(2H)-one,
   N-[2-(N-trifluoromethanesulfonyl)amino-4-methyl-benzyl]-3,4-dihydroisoquinolin-1(2H)-one,
   N-[2-(N-trifluoromethanesulfonyl)amino-4-methoxy-benzyl]-3,4-dihydroisoquinolin-1(2H)-one,
   N-[2-(N-trifluoromethanesulfonyl)amino-5-fluoro-benzyl]-3,4-dihydroisoquinolin-1(2H)-one,
   N-[2-(N-trifluoromethanesulfonyl)amino-5-chloro-benzyl]-3,4-dihydroisoquinolin-1(2H)-one,
   N-[2-(N-trifluoromethanesulfonyl)amino-5-bromo-benzyl]-3,4-dihydroisoquinolin-1(2H)-one,
   N-[2-(N-trifluoromethanesulfonyl)amino-5-methyl-benzyl]-3,4-dihydroisoquinolin-1(2H)-one,
   N-[2-(N-trifluoromethanesulfonyl)amino-6-chloro-benzyl]-3,4-dihydroisoquinolin-1(2H)-one,
   N-[2-(N-trifluoromethanesulfonyl)amino-3,5-dichloro-benzyl]-3,4-dihydroisoquinolin-1(2H)-one,
   N-[2-(N-trifluoromethanesulfonyl)amino-5-chloro-benzyl]-8-chloro-3,4-dihydroisoquinolin-1(2H)-one,
   N-[2-(N-trifluoromethanesulfonyl)amino-5-chloro-benzyl]-6-chloro-3,4-dihydroisoquinolin-1(2H)-one,
   N-[2-(N-trifluoromethanesulfonyl)amino-3-fluoro-benzyl]-5,8-difluoro-3,4-dihydroisoquinolin-1(2H)-one,
   N-[2-(N-trifluoromethanesulfonyl)amino-3-chloro-benzyl]-5,8-difluoro-3,4-dihydroisoquinolin-1(2H)-one,
   N-[2-(N-trifluoromethanesulfonyl)amino-3-methyl-benzyl]-5,8-difluoro-3,4-dihydroisoquinolin-1(2H)-one,
   N-[2-(N-trifluoromethanesulfonyl)amino-4-fluoro-benzyl]-5,8-difluoro-3,4-dihydroisoquinolin-1(2H)-one,
   N-[2-(N-trifluoromethanesulfonyl)amino-5-fluoro-benzyl]-5,8-difluoro-3,4-dihydroisoquinolin-1(2H)-one,
   N-[2-(N-trifluoromethanesulfonyl)amino-5-chloro-benzyl]-5,8-difluoro-3,4-dihydroisoquinolin-1(2H)-one,
   N-[2-(N-trifluoromethanesulfonyl)amino-5-methyl-benzyl]-5,8-difluoro-3,4-dihydroisoquinolin-1(2H)-one,
   N-[2-(N-trifluoromethanesulfonyl)amino-5-hydroxy-benzyl]-5,8-difluoro-3,4-dihydroisoquinolin-1(2H)-one,
   N-[2-(N-trifluoromethanesulfonyl)amino-5-methoxy-benzyl]-5,8-difluoro-3,4-dihydroisoquinolin-1(2H)-one,
   N-[2-(N-trifluoromethanesulfonyl)amino-6-fluoro-benzyl]-5,8-difluoro-3,4-dihydroisoquinolin-1(2H)-one,
   N-[2-(N-trifluoromethanesulfonyl)amino-6-chloro-benzyl]-5,8-difluoro-3,4-dihydroisoquinolin-1(2H)-one,
   N-[2-(N-trifluoromethanesulfonyl)amino-6-methyl-benzyl]-5,8-difluoro-3,4-dihydroisoquinolin-1(2H)-one,
   N-[2-(N-trifluoromethanesulfonyl)amino-naphthalen-3-yl-methyl]-5,8-difluoro-3,4-dihydroisoquinolin-1(2H)-one,
   N-[2-(N-ethoxycarbonyl-N-trifluoromethanesulfonyl)amino-5-chloro-benzyl]-8-chloro-3,4-dihydroisoquinolin-1(2H)-one,
   N-[2-(N-ethoxycarbonyl-N-trifluoromethanesulfonyl)amino-3-fluoro-benzyl]-5,8-difluoro-3,4-dihydroisoquinolin-1(2H)-one,
   N-[2-(N-ethoxycarbonyl-N-trifluoromethanesulfonyl)amino-3-chloro-benzyl]-5,8-difluoro-3,4-dihydroisoquinolin-1(2H)-one,
   N-[2-(N-ethoxycarbonyl-N-trifluoromethanesulfonyl)amino-3-methyl-benzyl]-5,8-difluoro-3,4-dihydroisoquinolin-1(2H)-one,
   N-[2-(N-ethoxycarbonyl-N-trifluoromethanesulfonyl)amino-4-fluoro-benzyl]-5,8-difluoro-3,4-dihydroisoquinolin-1(2H)-one,
   N-[2-(N-ethoxycarbonyl-N-trifluoromethanesulfonyl)amino-5-fluoro-benzyl]-5,8-difluoro-3,4-dihydroisoquinolin-1(2H)-one,
   N-[2-(N-ethoxycarbonyl-N-trifluoromethanesulfonyl)amino-5-chloro-benzyl]-5,8-difluoro-3,4-dihydroisoquinolin-1(2H)-one,
   N-[2-(N-ethoxycarbonyl-N-trifluoromethanesulfonyl)amino-5-methyl-benzyl]-5,8-difluoro-3,4-dihydroisoquinolin-1(2H)-one,
   N-[2-(N-ethoxycarbonyl-N-trifluoromethanesulfonyl)amino-5-trifluoromethylbenzyl]-5,8-difluoro-3,4-dihydroisoquinolin-1(2H)-one,
   N-[2-(N-ethoxycarbonyl-N-trifluoromethanesulfonyl)amino-5-hydroxy-benzyl]-5,8-difluoro-3,4-dihydroisoquinolin-1(2H)-one,
   N-[2-(N-ethoxycarbonyl-N-trifluoromethanesulfonyl)amino-5-methoxy-benzyl]-5,8-difluoro-3,4-dihydroisoquinolin-1(2H)-one,
   N-[2-(N-ethoxycarbonyl-N-trifluoromethanesulfonyl)amino-6-fluoro-benzyl]-5,8-difluoro-3,4-dihydroisoquinolin-1(2H)-one,
   N-[2-(N-ethoxycarbonyl-N-trifluoromethanesulfonyl)amino-6-chloro-benzyl]-5,8-difluoro-3,4-dihydroisoquinolin-1(2H)-one,
   N-[2-(N-ethoxycarbonyl-N-trifluoromethanesulfonyl)amino-6-methyl-benzyl]-5,8-difluoro-3,4-dihydroisoquinolin-1(2H)-one,
   N-[2-(N-ethoxycarbonyl-N-trifluoromethanesulfonyl)amino-naphthalen-3-yl-methyl]-5,8-difluoro-3,4-dihydroisoquinolin-1(2H)-one,
   N-[2-(N-pent-1-yloxycarbonyl-N-trifluoromethanesulfonyl)amino-4-fluoro-benzyl]-5,8-difluoro-3,4-dihydroisoquinolin-1 (2H)-one,
   N-[2-(N-pent-1-yloxycarbonyl-N-trifluoromethanesulfonyl)amino-5-fluoro-benzyl]-5,8-difluoro-3,4-dihydroisoquinolin-1 (2H)-one,
   N-[2-(N-pent-1-yloxycarbonyl-N-trifluoromethanesulfonyl)amino-6-fluoro-benzyl]-5,8-difluoro-3,4-dihydroisoquinolin-1 (2H)-one,
   N-[2-(N-trifluoromethanesulfonyl)aminobenzyl]-2,3,4,5-tetrahydro-1H-2-benzazepin-1-one,
   N-[2-(N-trifluoromethanesulfonyl)aminobenzyl]-9-chloro-2,3,4,5-tetrahydro-1H-2-benzazepin-1-one,
   N-[2-(N-trifluoromethanesulfonyl)aminobenzyl]-9-methyl-2,3,4,5-tetrahydro-1H-2-benzazepin-1-one,
   N-[2-(N-trifluoromethanesulfonyl)aminobenzyl]-7-fluoro-2,3,4,5-tetrahydro-1H-2-benzazepin-1-one,
   N-[2-(N-trifluoromethanesulfonyl)aminobenzyl]-7-chloro-2,3,4,5-tetrahydro-1H-2-benzazepin-1-one,
   N-[2-(N-trifluoromethanesulfonyl)aminobenzyl]-7-methyl-2,3,4,5-tetrahydro-1H-2-benzazepin-1-one,
   N-[2-(N-trifluoromethanesulfonyl)aminobenzyl]-4,5-dihydro-6H-thieno[2,3-c]pyrrol-6-one,
   N-[2-(N-trifluoromethanesulfonyl)aminobenzyl]-2-bromo-4,5-dihydro-6H-thieno[2,3-c]pyrrol-6-one,
   N-[2-(N-trifluoromethanesulfonyl)aminobenzyl]-2-(methylthio)-6,7-dihydro-5H-pyrrolo[3,4-d]pyrimidin-5-one,
   N-[2-(N-trifluoromethanesulfonyl)aminobenzyl]-5,6-dihydrofurano[2,3-c]pyrimidin-7(4H)-one,
   N-[2-(N-trifluoromethanesulfonyl)aminobenzyl]-5,6-dihydrothieno[2,3-c]pyridin-7(4H)-one,
   N-[2-(N-trifluoromethanesulfonyl)aminobenzyl]-6,7-dihydrothieno[3,2-c]pyridin-4(5H)-one,
   N-[2-(N-trifluoromethanesulfonyl)aminobenzyl]-3-methyl-5,6-dihydroisoxazolo[5,4-c]pyridin-7(4H)-one,
   N-[2-(N-trifluoromethanesulfonyl)aminobenzyl]-2-methyl-6,7-dihydrooxazolo[5,4-c]pyridin-4(5H)-one,
   N-[2-(N-trifluoromethanesulfonyl)aminobenzyl]-6,7-dihydrooxazolo[4,5-c]pyridin-4(5H)-one,
   N-[2-(N-trifluoromethanesulfonyl)aminobenzyl]-2-methyl-6,7-dihydrooxazolo[4,5-c]pyridin-4(5H)-one,
   5-[2-(N-trifluoromethanesulfonyl)aminobenzyl]-1,5,6,7-tetrahydro-4H-pyrazolo[4,3-c]pyridin-4-one,
   5-[2-(N-trifluoromethanesulfonyl)aminobenzyl]-1-methyl-1,5,6,7-tetrahydro-4H-pyrazolo[4,3-c]pyridin-4-one,
   5-[2-(N-trifluoromethanesulfonyl)aminobenzyl]-3-bromo-1-methyl-1,5,6,7-tetrahydro-4H-pyrazolo[4,3-c]pyridin-4-one,
   5-[2-(N-trifluoromethanesulfonyl)aminobenzyl]-3-methyl-1-phenyl-1,5,6,7-tetrahydro-4H-pyrazolo[4,3-c]pyridin-4-one,
   5-[2-(N-trifluoromethanesulfonyl)aminobenzyl]-3-methyl-1-(pyridin-2-yl)-1,5,6,7-tetrahydro-4H-pyrazolo[4,3-c]pyridin-4-one,
   5-[2-(N-trifluoromethanesulfonyl)aminobenzyl]-2-methyl-2,5,6,7-tetrahydro-4H-pyrazolo[4,3-c]pyridin-4-one,
   5-[2-(N-trifluoromethanesulfonyl)aminobenzyl]-2,3-dimethyl-2,5,6,7-tetrahydro-4H-pyrazolo[4,3-c]pyridin-4-one,
   5-[2-(N-trifluoromethanesulfonyl)aminobenzyl]-3-bromo-2-methyl-2,5,6,7-tetrahydro-4H-pyrazolo[4,3-c]pyridin-4-one,
   N-[2-(N-trifluoromethanesulfonyl)aminobenzyl]-6,7-dihydro-1,7-naphthyridin-8(5H)-one,
   N-[2-(N-trifluoromethanesulfonyl)aminobenzyl]-3,4-dihydro-2,6-naphthyridin-1(2H)-one,
   N-[2-(N-trifluoromethanesulfonyl)aminobenzyl]-2-methyl-7,8-dihydropyrido[4,3-d]pyrimidin-5(6H)-one,
   N-[2-(N-ethoxycarbonyl-N-trifluoromethanesulfonyl)aminobenzyl]-5,6-dihydrothieno[2,3-c]pyridin-7(4H)-one,
   5-[2-(N-ethoxycarbonyl-N-trifluoromethanesulfonyl)aminobenzyl]-3-bromo-1-methyl-1,5,6,7-tetrahydro-4H-pyrazolo[4,3-c]pyridin-4-one,
   5-[2-(N-ethoxycarbonyl-N-trifluoromethanesulfonyl)aminobenzyl]-2,3-dimethyl-2,5,6,7-tetrahydro-4H-pyrazolo[4,3-c]pyridin-4-one,
   N-[2-(N-ethoxycarbonyl-N-trifluoromethanesulfonyl)aminobenzyl]-6,7-dihydro-1,7-naphthyridin-8(5H)-one,
   N-[2-(N-ethoxycarbonyl-N-trifluoromethanesulfonyl)aminobenzyl]-3,4-dihydro-2,6-naphthyridin-1(2H)-one,
   N-[2-(N-ethoxycarbonyl-N-trifluoromethanesulfonyl)aminobenzyl]-7,8-dihydro-1,6-naphthyridin-5(6H)-one,
   N-[2-(N-trifluoromethanesulfonyl)aminobenzyl]-3,4-dihydroisoquinolin-1(2H)-thione,
   N-[2-(N-trifluoromethanesulfonyl)aminobenzyl]-5,8-difluoro-3,4-dihydroisoquinoline-1(2H)-thione,
   N-[2-(N-trifluoromethanesulfonyl)aminobenzyl]-3-methyl-3,4-dihydroisoquinolin-1(2H)-one,
   N-[2-(N-trifluoromethanesulfonyl)aminobenzyl]-3,3-dimethyl-3,4-dihydroisoquinolin-1(2H)-one,
   N-[2-(N-trifluoromethanesulfonyl)aminobenzyl]-2,2-dimethyl-2,3-dihydro-4H-benzo[e][1,3]oxazin-4-one,
   N-[2-(N-trifluoromethanesulfonyl)aminobenzyl]-1H-benzo[d][1,2]oxazin-4(3H)-one,
   N-[2-(N-trifluoromethanesulfonyl)aminobenzyl]-2,3-dihydro-4H-benzo[e][1,3]thiazin-4-one,
   N-[2-(N-trifluoromethanesulfonyl)aminobenzyl]-4-methyl-3,4-dihydroisoquinolin-1(2H)-one,
   N-[2-(N-trifluoromethanesulfonyl)aminobenzyl]-4-methyl-3,4-dihydroisoquinolin-1(2H)-one,
   N-[2-(N-trifluoromethanesulfonyl)aminobenzyl]-4,4-dimethyl-3,4-dihydroisoquinolin-1(2H)-one,
   N-[2-(N-trifluoromethanesulfonyl)aminobenzyl]-4-methoxyisoquinolin-1(2H)-one,
   N-[2-(N-ethoxycarbonyl-N-trifluoromethylsulfonyl)aminobenzyl]-5,8-difluoro-3,4-dihydroisoquinolin-1(2H)-thione,
   N-[2-(N-ethoxycarbonyl-N-trifluoromethylsulfonyl)aminobenzyl]-3-methyl-3,4-dihydroisoquinolin-1(2H)-one,
   N-[2-(N-ethoxycarbonyl-N-trifluoromethylsulfonyl)aminobenzyl]-1H-benzo[d][1,2]oxazin-4(3H)-one,
   N-[2-(N-ethoxycarbonyl-N-trifluoromethylsulfonyl)aminobenzyl]-4-methyl-3,4-dihydroisoquinolin-1(2H)-one,
   N-[2-(N-ethoxycarbonyl-N-trifluoromethylsulfonyl)aminobenzyl]-4,4-dimethyl-3,4-dihydroisoquinolin-1(2H)-one,
   N-[2-(N-ethoxycarbonyl-N-trifluoromethylsulfonyl)aminobenzyl]-isoquinolin-1(2H)-one,
   N-[2-(N-trifluoromethylsulfonyl)amino-phenylethan-1-yl]-5,8-difluoro-3,4-dihydroisoquinolin-1(2H)-one,
   N-[2-(N-difluoromethanesulfonyl)aminobenzyl]-5,8-difluoro-3,4-dihydroisoquinolin-1(2H)-one,
   N-[2-(N-trifluoromethanesulfonyl)aminobenzyl]-1,3-dihydroindol-2-one,
   N-[2-(N-trifluoromethanesulfonyl)aminobenzyl]-5-fluoro-1,3-dihydroindol-2-one,
   N-[2-(N-trifluoromethanesulfonyl)aminobenzyl]-5-chloro-1,3-dihydroindol-2-one,
   N-[2-(N-trifluoromethanesulfonyl)aminobenzyl]-5-bromo-1,3-dihydroindol-2-one,
   N-[2-(N-trifluoromethanesulfonyl)aminobenzyl]-6-chloro-1,3-dihydroindol-2-one,
   N-[2-(N-trifluoromethanesulfonyl)amino-5-trifluoromethyl-benzyl]-1,3-dihydroindol-2-one,
   N-[2-(N-ethoxycarbonyl-N-trifluoromethanesulfonyl)aminobenzyl]-1,3-dihydroindol-2-one,
   N-[2-(N-ethoxycarbonyl-N-trifluoromethanesulfonyl)aminobenzyl]-5-fluoro-1,3-dihydroindol-2-one,
   N-[2-(N-ethoxycarbonyl-N-trifluoromethanesulfonyl)aminobenzyl]-5-chloro-1,3-dihydroindol-2-one,
   N-[2-(N-ethoxycarbonyl-N-trifluoromethanesulfonyl)aminobenzyl]-5-bromo-1,3-dihydroindol-2-one,
   N-[2-(N-ethoxycarbonyl-N-trifluoromethanesulfonyl)aminobenzyl]-6-chloro-1,3-dihydroindol-2-one,
   N-[2-(N-ethoxycarbonyl-N-trifluoromethanesulfonyl)amino-5-trifluoromethylbenzyl]-1,3-dihydroindol-2-one,
   N-[2-(N-trifluoromethanesulfonyl)aminobenzyl]-1,4-dihydroisoquinolin-3(2H)-one,
   N-[2-(N-trifluoromethanesulfonyl)aminobenzyl]-3,4-dihydroquinolin-2(1H)-one,
   N-[2-(N-trifluoromethanesulfonyl)aminobenzyl]-3-methyl-3,4dihydroquinazolin-2(1H)-one,
   N-[2-(N-trifluoromethanesulfonyl)aminobenzyl]-phenanthridin-6(5H)-one,
   N-[2-(N-ethoxycarbonyl-N-trifluoromethanesulfonyl)aminobenzyl]-1,4-dihydroisoquinolin-3(2H)-one,
   N-[2-(N-ethoxycarbonyl-N-trifluoromethanesulfonyl)aminobenzyl]-3,4-dihydroquinolin-2(1H)-one,
   1-[2-(N-ethoxycarbonyl-N-trifluoromethanesulfonyl)aminobenzyl]-3-methyl-3,4-dihydroquinazolin-2(1H)-one, and
   N-[2-(N-ethoxycarbonyl-N-trifluoromethanesulfonyl)aminobenzyl]-phenanthridin-6(5H)-one.
(8) The compound or salt thereof according to any one of (1) to (7), wherein the compound represented by Formula [1] is a mixture of enantiomers or a mixture of diastereomers.
(9) A composition having herbicidal activity, comprising the compound or salt thereof represented by Formula [1] according to any one of (1) to (8) as an active ingredient.
(10) A method of weed control, comprising treating soil and/or a plant with an effective amount of the composition according to (9).
(11) A method of controlling paddy field weeds, comprising treating paddy field soil with an effective amount of the composition according to (9).

### ADVANTAGEOUS EFFECTS OF INVENTION

The haloalkyl sulfonanilide compound represented by Formula [1] or an agrochemically acceptable salt thereof exhibits high herbicidal activity, is highly safe for useful plants and crops, and exerts excellent efficacy as an agrochemical.

### DESCRIPTION OF EMBODIMENTS

Hereinafter, the present invention will be described in detail.

Examples of the above haloalkyl sulfonanilide compound in the present invention include not only a haloalkyl sulfonanilide compound represented by Formula [1] but also a salt, a hydrate, a solvate, or a crystalline polymorph of the compound, and an N-oxide of the haloalkyl sulfonanilide compound represented by Formula [1]. Here, the salt is not particularly limited, and examples include salts acceptable in the manufacture of agrochemicals. Specific examples include sodium salts, potassium salts, magnesium salts, calcium salts, and aluminum salts. Additional examples of the compound in the present invention (haloalkyl sulfonanilide compound represented by Formula [1]) include any stereoisomers or optical isomers that can be present in the compound of the present invention or a mixture that contains two or more kinds of the isomers at any ratio.

Formula [1] gives the specific definition of the haloalkyl sulfonanilide compound of the present invention. The preferred definitions of the groups involving the formulas given herein are described below. Such definitions apply to the final product represented by Formula [1] and, likewise, to all synthetic intermediates.

The following describes preferred embodiments.

A is an optionally substituted benzene ring or heteroaromatic ring, and preferably an optionally substituted benzene ring.

E is preferably -C(R⁵)(R⁶)-, an oxygen atom, or a sulfur atom, and more preferably - CH₂-.

o is preferably from 0 to 3, and more preferably from 0 to 2.

p is preferably from 0 to 3, and more preferably 0.

m is preferably 0 or **1,** and more preferably 0.

n is preferably 1 to 3, and more preferably 2.

R¹ is preferably fluoro C₁-C₆ alkyl, and more preferably trifluoromethyl.

R² is preferably a hydrogen atom, (C₃-C₆ cycloalkyl)carbonyl, (C₂-C₄ alkenyl)carbonyl, (C₁-C₅ alkoxy)carbonyl, (halo C₁-C₃ alkoxy)carbonyl, (C₃-C₄ cycloalkyloxy)carbonyl, heterocyclyloxycarbonyl, C₃-C₄ cycloalkyl(C₁-C₂ alkoxy)carbonyl, heterocyclyl(C₁-C₂ alkoxy)carbonyl, C₁-C₂ alkylsulfonyl, or (C₁-C₄ alkylthio)carbonyl, and more preferably a hydrogen atom, (C₃-C₆ cycloalkyl)carbonyl, (C₁-C₅ alkoxy)carbonyl, (halo C₁-C₃ alkoxy)carbonyl, heterocyclyloxycarbonyl, heterocyclyl(C₁-C₂ alkoxy)carbonyl, or methanesulfonyl.

R³ and R⁴ are, each independently, preferably a hydrogen atom, C₁-C₄ alkyl, or halo C₁-C₄ alkyl, and more preferably a hydrogen atom.

R⁵ and R⁶ are, each independently, preferably a hydrogen atom, C₁-C₄ alkyl, or halo C₁-C₄ alkyl, and more preferably a hydrogen atom.

R⁷ is preferably C₁-C₄ alkyl or phenyl, and more preferably methyl.

Examples of C₁-C₆ alkyl optionally substituted by one or more substituents selected from Z¹ include C₁-C₆ alkyl, halo C₁-C₆ alkyl, C₃-C₆ cycloalkyl C₁-C₆ alkyl, phenyl C₁-C₆ alkyl, heterocyclyl C₁-C₆ alkyl, heterocyclyl C₁-C₆ alkyl, cyano C₁-C₆ alkyl, C₁-C₆ alkoxy C₁-C₆ alkyl, halo C₁-C₆ alkoxy C₁-C₆ alkyl, C₃-C₆ cycloalkyloxy C₁-C₆ alkyl, (phenoxy)C₁-C₆ alkyl, (phenyl)C₁-C₆ alkoxy C₁-C₆ alkyl, tri-C₁-C₆ alkylsilyl C₁-C₆ alkoxy C₁-C₆ alkyl, C₁-C₆ alkyl diphenylsilyl C₁-C₆ alkoxy C₁-C₆ alkyl, phenylcarbonyloxy C₁-C₆ alkyl, heterocyclyl carbonyloxy C₁-C₆ alkyl, C₁-C₆ alkoxy C₁-C₆ alkoxy C₁-C₆ alkyl, halo C₁-C₆ alkoxy C₁-C₆ alkoxy C₁-C₆ alkyl, phenylcarbonyloxy C₁-C₆ alkoxy C₁-C₆ alkyl, (C₁-C₆ alkyl)carbonyloxy C₁-C₆ alkyl, (halo C₁-C₆ alkyl)carbonyloxy C₁-C₆ alkyl, (C₃-C₆ cycloalkyl)carbonyloxy C₁-C₆ alkyl, (C₁-C₆ alkoxy)carbonyloxy C₁-C₆ alkyl, phenoxycarbonyloxy C₁-C₆ alkyl, mono(C₁-C₆ alkyl)aminocarbonyloxy C₁-C₆ alkyl, di(C₁-C₆ alkyl)aminocarbonyloxyC₁-C₆ alkyl, phenylaminocarbonyloxy C₁-C₆ alkyl, C₁-C₆ alkyl(phenyl)aminocarbonyloxy C₁-C₆ alkyl, C₁-C₆ alkylthio C₁-C₆ alkyl, halo C₁-C₆ alkylthio C₁-C₆ alkyl, C₁-C₆ alkyl sulfinyl C₁-C₆ alkyl, halo C₁-C₆ alkylsulfinyl C₁-C₆ alkyl, C₁-C₆ alkylsulfonyl C₁-C₆ alkyl, halo C₁-C₆ alkylsulfonyl C₁-C₆ alkyl, phenylthio C₁-C₆ alkyl, phenylsulfinyl C₁-C₆ alkyl, phenylsulfonyl C₁-C₆ alkyl, phenyl C₁-C₆ alkylthio C₁-C₆ alkyl, phenyl C₁-C₆ alkylsulfinyl C₁-C₆ alkyl, phenyl C₁-C₆ alkylsulfonyl C₁-C₆ alkyl, thiocyanato C₁-C₆ alkyl, (C₁-C₆ alkyl)carbonyl C₁-C₆ alkyl, (halo C₁-C₆ alkyl)carbonyl C₁-C₆ alkyl, (C₁-C₆ alkoxy)carbonyl C₁-C₆ alkyl, (halo C₁-C₆ alkoxy)carbonyl C₁-C₆ alkyl, and phenylcarbonyl C₁-C₆ alkyl. Here, the phenyl and/or heterocyclyl moieties in the above groups are optionally substituted by one or more substituents independently selected from Z².

Z¹ is preferably C₃-C₆ cycloalkyl, phenyl optionally substituted by one or more substituents selected from Z², heterocyclyl optionally substituted by one or more substituents selected from Z², cyano, C₁-C₆ alkoxy optionally substituted by one or more substituents selected from Z³, C₃-C₆ cycloalkyloxy, phenoxy optionally substituted by one or more substituents selected from Z², C₁-C₆ alkylsulfonyl optionally substituted by one or more halogen atoms, (C₁-C₆ alkyl)carbonyl optionally substituted by one or more halogen atoms, or (C₁-C₆ alkoxy)carbonyl optionally substituted by one or more halogen atoms, and more preferably cyano, C₁-C₆ alkoxy optionally substituted by one or more substituents selected from Z³, C₁-C₆ alkylsulfonyl optionally substituted by one or more halogen atoms, or (C₁-C₆ alkyl)carbonyl optionally substituted by one or more halogen atoms.

Z² is preferably halogen, C₁-C₆ alkyl, halo C₁-C₆ alkyl C₁-C₆ alkoxy, halo C₁-C₆ alkoxy, (C₁-C₆ alkyl)carbonyl, (C₁-C₆ alkyl)carbonyl, (halo C₁-C₆ alkyl)carbonylcyano, or nitro, and more preferably halogen, C₁-C₆ alkyl, cyano, or nitro.

Z³ is preferably a halogen atom, tri-C₁-C₆ alkylsilyl, C₁-C₆ alkyl diphenylsilyl, or C₁-C₆ alkoxy optionally substituted by one or more halogen atoms, and more preferably a halogen atom, triC₁-C₆ alkylsilyl, or C₁-C₆ alkoxy optionally substituted by one or more halogen atoms.

Z⁴ is preferably phenyl optionally substituted by one or more substituents selected from Z².

Z⁶ is preferably di(C₁-C₆ alkyl)aminocarbonyl, hydroxy, amino, cyano, or nitro, and more preferably cyano or nitro.

Z⁷ is preferably halogen, C₁-C₆ alkyl, C₃-C₆ cycloalkyl, halo C₁-C₆ alkyl, C₁-C₆ alkoxy, halo C₁-C₆ alkoxy, C₁-C₆ alkylsulfonyl, halo C₁-C₆ alkylsulfonyl, (C₁-C₆ alkyl)carbonyl, (C₁-C₆ alkoxy)carbonyl, cyano, or nitro, and more preferably halogen, C₁-C₆ alkyl, C₁-C₆ alkoxy, cyano, or nitro.

Z⁸ is preferably phenylthio optionally substituted by one or more substituents selected from Z², or phenylsulfonyl optionally substituted by one or more substituents selected from Z², and more preferably phenylsulfonyl optionally substituted by one or more substituents selected from Z².

X is preferably, each independently, a hydrogen atom, halogen, methyl, methoxy, propargyloxy, trifluoromethyl, or trifluoromethoxy, and more preferably, each independently, a hydrogen atom, halogen, or methyl.

X¹ is preferably a hydrogen atom, halogen, methyl, trifluoromethyl, or methoxy, and more preferably a fluorine or chlorine atom.

X² and X³ are preferably, each independently, a hydrogen atom, halogen, methyl, trifluoromethyl, or methoxy, and more preferably a hydrogen atom.

X⁴ is preferably a hydrogen atom, halogen, methyl, trifluoromethyl, or methoxy, and more preferably a hydrogen atom, a fluorine atom, or a chloro atom.

Y is preferably, each independently, a hydrogen atom, halogen, methyl, or trifluoromethyl, and more preferably a hydrogen atom.

Y¹, Y², Y³, and Y⁴ are preferably, each independently, a hydrogen atom, halogen, methyl, or trifluoromethyl, and more preferably a hydrogen atom.

W is an oxygen or sulfur atom, and preferably an oxygen atom.

The heterocycle is preferably thienyl, furyl, oxiranyl, oxetanyl, tetrahydrofuranyl, or tetrahydropyranyl, and more preferably oxiranyl, oxetanyl, or tetrahydrofuranyl.

The heteroaromatic ring is preferably selected from the group consisting of Het-1 to Het-18 above, and more preferably Het-1 or Het-2.

In the general or preferred range, the definitions and descriptions of the groups given above may be combined with each other as necessary. That is, it is possible to combine between the respective ranges and the preferred range. They apply to both the final product and the corresponding precursors and synthetic intermediates.

Preferred is a compound represented by Formula [1] wherein R¹ represents trifluoromethyl.

More preferred is a compound represented by Formula [1] wherein R² represents cyclopropylcarbonyl.

Still more preferred is a compound represented by Formula [1] wherein R² represents ethoxycarbonyl.

Still more preferred is a compound represented by Formula [1] wherein R² represents 2-fluoroethoxycarbonyl.

Still more preferred is a compound represented by Formula [1] wherein R² represents 2,2-difluoroethoxycarbonyl.

Still more preferred is a compound represented by Formula [1] wherein R² represents (oxetan-3-il)oxycarbonyl.

Still more preferred is a compound represented by Formula [1] wherein R² represents (tetrahydrofuran-3-il)oxycarbonyl.

Still more preferred is a compound represented by Formula [1] wherein R² represents (oxylan-2-ylmethyl)oxycarbonyl.

Still more preferred is a compound represented by Formula [1] wherein R² represents (oxetan-2-ylmethyl)oxycarbonyl.

Still more preferred is a compound represented by Formula [1] wherein R² represents (oxetan-3-ylmethyl)oxycarbonyl.

Still more preferred is a compound represented by Formula [1] wherein R² represents methanesulfonyl.

Still more preferred is a compound represented by Formula [1] wherein W represents an oxygen atom, E represents -CH₂-, R³ and R⁴ each represent a hydrogen atom, m represents 0, n represents 2, X¹ represents a fluorine atom, and X², X³, and X⁴ each represent a hydrogen atom.

Still more preferred is a compound represented by Formula [1] wherein W represents an oxygen atom, E represents -CH₂-, R³ and R⁴ each represent a hydrogen atom, m represents 0, n represents 2, X¹ represents a chloro atom, and X², X³, and X⁴ each represent a hydrogen atom.

Still more preferred is a compound represented by Formula [1] wherein W represents an oxygen atom, E represents -CH₂-, R³ and R⁴ each represent a hydrogen atom, m represents 0, n represents 2, X¹ and X⁴ each represent a fluorine atom, and X² and X³ each represent a hydrogen atom.

Still more preferred is a compound represented by Formula [1] wherein W represents an oxygen atom, E represents -CH₂-, R³ and R⁴ each represent a hydrogen atom, m represents 0, n represents 2, X¹ and X⁴ each represent a fluorine atom, and X² and X³ each represent a hydrogen atom.

Still more preferred is a compound represented by Formula [1] wherein W represents an oxygen atom, E represents -CH₂-, R³ and R⁴ each represent a hydrogen atom, m represents 0, n represents 2, X¹ represents a fluorine atom, X² and X³ each represent a hydrogen atom, and X⁴ represents a chloro atom.

Still more preferred is a compound represented by Formula [1] wherein W represents an oxygen atom, E represents -CH₂-, R³ and R⁴ each represent a hydrogen atom, m represents 0, n represents 2, X¹ represents a fluorine atom, X² and X³ each represent a hydrogen atom, and X⁴ represents methyl.

Still more preferred is a compound represented by Formula [1] wherein W represents an oxygen atom, E represents -CH₂-, R³ and R⁴ each represent a hydrogen atom, m represents 0, n represents 2, X¹ represents a fluorine atom, X² and X³ each represent a hydrogen atom, and X⁴ represents trifluoromethyl.

Still more preferred is a compound represented by Formula [1] wherein W represents an oxygen atom, E represents -CH₂-, R³ and R⁴ each represent a hydrogen atom, m represents 0, n represents 2, X¹ represents a fluorine atom, X² and X³ each represent a hydrogen atom, and X⁴ represents methoxy.

Still more preferred is a compound represented by Formula [1] wherein W represents a sulfur atom, E represents -CH₂-, R³ and R⁴ each represent a hydrogen atom, m represents 0, n represents 2, X¹ represents a fluorine atom, and X², X³, and X⁴ each represent a hydrogen atom.

Still more preferred is a compound represented by Formula [1] wherein W represents a sulfur atom, E represents -CH₂-, R³ and R⁴ each represent a hydrogen atom, m represents 0, n represents 2, X¹ represents a chloro atom, and X², X³, and X⁴ each represent a hydrogen atom.

Still more preferred is a compound represented by Formula [1] wherein W represents a sulfur atom, E represents -CH₂-, R³ and R⁴ each represent a hydrogen atom, m represents 0, n represents 2, X¹ and X⁴ each represent a fluorine atom, and X² and X³ each represent a hydrogen atom.

Still more preferred is a compound represented by Formula [1] wherein W represents a sulfur atom, E represents -CH₂-, R³ and R⁴ each represent a hydrogen atom, m represents 0, n represents 2, X¹ and X⁴ each represent a fluorine atom, and X² and X³ each represent a hydrogen atom.

The following symbols herein have the following meanings:
i: iso, s: secondary, t: tertiary, c: cyclo, and p: para.

The following is an explanation of terms used herein.

Notations with element symbols and subscript numbers, such as C₁-C₆, indicate that the number of elements in the group as followed by the symbol is in the range indicated by the subscript number. For example, this case indicates that the number of carbon atoms is from 1 to 6, while the notation C₂-C₆ indicates that the number of carbon atoms is from 2 to 6.

Examples of the halogen include a fluorine, chlorine, bromine, and iodine atom. Note that the "halo" as used herein also denotes these halogens.

Examples of the alkyl include methyl, ethyl, propyl, i-propyl, butyl, i-butyl, t-butyl, s-butyl, pentyl, 1-methylbutyl, 2-methylbutyl, 3-methylbutyl, 1-ethylpropyl, 1,1-dimethylpropyl, 1,2-dimethylpropyl, neopentyl, hexyl, 1-methylpentyl, 2-methylpentyl, 3-methylpentyl, 4-methylpentyl, 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1,1,2-trimethylpropyl, 1-ethyl-1-methylpropyl, and 1-ethyl-2-methylpropyl. Each is selected from a specified range of the number of carbon atoms.

The haloalkyl means an alkyl group substituted with one or more halogen atoms. Examples of the haloalkyl include fluoromethyl, chloromethyl, bromomethyl, 2-fluoroethyl, 2-chloroethyl, 2-bromoethyl, 3-fluoropropyl, 3-chloropropyl, difluoromethyl, chlorodifluoromethyl, trifluoromethyl, dichloromethyl, trichloromethyl, 2,2-difluoroethyl, 2,2,2-trifluoroethyl, 2,2,2-trichloroethyl, chlorodifluoromethyl, bromodifluoromethyl, pentafluoroethyl, heptafluoropropyl, heptafluoroisopropyl, 4-chlorobutyl, and 4-fluorobutyl. Each is selected from a specified range of the number of carbon atoms.

The fluoroalkyl means an alkyl group substituted with one or more fluorine atoms. Examples of the fluoroalkyl include fluoromethyl, 2-fluoroethyl, 3-fluoropropyl, difluoromethyl, trifluoromethyl, 2,2-difluoroethyl, 2,2,2-trifluoroethyl, pentafluoroethyl, heptafluoropropyl, heptafluoroisopropyl, and 4-fluorobutyl. Each is selected from a specified range of the number of carbon atoms.

Examples of the alkenyl include ethenyl, 1-propenyl, 2-propenyl, 1-butenyl, 2-butenyl, 3-butenyl, 1-methyl-2-propenyl, 2-methyl-2-propenyl, 1-pentenyl, 2-pentenyl, 3-pentenyl, 4-pentenyl, 1,1-dimethyl-2-propenyl, 1,2-dimethyl-2-propenyl, 1-hexenyl, 1,1-dimethyl-2-butenyl, 1,2-dimethyl-2-butenyl, 1,3-dimethyl-2-butenyl, 2,3-dimethyl-2-butenyl, 1-ethyl-2-butenyl, 2-ethyl-2-butenyl, 1,1,2-trimethyl-2-propenyl, and 1-ethyl-1-methyl-2-propenyl. Each is selected from a specified range of the number of carbon atoms.

Examples of the haloalkenyl include 1-chloroethenyl, 2-chloroethenyl, 2-fluoroethenyl, 2,2-dichloroethenyl, 3-chloro-2-propenyl, 3-fluoro-2-propenyl, or 2-chloro-2-propenyl, and 4-chloro-3-butenyl. Each is selected from a specified range of the number of carbon atoms.

Examples of the alkynyl include ethynyl, 1-propynyl, 2-propynyl, 1-butynyl, 2-butynyl, 3-butynyl, 1-methyl-2-propynyl, 1-pentynyl, 1-methyl-2-butynyl, 2-methyl-3-butynyl, 1-hexynyl, 1-methyl-3-pentynyl, 2-methyl-3-pentynyl, 3-methyl-4-pentynyl, 4-methyl-2-pentynyl, 1,1-dimethyl-2-butynyl, 1,2-dimethyl-3-butynyl, 2,2-dimethyl-3-butynyl, 1-ethyl-2-butynyl, and 2-ethyl-3-butynyl. Each is selected from a specified range of the number of carbon atoms.

Examples of the haloalkynyl include chloroethynyl, fluoroethynyl, bromoethynyl, 3-chloro-2-propynyl, and 4-chloro-2-butynyl. Each is selected from a specified range of the number of carbon atoms.

Examples of the cycloalkyl include cyclopropyl, cyclobutyl, cyclopentyl, and cyclohexyl. Each is selected from a specified range of the number of carbon atoms.

Examples of the halocycloalkyl include 2-fluorocyclopropyl, 1-chlorocyclopropyl, 2-bromo-1-methylcyclopropyl, 2,2-difluorocyclopropyl, and 1,2-dichlorocyclopropyl. Each is selected from a specified range of the number of carbon atoms.

Examples of the cycloalkylalkyl include cyclopropylmethyl, 2-cyclopropylethyl, cyclopentylmethyl, and cyclohexylmethyl. Each is selected from a specified range of the number of carbon atoms.

Examples of the phenylalkyl and phenylalkyl substituted by one or more substituents selected from Z² include benzyl, 2-fluorobenzyl, 3-fluorobenzyl, 4-fluorobenzyl, 2-chlorobenzyl, 3-bromobenzyl, 4-iodobenzyl, 2,4-difluorobenzyl, 2-methylbenzyl, 3-methylbenzyl, 4-methylbenzyl, 2-methoxybenzyl, 4-methoxybenzyl, 4-aminobenzyl, 4-trifluoromethoxybenzyl, 2-methylthiobenzyl, 3-trifluoromethylthiobenzyl, 2-cyanobenzyl, 4-nitrobenzyl, 2-trifluoromethylbenzyl, and 2-phenethyl. Each is selected from a specified range of the number of carbon atoms.

Examples of the heterocyclyl alkyl and heterocyclyl C₁-C₆ alkyl substituted by one or more substituents selected from Z² include (oxiran-2-yl)methyl, (2-methyloxiran-2-yl)methyl, (3,3-dimethyloxiran-2-yl)methyl, (oxetan-2-yl)methyl, (oxetan-3-yl)methyl, (tetrahydrofuran-2-yl)methyl, (tetrahydrofuran-3-yl)methyl, (tetrahydrofuran-4-yl)methyl, (tetrahydrothiophen-2-yl)methyl, (1,1-dioxytetrahydrothiophen-2-yl)methyl, (tetrahydropyran-4-yl)methyl, (1,3-dioxan-2-yl)methyl, 2-(1,3-dioxan-2-yl)ethyl, (thiophene-2-yl)methyl, (thiophen-3-yl)methyl, (pyridin-2-yl)methyl, (pyridin-3-yl)methyl, (pyridin-4-yl)methyl, 1-(pyridin-4-yl)ethyl, and 2-(pyridin-4-yl)ethyl. Each is selected from a specified range of the number of carbon atoms.

Examples of the cyanoalkyl include cyanomethyl, 1-cyanoethyl, and 2-cyanoethyl. Each is selected from a specified range of the number of carbon atoms.

Examples of the alkoxyalkyl include methoxymethyl, ethoxymethyl, propyloxymethyl, i-propyloxymethyl, butyloxymethyl, i-butyloxymethyl, s-butyloxymethyl, t-butyloxymethyl, pentyloxymethyl, 1-methoxyethyl, 2-methoxyethyl, 2-ethoxyethyl, and 3-methoxypropyl. Each is selected from a specified range of the number of carbon atoms.

Examples of the haloalkoxyalkyl include fluoromethoxymethyl, chloromethoxymethyl, bromomethoxymethyl, 2,2,2-trifluoroethoxymethyl, and 2-(chloromethoxy)ethyl. Each is selected from a specified range of the number of carbon atoms.

Examples of the cycloalkyloxyalkyl include cyclopropyloxymethyl, 2-cyclopropyloxyethyl, cyclopentyloxymethyl, and cyclohexyloxymethyl. Each is selected from a specified range of the number of carbon atoms.

Examples of the phenoxyalkyl and phenoxyalkyl substituted by one or more substituents selected from Z² include phenoxymethyl, 2-fluorophenoxymethyl, 2-chlorophenoxymethyl, 3-bromophenoxymethyl, 4-iodophenoxymethyl, 4-methylphenoxymethyl, 2-methoxyphenoxymethyl, 4-aminophenoxymethyl, 4-trifluoromethoxyphenoxymethyl, 2-methylthiophenoxymethyl, 3-trifluoromethylthiophenoxymethyl, 2-cyanophenoxymethyl, 4-nitrophenoxymethyl, 2-trifluoromethylphenoxymethyl, and 2-(phenoxy)ethyl. Each is selected from a specified range of the number of carbon atoms.

Examples of the phenylalkoxyalkyl and phenylalkoxyalkyl substituted by one or more substituents selected from Z² include benzyloxymethyl, 2-fluorobenzyloxymethyl, 2-chlorobenzyloxymethyl, 3-bromobenzyloxymethyl, 4-iodobenzyloxymethyl, 4-methylbenzyloxymethyl, 2-methoxybenzyloxymethyl, 4-aminobenzyloxymethyl, 4-trifluoromethoxybenzyloxymethyl, 2-methylthiobenzyloxymethyl, 3-trifluoromethylthiobenzyloxymethyl, 2-cyanobenzyloxymethyl, 4-nitrobenzyloxymethyl, 2-trifluoromethylbenzyloxymethyl, 2-phenethyloxymethyl, 1-(benzyloxy)ethyl, and 2-(benzyloxy)ethyl. Each is selected from a specified range of the number of carbon atoms.

Examples of the trialkylsilylalkoxyalkyl include trimethylsilylmethoxymethyl, 2-(trimethylsilyl)ethoxymethyl, 2-(trimethylsilylmethoxy)ethyl, and 2-[2-(trimethylsilyl)ethoxy]ethyl. Each is selected from a specified range of the number of carbon atoms.

Examples of the phenylcarbonyloxyalkyl and phenylcarbonyloxyalkyl substituted by one or more substituents selected from Z² include phenylcarbonyloxymethyl, 2-fluorophenylcarbonyloxymethyl, 2-chlorophenylcarbonyloxymethyl, 3-bromophenylcarbonyloxymethyl, 4-iodophenylcarbonyloxymethyl, 4-methylphenylcarbonyloxymethyl, 2-methoxyphenylcarbonyloxymethyl, 4-aminophenylcarbonyloxymethyl, 4-trifluoromethoxyphenylcarbonyloxymethyl, 2-methylthiophenylcarbonyloxymethyl, 3-trifluoromethylthiophenylcarbonyloxymethyl, 2-cyanophenylcarbonyloxymethyl, 4-nitrophenylcarbonyloxymethyl, 2-trifluoromethylphenylcarbonyloxymethyl, and 2-(phenylcarbonyloxy)ethyl. Each is selected from a specified range of the number of carbon atoms.

Examples of the heterocyclyl carbonyloxyalkyl and heterocyclyl carbonyloxyalkyl substituted by one or more substituents selected from Z² include (tetrahydrofuran-2-yl)carbonyloxymethyl, (tetrahydrofuran-3-yl)carbonyloxymethyl, (tetrahydrothiophen-2-yl)carbonyloxymethyl, (tetrahydropyran-4-yl)carbonyloxymethyl, (furan-2-yl)carbonyloxymethyl, (3-methylfuran-2-yl)carbonyloxymethyl, (furan-3-yl)carbonyloxymethyl, (thiophen-2-yl)carbonyloxymethyl, (thiophen-3-yl)carbonyloxymethyl, (pyridin-2-yl)carbonyloxymethyl, (pyridin-3-yl)carbonyloxymethyl, and (pyridin-4-yl)carbonyloxymethyl. Each is selected from a specified range of the number of carbon atoms.

Examples of the alkoxyalkoxyalkyl include methoxymethoxymethyl, ethoxymethoxymethyl, propyloxymethoxymethyl, i-propoxymethoxymethyl, butyloxymethoxymethyl, 1-(methoxymethoxy)ethyl, 2-(methoxymethoxy)ethyl, 2-(ethoxymethoxy)ethyl, and 2-(ethoxy)ethoxymethyl. Each is selected from a specified range of the number of carbon atoms.

Examples of the haloalkoxyalkoxyalkyl include fluoromethoxymethoxymethyl, chloromethoxymethoxymethyl, bromomethoxymethoxymethyl, 2,2,2-trifluoroethoxymethoxymethyl, and 2-(chloromethoxymethoxy)ethyl. Each is selected from a specified range of the number of carbon atoms.

Examples of the phenylcarbonyloxyalkoxyalkyl and phenylcarbonyloxyalkoxyalkyl substituted by one or more substituents selected from Z² include phenylcarbonyloxymethoxymethyl, 2-fluorophenylcarbonyloxymethoxymethyl, 2-chlorophenylcarbonyloxymethoxymethyl, 3-bromophenylcarbonyloxymethoxymethyl, 4-iodophenylcarbonyloxymethoxymethyl, 4-methylphenylcarbonyloxymethoxymethyl, 2-methoxyphenylcarbonyloxymethoxymethyl, 4-aminophenylcarbonyloxymethoxymethyl, 4-trifluoromethoxyphenylcarbonyloxymethoxymethyl, 2-methylthiophenylcarbonyloxymethoxymethyl, 3-trifluoromethylthiophenylcarbonyloxymethoxymethyl, 2-cyanophenylcarbonyloxymethoxymethyl, 4-nitrophenylcarbonyloxymethoxymethyl, 2-trifluoromethylphenylcarbonyloxymethoxymethyl, 2-(phenylcarbonyloxy)ethoxymethyl, and 2-(phenylcarbonyloxymethoxy)ethyl.

Examples of the alkylcarbonyloxyalkyl include acetoxymethyl, propionyloxymethyl, butyryloxymethyl, i-butyryloxymethyl, valeroyloxymethyl, i-valeroyloxymethyl, 2-methylbutyryloxymethyl, pivaloyloxymethyl, heptanoyloxymethyl, 1-(acetoxy)ethyl, 2-(acetoxy)ethyl, 2-(propionyloxy)ethyl, and 3-(acetoxy)propyl. Each is selected from a specified range of the number of carbon atoms.

Examples of the haloalkylcarbonyloxyalkyl include fluoromethylcarbonyloxymethyl, chloromethylcarbonyloxymethyl, bromomethylcarbonyloxymethyl, 2,2,2-trifluoroethylcarbonyloxymethyl, and 2-(chloromethylcarbonyloxy)ethyl. Each is selected from a specified range of the number of carbon atoms.

Examples of the cycloalkylcarbonyloxyalkyl include cyclopropanecarbonyloxymethyl, cyclobutanecarbonyloxymethyl, cyclopentanecarbonyloxymethyl, and 2-(cyclohexanecarbonyloxy)ethyl. Each is selected from a specified range of the number of carbon atoms.

Examples of the alkoxycarbonyloxyalkyl include methoxycarbonyloxymethyl, ethoxycarbonyloxymethyl, propyloxycarbonyloxymethyl, i-propyloxycarbonyloxymethyl, butyloxycarbonyloxymethyl, 2-methoxycarbonyloxyethyl, 2-(ethoxycarbonyloxy)ethyl, and 3-(methoxycarbonyloxy)propyl. Each is selected from a specified range of the number of carbon atoms.

Examples of the phenoxycarbonyloxyalkyl and phenoxycarbonyloxyalkyl substituted by one or more substituents selected from Z² include phenoxycarbonyloxymethyl, 1-(phenoxycarbonyloxy)ethyl, 2-(phenoxycarbonyloxy)ethyl, 2-fluorophenoxycarbonyloxymethyl, 2-chlorophenoxycarbonyloxymethyl, 4-bromophenoxycarbonyloxymethyl, 4-methylphenoxycarbonyloxymethyl, 2-methoxyphenoxycarbonyloxymethyl, 3-aminophenoxycarbonyloxymethyl, 4-trifluoromethoxyphenoxycarbonyloxymethyl, 2-methylthiophenoxycarbonyloxymethyl, 3-trifluoromethylthiophenoxycarbonyloxymethyl, 4-cyanophenoxycarbonyloxymethyl, 2-nitrophenoxycarbonyloxymethyl, and 2-(4-trifluoromethylphenoxycarbonyloxy)ethyl. Each is selected from a specified range of the number of carbon atoms.

Examples of the monoalkylaminocarbonyloxyalkyl include methylaminocarbonyloxymethyl, ethylaminocarbonyloxymethyl, propylaminocarbonyloxymethyl, i-propylaminocarbonyloxymethyl, butylaminocarbonyloxymethyl, 2-(methylaminocarbonyloxy)ethyl, 2-(ethylaminocarbonyloxy)ethyl, and 3-(methylaminocarbonyloxyl)propyl. Each is selected from a specified range of the number of carbon atoms.

Examples of the dialkylaminocarbonyloxyalkyl include dimethylaminocarbonyloxymethyl, diethylaminocarbonyloxymethyl, ethyl(methyl)aminocarbonyloxymethyl, methyl(propyl)aminocarbonyloxymethyl, butyl(methyl)aminocarbonyloxymethyl, 2-(diethylaminocarbonyloxy)ethyl, and 2-{ethyl(methyl)aminocarbonyloxy}propyl. Each is selected from a specified range of the number of carbon atoms.

Examples of the phenylaminocarbonyloxyalkyl and phenylaminocarbonyloxyalkyl substituted by one or more substituents selected from Z² include phenylaminocarbonyloxymethyl, 2-fluorophenylaminocarbonyloxymethyl, 2-chlorophenylaminocarbonyloxymethyl, 3-bromophenylaminocarbonyloxymethyl, 4-iodophenylaminocarbonyloxymethyl, 4-methylphenylaminocarbonyloxymethyl, 2-methoxyphenylaminocarbonyloxymethyl, 4-aminophenylaminocarbonyloxymethyl, 4-trifluoromethoxyphenylaminocarbonyloxymethyl, 2-methylthiophenylaminocarbonyloxymethyl, 3-trifluoromethylphenylaminocarbonyloxymethyl, 2-cyanophenylaminocarbonyloxymethyl, 4-nitrophenylaminocarbonyloxymethyl, 2-trifluoromethylphenylaminocarbonyloxymethyl, and 2-(phenylaminocarbonyloxy)ethyl. Each is selected from a specified range of the number of carbon atoms.

Examples of the alkyl(phenyl)aminocarbonyloxyalkyl and alkyl(phenyl substituted by one or more substituents selected from Z²)aminocarbonyloxyalkylalkyl include methyl(phenyl)aminocarbonyloxymethyl, methyl(2-fluorophenyl)aminocarbonyloxymethyl, methyl(2-chlorophenyl)aminocarbonyloxymethyl, methyl(3-bromophenyl)aminocarbonyloxymethyl, methyl(4-iodophenyl)aminocarbonyloxymethyl, methyl(4-methylphenyl)aminocarbonyloxymethyl, methyl(2-methoxyphenyl)aminocarbonyloxymethyl, methyl(4-aminophenyl)aminocarbonyloxymethyl, methyl(4-trifluoromethoxy)phenylaminocarbonyloxymethyl, methyl(2-methylthiophenyl)aminocarbonyloxymethyl, methyl(3-trifluoromethylphenyl)aminocarbonyloxymethyl, methyl(2-cyanophenyl)aminocarbonyloxymethyl, methyl(4-nitrophenyl)aminocarbonyloxymethyl, ethyl(phenyl)aminocarbonyloxymethyl, and 2-{methyl(phenyl)aminocarbonyloxy}ethyl. Each is selected from a specified range of the number of carbon atoms.

Examples of the alkylthioalkyl include methylthiomethyl, ethylthiomethyl, propylthiomethyl, i-propylthiomethyl, butylthiomethyl, i-butylthiomethyl, s-butylthiomethyl, t-butylthiomethyl, pentylthiomethyl, 1-methylthioethyl, 2-methylthioethyl, 2-ethylthioethyl, and 3-methylthiopropyl. Each is selected from a specified range of the number of carbon atoms.

Examples of the haloalkylthioalkyl include fluoromethylthiomethyl, chloromethylthiomethyl, bromomethylthiomethyl, 2,2,2-trifluoroethylthiomethyl, and 2-(chloromethylthio)ethyl. Each is selected from a specified range of the number of carbon atoms.

Examples of the alkylsulfinyl include methylsulfinyl, ethylsulfinyl, propylsulfinyl, i-propylsulfinyl, butylsulfinyl, i-butylsulfinyl, s-butylsulfinyl, and t-butyl sulfinyl. Each is selected from a specified range of the number of carbon atoms.

Examples of the alkylsulfinylalkyl include methylsulfinylmethyl, ethylsulfinylmethyl, propylsulfinylmethyl, i-propylsulfinylmethyl, butylsulfinylmethyl, i-butylsulfinylmethyl, s-butylsulfinylmethyl, t-butylsulfinylmethyl, pentylsulfinylmethyl, 1-(methylsulfinyl)ethyl, 2-(methylsulfinyl)ethyl, 2-(ethylsulfinyl)ethyl, and 3-(methylsulfinyl)propyl. Each is selected from a specified range of the number of carbon atoms.

Examples of the haloalkylsulfinylalkyl include fluoromethylsulfinylmethyl, chloromethylsulfinylmethyl, bromomethylsulfinylmethyl, 2,2,2-trifluoroethylsulfinylmethyl, and 2-(chloromethylsulfinyl)ethyl. Each is selected from a specified range of the number of carbon atoms.

Examples of the alkylsulfonylalkyl include methylsulfonylmethyl, ethylsulfonylmethyl, propylsulfonylmethyl, i-propylsulfonylmethyl, butylsulfonylmethyl, i-butylsulfonylmethyl, s-butylsulfonylmethyl, t-butylsulfonylmethyl, pentylsulfonylmethyl, 1-(methylsulfonyl)ethyl, 2-(methylsulfonyl)ethyl, 2-(ethylsulfonyl)ethyl, and 3-(methylsulfonyl)propyl. Each is selected from a specified range of the number of carbon atoms.

Examples of the haloalkylsulfonylalkyl include fluoromethylsulfonylmethyl, chloromethylsulfonylmethyl, bromomethylsulfonylmethyl, 2,2,2-trifluoroethylsulfonylmethyl, and 2-(chloromethylsulfonyl)ethyl. Each is selected from a specified range of the number of carbon atoms.

Examples of the phenylthioalkyl and phenylthioalkyl substituted by one or more substituents selected from Z² include phenylthiomethyl, 2-fluorophenylthiomethyl, 2-chlorophenylthiomethyl, 3-bromophenylthiomethyl, 4-iodophenylthiomethyl, 4-methylphenylthiomethyl, 2-methoxyphenylthiomethyl, 4-aminophenylthiomethyl, 4-trifluoromethoxyphenylthiomethyl, 2-methylthiophenylthiomethyl, 3-trifluoromethylthiophenylthiomethyl, 2-cyanophenylthiomethyl, 4-nitrophenylthiomethyl, 2-trifluoromethylphenylthiomethyl, and 2-(phenylthio)ethyl. Each is selected from a specified range of the number of carbon atoms.

Examples of the phenylsulfinylalkyl and phenylsulfinylalkyl substituted by one or more substituents selected from Z² include phenylsulfinylmethyl, 2-fluorophenylsulfinylmethyl, 2-chlorophenylsulfinylmethyl, 3-bromophenylsulfinylmethyl, 4-iodophenylsulfinylmethyl, 4-methylphenylsulfinylmethyl, 2-methoxyphenylsulfinylmethyl, 4-aminophenylsulfinylmethyl, 4-trifluoromethoxyphenylsulfinylmethyl, 2-methylthiophenylsulfinylmethyl, 3-trifluoromethylthiophenylsulfinylmethyl, 2-cyanophenylsulfinylmethyl, 4-nitrophenylsulfinylmethyl, 2-trifluoromethylphenylsulfinylmethyl, and 2-(phenylsulfinyl)ethyl. Each is selected from a specified range of the number of carbon atoms.

Examples of the phenylsulfonylalkyl and phenylsulfonylalkyl substituted by one or more substituents selected from Z² include phenylsulfonylmethyl, 2-fluorophenylsulfonylmethyl, 2-chlorophenylsulfonylmethyl 3-bromophenylsulfonylmethyl, 4-iodophenylsulfonylmethyl, 4-methylphenylsulfonylmethyl, 2-methoxyphenylsulfonylmethyl, 4-aminophenylsulfonylmethyl, 4-trifluoromethoxyphenylsulfonylmethyl, 2-methylthiophenylsulfonylmethyl, 3-trifluoromethylthiophenylsulfonylmethyl, 2-cyanophenylsulfonylmethyl, 4-nitrophenylsulfonylmethyl, 2-trifluoromethylphenylsulfonylmethyl, and 2-(phenylsulfonyl)ethyl. Each is selected from a specified range of the number of carbon atoms.

Examples of the phenylalkylthioalkyl and phenylalkylthioalkyl substituted by one or more substituents selected from Z² include benzylthiomethyl, 2-fluorobenzylthiomethyl, 2-chlorobenzylthiomethyl, 3-bromobenzylthiomethyl, 4-iodobenzylthiomethyl, 4-methylbenzylthiomethyl, 2-methoxybenzylthiomethyl, 4-aminobenzylthiomethyl, 4-trifluoromethoxybenzylthiomethyl, 2-methylthiobenzylthiomethyl, 3-trifluoromethylthiobenzylthiomethyl, 2-cyanobenzylthiomethyl, 4-nitrobenzylthiomethyl, 2-trifluoromethylbenzylthiomethyl, phenethylthiomethyl, and 2-(benzylthio)ethyl. Each is selected from a specified range of the number of carbon atoms.

Examples of the phenylalkylsulfinylalkyl and phenylalkylsulfinylalkyl substituted by one or more substituents selected from Z² include benzylsulfinylmethyl, 2-fluorobenzylsulfinylmethyl, 2-chlorobenzylsulfinylmethyl, 3-bromobenzylsulfinylmethyl, 4-iodobenzylsulfinylmethyl, 4-methylbenzylsulfinylmethyl, 2-methoxybenzylsulfinylmethyl, 4-aminobenzylsulfinylmethyl, 4-trifluoromethoxybenzylsulfinylmethyl, 2-methylthiobenzylsulfinylmethyl, 3-trifluoromethylsulfinylbenzylthiomethyl, 2-cyanobenzylsulfinylmethyl, 4-nitrobenzylsulfinylmethyl, 2-trifluoromethylbenzylsulfinylmethyl, phenethylsulfinylmethyl, and 2-(benzylsulfinyl)ethyl. Each is selected from a specified range of the number of carbon atoms.

Examples of the phenylalkylsulfonylalkyl and phenylalkylsulfonylalkyl substituted by one or more substituents selected from Z² include benzylsulfonylmethyl, 2-fluorobenzylsulfonylmethyl, 2-chlorobenzylsulfonylmethyl, 3-bromobenzylsulfonylmethyl, 4-iodobenzylsulfonylmethyl, 4-methylbenzylsulfonylmethyl, 2-methoxybenzylsulfonylmethyl, 4-aminobenzylsulfonylmethyl, 4-trifluoromethoxybenzylsulfonylmethyl, 2-methylthiobenzylsulfonylmethyl, 3-trifluoromethylthiobenzylsulfonylmethyl, 2-cyanobenzylsulfonylmethyl, 4-nitrobenzylsulfonylmethyl, 2-trifluoromethylbenzylsulfonylmethyl, phenethylsulfonylmethyl, and 2-(benzylsulfonyl)ethyl. Each is selected from a specified range of the number of carbon atoms.

Examples of the thiocyanatoalkyl include cyanothiomethyl, 1-(cyanothio)ethyl, and 1-(cyanothio)ethyl. Each is selected from a specified range of the number of carbon atoms.

Examples of the alkylcarbonylalkyl include methylcarbonylmethyl, ethylcarbonylmethyl, propylcarbonylmethyl, i-propylcarbonylmethyl, butylcarbonylmethyl, i-butylcarbonylmethyl, s-butylcarbonylmethyl, t-butylcarbonylmethyl, pentylcarbonylmethyl, 1-(methylcarbonyl)ethyl, 2-(methylcarbonyl)ethyl, 2-(ethylcarbonyl)ethyl, and 3-(methylcarbonyl)propyl. Each is selected from a specified range of the number of carbon atoms.

Examples of the haloalkylcarbonylalkyl include fluoromethylcarbonylmethyl, chloromethylcarbonylmethyl, bromomethylcarbonylmethyl, 2,2,2-trifluoroethylcarbonylmethyl, and 2-(chloromethylcarbonyl)ethyl. Each is selected from a specified range of the number of carbon atoms.

Examples of the alkoxycarbonylalkyl include methoxycarbonylmethyl, ethoxycarbonylmethyl, propyloxycarbonylmethyl, i-propyloxycarbonylmethyl, butyloxycarbonylmethyl, 2-(methoxycarbonyl)ethyl, 2-(ethoxycarbonyl)ethyl, and 3-(methoxycarbonyl)propyl. Each is selected from a specified range of the number of carbon atoms.

Examples of the haloalkoxycarbonylalkyl include fluoromethoxycarbonylmethyl, chloromethoxycarbonylmethyl, bromomethoxycarbonylmethyl, 2,2,2-trifluoroethoxycarbonylmethyl, and 2-(chloromethoxycarbonyl)ethyl. Each is selected from a specified range of the number of carbon atoms.

Examples of the phenylcarbonylalkyl and phenylcarbonylalkyl substituted by one or more substituents selected from Z² include phenylcarbonylmethyl, 2-fluorophenylcarbonylmethyl, 2-chlorophenylcarbonylmethyl, 3-bromophenylcarbonylmethyl, 4-iodophenylcarbonylmethyl, 4-methylphenylcarbonylmethyl, 2-methoxyphenylcarbonylmethyl, 4-aminophenylcarbonylmethyl, 4-trifluoromethoxyphenylcarbonylmethyl, 2-methylthiophenylcarbonylmethyl, 3-trifluoromethylthiophenylcarbonylmethyl, 2-cyanophenylcarbonylmethyl, 4-nitrophenylcarbonylmethyl, 2-trifluoromethylphenylcarbonylmethyl, and 2-phenylcarbonylethyl. Each is selected from a specified range of the number of carbon atoms.

Examples of the alkylcarbonyl include acetyl, ethylcarbonyl, propionyl, butyryl, i-butyryl, valeroyl, i-valeroyl, 2-methylbutyryl, pivaloyl, hexanoyl, 2-ethylbutyryl, 2,2-dimethyl-1-propionyl, 2-methylvaleroyl, 4-methylvaleroyl, heptanoyl, 2,2-dimethylvaleroyl, 2-ethylhexanoyl, 2-propyl-valeroyl, octanoyl, nonanoyl, 3,5,5-trimethylhexanoyl, and decanoyl. Each is selected from a specified range of the number of carbon atoms.

Examples of the haloalkylcarbonyl include fluoroacetyl, chloroacetyl, difluoroacetyl, dichloroacetyl, trifluoroacetyl, chlorodifluoroacetyl, bromodifluoroacetyl, trichloroacetyl, pentafluoropropionyl, 4 chlorobutyryl, heptafluorobutyryl, and 3-chloro-2,2-dimethylpropionyl. Each is selected from a specified range of the number of carbon atoms.

Examples of the cycloalkylcarbonyl include cyclopropanecarbonyl, cyclobutanecarbonyl, cyclopentanecarbonyl, and cyclohexanecarbonyl. Each is selected from a specified range of the number of carbon atoms.

Examples of the halocycloalkylcarbonyl include 2-fluorocyclopropanecarbonyl, 1-chlorocyclopropanecarbonyl, 2-bromo-1-methylcyclopropanecarbonyl, 2,2-difluorocyclopropanecarbonyl, and 1,2-dichlorocyclopropanecarbonyl. Each is selected from a specified range of the number of carbon atoms.

Examples of the alkylcycloalkylcarbonyl include 2-methylcyclopropanecarbonyl, 2,2-dimethylcyclopropanecarbonyl, 2,2,4,4-tetramethylcyclobutanecarbonyl, and 4-(i-propyl)cyclohexanecarbonyl. Each is selected from a specified range of the number of carbon atoms.

Examples of the alkylhalocycloalkylcarbonyl include 2-chloro-3-methylcyclopropanecarbonyl, 2,2-dichloro-1-methylcyclopropanecarbonyl, and 3-fluoro-4-(i-propyl)cyclohexanecarbonyl. Each is selected from a specified range of the number of carbon atoms.

Examples of the alkenylcarbonyl include ethenylcarbonyl, 1-methylethenylcarbonyl, 1-propenylcarbonyl, 2-propenylcarbonyl, 1-butenylcarbonyl, 1-methyl-2-propenylcarbonyl, 2-methyl-1-propenylcarbonyl, 2-methyl-2-propenylcarbonyl, and 1-hexenylcarbonyl. Each is selected from a specified range of the number of carbon atoms.

Examples of the haloalkenylcarbonyl include 1-chloroethenylcarbonyl, 2-chloroethenylcarbonyl, 2-fluoroethenylcarbonyl, 2,2-dichloroethenylcarbonyl, 3-chloro-2-propenylcarbonyl, 3-fluoro-2-propenylcarbonyl, 2-chloro-2-propenylcarbonyl, and 4-chloro-3-butenylcarbonyl. Each is selected from a specified range of the number of carbon atoms.

Examples of the phenylalkenylcarbonyl and phenylalkenylcarbonyl substituted by one or more substituents selected from Z² include 1-phenylethenylcarbonyl, 2-phenylethenylcarbonyl, 3-phenyl-2-propenylcarbonyl, and 4-phenyl-3-butenylcarbonyl. Each is selected from a specified range of the number of carbon atoms.

Examples of alkoxyalkenylcarbonyl include 1-methoxyethenylcarbonyl, 1-ethoxyethenylcarbonyl, 1-(i-propyloxy)ethenylcarbonyl, 2-methoxyethenylcarbonyl, 2-ethoxyethenylcarbonyl, 3-methoxy-2-propenylcarbonyl, and 4-methoxy-3-butenylcarbonyl. Each is selected from a specified range of the number of carbon atoms.

Examples of the alkynylcarbonyl include ethynylcarbonyl, 1-propynylcarbonyl, 2-propynylcarbonyl, 1-butynylcarbonyl, 2-butynylcarbonyl, 3-butynylcarbonyl, 1-methyl-2-propynylcarbonyl, 1-pentynylcarbonyl, 1-methyl-2-butynylcarbonyl, 2-methyl-3-butynylcarbonyl, 1-hexynylcarbonyl, 1-methyl-3-pentynylcarbonyl, 2-methyl-3-pentynylcarbonyl, 3-methyl-4-pentynylcarbonyl, 4-methyl-2-pentynylcarbonyl, 1,1-dimethyl-2-butynylcarbonyl, 1,2-dimethyl-3-butynylcarbonyl, 2,2-dimethyl-3-butynylcarbonyl, 1-ethyl-2-butynylcarbonyl, and 2-ethyl-3-butynylcarbonyl. Each is selected from a specified range of the number of carbon atoms.

Examples of the phenylcarbonyl substituted by one or more substituents selected from Z² include 2-fluorophenylcarbonyl, 2-chlorophenylcarbonyl, 4-bromophenylcarbonyl, 4-methylphenylcarbonyl, 2-methoxyphenylcarbonyl, 3-aminophenylcarbonyl, 4-trifluoromethoxyphenylcarbonyl, 2-methylthiophenylcarbonyl, 3-trifluoromethylthiophenylcarbonyl, 4-cyanophenylcarbonyl, 2-nitrophenylcarbonyl, and 4-trifluoromethylphenylcarbonyl.

Examples of the heterocyclylcarbonyl and heterocyclylcarbonyl substituted by one or more substituents selected from Z² include (tetrahydrofuran-2-yl)carbonyl, (tetrahydrofuran-3-yl)carbonyl, (tetrahydrothiophen-2-yl)carbonyl, (tetrahydropyran-4-yl)carbonyl, (furan-2-yl)carbonyl, (3-methylfuran-2-yl)carbonyl, (furan-3-yl)carbonyl, (thiophene-2-yl)carbonyl, (thiophene-3-yl)carbonyl, (pyridin-2-yl)carbonyl, (N-methylpyrrol-2-yl)carbonyl, (pyridin-3-yl)carbonyl, and (pyridin-4-yl)carbonyl. Each is selected from a specified range of the number of carbon atoms.

Examples of the cycloalkylalkylcarbonyl include cyclopropylmethylcarbonyl, 2-cyclopropylethylcarbonyl, cyclopentylmethylcarbonyl, and cyclohexylmethylcarbonyl. Each is selected from a specified range of the number of carbon atoms.

Examples of the phenylalkylcarbonyl and phenylalkylcarbonyl substituted by one or more substituents selected from Z² include benzylcarbonyl, 2-fluorobenzylcarbonyl, 3-fluorobenzylcarbonyl, 4-fluorobenzylcarbonyl, 2-chlorobenzylcarbonyl, 3-bromobenzylcarbonyl, 4-iodobenzylcarbonyl, 2,4-difluorobenzylcarbonyl, 2-methylbenzylcarbonyl, 3-methylbenzylcarbonyl, 4-methylbenzylcarbonyl, 2-methoxybenzylcarbonyl, 4-aminobenzylcarbonyl, 4-trifluoromethoxybenzylcarbonyl, 2-methylthiobenzylcarbonyl, 3-trifluoromethylthiobenzylcarbonyl, 2-cyanobenzylcarbonyl, 4-nitrobenzylcarbonyl, 2-trifluoromethylbenzylcarbonyl, and 2-phenethylcarbonyl. Each is selected from a specified range of the number of carbon atoms.

Examples of the heterocyclylalkylcarbonyl and heterocyclylalkylcarbonyl substituted by one or more substituents selected from Z² include (oxiran-2-yl)methylcarbonyl, (2-(methyloxiran-2-yl)methylcarbonyl, (3,3-dimethyloxiran-2-yl)methylcarbonyl, (oxetan-2-yl)methylcarbonyl, (oxetan-3-yl)methylcarbonyl, 2-tetrahydrofurfurylcarbonyl, 3-tetrahydrofurfurylcarbonyl, (tetrahydrothiophen-2-yl)methylcarbonyl, (1,1-dioxytetrahydrothiophen-2-yl)methylcarbonyl, (tetrahydropyran-4-yl)methylcarbonyl, (1,3-dioxan-2-yl)methylcarbonyl, 2-furfuryl, 3-furfuryl, (thiophen-2-yl)methylcarbonyl, (thiophen-3-yl)methylcarbonyl, (pyridin-2-yl)methylcarbonyl, (pyridin-3-yl)methylcarbonyl, and (pyridin-4-yl)oxycarbonyl. Each is selected from a specified range of the number of carbon atoms.

Examples of the hydroxyalkylcarbonyl include hydroxyacetyl, 2-hydroxyethylcarbonyl, 3-hydroxypropionyl, 3-hydroxybutyryl, 2-hydroxy-i-butyryl, 5-hydroxyvaleroyl, 3-hydroxy-i-valeroyl, 5-hydroxy-2-methylbutyryl, 2-hydroxypivaloyl, 6-hydroxyhexanoyl, and 5-hydroxy-2,2-dimethylvaleroyl. Each is selected from a specified range of the number of carbon atoms.

Examples of the alkoxyalkylcarbonyl include methoxymethylcarbonyl, ethoxymethylcarbonyl, i-propyloxymethylcarbonyl, 2-methoxyethylcarbonyl, 3-methoxybutyryl, 2-methoxy-i-butyryl, 5-ethoxyvaleroyl, and 5-methoxy-2,2-dimethylvaleroyl. Each is selected from a specified range of the number of carbon atoms.

Examples of the haloalkoxyalkylcarbonyl include fluoromethoxyacetyl, chloromethoxyacetyl, bromomethoxyacetyl, 2,2,2-trifluoroethoxyacetyl, and 2-(chloromethoxycarbonyl)ethylcarbonyl. Each is selected from a specified range of the number of carbon atoms.

Examples of the phenoxyalkylcarbonyl and phenoxyalkylcarbonyl substituted by one or more substituents selected from Z² include phenoxymethylcarbonyl, 2-phenoxyethylcarbonyl, 2-fluorophenoxymethylcarbonyl, 2-chlorophenoxymethylcarbonyl, 4-bromophenoxymethylcarbonyl, 4-methylphenoxymethylcarbonyl, 2-methoxyphenoxymethylcarbonyl, 3-aminophenoxymethylcarbonyl, 4-trifluoromethoxyphenoxymethylcarbonyl, 2-methylthiophenoxymethylcarbonyl, 3-trifluoromethylthiophenoxymethylcarbonyl, 4-cyanophenoxymethylcarbonyl, 2-nitrophenoxymethylcarbonyl, and 4-trifluoromethylphenoxymethylcarbonyl.

Examples of the phenoxyhaloalkylcarbonyl and phenoxyhaloalkylcarbonyl substituted by one or more substituents selected from Z² include phenoxydifluoromethylcarbonyl, 2-phenoxy-2,2-difluoroethylcarbonyl, 2-fluorophenoxydichloromethylcarbonyl, 2-chlorophenoxichloromethylcarbonyl, 4-bromophenoxibromomethylcarbonyl, and 4-trifluoromethylphenoxydifluoromethylcarbonyl.

Examples of the trialkylsilyloxyalkylcarbonyl include trimethylsilyloxymethylcarbonyl, 2-(trimethylsilyloxy)ethylcarbonyl, 1-(trimethylsilyloxy)ethylcarbonyl, 2-(trimethylsilyloxy)ethylcarbonyl, and 2-(t-butyldimethylsilyloxy)ethylcarbonyl. Each is selected from a specified range of the number of carbon atoms.

Examples of the alkoxyalkoxyalkylcarbonyl include methoxymethoxymethylcarbonyl, ethoxymethoxymethylcarbonyl, propyloxymethoxymethylcarbonyl, i-propyloxymethoxymethylcarbonyl, butyloxymethoxymethylcarbonyl, 1-(methoxymethoxy)ethylcarbonyl, 2-(methoxymethoxy)ethylcarbonyl, 2-(ethoxymethoxy)ethylcarbonyl, and 2-(ethoxy)ethoxymethylcarbonyl. Each is selected from a specified range of the number of carbon atoms.

Examples of the alkylcarbonylalkylcarbonyl include methylcarbonylmethylcarbonyl, ethylcarbonylmethylcarbonyl, propylcarbonylmethylcarbonyl, i-propylcarbonylmethylcarbonyl, butylcarbonylmethylcarbonyl, i-butylcarbonylmethylcarbonyl, s-butylcarbonylmethylcarbonyl, t-butylcarbonylmethylcarbonyl, pentylcarbonylmethylcarbonyl, 1-(methylcarbonyl)ethylcarbonyl, 2-(methylcarbonyl)ethylcarbonyl, 2-(ethylcarbonyl)ethylcarbonyl, and 3-(methylcarbonyl)propylcarbonyl. Each is selected from a specified range of the number of carbon atoms.

Examples of the alkylcarbonyloxyalkylcarbonyl include acetoxymethylcarbonyl, propionyloxymethylcarbonyl, butyryloxymethylcarbonyl, i-butyryloxymethylcarbonyl, valeroyloxymethylcarbonyl, i-valeroyloxymethylcarbonyl, 2-methylbutyryloxymethylcarbonyl, pivaloyloxymethylcarbonyl, heptanoyloxymethylcarbonyl, 1-(acetoxy)ethylcarbonyl, 2-(acetoxy)ethylcarbonyl, 2-(propionyloxy)ethylcarbonyl, and 3-(acetoxy)propylcarbonyl. Each is selected from a specified range of the number of carbon atoms.

Examples of the alkoxycarbonylalkylcarbonyl include methoxycarbonylmethylcarbonyl, ethoxycarbonylmethylcarbonyl, propyloxycarbonylmethylcarbonyl, i-propyloxycarbonylmethylcarbonyl, butyloxycarbonylmethylcarbonyl, 2-(methoxycarbonyl)ethylcarbonyl, 2-(ethoxycarbonyl)ethylcarbonyl, and 3-(methoxycarbonyl)propylcarbonyl. Each is selected from a specified range of the number of carbon atoms.

Examples of the alkylthioalkylcarbonyl include methylthiomethylcarbonyl, ethylthiomethylcarbonyl, propylthiomethylcarbonyl, i-propylthiomethylcarbonyl, butylthiomethylcarbonyl, i-butylthiomethylcarbonyl, s-butylthiomethylcarbonyl, t-butylthiomethylcarbonyl, pentylthiomethylcarbonyl, 1-(methylthio)ethylcarbonyl, 2-(methylthio)ethylcarbonyl, 2-(ethylthio)ethylcarbonyl, and 3-(methylthio)propylcarbonyl. Each is selected from a specified range of the number of carbon atoms.

Examples of the haloalkylthioalkylcarbonyl include fluoromethylthiomethylcarbonyl, chloromethylthiomethylcarbonyl, bromomethylthiomethylcarbonyl, 2,2,2-trifluoroethylthiomethylcarbonyl, and 2-(chloromethylthio)ethylcarbonyl. Each is selected from a specified range of the number of carbon atoms.

Examples of the alkylsulfinyl include methylsulfinyl, ethylsulfinyl, propylsulfinyl, i-propylsulfinyl, butylsulfinyl, i-butylsulfinyl, s-butylsulfinyl, and t-butyl sulfinyl. Each is selected from a specified range of the number of carbon atoms.

Examples of the alkoxycarbonyl include methoxycarbonyl, ethoxycarbonyl, propyloxycarbonyl, i-propyloxycarbonyl, butyloxycarbonyl, s-butyloxycarbonyl, i-butyloxycarbonyl, t-butyloxycarbonyl, pentyloxycarbonyl, (2,2-dimethyl-1-propyl)oxycarbonyl, hexyloxycarbonyl, and heptyloxycarbonyl. Each is selected from a specified range of the number of carbon atoms.

Examples of the haloalkoxycarbonyl include fluoromethoxycarbonyl, difluoromethoxycarbonyl, trifluoromethoxycarbonyl, 1-fluoroethoxycarbonyl, 2-fluoroethoxycarbonyl, 2-chloroethoxycarbonyl, 2-bromoethoxycarbonyl, 2,2-difluoroethoxycarbonyl, 2,2,2-trifluoroethoxycarbonyl, 1-fluoropropyloxycarbonyl, 2-fluoropropyloxycarbonyl, 3-fluoropropyloxycarbonyl, 3-chloropropyloxycarbonyl, 3-bromopropyloxycarbonyl, (1,3-difluoropropan-2-yl)oxycarbonyl, (2,2-difluoro-1-methylethyl)oxycarbonyl, (2,2,2-trifluoro-1-methyl-ethyl)oxycarbonyl, 4-fluorobutyloxycarbonyl, and 4-chlorobutyloxycarbonyl. Each is selected from a specified range of the number of carbon atoms.

Examples of the cycloalkyloxycarbonyl include cyclopropyloxycarbonyl, cyclobutyloxycarbonyl, cyclopentyloxycarbonyl, and cyclohexyloxycarbonyl. Each is selected from a specified range of the number of carbon atoms.

Examples of the alkenyloxycarbonyl include ethenyloxycarbonyl, 1-propenyloxycarbonyl, 2-propenyloxycarbonyl, 1-butenyloxycarbonyl, 1-methyl-2-propenyloxycarbonyl, 2-methyl-2-propenyloxycarbonyl, and 1-hexenyloxycarbonyl. Each is selected from a specified range of the number of carbon atoms.

Examples of the haloalkenyloxycarbonyl include 1-chloroethenyloxycarbonyl, 2-chloroethenyloxycarbonyl, 2-fluoroethenyloxycarbonyl, 2,2-dichloroethenyloxycarbonyl, 3-chloro-2-propenyloxycarbonyl, 3-fluoro-2-propenyloxycarbonyl, 2-chloro-2-propenyloxycarbonyl, and 4-chloro-3-butenyloxycarbonyl. Each is selected from a specified range of the number of carbon atoms.

Examples of the alkynyloxycarbonyl include ethynyloxycarbonyl, 1-propynyloxycarbonyl, 2-propynyloxycarbonyl, 1-butynyloxycarbonyl, 1-methyl-2-propynyloxycarbonyl, 2-methyl-2-propynyloxycarbonyl, and 1-hexynyloxycarbonyl. Each is selected from a specified range of the number of carbon atoms.

Examples of the phenoxycarbonyl substituted by one or more substituents selected from Z² include 2-fluorophenoxycarbonyl, 2-chlorophenoxycarbonyl, 4-bromophenoxycarbonyl, 4-methylphenoxycarbonyl, 2-methoxyphenoxycarbonyl, 3-aminophenoxycarbonyl, 4-trifluoromethoxyphenoxycarbonyl, 2-methylthiophenoxycarbonyl, 3-trifluoromethylthiophenoxycarbonyl, 4-cyanophenoxycarbonyl, 2-nitrophenoxycarbonyl, 4-nitrophenoxycarbonyl, and 4-trifluoromethylphenoxycarbonyl.

Examples of the heterocyclyloxycarbonyl and heterocyclyloxycarbonyl substituted by one or more substituents selected from Z² include (oxiran-2-yl)oxycarbonyl, (2-methyloxiran-2-yl)oxycarbonyl, (3,3-dimethyloxiran-2-yl)oxycarbonyl, (oxetan-2-yl)oxycarbonyl, (oxetan-3-yl)oxycarbonyl, (tetrahydrofuran-2-yl)oxycarbonyl, (tetrahydrofuran-3-yl)oxycarbonyl, (tetrahydrofuran-4-yl)oxycarbonyl, (tetrahydrothiophen-2-yl)oxycarbonyl, tetrahydrothiophen-3-yl)oxycarbonyl, (1,1-dioxytetrahydrothiophen-3-yl)oxycarbonyl, (2,5-dioxopyrrolidine-1-yl)oxycarbonyl, (tetrahydropyran-3-yl)oxycarbonyl, (tetrahydropyran-4-yl)oxycarbonyl, (tetrahydrothiopyran-4-yl)oxycarbonyl, (1,1-dioxidotetrahydro-2H-thiopyran-4-yl)oxycarbonyl, (1,3-dioxan-5-yl)oxycarbonyl, (2,2-dimethyl-1,3-dioxan-5-yl)oxycarbonyl, (thiophene-2-yl)oxycarbonyl, (thiophene-3-yl)oxycarbonyl, (pyridin-2-yl)oxycarbonyl, (pyridin-3-yl)oxycarbonyl, and (pyridin-4-yl)oxycarbonyl. Each is selected from a specified range of the number of carbon atoms.

Examples of the cycloalkylalkoxycarbonyl include cyclopropylmethoxycarbonyl, 2-cyclopropylethoxyoxycarbonyl, cyclopentylmethoxycarbonyl, and cyclohexylmethoxycarbonyl. Each is selected from a specified range of the number of carbon atoms.

Examples of the phenylalkoxycarbonyl and phenylalkoxycarbonyl substituted by one or more substituents selected from Z² include benzyloxycarbonyl, 2-fluorobenzyloxycarbonyl, 2-chlorobenzyloxycarbonyl, 3-bromobenzyloxycarbonyl, 4-iodobenzyloxycarbonyl, 4-methylbenzyloxycarbonyl, 2-methoxybenzyloxycarbonyl, 4-aminobenzyloxycarbonyl, 4-trifluoromethoxybenzyloxycarbonyl, 2-methylthiobenzyloxycarbonyl, 3-trifluoromethylthiobenzyloxycarbonyl, 2-cyanobenzyloxycarbonyl, 4-nitrobenzyloxycarbonyl, 2-trifluoromethylbenzyloxycarbonyl, and 2-phenethyloxycarbonyl.

Examples of the heterocyclylalkoxycarbonyl and heterocyclylalkoxycarbonyl substituted by one or more substituents selected from Z² include (oxiran-2-yl)methoxycarbonyl, (2-methyloxiran-2-yl)methoxycarbonyl, (3,3-dimethyloxiran-2-yl)methoxycarbonyl, 1-(oxiran-2-yl)ethoxycarbonyl, (oxetan-2-yl)ethoxycarbonyl, (oxetan-3-yl)methoxycarbonyl, (tetrahydrofuran-2-yl)methoxycarbonyl, (tetrahydrofuran-3-yl)methoxycarbonyl, (tetrahydrofuran-4-yl)methoxycarbonyl, (tetrahydrothiophen-2-yl)methoxycarbonyl, (1,1-dioxytetrahydrothiophen-2-yl)methoxycarbonyl, [(2-oxo-1,3-dioxolan-4-yl)methyl]oxycarbonyl, (tetrahydropyran-2-yl)methoxycarbonyl, (tetrahydropyran-4-yl)methoxycarbonyl, (1,3-dioxan-2-yl)methoxycarbonyl, (thiophen-2-yl)methoxycarbonyl, (thiophen-3-yl)methoxycarbonyl, [(5-methyl-2-oxo-1,3-dioxol-4-yl)methyl]oxycarbonyl, (pyridin-2-yl)methoxycarbonyl, (pyridin-3-yl)methoxycarbonyl, and (pyridin-4-yl)methoxycarbonyl. Each is selected from a specified range of the number of carbon atoms.

Examples of the cyanoalkoxycarbonyl include cyanomethoxycarbonyl, 1-cyanoethoxycarbonyl, and 2-cyanoethoxycarbonyl. Each is selected from a specified range of the number of carbon atoms.

Examples of the hydroxyalkoxycarbonyl include 2-hydroxyethoxycarbonyl, 3-hydroxypropyloxycarbonyl, 3-hydroxybutoxycarbonyl, 2-hydroxy-i-butoxycarbonyl, and 4-hydroxy-2-methylbutoxycarbonyl. Each is selected from a specified range of the number of carbon atoms.

Examples of the alkoxyalkoxycarbonyl include methoxymethoxycarbonyl, ethoxymethoxycarbonyl, propyloxymethoxycarbonyl, i-propyloxymethoxycarbonyl, butyloxymethoxycarbonyl, 1-methoxyethoxycarbonyl, 2-methoxyethoxycarbonyl, (1,3-dimethoxypropan-2-yl)oxycarbonyl, (1,3-diethoxypropan-2-yl)oxycarbonyl, and (1-methoxy-3-ethoxypropan-2-yl)oxycarbonyl. Each is selected from a specified range of the number of carbon atoms.

Examples of the haloalkoxyalkoxycarbonyl include fluoromethoxymethoxycarbonyl, chloromethoxymethoxycarbonyl, bromomethoxymethoxycarbonyl, and 2,2,2-trifluoroethoxymethoxycarbonyl. Each is selected from a specified range of the number of carbon atoms.

Examples of the trialkylsilyloxyalkoxycarbonyl include trimethylsilyloxymethoxycarbonyl, 2-(trimethylsilyloxy)ethoxycarbonyl, 1-(trimethylsilyloxy)ethoxycarbonyl, and 2-(trimethylsilyloxy)ethoxycarbonyl. Each is selected from a specified range of the number of carbon atoms.

Examples of the alkyldiphenylsilyloxyalkoxycarbonyl include methyldiphenylsilyloxymethoxycarbonyl, ethyldiphenylsilyloxymethoxycarbonyl, and t-butyldiphenylsilyloxymethoxycarbony. Each is selected from a specified range of the number of carbon atoms.

Examples of the alkoxyalkoxyalkoxycarbonyl include methoxymethoxymethoxycarbonyl, ethoxymethoxymethoxycarbonyl, propyloxymethoxymethoxycarbonyl, i-propyloxymethoxymethoxycarbonyl, 1-(methoxymethoxy)ethoxycarbonyl, and 2-(methoxymethoxy)ethoxycarbonyl. Each is selected from a specified range of the number of carbon atoms.

Examples of the alkylcarbonyloxyalkoxycarbonyl include acetoxymethoxycarbonyl, propionyloxymethoxycarbonyl, butyryloxymethoxycarbonyl, i-butyryloxymethoxycarbonyl, valeroyloxymethoxycarbonyl, i-valeroyloxymethoxycarbonyl, 2-methylbutyryloxymethoxycarbonyl, pivaloyloxymethoxycarbonyl, heptanoyloxymethoxycarbonyl, 1-(acetoxy)ethoxycarbonyl, 2-(acetoxy)ethoxycarbonyl, 2-(propionyloxy)ethoxycarbonyl, and 3-(acetoxy)propyloxycarbonyl. Each is selected from a specified range of the number of carbon atoms.

Examples of the alkylsulfonyloxyalkoxycarbonyl include methylsulfonyloxymethoxycarbonyl, ethylsulfonyloxymethoxycarbonyl, propylsulfonyloxymethoxycarbonyl, i-propylsulfonyloxymethoxycarbonyl, butylsulfonyloxymethoxycarbonyl, i-butylsulfonyloxymethoxycarbonyl, s-butylsulfonyloxymethoxycarbonyl, t-butylsulfonyloxymethoxycarbonyl, 1-(methylsulfonyloxy)ethoxycarbonyl, and 2-(methylsulfonyloxy)ethoxycarbonyl. Each is selected from a specified range of the number of carbon atoms.

Examples of the alkylcarbonylalkoxycarbonyl include methylcarbonylmethoxycarbonyl, propylcarbonylmethoxycarbonyl, i-propylcarbonylmethoxycarbonyl, 1-(methylcarbonyl)ethoxycarbonyl, and 2-(methylcarbonyl)ethoxycarbonyl. Each is selected from a specified range of the number of carbon atoms.

Examples of the alkoxycarbonylalkoxycarbonyl include methoxycarbonylmethoxycarbonyl, ethoxycarbonylmethoxycarbonyl, propyloxycarbonylmethoxycarbonyl, i-propyloxycarbonylmethoxycarbonyl, butyloxycarbonylmethoxycarbonyl, 2-(methoxycarbonyl)ethoxycarbonyl, 2-(ethoxycarbonyl)ethoxycarbonyl, and 3-(methoxycarbonyl)propyloxycarbonyl. Each is selected from a specified range of the number of carbon atoms.

Examples of the alkylthioalkoxycarbonyl include methylthiomethoxycarbonyl, ethylthiomethoxycarbonyl, propylthiomethoxycarbonyl, i-propylthiomethoxycarbonyl, butylthiomethoxycarbonyl, 2-methylthioethoxycarbonyl, and 1-methylthioethoxycarbonyl. Each is selected from a specified range of the number of carbon atoms.

Examples of the haloalkylthioalkoxycarbonyl include fluoromethylthiomethoxycarbonyl, chloromethylthiomethoxycarbonyl, bromomethylthiomethoxycarbonyl, 2,2,2-trifluoroethylthiomethoxycarbonyl, and 2-(chloromethylthio)ethoxycarbonyl. Each is selected from a specified range of the number of carbon atoms.

Examples of the alkylsulfinylalkoxycarbonyl include methylsulfinylmethoxycarbonyl, ethylsulfinylmethoxycarbonyl, propylsulfinylmethoxycarbonyl, i-propylsulfinylmethoxycarbonyl, butylsulfinylmethoxycarbonyl, 2-methylsulfinyl ethoxycarbonyl, and 1-methylsulfinylethoxycarbonyl. Each is selected from a specified range of the number of carbon atoms.

Examples of the alkylsulfonylalkoxycarbonyl include methylsulfonylmethoxycarbonyl, ethylsulfonylmethoxycarbonyl, propylsulfonylmethoxycarbonyl, i-propylsulfonylmethoxycarbonyl, butylsulfonylmethoxycarbonyl, 2-methylsulfonylethoxycarbonyl, and 1-methylsulfonylethoxycarbonyl. Each is selected from a specified range of the number of carbon atoms.

Examples of the alkylthiocarbonyl include methylthiocarbonyl, ethylthiocarbonyl, propylthiocarbonyl, i-propylthiocarbonyl, butylthiocarbonyl, i-butylthiocarbonyl, s-butylthiocarbonyl, and t-butylthiocarbony. Each is selected from a specified range of the number of carbon atoms.

Examples of the haloalkylthiocarbonyl include fluoromethylthiocarbonyl, chlorodifluoromethylthiocarbonyl, bromodifluoromethylthiocarbonyl, trifluoromethylthiocarbonyl, trichloromethylthiocarbonyl, 2,2,2-trifluoroethylthiocarbonyl, 1,1,2,2-tetrafluoroethylthiocarbonyl, 2-fluoroethylthiocarbonyl, and pentafluoroethylthiocarbonyl. Each is selected from a specified range of the number of carbon atoms.

Examples of the monoalkylaminocarbonyl include methylaminocarbonyl, ethylaminocarbonyl, propylaminocarbonyl, i-propylaminocarbonyl, butylaminocarbonyl, s-butylaminocarbonyl, i-butylaminocarbonyl, t-butylaminocarbonyl, pentylaminocarbonyl, and hexyl aminocarbonyl. Each is selected from a specified range of the number of carbon atoms.

Examples of the monohaloalkylaminocarbonyl include fluoromethylaminocarbonyl, difluoromethylaminocarbonyl, trifluoromethylaminocarbonyl, chloromethylaminocarbonyl, bromomethylaminocarbonyl, iodomethylaminocarbonyl, 2-fluoroethylaminocarbonyl, 2-chloroethylaminocarbonyl, 3-fluoropropylaminocarbonyl, 2-fluoropropylaminocarbonyl, and 1-fluoropropylaminocarbonyl. Each is selected from a specified range of the number of carbon atoms.

Examples of the dialkylaminocarbonyl include dimethylaminocarbonyl, diethylaminocarbonyl, dipropylaminocarbonyl, di(i-propyl)aminocarbonyl, dibutylaminocarbonyl, di(s-butyl)aminocarbonyl, di(i-butyl)aminocarbonyl, di(t-butyl)aminocarbonyl, dipentylaminocarbonyl, dihexylaminocarbonyl, ethyl(methyl)aminocarbonyl, and methyl(propyl)aminocarbony. Each is selected from a specified range of the number of carbon atoms.

Examples of the di(haloalkyl)aminocarbonyl include di(fluoromethyl)aminocarbonyl, di(chloromethyl)aminocarbonyl, di(bromomethyl)aminocarbonyl, fluoromethyl(methyl)aminocarbonyl, chloromethyl(methyl)aminocarbonyl, chloromethyl(ethyl)aminocarbonyl, di(2-fluoroethyl)aminocarbonyl, and (2-fluoroethyl)methylaminocarbonyl. Each is selected from a specified range of the number of carbon atoms.

Examples of the alkoxyoxalyl include methoxycarbonylcarbonyl, ethoxycarbonylcarbonyl, i-propyloxycarbonylcarbonyl, and t-butoxycarbonylcarbonyl. Each is selected from a specified range of the number of carbon atoms.

Examples of the alkylsulfonyl include methylsulfonyl, ethylsulfonyl, propylsulfonyl, i-propylsulfonyl, butylsulfonyl, i-butylsulfonyl, s-butylsulfonyl, and t-butyl sulfonyl. Each is selected from a specified range of the number of carbon atoms.

Examples of the haloalkylsulfonyl include fluoromethylsulfonyl, chloromethylsulfonyl, chlorodifluoromethylsulfonyl, bromodifluoromethylsulfonyl, trifluoromethylsulfonyl, trichloromethylsulfonyl, 2,2,2-trifluoroethylsulfonyl, 1,1,2,2-tetrafluoroethylsulfonyl, 2-fluoroethylsulfonyl, and pentafluoroethylsulfonyl. Each is selected from a specified range of the number of carbon atoms.

Examples of the cycloalkylsulfonyl include cyclopropanesulfonyl, cyclobutanesulfonyl, cyclopentanesulfonyl, and cyclohexanesulfonyl. Each is selected from a specified range of the number of carbon atoms.

Examples of the alkenylsulfonyl include ethenylsulfonyl, 1-propenylsulfonyl, 2-propenylsulfonyl, 1-butenylsulfonyl, 1-methyl-2-propenylsulfonyl, 2-methyl-2-propenylsulfonyl, and 1-hexenylsulfonyl. Each is selected from a specified range of the number of carbon atoms.

Examples of the haloalkenylsulfonyl include 1-chloroethenylsulfonyl, 2-chloroethenylsulfonyl, 2-fluoroethenylsulfonyl, 2,2-dichloroethenylsulfonyl, 3-chloro-2-propenylsulfonyl, 3-fluoro-2-propenylsulfonyl, 2-chloro-2-propenylsulfonyl, and 4-chloro-3-butenylsulfonyl. Each is selected from a specified range of the number of carbon atoms.

Examples of the phenylsulfonyl substituted by one or more substituents selected from Z² include 2-fluorophenylsulfonyl, 2-chlorophenylsulfonyl, 4-bromophenylsulfonyl, 4-methylphenylsulfonyl, 2-methoxyphenylsulfonyl, 3-aminophenylsulfonyl, 4-trifluoromethoxyphenylsulfonyl, 2-methylthiophenylsulfonyl, 3-trifluoromethylthiophenylsulfonyl, 4-cyanophenylsulfonyl, 2-nitrophenylsulfonyl, and 4-trifluoromethylphenylsulfonyl.

Examples of the heterocyclylsulfonyl and heterocyclylsulfonyl substituted by one or more substituents selected from Z² include (furan-2-yl)sulfonyl, (3-methylfuran-2-yl)sulfonyl, (furan-3-yl)sulfonyl, (thiophene-2-yl)sulfonyl, (thiophene-3-yl)sulfonyl, (pyridin-2-yl)sulfonyl, (pyridin-3-yl)sulfonyl, and (pyridin-4-yl)sulfonyl. Each is selected from a specified range of the number of carbon atoms.

Examples of the cycloalkylalkylsulfonyl include cyclopropylmethylsulfonyl, 2-cyclopropylethylsulfonyl, cyclopentylmethylsulfonyl, and cyclohexylmethylsulfonyl. Each is selected from a specified range of the number of carbon atoms.

Examples of the phenylalkylsulfonyl and phenylalkylsulfonyl substituted by one or more substituents selected from Z² include benzylsulfonyl, 2-fluorobenzylsulfonyl, 3-fluorobenzylsulfonyl, 4-fluorobenzylsulfonyl, 2-chlorobenzylsulfonyl, 3-bromobenzylsulfonyl, 4-iodobenzylsulfonyl, 2,4-difluorobenzylsulfonyl, 2-methylbenzylsulfonyl, 3-methylbenzylsulfonyl, 4-methylbenzylsulfonyl, 2-methoxybenzylsulfonyl, 4-aminobenzylsulfonyl, 4-trifluoromethoxybenzylsulfonyl, 2-methylthiobenzylsulfonyl, 3-trifluoromethylthiobenzylsulfonyl, 2-cyanobenzylsulfonyl, 4-nitrobenzylsulfonyl, 2-trifluoromethylbenzylsulfonyl, and 2-phenethylsulfonyl. Each is selected from a specified range of the number of carbon atoms.

Examples of the alkoxyalkylsulfonyl include methoxymethylsulfonyl, ethoxymethylsulfonyl, i-propyloxymethylsulfonyl, 3-(methoxy)butylsulfonyl, and 2-(methoxy)-i-butylsulfony. Each is selected from a specified range of the number of carbon atoms.

Examples of the monoalkylaminosulfonyl include methylaminosulfonyl, ethylaminosulfonyl, propylaminosulfonyl, i-propylaminosulfonyl, butylaminosulfonyl, s-butylaminosulfonyl, i-butylaminosulfonyl, t-butylaminosulfonyl, pentylaminosulfonyl, and hexylaminosulfonyl. Each is selected from a specified range of the number of carbon atoms.

Examples of the dialkylaminosulfonyl include dimethylaminosulfonyl, diethylaminosulfonyl, dipropylaminosulfonyl, di(i-propyl)aminosulfonyl, dibutylaminosulfonyl, di(s-butyl)aminosulfonyl, di(i-butyl)aminosulfonyl, di(t-butyl)aminosulfonyl, dipentylaminosulfonyl, dihexylaminosulfonyl, ethyl(methyl)aminosulfonyl, and methyl(propyl)aminosulfonyl. Each is selected from a specified range of the number of carbon atoms.

Examples of the alkylthio include methylthio, ethylthio, propylthio, i-propylthio, butylthio, i-butylthio, s-butylthio, and t-butylthio. Each is selected from a specified range of the number of carbon atoms.

Examples of the haloalkylthio include fluoromethylthio, chloromethylthio, chlorodifluoromethylthio, bromodifluoromethylthio, trifluoromethylthio, trichloromethylthio, 2,2,2-trifluoroethylthio, 1,1,2,2-tetrafluoroethylthio, 2-fluoroethylthio, and pentafluoroethylthio. Each is selected from a specified range of the number of carbon atoms.

Examples of the alkoxy include methoxy, ethoxy, propyloxy, i-propyloxy, butyloxy, i-butyloxy, s-butyloxy, t-butyloxy, pentyloxy, 1-methylbutyloxy, 2-methylbutyloxy, 3-methylbutyloxy, 1,1-dimethylpropyloxy, 1,2-dimethylpropyloxy, 2,2-dimethylpropyloxy, 1-ethylpropyloxy, hexyloxy, 1-methylpentyloxy, 2-methylpentyloxy, 3-methylpentyloxy, 4-methylpentyloxy, 1,1-dimethylbutyloxy, 1,2-dimethylbutyloxy, 1,3-dimethylbutyloxy, 2,2-dimethylbutyloxy, 2,3-dimethylbutyloxy, 3,3-dimethylbutyloxy, 1-ethylbutyloxy, 2-ethylbutyloxy, 1,1,2-trimethylpropyloxy, 1,2,2-trimethylpropyloxy, 1-ethyl-1-methylpropyloxy, and 1-ethyl-2-methylpropyloxy. Each is selected from a specified range of the number of carbon atoms.

Examples of the haloalkoxy include fluoromethoxy, difluoromethoxy, trifluoromethoxy, chlorodifluoromethoxy, bromodifluoromethoxy, dichlorofluoromethoxy, chloromethoxy, dichloromethoxy, trichloromethoxy, bromomethoxy, 1-fluoroethoxy, 2-fluoroethoxy, 2-chloroethoxy, 2-bromoethoxy, 2,2-difluoroethoxy, 2,2,2-trifluoroethoxy, 1,1,2,2-tetrafluoroethoxy, pentafluoroethoxy, 2,2,2-trichloroethoxy, 2,2,2-trifluoroethoxy, 1-fluoropropyloxy, 2-fluoropropyloxy, 3-fluoropropyloxy, 3-chloropropyloxy, 3-bromopropyloxy, 1-fluorobutyloxy, 2-fluorobutyloxy, 3-fluorobutyloxy, 4-fluorobutyloxy, and 4-chlorobutyloxy. Each is selected from a specified range of the number of carbon atoms.

The wording "R³ and R⁴ are optionally bonded together to form a 3- to 7-membered ring" means, for example, that it is possible to form cyclopropyl, cyclobutyl, cyclopentyl, cyclohexyl, cycloheptyl, epoxy, tetrahydrofuran, tetrahydropyran, tetrahydrothiophene, tetrahydrothiopyran, pyrrolidine, or piperidine, etc. that includes carbon attached to R³ and R⁴.

Examples of the hydroxyalkyl include hydroxymethyl, 1-hydroxyethyl, 2-hydroxyethyl, 1-hydroxypropyl, 2-hydroxypropyl, 3-hydroxypropyl, 1-hydroxy-1-methylethyl, 2-hydroxy-1-methylethyl, and 2-hydroxy-1,1-dimethylethyl. Each is selected from a specified range of the number of carbon atoms.

Examples of the monoalkylaminoalkyl include methylaminomethyl, ethylaminomethyl, propylaminomethyl, i-propylaminomethyl, butylaminomethyl, 2-(methylamino)ethyl, 2-(ethylamino)ethyl, and 3-(methylamino)propyl. Each is selected from a specified range of the number of carbon atoms.

Examples of the dialkylaminoalkyl include dimethylaminomethyl, diethylaminomethyl, ethyl(methyl)aminomethyl, methyl(propyl)aminomethyl, butyl(methyl)aminomethyl, 2-(diethylamino)ethyl, and 2-{ethyl(methyl)amino}propyl. Each is selected from a specified range of the number of carbon atoms.

Examples of the phenyl optionally substituted by one or more substituents selected from Z² include phenyl, 2-fluorophenyl, 3-fluorophenyl, 4-fluorophenyl, 2- chlorophenyl, 3-bromophenyl, 4-iodophenyl, 2,3-difluorophenyl, 2,4-difluorophenyl, 2,6-difluorophenyl, 3,4-difluorophenyl, 3,5-difluorophenyl, 2,4-dichlorophenyl, 2-fluoro-4-chlorophenyl, 2,3,4,5,6-pentafluorophenyl, 2-methylphenyl, 2,5 dimethylphenyl, 4-ethenylphenyl, 2-ethynylphenyl, 3-cyclopropylphenyl, 2-cyclopentylphenyl, 4-cyclohexylphenyl, 2-methoxyphenyl, 3,4-dimethoxyphenyl, 3,4,5-trimethoxyphenyl, 4-methoxymethylphenyl, 2-aminophenyl, 4-dimethylaminophenyl, 2-aminocarbonylphenyl, 2-dimethylaminocarbonylphenyl, 4-trifluoromethoxyphenyl, 2-difluoromethoxyphenyl, 2-ethenyloxyphenyl, 3-cyclopropyloxyphenyl, 2-biphenyl, 4-acetylphenyl, 3-methoxycarbonylphenyl, 2-methylthiophenyl, 3-trifluoromethylthiophenyl, 4-difluoromethylthiophenyl, 2-methylsulfinylphenyl, 3-trifluoromethylsulfinylphenyl, 4-difluoromethylsulfinylphenyl, 2-methylsulfonylphenyl, 3-trifluoromethylsulfonylphenyl, 4-difluoromethylsulfonylphenyl, 2-cyanophenyl, 3-nitrophenyl, 4-trifluoromethylphenyl, 2-difluoromethylphenyl, 2,3-methylenedioxyphenyl, and 3,4-methylenedioxyphenyl.

Examples of the heterocyclyl are shown below. The thienyl is thiophen-2-yl or thiophen-3-yl. The furyl is furan-2-yl or furan-3-yl. The pyrrolyl is pyrrol-1-yl, pyrrol-2-yl or pyrrol-3-yl. The oxazolyl is oxazol-2-yl, oxazol-4-yl, or oxazol-5-yl. The isoxazolyl is isoxazol-3-yl, isoxazol-4-yl, or isoxazol-5-yl. The isoxazolinyl is isoxazolin-3-yl, isoxazolin-4-yl, or isoxazolin-5-yl. The thiazolyl is thiazol-2-yl, thiazol-4-yl, or thiazol-5-yl. The isothiazolyl is isothiazol-3-yl, isothiazol-4-yl, or isothiazol-5-yl. The pyrazolyl is pyrazol-1-yl, pyrazol-3-yl, pyrazol-4-yl, or pyrazol 5-yl. The imidazolyl is imidazol-1-yl, imidazol-2-yl, or imidazol-4-yl. The 1,3,4-oxadiazolyl is 1,3,4-oxadiazol-2-yl. The 1,2,4-oxadiazolyl is 1,2,4-oxadiazol-3-yl or 1,2,4-oxadiazol-5-yl. The 1,3,4-thiadiazolyl is 1,3,4-thiadiazol-2-yl. The 1,2,4-thiadiazolyl is 1,2,4-thiadiazol-3-yl or 1,2,4-thiadiazol-5-yl. The 1,2,4-triazolyl is 1,2,4-triazol-1-yl, 1,2,4-triazol-3-yl, or 1,2,4-triazol-5-yl. The 1,2,3-thiadiazolyl is 1,2,3-thiadiazol-4-yl or 1,2,3-thiadiazol-5-yl. The 1,2,3-triazolyl is 1,2,3-triazol-1-yl, 1,2,3-triazol-2-yl, or 1,2,3-triazol-4-yl. The 1,2,3,4-tetrazolyl is 1,2,3,4-tetrazol-1-yl, 1,2,3,4-tetrazol-2-yl, or 1,2,3,4-tetrazol-5-yl. The pyridyl is pyridin-2-yl, pyridin-3-yl, or pyridin-4-yl. The pyrimidinyl is pyrimidin-2-yl, pyrimidin-4-yl, or pyrimidin-5-yl. The pyrazinyl is pyrazin-2-yl. The pyridazinyl is pyridazin-3-yl or pyridazin-4-yl. The 1,3,5-triazinyl is 1,3,5-triazin-2-yl. The 1,2,4-triazinyl is 1,2,4-triazin-3-yl, 1,2,4-triazin-5-yl, or 1,2,4-triazine-6-yl. The benzothienyl is benzothiophen-2-yl, benzothiophen-3-yl, benzothiophen-4-yl, benzothiophen-5-yl, benzothiophen-6-yl, or benzothiophen-7-yl. The benzofuryl is benzofuran-2-yl, benzofuran-3-yl, benzofuran-4-yl, benzofuran-5-yl, benzofuran-6-yl, or benzofuran-7-yl. The indole is indole-1-yl, indole-2-yl, indole-3-yl, indole-4-yl, indole-5-yl, indole-6-yl, or indole-7-yl. The benzothiazolyl is benzothiazol-2-yl, benzothiazol-4-yl, benzothiazol-5-yl, benzothiazol-6-yll, or benzothiazol-7-yl. The benzoimidazolyl is benzoimidazol-1-yl, benzoimidazol-2-yl, benzoimidazol-4-yl, benzoimidazol-5-yl, benzoimidazol-6-yl, or benzoimidazol-7-yl. The benzoisoxazolyl is benzoisoxazol-3-yl, benzoisoxazol-4-yl, benzoisoxazol-5-yl, benzoisoxazol-6-yl, or benzoisoxazol-7-yl. The benzoisothiazolyl is benzoisothiazol-3-yl, benzoisothiazol-4-yl, benzoisothiazol-5-yl, benzoisothiazol-6-yl, or benzoisothiazol-7-yl. The indazolyl is indazol-1-yl, indazol-3-yl, indazol-4-yl, indazol-5-yl, indazol-6-yl, or indazol-7-yl. The benzoxazolyl is benzoxazol-2-yl, benzoxazol-4-yl, benzoxazol-5-yl, benzoxazol-6-yl, or benzoxazol-7-yl. The quinolyl is quinolin-2-yl, quinolin-3-yl, quinolin-4-yl, quinolin-5-yl, quinolin-6-yl, quinolin-7-yl, or quinolin-8-yl. The isoquinolyl is isoquinolin-1-yl, isoquinolin-3-yl, isoquinolin-4-yl, isoquinolin-5-yl, isoquinolin-6-yl, isoquinolin-7-yl, or isoquinolin-8-yl. The quinoxalinyl is quinoxalin-2-yl, quinoxalin-3-yl, quinoxalin-5-yl, quinoxalin-6-yl, quinoxalin-7-yl, or quinoxalin-8-yl. The phthalazinyl is phthalazin-1-yl, phthalazin-4-yl, phthalazin-5-yl, phthalazin-6-yl, phthalazin-7-yl, or phthalazin-8-yl. The cinnorinyl is cinnolin-3-yl, cinnolin-4-yl, cinnolin-5-yl, cinnolin-6-yl, cinnolin-7-yl, or cinnolin-8-yl. The quinazolinyl is quinazolin-2-yl, quinazolin-4-yl, quinazolin-5-yl, quinazolin-6-yl, quinazolin-7-yl, or quinazolin-8-yl. The oxiranyl is oxiran-2-yl. The oxetanyl is oxetan-2-yl or oxetan-3-yl. The tetrahydrofuranyl is tetrahydrofuran-2-yl or tetrahydrofuran-3-yl. The tetrahydrothienyl is tetrahydrothiophen-2-yl or tetrahydrothiophen-3-yl. The 1,1-dioxytetrahydrothienyl is 1,1-dioxytetrahydrothiophen-2-yl or 1,1-dioxytetrahydrothiophen-3-yl. The tetrahydropyranyl is tetrahydropyran-2-yl, tetrahydropyran-3-yl, or tetrahydropyran-4-yl. The tetrahydrothiopyranyl is tetrahydrothiopyran-2-yl, tetrahydrothiopyran-3-yl, or tetrahydrothiopyran-4-yl. The 1,3-dioxanyl is 1,3-dioxan-2-yl, 1,3-dioxan-4-yl or 1,3-dioxan-5-yl. The 2-oxo-1,3-dioxolanyl is 2-oxo-1,3-dioxolan-4-yl. The 2-oxo-1,3-dioxolyl is 2-oxo-1,3-dioxol-4-yl. The 2,5-dioxopyrrolidinyl is 2,5-dioxopyrrolidin-1-yl.

Examples of the phenylaminocarbonyl substituted by one or more substituents selected from Z² include 2-fluorophenylaminocarbonyl, 2-chlorophenylaminocarbonyl, 4-bromophenylaminocarbonyl, 4-methylphenylaminocarbonyl, 2-methoxyphenylaminocarbonyl, 3-aminophenylaminocarbonyl, 4-trifluoromethoxyphenylaminocarbonyl, 2-methylthiophenylaminocarbonyl, 3-trifluoromethylthiophenylaminocarbonyl, 4-cyanophenylaminocarbonyl, 2-nitrophenylaminocarbonyl, and 4-trifluoromethylphenylaminocarbonyl.

Examples of the phenylalkylaminocarbonyl and phenylalkylaminocarbonyl substituted by one or more substituents selected from Z² include benzylaminocarbonyl, 2-fluorobenzylaminocarbonyl, 2-chlorobenzylaminocarbonyl, 3-bromobenzylaminocarbonyl, 4-iodobenzylaminocarbonyl, 4-methylbenzylaminocarbonyl, 2-methoxybenzylaminocarbonyl, 4-aminobenzylaminocarbonyl, 4-trifluoromethoxybenzylaminocarbonyl, 2-methylthiobenzylaminocarbonyl, 3-trifluoromethylthiobenzylaminocarbonyl, 2-cyanobenzylaminocarbonyl, 4-nitrobenzylaminocarbonyl, 2-trifluoromethylbenzylaminocarbonyl, and 2-phenethylaminocarbonyl.

Examples of the phenoxy substituted by one or more substituents selected from Z² include 2-fluorophenoxy, 2-chlorophenoxy, 4-bromophenoxy, 4-methylphenoxy, 2-methoxyphenoxy, 3-aminophenoxy, 4-trifluoromethoxyphenoxy, 2-methylthiophenoxy, 3-trifluoromethylthiophenoxy, 4-cyanophenoxy, 2-nitrophenoxy, and 4-trifluoromethylphenoxy.

Examples of the phenylthio substituted by one or more substituents selected from Z² include 2-fluorophenylthio, 2-chlorophenylthio, 4-bromophenylthio, 4-methylphenylthio, 2-methoxyphenylthio, 3-aminophenylthio, 4-trifluoromethoxyphenylthio, 2-methylthiophenylthio, 3-trifluoromethylthiophenylthio, 4-cyanophenylthio, 2-nitrophenylthio, and 4-trifluoromethylphenylthio.

Examples of the monoalkylamino include methylamino, ethylamino, propylamino, i-propylamino, butylamino, s-butylamino, i-butylamino, t-butylamino, pentylamino, and hexylamino. Each is selected from a specified range of the number of carbon atoms.

Examples of the di(alkyl)amino include dimethylamino, diethylamino, dipropylamino, di(i-propyl)amino, dibutylamino, di(s-butyl)amino, di(i-butyl)amino, di(t-butyl)amino, dipentylamino, dihexylamino, ethyl(methyl)amino, and methyl(propyl)amino. Each is selected from a specified range of the number of carbon atoms.

Examples of the alkylcarbonyloxy include acetoxy, propionyloxy, butyryloxy, i-butyryloxy, valeroyloxy, i-valeroyloxy, 2-methylbutyryloxy, pivaloyloxy, and heptanoyloxy. Each is selected from a specified range of the number of carbon atoms.

The wording "R⁵ and R⁶ on the same carbon are optionally bonded together to form a 3- to 7-membered ring optionally interrupted by one or two heteroatoms selected from the group consisting of an oxygen atom, a sulfur atom, and a nitrogen atom where the nitrogen atom is optionally substituted by C₁-C₆ alkyl, C₂-C₆ alkenyl, C₂-C₆ alkynyl, or C₃-C₆ cycloalkyl or optionally substituted, or to form an olefin" means, for example, that it is possible to form cyclopropyl, dichlorocyclopropyl, dimethylcyclopropyl, cyclobutyl, cyclopentyl, cyclohexyl, cycloheptyl, epoxy, tetrahydrofuran, tetrahydropyran, tetrahydrothiophene, tetrahydrothiopyran, pyrrolidine, or piperidine, etc., that includes carbon attached to R⁵ and R⁶ on the same carbon of R⁵ and R⁶.

Two R⁵ on adjacent carbon atoms are optionally bonded together with a carbon atom to which they are attached to form a 3- to 8-membered carbocycle or heterocycle. Here, the heterocycle contains one or two heteroatoms selected from an oxygen atom, a sulfur atom, or a nitrogen atom. The nitrogen atom is optionally substituted by C₁-C₆ alkyl, C₂-C₆ alkenyl, C₂-C₆ alkynyl, or C₃-C₆ cycloalkyl. The 3- to 8-membered carbocycle or heterocycle is optionally substituted. Examples of the carbocycle herein include a benzene ring, a cyclopropane ring, a cyclobutane ring, a cyclopentane ring, a cyclohexane ring, a cycloheptane ring, and a cyclooctane ring. Examples of the heterocycle include furan, tetrahydrofuran, thiophene, tetrahydrothiophene, pyrrole, pyrrolidine, pyridine, piperidine, and pyrimidine.

The wording "R⁵ and R⁶ are optionally bonded together with R⁵ or R⁶ on adjacent carbon to form a bond" means, for example, that a double bond can be formed between carbon attached to R⁵ and R⁶ and adjacent carbon attached to R⁵ and R⁶.

Examples of the alkenyloxy include ethenyloxy, 1-propenyloxy, 2-propenyloxy, 1-butenyloxy, 1-methyl-2-propenyloxy, 2-methyl-2-propenyloxy, and 1-hexenyloxy. Each is selected from a specified range of the number of carbon atoms.

Examples of the alkynyloxy include ethynyloxy, 1-propynyloxy, 2-propynyloxy, 1-butynyloxy, 1-methyl-2-propynyloxy, 2-methyl-2-propynyloxy, and 1-hexynyloxy. Each is selected from a specified range of the number of carbon atoms.

Examples of the cycloalkylalkyloxy include cyclopropylmethoxy, 2-cyclopropylethoxy, cyclopentylmethoxy, and cyclohexylmethoxy. Each is selected from a specified range of the number of carbon atoms.

Examples of the phenylalkyloxy include benzyloxy, 2-fluorobenzyloxy, 3-fluorobenzyloxy, 4-fluorobenzyloxy, 2-chlorobenzyloxy, 3-bromobenzyloxy, 4-iodobenzyloxy, 2,4-difluorobenzyloxy, 2-methylbenzyloxy, 3-methylbenzyloxy, 4-methylbenzyloxy, 2-methoxybenzyloxy, 4-aminobenzyloxy, 4-trifluoromethoxybenzyloxy, 2-methylthiobenzyloxy, 3-trifluoromethylthiobenzyloxy, 2-cyanobenzyloxy, 4-nitrobenzyloxy, 2-trifluoromethylbenzyloxy, and 2-phenethyloxy. Each is selected from a specified range of the number of carbon atoms.

Examples of the phenylcarbonyloxy substituted by one or more substituents selected from Z² include phenylcarbonyloxy, 2-fluorophenylcarbonyloxy, 2-chlorophenylcarbonyloxy, 3-bromophenylcarbonyloxy, 4-iodophenylcarbonyloxy, 4-methylphenylcarbonyloxy, 2-methoxyphenylcarbonyloxy, 4-aminophenylcarbonyloxy, 4-trifluoromethoxyphenylcarbonyloxy, 2-methylthiophenylcarbonyloxy, 3-trifluoromethylthiophenylcarbonyloxy, 2-cyanophenylcarbonyloxy, 4-nitrophenylcarbonyloxy, and 2-trifluoromethylphenylcarbonyloxy. Each is selected from a specified range of the number of carbon atoms.

Examples of the heterocyclylcarbonyloxy substituted by one or more substituents selected from Z² include (tetrahydrofuran-2-yl)carbonyloxy, (tetrahydrofuran-3-yl)carbonyloxy, (tetrahydrothiophen-2-yl)carbonyloxy, (pyrrolidin-1-yl)carbonyloxy, (tetrahydropyran-4-yl)carbonyloxy, (furan-2-yl)carbonyloxy, (3-methylfuran-2-yl)carbonyloxy, (furan-3-yl)carbonyloxy, (thiophene-2-yl)carbonyloxy, (thiophene-3-yl)carbonyloxy, (1H-imidazol-1-yl)carbonyloxy, (pyridin-2-yl)carbonyloxy, (pyridin-3-yl)carbonyloxy, and (pyridin-4-yl)carbonyloxy. Each is selected from a specified range of the number of carbon atoms.

Examples of the alkoxycarbonyloxy include methoxycarbonyloxy, ethoxycarbonyloxy, propyloxycarbonyloxy, i-propyloxycarbonyloxy, and butyloxycarbonyloxy. Each is selected from a specified range of the number of carbon atoms.

Examples of the alkylthiocarbonyloxy include methylthiocarbonyloxy, ethylthiocarbonyloxy, propylthiocarbonyloxy, i-propylthiocarbonyloxy, butylthiocarbonyloxy, i-butylthiocarbonyloxy, s-butylthiocarbonyloxy, and t-butylthiocarbonyloxy. Each is selected from a specified range of the number of carbon atoms.

Examples of the alkylsulfonyloxy include methylsulfonyloxy, ethylsulfonyloxy, propylsulfonyloxy, i-propylsulfonyloxy, butylsulfonyloxy, i-butylsulfonyloxy, s-butylsulfonyloxy, and t-butylsulfonyloxy. Each is selected from a specified range of the number of carbon atoms.

Examples of the haloalkylsulfonyloxy include fluoromethylsulfonyloxy, chloromethylsulfonyloxy, chlorodifluoromethylsulfonyloxy, bromodifluoromethylsulfonyloxy, trifluoromethylsulfonyloxy, trichloromethylsulfonyloxy, 2,2,2-trifluoroethylsulfonyloxy, 1,1,2,2-tetrafluoroethylsulfonyloxy, 2-fluoroethylsulfonyloxy, and pentafluoroethylsulfonyloxy. Each is selected from a specified range of the number of carbon atoms.

Examples of the dialkylphosphorooxy include dimethylphosphorooxy, dimethylphosphorooxy, and dipropylphosphorooxy. Each is selected from a specified range of the number of carbon atoms.

Examples of the trialkylsilyloxy include trimethylsilyloxy, triethylsilyloxy, and t-butyldimethylsilyloxy. Each is selected from a specified range of the number of carbon atoms.

Examples of the haloalkylsulfinyl include fluoromethylsulfinyl, chloromethylsulfinyl, chlorodifluoromethylsulfinyl, bromodifluoromethylsulfinyl, trifluoromethylsulfinyl, trichloromethylsulfinyl, 2,2,2-trifluoroethylsulfinyl, 1,1,2,2-tetrafluoroethylsulfinyl, 2-fluoroethylsulfinyl, and pentafluoroethylsulfinyl. Each is selected from a specified range of the number of carbon atoms.

Examples of the phenylsulfinyl substituted by one or more substituents selected from Z² include 2-fluorophenylsulfinyl, 2-chlorophenylsulfinyl, 4-bromophenylsulfinyl, 4-methylphenylsulfinyl, 2-methoxyphenylsulfinyl, 3-aminophenylsulfinyl, 4-trifluoromethoxyphenylsulfinyl, 2-methylthiophenylsulfinyl, 3-trifluoromethylthiophenylsulfinyl, 4-cyanophenylsulfinyl, 2-nitrophenylsulfinyl, and 4-trifluoromethylphenylsulfinyl.

Examples of the dialkylamino include dimethylamino, diethylamino, ethyl(methyl)amino, methyl(propyl)amino, and butyl(methyl)amino. Each is selected from a specified range of the number of carbon atoms.

Examples of the alkylcarbonylamino include acetylamino, propionylamino, butyrylamino, i-butyrylamino, valeroylamino, i-valeroylamino, 2-methylbutyrylamino, pivaloylamino, hexanoylamino, 2-ethylbutyrylamino, 2-methylvaleroylamino, and 4-methylvaleroylamino. Each is selected from a specified range of the number of carbon atoms.

Examples of the bis(alkylcarbonyl)amino include bisacetylamino, N-acetyl-N-propionylamino, and N-acetyl-N-pivaloylamino. Each is selected from a specified range of the number of carbon atoms.

Examples of the haloalkylsulfonylamino include fluoromethylsulfonylamino, chloromethylsulfonylamino, chlorodifluoromethylsulfonylamino, bromodifluoromethylsulfonylamino, trifluoromethylsulfonylamino, trichloromethylsulfonylamino, 2,2,2-trifluoroethylsulfonylamino, 1,1,2,2-tetrafluoroethylsulfonylamino, 2-fluoroethylsulfonylamino, and pentafluoroethylsulfonylamino. Each is selected from a specified range of the number of carbon atoms.

The wording "two X attached to an adjacent carbon or nitrogen atom on a benzene or heterocyclic ring are bonded together with a ring atom to which they are attached to form a 4- to 6-membered carbocycle or heterocycle where the heterocycle contains one or two heteroatoms selected from the group consisting of an oxygen atom, a sulfur atom, and a nitrogen atom, provided that the nitrogen atom is optionally substituted by C₁-C₆ alkyl, C₂-C₆ alkenyl, C₂-C₆ alkynyl, or C₃-C₆ cycloalkyl" means, for example, that it is possible to form indene, dihydroindene, naphthalene, dihydronaphthalene, tetrahydronaphthalene, benzofuran, dihydrobenzofuran, chromene, chromane, benzothiophene, dihydrobenzothiophene, benzoxazole, benzothiazole, thiochromene, thiochromane, indole, indoline, quinoline, dihydroquinoline, tetrahydroquinoline, cyclopentapyridine, pyrrolopyridine, naphthyridine, pyrazolopyridine, or triazolopyrimidine, etc., that includes a benzene or heterocyclic ring attached to X.

The wording "two Y are optionally bonded together with an adjacent carbon or nitrogen atom on a benzene ring to form a 5- or 6-membered carbocycle or a 5- or 6-membered heterocycle, provided that the heterocycle contains one or two heteroatoms selected from the group consisting of an oxygen atom, a sulfur atom, and a nitrogen atom where the nitrogen atom is optionally substituted by C₁-C₆ alkyl, C₂-C₆ alkenyl, C₂-C₆ alkynyl, or C₃-C₆ cycloalkyl, and the carbocycle or heterocycle is optionally substituted by one or more halogen atoms" means, for example, that it is possible to form indene, dihydroindene, naphthalene, dihydronaphthalene, tetrahydronaphthalene, benzofuran, dihydrobenzofuran, chromene, chromane, benzothiophene, dihydrobenzothiophene, benzoxazole, benzothiazole, thiochromene, thiochromane, indole, indoline, quinoline, dihydroquinoline, tetrahydroquinoline, cyclopentapyridine, pyrrolopyridine, naphthyridine, pyrazolopyridine, triazolopyrimidine, etc., that includes a benzene or heterocyclic ring attached to Y.

The wording "two Z² are optionally bonded together with an adjacent carbon or nitrogen atom on a benzene or heterocyclic ring to form a 5- or 6-membered carbocycle or a 5- or 6-membered heterocycle, provided that the heterocycle contains one or two heteroatoms selected from the group consisting of an oxygen atom, a sulfur atom, and a nitrogen atom where the nitrogen atom is optionally substituted by C₁-C₆ alkyl, C₂-C₆ alkenyl, C₂-C₆ alkynyl, or C₃-C₆ cycloalkyl, and the carbocycle or heterocycle is optionally substituted by one or more halogen atoms" means, for example, that it is possible to form indene, dihydroindene, naphthalene, dihydronaphthalene, tetrahydronaphthalene, benzofuran, dihydrobenzofuran, benzoxazole, benzothiazole, chromene, chromane, benzothiophene, dihydrobenzothiophene, thiochromene, thiochromane, indole, indoline, quinoline, dihydroquinoline, tetrahydroquinoline, cyclopentapyridine, pyrrolopyridine, naphthyridine, pyrazolopyridine, or triazolopyrimidine, etc., that includes a benzene or heterocyclic ring attached to Z².

The following notations in the tables herein represent the respective corresponding groups as follows.

For example,
Me represents a methyl group,
Et represents an ethyl group,
n-Pr represents a normal propyl group,
i-Pr represents an isopropyl group,
c-Pr represents a cyclopropyl group,
n-Bu represents a normal butyl group,
i-Bu represents an isobutyl group,
s-Bu represents a secondary butyl group,
c-Bu represents a cyclobutyl group,
t-Bu represents a tertiary butyl group,
n-Pen represents a normal pentyl group,
c-Pen represents a cyclopentyl group,
n-Hex represents a normal hexyl group,
c-Hex represents a cyclohexyl group,
n-Hept represents a normal heptyl group,
allyl represents a 2-propen-1-yl group,
Ph represents a phenyl group,
2-Py represents a pyridin-2-yl group,
Prorargyl represents a 2-propyn-1-yl group,
Bn represents a benzene methyl group,
CN represents a cyano group,
Ac represents an acetyl group,
Bz represents a benzene carbonyl group,
Boc represents a tertiary butoxycarbonyl group,
Ms represents a methanesulfonyl group,
Tf represents a trifluoromethanesulfonyl group,
TBDMS represents a tertiary-butyldimethylsilyl group,
TBDPS represents a tertiary-butyldiphenylsilyl group,
MOM represents a methoxymethyl group,
OMe represents a methoxy group, and
OPh represents a phenoxy group.

Typical methods for producing a compound represented by Formula [1] according to the present invention are exemplified below, but the present invention is not limited to these methods. Note that the reactor may include a magnetic stirrer or a mechanical stirrer, and a microwave synthesizer can also be used for the reaction.

### <Production Method 1>

The compound represented by Formula [1] according to the present invention can be produced by a method comprising the reaction scheme illustrated below: 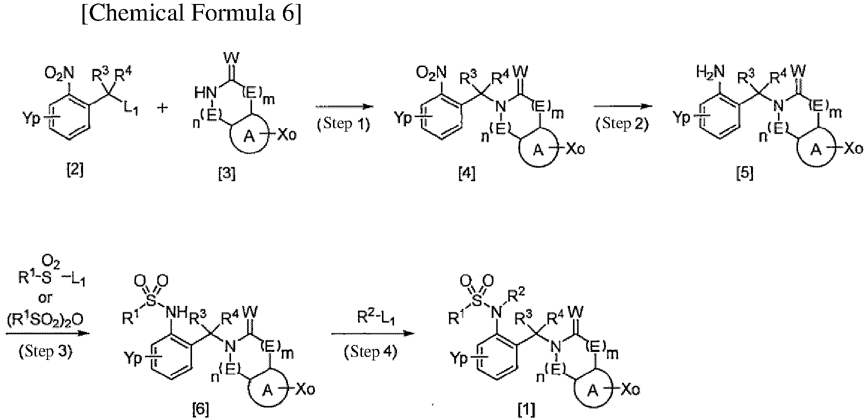 wherein R¹, R², R³, R⁴, A, E, W, X, Y, m, n, o, and p are as defined in Formula [1] of the above item (1) and L₁ represents a leaving group such as a halogen.

### (Step 1)

A compound represented by Formula [2] and a compound represented by Formula [3] may be reacted in a solvent in the presence of a base to produce a compound represented by Formula [4].

The amount of the compound of Formula [3] used in this step may be selected from 0.5 to 10 moles per mole of the compound of Formula [2], and is preferably from 1.0 to 1.2 moles.

Examples of the base that can be used in this step include: organic amines such as triethylamine, pyridine, 4-dimethylaminopyridine, N,N-dimethylaniline, and 1,8-diazabicyclo[5,4,0]-7-undecene; metal carbonates such as sodium carbonate, potassium carbonate, magnesium carbonate, and calcium carbonate; metal bicarbonates such as sodium bicarbonate, and potassium bicarbonate; carboxylic acid metal salts represented by metal acetates such as sodium acetate, potassium acetate, calcium acetate, and magnesium acetate; metal alkoxides such as sodium methoxide, sodium ethoxide, sodium t-butoxide, potassium methoxide, and potassium t-butoxide; metal hydroxides such as sodium hydroxide, potassium hydroxide, calcium hydroxide, and magnesium hydroxide; metal hydrides such as lithium hydride, sodium hydride, and calcium hydride; or a mixture of two or more of these.

The amount of the base used may be selected from 0.5 to 10 moles per mole of the compound of Formula [2], and is preferably from 1.0 to 1.2 moles.

The solvent that can be used in this step may be any solvent that does not inhibit the progress of the reaction. Examples of the solvent that can be used include: nitriles such as acetonitrile; ethers such as diethyl ether, diisopropyl ether, tetrahydrofuran, dioxane, monoglyme, and diglyme; halogenated hydrocarbons such as dichloromethane, dichloroethane, chloroform, carbon tetrachloride, and tetrachloroethane; aromatic hydrocarbons such as benzene, chlorobenzene, nitrobenzene, and toluene; amides such as N,N-dimethylformamide and N,N-dimethylacetamide; imidazolinones such as 1,3-dimethyl-2-imidazolinone; and sulfur compounds such as dimethyl sulfoxide. Further, a mixed solvent containing two or more of these solvents can also be used.

The volume of the solvent used may be selected from 0.01 to 100 L per mole of the compound of Formula [2], and is preferably from 0.1 to 10 L.

The reaction temperature may be selected from -20°C to a point (in the boiling range of inert solvent used), and preferably ranges from 0°C to 100°C.

In addition, the reaction can also be carried out using a phase-transfer catalyst such as a quaternary ammonium salt. Examples of the phase-transfer catalyst include quaternary ammonium salts such as tetra-n-butylammonium bromide and benzyltriethylammonium bromide, 18-crown 6-ether, or a combination of two or more of these. In the case of using a phase-transfer catalyst, the amount used may be selected from 0.0001 to 1.0 moles per mole of the compound of Formula [2], and is preferably from 0.001 to 0.1 moles.

The reaction time may vary depending on, for instance, the reaction temperature, the reaction substrate, and the reaction volume, but usually ranges from 10 minutes to 48 hours.

The compound of Formula [4], a target substance of the reaction, is collected from the reaction system after the completion of the reaction according to the standard method, and may be purified by a procedure such as column chromatography and/or recrystallization if necessary.

### (Step 2)

The compound represented by Formula [4] may be reduced in a solvent to produce a compound represented by Formula [5].

Examples of the reduction process that can be used in this step include a process using a reducing agent such as zinc powder, reduced iron, tin powder, tin chloride, or titanium chloride; a process using a hydrogen donor such as hydrazine in the presence of Raney nickel; and a catalytic hydrogen reduction or catalytic hydrogen transfer reduction in the presence of a catalyst such as Raney nickel, palladium carbon, osmium carbon, palladium hydroxide, or platinum oxide.

In the case of using a reducing agent in this step, the amount of the reducing agent used may be selected from 0.05 to 10 moles per mole of the compound of Formula [4], and is preferably from 0.1 to 1.2 moles. The preferable reducing agent used may be zinc powder or reduced iron.

In the case of using a hydrogen donor in this step, the amount of the hydrogen donor used may be selected from 0.05 to 10 moles per mole of the compound of Formula [4], and is preferably from 0.1 to 1.2 moles. The preferable hydrogen donor used may be hydrazine.

In the case of using a catalyst in this step, the amount of the catalyst used may be selected from 0.05 to 10 moles per mole of the compound of Formula [4], and is preferably from 0.1 to 1.2 moles. The preferable catalyst used may be Raney nickel or palladium carbon.

Examples of the solvent that can be used in this step include the same as those described in step 1.

In addition, the volume of the solvent used may be selected from 0.01 to 100 L per mole of the compound of Formula [4], and is preferably from 0.1 to 10 L.

The reaction temperature may be selected from 0°C to a point (in the boiling range of inert solvent used), and preferably ranges from 0°C to 200°C.

The reaction time may vary depending on, for instance, the reaction temperature, the reaction substrate, and the reaction volume, but usually ranges from 10 minutes to 48 hours.

The compound of Formula [5], a target substance of the reaction, is collected from the reaction system after the completion of the reaction according to the standard method, and may be purified by a procedure such as column chromatography and/or recrystallization if necessary.

### (Step 3)

The compound represented by Formula [5] and a haloalkylsulfonyl derivative represented by general formula: R¹SO₂L₁ or (R¹SO₂)₂O may be reacted in a solvent in the presence or absence of a base to produce a compound represented by Formula [6].

The amount of the haloalkylsulfonyl derivative represented by general formula: R¹SO₂L₁ or (R¹SO₂)₂O as used in this step may be selected from 0.5 to 10 moles per mole of the compound of Formula [5], and is preferably from 1.0 to 1.2 moles.

Examples of the solvent and the base that can be used in this step include the same compounds described in step 1.

The amount of the base used in this reaction may be selected from 0 to 100 moles per mole of the compound of Formula [5], and is preferably from 0 to 10 moles.

In addition, the volume of the solvent used may be selected from 0.01 to 100 L per mole of the compound of Formula [5], and is preferably from 0.1 to 10 L.

The reaction temperature may be selected from -78°C to a point (in the boiling range of inert solvent used), and preferably ranges from 0°C to 100°C.

The reaction time may vary depending on, for instance, the reaction temperature, the reaction substrate, and the reaction volume, but usually ranges from 10 minutes to 48 hours.

The compound of Formula [6], a target substance of the reaction, is collected from the reaction system after the completion of the reaction according to the standard method, and may be purified by a procedure such as column chromatography and/or recrystallization if necessary.

### (Step 4)

The compound represented by Formula [6] and a compound represented by general formula: R²L₁ may be reacted in a solvent in the presence of a base to produce a compound represented by Formula [1].

The amount of the compound of general formula R²L₁ used in this step may be selected from 0.5 to 10 moles per mole of the compound of Formula [6], and is preferably from 1.0 to 1.2 moles.

Examples of the solvent and the base that can be used in this step include the same compounds described in step 1.

The amount of the base used in this reaction may be selected from 0 to 100 moles per mole of the compound of Formula [6], and is preferably from 0.1 to 10 moles.

In addition, the volume of the solvent used may be selected from 0.01 to 100 L per mole of the compound of Formula [6], and is preferably from 0.1 to 10 L.

The reaction temperature may be selected from -20°C to a point (in the boiling range of inert solvent used), and preferably ranges from 0°C to 100°C.

The reaction time may vary depending on, for instance, the reaction temperature, the reaction substrate, and the reaction volume, but usually ranges from 10 minutes to 48 hours.

In addition, the reaction can also be carried out using a phase-transfer catalyst such as a quaternary ammonium salt. Examples of the phase-transfer catalyst include the same catalysts described in step 1. In the case of using a phase-transfer catalyst, the amount used may be selected from 0.0001 to 1.0 moles per mole of the compound of Formula [6], and is preferably from 0.001 to 0.1 moles.

The compound of Formula [1], a target substance of the reaction, is collected from the reaction system after the completion of the reaction according to the standard method, and may be purified by a procedure such as column chromatography and/or recrystallization if necessary.

### <Production Method 2>

The compound represented by Formula [1] according to the present invention can also be produced by a method comprising the reaction scheme illustrated below: 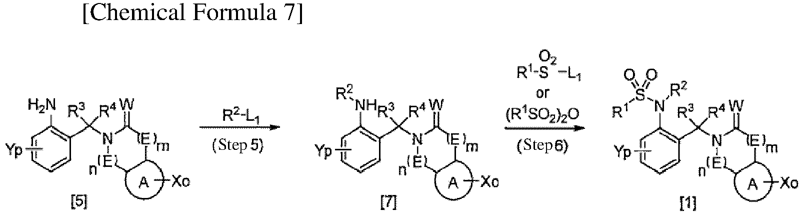 wherein R¹, R², R³, R⁴, A, E, W, X, Y, m, n, o, and p are as defined in Formula [1] of the above item (1) and L₁ has the same meaning as above.

### (Step 5)

The compound represented by Formula [5] and a compound represented by general formula: R²L₁ may be reacted in a solvent in the presence or absence of a base to produce a compound represented by Formula [7].

The amount of the compound of general formula R²L₁ used in this step may be selected from 0.5 to 10 moles per mole of the compound of Formula [5], and is preferably from 1.0 to 1.2 moles.

Examples of the solvent and the base that can be used in this step include the same compounds described in step 1.

The amount of the base used in this reaction may be selected from 0 to 100 moles per mole of the compound of Formula [5], and is preferably from 0 to 10 moles.

In addition, the volume of the solvent used may be selected from 0.01 to 100 L per mole of the compound of Formula [5], and is preferably from 0.1 to 10 L.

The reaction temperature may be selected from -78°C to a point (in the boiling range of inert solvent used), and preferably ranges from 0°C to 100°C.

The reaction time may vary depending on, for instance, the reaction temperature, the reaction substrate, and the reaction volume, but usually ranges from 10 minutes to 48 hours.

In addition, the reaction can also be carried out using a phase-transfer catalyst such as a quaternary ammonium salt. Examples of the phase-transfer catalyst include quaternary ammonium salts such as tetra-n-butylammonium bromide and benzyltriethylammonium bromide, and 18-crown 6-ether, or a combination of two or more of these. In the case of using a phase-transfer catalyst, the amount used may be selected from 0.0001 to 1.0 moles per mole of the compound of Formula [5], and is preferably from 0.001 to 0.1 moles.

The compound of Formula [7], a target substance of the reaction, is collected from the reaction system after the completion of the reaction according to the standard method, and may be purified by a procedure such as column chromatography and/or recrystallization if necessary.

### (Step 6)

The compound represented by Formula [7] and a haloalkylsulfonyl derivative represented by general formula: R¹SO₂L₁ or (R¹SO₂)₂O may be reacted in a solvent in the presence of a base to produce a compound represented by Formula [1].

The amount of the haloalkylsulfonyl derivative represented by R¹SO₂L₁ or (R¹SO₂)₂O as used in this step may be selected from 0.5 to 10 moles per mole of the compound of Formula [7], and is preferably from 1.0 to 1.2 moles.

Examples of the solvent and the base that can be used in this step include the same compounds described in step 1.

The amount of the base used in this reaction may be selected from 0.01 to 100 moles per mole of the compound of Formula [7], and is preferably from 0.1 to 10 moles.

In addition, the volume of the solvent used may be selected from 0.01 to 100 L per mole of the compound of Formula [7], and is preferably from 0.1 to 10 L.

The reaction temperature may be selected from -78°C to a point (in the boiling range of inert solvent used), and preferably ranges from 0°C to 100°C.

The reaction time may vary depending on, for instance, the reaction temperature, the reaction substrate, and the reaction volume, but usually ranges from 10 minutes to 48 hours.

The compound of Formula [1], a target substance of the reaction, is collected from the reaction system after the completion of the reaction according to the standard method, and may be purified by a procedure such as column chromatography and/or recrystallization if necessary.

### <Production Method 3>

The compound represented by Formula [6] according to the present invention can also be produced by a method comprising the reaction scheme illustrated below: 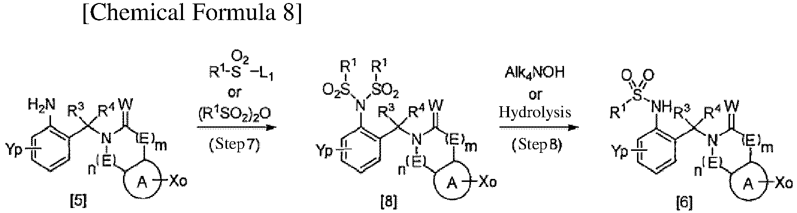 wherein R¹, R², R³, R⁴, A, E, W, X, Y, m, n, o, and p are as defined in Formula [1] of the above item (1), L₁ has the same meaning as above, and Alk each independently represents alkyl such as C₁-C₆ alkyl.

### (Step 7)

The compound represented by Formula [5] and a haloalkylsulfonyl derivative represented by general formula: R¹SO₂L₁ or (R¹SO₂)₂O may be reacted in a solvent in the presence or absence of a base to produce a compound represented by Formula [8].

The amount of the haloalkylsulfonyl derivative represented by general formula: R¹SO₂L₁ or (R¹SO₂)₂O as used in this step may be selected from 0.5 to 10 moles per mole of the compound of Formula [5], and is preferably from 1.0 to 1.2 moles.

Examples of the solvent and the base that can be used in this step include the same compounds described in step 1.

The amount of the base used in this reaction may be selected from 0 to 100 moles per mole of the compound of Formula [5], and is preferably from 0 to 10 moles.

In addition, the volume of the solvent used may be selected from 0.01 to 100 L per mole of the compound of Formula [5], and is preferably from 0.1 to 10 L.

The reaction temperature may be selected from -78°C to a point (in the boiling range of inert solvent used), and preferably ranges from 0°C to 100°C.

The reaction time may vary depending on, for instance, the reaction temperature, the reaction substrate, and the reaction volume, but usually ranges from 10 minutes to 48 hours.

The compound of Formula [8], a target substance of the reaction, is collected from the reaction system after the completion of the reaction according to the standard method, and may be purified by a procedure such as column chromatography and/or recrystallization if necessary.

### (Step 8)

The compound represented by Formula [8] may be reacted with a compound represented by general formula: Alk₄NOH in a solvent or hydrolyzed using a base to produce a compound represented by Formula [6].

Examples of the solvent and the base that can be used in this step include the same compounds described in step 1.

Examples of the compound represented by general formula: Alk₄NOH that can be used in this step include tetramethylammonium hydroxide and tetrabutylammonium hydroxide.

The amount of the compound of general formula Alk₄NOH used in this reaction may be selected from 0 to 10 moles per mole of the compound of Formula [8], and is preferably from 0 to 2 moles and more preferably from 0.1 to 2 moles.

The amount of the base used in this reaction may be selected from 0 to 10 moles per mole of the compound of Formula [8], and is preferably from 0 to 2 moles and more preferably from 0.1 to 2 moles.

In addition, the volume of the solvent used may be selected from 0.01 to 100 L per mole of the compound of Formula [8], and is preferably from 0.1 to 10 L.

The reaction temperature may be selected from -20°C to a point (in the boiling range of inert solvent used), and preferably ranges from 0°C to 100°C.

The reaction time may vary depending on, for instance, the reaction temperature, the reaction substrate, and the reaction volume, but usually ranges from 10 minutes to 48 hours.

The compound of Formula [6], a target substance of the reaction, is collected from the reaction system after the completion of the reaction according to the standard method, and may be purified by a procedure such as column chromatography and/or recrystallization if necessary.

### <Production Method 4>

The compound represented by Formula [6] according to the present invention can also be produced by a method comprising the reaction scheme illustrated below: 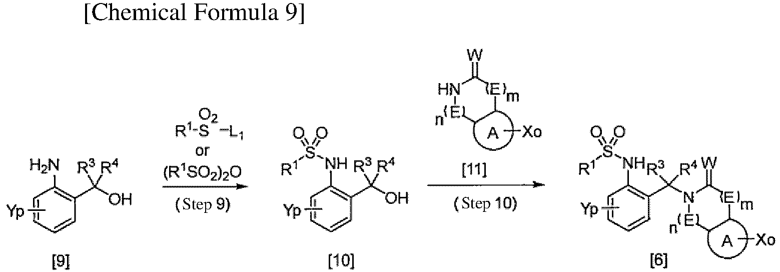 wherein R¹, R³, R⁴, A, E, W, X, Y, m, n, o, and p are as defined in Formula [1] of the above item (1) and L₁ has the same meaning as above.

### (Step 9)

The compound represented by Formula [9] and a haloalkylsulfonyl derivative represented by general formula: R¹SO₂L₁ or (R¹SO₂)₂O may be reacted in a solvent in the presence or absence of a base to produce a compound represented by Formula [10].

The amount of the haloalkylsulfonyl derivative represented by general formula: R¹SO₂L₁ or (R¹SO₂)₂O as used in this step may be selected from 0.5 to 10 moles per mole of the compound of Formula [9], and is preferably from 1.0 to 1.2 moles.

Examples of the solvent and the base that can be used in this step include the same compounds described in step 1.

The amount of the base used in this reaction may be selected from 0 to 100 moles per mole of the compound of Formula [9], and is preferably from 0 to 10 moles.

In addition, the volume of the solvent used may be selected from 0.01 to 100 L per mole of the compound of Formula [9], and is preferably from 0.1 to 10 L.

The reaction temperature may be selected from -78°C to a point (in the boiling range of inert solvent used), and preferably ranges from 0°C to 100°C.

The reaction time may vary depending on, for instance, the reaction temperature, the reaction substrate, and the reaction volume, but usually ranges from 10 minutes to 48 hours.

The compound of Formula [10], a target substance of the reaction, is collected from the reaction system after the completion of the reaction according to the standard method, and may be purified by a procedure such as column chromatography and/or recrystallization if necessary.

### (Step 10)

The compound represented by Formula [10] and a compound represented by Formula [11] may be reacted in a solvent in the presence of an acid to produce a compound represented by Formula [6].

The amount of the compound of Formula [11] used in this step may be selected from 0.5 to 10 moles per mole of the compound of Formula [10], and is preferably from 1.0 to 1.2 moles.

Examples of the acid that can be used in this step include organic acids such as formic acid, acetic acid, methanesulfonic acid, p-toluenesulfonic acid, and trifluoroacetic acid, trifluoromethanesulfonic acid; inorganic acids such as hydrochloric acid, sulfuric acid, and hydrogen bromide; and Lewis acids such as zinc chloride, titanium tetrachloride, tin chloride, aluminum chloride, iron chloride, and boron trifluoride-ether complex; or a mixture of two or more of these.

In addition, the reaction can also be carried out using a phase-transfer catalyst such as a quaternary ammonium salt. Examples of the phase-transfer catalyst include the same compounds described in step 1. In the case of using a phase-transfer catalyst, the amount used may be selected from 0.0001 to 1.0 moles per mole of the compound of Formula [10], and is preferably from 0.001 to 0.1 moles.

Examples of the solvent that can be used in this step include the same compounds described in step 1.

The amount of the acid used in this reaction may be selected from 0.01 to 100 moles per mole of the compound of Formula [10], and is preferably from 0.1 to 10 moles.

In addition, the volume of the solvent used may be selected from 0.01 to 100 L per mole of the compound of Formula [10], and is preferably from 0.1 to 10 L.

The reaction temperature may be selected from 0°C to a point (in the boiling range of inert solvent used), and preferably ranges from 0°C to 100°C.

The reaction time may vary depending on, for instance, the reaction temperature, the reaction substrate, and the reaction volume, but usually ranges from 10 minutes to 72 hours.

The compound of Formula [6], a target substance of the reaction, is collected from the reaction system after the completion of the reaction according to the standard method, and may be purified by a procedure such as column chromatography and/or recrystallization if necessary.

### <Production Method 5>

The compound represented by Formula [12] according to the present invention can be produced by a method comprising the reaction scheme illustrated below: 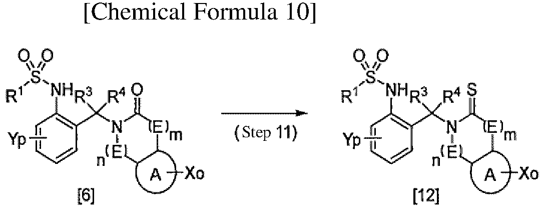 wherein R¹, R³, R⁴, A, E, W, X, Y, m, n, o, and p are as defined in Formula [1] of the above item (1).

### (Step 11)

The compound represented by Formula [6] and a sulfurizing agent may be reacted in a solvent to produce a compound represented by Formula [12].

Examples of the sulfurizing agent that can be used in this step include P₄S₈ and a Lawesson's reagent.

The amount of the sulfurizing agent used in this step may be selected from 0.05 to 10 moles per mole of the compound of Formula [6], and is preferably from 0.1 to 1.2 moles.

Examples of the solvent that can be used in this step include the same compounds described in step 1.

In addition, the volume of the solvent used may be selected from 0.01 to 100 L per mole of the compound of Formula [6], and is preferably from 0.1 to 10 L.

The reaction temperature may be selected from -20°C to a point (in the boiling range of inert solvent used), and preferably ranges from 0°C to 100°C.

The reaction time may vary depending on, for instance, the reaction temperature, the reaction substrate, and the reaction volume, but usually ranges from 10 minutes to 48 hours.

The compound of Formula [12], a target substance of the reaction, is collected from the reaction system after the completion of the reaction according to the standard method, and may be purified by a procedure such as column chromatography and/or recrystallization if necessary.

### <Intermediate Production Method 1>

The intermediate compound represented by Formula [3a] can be produced by a method comprising the reaction scheme illustrated below: 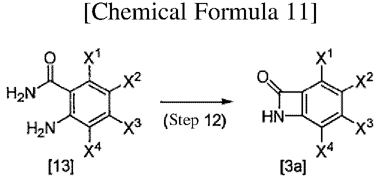 wherein X¹, X², X³, and X⁴ are each as defined in Formula [1] in the above item (1).

### (Step 12)

A compound represented by Formula [13] and a diazotizing reagent may be reacted in a solvent in the presence of an acid to produce a compound represented by Formula [3a].

Examples of the diazotizing reagent that can be used in this step include t-butyl nitrite, and sodium nitrite, or a combination thereof.

The amount of the diazotizing agent used in this step may be selected from 0.05 to 10 moles per mole of the compound of Formula [13], and is preferably from 0.1 to 1.2 moles.

Examples of the solvent that can be used in this step include the same as those described in step 1.

Examples of the acid that can be used in this step include organic acids such as formic acid, acetic acid, glacial acetic acid, methanesulfonic acid, p-toluenesulfonic acid, trifluoroacetic acid, and trifluoromethanesulfonic acid; or a mixture of these.

The amount of the acid used in this reaction may be selected from 0.01 to 100 moles per mole of the compound of Formula [13], and is preferably from 0.1 to 10 moles.

In addition, the volume of the solvent used may be selected from 0.01 to 100 L per mole of the compound of Formula [13], and is preferably from 0.1 to 10 L.

The reaction temperature may be selected from -20°C to a point in the boiling range of inert solvent used, and preferably ranges from 0°C to 100°C.

The reaction time may vary depending on, for instance, the reaction temperature, the reaction substrate, and the reaction volume, but usually ranges from 10 minutes to 48 hours.

The compound of Formula [3a], a target substance of the reaction, is collected from the reaction system after the completion of the reaction according to the standard method, and may be purified by a procedure such as column chromatography and/or recrystallization if necessary.

### <Intermediate Production Method 2>

The intermediate compound represented by Formula [3b] can be produced by a method comprising the reaction scheme illustrated below: 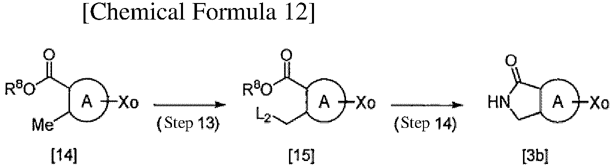 wherein R⁸ represents C₁-C₆ alkyl such as methyl and ethyl, phenyl, or substituted phenyl having, on the ring, one or more substituents selected from Z², A, X, Z, and o are each as defined in Formula [1] in the above item (1), and L₂ represents halogen such as a chlorine atom and a bromine atom.

### (Step 13)

A compound represented by Formula [14] and a halogenating reagent may be reacted in a solvent to produce a compound represented by Formula [15].

Examples of the halogenating reagent that can be used in this step include chlorinating reagents such as chlorine, sulfuryl chloride, and N-chlorosuccinimide; and brominating reagents such as bromine and N-bromosuccinimide; or a mixture containing two or more of these compounds.

The amount of the halogenating reagent used in this step may be selected from 0.05 to 10 moles per mole of the compound of Formula [14], and is preferably from 0.1 to 1.2 moles.

Examples of the solvent that can be used in this step include the same as those described in step 1.

In addition, the volume of the solvent used may be selected from 0.01 to 100 L per mole of the compound of Formula [14], and is preferably from 0.1 to 10 L.

The reaction temperature may be selected from -20°C to a point in the boiling range of inert solvent used, and preferably ranges from 0°C to 100°C.

The reaction time may vary depending on, for instance, the reaction temperature, the reaction substrate, and the reaction volume, but usually ranges from 10 minutes to 48 hours.

The compound of Formula [15], a target substance of the reaction, is collected from the reaction system after the completion of the reaction according to the standard method, and may be purified by a procedure such as column chromatography and/or recrystallization if necessary.

### (Step 14)

The compound represented by Formula [15] and an ammonia reagent may be reacted in a solvent to produce a compound represented by Formula [3b].

Examples of the ammonia reagent that can be used in this step include ammonia water.

The amount of the ammonia reagent used in this step may be selected from 0.05 to 10 moles per mole of the compound of Formula [15], and is preferably from 0.1 to 1.2 moles.

Examples of the solvent that can be used in this step include the same as those described in step 1.

In addition, the volume of the solvent used may be selected from 0.01 to 100 L per mole of the compound of Formula [15], and is preferably from 0.1 to 10 L.

The reaction temperature may be selected from -20°C to a point in the boiling range of inert solvent used, and preferably ranges from 0°C to 100°C.

The reaction time may vary depending on, for instance, the reaction temperature, the reaction substrate, and the reaction volume, but usually ranges from 10 minutes to 48 hours.

The compound of Formula [3b], a target substance of the reaction, is collected from the reaction system after the completion of the reaction according to the standard method, and may be purified by a procedure such as column chromatography and/or recrystallization if necessary.

### <Intermediate Production Method 3>

The intermediate compound represented by Formula [3c] can be produced by a method comprising the reaction scheme illustrated below: 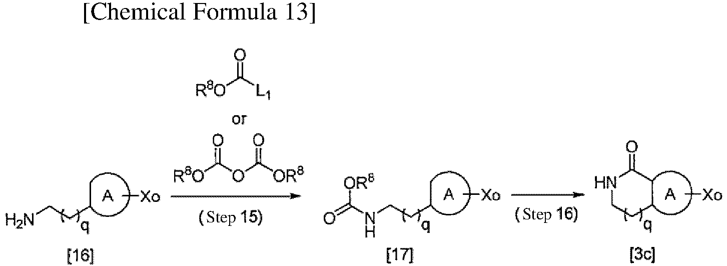 wherein A, X, Z, and o are as defined in Formula [1] of the above item (1) and L¹ and R⁸ have the same meanings as above and q represents 1 or 2.

### (Step 15)

A compound represented by Formula [16] and an alkoxy carbonyl derivative, phenoxy carbonyl derivative or substituted phenoxy carbonyl derivative represented by general formula: R⁸OC(O)L or (R⁸OC(O))₂O may be reacted in a solvent in the presence or absence of a base to produce a compound represented by Formula [17].

The amount of the alkoxy carbonyl derivative, phenoxy carbonyl derivative or substituted phenoxy carbonyl derivative represented by general formula: R⁸OC(O)L or (R⁸OC(O))₂O as used in this step may be selected from 0.5 to 10 moles per mole of the compound of Formula [16], and is preferably from 1.0 to 1.2 moles.

Examples of the solvent and the base that can be used in this step include the same compounds described in step 1.

The amount of the base used in this reaction may be selected from 0 to 100 moles per mole of the compound of Formula [16], and is preferably from 0 to 10 moles.

In addition, the volume of the solvent used may be selected from 0.01 to 100 L per mole of the compound of Formula [16], and is preferably from 0.1 to 10 L.

The reaction temperature may be selected from -78°C to a point in the boiling range of inert solvent used, and preferably ranges from 0°C to 100°C.

The reaction time may vary depending on, for instance, the reaction temperature, the reaction substrate, and the reaction volume, but usually ranges from 10 minutes to 48 hours.

The compound of Formula [17], a target substance of the reaction, is collected from the reaction system after the completion of the reaction according to the standard method, and may be purified by a procedure such as column chromatography and/or recrystallization if necessary.

### (Step 16)

The compound represented by Formula [17] may be reacted in a solvent in the presence of an acid to produce a compound represented by Formula [3c].

Examples of the acid that can be used in this step include: organic acids such as formic acid, acetic acid, methanesulfonic acid, p-toluenesulfonic acid, trifluoroacetic acid, and trifluoromethanesulfonic acid; inorganic acids such as hydrochloric acid, sulfuric acid, and hydrogen bromide; and Lewis acids such as zinc chloride, titanium tetrachloride, tin chloride, aluminum chloride, iron chloride, and boron trifluoride-ether complex; or a mixture of two or more of these compounds.

In addition, the reaction can also be carried out using a phase-transfer catalyst such as a quaternary ammonium salt. Examples of the phase-transfer catalyst include the same catalysts described in step 1. In the case of using a phase-transfer catalyst, the amount used may be selected from 0.0001 to 1.0 moles per mole of the compound of Formula [17], and is preferably from 0.001 to 0.1 moles.

Examples of the solvent that can be used in this step include the same as those described in step 1.

The amount of the acid used in this reaction may be selected from 0.01 to 100 moles per mole of the compound of Formula [17], and is preferably from 0.1 to 10 moles.

In addition, the volume of the solvent used may be selected from 0.01 to 100 L per mole of the compound of Formula [17], and is preferably from 0.1 to 10 L.

The reaction temperature may be selected from 0°C to a point in the boiling range of inert solvent used, and preferably ranges from 0°C to 100°C.

The reaction time may vary depending on, for instance, the reaction temperature, the reaction substrate, and the reaction volume, but usually ranges from 10 minutes to 72 hours.

The compound of Formula [3c], a target substance of the reaction, is collected from the reaction system after the completion of the reaction according to the standard method, and may be purified by a procedure such as column chromatography and/or recrystallization if necessary.

### <Intermediate Production Method 4>

The intermediate compound represented by Formula [5a] can be produced by a method comprising the reaction scheme illustrated below: 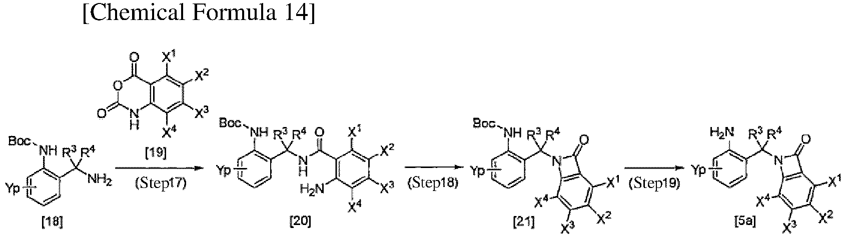 wherein R¹, R³, R⁴, X¹, X², X³, X⁴, Y and p are as defined in Formula [1] of the above item (1).

### (Step 17)

A compound represented by Formula [18] and a compound represented by Formula [19] may be reacted in a solvent to produce a compound represented by Formula [20].

The amount of the compound of Formula [19] used in this step may be selected from 0.5 to 10 moles per mole of the compound of Formula [18], and is preferably from 1.0 to 1.2 moles.

Examples of the solvent that can be used in this step include the same as those described in step 1.

In addition, the volume of the solvent used may be selected from 0.01 to 100 L per mole of the compound of Formula [18], and is preferably from 0.1 to 10 L.

The reaction temperature may be selected from 0°C to a point in the boiling range of inert solvent used, and preferably ranges from 0°C to 100°C.

The reaction time may vary depending on, for instance, the reaction temperature, the reaction substrate, and the reaction volume, but usually ranges from 10 minutes to 72 hours.

The compound of Formula [20], a target substance of the reaction, is collected from the reaction system after the completion of the reaction according to the standard method, and may be purified by a procedure such as column chromatography and/or recrystallization if necessary.

### (Step 18)

The compound represented by Formula [20] and a diazotizing reagent may be reacted in a solvent in the presence of an acid to produce a compound represented by Formula [21].

Examples of the diazotizing reagent that can be used in this step include t-butyl nitrite, sodium nitrite, and a mixture thereof.

The amount of the diazotizing agent used in this step may be selected from 0.05 to 10 moles per mole of the compound of Formula [20], and is preferably from 0.1 to 1.2 moles.

Examples of the solvent that can be used in this step include the same as those described in step 1.

Examples of the acid that can be used in this step include organic acids such as formic acid, acetic acid, glacial acetic acid, methanesulfonic acid, p-toluenesulfonic acid, trifluoroacetic acid, and trifluoromethanesulfonic acid; or a mixture of two or more of these.

The amount of the acid used in this reaction may be selected from 0.01 to 100 moles per mole of the compound of Formula [20], and is preferably from 0.1 to 10 moles.

In addition, the volume of the solvent used may be selected from 0.01 to 100 L per mole of the compound of Formula [20], and is preferably from 0.1 to 10 L.

The reaction temperature may be selected from -20°C to a point in the boiling range of inert solvent used, and preferably ranges from 0°C to 100°C.

The reaction time may vary depending on, for instance, the reaction temperature, the reaction substrate, and the reaction volume, but usually ranges from 10 minutes to 48 hours.

The compound of Formula [21], a target substance of the reaction, is collected from the reaction system after the completion of the reaction according to the standard method, and may be purified by a procedure such as column chromatography and/or recrystallization if necessary.

### (Step 19)

The compound represented by Formula [21] may be reacted in a solvent in the presence of an acid to produce a compound represented by Formula [5a].

Examples of the solvent and the acid that can be used in this step include the same compounds described in steps 1 and 10.

The amount of the acid used in this reaction may be selected from 0.01 to 100 moles per mole of the compound of Formula [21], and is preferably from 0.1 to 10 moles.

In addition, the volume of the solvent used may be selected from 0.01 to 100 L per mole of the compound of Formula [21], and is preferably from 0.1 to 10 L.

The reaction temperature may be selected from 0°C to a point in the boiling range of inert solvent used, and preferably ranges from 0°C to 100°C.

The reaction time may vary depending on, for instance, the reaction temperature, the reaction substrate, and the reaction volume, but usually ranges from 10 minutes to 72 hours.

The compound of Formula [5a], a target substance of the reaction, is collected from the reaction system after the completion of the reaction according to the standard method, and may be purified by a procedure such as column chromatography and/or recrystallization if necessary.

The protecting group of the amine may be any protecting group that does not interfere with the sulfurization reaction. The use and choice of the protecting group and deprotection are obvious to those skilled in the field of chemical synthesis (see, for example, Protective Groups in Organic Synthesis, 4th edition by T. W. Greene and P. G. Wuts; Wiley: New York, 2007).

It is assumed that some of the reagents and reaction conditions described above for the preparation of compound of Formula [1] may not be compatible with certain functional groups present in the intermediates. In these examples, the desired product can be obtained by incorporating protection/deprotection techniques or functional group interconversion into the synthesis. The use and choice of the protecting group should be obvious to those skilled in the field of chemical synthesis (see, for example, Protective Groups in Organic Synthesis, 4th edition by T. W. Greene and P. G. Wuts; Wiley: New York, 2007). In some cases, the skilled person will recognize that after the introduction of certain reagents as described in the individual schemes, it may be necessary to perform additional canonical synthetic steps not described to complete the synthesis of the compound of Formula [1]. The skilled person will also recognize that it may be necessary to perform a combination of the steps illustrated in the above schemes in an order other than that shown in the specific order proposed for the preparation of the compound of Formula [1].

By using the methods described herein in combination with methods well-known in the conventional art, the compounds shown in the following tables can be prepared.

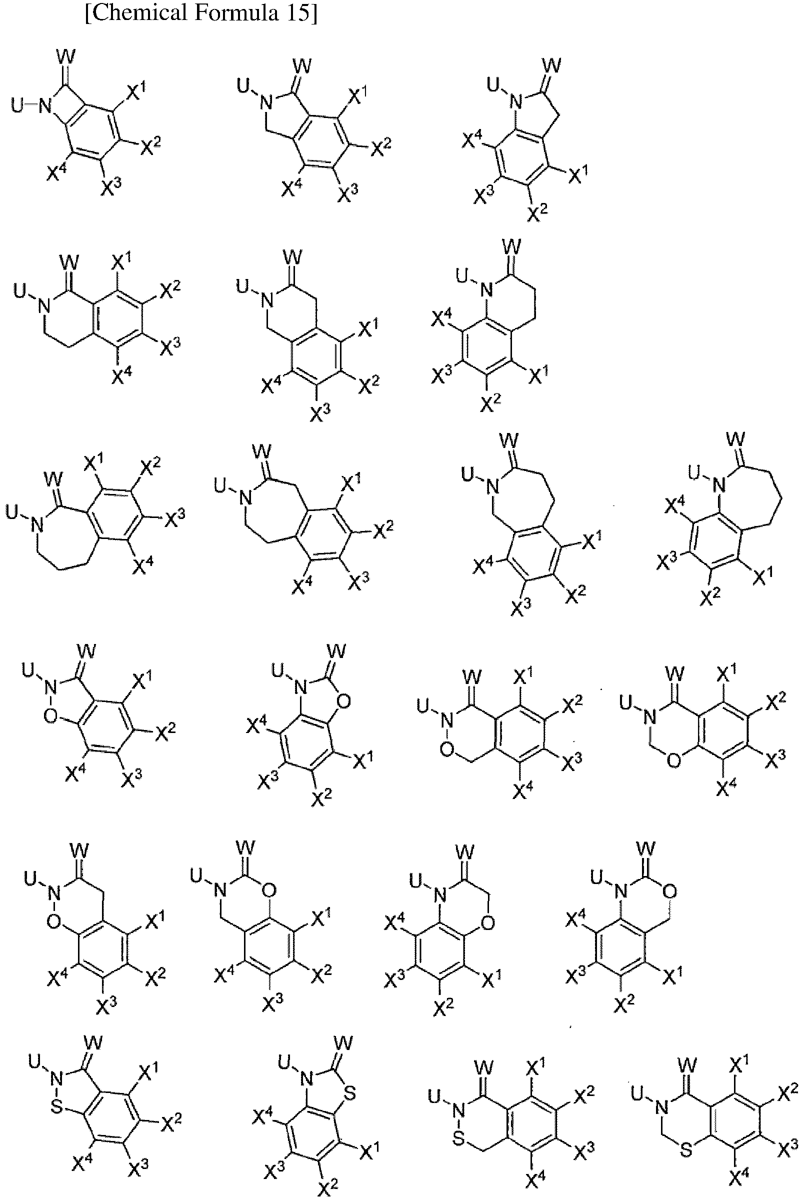

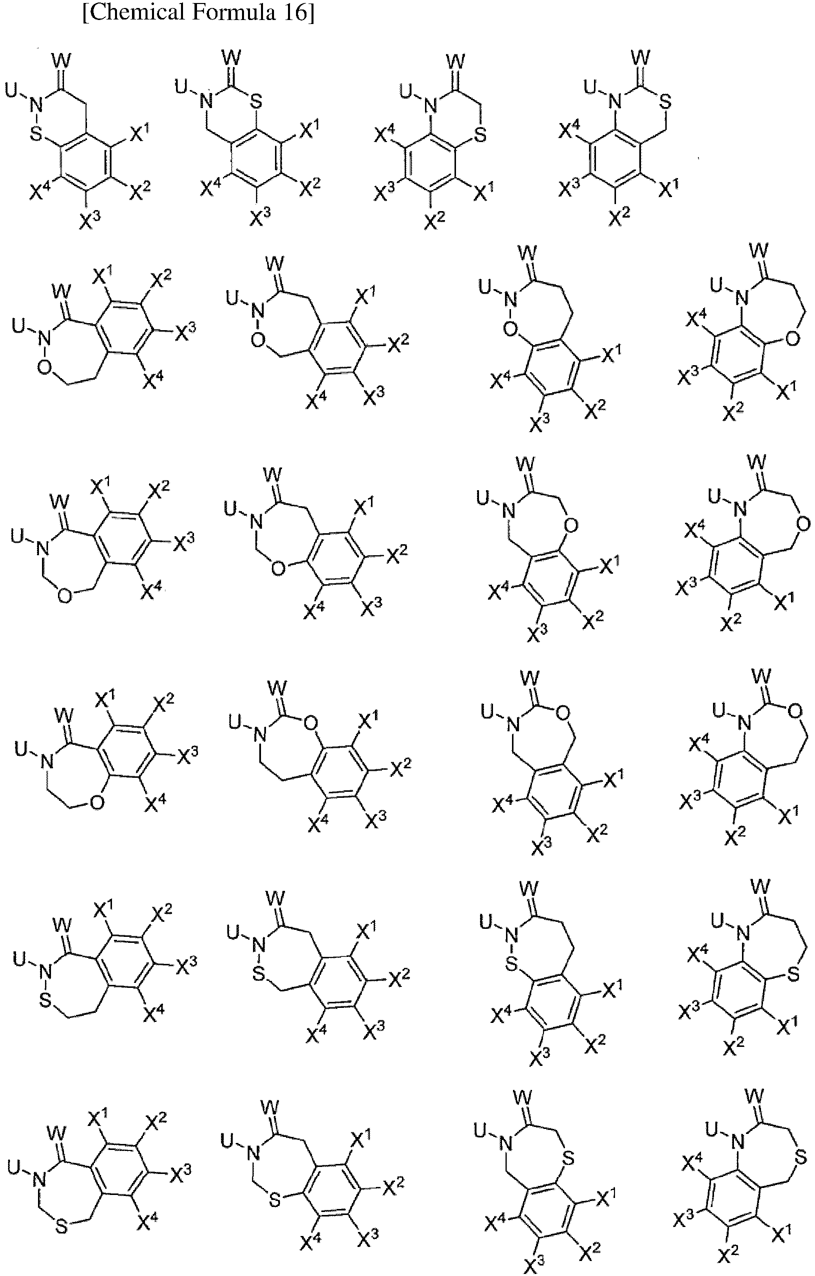

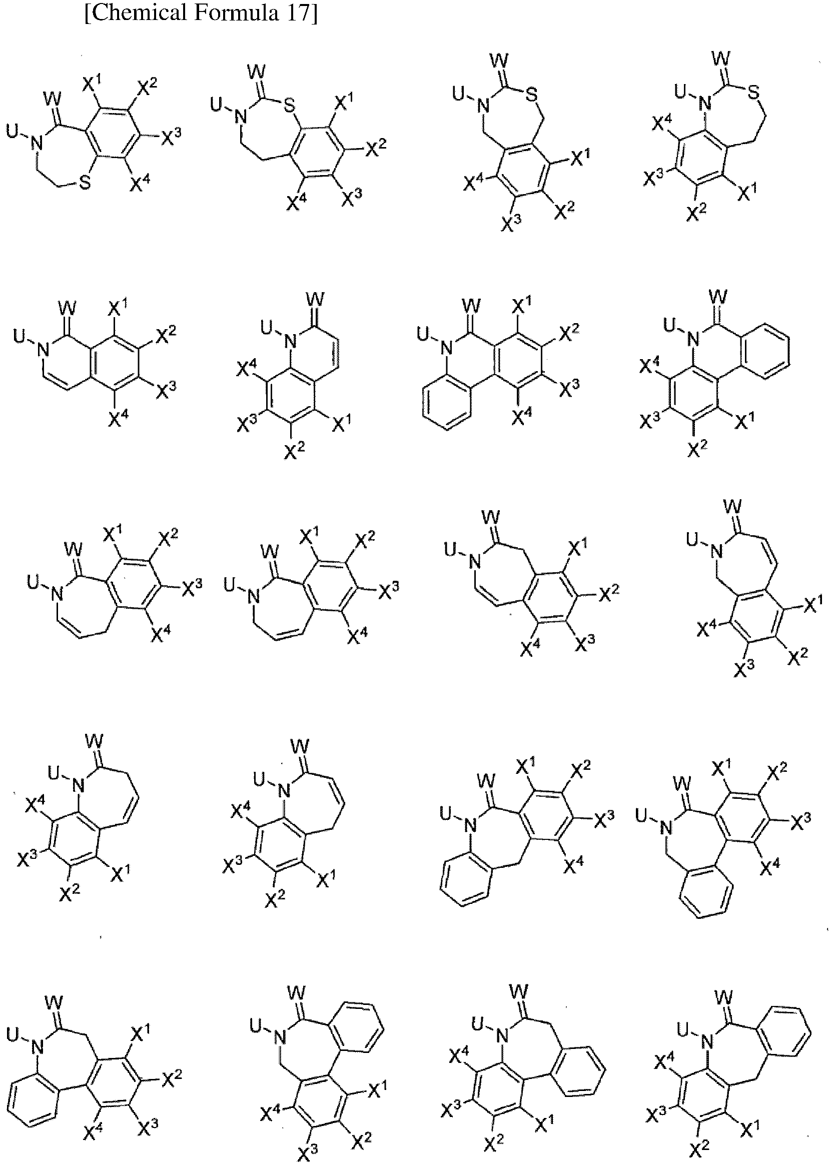

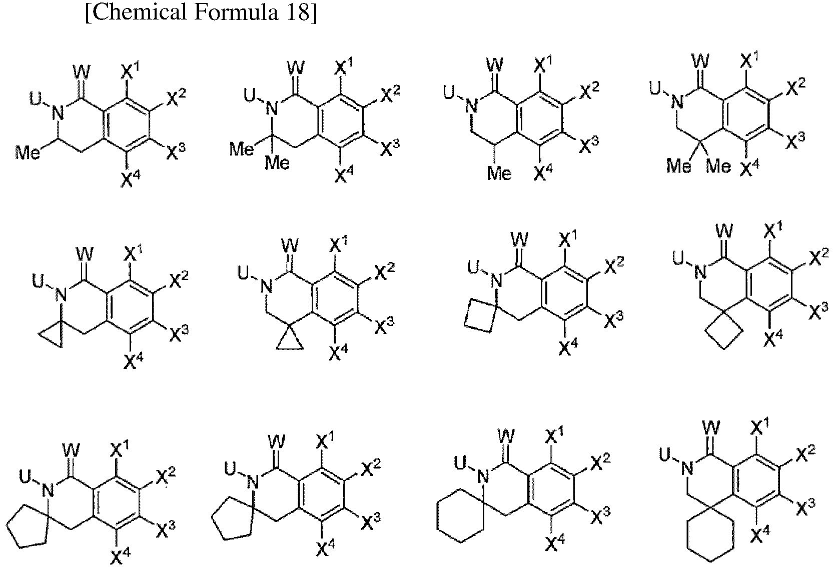

U is represented by the following general formula (22):

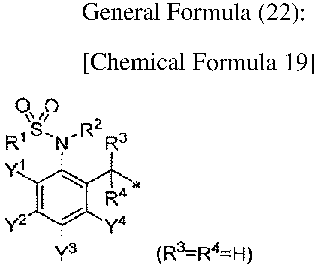

**[Table 1]**

| R¹ | R² | W | X¹ | X² | X³ | X⁴ | Y¹ | Y² | Y³ | Y⁴ |
|---|---|---|---|---|---|---|---|---|---|---|
| CHF₂ | H | O | H | H | H | H | H | H | H | H |
| CHF₂ | COMe | O | H | H | H | H | H | H | H | H |
| CHF₂ | COMe | O | F | H | H | H | H | H | H | H |
| CHF₂ | COMe | O | Cl | H | H | H | H | H | H | H |
| CHF₂ | COMe | O | F | H | H | F | H | H | H | H |
| CHF₂ | COMe | O | Cl | H | H | Cl | H | H | H | H |
| CHF₂ | COc-Pr | O | H | H | H | H | H | H | H | H |
| CHF₂ | COc-Pr | O | H | H | H | H | H | H | H | H |
| CHF₂ | COc-Pr | O | F | H | H | H | H | H | H | H |
| CHF₂ | COc-Pr | O | Cl | H | H | H | H | H | H | H |
| CHF₂ | COc-Pr | O | F | H | H | F | H | H | H | H |
| CHF₂ | COc-Pr | O | Cl | H | H | Cl | H | H | H | H |
| CHF₂ | COc-Pr | O | H | H | H | H | H | H | H | H |
| CHF₂ | CO₂Me | O | H | H | H | H | H | H | H | H |
| CHF₂ | CO₂Me | O | H | H | H | H | H | H | H | H |
| CHF₂ | CO₂Me | O | F | H | H | H | H | H | H | H |
| CHF₂ | CO₂Me | O | Cl | H | H | H | H | H | H | H |
| CHF₂ | CO₂Me | O | F | H | H | F | H | H | H | H |
| CHF₂ | CO₂Me | O | Cl | H | H | Cl | H | H | H | H |
| CHF₂ | CO₂Me | O | H | H | H | H | H | H | H | H |
| CHF₂ | CO₂Et | O | H | H | H | H | H | H | H | H |
| CHF₂ | CO₂Et | O | F | H | H | H | H | H | H | H |
| CHF₂ | CO₂Et | O | Cl | H | H | H | H | H | H | H |
| CHF₂ | CO₂Et | O | F | H | H | F | H | H | H | H |
| CHF₂ | CO₂Et | O | Cl | H | H | Cl | H | H | H | H |
| CHF₂ | CO₂Et | O | H | H | H | H | H | H | H | H |
| CHF₂ | CO₂CH₂CH₂F | O | H | H | H | H | H | H | H | H |
| CHF₂ | CO₂CH₂CH₂F | O | F | H | H | H | H | H | H | H |
| CHF₂ | CO₂CH₂CH₂F | O | Cl | H | H | H | H | H | H | H |

**[Table 2]**

| R¹ | R² | W | X¹ | X² | X³ | X⁴ | Y¹ | Y² | Y³ | Y⁴ |
|---|---|---|---|---|---|---|---|---|---|---|
| CHF₂ | CO₂CH₂CH₂F | O | F | H | H | F | H | H | H | H |
| CHF₂ | CO₂CH₂CH₂F | O | Cl | H | H | Cl | H | H | H | H |
| CHF₂ | CO₂CH₂CH₂F | O | H | H | H | H | H | H | H | H |
| CHF₂ | CO₂CH₂CHF₂ | O | H | H | H | H | H | H | H | H |
| CHF₂ | CO₂CH₂CHF₂ | O | F | H | H | H | H | H | H | H |
| CHF₂ | CO₂CH₂CHF₂ | O | Cl | H | H | H | H | H | H | H |
| CHF₂ | CO₂CH₂CHF₂ | O | F | H | H | F | H | H | H | H |
| CHF₂ | CO₂CH₂CHF₂ | O | Cl | H | H | Cl | H | H | H | H |
| CHF₂ | CO₂CH₂CHF₂ | O | H | H | H | H | H | H | H | H |
| CHF₂ | CO₂(3-oxetanyl) | O | H | H | H | H | H | H | H | H |
| CHF₂ | CO₂(3-oxetanyl) | O | F | H | H | H | H | H | H | H |
| CHF₂ | CO₂(3-oxetanyl) | O | Cl | H | H | H | H | H | H | H |
| CHF₂ | CO₂(3-oxetanyl) | O | F | H | H | F | H | H | H | H |
| CHF₂ | CO₂(3-oxetanyl) | O | Cl | H | H | Cl | H | H | H | H |
| CHF₂ | CO₂(3-oxetanyl) | O | H | H | H | H | H | H | H | H |
| CHF₂ | CO₂(3-tetrahydrofuryl) | O | H | H | H | H | H | H | H | H |
| CHF₂ | CO₂(3-tetrahydrofuryl) | O | F | H | H | H | H | H | H | H |
| CHF₂ | CO₂(3-tetrahydrofuryl) | O | Cl | H | H | H | H | H | H | H |
| CHF₂ | CO₂(3-tetrahydrofuryl) | O | F | H | H | F | H | H | H | H |
| CHF₂ | CO₂(3-tetrahydrofuryl) | O | Cl | H | H | Cl | H | H | H | H |
| CHF₂ | CO₂(3-tetrahydrofuryl) | O | H | H | H | H | H | H | H | H |
| CHF₂ | CO(SMe) | O | H | H | H | H | H | H | H | H |
| CHF₂ | CO(SMe) | O | F | H | H | H | H | H | H | H |
| CHF₂ | CO(SMe) | O | Cl | H | H | H | H | H | H | H |
| CHF₂ | CO(SMe) | O | F | H | H | F | H | H | H | H |
| CHF₂ | CO(SMe) | O | Cl | H | H | Cl | H | H | H | H |
| CHF₂ | CO(SMe) | O | H | H | H | H | H | H | H | H |
| CHF₂ | SO₂Me | O | H | H | H | H | H | H | H | H |
| CHF₂ | SO₂Me | O | F | H | H | H | H | H | H | H |

**[Table 3]**

| R¹ | R² | W | X¹ | X² | X³ | X⁴ | Y¹ | Y² | Y³ | Y⁴ |
|---|---|---|---|---|---|---|---|---|---|---|
| CHF₂ | SO₂Me | O | Cl | H | H | H | H | H | H | H |
| CHF₂ | SO₂Me | O | F | H | H | F | H | H | H | H |
| CHF₂ | SO₂Me | O | Cl | H | H | Cl | H | H | H | H |
| CHF₂ | SO₂Me | O | H | H | H | H | H | H | H | H |
| CHF₂ | SO₂CF₃ | O | H | H | H | H | H | H | H | H |
| CHF₂ | SO₂CF₃ | O | F | H | H | H | H | H | H | H |
| CHF₂ | SO₂CF₃ | O | Cl | H | H | H | H | H | H | H |
| CHF₂ | SO₂CF₃ | O | F | H | H | F | H | H | H | H |
| CHF₂ | SO₂CF₃ | O | Cl | H | H | Cl | H | H | H | H |
| CHF₂ | SO₂CF₃ | O | H | H | H | H | H | H | H | H |
| CClF₂ | H | O | H | H | H | H | H | H | H | H |
| CClF₂ | H | O | F | H | H | H | H | H | H | H |
| CClF₂ | H | O | Cl | H | H | H | H | H | H | H |
| CClF₂ | H | O | F | H | H | F | H | H | H | H |
| CClF₂ | H | O | Cl | H | H | Cl | H | H | H | H |
| CClF₂ | H | O | H | H | H | H | H | H | H | H |
| CBrF₂ | H | O | H | H | H | H | H | H | H | H |
| CBrF₂ | H | O | F | H | H | H | H | H | H | H |
| CBrF₂ | H | O | Cl | H | H | H | H | H | H | H |
| CBrF₂ | H | O | F | H | H | F | H | H | H | H |
| CBrF₂ | H | O | Cl | H | H | Cl | H | H | H | H |
| CBrF₂ | H | O | H | H | H | H | H | H | H | H |
| CCl₂F | H | O | H | H | H | H | H | H | H | H |
| CCl₂F | H | O | F | H | H | H | H | H | H | H |
| CCl₂F | H | O | Cl | H | H | H | H | H | H | H |
| CCl₂F | H | O | F | H | H | F | H | H | H | H |
| CCl₂F | H | O | Cl | H | H | Cl | H | H | H | H |
| CCl₂F | H | O | H | H | H | H | H | H | H | H |
| CCl₃ | H | O | H | H | H | H | H | H | H | H |

**[Table 4]**

| R¹ | R² | W | X¹ | X² | X³ | X⁴ | Y¹ | Y² | Y³ | Y⁴ |
|---|---|---|---|---|---|---|---|---|---|---|
| CCl₃ | H | O | F | H | H | H | H | H | H | H |
| CCl₃ | H | O | Cl | H | H | H | H | H | H | H |
| CCl₃ | H | O | F | H | H | F | H | H | H | H |
| CCl₃ | H | O | Cl | H | H | Cl | H | H | H | H |
| CCl₃ | H | O | H | H | H | H | H | H | H | H |
| CF₃ | H | O | H | H | H | H | H | H | H | H |
| CF₃ | Me | O | H | H | H | H | H | H | H | H |
| CF₃ | Et | O | H | H | H | H | H | H | H | H |
| CF₃ | CH₂CF₃ | O | H | H | H | H | H | H | H | H |
| CF₃ | CH₂CH=CH₂ | O | H | H | H | H | H | H | H | H |
| CF₃ | CH₂C≡CH | O | H | H | H | H | H | H | H | H |
| CF₃ | CH₂OMe | O | H | H | H | H | H | H | H | H |
| CF₃ | CH₂OCH₂CF₃ | O | H | H | H | H | H | H | H | H |
| CF₃ | CH₂OCH₂CH₂OMe | O | H | H | H | H | H | H | H | H |
| CF₃ | CH₂SMe | O | H | H | H | H | H | H | H | H |
| CF₃ | CH₂COMe | O | H | H | H | H | H | H | H | H |
| CF₃ | CH₂Ph | O | H | H | H | H | H | H | H | H |
| CF₃ | CH₂(4-ClPh) | O | H | H | H | H | H | H | H | H |
| CF₃ | CH₂(4-MePh) | O | H | H | H | H | H | H | H | H |
| CF₃ | CH₂(4-MeOPh) | O | H | H | H | H | H | H | H | H |
| CF₃ | CH₂CH₂OPh | O | H | H | H | H | H | H | H | H |
| CF₃ | COMe | O | H | H | H | H | H | H | H | H |
| CF₃ | COEt | O | H | H | H | H | H | H | H | H |
| CF₃ | COn-Pr | O | H | H | H | H | H | H | H | H |
| CF₃ | COi-Pr | O | H | H | H | H | H | H | H | H |
| CF₃ | COn-Bu | O | H | H | H | H | H | H | H | H |
| CF₃ | COs-Bu | O | H | H | H | H | H | H | H | H |
| CF₃ | COi-Bu | O | H | H | H | H | H | H | H | H |
| CF₃ | COt-Bu | O | H | H | H | H | H | H | H | H |

**[Table 5]**

| R¹ | R² | W | X¹ | X² | X³ | X⁴ | Y¹ | Y² | Y³ | Y⁴ |
|---|---|---|---|---|---|---|---|---|---|---|
| CF₃ | COn-Pen | O | H | H | H | H | H | H | H | H |
| CF₃ | COn-Hex | O | H | H | H | H | H | H | H | H |
| CF₃ | COCF₃ | O | H | H | H | H | H | H | H | H |
| CF₃ | COCH₂CF₃ | O | H | H | H | H | H | H | H | H |
| CF₃ | COCH=CH₂ | O | H | H | H | H | H | H | H | H |
| CF₃ | COC=CH | O | H | H | H | H | H | H | H | H |
| CF₃ | COc-Pr | O | H | H | H | H | H | H | H | H |
| CF₃ | COc-Bu | O | H | H | H | H | H | H | H | H |
| CF₃ | COc-Pen | O | H | H | H | H | H | H | H | H |
| CF₃ | COc-Hex | O | H | H | H | H | H | H | H | H |
| CF₃ | COCH₂c-Pr | O | H | H | H | H | H | H | H | H |
| CF₃ | COCH₂OMe | O | H | H | H | H | H | H | H | H |
| CF₃ | COCH₂OCH₂CF₃ | O | H | H | H | H | H | H | H | H |
| CF₃ | COCH₂OCH₂CH₂CF₃ | O | H | H | H | H | H | H | H | H |
| CF₃ | COCH₂SMe | O | H | H | H | H | H | H | H | H |
| CF₃ | COCH₂SCH₂CF₃ | O | H | H | H | H | H | H | H | H |
| CF₃ | Bz | O | H | H | H | H | H | H | H | H |
| CF₃ | 4-ClBz | O | H | H | H | H | H | H | H | H |
| CF₃ | 4-MeBz | O | H | H | H | H | H | H | H | H |
| CF₃ | COCH₂Ph | O | H | H | H | H | H | H | H | H |
| CF₃ | COCH₂(4-ClPh) | O | H | H | H | H | H | H | H | H |
| CF₃ | COCH₂(4-MePh) | O | H | H | H | H | H | H | H | H |
| CF₃ | CO(2-tetrahydrofuryl) | O | H | H | H | H | H | H | H | H |
| CF₃ | CO(3-tetrahydrofuryl) | O | H | H | H | H | H | H | H | H |
| CF₃ | CO(2-pyridyl) | O | H | H | H | H | H | H | H | H |
| CF₃ | CO(3-pyridyl) | O | H | H | H | H | H | H | H | H |
| CF₃ | CO(4-pyridyl) | O | H | H | H | H | H | H | H | H |
| CF₃ | CO(2-thienyl) | O | H | H | H | H | H | H | H | H |
| CF₃ | CO(3-thienyl) | O | H | H | H | H | H | H | H | H |

**[Table 6]**

| R¹ | R² | W | X¹ | X² | X³ | X⁴ | Y¹ | Y² | Y³ | Y⁴ |
|---|---|---|---|---|---|---|---|---|---|---|
| CF₃ | CO(2-tetrahydrofurfuryl) | O | H | H | H | H | H | H | H | H |
| CF₃ | COCH₂(2-pyridyl) | O | H | H | H | H | H | H | H | H |
| CF₃ | COCH₂(2-thienyl) | O | H | H | H | H | H | H | H | H |
| CF₃ | CO₂Me | O | H | H | H | H | H | H | H | H |
| CF₃ | CO₂Et | O | H | H | H | H | H | H | H | H |
| CF₃ | CO₂n-Pr | O | H | H | H | H | H | H | H | H |
| CF₃ | CO₂i-Pr | O | H | H | H | H | H | H | H | H |
| CF₃ | CO₂n-Bu | O | H | H | H | H | H | H | H | H |
| CF₃ | CO₂s-Bu | O | H | H | H | H | H | H | H | H |
| CF₃ | CO₂i-Bu | O | H | H | H | H | H | H | H | H |
| CF₃ | CO₂t-Bu | O | H | H | H | H | H | H | H | H |
| CF₃ | CO₂n-Pen | O | H | H | H | H | H | H | H | H |
| CF₃ | CO₂n-Hex | O | H | H | H | H | H | H | H | H |
| CF₃ | CO₂CH₂CH₂F | O | H | H | H | H | H | H | H | H |
| CF₃ | CO₂CH₂CHF₂ | O | H | H | H | H | H | H | H | H |
| CF₃ | CO₂CH₂CF₃ | O | H | H | H | H | H | H | H | H |
| CF₃ | CO₂CH=CH₂ | O | H | H | H | H | H | H | H | H |
| CF₃ | CO₂C≡CH | O | H | H | H | H | H | H | H | H |
| CF₃ | CO₂c-Pr | O | H | H | H | H | H | H | H | H |
| CF₃ | CO₂CH₂c-Pr | O | H | H | H | H | H | H | H | H |
| CF₃ | CO₂CH₂CH₂OMe | O | H | H | H | H | H | H | H | H |
| CF₃ | CO₂CH₂CH₂OCH₂CF₂ | O | H | H | H | H | H | H | H | H |
| CF₃ | CO₂CH₂CH₂OCH₂CH₂OMe | O | H | H | H | H | H | H | H | H |
| CF₃ | CO₂CH₂CH₂SMe | O | H | H | H | H | H | H | H | H |
| CF₃ | CO₂CH₂CH₂SCH₂CF₃ | O | H | H | H | H | H | H | H | H |
| CF₃ | CO₂Ph | O | H | H | H | H | H | H | H | H |
| CF₃ | CO₂(4-ClPh) | O | H | H | H | H | H | H | H | H |
| CF₃ | CO₂(4-MePh) | O | H | H | H | H | H | H | H | H |
| CF₃ | CO₂CH₂Ph | O | H | H | H | H | H | H | H | H |

**[Table 7]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂CH₂(4-ClPh) | O | H | H | H | H | H | H | H | H |
| CF₃ | CO₂CH₂(4-MePh) | O | H | H | H | H | H | H | H | H |
| CF₃ | CO₂(3-oxetanyl) | O | H | H | H | H | H | H | H | H |
| CF₃ | CO₂(2-tetrahydrofuryl) | O | H | H | H | H | H | H | H | H |
| CF₃ | CO₂(3-tetrahydrofuryl) | O | H | H | H | H | H | H | H | H |
| CF₃ | CO₂(2-pyridyl) | O | H | H | H | H | H | H | H | H |
| CF₃ | CO₂(2-thienyl) | O | H | H | H | H | H | H | H | H |
| CF₃ | CO₂(2-tetrahydrofurfuryl) | O | H | H | H | H | H | H | H | H |
| CF₃ | CO₂CH₂(2-pyridyl) | O | H | H | H | H | H | H | H | H |
| CF₃ | CO₂CH₂(2-thienyl) | O | H | H | H | H | H | H | H | H |
| CF₃ | CO(SMe) | O | H | H | H | H | H | H | H | H |
| CF₃ | CO(SEt) | O | H | H | H | H | H | H | H | H |
| CF₃ | CO(SCF₃) | O | H | H | H | H | H | H | H | H |
| CF₃ | CO(SCH₂CF₃) | O | H | H | H | H | H | H | H | H |
| CF₃ | CONHEt | O | H | H | H | H | H | H | H | H |
| CF₃ | CONHCH₂CF₃ | O | H | H | H | H | H | H | H | H |
| CF₃ | CONEt₂ | O | H | H | H | H | H | H | H | H |
| CF₃ | SO₂Me | O | H | H | H | H | H | H | H | H |
| CF₃ | SO₂Et | O | H | H | H | H | H | H | H | H |
| CF₃ | SO₂n-Pr | O | H | H | H | H | H | H | H | H |
| CF₃ | SO₂i-Pr | O | H | H | H | H | H | H | H | H |
| CF₃ | SO₂n-Bu | O | H | H | H | H | H | H | H | H |
| CF₃ | SO₂i-Bu | O | H | H | H | H | H | H | H | H |
| CF₃ | SO₂CH₂Cl | O | H | H | H | H | H | H | H | H |
| CF₃ | SO₂CCl₃ | O | H | H | H | H | H | H | H | H |
| CF₃ | SO₂CHF₂ | O | H | H | H | H | H | H | H | H |
| CF₃ | SO₂CF₃ | O | H | H | H | H | H | H | H | H |
| CF₃ | SO₂CH₂CF₃ | O | H | H | H | H | H | H | H | H |
| CF₃ | SO₂CH=CH₂ | O | H | H | H | H | H | H | H | H |

**[Table 8]**

| R¹ | R² | W | X¹ | X² | X³ | X⁴ | Y¹ | Y² | Y³ | Y⁴ |
|---|---|---|---|---|---|---|---|---|---|---|
| CF₃ | SO₂CH₂CH=CH₂ | O | H | H | H | H | H | H | H | H |
| CF₃ | SO₂CH₂C≡CH | O | H | H | H | H | H | H | H | H |
| CF₃ | SO₂c-Pr | O | H | H | H | H | H | H | H | H |
| CF₃ | SO₂c-Hex | O | H | H | H | H | H | H | H | H |
| CF₃ | SO₂CH₂c-Pr | O | H | H | H | H | H | H | H | H |
| CF₃ | SO₂CH₂CH₂OMe | O | H | H | H | H | H | H | H | H |
| CF₃ | SO₂Ph | O | H | H | H | H | H | H | H | H |
| CF₃ | SO₂(4-ClPh) | O | H | H | H | H | H | H | H | H |
| CF₃ | SO₂(4-MePh) | O | H | H | H | H | H | H | H | H |
| CF₃ | SO₂CH₂Ph | O | H | H | H | H | H | H | H | H |
| CF₃ | SO₂CH₂(4-ClPh) | O | H | H | H | H | H | H | H | H |
| CF₃ | SO₂CH₂(4-MePh) | O | H | H | H | H | H | H | H | H |
| CF₃ | SO₂NHMe | O | H | H | H | H | H | H | H | H |
| CF₃ | SO₂NMe₂ | O | H | H | H | H | H | H | H | H |
| CF₃ | H | O | F | H | H | H | H | H | H | H |
| CF₃ | Me | O | F | H | H | H | H | H | H | H |
| CF₃ | Et | O | F | H | H | H | H | H | H | H |
| CF₃ | CH₂CF₃ | O | F | H | H | H | H | H | H | H |
| CF₃ | CH₂CH=CH₂ | O | F | H | H | H | H | H | H | H |
| CF₃ | CH₂C≡CH | O | F | H | H | H | H | H | H | H |
| CF₃ | CH₂OMe | O | F | H | H | H | H | H | H | H |
| CF₃ | CH₂OCH₂CF₃ | O | F | H | H | H | H | H | H | H |
| CF₃ | CH₂OCH₂CH₂OMe | O | F | H | H | H | H | H | H | H |
| CF₃ | CH₂SMe | O | F | H | H | H | H | H | H | H |
| CF₃ | CH₂COMe | O | F | H | H | H | H | H | H | H |
| CF₃ | CH₂Ph | O | F | H | H | H | H | H | H | H |
| CF₃ | CH₂(4-ClPh) | O | F | H | H | H | H | H | H | H |
| CF₃ | CH₂(4-MePh) | O | F | H | H | H | H | H | H | H |
| CF₃ | CH₂(4-MeOPh) | O | F | H | H | H | H | H | H | H |

**[Table 9]**

| R¹ | R² | W | X¹ | X² | X³ | X⁴ | Y¹ | Y² | Y³ | Y⁴ |
|---|---|---|---|---|---|---|---|---|---|---|
| CF₃ | CH₂CH₂OPh | O | F | H | H | H | H | H | H | H |
| CF₃ | COMe | O | F | H | H | H | H | H | H | H |
| CF₃ | COEt | O | F | H | H | H | H | H | H | H |
| CF₃ | COn-Pr | O | F | H | H | H | H | H | H | H |
| CF₃ | COi-Pr | O | F | H | H | H | H | H | H | H |
| CF₃ | COn-Bu | O | F | H | H | H | H | H | H | H |
| CF₃ | COs-Bu | O | F | H | H | H | H | H | H | H |
| CF₃ | COi-Bu | O | F | H | H | H | H | H | H | H |
| CF₃ | COt-Bu | O | F | H | H | H | H | H | H | H |
| CF₃ | COn-Pen | O | F | H | H | H | H | H | H | H |
| CF₃ | COn-Hex | O | F | H | H | H | H | H | H | H |
| CF₃ | COCF₃ | O | F | H | H | H | H | H | H | H |
| CF₃ | COCH₂CF₃ | O | F | H | H | H | H | H | H | H |
| CF₃ | COCH=CH₂ | O | F | H | H | H | H | H | H | H |
| CF₃ | COC≡CH | O | F | H | H | H | H | H | H | H |
| CF₃ | COc-Pr | O | F | H | H | H | H | H | H | H |
| CF₃ | COc-Bu | O | F | H | H | H | H | H | H | H |
| CF₃ | COc-Pen | O | F | H | H | H | H | H | H | H |
| CF₃ | COc-Hex | O | F | H | H | H | H | H | H | H |
| CF₃ | COCH₂c-Pr | O | F | H | H | H | H | H | H | H |
| CF₃ | COCH₂OMe | O | F | H | H | H | H | H | H | H |
| CF₃ | COCH₂OCH₂CF₃ | O | F | H | H | H | H | H | H | H |
| CF₃ | COCH₂OCH₂CH₂CF₃ | O | F | H | H | H | H | H | H | H |
| CF₃ | COCH₂SMe | O | F | H | H | H | H | H | H | H |
| CF₃ | COCH₂SCH₂CF₃ | O | F | H | H | H | H | H | H | H |
| CF₃ | Bz | O | F | H | H | H | H | H | H | H |
| CF₃ | 4-ClBz | O | F | H | H | H | H | H | H | H |
| CF₃ | 4-MeBz | O | F | H | H | H | H | H | H | H |
| CF₃ | COCH₂Ph | O | F | H | H | H | H | H | H | H |

**[Table 10]**

| R¹ | R² | W | X¹ | X² | X³ | X⁴ | Y¹ | Y² | Y³ | Y⁴ |
|---|---|---|---|---|---|---|---|---|---|---|
| CF₃ | COCH₂(4-ClPh) | O | F | H | H | H | H | H | H | H |
| CF₃ | COCH₂(4-MePh) | O | F | H | H | H | H | H | H | H |
| CF₃ | CO(2-tetrahydrofuryl) | O | F | H | H | H | H | H | H | H |
| CF₃ | CO(3-tetrahydrofuryl) | O | F | H | H | H | H | H | H | H |
| CF₃ | CO(2-pyridyl) | O | F | H | H | H | H | H | H | H |
| CF₃ | CO(3-pyridyl) | O | F | H | H | H | H | H | H | H |
| CF₃ | CO(4-pyridyl) | O | F | H | H | H | H | H | H | H |
| CF₃ | CO(2-thienyl) | O | F | H | H | H | H | H | H | H |
| CF₃ | CO(3-thienyl) | O | F | H | H | H | H | H | H | H |
| CF₃ | CO(2-tetrahydrofurfuryl) | O | F | H | H | H | H | H | H | H |
| CF₃ | COCH₂(2-pyridyl) | O | F | H | H | H | H | H | H | H |
| CF₃ | COCH₂(2-thienyl) | O | F | H | H | H | H | H | H | H |
| CF₃ | CO₂Me | O | F | H | H | H | H | H | H | H |
| CF₃ | CO₂Et | O | F | H | H | H | H | H | H | H |
| CF₃ | CO₂n-Pr | O | F | H | H | H | H | H | H | H |
| CF₃ | CO₂i-Pr | O | F | H | H | H | H | H | H | H |
| CF₃ | CO₂n-Bu | O | F | H | H | H | H | H | H | H |
| CF₃ | CO₂s-Bu | O | F | H | H | H | H | H | H | H |
| CF₃ | CO₂i-Bu | O | F | H | H | H | H | H | H | H |
| CF₃ | CO₂t-Bu | O | F | H | H | H | H | H | H | H |
| CF₃ | CO₂n-Pen | O | F | H | H | H | H | H | H | H |
| CF₃ | CO₂n-Hex | O | F | H | H | H | H | H | H | H |
| CF₃ | CO₂CH₂CH₂F | O | F | H | H | H | H | H | H | H |
| CF₃ | CO₂CH₂CHF₂ | O | F | H | H | H | H | H | H | H |
| CF₃ | CO₂CH₂CF₃ | O | F | H | H | H | H | H | H | H |
| CF₃ | CO₂CH=CH₂ | O | F | H | H | H | H | H | H | H |
| CF₃ | CO₂C≡CH | O | F | H | H | H | H | H | H | H |
| CF₃ | CO₂c-Pr | O | F | H | H | H | H | H | H | H |
| CF₃ | CO₂CH₂c-Pr | O | F | H | H | H | H | H | H | H |

**[Table 11]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂CH₂CH₂OMe | O | F | H | H | H | H | H | H | H |
| CF₃ | CO₂CH₂CH₂OCH₂CF₃ | O | F | H | H | H | H | H | H | H |
| CF₃ | CO₂CH₂CH₂OCH₂CH₂OMe | O | F | H | H | H | H | H | H | H |
| CF₃ | CO₂CH₂CH₂SMe | O | F | H | H | H | H | H | H | H |
| CF₃ | CO₂CH₂CH₂SCH₂CF₃ | O | F | H | H | H | H | H | H | H |
| CF₃ | CO₂Ph | O | F | H | H | H | H | H | H | H |
| CF₃ | CO₂(4-ClPh) | O | F | H | H | H | H | H | H | H |
| CF₃ | CO₂(4-MePh) | O | F | H | H | H | H | H | H | H |
| CF₃ | CO₂CH₂Ph | O | F | H | H | H | H | H | H | H |
| CF₃ | CO₂CH₂(4-ClPh) | O | F | H | H | H | H | H | H | H |
| CF₃ | CO₂CH₂(4-MePh) | O | F | H | H | H | H | H | H | H |
| CF₃ | CO₂(3-oxetanyl) | O | F | H | H | H | H | H | H | H |
| CF₃ | CO₂(2-tetrahydrofuryl) | O | F | H | H | H | H | H | H | H |
| CF₃ | CO₂(3-tetrahydrofuryl) | O | F | H | H | H | H | H | H | H |
| CF₃ | CO₂(2-pyridyl) | O | F | H | H | H | H | H | H | H |
| CF₃ | CO₂(2-thienyl) | O | F | H | H | H | H | H | H | H |
| CF₃ | CO₂(2-tetrahydrofurfuryl) | O | F | H | H | H | H | H | H | H |
| CF₃ | CO₂CH₂(2-pyridyl) | O | F | H | H | H | H | H | H | H |
| CF₃ | CO₂CH₂(2-thienyl) | O | F | H | H | H | H | H | H | H |
| CF₃ | CO(SMe) | O | F | H | H | H | H | H | H | H |
| CF₃ | CO(SEt) | O | F | H | H | H | H | H | H | H |
| CF₃ | CO(SCF₃) | O | F | H | H | H | H | H | H | H |
| CF₃ | CO(SCH₂CF₃) | O | F | H | H | H | H | H | H | H |
| CF₃ | CONHEt | O | F | H | H | H | H | H | H | H |
| CF₃ | CONHCH₂CF₃ | O | F | H | H | H | H | H | H | H |
| CF₃ | CONEt₂ | O | F | H | H | H | H | H | H | H |
| CF₃ | SO₂Me | O | F | H | H | H | H | H | H | H |
| CF₃ | SO₂Et | O | F | H | H | H | H | H | H | H |
| CF₃ | SO₂n-Pr | O | F | H | H | H | H | H | H | H |

**[Table 12]**

| R¹ | R² | W | X¹ | X² | X³ | X⁴ | Y¹ | Y² | Y³ | Y⁴ |
|---|---|---|---|---|---|---|---|---|---|---|
| CF₃ | SO₂i-Pr | O | F | H | H | H | H | H | H | H |
| CF₃ | SO₂n-Bu | O | F | H | H | H | H | H | H | H |
| CF₃ | SO₂i-Bu | O | F | H | H | H | H | H | H | H |
| CF₃ | SO₂CH₂Cl | O | F | H | H | H | H | H | H | H |
| CF₃ | SO₂CCl₃ | O | F | H | H | H | H | H | H | H |
| CF₃ | SO₂CHF₂ | O | F | H | H | H | H | H | H | H |
| CF₃ | SO₂CF₃ | O | F | H | H | H | H | H | H | H |
| CF₃ | SO₂CH₂CF₃ | O | F | H | H | H | H | H | H | H |
| CF₃ | SO₂CH=CH₂ | O | F | H | H | H | H | H | H | H |
| CF₃ | SO₂CH₂CH=CH₂ | O | F | H | H | H | H | H | H | H |
| CF₃ | SO₂CH₂C≡CH | O | F | H | H | H | H | H | H | H |
| CF₃ | SO₂c-Pr | O | F | H | H | H | H | H | H | H |
| CF₃ | SO₂c-Hex | O | F | H | H | H | H | H | H | H |
| CF₃ | SO₂CH₂c-Pr | O | F | H | H | H | H | H | H | H |
| CF₃ | SO₂CH₂CH₂OMe | O | F | H | H | H | H | H | H | H |
| CF₃ | SO₂Ph | O | F | H | H | H | H | H | H | H |
| CF₃ | SO₂(4-ClPh) | O | F | H | H | H | H | H | H | H |
| CF₃ | SO₂(4-MePh) | O | F | H | H | H | H | H | H | H |
| CF₃ | SO₂CH₂Ph | O | F | H | H | H | H | H | H | H |
| CF₃ | SO₂CH₂(4-ClPh) | O | F | H | H | H | H | H | H | H |
| CF₃ | SO₂CH₂(4-MePh) | O | F | H | H | H | H | H | H | H |
| CF₃ | SO₂NHMe | O | F | H | H | H | H | H | H | H |
| CF₃ | SO₂NMe₂ | O | F | H | H | H | H | H | H | H |
| CF₃ | H | O | F | H | H | F | H | H | H | H |
| CF₃ | Me | O | F | H | H | F | H | H | H | H |
| CF₃ | Et | O | F | H | H | F | H | H | H | H |
| CF₃ | CH₂CF₃ | O | F | H | H | F | H | H | H | H |
| CF₃ | CH₂CH=CH₂ | O | F | H | H | F | H | H | H | H |
| CF₃ | CH₂C≡CH | O | F | H | H | F | H | H | H | H |

**[Table 13]**

| R¹ | R² | W | X¹ | X² | X³ | X⁴ | Y¹ | Y² | Y³ | Y⁴ |
|---|---|---|---|---|---|---|---|---|---|---|
| CF₃ | CH₂OMe | O | F | H | H | F | H | H | H | H |
| CF₃ | CH₂OCH₂CF₃ | O | F | H | H | F | H | H | H | H |
| CF₃ | CH₂OCH₂CH₂OMe | O | F | H | H | F | H | H | H | H |
| CF₃ | CH₂SMe | O | F | H | H | F | H | H | H | H |
| CF₃ | CH₂COMe | O | F | H | H | F | H | H | H | H |
| CF₃ | CH₂Ph | O | F | H | H | F | H | H | H | H |
| CF₃ | CH₂(4-ClPh) | O | F | H | H | F | H | H | H | H |
| CF₃ | CH₂(4-MePh) | O | F | H | H | F | H | H | H | H |
| CF₃ | CH₂(4-MeOPh) | O | F | H | H | F | H | H | H | H |
| CF₃ | CH₂CH₂OPh | O | F | H | H | F | H | H | H | H |
| CF₃ | COMe | O | F | H | H | F | H | H | H | H |
| CF₃ | COEt | O | F | H | H | F | H | H | H | H |
| CF₃ | COn-Pr | O | F | H | H | F | H | H | H | H |
| CF₃ | COi-Pr | O | F | H | H | F | H | H | H | H |
| CF₃ | COn-Bu | O | F | H | H | F | H | H | H | H |
| CF₃ | COs-Bu | O | F | H | H | F | H | H | H | H |
| CF₃ | COi-Bu | O | F | H | H | F | H | H | H | H |
| CF₃ | COt-Bu | O | F | H | H | F | H | H | H | H |
| CF₃ | COn-Pen | O | F | H | H | F | H | H | H | H |
| CF₃ | COn-Hex | O | F | H | H | F | H | H | H | H |
| CF₃ | COCF₃ | O | F | H | H | F | H | H | H | H |
| CF₃ | COCH₂CF₃ | O | F | H | H | F | H | H | H | H |
| CF₃ | COCH=CH₂ | O | F | H | H | F | H | H | H | H |
| CF₃ | COC≡CH | O | F | H | H | F | H | H | H | H |
| CF₃ | COc-Pr | O | F | H | H | F | H | H | H | H |
| CF₃ | COc-Bu | O | F | H | H | F | H | H | H | H |
| CF₃ | COc-Pen | O | F | H | H | F | H | H | H | H |
| CF₃ | COc-Hex | O | F | H | H | F | H | H | H | H |
| CF₃ | COCH₂c-Pr | O | F | H | H | F | H | H | H | H |

**[Table 14]**

| R¹ | R² | W | X¹ | X² | X³ | X⁴ | Y¹ | Y² | Y³ | Y⁴ |
|---|---|---|---|---|---|---|---|---|---|---|
| CF₃ | COCH₂OMe | O | F | H | H | F | H | H | H | H |
| CF₃ | COCH₂OCH₂CF₃ | O | F | H | H | F | H | H | H | H |
| CF₃ | COCH₂OCH₂CH₂CF₃ | O | F | H | H | F | H | H | H | H |
| CF₃ | COCH₂SMe | O | F | H | H | F | H | H | H | H |
| CF₃ | COCH₂SCH₂CF₃ | O | F | H | H | F | H | H | H | H |
| CF₃ | Bz | O | F | H | H | F | H | H | H | H |
| CF₃ | 4-ClBz | O | F | H | H | F | H | H | H | H |
| CF₃ | 4-MeBz | O | F | H | H | F | H | H | H | H |
| CF₃ | COCH₂Ph | O | F | H | H | F | H | H | H | H |
| CF₃ | COCH₂(4-ClPh) | O | F | H | H | F | H | H | H | H |
| CF₃ | COCH₂(4-MePh) | O | F | H | H | F | H | H | H | H |
| CF₃ | CO(2-tetrahydrofuryl) | O | F | H | H | F | H | H | H | H |
| CF₃ | CO(3-tetrahydrofuryl) | O | F | H | H | F | H | H | H | H |
| CF₃ | CO(2-pyridyl) | O | F | H | H | F | H | H | H | H |
| CF₃ | CO(3-pyridyl) | O | F | H | H | F | H | H | H | H |
| CF₃ | CO(4-pyridyl) | O | F | H | H | F | H | H | H | H |
| CF₃ | CO(2-thienyl) | O | F | H | H | F | H | H | H | H |
| CF₃ | CO(3-thienyl) | O | F | H | H | F | H | H | H | H |
| CF₃ | CO(2-tetrahydrofurfuryl) | O | F | H | H | F | H | H | H | H |
| CF₃ | COCH₂(2-pyridyl) | O | F | H | H | F | H | H | H | H |
| CF₃ | COCH₂(2-thienyl) | O | F | H | H | F | H | H | H | H |
| CF₃ | CO₂Me | O | F | H | H | F | H | H | H | H |
| CF₃ | CO₂Et | O | F | H | H | F | H | H | H | H |
| CF₃ | CO₂n-Pr | O | F | H | H | F | H | H | H | H |
| CF₃ | CO₂i-Pr | O | F | H | H | F | H | H | H | H |
| CF₃ | CO₂n-Bu | O | F | H | H | F | H | H | H | H |
| CF₃ | CO₂s-Bu | O | F | H | H | F | H | H | H | H |
| CF₃ | CO₂i-Bu | O | F | H | H | F | H | H | H | H |
| CF₃ | CO₂t-Bu | O | F | H | H | F | H | H | H | H |

**[Table 15]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂n-Pen | O | F | H | H | F | H | H | H | H |
| CF₃ | CO₂n-Hex | O | F | H | H | F | H | H | H | H |
| CF₃ | CO₂CH₂CH₂F | O | F | H | H | F | H | H | H | H |
| CF₃ | CO₂CH₂CHF₂ | O | F | H | H | F | H | H | H | H |
| CF₃ | CO₂CH₂CF₃ | O | F | H | H | F | H | H | H | H |
| CF₃ | CO₂CH=CH₂ | O | F | H | H | F | H | H | H | H |
| CF₃ | CO₂C≡CH | O | F | H | H | F | H | H | H | H |
| CF₃ | CO₂c-Pr | O | F | H | H | F | H | H | H | H |
| CF₃ | CO₂CH₂c-Pr | O | F | H | H | F | H | H | H | H |
| CF₃ | CO₂CH₂CH₂OMe | O | F | H | H | F | H | H | H | H |
| CF₃ | CO₂CH₂CH₂OCH₂CF₃ | O | F | H | H | F | H | H | H | H |
| CF₃ | CO₂CH₂CH₂OCH₂CH₂OMe | O | F | H | H | F | H | H | H | H |
| CF₃ | CO₂CH₂CH₂SMe | O | F | H | H | F | H | H | H | H |
| CF₃ | CO₂CH₂CH₂SCH₂CF₃ | O | F | H | H | F | H | H | H | H |
| CF₃ | CO₂Ph | O | F | H | H | F | H | H | H | H |
| CF₃ | CO₂(4-ClPh) | O | F | H | H | F | H | H | H | H |
| CF₃ | CO₂(4-MePh) | O | F | H | H | F | H | H | H | H |
| CF₃ | CO₂CH₂Ph | O | F | H | H | F | H | H | H | H |
| CF₃ | CO₂CH₂(4-ClPh) | O | F | H | H | F | H | H | H | H |
| CF₃ | CO₂CH₂(4-MePh) | O | F | H | H | F | H | H | H | H |
| CF₃ | CO₂(3-oxetanyl) | O | F | H | H | F | H | H | H | H |
| CF₃ | CO₂(2-tetrahydrofuryl) | O | F | H | H | F | H | H | H | H |
| CF₃ | CO₂(3-tetrahydrofuryl) | O | F | H | H | F | H | H | H | H |
| CF₃ | CO₂(2-pyridyl) | O | F | H | H | F | H | H | H | H |
| CF₃ | CO₂(2-thienyl) | O | F | H | H | F | H | H | H | H |
| CF₃ | CO₂(2-tetrahydrofurfuryl) | O | F | H | H | F | H | H | H | H |
| CF₃ | CO₂CH₂(2-pyridyl) | O | F | H | H | F | H | H | H | H |
| CF₃ | CO₂CH₂(2-thienyl) | O | F | H | H | F | H | H | H | H |
| CF₃ | CO(SMe) | O | F | H | H | F | H | H | H | H |

**[Table 16]**

| R¹ | R² | W | X¹ | X² | X³ | X⁴ | Y¹ | Y² | Y³ | Y⁴ |
|---|---|---|---|---|---|---|---|---|---|---|
| CF₃ | CO(SEt) | O | F | H | H | F | H | H | H | H |
| CF₃ | CO(SCF₃) | O | F | H | H | F | H | H | H | H |
| CF₃ | CO(SCH₂CF₃) | O | F | H | H | F | H | H | H | H |
| CF₃ | CONHEt | O | F | H | H | F | H | H | H | H |
| CF₃ | CONHCH₂CF₃ | O | F | H | H | F | H | H | H | H |
| CF₃ | CONEt₂ | O | F | H | H | F | H | H | H | H |
| CF₃ | SO₂Me | O | F | H | H | F | H | H | H | H |
| CF₃ | SO₂Et | O | F | H | H | F | H | H | H | H |
| CF₃ | SO₂n-Pr | O | F | H | H | F | H | H | H | H |
| CF₃ | SO₂i-Pr | O | F | H | H | F | H | H | H | H |
| CF₃ | SO₂n-Bu | O | F | H | H | F | H | H | H | H |
| CF₃ | SO₂i-Bu | O | F | H | H | F | H | H | H | H |
| CF₃ | SO₂CH₂Cl | O | F | H | H | F | H | H | H | H |
| CF₃ | SO₂CCl₃ | O | F | H | H | F | H | H | H | H |
| CF₃ | SO₂CHF₂ | O | F | H | H | F | H | H | H | H |
| CF₃ | SO₂CF₃ | O | F | H | H | F | H | H | H | H |
| CF₃ | SO₂CH₂CF₃ | O | F | H | H | F | H | H | H | H |
| CF₃ | SO₂CH=CH₂ | O | F | H | H | F | H | H | H | H |
| CF₃ | SO₂CH₂CH=CH₂ | O | F | H | H | F | H | H | H | H |
| CF₃ | SO₂CH₂C≡CH | O | F | H | H | F | H | H | H | H |
| CF₃ | SO₂c-Pr | O | F | H | H | F | H | H | H | H |
| CF₃ | SO₂c-Hex | O | F | H | H | F | H | H | H | H |
| CF₃ | SO₂CH₂c-Pr | O | F | H | H | F | H | H | H | H |
| CF₃ | SO₂CH₂CH₂OMe | O | F | H | H | F | H | H | H | H |
| CF₃ | SO₂Ph | O | F | H | H | F | H | H | H | H |
| CF₃ | SO₂(4-ClPh) | O | F | H | H | F | H | H | H | H |
| CF₃ | SO₂(4-MePh) | O | F | H | H | F | H | H | H | H |
| CF₃ | SO₂CH₂Ph | O | F | H | H | F | H | H | H | H |
| CF₃ | SO₂CH₂(4-ClPh) | O | F | H | H | F | H | H | H | H |

**[Table 17]**

| R¹ | R² | W | X¹ | X² | X³ | X⁴ | Y¹ | Y² | Y³ | Y⁴ |
|---|---|---|---|---|---|---|---|---|---|---|
| CF₃ | SO₂CH₂(4-MePh) | O | F | H | H | F | H | H | H | H |
| CF₃ | SO₂NHMe | O | F | H | H | F | H | H | H | H |
| CF₃ | SO₂NMe₂ | O | F | H | H | F | H | H | H | H |
| CF₃ | COc-Pr | O | Cl | H | H | H | H | H | H | H |
| CF₃ | COc-Pr | O | Br | H | H | H | H | H | H | H |
| CF₃ | COc-Pr | O | I | H | H | H | H | H | H | H |
| CF₃ | COc-Pr | O | Me | H | H | H | H | H | H | H |
| CF₃ | COc-Pr | O | Et | H | H | H | H | H | H | H |
| CF₃ | COc-Pr | O | n-Pr | H | H | H | H | H | H | H |
| CF₃ | COc-Pr | O | i-Pr | H | H | H | H | H | H | H |
| CF₃ | COc-Pr | O | n-Bu | H | H | H | H | H | H | H |
| CF₃ | COc-Pr | O | s-Bu | H | H | H | H | H | H | H |
| CF₃ | COc-Pr | O | i-Bu | H | H | H | H | H | H | H |
| CF₃ | COc-Pr | O | t-Bu | H | H | H | H | H | H | H |
| CF₃ | COc-Pr | O | c-Pr | H | H | H | H | H | H | H |
| CF₃ | COc-Pr | O | c-Bu | H | H | H | H | H | H | H |
| CF₃ | COc-Pr | O | OMe | H | H | H | H | H | H | H |
| CF₃ | COc-Pr | O | OEt | H | H | H | H | H | H | H |
| CF₃ | COc-Pr | O | OCHF₂ | H | H | H | H | H | H | H |
| CF₃ | COc-Pr | O | OCF₃ | H | H | H | H | H | H | H |
| CF₃ | COc-Pr | O | OCH₂CH=CH₂ | H | H | H | H | H | H | H |
| CF₃ | COc-Pr | O | OCH₂C≡CH | H | H | H | H | H | H | H |
| CF₃ | COc-Pr | O | OCH₂c-Pr | H | H | H | H | H | H | H |
| CF₃ | COc-Pr | O | Bn | H | H | H | H | H | H | H |
| CF₃ | COc-Pr | O | 4-ClBn | H | H | H | H | H | H | H |
| CF₃ | COc-Pr | O | 4-MeBn | H | H | H | H | H | H | H |
| CF₃ | COc-Pr | O | 4-OMeBn | H | H | H | H | H | H | H |
| CF₃ | COc-Pr | O | OAc | H | H | H | H | H | H | H |
| CF₃ | COc-Pr | O | OBz | H | H | H | H | H | H | H |
| CF₃ | COc-Pr | O | O(4-ClBz) | H | H | H | H | H | H | H |

**[Table 18]**

| R¹ | R² | W | X¹ | X² | X³ | X⁴ | Y¹ | Y² | Y³ | Y⁴ |
|---|---|---|---|---|---|---|---|---|---|---|
| CF₃ | COc-Pr | O | OCO₂Et | H | H | H | H | H | H | H |
| CF₃ | COc-Pr | O | OCO(SMe) | H | H | H | H | H | H | H |
| CF₃ | COc-Pr | O | OSO₂Me | H | H | H | H | H | H | H |
| CF₃ | COc-Pr | O | OSO₂CF₃ | H | H | H | H | H | H | H |
| CF₃ | COc-Pr | O | OSO₂Ph | H | H | H | H | H | H | H |
| CF₃ | COc-Pr | O | OPO(OEt)₂ | H | H | H | H | H | H | H |
| CF₃ | COc-Pr | O | OCH₂OMe | H | H | H | H | H | H | H |
| CF₃ | COc-Pr | O | SMe | H | H | H | H | H | H | H |
| CF₃ | COc-Pr | O | SEt | H | H | H | H | H | H | H |
| CF₃ | COc-Pr | O | SCF₃ | H | H | H | H | H | H | H |
| CF₃ | COc-Pr | O | SO₂Me | H | H | H | H | H | H | H |
| CF₃ | COc-Pr | O | SO₂CF₃ | H | H | H | H | H | H | H |
| CF₃ | COc-Pr | O | Ph | H | H | H | H | H | H | H |
| CF₃ | COc-Pr | O | 4-CIPh | H | H | H | H | H | H | H |
| CF₃ | COc-Pr | O | 4-MePh | H | H | H | H | H | H | H |
| CF₃ | COc-Pr | O | NMe₂ | H | H | H | H | H | H | H |
| CF₃ | COc-Pr | O | NHAc | H | H | H | H | H | H | H |
| CF₃ | COc-Pr | O | NAc₂ | H | H | H | H | H | H | H |
| CF₃ | COc-Pr | O | NHSO₂CF₃ | H | H | H | H | H | H | H |
| CF₃ | COc-Pr | O | N-morpholinyl | H | H | H | H | H | H | H |
| CF₃ | COc-Pr | O | CO₂Me | H | H | H | H | H | H | H |
| CF₃ | COc-Pr | O | CO₂Et | H | H | H | H | H | H | H |
| CF₃ | COc-Pr | O | OH | H | H | H | H | H | H | H |
| CF₃ | COc-Pr | O | CN | H | H | H | H | H | H | H |
| CF₃ | COc-Pr | O | NO₂ | H | H | H | H | H | H | H |
| CF₃ | COc-Pr | O | H | F | H | H | H | H | H | H |
| CF₃ | COc-Pr | O | H | Cl | H | H | H | H | H | H |
| CF₃ | COc-Pr | O | H | Br | H | H | H | H | H | H |
| CF₃ | COc-Pr | O | H | I | H | H | H | H | H | H |
| CF₃ | COc-Pr | O | H | Me | H | H | H | H | H | H |

**[Table 19]**

| R¹ | R² | W | X¹ | X² | X³ | X⁴ | Y¹ | Y² | Y³ | Y⁴ |
|---|---|---|---|---|---|---|---|---|---|---|
| CF₃ | COc-Pr | O | H | c-Pr | H | H | H | H | H | H |
| CF₃ | COc-Pr | O | H | OMe | H | H | H | H | H | H |
| CF₃ | COc-Pr | O | H | OCF₃ | H | H | H | H | H | H |
| CF₃ | COc-Pr | O | H | OCH₂C≡CH | H | H | H | H | H | H |
| CF₃ | COc-Pr | O | H | Bn | H | H | H | H | H | H |
| CF₃ | COc-Pr | O | H | OAc | H | H | H | H | H | H |
| CF₃ | COc-Pr | O | H | OSO₂Me | H | H | H | H | H | H |
| CF₃ | COc-Pr | O | H | SMe | H | H | H | H | H | H |
| CF₃ | COc-Pr | O | H | SO₂Me | H | H | H | H | H | H |
| CF₃ | COc-Pr | O | H | Ph | H | H | H | H | H | H |
| CF₃ | COc-Pr | O | H | NMe₂ | H | H | H | H | H | H |
| CF₃ | COc-Pr | O | H | CN | H | H | H | H | H | H |
| CF₃ | COc-Pr | O | H | NO₂ | H | H | H | H | H | H |
| CF₃ | COc-Pr | O | H | H | F | H | H | H | H | H |
| CF₃ | COc-Pr | O | H | H | Cl | H | H | H | H | H |
| CF₃ | COc-Pr | O | H | H | Br | H | H | H | H | H |
| CF₃ | COc-Pr | O | H | H | I | H | H | H | H | H |
| CF₃ | COc-Pr | O | H | H | Me | H | H | H | H | H |
| CF₃ | COc-Pr | O | H | H | c-Pr | H | H | H | H | H |
| CF₃ | COc-Pr | O | H | H | OMe | H | H | H | H | H |
| CF₃ | COc-Pr | O | H | H | OCF₃ | H | H | H | H | H |
| CF₃ | COc-Pr | O | H | H | OCH₁C≡CH | H | H | H | H | H |
| CF₃ | COc-Pr | O | H | H | Bn | H | H | H | H | H |
| CF₃ | COc-Pr | O | H | H | OAc | H | H | H | H | H |
| CF₃ | COc-Pr | O | H | H | OSO₂Me | H | H | H | H | H |
| CF₃ | COc-Pr | O | H | H | SMe | H | H | H | H | H |
| CF₃ | COc-Pr | O | H | H | SO₂Me | H | H | H | H | H |
| CF₃ | COc-Pr | O | H | H | Ph | H | H | H | H | H |
| CF₃ | COc-Pr | O | H | H | NMe₂ | H | H | H | H | H |
| CF₃ | COc-Pr | O | H | H | CN | H | H | H | H | H |

**[Table 20]**

| R¹ | R² | W | X¹ | X² | X³ | X⁴ | Y¹ | Y² | Y³ | Y⁴ |
|---|---|---|---|---|---|---|---|---|---|---|
| CF₃ | COc-Pr | O | H | H | NO₂ | H | H | H | H | H |
| CF₃ | COc-Pr | O | H | H | H | F | H | H | H | H |
| CF₃ | COc-Pr | O | H | H | H | Cl | H | H | H | H |
| CF₃ | COc-Pr | O | H | H | H | Br | H | H | H | H |
| CF₃ | COc-Pr | O | H | H | H | I | H | H | H | H |
| CF₃ | COc-Pr | O | H | H | H | Me | H | H | H | H |
| CF₃ | COc-Pr | O | H | H | H | c-Pr | H | H | H | H |
| CF₃ | COc-Pr | O | H | H | H | OMe | H | H | H | H |
| CF₃ | COc-Pr | O | H | H | H | OCF₃ | H | H | H | H |
| CF₃ | COc-Pr | O | H | H | H | OCH₂C ≡ CH | H | H | H | H |
| CF₃ | COc-Pr | O | H | H | H | Bn | H | H | H | H |
| CF₃ | COc-Pr | O | H | H | H | OAc | H | H | H | H |
| CF₃ | COc-Pr | O | H | H | H | OSO₂Me | H | H | H | H |
| CF₃ | COc-Pr | O | H | H | H | SMe | H | H | H | H |
| CF₃ | COc-Pr | O | H | H | H | SO₂Me | H | H | H | H |
| CF₃ | COc-Pr | O | H | H | H | Ph | H | H | H | H |
| CF₃ | COc-Pr | O | H | H | H | NMe₂ | H | H | H | H |
| CF₃ | COc-Pr | O | H | H | H | CN | H | H | H | H |
| CF₃ | COc-Pr | O | H | H | H | NO₂ | H | H | H | H |
| CF₃ | COc-Pr | O | F | F | H | H | H | H | H | H |
| CF₃ | COc-Pr | O | F | H | F | H | H | H | H | H |
| CF₃ | COc-Pr | O | F | Cl | H | H | H | H | H | H |
| CF₃ | COc-Pr | O | F | H | Cl | H | H | H | H | H |
| CF₃ | COc-Pr | O | F | H | H | Cl | H | H | H | H |
| CF₃ | COc-Pr | O | F | H | H | Br | H | H | H | H |
| CF₃ | COc-Pr | O | F | H | H | I | H | H | H | H |
| CF₃ | COc-Pr | O | F | H | H | Me | H | H | H | H |
| CF₃ | COc-Pr | O | F | H | H | c-Pr | H | H | H | H |
| CF₃ | COc-Pr | O | F | H | H | OMe | H | H | H | H |
| CF₃ | COc-Pr | O | F | H | H | OCF₃ | H | H | H | H |

**[Table 21]**

| R¹ | R² | W | X¹ | X² | X³ | X⁴ | Y¹ | Y² | Y³ | Y⁴ |
|---|---|---|---|---|---|---|---|---|---|---|
| CF₃ | COc-Pr | O | F | H | H | OCH₂C ≡ CH | H | H | H | H |
| CF₃ | COc-Pr | O | F | H | H | OSO₂Me | H | H | H | H |
| CF₃ | COc-Pr | O | F | H | H | SMe | H | H | H | H |
| CF₃ | COc-Pr | O | F | H | H | SO₂Me | H | H | H | H |
| CF₃ | COc-Pr | O | F | H | H | NMe₂ | H | H | H | H |
| CF₃ | COc-Pr | O | F | H | H | CN | H | H | H | H |
| CF₃ | COc-Pr | O | F | H | H | NO₂ | H | H | H | H |
| CF₃ | COc-Pr | O | F | F | F | H | H | H | H | H |
| CF₃ | COc-Pr | O | F | F | H | F | H | H | H | H |
| CF₃ | COc-Pr | O | F | H | F | F | H | H | H | H |
| CF₃ | COc-Pr | O | F | F | F | F | H | H | H | H |
| CF₃ | COc-Pr | O | Cl | F | H | H | H | H | H | H |
| CF₃ | COc-Pr | O | Cl | H | F | H | H | H | H | H |
| CF₃ | COc-Pr | O | Cl | H | H | F | H | H | H | H |
| CF₃ | COc-Pr | O | Cl | Cl | H | H | H | H | H | H |
| CF₃ | COc-Pr | O | Cl | H | Cl | H | H | H | H | H |
| CF₃ | COc-Pr | O | Cl | H | H | Cl | H | H | H | H |
| CF₃ | COc-Pr | O | Cl | H | H | Br | H | H | H | H |
| CF₃ | COc-Pr | O | Cl | H | H | I | H | H | H | H |
| CF₃ | COc-Pr | O | Cl | H | H | Me | H | H | H | H |
| CF₃ | COc-Pr | O | Cl | H | H | c-Pr | H | H | H | H |
| CF₃ | COc-Pr | O | Cl | H | H | OMe | H | H | H | H |
| CF₃ | COc-Pr | O | Cl | H | H | OCF₃ | H | H | H | H |
| CF₃ | COc-Pr | O | Cl | H | H | OCH₂C ≡ CH | H | H | H | H |
| CF₃ | COc-Pr | O | Cl | H | H | OSO₂Me | H | H | H | H |
| CF₃ | COc-Pr | O | Cl | H | H | SMe | H | H | H | H |
| CF₃ | COc-Pr | O | Cl | H | H | SO₂Me | H | H | H | H |
| CF₃ | COc-Pr | O | Cl | H | H | NMe₂ | H | H | H | H |
| CF₃ | COc-Pr | O | Cl | H | H | CN | H | H | H | H |
| CF₃ | COc-Pr | O | Cl | H | H | NO₂ | H | H | H | H |

**[Table 22]**

| R¹ | R² | W | X¹ | X² | X³ | X⁴ | Y¹ | Y² | Y³ | Y⁴ |
|---|---|---|---|---|---|---|---|---|---|---|
| CF₃ | COc-Pr | O | Br | H | H | F | H | H | H | H |
| CF₃ | COc-Pr | O | Br | H | H | Cl | H | H | H | H |
| CF₃ | COc-Pr | O | Br | H | H | Me | H | H | H | H |
| CF₃ | COc-Pr | O | Br | H | H | OMe | H | H | H | H |
| CF₃ | COc-Pr | O | Me | H | H | F | H | H | H | H |
| CF₃ | COc-Pr | O | Me | H | H | Cl | H | H | H | H |
| CF₃ | COc-Pr | O | Me | H | H | Me | H | H | H | H |
| CF₃ | COc-Pr | O | Me | H | H | OMe | H | H | H | H |
| CF₃ | COc-Pr | O | OMe | H | H | F | H | H | H | H |
| CF₃ | COc-Pr | O | OMe | H | H | Cl | H | H | H | H |
| CF₃ | COc-Pr | O | OMe | H | H | Me | H | H | H | H |
| CF₃ | COc-Pr | O | OMe | H | H | OMe | H | H | H | H |
| CF₃ | COc-Pr | O | H | F | F | H | H | H | H | H |
| CF₃ | COc-Pr | O | H | F | Cl | H | H | H | H | H |
| CF₃ | COc-Pr | O | H | F | H | F | H | H | H | H |
| CF₃ | COc-Pr | O | H | F | H | Cl | H | H | H | H |
| CF₃ | COc-Pr | O | H | Cl | Cl | H | H | H | H | H |
| CF₃ | COc-Pr | O | H | Cl | F | H | H | H | H | H |
| CF₃ | COc-Pr | O | H | Cl | H | F | H | H | H | H |
| CF₃ | COc-Pr | O | H | Cl | H | Cl | H | H | H | H |
| CF₃ | COc-Pr | O | H | H | F | F | H | H | H | H |
| CF₃ | COc-Pr | O | H | H | F | Cl | H | H | H | H |
| CF₃ | COc-Pr | O | H | H | Cl | Cl | H | H | H | H |
| CF₃ | COc-Pr | O | F | F | F | F | H | H | H | H |
| CF₃ | COc-Pr | O | Cl | Cl | Cl | Cl | H | H | H | H |
| CF₃ | COc-Pr | O | -CH=CH-CH=CH- | | H | H | H | H | H | H |
| CF₃ | COc-Pr | O | -CH=N-CH=CH- | | H | H | H | H | H | H |
| CF₃ | COc-Pr | O | -S-CH=N- | | H | H | H | H | H | H |
| CF₃ | COc-Pr | O | -N=CH-S- | | H | H | H | H | H | H |
| CF₃ | COc-Pr | O | H | -CH=CH-CH=CH- | | H | H | H | H | H |

**[Table 23]**

| R¹ | R² | W | X¹ | X² | X³ | X⁴ | Y¹ | Y² | Y³ | Y⁴ |
|---|---|---|---|---|---|---|---|---|---|---|
| CF₃ | COc-Pr | O | H | -CH=N-CH=CH- | | H | H | H | H | H |
| CF₃ | COc-Pr | O | H | -S-CH=N- | | H | H | H | H | H |
| CF₃ | COc-Pr | O | H | -N=CH-S- | | H | H | H | H | H |
| CF₃ | COc-Pr | O | H | H | -CH=CH-CH=CH- | | H | H | H | H |
| CF₃ | COc-Pr | O | H | H | -CH=N-CH=CH- | | H | H | H | H |
| CF₃ | COc-Pr | O | H | H | -S-CH=N- | | H | H | H | H |
| CF₃ | COc-Pr | O | H | H | -N=CH-S- | | H | H | H | H |
| CF₃ | CO₂Et | O | Cl | H | H | H | H | H | H | H |
| CF₃ | CO₂Et | O | Br | H | H | H | H | H | H | H |
| CF₃ | CO₂Et | O | I | H | H | H | H | H | H | H |
| CF₃ | CO₂Et | O | Me | H | H | H | H | H | H | H |
| CF₃ | CO₂Et | O | Et | H | H | H | H | H | H | H |
| CF₃ | CO₂Et | O | n-Pr | H | H | H | H | H | H | H |
| CF₃ | CO₂Et | O | i-Pr | H | H | H | H | H | H | H |
| CF₃ | CO₂Et | O | n-Bu | H | H | H | H | H | H | H |
| CF₃ | CO₂Et | O | s-Bu | H | H | H | H | H | H | H |
| CF₃ | CO₂Et | O | i-Bu | H | H | H | H | H | H | H |
| CF₃ | CO₂Et | O | t-Bu | H | H | H | H | H | H | H |
| CF₃ | CO₂Et | O | c-Pr | H | H | H | H | H | H | H |
| CF₃ | CO₂Et | O | c-Bu | H | H | H | H | H | H | H |
| CF₃ | CO₂Et | O | OMe | H | H | H | H | H | H | H |
| CF₃ | CO₂Et | O | OEt | H | H | H | H | H | H | H |
| CF₃ | CO₂Et | O | OCHF₂ | H | H | H | H | H | H | H |
| CF₃ | CO₂Et | O | OCF₃ | H | H | H | H | H | H | H |
| CF₃ | CO₂Et | O | OCH₂CH=CH₂ | H | H | H | H | H | H | H |
| CF₃ | CO₂Et | O | OCH₂C≡CH | H | H | H | H | H | H | H |
| CF₃ | CO₂Et | O | OCH₂c-Pr | H | H | H | H | H | H | H |
| CF₃ | CO₂Et | O | Bn | H | H | H | H | H | H | H |
| CF₃ | CO₂Et | O | 4-ClBn | H | H | H | H | H | H | H |
| CF₃ | CO₂Et | O | 4-MeBn | H | H | H | H | H | H | H |

**[Table 24]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂Et | O | 4-OMeBn | H | H | H | H | H | H | H |
| CF₃ | CO₂Et | O | OAc | H | H | H | H | H | H | H |
| CF₃ | CO₂Et | O | OBz | H | H | H | H | H | H | H |
| CF₃ | CO₂Et | O | O(4-ClBz) | H | H | H | H | H | H | H |
| CF₃ | CO₂Et | O | OCO₂Et | H | H | H | H | H | H | H |
| CF₃ | CO₂Et | O | OCO(SMe) | H | H | H | H | H | H | H |
| CF₃ | CO₂Et | O | OSO₂Me | H | H | H | H | H | H | H |
| CF₃ | CO₂Et | O | OSO₂CF₃ | H | H | H | H | H | H | H |
| CF₃ | CO₂Et | O | OSO₂Ph | H | H | H | H | H | H | H |
| CF₃ | CO₂Et | O | OPO(OEt)₂ | H | H | H | H | H | H | H |
| CF₃ | CO₂Et | O | OCH₂OMe | H | H | H | H | H | H | H |
| CF₃ | CO₂Et | O | SMe | H | H | H | H | H | H | H |
| CF₃ | CO₂Et | O | SEt | H | H | H | H | H | H | H |
| CF₃ | CO₂Et | O | SCF₃ | H | H | H | H | H | H | H |
| CF₃ | CO₂Et | O | SO₂Me | H | H | H | H | H | H | H |
| CF₃ | CO₂Et | O | SO₂CF₃ | H | H | H | H | H | H | H |
| CF₃ | CO₂Et | O | Ph | H | H | H | H | H | H | H |
| CF₃ | CO₂Et | O | 4-ClPh | H | H | H | H | H | H | H |
| CF₃ | CO₂Et | O | 4-MePh | H | H | H | H | H | H | H |
| CF₃ | CO₂Et | O | NMe₂ | H | H | H | H | H | H | H |
| CF₃ | CO₂Et | O | NHAc | H | H | H | H | H | H | H |
| CF₃ | CO₂Et | O | NAc₂ | H | H | H | H | H | H | H |
| CF₃ | CO₂Et | O | NHSO₂CF₃ | H | H | H | H | H | H | H |
| CF₃ | CO₂Et | O | N-morpholinyl | H | H | H | H | H | H | H |
| CF₃ | CO₂Et | O | CO₂Me | H | H | H | H | H | H | H |
| CF₃ | CO₂Et | O | CO₂Et | H | H | H | H | H | H | H |
| CF₃ | CO₂Et | O | OH | H | H | H | H | H | H | H |
| CF₃ | CO₂Et | O | CN | H | H | H | H | H | H | H |
| CF₃ | CO₂Et | O | NO₂ | H | H | H | H | H | H | H |
| CF₃ | CO₂Et | O | H | F | H | H | H | H | H | H |

**[Table 25]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂Et | O | H | Cl | H | H | H | H | H | H |
| CF₃ | CO₂Et | O | H | Br | H | H | H | H | H | H |
| CF₃ | CO₂Et | O | H | I | H | H | H | H | H | H |
| CF₃ | CO₂Et | O | H | Me | H | H | H | H | H | H |
| CF₃ | CO₂Et | O | H | c-Pr | H | H | H | H | H | H |
| CF₃ | CO₂Et | O | H | OMe | H | H | H | H | H | H |
| CF₃ | CO₂Et | O | H | OCF₃ | H | H | H | H | H | H |
| CF₃ | CO₂Et | O | H | OCH₂C≡CH | H | H | H | H | H | H |
| CF₃ | CO₂Et | O | H | Bn | H | H | H | H | H | H |
| CF₃ | CO₂Et | O | H | OAc | H | H | H | H | H | H |
| CF₃ | CO₂Et | O | H | OSO₂Me | H | H | H | H | H | H |
| CF₃ | CO₂Et | O | H | SMe | H | H | H | H | H | H |
| CF₃ | CO₂Et | O | H | SO₂Me | H | H | H | H | H | H |
| CF₃ | CO₂Et | O | H | Ph | H | H | H | H | H | H |
| CF₃ | CO₂Et | O | H | NMe₂ | H | H | H | H | H | H |
| CF₃ | CO₂Et | O | H | CN | H | H | H | H | H | H |
| CF₃ | CO₂Et | O | H | NO₂ | H | H | H | H | H | H |
| CF₃ | CO₂Et | O | H | H | F | H | H | H | H | H |
| CF₃ | CO₂Et | O | H | H | Cl | H | H | H | H | H |
| CF₃ | CO₂Et | O | H | H | Br | H | H | H | H | H |
| CF₃ | CO₂Et | O | H | H | I | H | H | H | H | H |
| CF₃ | CO₂Et | O | H | H | Me | H | H | H | H | H |
| CF₃ | CO₂Et | O | H | H | c-Pr | H | H | H | H | H |
| CF₃ | CO₂Et | O | H | H | OMe | H | H | H | H | H |
| CF₃ | CO₂Et | O | H | H | OCF₃ | H | H | H | H | H |
| CF₃ | CO₂Et | O | H | H | OCH₂C≡CH | H | H | H | H | H |
| CF₃ | CO₂Et | O | H | H | Bn | H | H | H | H | H |
| CF₃ | CO₂Et | O | H | H | OAc | H | H | H | H | H |
| CF₃ | CO₂Et | O | H | H | OSO₂Me | H | H | H | H | H |
| CF₃ | CO₂Et | O | H | H | SMe | H | H | H | H | H |

**[Table 26]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂Et | O | H | H | SO₂Me | H | H | H | H | H |
| CF₃ | CO₂Et | O | H | H | Ph | H | H | H | H | H |
| CF₃ | CO₂Et | O | H | H | NMe₂ | H | H | H | H | H |
| CF₃ | CO₂Et | O | H | H | CN | H | H | H | H | H |
| CF₃ | CO₂Et | O | H | H | NO₂ | H | H | H | H | H |
| CF₃ | CO₂Et | O | H | H | H | F | H | H | H | H |
| CF₃ | CO₂Et | O | H | H | H | Cl | H | H | H | H |
| CF₃ | CO₂Et | O | H | H | H | Br | H | H | H | H |
| CF₃ | CO₂Et | O | H | H | H | I | H | H | H | H |
| CF₃ | CO₂Et | O | H | H | H | Me | H | H | H | H |
| CF₃ | CO₂Et | O | H | H | H | c-Pr | H | H | H | H |
| CF₃ | CO₂Et | O | H | H | H | OMe | H | H | H | H |
| CF₃ | CO₂Et | O | H | H | H | OCF₃ | H | H | H | H |
| CF₃ | CO₂Et | O | H | H | H | OCH₂C≡CH | H | H | H | H |
| CF₃ | CO₂Et | O | H | H | H | Bn | H | H | H | H |
| CF₃ | CO₂Et | O | H | H | H | OAc | H | H | H | H |
| CF₃ | CO₂Et | O | H | H | H | OSO₂Me | H | H | H | H |
| CF₃ | CO₂Et | O | H | H | H | SMe | H | H | H | H |
| CF₃ | CO₂Et | O | H | H | H | SO₂Me | H | H | H | H |
| CF₃ | CO₂Et | O | H | H | H | Ph | H | H | H | H |
| CF₃ | CO₂Et | O | H | H | H | NMe₂ | H | H | H | H |
| CF₃ | CO₂Et | O | H | H | H | CN | H | H | H | H |
| CF₃ | CO₂Et | O | H | H | H | NO₂ | H | H | H | H |
| CF₃ | CO₂Et | O | F | F | H | H | H | H | H | H |
| CF₃ | CO₂Et | O | F | H | F | H | H | H | H | H |
| CF₃ | CO₂Et | O | F | Cl | H | H | H | H | H | H |
| CF₃ | CO₂Et | O | F | H | Cl | H | H | H | H | H |
| CF₃ | CO₂Et | O | F | H | H | Cl | H | H | H | H |
| CF₃ | CO₂Et | O | F | H | H | Br | H | H | H | H |
| CF₃ | CO₂Et | O | F | H | H | I | H | H | H | H |

**[Table 27]**

| R¹ | R² | W | X¹ | X² | X³ | X⁴ | Y¹ | Y² | Y³ | |
|---|---|---|---|---|---|---|---|---|---|---|
| CF₃ | CO₂Et | O | F | H | H | Me | H | H | H | H |
| CF₃ | CO₂Et | O | F | H | H | c-Pr | H | H | H | H |
| CF₃ | CO₂Et | O | F | H | H | OMe | H | H | H | H |
| CF₃ | CO₂Et | O | F | H | H | OCF₃ | H | H | H | H |
| CF₃ | CO₂Et | O | F | H | H | OCH₂C≡CH | H | H | H | H |
| CF₃ | CO₂Et | O | F | H | H | OSO₂Me | H | H | H | H |
| CF₃ | CO₂Et | O | F | H | H | SMe | H | H | H | H |
| CF₃ | CO₂Et | O | F | H | H | SO₂Me | H | H | H | H |
| CF₃ | CO₂Et | O | F | H | H | NMe₂ | H | H | H | H |
| CF₃ | CO₂Et | O | F | H | H | CN | H | H | H | H |
| CF₃ | CO₂Et | O | F | H | H | NO₂ | H | H | H | H |
| CF₃ | CO₂Et | O | F | F | F | H | H | H | H | H |
| CF₃ | CO₂Et | O | F | F | H | F | H | H | H | H |
| CF₃ | CO₂Et | O | F | H | F | F | H | H | H | H |
| CF₃ | CO₂Et | O | F | F | F | F | H | H | H | Y⁴ H |
| CF₃ | CO₂Et | O | Cl | F | H | H | H | H | H | H |
| CF₃ | CO₂Et | O | Cl | H | F | H | H | H | H | H |
| CF₃ | CO₂Et | O | Cl | H | H | F | H | H | H | H |
| CF₃ | CO₂Et | O | Cl | Cl | H | H | H | H | H | H |
| CF₃ | CO₂Et | O | Cl | H | Cl | H | H | H | H | H |
| CF₃ | CO₂Et | O | Cl | H | H | Cl | H | H | H | H |
| CF₃ | CO₂Et | O | Cl | H | H | Br | H | H | H | H |
| CF₃ | CO₂Et | O | Cl | H | H | I | H | H | H | H |
| CF₃ | CO₂Et | O | Cl | H | H | Me | H | H | H | H |
| CF₃ | CO₂Et | O | Cl | H | H | c-Pr | H | H | H | H |
| CF₃ | CO₂Et | O | Cl | H | H | OMe | H | H | H | H |
| CF₃ | CO₂Et | O | Cl | H | H | OCF₃ | H | H | H | H |
| CF₃ | CO₂Et | O | Cl | H | H | OCH₂C≡CH | H | H | H | H |
| CF₃ | CO₂Et | O | Cl | H | H | OSO₂Me | H | H | H | H |
| CF₃ | CO₂Et | O | Cl | H | H | SMe | H | H | H | H |

**[Table 28]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂Et | O | Cl | H | H | SO₂Me | H | H | H | H |
| CF₃ | CO₂Et | O | Cl | H | H | NMe₂ | H | H | H | H |
| CF₃ | CO₂Et | O | Cl | H | H | CN | H | H | H | H |
| CF₃ | CO₂Et | O | Cl | H | H | NO₂ | H | H | H | H |
| CF₃ | CO₂Et | O | Br | H | H | F | H | H | H | H |
| CF₃ | CO₂Et | O | Br | H | H | Cl | H | H | H | H |
| CF₃ | CO₂Et | O | Br | H | H | Me | H | H | H | H |
| CF₃ | CO₂Et | O | Br | H | H | OMe | H | H | H | H |
| CF₃ | CO₂Et | O | Me | H | H | F | H | H | H | H |
| CF₃ | CO₂Et | O | Me | H | H | Cl | H | H | H | H |
| CF₃ | CO₂Et | O | Me | H | H | Me | H | H | H | H |
| CF₃ | CO₂Et | O | Me | H | H | OMe | H | H | H | H |
| CF₃ | CO₂Et | O | OMe | H | H | F | H | H | H | H |
| CF₃ | CO₂Et | O | OMe | H | H | Cl | H | H | H | H |
| CF₃ | CO₂Et | O | OMe | H | H | Me | H | H | H | H |
| CF₃ | CO₂Et | O | OMe | H | H | OMe | H | H | H | H |
| CF₃ | CO₂Et | O | H | F | F | H | H | H | H | H |
| CF₃ | CO₂Et | O | H | F | Cl | H | H | H | H | H |
| CF₃ | CO₂Et | O | H | F | H | F | H | H | H | H |
| CF₃ | CO₂Et | O | H | F | H | Cl | H | H | H | H |
| CF₃ | CO₂Et | O | H | Cl | Cl | H | H | H | H | H |
| CF₃ | CO₂Et | O | H | Cl | F | H | H | H | H | H |
| CF₃ | CO₂Et | O | H | Cl | H | F | H | H | H | H |
| CF₃ | CO₂Et | O | H | Cl | H | Cl | H | H | H | H |
| CF₃ | CO₂Et | O | H | H | F | F | H | H | H | H |
| CF₃ | CO₂Et | O | H | H | F | Cl | H | H | H | H |
| CF₃ | CO₂Et | O | H | H | Cl | Cl | H | H | H | H |
| CF₃ | CO₂Et | O | F | F | F | F | H | H | H | H |
| CF₃ | CO₂Et | O | Cl | Cl | Cl | Cl | H | H | H | H |
| CF₃ | CO₂Et | O | -CH=CH-CH=CH- | | H | H | H | H | H | H |

**[Table 29]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂Et | O | -CH=N-CH=CH- | | H | H | H | H | H | H |
| CF₃ | CO₂Et | O | -S-CH=N- | | H | H | H | H | H | H |
| CF₃ | CO₂Et | O | -N=CH-S- | | H | H | H | H | H | H |
| CF₃ | CO₂Et | O | H | -CH=CH-CH=CH- | | H | H | H | H | H |
| CF₃ | CO₂Et | O | H | -CH=N-CH=CH- | | H | H | H | H | H |
| CF₃ | CO₂Et | O | H | -S-CH=N- | | H | H | H | H | H |
| CF₃ | CO₂Et | O | H | -N=CH-S- | | H | H | H | H | H |
| CF₃ | CO₂Et | O | H | H | -CH=CH-CH=CH- | | H | H | H | H |
| CF₃ | CO₂Et | O | H | H | -CH=N-CH=CH- | | H | H | H | H |
| CF₃ | CO₂Et | O | H | H | -S-CH=N- | | H | H | H | H |
| CF₃ | CO₂Et | O | H | H | -N=CH-S- | | H | H | H | H |
| CF₃ | CO₂CH₂CH₂F | O | Cl | H | H | H | H | H | H | H |
| CF₃ | CO₂CH₂CH₂F | O | Br | H | H | H | H | H | H | H |
| CF₃ | CO₂CH₂CH₂F | O | I | H | H | H | H | H | H | H |
| CF₃ | CO₂CH₂CH₂F | O | Me | H | H | H | H | H | H | H |
| CF₃ | CO₂CH₂CH₂F | O | Et | H | H | H | H | H | H | H |
| CF₃ | CO₂CH₂CH₂F | O | n-Pr | H | H | H | H | H | H | H |
| CF₃ | CO₂CH₂CH₂F | O | i-Pr | H | H | H | H | H | H | H |
| CF₃ | CO₂CH₂CH₂F | O | n-Bu | H | H | H | H | H | H | H |
| CF₃ | CO₂CH₂CH₂F | O | s-Bu | H | H | H | H | H | H | H |
| CF₃ | CO₂CH₂CH₂F | O | i-Bu | H | H | H | H | H | H | H |
| CF₃ | CO₂CH₂CH₂F | O | t-Bu | H | H | H | H | H | H | H |
| CF₃ | CO₂CH₂CH₂F | O | c-Pr | H | H | H | H | H | H | H |
| CF₃ | CO₂CH₂CH₂F | O | c-Bu | H | H | H | H | H | H | H |
| CF₃ | CO₂CH₂CH₂F | O | OMe | H | H | H | H | H | H | H |
| CF₃ | CO₂CH₂CH₂F | O | OEt | H | H | H | H | H | H | H |
| CF₃ | CO₂CH₂CH₂F | O | OCHF₂ | H | H | H | H | H | H | H |
| CF₃ | CO₂CH₂CH₂F | O | OCF₃ | H | H | H | H | H | H | H |
| CF₃ | CO₂CH₂CH₂F | O | OCH₂CH=CH₂ | H | H | H | H | H | H | H |
| CF₃ | CO₂CH₂CH₂F | O | OCH₂C=CH | H | H | H | H | H | H | H |

**[Table 30]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂CH₂CH₂F | O | OCH₂c-Pr | H | H | H | H | H | H | H |
| CF₃ | CO₂CH₂CH₂F | O | Bn | H | H | H | H | H | H | H |
| CF₃ | CO₂CH₂CH₂F | O | 4-ClBn | H | H | H | H | H | H | H |
| CF₃ | CO₂CH₂CH₂F | O | 4-MeBn | H | H | H | H | H | H | H |
| CF₃ | CO₂CH₂CH₂F | O | 4-OMeBn | H | H | H | H | H | H | H |
| CF₃ | CO₂CH₂CH₂F | O | OAc | H | H | H | H | H | H | H |
| CF₃ | CO₂CH₂CH₂F | O | OBz | H | H | H | H | H | H | H |
| CF₃ | CO₂CH₂CH₂F | O | O(4-ClBz) | H | H | H | H | H | H | H |
| CF₃ | CO₂CH₂CH₂F | O | OCO₂Et | H | H | H | H | H | H | H |
| CF₃ | CO₂CH₂CH₂F | O | OCO(SMe) | H | H | H | H | H | H | H |
| CF₃ | CO₂CH₂CH₂F | O | OSO₂Me | H | H | H | H | H | H | H |
| CF₃ | CO₂CH₂CH₂F | O | OSO₂CF₃ | H | H | H | H | H | H | H |
| CF₃ | CO₂CH₂CH₂F | O | OSO₂Ph | H | H | H | H | H | H | H |
| CF₃ | CO₂CH₂CH₂F | O | OPO(OEt)₂ | H | H | H | H | H | H | H |
| CF₃ | CO₂CH₂CH₂F | O | OCH₂OMe | H | H | H | H | H | H | H |
| CF₃ | CO₂CH₂CH₂F | O | SMe | H | H | H | H | H | H | H |
| CF₃ | CO₂CH₂CH₂F | O | SEt | H | H | H | H | H | H | H |
| CF₃ | CO₂CH₂CH₂F | O | SCF₃ | H | H | H | H | H | H | H |
| CF₃ | CO₂CH₂CH₂F | O | SO₂Me | H | H | H | H | H | H | H |
| CF₃ | CO₂CH₂CH₂F | O | SO₂CF₃ | H | H | H | H | H | H | H |
| CF₃ | CO₂CH₂CH₂F | O | Ph | H | H | H | H | H | H | H |
| CF₃ | CO₂CH₂CH₂F | O | 4-ClPh | H | H | H | H | H | H | H |
| CF₃ | CO₂CH₂CH₂F | O | 4-MePh | H | H | H | H | H | H | H |
| CF₃ | CO₂CH₂CH₂F | O | NMe₂ | H | H | H | H | H | H | H |
| CF₃ | CO₂CH₂CH₂F | O | NHAc | H | H | H | H | H | H | H |
| CF₃ | CO₂CH₂CH₂F | O | NAc₂ | H | H | H | H | H | H | H |
| CF₃ | CO₂CH₂CH₂F | O | NHSO₂CF₃ | H | H | H | H | H | H | H |
| CF₃ | CO₂CH₂CH₂F | O | N-morpholinyl | H | H | H | H | H | H | H |
| CF₃ | CO₂CH₂CH₂F | O | CO₂Me | H | H | H | H | H | H | H |
| CF₃ | CO₂CH₂CH₂F | O | CO₂Et | H | H | H | H | H | H | H |

**[Table 31]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂CH₂CH₂F | O | OH | H | H | H | H | H | H | H |
| CF₃ | CO₂CH₂CH₂F | O | CN | H | H | H | H | H | H | H |
| CF₃ | CO₂CH₂CH₂ F | O | NO₂ | H | H | H | H | H | H | H |
| CF₃ | CO₂CH₂CH₂F | O | H | F | H | H | H | H | H | H |
| CF₃ | CO₂CH₂CH₂ F | O | H | Cl | H | H | H | H | H | H |
| CF₃ | CO₂CH₂CH₂F | O | H | Br | H | H | H | H | H | H |
| CF₃ | CO₂CH₂CH₂F | O | H | I | H | H | H | H | H | H |
| CF₃ | CO₂CH₂CH₂F | O | H | Me | H | H | H | H | H | H |
| CF₃ | CO₂CH₂CH₂F | O | H | c-Pr | H | H | H | H | H | H |
| CF₃ | CO₂CH₂CH₂F | O | H | OMe | H | H | H | H | H | H |
| CF₃ | CO₂CH₂CH₂F | O | H | OCF₃ | H | H | H | H | H | H |
| CF₃ | CO₂CH₂CH₂F | O | H | OCH₂C≡CH | H | H | H | H | H | H |
| CF₃ | CO₂CH₂CH₂F | O | H | Bn | H | H | H | H | H | H |
| CF₃ | CO₂CH₂CH₂F | O | H | OAc | H | H | H | H | H | H |
| CF₃ | CO₂CH₂CH₂F | O | H | OSO₂Me | H | H | H | H | H | H |
| CF₃ | CO₂CH₂CH₂F | O | H | SMe | H | H | H | H | H | H |
| CF₃ | CO₂CH₂CH₂F | O | H | SO₂Me | H | H | H | H | H | H |
| CF₃ | CO₂CH₂CH₂F | O | H | Ph | H | H | H | H | H | H |
| CF₃ | CO₂CH₂CH₂F | O | H | NMe₂ | H | H | H | H | H | H |
| CF₃ | CO₂CH₂CH₂ F | O | H | CN | H | H | H | H | H | H |
| CF₃ | CO₂CH₂CH₂F | O | H | NO₂ | H | H | H | H | H | H |
| CF₃ | CO₂CH₂CH₂F | O | H | H | F | H | H | H | H | H |
| CF₃ | CO₂CH₂CH₂F | O | H | H | Cl | H | H | H | H | H |
| CF₃ | CO₂CH₂CH₂F | O | H | H | Br | H | H | H | H | H |
| CF₃ | CO₂CH₂CH₂F | O | H | H | I | H | H | H | H | H |
| CF₃ | CO₂CH₂CH₂F | O | H | H | Me | H | H | H | H | H |
| CF₃ | CO₂CH₂CH₂F | O | H | H | c-Pr | H | H | H | H | H |
| CF₃ | CO₂CH₂CH₂F | O | H | H | OMe | H | H | H | H | H |
| CF₃ | CO₂CH₂CH₂F | O | H | H | OCF₃ | H | H | H | H | H |
| CF₃ | CO₂CH₂CH₂ F | O | H | H | OCH₂C≡CH | H | H | H | H | H |

**[Table 32]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂CH₂CH₂F | O | H | H | Bn | H | H | H | H | H |
| CF₃ | CO₂CH₂CH₂F | O | H | H | OAc | H | H | H | H | H |
| CF₃ | CO₂CH₂CH₂F | O | H | H | OSO₂Me | H | H | H | H | H |
| CF₃ | CO₂CH₂CH₂F | O | H | H | SMe | H | H | H | H | H |
| CF₃ | CO₂CH₂CH₂F | O | H | H | SO₂Me | H | H | H | H | H |
| CF₃ | CO₂CH₂CH₂F | O | H | H | Ph | H | H | H | H | H |
| CF₃ | CO₂CH₂CH₂F | O | H | H | NMe₂ | H | H | H | H | H |
| CF₃ | CO₂CH₂CH₂F | O | H | H | CN | H | H | H | H | H |
| CF₃ | CO₂CH₂CH₂F | O | H | H | NO₂ | H | H | H | H | H |
| CF₃ | CO₂CH₂CH₂F | O | H | H | H | F | H | H | H | H |
| CF₃ | CO₂CH₂CH₂F | O | H | H | H | Cl | H | H | H | H |
| CF₃ | CO₂CH₂CH₂F | O | H | H | H | Br | H | H | H | H |
| CF₃ | CO₂CH₂CH₂F | O | H | H | H | I | H | H | H | H |
| CF₃ | CO₂CH₂CH₂F | O | H | H | H | Me | H | H | H | H |
| CF₃ | CO₂CH₂CH₂F | O | H | H | H | c-Pr | H | H | H | H |
| CF₃ | CO₂CH₂CH₂F | O | H | H | H | OMe | H | H | H | H |
| CF₃ | CO₂CH₂CH₂F | O | H | H | H | OCF₃ | H | H | H | H |
| CF₃ | CO₂CH₂CH₂F | O | H | H | H | OCH₂C≡CH | H | H | H | H |
| CF₃ | CO₂CH₂CH₂F | O | H | H | H | Bn | H | H | H | H |
| CF₃ | CO₂CH₂CH₂F | O | H | H | H | OAc | H | H | H | H |
| CF₃ | CO₂CH₂CH₂F | O | H | H | H | OSO₂Me | H | H | H | H |
| CF₃ | CO₂CH₂CH₂F | O | H | H | H | SMe | H | H | H | H |
| CF₃ | CO₂CH₂CH₂F | O | H | H | | SO₂Me | H | H | H | H |
| CF₃ | CO₂CH₂CH₂F | O | H | H | H | Ph | H | H | H | H |
| CF₃ | CO₂CH₂CH₂F | O | H | H | H | NMe₂ | H | H | H | H |
| CF₃ | CO₂CH₂CH₂F | O | H | H | H | CN | H | H | H | H |
| CF₃ | CO₂CH₂CH₂F | O | H | H | H | NO₂ | H | H | H | H |
| CF₃ | CO₂CH₂CH₂F | O | F | F | H | H | H | H | H | H |
| CF₃ | CO₂CH₂CH₂F | O | F | H | F | H | H | H | H | H |
| CF₃ | CO₂CH₂CH₂F | O | F | Cl | H | H | H | H | H | H |

**[Table 33]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂CH₂CH₂F | O | F | H | Cl | H | H | H | H | H |
| CF₃ | CO₂CH₂CH₂F | O | F | H | H | Cl | H | H | H | H |
| CF₃ | CO₂CH₂CH₂F | O | F | H | H | Br | H | H | H | H |
| CF₃ | CO₂CH₂CH₂F | O | F | H | H | I | H | H | H | H |
| CF₃ | CO₂CH₂CH₂F | O | F | H | H | Me | H | H | H | H |
| CF₃ | CO₂CH₂CH₂F | O | F | H | H | c-Pr | H | H | H | H |
| CF₃ | CO₂CH₂CH₂F | O | F | H | H | OMe | H | H | H | H |
| CF₃ | CO₂CH₂CH₂F | O | F | H | H | OCF₃ | H | H | H | H |
| CF₃ | CO₂CH₂CH₂F | O | F | H | H | OCH₂C≡CH | H | H | H | H |
| CF₃ | CO₂CH₂CH₂F | O | F | H | H | OSO₂Me | H | H | H | H |
| CF₃ | CO₂CH₂CH₂F | O | F | H | H | SMe | H | H | H | H |
| CF₃ | CO₂CH₂CH₂F | O | F | H | H | SO₂Me | H | H | H | H |
| CF₃ | CO₂CH₂CH₂F | O | F | H | H | NMe₂ | H | H | H | H |
| CF₃ | CO₂CH₂CH₂F | O | F | H | H | CN | H | H | H | H |
| CF₃ | CO₂CH₂CH₂F | O | F | H | H | NO₂ | H | H | H | H |
| CF₃ | CO₂CH₂CH₂F | O | F | F | F | H | H | H | H | H |
| CF₃ | CO₂CH₂CH₂F | O | F | F | H | F | H | H | H | H |
| CF₃ | CO₂CH₂CH₂F | O | F | H | F | F | H | H | H | H |
| CF₃ | CO₂CH₂CH₂F | O | F | F | F | F | H | H | H | H |
| CF₃ | CO₂CH₂CH₂F | O | Cl | F | H | H | H | H | H | H |
| CF₃ | CO₂CH₂CH₂F | O | Cl | H | F | H | H | H | H | H |
| CF₃ | CO₂CH₂CH₂F | O | Cl | H | H | F | H | H | H | H |
| CF₃ | CO₂CH₂CH₂F | O | Cl | Cl | H | H | H | H | H | H |
| CF₃ | CO₂CH₂CH₂F | O | Cl | H | Cl | H | H | H | H | H |
| CF₃ | CO₂CH₂CH₂F | O | Cl | H | H | Cl | H | H | H | H |
| CF₃ | CO₂CH₂CH₂F | O | Cl | H | H | Br | H | H | H | H |
| CF₃ | CO₂CH₂CH₂F | O | Cl | H | H | I | H | H | H | H |
| CF₃ | CO₂CH₂CH₂F | O | Cl | H | H | Me | H | H | H | H |
| CF₃ | CO₂CH₂CH₂F | O | Cl | H | H | c-Pr | H | H | H | H |
| CF₃ | CO₂CH₂CH₂F | O | Cl | H | H | OMe | H | H | H | H |

**[Table 34]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂CH₂CH₂F | O | Cl | H | H | OCF₃ | H | H | H | H |
| CF₃ | CO₂CH₂CH₂F | O | Cl | H | H | OCH₂C≡CH | H | H | H | H |
| .CF₃ | CO₂CH₂CH₂F | O | Cl | H | H | OSO₂Me | H | H | H | H |
| CF₃ | CO₂CH₂CH₂F | O | Cl | H | H | SMe | H | H | H | H |
| CF₃ | CO₂CH₂CH₂F | O | Cl | H | H | SO₂Me | H | H | H | H |
| CF₃ | CO₂CH₂CH₂F | O | Cl | H | H | NMe₂ | H | H | H | H |
| CF₃ | CO₂CH₂CH₂F | O | Cl | H | H | CN | H | H | H | H |
| CF₃ | CO₂CH₂CH₂F | O | Cl | H | H | NO₂ | H | H | H | H |
| CF₃ | CO₂CH₂CH₂F | O | Br | H | H | F | H | H | H | H |
| CF₃ | CO₂CH₂CH₂F | O | Br | H | H | CI | H | H | H | H |
| CF₃ | CO₂CH₂CH₂F | O | Br | H | H | Me | H | H | H | H |
| CF₃ | CO₂CH₂CH₂F | O | Br | H | H | OMe | H | H | H | H |
| CF₃ | CO₂CH₂CH₂F | O | Me | H | H | F | H | H | H | H |
| CF₃ | CO₂CH₂CH₂F | O | Me | H | H | Cl | H | H | H | H |
| CF₃ | CO₂CH₂CH₂F | O | Me | H | H | Me | H | H | H | H |
| CF₃ | CO₂CH₂CH₂F | O | Me | H | H | OMe | H | H | H | H |
| CF₃ | CO₂CH₂CH₂F | O | OMe | H | H | F | H | H | H | H |
| CF₃ | CO₂CH₂CH₂F | O | OMe | H | H | Cl | H | H | H | H |
| CF₃ | CO₂CH₂CH₂F | O | OMe | H | H | Me | H | H | H | H |
| CF₃ | CO₂CH₂CH₂F | O | OMe | H | H | OMe | H | H | H | H |
| CF₃ | CO₂CH₂CH₂F | O | H | F | F | H | H | H | H | H |
| CF₃ | CO₂CH₂CH₂F | O | H | F | Cl | H | H | H | H | H |
| CF₃ | CO₂CH₂CH₂F | O | H | F | H | F | H | H | H | H |
| CF₃ | CO₂CH₂CH₂F | O | H | F | H | Cl | H | H | H | H |
| CF₃ | CO₂CH₂CH₂F | O | H | Cl | Cl | H | H | H | H | H |
| CF₃ | CO₂CH₂CH₂F | O | H | Cl | F | H | H | H | H | H |
| CF₃ | CO₂CH₂CH₂F | O | H | Cl | H | F | H | H | H | H |
| CF₃ | CO₂CH₂CH₂F | O | H | Cl | H | Cl | H | H | H | H |
| CF₃ | CO₂CH₂CH₂F | O | H | H | F | F | H | H | H | H |
| CF₃ | CO₂CH₂CH₂F | O | H | H | F | Cl | H | H | H | H |

**[Table 35]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂CH₂CH₂F | O | H | H | Cl | Cl | H | H | H | H |
| CF₃ | CO₂CH₂CH₂F | O | F | F | F | F | H | H | H | H |
| CF₃ | CO₂CH₂CH₂F | O | Cl | Cl | Cl | Cl | H | H | H | H |
| CF₃ | CO₂CH₂CH₂F | O | -CH=CH-CH=CH- | | H | H | H | H | H | H |
| CF₃ | CO₂CH₂CH₂F | O | -CH=N-CH=CH- | | H | H | H | H | H | H |
| CF₃ | CO₂CH₂CH₂F | O | -S-CH=N- | | H | H | H | H | H | H |
| CF₃ | CO₂CH₂CH₂F | O | -N=CH-S- | | H | H | H | H | H | H |
| CF₃ | CO₂CH₂CH₂F | O | H | -CH=CH-CH=CH- | | H | H | H | H | H |
| CF₃ | CO₂CH₂CH₂F | O | H | -CH=N-CH=CH- | | H | H | H | H | H |
| CF₃ | CO₂CH₂CH₂F | O | H | -S-CH=N- | | H | H | H | H | H |
| CF₃ | CO₂CH₂CH₂F | O | H | -N=CH-S- | | H | H | H | H | H |
| CF₃ | CO₂CH₂CH₂F | O | H | H | -CH=CH-CH=CH- | | H | H | H | H |
| CF₃ | CO₂CH₂CH₂F | O | H | H | -CH=N-CH=CH- | | H | H | H | H |
| CF₃ | CO₂CH₂CH₂F | O | H | H | -S-CH=N- | | H | H | H | H |
| CF₃ | CO₂CH₂CH₂F | O | H | H | -N=CH-S- | | H | H | H | H |
| CF₃ | CO₂CH₂CHF₂ | O | Cl | H | H | H | H | H | H | H |
| CF₃ | CO₂CH₂CHF₂ | O | Br | H | H | H | H | H | H | H |
| CF₃ | CO₂CH₂CHF₂ | O | I | H | H | H | H | H | H | H |
| CF₃ | CO₂CH₂CHF₂ | O | Me | H | H | H | H | H | H | H |
| CF₃ | CO₂CH₂CHF₂ | O | Et | H | H | H | H | H | H | H |
| CF₃ | CO₂CH₂CHF₂ | O | n-Pr | H | H | H | H | H | H | H |
| CF₃ | CO₂CH₂CHF₂ | O | i-Pr | H | H | H | H | H | H | H |
| CF₃ | CO₂CH₂CHF₂ | O | n-Bu | H | H | H | H | H | H | H |
| CF₃ | CO₂CH₂CHF₂ | O | s-Bu | H | H | H | H | H | H | H |
| CF₃ | CO₂CH₂CHF₂ | O | i-Bu | H | H | H | H | H | H | H |
| CF₃ | CO₂CH₂CHF₂ | O | t-Bu | H | H | H | H | H | H | H |
| CF₃ | CO₂CH₂CHF₂ | O | c-Pr | H | H | H | H | H | H | H |
| CF₃ | CO₂CH₂CHF₂ | O | c-Bu | H | H | H | H | H | H | H |
| CF₃ | CO₂CH₂CHF₂ | O | OMe | H | H | H | H | H | H | H |
| CF₃ | CO₂CH₂CHF₂ | O | OEt | H | H | H | H | H | H | H |

**[Table 36]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂CH₂CHF₂ | O | OCHF₂ | H | H | H | H | H | H | H |
| CF₃ | CO₂CH₂CHF₂ | O | OCF₃ | H | H | H | H | H | H | H |
| CF₃ | CO₂CH₂CHF₂ | O | OCH₂CH=CH₂ | H | H | H | H | H | H | H |
| CF₃ | CO₂CH₂CHF₂ | O | OCH₂C≡ CH | H | H | H | H | H | H | H |
| CF₃ | CO₂CH₂CHF₂ | O | OCH₂c-Pr | H | H | H | H | H | H | H |
| CF₃ | CO₂CH₂CHF₂ | O | Bn | H | H | H | H | H | H | H |
| CF₃ | CO₂CH₂CHF₂ | O | 4-ClBn | H | H | H | H | H | H | H |
| CF₃ | CO₂CH₂CHF₂ | O | 4-MeBn | H | H | H | H | H | H | H |
| CF₃ | CO₂CH₂CHF₂ | O | 4-OMeBn | H | H | H | H | H | H | H |
| CF₃ | CO₂CH₂CHF₂ | O | OAc | H | H | H | H | H | H | H |
| CF₃ | CO₂CH₂CHF₂ | O | OBz | H | H | H | H | H | H | H |
| CF₃ | CO₂CH₂CHF₂ | O | O(4-ClBz) | H | H | H | H | H | H | H |
| CF₃ | CO₂CH₂CHF₂ | O | OCO₂Et | H | H | H | H | H | H | H |
| CF₃ | CO₂CH₂CHF₂ | O | OCO(SMe) | H | H | H | H | H | H | H |
| CF₃ | CO₂CH₂CHF₂ | O | OSO₂Me | H | H | H | H | H | H | H |
| CF₃ | CO₂CH₂CHF₂ | O | OSO₂CF₃ | H | H | H | H | H | H | H |
| CF₃ | CO₂CH₂CHF₂ | O | OSO₂Ph | H | H | H | H | H | H | H |
| CF₃ | CO₂CH₂CHF₂ | O | OPO(OEt)₂ | H | H | H | H | H | H | H |
| CF₃ | CO₂CH₂CHF₂ | O | OCH₂OMe | H | H | H | H | H | H | H |
| CF₃ | CO₂CH₂CHF₂ | O | SMe | H | H | H | H | H | H | H |
| CF₃ | CO₂CH₂CHF₂ | O | SEt | H | H | H | H | H | H | H |
| CF₃ | CO₂CH₂CHF₂ | O | SCF₃ | H | H | H | H | H | H | H |
| CF₃ | CO₂CH₂CHF₂ | O | SO₂Me | H | H | H | H | H | H | H |
| CF₃ | CO₂CH₂CHF₂ | O | SO₂CF₃ | H | H | H | H | H | H | H |
| CF₃ | CO₂CH₂CHF₂ | O | Ph | H | H | H | H | H | H | H |
| CF₃ | CO₂CH₂CHF₂ | O | 4-CIPh | H | H | H | H | H | H | H |
| CF₃ | CO₂CH₂CHF₂ | O | 4-MePh | H | H | H | H | H | H | H |
| CF₃ | CO₂CH₂CHF₂ | O | NMe₂ | H | H | H | H | H | H | H |
| CF₃ | CO₂CH₂CHF₂ | O | NHAc | H | H | H | H | H | H | H |
| CF₃ | CO₂CH₂CHF₂ | O | NAc₂ | H | H | H | H | H | H | H |

**[Table 37]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂CH₂CHF₂ | O | NHSO₂CF₃ | H | H | H | H | H | H | H |
| CF₃ | CO₂CH₂CHF₂ | O | N-morpholinyl | H | H | H | H | H | H | H |
| CF₃ | CO₂CH₂CHF₂ | O | CO₂Me | H | H | H | H | H | H | H |
| CF₃ | CO₂CH₂CHF₂ | O | CO₂Et | H | H | H | H | H | H | H |
| CF₃ | CO₂CH₂CHF₂ | O | OH | H | H | H | H | H | H | H |
| CF₃ | CO₂CH₂CHF₂ | O | CN | H | H | H | H | H | H | H |
| CF₃ | CO₂CH₂CHF₂ | O | NO₂ | H | H | H | H | H | H | H |
| CF₃ | CO₂CH₂CHF₂ | O | H | F | H | H | H | H | H | H |
| CF₃ | CO₂CH₂CHF₂ | O | H | Cl | H | H | H | H | H | H |
| CF₃ | CO₂CH_{2C}HF₂ | O | H | Br | H | H | H | H | H | H |
| CF₃ | CO₂CH₂CHF₂ | O | H | I | H | H | H | H | H | H |
| CF₃ | CO₂CH₂CHF₂ | O | H | Me | H | H | H | H | H | H |
| CF₃ | CO₂CH₂CHF₂ | O | H | c-Pr | H | H | H | H | H | H |
| CF₃ | CO₂CH₂CHF₂ | O | H | OMe | H | H | H | H | H | H |
| CF₃ | CO₂CH₂CHF₂ | O | H | OCF₃ | H | H | H | H | H | H |
| CF₃ | CO₂CH₂CHF₂ | O | H | OCH₂C≡CH | H | H | H | H | H | H |
| CF₃ | CO₂CH₂CHF₂ | O | H | Bn | H | H | H | H | H | H |
| CF₃ | CO₂CH₂CHF₂ | O | H | OAc | H | H | H | H | H | H |
| CF₃ | CO₂CH₂CHF₂ | O | H | OSO₂Me | H | H | H | H | H | H |
| CF₃ | CO₂CH₂CHF₂ | O | H | SMe | H | H | H | H | H | H |
| CF₃ | CO₂CH₂CHF₂ | O | H | SO₂Me | H | H | H | H | H | H |
| CF₃ | CO₂CH₂CHF₂ | O | H | Ph | H | H | H | H | H | H |
| CF₃ | CO₂CH₂CHF₂ | O | H | NMe₂ | H | H | H | H | H | H |
| CF₃ | CO₂CH₂CHF₂ | O | H | CN | H | H | H | H | H | H |
| CF₃ | CO₂CH₂CHF₂ | O | H | NO₂ | H | H | H | H | H | H |
| CF₃ | CO₂CH₂CHF₂ | O | H | H | F | H | H | H | H | H |
| CF₃ | CO₂CH₂CHF₂ | O | H | H | Cl | H | H | H | H | H |
| CF₃ | CO₂CH₂CHF₂ | O | H | H | Br | H | H | H | H | H |
| CF₃ | CO₂CH₂CHF₂ | O | H | H | I | H | H | H | H | H |
| CF₃ | CO₂CH₂CHF₂ | O | H | H | Me | H | H | H | H | H |

**[Table 38]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂CH₂CHF₂ | O | H | H | c-Pr | H | H | H | H | H |
| CF₃ | CO₂CH₂CHF₂ | O | H | H | OMe | H | H | H | H | H |
| CF₃ | CO₂CH₂CHF₂ | O | H | H | OCF₃ | H | H | H | H | H |
| CF₃ | CO₂CH₂CHF₂ | O | H | H | OCH₂C≡CH | H | H | H | H | H |
| CF₃ | CO₂CH₂CHF₂ | O | H | H | Bn | H | H | H | H | H |
| CF₃ | CO₂CH₂CHF₂ | O | H | H | OAc | H | H | H | H | H |
| CF₃ | CO₂CH₂CHF₂ | O | H | H | OSO₂Me | H | H | H | H | H |
| CF₃ | CO₂CH₂CHF₂ | O | H | H | SMe | H | H | H | H | H |
| CF₃ | CO₂CH₂CHF₂ | O | H | H | SO₂Me | H | H | H | H | H |
| CF₃ | CO₂CH₂CHF₂ | O | H | H | Ph | H | H | H | H | H |
| CF₃ | CO₂CH₂CHF₂ | O | H | H | NMe₂ | H | H | H | H | H |
| CF₃ | CO₂CH₂CHF₂ | O | H | H | CN | H | H | H | H | H |
| CF₃ | CO₂CH₂CHF₂ | O | H | H | NO₂ | H | H | H | H | H |
| CF₃ | CO₂CH₂CHF₂ | O | H | H | H | F | H | H | H | H |
| CF₃ | CO₂CH₂CHF₂ | O | H | H | H | Cl | H | H | H | H |
| CF₃ | CO₂CH₂CHF₂ | O | H | H | H | Br | H | H | H | H |
| CF₃ | CO₂CH₂CHF₂ | O | H | H | H | I | H | H | H | H |
| CF₃ | CO₂CH₂CHF₂ | O | H | H | H | Me | H | H | H | H |
| CF₃ | CO₂CH₂CHF₂ | O | H | H | H | c-Pr | H | H | H | H |
| CF₃ | CO₂CH₂CHF₂ | O | H | H | H | OMe | H | H | H | H |
| CF₃ | CO₂CH₂CHF₂ | O | H | H | H | OCF₃ | H | H | H | H |
| CF₃ | CO₂CH₂CHF₂ | O | H | H | H | OCH₂C≡CH | H | H | H | H |
| CF₃ | CO₂CH₂CHF₂ | O | H | H | H | Bn | H | H | H | H |
| CF₃ | CO₂CH₂CHF₂ | O | H | H | H | OAc | H | H | H | H |
| CF₃ | CO₂CH₂CHF₂ | O | H | H | H | OSO₂Me | H | H | H | H |
| CF₃ | CO₂CH₂CHF₂ | O | H | H | H | SMe | H | H | H | H |
| CF₃ | CO₂CH₂CHF₂ | O | H | H | H | SO₂Me | H | H | H | H |
| CF₃ | CO₂CH₂CHF₂ | O | H | H | H | Ph | H | H | H | H |
| CF₃ | CO₂CH₂CHF₂ | O | H | H | H | NMe₂ | H | H | H | H |
| CF₃ | CO₂CH₂CHF₂ | O | H | H | H | CN | H | H | H | H |

**[Table 39]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂CH₂CHF₂ | O | H | H | H | NO₂ | H | H | H | H |
| CF₃ | CO₂CH₂CHF₂ | O | F | F | H | H | H | H | H | H |
| CF₃ | CO₂CH₂CHF₂ | O | F | H | F | H | H | H | H | H |
| CF₃ | CO₂CH₂CHF₂ | O | F | Cl | H | H | H | H | H | H |
| CF₃ | CO₂CH₂CHF₂ | O | F | H | Cl | H | H | H | H | H |
| CF₃ | CO₂CH₂CHF₂ | O | F | H | H | CI | H | H | H | H |
| CF₃ | CO₂CH₂CHF₂ | O | F | H | H | Br | H | H | H | H |
| CF₃ | CO₂CH₂CHF₂ | O | F | H | H | I | H | H | H | H |
| CF₃ | CO₂CH₂CHF₂ | O | F | H | H | Me | H | H | H | H |
| CF₃ | CO₂CH₂CHF₂ | O | F | H | H | c-Pr | H | H | H | H |
| CF₃ | CO₂CH₂CHF₂ | O | F | H | H | OMe | H | H | H | H |
| CF₃ | CO₂CH₂CHF₂ | O | F | H | H | OCF₃ | H | H | H | H |
| CF₃ | CO₂CH₂CHF₂ | O | F | H | H | OCH₂C≡CH | H | H | H | H |
| CF₃ | CO₂CH₂CHF₂ | O | F | H | H | OSO₂Me | H | H | H | H |
| CF₃ | CO₂CH₂CHF₂ | O | F | H | H | SMe | H | H | H | H |
| CF₃ | CO₂CH₂CHF₂ | O | F | H | H | SO₂Me | H | H | H | H |
| CF₃ | CO₂CH₂CHF₂ | O | F | H | H | NMe₂ | H | H | H | H |
| CF₃ | CO₂CH₂CHF₂ | O | F | H | H | CN | H | H | H | H |
| CF₃ | CO₂CH₂CHF₂ | O | F | H | H | NO₂ | H | H | H | H |
| CF₃ | CO₂CH₂CHF₂ | O | F | F | F | H | H | H | H | H |
| CF₃ | CO₂CH₂CHF₂ | O | F | F | H | F | H | H | H | H |
| CF₃ | CO₂CH₂CHF₂ | O | F | H | F | F | H | H | H | H |
| CF₃ | CO₂CH₂CHF₂ | O | F | F | F | F | H | H | H | H |
| CF₃ | CO₂CH₂CHF₂ | O | CI | F | H | H | H | H | H | H |
| CF₃ | CO₂CH₂CHF₂ | O | Cl | H | F | H | H | H | H | H |
| CF₃ | CO₂CH₂CHF₂ | O | Cl | H | H | F | H | H | H | H |
| CF₃ | CO₂CH₂CHF₂ | O | Cl | Cl | H | H | H | H | H | H |
| CF₃ | CO₂CH₂CHF₂ | O | Cl | H | Cl | H | H | H | H | H |
| CF₃ | CO₂CH₂CHF₂ | O | Cl | H | H | CI | H | H | H | H |
| CF₃ | CO₂CH₂CHF₂ | O | Cl | H | H | Br | H | H | H | H |

**[Table 40]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂CH₂CHF₂ | O | Cl | H | H | I | H | H | H | H |
| CF₃ | CO₂CH₂CHF₂ | O | Cl | H | H | Me | H | H | H | H |
| CF₃ | CO₂CH₂CHF₂ | O | Cl | H | H | c-Pr | H | H | H | H |
| CF₃ | CO₂CH₂CHF₂ | O | Cl | H | H | OMe | H | H | H | H |
| CF₃ | CO₂CH₂CHF₂ | O | Cl | H | H | OCF₃ | H | H | H | H |
| CF₃ | CO₂CH₂CHF₂ | O | Cl | H | H | OCH₂C≡CH | H | H | H | H |
| CF₃ | CO₂CH₂CHF₂ | O | Cl | H | H | OSO₂Me | H | H | H | H |
| CF₃ | CO₂CH₂CHF₂ | O | Cl | H | H | SMe | H | H | H | H |
| CF₃ | CO₂CH₂CHF₂ | O | Cl | H | H | SO₂Me | H | H | H | H |
| CF₃ | CO₂CH₂CHF₂ | O | Cl | H | H | NMe₂ | H | H | H | H |
| CF₃ | CO₂CH₂CHF₂ | O | Cl | H | H | CN | H | H | H | H |
| CF₃ | CO₂CH₂CHF₂ | O | Cl | H | H | NO₂ | H | H | H | H |
| CF₃ | CO₂CH₂CHF₂ | O | Br | H | H | F | H | H | H | H |
| CF₃ | CO₂CH₂CHF₂ | O | Br | H | H | Cl | H | H | H | H |
| CF₃ | CO₂CH₂CHF₂ | O | Br | H | H | Me | H | H | H | H |
| CF₃ | CO₂CH₂CHF₂ | O | Br | H | H | OMe | H | H | H | H |
| CF₃ | CO₂CH₂CHF₂ | O | Me | H | H | F | H | H | H | H |
| CF₃ | CO₂CH₂CHF₂ | O | Me | H | H | Cl | H | H | H | H |
| CF₃ | CO₂CH₂CHF₂ | O | Me | H | H | Me | H | H | H | H |
| CF₃ | CO₂CH₂CHF₂ | O | Me | H | H | OMe | H | H | H | H |
| CF₃ | CO₂CH₂CHF₂ | O | OMe | H | H | F | H | H | H | H |
| CF₃ | CO₂CH₂CHF₂ | O | OMe | H | H | Cl | H | H | H | H |
| CF₃ | CO₂CH₂CHF₂ | O | OMe | H | H | Me | H | H | H | H |
| CF₃ | CO₂CH₂CHF₂ | O | OMe | H | H | OMe | H | H | H | H |
| CF₃ | CO₂CH₂CHF₂ | O | H | F | F | H | H | H | H | H |
| CF₃ | CO₂CH₂CHF₂ | O | H | F | Cl | H | H | H | H | H |
| CF₃ | CO₂CH₂CHF₂ | O | H | F | H | F | H | H | H | H |
| CF₃ | CO₂CH₂CHF₂ | O | H | F | H | Cl | H | H | H | H |
| CF₃ | CO₂CH₂CHF₂ | O | H | Cl | Cl | H | H | H | H | H |
| CF₃ | CO₂CH₂CHF₂ | O | H | Cl | F | H | H | H | H | H |

**[Table 41]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂CH₂CHF₂ | O | H | Cl | H | F | H | H | H | H |
| CF₃ | CO₂CH₂CHF₂ | O | H | Cl | H | Cl | H | H | H | H |
| CF₃ | CO₂CH₂CHF₂ | O | H | H | F | F | H | H | H | H |
| CF₃ | CO₂CH₂CHF₂ | O | H | H | F | Cl | H | H | H | H |
| CF₃ | CO₂CH₂CHF₂ | O | H | H | Cl | Cl | H | H | H | H |
| CF₃ | CO₂CH₂CHF₂ | O | F | F | F | F | H | H | H | H |
| CF₃ | CO₂CH₂CHF₂ | O | Cl | Cl | Cl | Cl | H | H | H | H |
| CF₃ | CO₂CH₂CHF₂ | O | -CH=CH-CH=CH- | | H | H | H | H | H | H |
| CF₃ | CO₂CH₂CHF₂ | O | -CH=N-CH=CH- | | H | H | H | H | H | H |
| CF₃ | CO₂CH₂CHF₂ | O | -S-CH=N- | | H | H | H | H | H | H |
| CF₃ | CO₂CH₂CHF₂ | O | -N=CH-S- | | H | H | H | H | H | H |
| CF₃ | CO₂CH₂CHF₂ | O | H | -CH=CH-CH=CH- | | H | H | H | H | H |
| CF₃ | CO₂CH₂CHF₂ | O | H | -CH=N-CH=CH- | | H | H | H | H | H |
| CF₃ | CO₂CH₂CHF₂ | O | H | -S-CH=N- | | H | H | H | H | H |
| CF₃ | CO₂CH₂CHF₂ | O | H | -N=CH-S- | | H | H | H | H | H |
| CF₃ | CO₂CH₂CHF₂ | O | H | H | -CH=CH-CH=CH- | | H | H | H | H |
| CF₃ | CO₂CH₂CHF₂ | O | H | H | -CH=N-CH=CH- | | H | H | H | H |
| CF₃ | CO₂CH₂CHF₂ | O | H | H | -S-CH=N- | | H | H | H | H |
| CF₃ | CO₂CH₂CHF₂ | O | H | H | -N=CH-S- | | H | H | H | H |
| CF₃ | CO₂(3-oxetanyl) | O | Cl | H | H | H | H | H | H | H |
| CF₃ | CO₂(3-oxetanyl) | O | Br | H | H | H | H | H | H | H |
| CF₃ | CO₂(3-oxetanyl) | O | I | H | H | H | H | H | H | H |
| CF₃ | CO₂(3-oxetanyl) | O | Me | H | H | H | H | H | H | H |
| CF₃ | CO₂(3-oxetanyl) | O | Et | H | H | H | H | H | H | H |
| CF₃ | CO₂(3-oxetanyl) | O | n-Pr | H | H | H | H | H | H | H |
| CF₃ | CO₂(3-oxetanyl) | O | i-Pr | H | H | H | H | H | H | H |
| CF₃ | CO₂(3-oxetanyl) | O | n-Bu | H | H | H | H | H | H | H |
| CF₃ | CO₂(3-oxetanyl) | O | s-Bu | H | H | H | H | H | H | H |
| CF₃ | CO₂(3-oxetanyl) | O | i-Bu | H | H | H | H | H | H | H |
| CF₃ | CO₂(3-oxetanyl) | O | t-Bu | H | H | H | H | H | H | H |

**[Table 42]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂(3-oxetanyl) | O | c-Pr | H | H | H | H | H | H | H |
| CF₃ | CO₂(3-oxetanyl) | O | c-Bu | H | H | H | H | H | H | H |
| CF₃ | CO₂(3-oxetanyl) | O | OMe | H | H | H | H | H | H | H |
| CF₃ | CO₂(3-oxetanyl) | O | OEt | H | H | H | H | H | H | H |
| CF₃ | CO₂(3-oxetanyl) | O | OCHF₂ | H | H | H | H | H | H | H |
| CF₃ | CO₂(3-oxetanyl) | O | OCF₃ | H | H | H | H | H | H | H |
| CF₃ | CO₂(3-oxetanyl) | O | OCH₂CH=CH₂ | H | H | H | H | H | H | H |
| CF₃ | CO₂(3-oxetanyl) | O | OCH₂C≡CH | H | H | H | H | H | H | H |
| CF₃ | CO₂(3-oxetanyl) | O | OCH₂c-Pr | H | H | H | H | H | H | H |
| CF₃ | CO₂(3-oxetanyl) | O | Bn | H | H | H | H | H | H | H |
| CF₃ | CO₂(3-oxetanyl) | O | 4-CIBn | H | H | H | H | H | H | H |
| CF₃ | CO₂(3-oxetanyl) | O | 4-MeBn | H | H | H | H | H | H | H |
| CF₃ | CO₂(3-oxetanyl) | O | 4-OMeBn | H | H | H | H | H | H | H |
| CF₃ | CO₂(3-oxetanyl) | O | OAc | H | H | H | H | H | H | H |
| CF₃ | CO₂(3-oxetanyl) | O | OBz | H | H | H | H | H | H | H |
| CF₃ | CO₂(3-oxetanyl) | O | O(4-ClBz) | H | H | H | H | H | H | H |
| CF₃ | CO₂(3-oxetanyl) | O | OCO₂Et | H | H | H | H | H | H | H |
| CF₃ | CO₂(3-oxetanyl) | O | OCO(SMe) | H | H | H | H | H | H | H |
| CF₃ | CO₂(3-oxetanyl) | O | OSO₂Me | H | H | H | H | H | H | H |
| CF₃ | CO₂(3-oxetanyl) | O | OSO₂CF₃ | H | H | H | H | H | H | H |
| CF₃ | CO₂(3-oxetanyl) | O | OSO₂Ph | H | H | H | H | H | H | H |
| CF₃ | CO₂(3-oxetanyl) | O | OPO(OEt)₂ | H | H | H | H | H | H | H |
| CF₃ | CO₂(3-oxetanyl) | O | OCH₂OMe | H | H | H | H | H | H | H |
| CF₃ | CO₂(3-oxetanyl) | O | SMe | H | H | H | H | H | H | H |
| CF₃ | CO₂(3-oxetanyl) | O | SEt | H | H | H | H | H | H | H |
| CF₃ | CO₂(3-oxetanyl) | O | SCF₃ | H | H | H | H | H | H | H |
| CF₃ | CO₂(3-oxetanyl) | O | SO₂Me | H | H | H | H | H | H | H |
| CF₃ | CO₂(3-oxetanyl) | O | SO₂CF₃ | H | H | H | H | H | H | H |
| CF₃ | CO₂(3-oxetanyl) | O | Ph | H | H | H | H | H | H | H |
| CF₃ | CO₂(3-oxetanyl) | O | 4-CIPh | H | H | H | H | H | H | H |

**[Table 43]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂(3-oxetanyl) | O | 4-MePh | H | H | H | H | H | H | H |
| CF₃ | CO₂(3-oxetanyl) | O | NMe₂ | H | H | H | H | H | H | H |
| CF₃ | CO₂(3-oxetanyl) | O | NHAc | H | H | H | H | H | H | H |
| CF₃ | CO₂(3-oxetanyl) | O | NAc₂ | H | H | H | H | H | H | H |
| CF₃ | CO₂(3-oxetanyl) | O | NHSO₂CF₃ | H | H | H | H | H | H | H |
| CF₃ | CO₂(3-oxetanyl) | O | N-morpholinyl | H | H | H | H | H | H | H |
| CF₃ | CO₂(3-oxetanyl) | O | CO₂Me | H | H | H | H | H | H | H |
| CF₃ | CO₂(3-oxetanyl) | O | CO₂Et | H | H | H | H | H | H | H |
| CF₃ | CO₂(3-oxetanyl) | O | OH | H | H | H | H | H | H | H |
| CF₃ | CO₂(3-oxetanyl) | O | CN | H | H | H | H | H | H | H |
| CF₃ | CO₂(3-oxetanyl) | O | NO₂ | H | H | H | H | H | H | H |
| CF₃ | CO₂(3-oxetanyl) | O | H | F | H | H | H | H | H | H |
| CF₃ | CO₂(3-oxetanyl) | O | H | CI | H | H | H | H | H | H |
| CF₃ | CO₂(3-oxetanyl) | O | H | Br | H | H | H | H | H | H |
| CF₃ | CO₂(3-oxetanyl) | O | H | I | H | H | H | H | H | H |
| CF₃ | CO₂(3-oxetanyl) | O | H | Me | H | H | H | H | H | H |
| CF₃ | CO₂(3-oxetanyl) | O | H | c-Pr | H | H | H | H | H | H |
| CF₃ | CO₂(3-oxetanyl) | O | H | OMe | H | H | H | H | H | H |
| CF₃ | CO₂(3-oxetanyl) | O | H | OCF₃ | H | H | H | H | H | H |
| CF₃ | CO₂(3-oxetanyl) | O | H | OCH₂C≡CH | H | H | H | H | H | H |
| CF₃ | CO₂(3-oxetanyl) | O | H | Bn | H | H | H | H | H | H |
| CF₃ | CO₂(3-oxetanyl) | O | H | OAc | H | H | H | H | H | H |
| CF₃ | CO₂(3-oxetanyl) | O | H | OSO₂Me | H | H | H | H | H | H |
| CF₃ | CO₂(3-oxetanyl) | O | H | SMe | H | H | H | H | H | H |
| CF₃ | CO₂(3-oxetanyl) | O | H | SO₂Me | H | H | H | H | H | H |
| CF₃ | CO₂(3-oxetanyl) | O | H | Ph | H | H | H | H | H | H |
| CF₃ | CO₂(3-oxetanyl) | O | H | NMe₂ | H | H | H | H | H | H |
| CF₃ | CO₂(3-oxetanyl) | O | H | CN | H | H | H | H | H | H |
| CF₃ | CO₂(3-oxetanyl) | O | H | NO₂ | H | H | H | H | H | H |
| CF₃ | CO₂(3-oxetanyl) | O | H | H | F | H | H | H | H | H |

**[Table 44]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂(3-oxetanyl) | O | H | H | Cl | H | H | H | H | H |
| CF₃ | CO₂(3-oxetanyl) | O | H | H | Br | H | H | H | H | H |
| CF₃ | CO₂(3-oxetanyl) | O | H | H | I | H | H | H | H | H |
| CF₃ | CO₂(3-oxetanyl) | O | H | H | Me | H | H | H | H | H |
| CF₃ | CO₂(3-oxetanyl) | O | H | H | c-Pr | H | H | H | H | H |
| CF₃ | CO₂(3-oxetanyl) | O | H | H | OMe | H | H | H | H | H |
| CF₃ | CO₂(3-oxetanyl) | O | H | H | OCF₃ | H | H | H | H | H |
| CF₃ | CO₂(3-oxetanyl) | O | H | H | OCH₂C≡CH | H | H | H | H | H |
| CF₃ | CO₂(3-oxetanyl) | O | H | H | Bn | H | H | H | H | H |
| CF₃ | CO₂(3-oxetanyl) | O | H | H | OAc | H | H | H | H | H |
| CF₃ | CO₂(3-oxetanyl) | O | H | H | OSO₂Me | H | H | H | H | H |
| CF₃ | CO₂(3-oxetanyl) | O | H | H | SMe | H | H | H | H | H |
| CF₃ | CO₂(3-oxetanyl) | O | H | H | SO₂Me | H | H | H | H | H |
| CF₃ | CO₂(3-oxetanyl) | O | H | H | Ph | H | H | H | H | H |
| CF₃ | CO₂(3-oxetanyl) | O | H | H | NMe2 | H | H | H | H | H |
| CF₃ | CO₂(3-oxetanyl) | O | H | H | CN | H | H | H | H | H |
| CF₃ | CO₂(3-oxetanyl) | O | H | H | NO₂ | H | H | H | H | H |
| CF₃ | CO₂(3-oxetanyl) | O | H | H | H | F | H | H | H | H |
| CF₃ | CO₂(3-oxetanyl) | O | H | H | H | Cl | H | H | H | H |
| CF₃ | CO₂(3-oxetanyl) | O | H | H | H | Br | H | H | H | H |
| CF₃ | CO₂(3-oxetanyl) | O | H | H | H | I | H | H | H | H |
| CF₃ | CO₂(3-oxetanyl) | O | H | H | H | Me | H | H | H | H |
| CF₃ | CO₂(3-oxetanyl) | O | H | H | H | c-Pr | H | H | H | H |
| CF₃ | CO₂(3-oxetanyl) | O | H | H | H | OMe | H | H | H | H |
| CF₃ | CO₂(3-oxetanyl) | O | H | H | H | OCF₃ | H | H | H | H |
| CF₃ | CO₂(3-oxetanyl) | O | H | H | H | OCH₂C≡CH | H | H | H | H |
| CF₃ | CO₂(3-oxetanyl) | O | H | H | H | Bn | H | H | H | H |
| CF₃ | CO₂(3-oxetanyl) | O | H | H | H | OAc | H | H | H | H |
| CF₃ | CO₂(3-oxetanyl) | O | H | H | H | OSO₂Me | H | H | H | H |
| CF₃ | CO₂(3-oxetanyl) | O | H | H | H | SMe | H | H | H | H |

**[Table 45]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂(3-oxetanyl) | O | H | H | H | SO₂Me | H | H | H | H |
| CF₃ | CO₂(3-oxetanyl) | O | H | H | H | Ph | H | H | H | H |
| CF₃ | CO₂(3-oxetanyl) | O | H | H | H | NMe₂ | H | H | H | H |
| CF₃ | CO₂(3-oxetanyl) | O | H | H | H | CN | H | H | H | H |
| CF₃ | CO₂(3-oxetanyl) | O | H | H | H | NO₂ | H | H | H | H |
| CF₃ | CO₂(3-oxetanyl) | O | F | F | H | H | H | H | H | H |
| CF₃ | CO₂(3-oxetanyl) | O | F | H | F | H | H | H | H | H |
| CF₃ | CO₂(3-oxetanyl) | O | F | CI | H | H | H | H | H | H |
| CF₃ | CO₂(3-oxetanyl) | O | F | H | Cl | H | H | H | H | H |
| CF₃ | CO₂(3-oxetanyl) | O | F | H | H | Cl | H | H | H | H |
| CF₃ | CO₂(3-oxetanyl) | O | F | H | H | Br | H | H | H | H |
| CF₃ | CO₂(3-oxetanyl) | O | F | H | H | I | H | H | H | H |
| CF₃ | CO₂(3-oxetanyl) | O | F | H | H | Me | H | H | H | H |
| CF₃ | CO₂(3-oxetanyl) | O | F | H | H | c-Pr | H | H | H | H |
| CF₃ | CO₂(3-oxetanyl) | O | F | H | H | OMe | H | H | H | H |
| CF₃ | CO₂(3-oxetanyl) | O | F | H | H | OCF₃ | H | H | H | H |
| CF₃ | CO₂(3-oxetanyl) | O | F | H | H | OCH₂C≡CH | H | H | H | H |
| CF₃ | CO₂(3-oxetanyl) | O | F | H | H | OSO₂Me | H | H | H | H |
| CF₃ | CO₂(3-oxetanyl) | O | F | H | H | SMe | H | H | H | H |
| CF₃ | CO₂(3-oxetanyl) | O | F | H | H | SO₂Me | H | H | H | H |
| CF₃ | CO₂(3-oxetanyl) | O | F | H | H | NMe₂ | H | H | H | H |
| CF₃ | CO₂(3-oxetanyl) | O | F | H | H | CN | H | H | H | H |
| CF₃ | CO₂(3-oxetanyl) | O | F | H | H | NO₂ | H | H | H | H |
| CF₃ | CO₂(3-oxetanyl) | O | F | F | F | H | H | H | H | H |
| CF₃ | CO₂(3-oxetanyl) | O | F | F | H | F | H | H | H | H |
| CF₃ | CO₂(3-oxetanyl) | O | F | H | F | F | H | H | H | H |
| CF₃ | CO₂(3-oxetanyl) | O | F | F | F | F | H | H | H | H |
| CF₃ | CO₂(3-oxetanyl) | O | Cl | F | H | H | H | H | H | H |
| CF₃ | CO₂(3-oxetanyl) | O | Cl | H | F | H | H | H | H | H |
| CF₃ | CO₂(3-oxetanyl) | O | Cl | H | H | F | H | H | H | H |

**[Table 46]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂(3-oxetanyl) | O | Cl | Cl | H | H | H | H | H | H |
| CF₃ | CO₂(3-oxetanyl) | O | Cl | H | Cl | H | H | H | H | H |
| CF₃ | CO₂(3-oxetanyl) | O | Cl | H | H | Cl | H | H | H | H |
| CF₃ | CO₂(3-oxetanyl) | O | Cl | H | H | Br | H | H | H | H |
| CF₃ | CO₂(3-oxetanyl) | O | Cl | H | H | I | H | H | H | H |
| CF₃ | CO₂(3-oxetanyl) | O | Cl | H | H | Me | H | H | H | H |
| CF₃ | CO₂(3-oxetanyl) | O | CI | H | H | c-Pr | H | H | H | H |
| CF₃ | CO₂(3-oxetanyl) | O | Cl | H | H | OMe | H | H | H | H |
| CF₃ | CO₂(3-oxetanyl) | O | Cl | H | H | OCF₃ | H | H | H | H |
| CF₃ | CO₂(3-oxetanyl) | O | Cl | H | H | OCH₂C≡CH | H | H | H | H |
| CF₃ | CO₂(3-oxetanyl) | O | CI | H | H | OSO₂Me | H | H | H | H |
| CF₃ | CO₂(3-oxetanyl) | O | Cl | H | H | SMe | H | H | H | H |
| CF₃ | CO₂(3-oxetanyl) | O | Cl | H | H | SO₂Me | H | H | H | H |
| CF₃ | CO₂(3-oxetanyl) | O | Cl | H | H | NMe₂ | H | H | H | H |
| CF₃ | CO₂(3-oxetanyl) | O | Cl | H | H | CN | H | H | H | H |
| CF₃ | CO₂(3-oxetanyl) | O | Cl | H | H | NO₂ | H | H | H | H |
| CF₃ | CO₂(3-oxetanyl) | O | Br | H | H | F | H | H | H | H |
| CF₃ | CO₂(3-oxetanyl) | O | Br | H | H | Cl | H | H | H | H |
| CF₃ | CO₂(3-oxetanyl) | O | Br | H | H | Me | H | H | H | H |
| CF₃ | CO₂(3-oxetanyl) | O | Br | H | H | OMe | H | H | H | H |
| CF₃ | CO₂(3-oxetanyl) | O | Me | H | H | F | H | H | H | H |
| CF₃ | CO₂(3-oxetanyl) | O | Me | H | H | Cl | H | H | H | H |
| CF₃ | CO₂(3-oxetanyl) | O | Me | H | H | Me | H | H | H | H |
| CF₃ | CO₂(3-oxetanyl) | O | Me | H | H | OMe | H | H | H | H |
| CF₃ | CO₂(3-oxetanyl) | O | OMe | H | H | F | H | H | H | H |
| CF₃ | CO₂(3-oxetanyl) | O | OMe | H | H | Cl | H | H | H | H |
| CF₃ | CO₂(3-oxetanyl) | O | OMe | H | H | Me | H | H | H | H |
| CF₃ | CO₂(3-oxetanyl) | O | OMe | H | H | OMe | H | H | H | H |
| CF₃ | CO₂(3-oxetanyl) | O | H | F | F | H | H | H | H | H |
| CF₃ | CO₂(3-oxetanyl) | O | H | F | CI | H | H | H | H | H |

**[Table 47]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂(3-oxetanyl) | O | H | F | H | F | H | H | H | H |
| CF₃ | CO₂(3-oxetanyl) | O | H | F | H | Cl | H | H | H | H |
| CF₃ | CO₂(3-oxetanyl) | O | H | Cl | Cl | H | H | H | H | H |
| CF₃ | CO₂(3-oxetanyl) | O | H | Cl | F | H | H | H | H | H |
| CF₃ | CO₂(3-oxetanyl) | O | H | Cl | H | F | H | H | H | H |
| CF₃ | CO₂(3-oxetanyl) | O | H | Cl | H | Cl | H | H | H | H |
| CF₃ | CO₂(3-oxetanyl) | O | H | H | F | F | H | H | H | H |
| CF₃ | CO₂(3-oxetanyl) | O | H | H | F | Cl | H | H | H | H |
| CF₃ | CO₂(3-oxetanyl) | O | H | H | Cl | Cl | H | H | H | H |
| CF₃ | CO₂(3-oxetanyl) | O | F | F | F | F | H | H | H | H |
| CF₃ | CO₂(3-oxetanyl) | O | Cl | Cl | Cl | Cl | H | H | H | H |
| CF₃ | CO₂(3-oxetanyl) | O | -CH=CH-CH=CH- | | H | H | H | H | H | H |
| CF₃ | CO₂(3-oxetanyl) | O | -CH=N-CH=CH- | | H | H | H | H | H | H |
| CF₃ | CO₂(3-oxetanyl) | O | -S-CH=N- | | H | H | H | H | H | H |
| CF₃ | CO₂(3-oxetanyl) | O | -N=CH-S- | | H | H | H | H | H | H |
| CF₃ | CO₂(3-oxetanyl) | O | H | -CH=CH-CH=CH- | | H | H | H | H | H |
| CF₃ | CO₂(3-oxetanyl) | O | H | -CH=N-CH=CH- | | H | H | H | H | H |
| CF₃ | CO₂(3-oxetanyl) | O | H | -S-CH=N- | | H | H | H | H | H |
| CF₃ | CO₂(3-oxetanyl) | O | H | -N=CH-S- | | H | H | H | H | H |
| CF₃ | CO₂(3-oxetanyl) | O | H | H | -CH=CH-CH=CH- | | H | H | H | H |
| CF₃ | CO₂(3-oxetanyl) | O | H | H | -CH=N-CH=CH- | | H | H | H | H |
| CF₃ | CO₂(3-oxetanyl) | O | H | H | -S-CH=N- | | H | H | H | H |
| CF₃ | CO₂(3-oxetanyl) | O | H | H | -N=CH-S- | | H | H | H | H |
| CF₃ | CO₂(3-tetrahydrofuryl) | O | Cl | H | H | H | H | H | H | H |
| CF₃ | CO₂(3-tetrahydrofuryl) | O | Br | H | H | H | H | H | H | H |
| CF₃ | CO₂(3-tetrahydrofuryl) | O | I | H | H | H | H | H | H | H |
| CF₃ | CO₂(3-tetrahydrofuryl) | O | Me | H | H | H | H | H | H | H |
| CF₃ | CO₂(3-tetrahydrofuryl) | O | Et | H | H | H | H | H | H | H |
| CF₃ | CO₂(3-tetrahydrofuryl) | O | n-Pr | H | H | H | H | H | H | H |
| CF₃ | CO₂(3-tetrahydrofuryl) | O | i-Pr | H | H | H | H | H | H | H |

**[Table 48]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂(3-tetrahydrofuryl) | O | n-Bu | H | H | H | H | H | H | H |
| CF₃ | CO₂(3-tetrahydrofuryl) | O | s-Bu | H | H | H | H | H | H | H |
| CF₃ | CO₂(3-tetrahydrofuryl) | O | i-Bu | H | H | H | H | H | H | H |
| CF₃ | CO₂(3-tetrahydrofuryl) | O | t-Bu | H | H | H | H | H | H | H |
| CF₃ | CO₂(3-tetrahydrofuryl) | O | c-Pr | H | H | H | H | H | H | H |
| CF₃ | CO₂(3-tetrahydrofuryl) | O | c-Bu | H | H | H | H | H | H | H |
| CF₃ | CO₂(3-tetrahydrofuryl) | O | OMe | H | H | H | H | H | H | H |
| CF₃ | CO₂(3-tetrahydrofuryl) | O | OEt | H | H | H | H | H | H | H |
| CF₃ | CO₂(3-tetrahydrofuryl) | O | OCHF₂ | H | H | H | H | H | H | H |
| CF₃ | CO₂(3-tetrahydrofuryl) | O | OCF₃ | H | H | H | H | H | H | H |
| CF₃ | CO₂(3-tetrahydrofuryl) | O | OCH₂CH=CH₂ | H | H | H | H | H | H | H |
| CF₃ | CO₂(3-tetrahydrofuryl) | O | OCH₂C≡CN | H | H | H | H | H | H | H |
| CF₃ | CO₂(3-tetrahydrofuryl) | O | OCH₂c-Pr | H | H | H | H | H | H | H |
| CF₃ | CO₂(3-tetrahydrofuryl) | O | Bn | H | H | H | H | H | H | H |
| CF₃ | CO₂(3-tetrahydrofuryl) | O | 4-CIBn | H | H | H | H | H | H | H |
| CF₃ | CO₂(3-tetrahydrofuryl) | O | 4-MeBn | H | H | H | H | H | H | H |
| CF₃ | CO₂(3-tetrahydrofuryl) | O | 4-OMeBn | H | H | H | H | H | H | H |
| CF₃ | CO₂(3-tetrahydrofuryl) | O | OAc | H | H | H | H | H | H | H |
| CF₃ | CO₂(3-tetrahydrofuryl) | O | OBz | H | H | H | H | H | H | H |
| CF₃ | CO₂(3-tetrahydrofuryl) | O | O(4-ClBz) | H | H | H | H | H | H | H |
| CF₃ | CO₂(3-tetrahydrofuryl) | O | OCO₂Et | H | H | H | H | H | H | H |
| CF₃ | CO₂(3-tetrahydrofuryl) | O | OCO(SMe) | H | H | H | H | H | H | H |
| CF₃ | CO₂(3-tetrahydrofuryl) | O | OSO₂Me | H | H | H | H | H | H | H |
| CF₃ | CO₂(3-tetrahydrofuryl) | O | OSO₂CF₃ | H | H | H | H | H | H | H |
| CF₃ | CO₂(3-tetrahydrofuryl) | O | OSO₂Ph | H | H | H | H | H | H | H |
| CF₃ | CO₂(3-tetrahydrofuryl) | O | OPO(OEt)₂ | H | H | H | H | H | H | H |
| CF₃ | CO₂(3-tetrahydrofuryl) | O | OCH₂OMe | H | H | H | H | H | H | H |
| CF₃ | CO₂(3-tetrahydrofuryl) | O | SMe | H | H | H | H | H | H | H |
| CF₃ | CO₂(3-tetrahydrofuryl) | O | SEt | H | H | H | H | H | H | H |
| CF₃ | CO₂(3-tetrahydrofuryl) | O | SCF₃ | H | H | H | H | H | H | H |

**[Table 49]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂(3-tetrahydrofuryl) | O | SO₂Me | H | H | H | H | H | H | H |
| CF₃ | CO₂(3-tetrahydrofuryl) | O | SO₂CF₃ | H | H | H | H | H | H | H |
| CF₃ | CO₂(3-tetrahydrofuryl) | O | Ph | H | H | H | H | H | H | H |
| CF₃ | CO₂(3-tetrahydrofuryl) | O | 4-CIPh | H | H | H | H | H | H | H |
| CF₃ | CO₂(3-tetrahydrofuryl) | O | 4-MePh | H | H | H | H | H | H | H |
| CF₃ | CO₂(3-tetrahydrofuryl) | O | NMe₂ | H | H | H | H | H | H | H |
| CF₃ | CO₂(3-tetrahydrofuryl) | O | NHAc | H | H | H | H | H | H | H |
| CF₃ | CO₂(3-tetrahydrofuryl) | O | NAc₂ | H | H | H | H | H | H | H |
| CF₃ | CO₂(3-tetrahydrofuryl) | O | NHSO₂CF₃ | H | H | H | H | H | H | H |
| CF₃ | CO₂(3-tetrahydrofuryl) | O | N-morpholinyl | H | H | H | H | H | H | H |
| CF₃ | CO₂(3-tetrahydrofuryl) | O | CO₂Me | H | H | H | H | H | H | H |
| CF₃ | CO₂(3-tetrahydrofuryl) | O | CO₂Et | H | H | H | H | H | H | H |
| CF₃ | CO₂(3-tetrahydrofuryl) | O | OH | H | H | H | H | H | H | H |
| CF₃ | CO₂(3-tetrahydrofuryl) | O | CN | H | H | H | H | H | H | H |
| CF₃ | CO₂(3-tetrahydrofuryl) | O | NO₂ | H | H | H | H | H | H | H |
| CF₃ | CO₂(3-tetrahydrofuryl) | O | H | F | H | H | H | H | H | H |
| CF₃ | CO₂(3-tetrahydrofuryl) | O | H | Cl | H | H | H | H | H | H |
| CF₃ | CO₂(3-tetrahydrofuryl) | O | H | Br | H | H | H | H | H | H |
| CF₃ | CO₂(3-tetrahydrofuryl) | O | H | I | H | H | H | H | H | H |
| CF₃ | CO₂(3-tetrahydrofuryl) | O | H | Me | H | H | H | H | H | H |
| CF₃ | CO₂(3-tetrahydrofuryl) | O | H | c-Pr | H | H | H | H | H | H |
| CF₃ | CO₂(3-tetrahydrofuryl) | O | H | OMe | H | H | H | H | H | H |
| CF₃ | CO₂(3-tetrahydrofuryl) | O | H | OCF₃ | H | H | H | H | H | H |
| CF₃ | CO₂(3-tetrahydrofuryl) | O | H | OCH₂C≡CH | H | H | H | H | H | H |
| CF₃ | CO₂(3-tetrahydrofuryl) | O | H | Bn | H | H | H | H | H | H |
| CF₃ | CO₂(3-tetrahydrofuryl) | O | H | OAc | H | H | H | H | H | H |
| CF₃ | CO₂(3-tetrahydrofuryl) | O | H | OSO₂Me | H | H | H | H | H | H |
| CF₃ | CO₂(3-tetrahydrofuryl) | O | H | SMe | H | H | H | H | H | H |
| CF₃ | CO₂(3-tetrahydrofuryl) | O | H | SO₂Me | H | H | H | H | H | H |
| CF₃ | CO₂(3-tetrahydrofuryl) | O | H | Ph | H | H | H | H | H | H |

**[Table 50]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂(3-tetrahydrofuryl) | O | H | NMe₂ | H | H | H | H | H | H |
| CF₃ | CO₂(3-tetrahydrofuryl) | O | H | CN | H | H | H | H | H | H |
| CF₃ | CO₂(3-tetrahydrofuryl) | O | H | NO₂ | H | H | H | H | H | H |
| CF₃ | CO₂(3-tetrahydrofuryl) | O | H | H | F | H | H | H | H | H |
| CF₃ | CO₂(3-tetrahydrofuryl) | O | H | H | CI | H | H | H | H | H |
| CF₃ | CO₂(3-tetrahydrofuryl) | O | H | H | Br | H | H | H | H | H |
| CF₃ | CO₂(3-tetrahydrofuryl) | O | H | H | I | H | H | H | H | H |
| CF₃ | CO₂(3-tetrahydrofuryl) | O | H | H | Me | H | H | H | H | H |
| CF₃ | CO₂(3-tetrahydrofuryl) | O | H | H | c-Pr | H | H | H | H | H |
| CF₃ | CO₂(3-tetrahydrofuryl) | O | H | H | OMe | H | H | H | H | H |
| CF₃ | CO₂(3-tetrahydrofuryl) | O | H | H | OCF₃ | H | H | H | H | H |
| CF₃ | CO₂(3-tetrahydrofuryl) | O | H | H | OCH₂C≡CH | H | H | H | H | H |
| CF₃ | CO₂(3-tetrahydrofuryl) | O | H | H | Bn | H | H | H | H | H |
| CF₃ | CO₂(3-tetrahydrofuryl) | O | H | H | OAc | H | H | H | H | H |
| CF₃ | CO₂(3-tetrahydrofuryl) | O | H | H | OSO₂Me | H | H | H | H | H |
| CF₃ | CO₂(3-tetrahydrofuryl) | O | H | H | SMe | H | H | H | H | H |
| CF₃ | CO₂(3-tetrahydrofuryl) | O | H | H | SO₂Me | H | H | H | H | H |
| CF₃ | CO₂(3-tetrahydrofuryl) | O | H | H | Ph | H | H | H | H | H |
| CF₃ | CO₂(3-tetrahydrofuryl) | O | H | H | NMe₂ | H | H | H | H | H |
| CF₃ | CO₂(3-tetrahydrofuryl) | O | H | H | CN | H | H | H | H | H |
| CF₃ | CO₂(3-tetrahydrofuryl) | O | H | H | NO₂ | H | H | H | H | H |
| CF₃ | CO₂(3-tetrahydrofuryl) | O | H | H | H | F | H | H | H | H |
| CF₃ | CO₂(3-tetrahydrofuryl) | O | H | H | H | Cl | H | H | H | H |
| CF₃ | CO₂(3-tetrahydrofuryl) | O | H | H | H | Br | H | H | H | H |
| CF₃ | CO₂(3-tetrahydrofuryl) | O | H | H | H | I | H | H | H | H |
| CF₃ | CO₂(3-tetrahydrofuryl) | O | H | H | H | Me | H | H | H | H |
| CF₃ | CO₂(3-tetrahydrofuryl) | O | H | H | H | c-Pr | H | H | H | H |
| CF₃ | CO₂(3-tetrahydrofuryl) | O | H | H | H | OMe | H | H | H | H |
| CF₃ | CO₂(3-tetrahydrofuryl) | O | H | H | H | OCF₃ | H | H | H | H |
| CF₃ | CO₂(3-tetrahydrofuryl) | O | H | H | H | OCH₂C≡CH | H | H | H | H |

**[Table 51]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂(3-tetrahydrofuryl) | O | H | H | H | Bn | H | H | H | H |
| CF₃ | CO₂(3-tetrahydrofuryl) | O | H | H | H | OAc | H | H | H | H |
| CF₃ | CO₂(3-tetrahydrofuryl) | O | H | H | H | OSO₂Me | H | H | H | H |
| CF₃ | CO₂(3-tetrahydrofuryl) | O | H | H | H | SMe | H | H | H | H |
| CF₃ | CO₂(3-tetrahydrofuryl) | O | H | H | H | SO₂Me | H | H | H | H |
| CF₃ | CO₂(3-tetrahydrofuryl) | O | H | H | H | Ph | H | H | H | H |
| CF₃ | CO₂(3-tetrahydrofuryl) | O | H | H | H | NMe₂ | H | H | H | H |
| CF₃ | CO₂(3-tetrahydrofuryl) | O | H | H | H | CN | H | H | H | H |
| CF₃ | CO₂(3-tetrahydrofuryl) | O | H | H | H | NO₂ | H | H | H | H |
| CF₃ | CO₂(3-tetrahydrofuryl) | O | F | F | H | H | H | H | H | H |
| CF₃ | CO₂(3-tetrahydrofuryl) | O | F | H | F | H | H | H | H | H |
| CF₃ | CO₂(3-tetrahydrofuryl) | O | F | Cl | H | H | H | H | H | H |
| CF₃ | CO₂(3-tetrahydrofuryl) | O | F | H | Cl | H | H | H | H | H |
| CF₃ | CO₂(3-tetrahydrofuryl) | O | F | H | H | Cl | H | H | H | H |
| CF₃ | CO₂(3-tetrahydrofuryl) | O | F | H | H | Br | H | H | H | H |
| CF₃ | CO₂(3-tetrahydrofuryl) | O | F | H | H | I | H | H | H | H |
| CF₃ | CO₂(3-tetrahydrofuryl) | O | F | H | H | Me | H | H | H | H |
| CF₃ | CO₂(3-tetrahydrofuryl) | O | F | H | H | c-Pr | H | H | H | H |
| CF₃ | CO₂(3-tetrahydrofuryl) | O | F | H | H | OMe | H | H | H | H |
| CF₃ | CO₂(3-tetrahydrofuryl) | O | F | H | H | OCF₃ | H | H | H | H |
| CF₃ | CO₂(3-tetrahydrofuryl) | O | F | H | H | OCH₂C≡CH | H | H | H | H |
| CF₃ | CO₂(3-tetrahydrofuryl) | O | F | H | H | OSO₂Me | H | H | H | H |
| CF₃ | CO₂(3-tetrahydrofuryl) | O | F | H | H | SMe | H | H | H | H |
| CF₃ | CO₂(3-tetrahydrofuryl) | O | F | H | H | SO₂Me | H | H | H | H |
| CF₃ | CO₂(3-tetrahydrofuryl) | O | F | H | H | NMe₂ | H | H | H | H |
| CF₃ | CO₂(3-tetrahydrofuryl) | O | F | H | H | CN | H | H | H | H |
| CF₃ | CO₂(3-tetrahydrofuryl) | O | F | H | H | NO₂ | H | H | H | H |
| CF₃ | CO₂(3-tetrahydrofuryl) | O | F | F | F | H | H | H | H | H |
| CF₃ | CO₂(3-tetrahydrofuryl) | O | F | F | H | F | H | H | H | H |
| CF₃ | CO₂(3-tetrahydrofuryl) | O | F | H | F | F | H | H | H | H |

**[Table 52]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂(3-tetrahydrofuryl) | O | F | F | F | F | H | H | H | H |
| CF₃ | CO₂(3-tetrahydrofuryl) | O | Cl | F | H | H | H | H | H | H |
| CF₃ | CO₂(3-tetrahydrofuryl) | O | Cl | H | F | H | H | H | H | H |
| CF₃ | CO₂(3-tetrahydrofuryl) | O | Cl | H | H | F | H | H | H | H |
| CF₃ | CO₂(3-tetrahydrofuryl) | O | Cl | Cl | H | H | H | H | H | H |
| CF₃ | CO₂(3-tetrahydrofuryl) | O | Cl | H | Cl | H | H | H | H | H |
| CF₃ | CO₂(3-tetrahydrofuryl) | O | Cl | H | H | Cl | H | H | H | H |
| CF₃ | CO₂(3-tetrahydrofuryl) | O | Cl | H | H | Br | H | H | H | H |
| CF₃ | CO₂(3-tetrahydrofuryl) | O | Cl | H | H | I | H | H | H | H |
| CF₃ | CO₂(3-tetrahydrofuryl) | O | Cl | H | H | Me | H | H | H | H |
| CF₃ | CO₂(3-tetrahydrofuryl) | O | Cl | H | H | c-Pr | H | H | H | H |
| CF₃ | CO₂(3-tetrahydrofuryl) | O | Cl | H | H | OMe | H | H | H | H |
| CF₃ | CO₂(3-tetrahydrofuryl) | O | Cl | H | H | OCF₃ | H | H | H | H |
| CF₃ | CO₂(3-tetrahydrofuryl) | O | Cl | H | H | OCH₂C≡CH | H | H | H | H |
| CF₃ | CO₂(3-tetrahydrofuryl) | O | Cl | H | H | OSO₂Me | H | H | H | H |
| CF₃ | CO₂(3-tetrahydrofuryl) | O | Cl | H | H | SMe | H | H | H | H |
| CF₃ | CO₂(3-tetrahydrofuryl) | O | Cl | H | H | SO₂Me | H | H | H | H |
| CF₃ | CO₂(3-tetrahydrofuryl) | O | Cl | H | H | NMe₂ | H | H | H | H |
| CF₃ | CO₂(3-tetrahydrofuryl) | O | Cl | H | H | CN | H | H | H | H |
| CF₃ | CO₂(3-tetrahydrofuryl) | O | Cl | H | H | NO₂ | H | H | H | H |
| CF₃ | CO₂(3-tetrahydrofuryl) | O | Br | H | H | F | H | H | H | H |
| CF₃ | CO₂(3-tetrahydrofuryl) | O | Br | H | H | Cl | H | H | H | H |
| CF₃ | CO₂(3-tetrahydrofuryl) | O | Br | H | H | Me | H | H | H | H |
| CF₃ | CO₂(3-tetrahydrofuryl) | O | Br | H | H | OMe | H | H | H | H |
| CF₃ | CO₂(3-tetrahydrofuryl) | O | Me | H | H | F | H | H | H | H |
| CF₃ | CO₂(3-tetrahydrofuryl) | O | Me | H | H | Cl | H | H | H | H |
| CF₃ | CO₂(3-tetrahydrofuryl) | O | Me | H | H | Me | H | H | H | H |
| CF₃ | CO₂(3-tetrahydrofuryl) | O | Me | H | H | OMe | H | H | H | H |
| CF₃ | CO₂(3-tetrahydrofuryl) | O | OMe | H | H | F | H | H | H | H |
| CF₃ | CO₂(3-tetrahydrofuryl) | O | OMe | H | H | Cl | H | H | H | H |

**[Table 53]**

| R¹ | R² | W | X¹ | X² | X³ | X⁴ | Y¹ | Y² | Y³ | Y⁴ |
|---|---|---|---|---|---|---|---|---|---|---|
| CF₃ | CO₂(3-tetrahydrofuryl) | O | OMe | H | H | Me | H | H | H | H |
| CF₃ | CO₂(3-tetrahydrofuryl) | O | OMe | H | H | OMe | H | H | H | H |
| CF₃ | CO₂(3-tetrahydrofuryl) | O | H | F | F | H | H | H | H | H |
| CF₃ | CO₂(3-tetrahydrofuryl) | O | H | F | Cl | H | H | H | H | H |
| CF₃ | CO₂(3-tetrahydrofuryl) | O | H | F | H | F | H | H | H | H |
| CF₃ | CO₂(3-tetrahydrofuryl) | O | H | F | H | Cl | H | H | H | H |
| CF₃ | CO₂(3-tetrahydrofuryl) | O | H | Cl | Cl | H | H | H | H | H |
| CF₃ | CO₂(3-tetrahydrofuryl) | O | H | Cl | F | H | H | H | H | H |
| CF₃ | CO₂(3-tetrahydrofuryl) | O | H | Cl | H | F | H | H | H | H |
| CF₃ | CO₂(3-tetrahydrofuryl) | O | H | Cl | H | Cl | H | H | H | H |
| CF₃ | CO₂(3-tetrahydrofuryl) | O | H | H | F | F | H | H | H | H |
| CF₃ | CO₂(3-tetrahydrofuryl) | O | H | H | F | Cl | H | H | H | H |
| CF₃ | CO₂(3-tetrahydrofuryl) | O | H | H | Cl | Cl | H | H | H | H |
| CF₃ | CO₂(3-tetrahydrofuryl) | O | F | F | F | F | H | H | H | H |
| CF₃ | CO₂(3-tetrahydrofuryl) | O | Cl | Cl | Cl | Cl | H | H | H | H |
| CF₃ | CO₂(3-tetrahydrofuryl) | O | -CH=CH-CH=CH- | | H | H | H | H | H | H |
| CF₃ | CO₂(3-tetrahydrofuryl) | O | -CH=N-CH=CH- | | H | H | H | H | H | H |
| CF₃ | CO₂(3-tetrahydrofuryl) | O | -S-CH=N- | | H | H | H | H | H | H |
| CF₃ | CO₂(3-tetrahydrofuryl) | O | -N=CH-S- | | H | H | H | H | H | H |
| CF₃ | CO₂(3-tetrahydrofuryl) | O | H | -CH=CH-CH=CH- | | H | H | H | H | H |
| CF₃ | CO₂(3-tetrahydrofuryl) | O | H | -CH=N-CH=CH- | | H | H | H | H | H |
| CF₃ | CO₂(3-tetrahydrofuryl) | O | H | -S-CH=N- | | H | H | H | H | H |
| CF₃ | CO₂(3-tetrahydrofuryl) | O | H | -N=CH-S- | | H | H | H | H | H |
| CF3 | CO₂(3-tetrahydrofuryl) | O | H | H | -CH=CH-CH=CH- | | H | H | H | H |
| CF₃ | CO₂(3-tetrahydrofuryl) | O | H | H | -CH=N-CH=CH- | | H | H | H | H |
| CF₃ | CO₂(3-tetrahydrofuryl) | O | H | H | -S-CH=N- | | H | H | H | H |
| CF₃ | CO₂(3-tetrahydrofuryl) | O | H | H | -N=CH-S- | | H | H | H | H |
| CF₃ | SO₂Me | O | Cl | H | H | H | H | H | H | H |
| CF₃ | SO₂Me | O | Br | H | H | H | H | H | H | H |
| CF₃ | SO₂Me | O | I | H | H | H | H | H | H | H |

**[Table 54]**

| R¹ | R² | W | X¹ | X² | X³ | X⁴ | Y¹ | Y² | Y³ | Y⁴ |
|---|---|---|---|---|---|---|---|---|---|---|
| CF₃ | SO₂Me | O | Me | H | H | H | H | H | H | H |
| CF₃ | SO₂Me | O | Et | H | H | H | H | H | H | H |
| CF₃ | SO₂Me | O | n-Pr | H | H | H | H | H | H | H |
| CF₃ | SO₂Me | O | i-Pr | H | H | H | H | H | H | H |
| CF₃ | SO₂Me | O | n-Bu | H | H | H | H | H | H | H |
| CF₃ | SO₂Me | O | s-Bu | H | H | H | H | H | H | H |
| CF₃ | SO₂Me | O | i-Bu | H | H | H | H | H | H | H |
| CF₃ | SO₂Me | O | t-Bu | H | H | H | H | H | H | H |
| CF₃ | SO₂Me | O | c-Pr | H | H | H | H | H | H | H |
| CF₃ | S0₂Me | O | c-Bu | H | H | H | H | H | H | H |
| CF₃ | SO₂Me | O | OMe | H | H | H | H | H | H | H |
| CF₃ | SO₂Me | O | OEt | H | H | H | H | H | H | H |
| CF₃ | SO₂Me | O | OCHF₂ | H | H | H | H | H | H | H |
| CF₃ | SO₂Me | O | OCF₃ | H | H | H | H | H | H | H |
| CF₃ | SO₂Me | O | OCH₂CH=CH₂ | H | H | H | H | H | H | H |
| CF₃ | SO₂Me | O | OCH₂C≡CH | H | H | H | H | H | H | H |
| CF₃ | SO₂Me | O | OCH₂c-Pr | H | H | H | H | H | H | H |
| CF₃ | SO₂Me | O | Bn | H | H | H | H | H | H | H |
| CF₃ | SO₂Me | O | 4-CIBn | H | H | H | H | H | H | H |
| CF₃ | SO₂Me | O | 4-MeBn | H | H | H | H | H | H | H |
| CF₃ | SO₂Me | O | 4-OMeBn | H | H | H | H | H | H | H |
| CF₃ | SO₂Me | O | OAc | H | H | H | H | H | H | H |
| CF₃ | SO₂Me | O | OBz | H | H | H | H | H | H | H |
| CF₃ | SO₂Me | O | O(4-ClBz) | H | H | H | H | H | H | H |
| CF₃ | SO₂Me | O | OCO₂Et | H | H | H | H | H | H | H |
| CF₃ | SO₂Me | O | OCO(SMe) | H | H | H | H | H | H | H |
| CF₃ | SO₂Me | O | OSO₂Me | H | H | H | H | H | H | H |
| CF₃ | SO₂Me | O | OSO₂CF₃ | H | H | H | H | H | H | H |
| CF₃ | SO₂Me | O | OSO₂Ph | H | H | H | H | H | H | H |
| CF₃ | SO₂Me | O | OPO(OEt)₂ | H | H | H | H | H | H | H |

**[Table 55]**

| R¹ | R² | W | X¹ | X² | X³ | X⁴ | Y¹ | Y² | Y³ | Y⁴ |
|---|---|---|---|---|---|---|---|---|---|---|
| CF₃ | SO₂Me | O | OCH₂OMe | H | H | H | H | H | H | H |
| CF₃ | SO₂Me | O | SMe | H | H | H | H | H | H | H |
| CF₃ | SO₂Me | O | SEt | H | H | H | H | H | H | H |
| CF₃ | SO₂Me | O | SCF₃ | H | H | H | H | H | H | H |
| CF₃ | SO₂Me | O | SO₂Me | H | H | H | H | H | H | H |
| CF₃ | SO₂Me | O | SO₂CF₃ | H | H | H | H | H | H | H |
| CF₃ | SO₂Me | O | Ph | H | H | H | H | H | H | H |
| CF₃ | SO₂Me | O | 4-ClPh | H | H | H | H | H | H | H |
| CF₃ | SO₂Me | O | 4-MePh | H | H | H | H | H | H | H |
| CF₃ | SO₂Me | O | NMe₂ | H | H | H | H | H | H | H |
| CF₃ | SO₂Me | O | NHAc | H | H | H | H | H | H | H |
| CF₃ | SO₂Me | O | NAc₂ | H | H | H | H | H | H | H |
| CF₃ | SO₂Me | O | NHSO₂CF₃ | H | H | H | H | H | H | H |
| CF₃ | SO₂Me | O | N-morpholinyl | H | H | H | H | H | H | H |
| CF₃ | SO₂Me | O | CO₂Me | H | H | H | H | H | H | H |
| CF₃ | SO₂Me | O | CO₂Et | H | H | H | H | H | H | H |
| CF₃ | SO₂Me | O | OH | H | H | H | H | H | H | H |
| CF₃ | SO₂Me | O | CN | H | H | H | H | H | H | H |
| CF₃ | SO₂Me | O | NO₂ | H | H | H | H | H | H | H |
| CF₃ | SO₂Me | O | H | F | H | H | H | H | H | H |
| CF₃ | SO₂Me | O | H | Cl | H | H | H | H | H | H |
| CF₃ | SO₂Me | O | H | Br | H | H | H | H | H | H |
| CF₃ | SO₂Me | O | H | I | H | H | H | H | H | H |
| CF₃ | SO₂Me | O | H | Me | H | H | H | H | H | H |
| CF₃ | SO₂Me | O | H | c-Pr | H | H | H | H | H | H |
| CF₃ | SO₂Me | O | H | OMe | H | H | H | H | H | H |
| CF₃ | SO₂Me | O | H | OCF₃ | H | H | H | H | H | H |
| CF₃ | SO₂Me | O | H | OCH₂C≡CH | H | H | H | H | H | H |
| CF₃ | SO₂Me | O | H | Bn | H | H | H | H | H | H |
| CF₃ | SO₂Me | O | H | OAc | H | H | H | H | H | H |

**[Table 56]**

| R¹ | R² | W | X¹ | X² | X³ | X⁴ | Y¹ | Y² | Y³ | Y⁴ |
|---|---|---|---|---|---|---|---|---|---|---|
| CF₃ | SO₂Me | O | H | OSO₂Me | H | H | H | H | H | H |
| CF₃ | SO₂Me | O | H | SMe | H | H | H | H | H | H |
| CF₃ | S0₂Me | O | H | SO₂Me | H | H | H | H | H | H |
| CF₃ | SO₂Me | O | H | Ph | H | H | H | H | H | H |
| CF₃ | SO₂Me | O | H | NMe₂ | H | H | H | H | H | H |
| CF₃ | SO₂Me | O | H | CN | H | H | H | H | H | H |
| CF₃ | SO₂Me | O | H | NO₂ | H | H | H | H | H | H |
| CF₃ | SO₂Me | O | H | H | F | H | H | H | H | H |
| CF₃ | SO₂Me | O | H | H | Cl | H | H | H | H | H |
| CF₃ | SO₂Me | O | H | H | Br | H | H | H | H | H |
| CF₃ | S0₂Me | O | H | H | I | H | H | H | H | H |
| CF₃ | SO₂Me | O | H | H | Me | H | H | H | H | H |
| CF₃ | SO₂Me | O | H | H | c-Pr | H | H | H | H | H |
| CF₃ | SO₂Me | O | H | H | OMe | H | H | H | H | H |
| CF₃ | SO₂Me | O | H | H | OCF₃ | H | H | H | H | H |
| CF₃ | SO₂Me | O | H | H | OCH₂C≡CH | H | H | H | H | H |
| CF₃ | SO₂Me | O | H | H | Bn | H | H | H | H | H |
| CF₃ | SO₂Me | O | H | H | OAc | H | H | H | H | H |
| CF₃ | SO₂Me | O | H | H | OSO₂Me | H | H | H | H | H |
| CF₃ | SO₂Me | O | H | H | SMe | H | H | H | H | H |
| CF₃ | SO₂Me | O | H | H | SO₂Me | H | H | H | H | H |
| CF₃ | SO₂Me | O | H | H | Ph | H | H | H | H | H |
| CF₃ | SO₂Me | O | H | H | NMe₂ | H | H | H | H | H |
| CF₃ | SO₂Me | O | H | H | CN | H | H | H | H | H |
| CF₃ | SO₂Me | O | H | H | NO₂ | H | H | H | H | H |
| CF₃ | SO₂Me | O | H | H | H | F | H | H | H | H |
| CF₃ | SO₂Me | O | H | H | H | Cl | H | H | H | H |
| CF₃ | SO₂Me | O | H | H | H | Br | H | H | H | H |
| CF₃ | SO₂Me | O | H | H | H | I | H | H | H | H |
| CF₃ | SO₂Me | O | H | H | H | Me | H | H | H | H |

**[Table 57]**

| R¹ | R² | W | X¹ | X² | X³ | X⁴ | Y¹ | Y² | Y³ | Y⁴ |
|---|---|---|---|---|---|---|---|---|---|---|
| CF₃ | SO₂Me | O | H | H | H | c-Pr | H | H | H | H |
| CF₃ | SO₂Me | O | H | H | H | OMe | H | H | H | H |
| CF₃ | SO₂Me | O | H | H | H | OCF₃ | H | H | H | H |
| CF₃ | SO₂Me | O | H | H | H | OCH₂C≡CH | H | H | H | H |
| CF₃ | SO₂Me | O | H | H | H | Bn | H | H | H | H |
| CF₃ | SO₂Me | O | H | H | H | OAc | H | H | H | H |
| CF₃ | SO₂Me | O | H | H | H | OSO₂Me | H | H | H | H |
| CF₃ | SO₂Me | O | H | H | H | SMe | H | H | H | H |
| CF₃ | SO₂Me | O | H | H | H | SO₂Me | H | H | H | H |
| CF₃ | SO₂Me | O | H | H | H | Ph | H | H | H | H |
| CF₃ | SO₂Me | O | H | H | H | NMe₂ | H | H | H | H |
| CF₃ | SO₂Me | O | H | H | H | CN | H | H | H | H |
| CF₃ | SO₂Me | O | H | H | H | NO₂ | H | H | H | H |
| CF₃ | SO₂Me | O | F | F | H | H | H | H | H | H |
| CF₃ | SO₂Me | O | F | H | F | H | H | H | H | H |
| CF₃ | SO₂Me | O | F | Cl | H | H | H | H | H | H |
| CF₃ | SO₂Me | O | F | H | CI | H | H | H | H | H |
| CF₃ | SO₂Me | O | F | H | H | Cl | H | H | H | H |
| CF₃ | SO₂Me | O | F | H | H | Br | H | H | H | H |
| CF₃ | SO₂Me | O | F | H | H | I | H | H | H | H |
| CF₃ | SO₂Me | O | F | H | H | Me | H | H | H | H |
| CF₃ | SO₂Me | O | F | H | H | c-Pr | H | H | H | H |
| CF₃ | SO₂Me | O | F | H | H | OMe | H | H | H | H |
| CF₃ | SO₂Me | O | F | H | H | OCF₃ | H | H | H | H |
| CF₃ | SO₂Me | O | F | H | H | OCH₂C≡CH | H | H | H | H |
| CF₃ | SO₂Me | O | F | H | H | OSO₂Me | H | H | H | H |
| CF₃ | SO₂Me | O | F | H | H | SMe | H | H | H | H |
| CF₃ | SO₂Me | O | F | H | H | SO₂Me | H | H | H | H |
| CF₃ | SO₂Me | O | F | H | H | NMe₂ | H | H | H | H |
| CF₃ | SO₂Me | O | F | H | H | CN | H | H | H | H |

**[Table 58]**

| R¹ | R² | W | X¹ | X² | X³ | X⁴ | Y¹ | Y² | Y³ | Y⁴ |
|---|---|---|---|---|---|---|---|---|---|---|
| CF₃ | SO₂Me | O | F | H | H | NO₂ | H | H | H | H |
| CF₃ | SO₂Me | O | F | F | F | H | H | H | H | H |
| CF₃ | SO₂Me | O | F | F | H | F | H | H | H | H |
| CF₃ | SO₂Me | O | F | H | F | F | H | H | H | H |
| CF₃ | SO₂Me | O | F | F | F | F | H | H | H | H |
| CF₃ | SO₂Me | O | Cl | F | H | H | H | H | H | H |
| CF₃ | SO₂Me | O | Cl | H | F | H | H | H | H | H |
| CF₃ | SO₂Me | O | Cl | H | H | F | H | H | H | H |
| CF₃ | SO₂Me | O | Cl | Cl | H | H | H | H | H | H |
| CF₃ | SO₂Me | O | Cl | H | Cl | H | H | H | H | H |
| CF₃ | SO₂Me | O | Cl | H | H | C l | H | H | H | H |
| CF₃ | SO₂Me | O | Cl | H | H | Br | H | H | H | H |
| CF₃ | SO₂Me | O | Cl | H | H | I | H | H | H | H |
| CF₃ | SO₂Me | O | Cl | H | H | Me | H | H | H | H |
| CF₃ | SO₂Me | O | Cl | H | H | c-Pr | H | H | H | H |
| CF₃ | SO₂Me | O | Cl | H | H | OMe | H | H | H | H |
| CF₃ | SO₂Me | O | Cl | H | H | OCF₃ | H | H | H | H |
| CF₃ | SO₂Me | O | Cl | H | H | OCH₂C≡CH | H | H | H | H |
| CF₃ | SO₂Me | O | Cl | H | H | OSO₂Me | H | H | H | H |
| CF₃ | SO₂Me | O | Cl | H | H | SMe | H | H | H | H |
| CF₃ | SO₂Me | O | Cl | H | H | SO₂Me | H | H | H | H |
| CF₃ | SO₂Me | O | Cl | H | H | NMe₂ | H | H | H | H |
| CF₃ | SO₂Me | O | Cl | H | H | CN | H | H | H | H |
| CF₃ | SO₂Me | O | Cl | H | H | NO₂ | H | H | H | H |
| CF₃ | SO₂Me | O | Br | H | H | F | H | H | H | H |
| CF₃ | SO₂Me | O | Br | H | H | Cl | H | H | H | H |
| CF₃ | SO₂Me | O | Br | H | H | Me | H | H | H | H |
| CF₃ | SO₂Me | O | Br | H | H | OMe | H | H | H | H |
| CF₃ | SO₂Me | O | Me | H | H | F | H | H | H | H |
| CF₃ | SO₂Me | O | Me | H | H | Cl | H | H | H | H |

**[Table 59]**

| R¹ | R² | W | X¹ | X² | X³ | X⁴ | Y¹ | Y² | Y³ | Y⁴ |
|---|---|---|---|---|---|---|---|---|---|---|
| CF₃ | SO₂Me | O | Me | H | H | Me | H | H | H | H |
| CF₃ | SO₂Me | O | Me | H | H | OMe | H | H | H | H |
| CF₃ | SO₂Me | O | OMe | H | H | F | H | H | H | H |
| CF₃ | SO₂Me | O | OMe | H | H | Cl | H | H | H | H |
| CF₃ | SO₂Me | O | OMe | H | H | Me | H | H | H | H |
| CF₃ | SO₂Me | O | OMe | H | H | OMe | H | H | H | H |
| CF₃ | SO₂Me | O | H | F | F | H | H | H | H | H |
| CF₃ | SO₂Me | O | H | F | Cl | H | H | H | H | H |
| CF₃ | SO₂Me | O | H | F | H | F | H | H | H | H |
| CF₃ | SO₂Me | O | H | F | H | Cl | H | H | H | H |
| CF₃ | SO₂Me | O | H | Cl | Cl | H | H | H | H | H |
| CF₃ | SO₂Me | O | H | Cl | F | H | H | H | H | H |
| CF₃ | SO₂Me | O | H | Cl | H | F | H | H | H | H |
| CF₃ | SO₂Me | O | H | Cl | H | Cl | H | H | H | H |
| CF₃ | SO₂Me | O | H | H | F | F | H | H | H | H |
| CF₃ | SO₂Me | O | H | H | F | Cl | H | H | H | H |
| CF₃ | SO₂Me | O | H | H | Cl | Cl | H | H | H | H |
| CF₃ | SO₂Me | O | F | F | F | F | H | H | H | H |
| CF₃ | SO₂Me | O | Cl | Cl | Cl | Cl | H | H | H | H |
| CF₃ | SO₂Me | O | -CH=CH-CH=CH- | | H | H | H | H | H | H |
| CF₃ | SO₂Me | O | -CH=N-CH=CH- | | H | H | H | H | H | H |
| CF₃ | SO₂Me | O | -S-CH=N- | | H | H | H | H | H | H |
| CF₃ | SO₂Me | O | -N=CH-S- | | H | H | H | H | H | H |
| CF₃ | SO₂Me | O | H | -CH=CH-CH=CH- | | H | H | H | H | H |
| CF₃ | SO₂Me | O | H | -CH=N-CH=CH- | | H | H | H | H | H |
| CF₃ | SO₂Me | O | H | -S-CH=N- | | H | H | H | H | H |
| CF₃ | SO₂Me | O | H | -N=CH-S- | | H | H | H | H | H |
| CF₃ | SO₂Me | O | H | H | -CH=CH-CH=CH- | | H | H | H | H |
| CF₃ | SO₂Me | O | H | H | -CH=N-CH=CH- | | H | H | H | H |
| CF₃ | SO₂Me | O | H | H | -S-CH=N- | | H | H | H | H |

**[Table 60]**

| R¹ | R² | W | X¹ | X² | X³ | X⁴ | Y¹ | Y² | Y³ | Y⁴ |
|---|---|---|---|---|---|---|---|---|---|---|
| CF₃ | SO₂Me | O | H | H | -N=CH-S- | | H | H | H | H |
| CF₃ | H | O | H | H | H | H | F | H | H | H |
| CF₃ | H | O | H | H | H | H | H | F | H | H |
| CF₃ | H | O | H | H | H | H | H | H | F | H |
| CF₃ | H | O | H | H | H | H | H | H | H | F |
| CF₃ | H | O | H | H | H | H | H | F | F | H |
| CF₃ | H | O | H | H | H | H | Cl | H | H | H |
| CF₃ | H | O | H | H | H | H | H | Cl | H | H |
| CF₃ | H | O | H | H | H | H | H | H | Cl | H |
| CF₃ | H | O | H | H | H | H | H | H | H | Cl |
| CF₃ | H | O | H | H | H | H | H | Cl | Cl | H |
| CF₃ | H | O | H | H | H | H | H | H | Br | H |
| CF₃ | H | O | H | H | H | H | H | H | Me | H |
| CF₃ | H | O | H | H | H | H | H | H | OMe | H |
| CF₃ | H | O | H | H | H | H | H | H | CF₃ | H |
| CF₃ | H | O | F | H | H | H | F | H | H | H |
| CF₃ | H | O | F | H | H | H | H | F | H | H |
| CF₃ | H | O | F | H | H | H | H | H | F | H |
| CF₃ | H | O | F | H | H | H | H | H | H | F |
| CF₃ | H | O | F | H | H | H | H | F | F | H |
| CF₃ | H | O | F | H | H | H | Cl | H | H | H |
| CF₃ | H | O | F | H | H | H | H | Cl | H | H |
| CF₃ | H | O | F | H | H | H | H | H | Cl | H |
| CF₃ | H | O | F | H | H | H | H | H | H | Cl |
| CF₃ | H | O | F | H | H | H | H | Cl | CI | H |
| CF₃ | H | O | F | H | H | H | H | H | Br | H |
| CF₃ | H | O | F | H | H | H | H | H | Me | H |
| CF₃ | H | O | F | H | H | H | H | H | OMe | H |
| CF₃ | H | O | F | H | H | H | H | H | CF₃ | H |
| CF₃ | H | O | F | H | H | F | F | H | H | H |

**[Table 61]**

| R¹ | R² | W | X¹ | X² | X³ | X⁴ | Y¹ | Y² | Y³ | Y⁴ |
|---|---|---|---|---|---|---|---|---|---|---|
| CF₃ | H | O | F | H | H | F | H | F | H | H |
| CF₃ | H | O | F | H | H | F | H | H | F | H |
| CF₃ | H | O | F | H | H | F | H | H | H | F |
| CF₃ | H | O | F | H | H | F | H | F | F | H |
| CF₃ | H | O | F | H | H | F | Cl | H | H | H |
| CF₃ | H | O | F | H | H | F | H | Cl | H | H |
| CF₃ | H | O | F | H | H | F | H | H | Cl | H |
| CF₃ | H | O | F | H | H | F | H | H | H | Cl |
| CF₃ | H | O | F | H | H | F | H | Cl | Cl | H |
| CF₃ | H | O | F | H | H | F | H | H | Br | H |
| CF₃ | H | O | F | H | H | F | H | H | Me | H |
| CF₃ | H | O | F | H | H | F | H | H | OMe | H |
| CF₃ | H | O | F | H | H | F | H | H | CF₃ | H |
| CF₃ | H | S | H | H | H | H | H | H | H | H |
| CF₃ | COc-Pr | S | H | H | H | H | H | H | H | H |
| CF₃ | CO₂Et | S | H | H | H | H | H | H | H | H |
| CF₃ | CO₂CH₂CH₂F | S | H | H | H | H | H | H | H | H |
| CF₃ | CO₂CH₂CHF₂ | S | H | H | H | H | H | H | H | H |
| CF₃ | CO₂(3-oxetanyl) | S | H | H | H | H | H | H | H | H |
| CF₃ | CO₂(3-tetrahydrofuryl) | S | H | H | H | H | H | H | H | H |
| CF₃ | SO₂Me | S | H | H | H | H | H | H | H | H |
| CF₃ | H | S | F | H | H | H | H | H | H | H |
| CF₃ | COc-Pr | S | F | H | H | H | H | H | H | H |
| CF₃ | CO₂Et | S | F | H | H | H | H | H | H | H |
| CF₃ | CO₂CH₂CH₂F | S | F | H | H | H | H | H | H | H |
| CF₃ | CO₂CH₂CHF₂ | S | F | H | H | H | H | H | H | H |
| CF₃ | CO₂(3-oxetanyl) | S | F | H | H | H | H | H | H | H |
| CF₃ | CO₂(3-tetrahydrofuryl) | S | F | H | H | H | H | H | H | H |
| CF₃ | SO₂Me | S | F | H | H | H | H | H | H | H |
| CF₃ | H | S | F | H | H | F | H | H | H | H |

**[Table 62]**

| R¹ | R² | W | X¹ | X² | X³ | X⁴ | Y¹ | Y² | Y³ | Y⁴ |
|---|---|---|---|---|---|---|---|---|---|---|
| CF₃ | COc-Pr | S | F | H | H | F | H | H | H | H |
| CF₃ | CO₂Et | S | F | H | H | F | H | H | H | H |
| CF₃ | CO₂CH₂CH₂F | S | F | H | H | F | H | H | H | H |
| CF₃ | CO₂CH₂CHF₂ | S | F | H | H | F | H | H | H | H |
| CF₃ | CO₂(3-oxetanyl) | S | F | H | H | F | H | H | H | H |
| CF₃ | CO₂(3-tetrahydrofuryl) | S | F | H | H | F | H | H | H | H |
| CF₃ | SO₂Me | S | F | H | H | F | H | H | H | H |

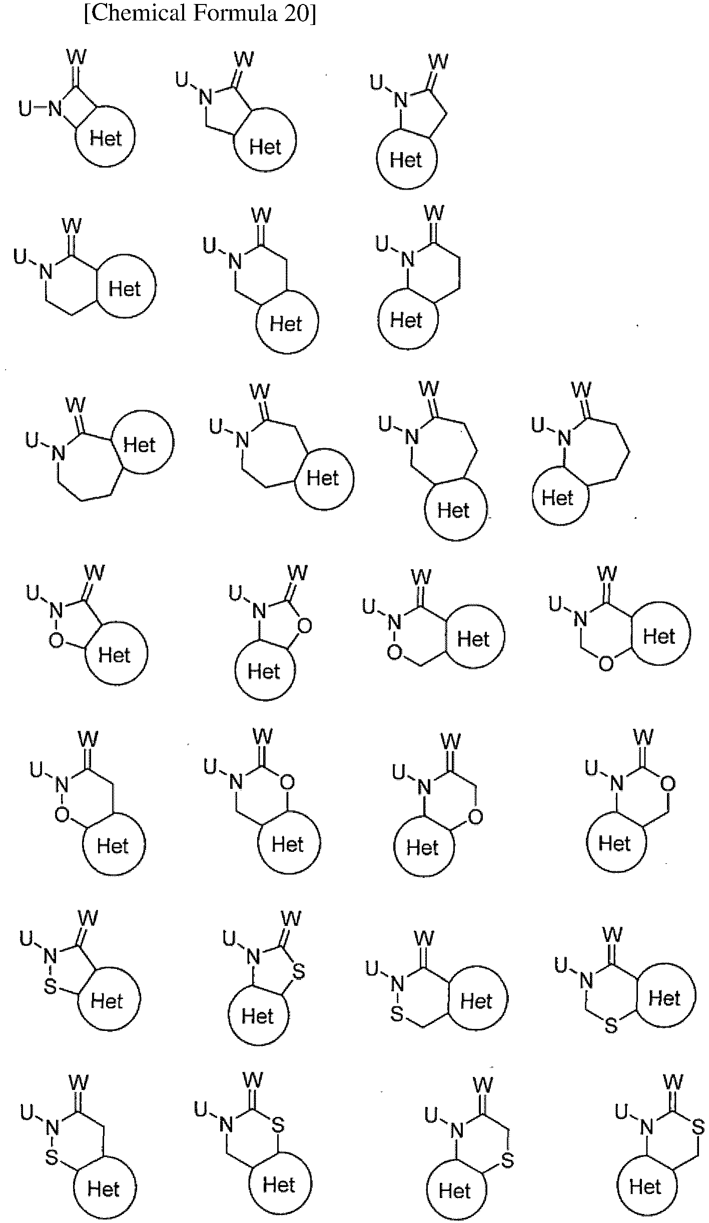

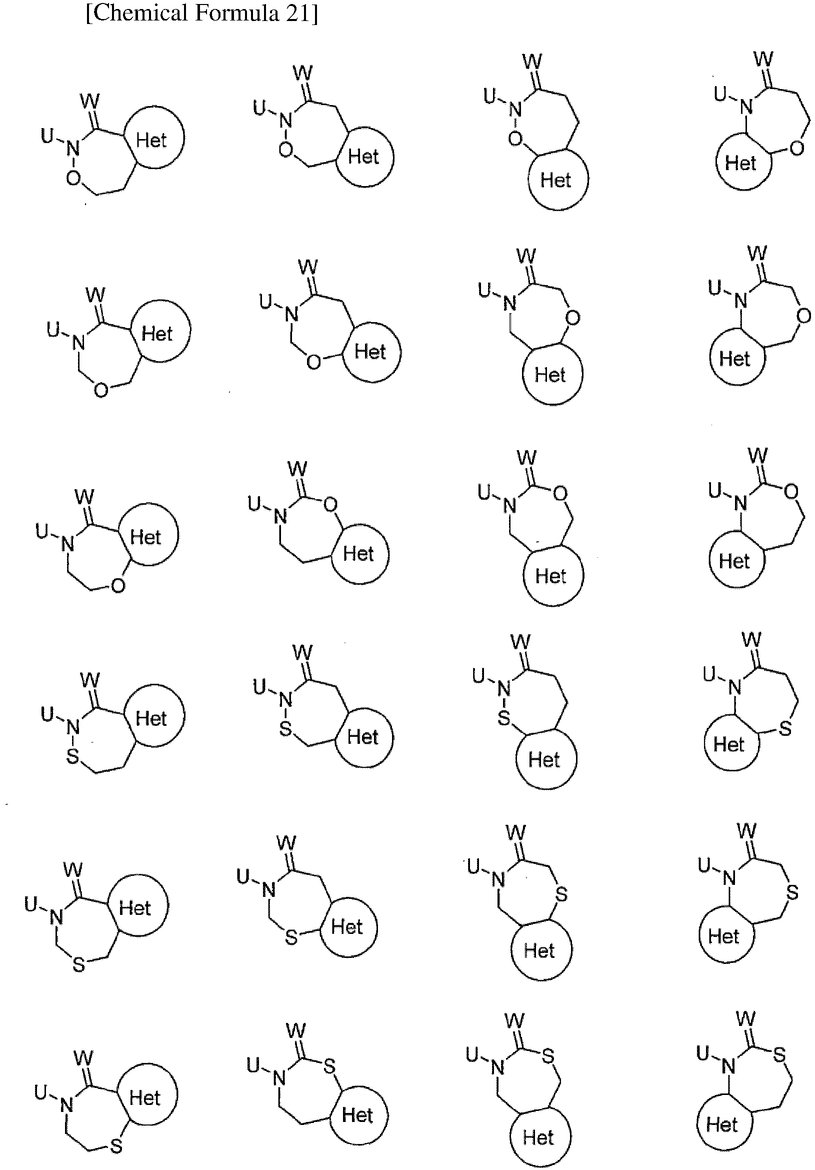

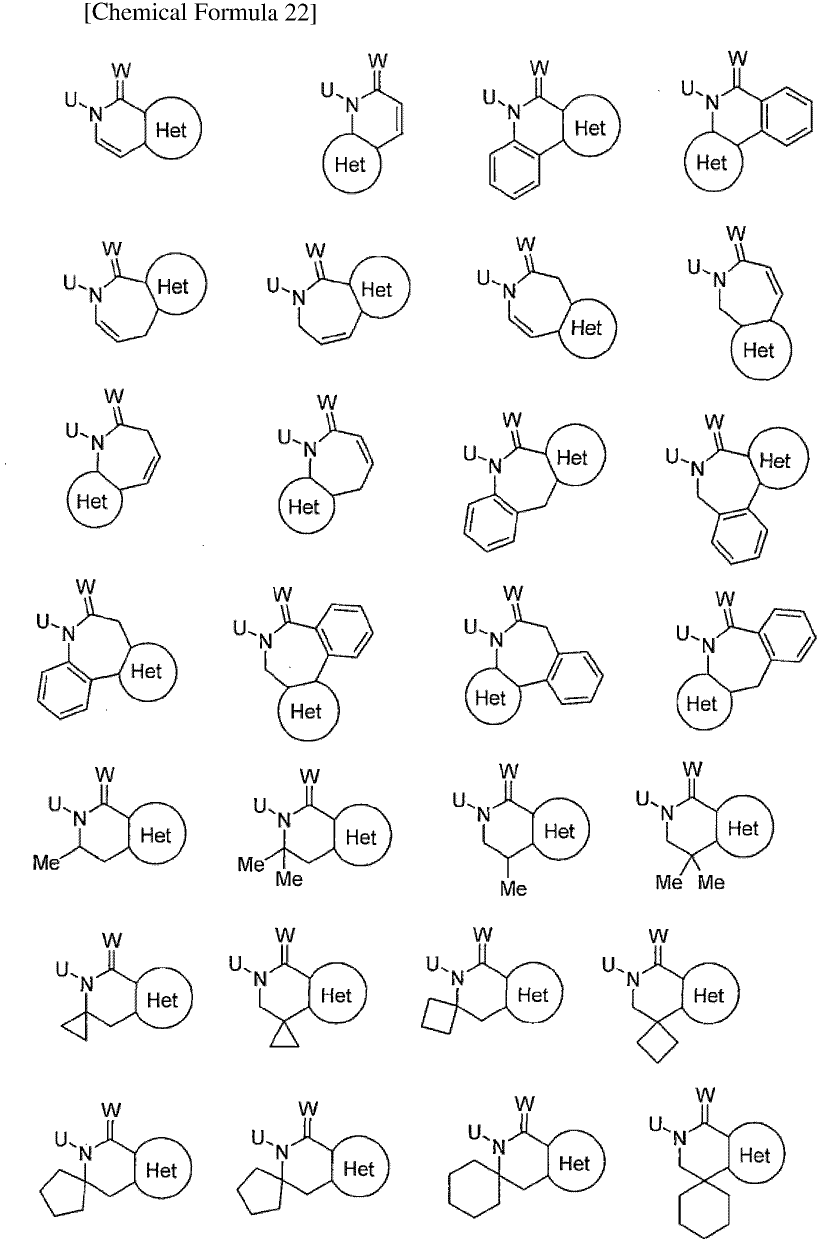

U is the same as general formula (22) above.

Het-1 to Het-58 in the tables are represented by the following structures, respectively.

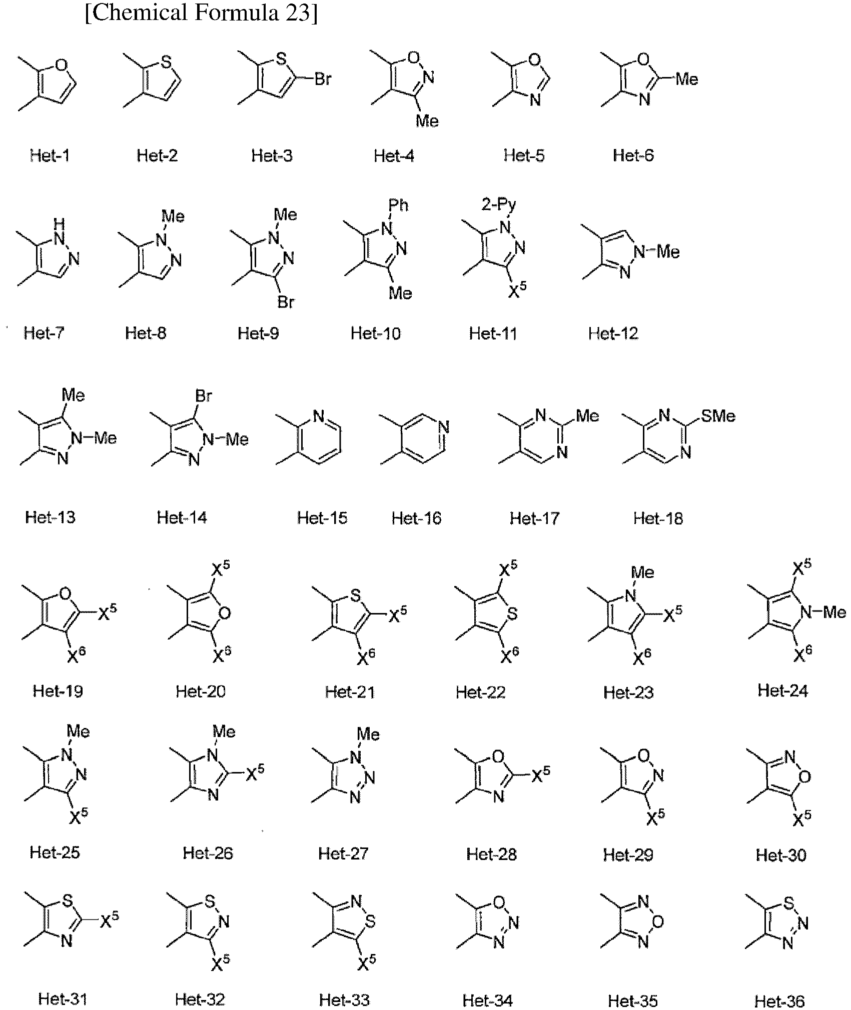

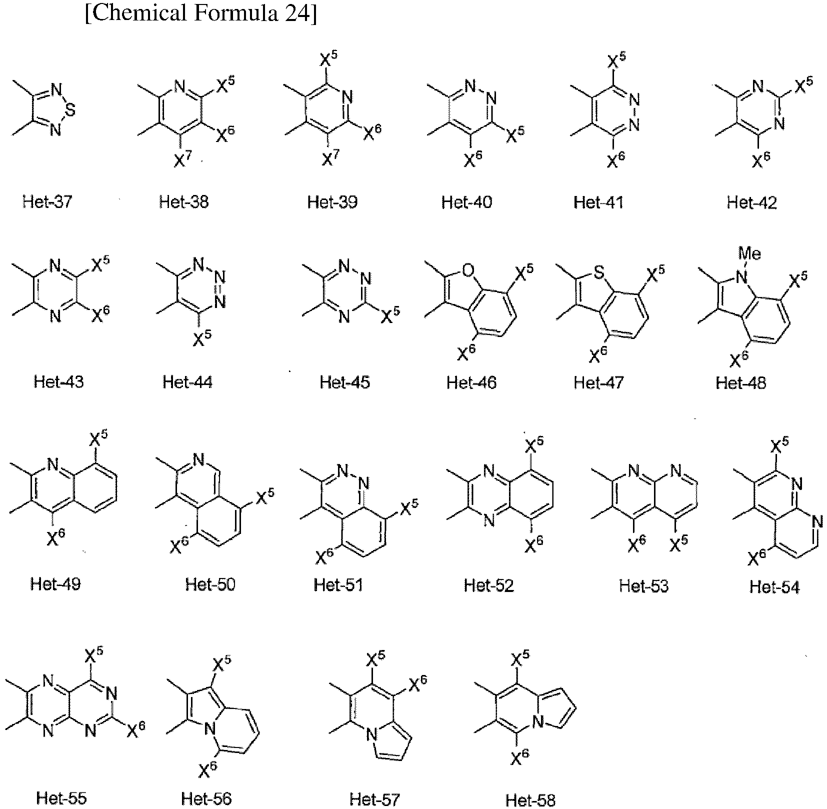

**[Table 63]**

| R¹ | R² | W | Het-No. | Y¹ | Y² | Y³ | Y⁴ |
|---|---|---|---|---|---|---|---|
| CHF₂ | COMe | O | Het-1 | H | H | H | H |
| CHF₂ | COMe | O | Het-2 | H | H | H | H |
| CHF₂ | COc-Pr | O | Het-1 | H | H | H | H |
| CHF₂ | COc-Pr | O | Het-2 | H | H | H | H |
| CHF₂ | CO₂Me | O | Het-1 | H | H | H | H |
| CHF₂ | CO₂Me | O | Het-2 | H | H | H | H |
| CHF₂ | CO₂Et | O | Het-1 | H | H | H | H |
| CHF₂ | CO₂Et | O | Net-2 | H | H | H | H |
| CHF₂ | CO₂CH₂CH₂F | O | Het-1 | H | H | H | H |
| CHF₂ | CO₂CH₂CH₂F | O | Het-2 | H | H | H | H |
| CHF₂ | CO₂CH₂CHF₂ | O | Het-1 | H | H | H | H |
| CHF₂ | CO₂CH₂CHF₂ | O | Het-2 | H | H | H | H |
| CHF₂ | CO₂ (3-oxetanyl) | O | Het-1 | H | H | H | H |
| CHF₂ | CO₂ (3-oxetanyl) | O | Het-2 | H | H | H | H |
| CHF₂ | CO₂(3-tetrahydrofuryl) | O | Het-1 | H | H | H | H |
| CHF₂ | CO₂ (3-tetrahydrofuryl) | O | Het-2 | H | H | H | H |
| CHF₂ | CO (SMe) | O | Het-1 | H | H | H | H |
| CHF₂ | CO (SMe) | O | Het-2 | H | H | H | H |
| CHF₂ | SO₂Me | O | Het-1 | H | H | H | H |
| CHF₂ | SO₂Me | O | Het-2 | H | H | H | H |
| CHF₂ | SO₂CF₃ | O | Het-1 | H | H | H | H |
| CHF₂ | SO₂CF₃ | O | Het-2 | H | H | H | H |
| CClF₂ | H | O | Het-1 | H | H | H | H |
| CClF₂ | H | O | Het-2 | H | H | H | H |
| CBrF₂ | H | O | Het-1 | H | H | H | H |
| CBrF₂ | H | O | Het-2 | H | H | H | H |
| CCl₂F | H | O | Het-1 | H | H | H | H |
| CCl₂F | H | O | Het-2 | H | H | H | H |
| CCl₃ | H | O | Het-1 | H | H | H | H |
| CCl₃ | H | O | Het-2 | H | H | H | H |

**[Table 64]**

| R¹ | R² | W | Het-No. | Y¹ | Y² | Y³ | Y⁴ |
|---|---|---|---|---|---|---|---|
| CF₃ | H | O | Het-1 | H | H | H | H |
| CF₃ | Me | O | Het-1 | H | H | H | H |
| CF₃ | Et | O | Het-1 | H | H | H | H |
| CF₃ | CH₂CF₃ | O | Het-1 | H | H | H | H |
| CF₃ | CH₂CH=CH₂ | O | Het-1 | H | H | H | H |
| CF₃ | CH₂C≡CH | O | Het-1 | H | H | H | H |
| CF₃ | CH₂OMe | O | Het-1 | H | H | H | H |
| CF₃ | CH₂OCH₂CF₃ | O | Het-1 | H | H | H | H |
| CF₃ | CH₂OCH₂CH₂OMe | O | Het-1 | H | H | H | H |
| CF₃ | CH₂SMe | O | Het-1 | H | H | H | H |
| CF₃ | CH₂COMe | O | Het-1 | H | H | H | H |
| CF₃ | CH₂Ph | O | Het-1 | H | H | H | H |
| CF₃ | CH₂ (4-ClPh) | O | Het-1 | H | H | H | H |
| CF₃ | CH₂ (4-MePh) | O | Het-1 | H | H | H | H |
| CF₃ | CH₂ (4-MeOPh) | O | Het-1 | H | H | H | H |
| CF₃ | CH₂CH₂OPh | O | Het-1 | H | H | H | H |
| CF₃ | COMe | O | Het-1 | H | H | H | H |
| CF₃ | COEt | O | Het-1 | H | H | H | H |
| CF₃ | COn-Pr | O | Het-1 | H | H | H | H |
| CF₃ | COi-Pr | O | Het-1 | H | H | H | H |
| CF₃ | COn-Bu | O | Het-1 | H | H | H | H |
| CF₃ | COs-Bu | O | Het-1 | H | H | H | H |
| CF₃ | COi-Bu | O | Het-1 | H | H | H | H |
| CF₃ | COt-Bu | O | Het-1 | H | H | H | H |
| CF₃ | COn-Pen | O | Het-1 | H | H | H | H |
| CF₃ | COn-Hex | O | Het-1 | H | H | H | H |
| CF₃ | COCF₃ | O | Het-1 | H | H | H | H |
| CF₃ | COCH₂CF₃ | O | Het-1 | H | H | H | H |
| CF₃ | COCH=CH₂ | O | Het-1 | H | H | H | H |
| CF₃ | COC≡CH | O | Het-1 | H | H | H | H |

**[Table 65]**

| R¹ | R² | W | Het-No. | Y¹ | Y² | Y³ | Y⁴ |
|---|---|---|---|---|---|---|---|
| CF₃ | COc-Pr | O | Het-1 | H | H | H | H |
| CF₃ | COc-Bu | O | Het-1 | H | H | H | H |
| CF₃ | COc-Pen | O | Het-1 | H | H | H | H |
| CF₃ | COc-Hex | O | Het-1 | H | H | H | H |
| CF₃ | COCH₂c-Pr | O | Het-1 | H | H | H | H |
| CF₃ | COCH₂OMe | O | Het-1 | H | H | H | H |
| CF₃ | COCH₂OCH₂CF₃ | O | Het-1 | H | H | H | H |
| CF₃ | COCH₂OCH₂CH₂CF₃ | O | Het-1 | H | H | H | H |
| CF₃ | COCH₂SMe | O | Het-1 | H | H | H | H |
| CF₃ | COCH₂SCH₂CF₃ | O | Het-1 | H | H | H | H |
| CF₃ | Bz | O | Het-1 | H | H | H | H |
| CF₃ | 4-ClBz | O | Het-1 | H | H | H | H |
| CF₃ | 4-MeBz | O | Het-1 | H | H | H | H |
| CF₃ | COCH₂Ph | O | Het-1 | H | H | H | H |
| CF₃ | COCH₂ (4-ClPh) | O | Het-1 | H | H | H | H |
| CF₃ | COCH₂ (4-MePh) | O | Het-1 | H | H | H | H |
| CF₃ | CO (2-tetrahydrofuryl) | O | Het-1 | H | H | H | H |
| CF₃ | CO (3-tetrahydrofuryl) | O | Het-1 | H | H | H | H |
| CF₃ | CO (2-pyridyl) | O | Het-1 | H | H | H | H |
| CF₃ | CO (3-pyridyl) | O | Het-1 | H | H | H | H |
| CF₃ | CO (4-pyridyl) | O | Het-1 | H | H | H | H |
| CF₃ | CO (2-thienyl) | O | Het-1 | H | H | H | H |
| CF₃ | CO (3-thienyl) | O | Het-1 | H | H | H | H |
| CF₃ | CO (2-tetrahydrofurfuryl) | O | Het-1 | H | H | H | H |
| CF₃ | COCH₂ (2-pyridyl) | O | Het-1 | H | H | H | H |
| CF₃ | COCH₂ (2-thienyl) | O | Het-1 | H | H | H | H |
| CF₃ | CO₂Me | O | Het-1 | H | H | H | H |
| CF₃ | CO₂Et | O | Het-1 | H | H | H | H |
| CF₃ | CO₂n-Pr | O | Het-1 | H | H | H | H |
| CF₃ | CO₂i-Pr | O | Het-1 | H | H | H | H |

**[Table 66]**

| R¹ | R² | W | Het-No. | Y¹ | Y² | Y³ | Y⁴ |
|---|---|---|---|---|---|---|---|
| CF₃ | CO₂n-Bu | O | Het-1 | H | H | H | H |
| CF₃ | CO₂s-Bu | O | Het-1 | H | H | H | H |
| CF₃ | CO₂i-Bu | O | Het-1 | H | H | H | H |
| CF₃ | CO₂t-Bu | O | Het-1 | H | H | H | H |
| CF₃ | CO₂n-Pen | O | Het-1 | H | H | H | H |
| CF₃ | CO₂n-Hex | O | Het-1 | H | H | H | H |
| CF₃ | CO₂CH₂CH₂F | O | Het-1 | H | H | H | H |
| CF₃ | CO₂CH₂CHF₂ | O | Het-1 | H | H | H | H |
| CF₃ | CO₂CH₂CF₃ | O | Het-1 | H | H | H | H |
| CF₃ | CO₂CH=CH₂ | O | Het-1 | H | H | H | H |
| CF₃ | CO₂C ≡ CH | O | Het-1 | H | H | H | H |
| CF₃ | CO₂c-Pr | O | Het-1 | H | H | H | H |
| CF₃ | CO₂CH₂c-Pr | O | Het-1 | H | H | H | H |
| CF₃ | CO₂CH₂CH₂OMe | O | Het-1 | H | H | H | H |
| CF₃ | CO₂CH₂CH₂OCH₂CF₃ | O | Het-1 | H | H | H | H |
| CF₃ | CO₂CH₂CH₂OCH₂CH₂OMe | O | Het-1 | H | H | H | H |
| CF₃ | CO₂CH₂CH₂SMe | O | Het-1 | H | H | H | H |
| CF₃ | CO₂CH₂CH₂SCH₂CF₃ | O | Het-1 | H | H | H | H |
| GF₃ | CO₂Ph | O | Het-1 | H | H | H | H |
| CF₃ | CO₂ (4-ClPh) | O | Het-1 | H | H | H | H |
| CF₃ | CO₂ (4-MePh) | O | Het-1 | H | H | H | H |
| CF₃ | CO₂CH₂Ph | O | Het-1 | H | H | H | H |
| CF₃ | CO₂CH₂ (4-ClPh) | O | Het-1 | H | H | H | H |
| CF₃ | CO₂CH₂ (4-MePh) | O | Het-1 | H | H | H | H |
| CF₃ | CO₂ (3-oxetanyl) | O | Het-1 | H | H | H | H |
| CF₃ | CO₂ (2-tetrahydrofuryl) | O | Het-1 | H | H | H | H |
| CF₃ | CO₂ (3-tetrahydrofuryl) | O | Het-1 | H | H | H | H |
| CF₃ | CO₂ (2-pyridyl) | O | Het-1 | H | H | H | H |
| CF₃ | CO₂ (2-thienyl) | O | Het-1 | H | H | H | H |
| CF₃ | CO₂ (2-tetrahydrofurfuryl) | O | Het-1 | H | H | H | H |

**[Table 67]**

| R¹ | R² | W | Het-No. | Y¹ | Y² | Y³ | Y⁴ |
|---|---|---|---|---|---|---|---|
| CF₃ | CO₂CH₂ (2-pyridyl) | O | Het-1 | H | H | H | H |
| CF₃ | CO₂CH₂ (2-thienyl) | O | Het-1 | H | H | H | H |
| CF₃ | CO (SMe) | O | Het-1 | H | H | H | H |
| CF₃ | CO (SEt) | O | Het-1 | H | H | H | H |
| CF₃ | CO (SCF₃) | O | Het-1 | H | H | H | H |
| CF₃ | CO (SCH₂CF₃) | O | Het-1 | H | H | H | H |
| CF₃ | CONHEt | O | Het-1 | H | H | H | H |
| CF₃ | CONHCH₂CF₃ | O | Het-1 | H | H | H | H |
| CF₃ | CONEt₂ | O | Het-1 | H | H | H | H |
| CF₃ | SO₂Me | O | Het-1 | H | H | H | H |
| CF₃ | SO₂Et | O | Het-1 | H | H | H | H |
| CF₃ | SO₂n-Pr | O | Het-1 | H | H | H | H |
| CF₃ | SO₂i-Pr | O | Het-1 | H | H | H | H |
| CF₃ | SO₂n-Bu | O | Het-1 | H | H | H | H |
| CF₃ | SO₂i-Bu | O | Het-1 | H | H | H | H |
| CF₃ | SO₂CH₂Cl | O | Het-1 | H | H | H | H |
| CF₃ | SO₂CCl₃ | O | Het-1 | H | H | H | H |
| CF₃ | SO₂CHF₂ | O | Het-1 | H | H | H | H |
| CF₃ | SO₂CF₃ | O | Het-1 | H | H | H | H |
| CF₃ | SO₂CH₂CF₃ | O | Het-1 | H | H | H | H |
| CF₃ | SO₂CH=CH₂ | O | Het-1 | H | H | H | H |
| CF₃ | SO₂CH₂CH=CH₂ | O | Het-1 | H | H | H | H |
| CF₃ | SO₂CH₂C≡CH | O | Het-1 | H | H | H | H |
| CF₃ | SO₂c-Pr | O | Het-1 | H | H | H | H |
| CF₃ | SO₂c-Nex | O | Het-1 | H | H | H | H |
| CF₃ | SO₂CH₂c-Pr | O | Het-1 | H | H | H | H |
| CF₃ | SO₂CH₂CH₂OMe | O | Het-1 | H | H | H | H |
| CF₃ | SO₂Ph | O | Het-1 | H | H | H | H |
| CF₃ | SO₂ (4-ClPh) | O | Het-1 | H | H | H | H |
| CF₃ | SO₂ (4-MePh) | O | Het-1 | H | H | H | H |

**[Table 68]**

| R¹ | R² | W | Het-No. | Y¹ | Y² | Y³ | Y⁴ |
|---|---|---|---|---|---|---|---|
| CF₃ | SO₂CH₂Ph | O | Het-1 | H | H | H | H |
| CF₃ | SO₂CH₂ (4-ClPh) | O | Het-1 | H | H | H | H |
| CF₃ | SO₂CH₂ (4-MePh) | O | Het-1 | H | H | H | H |
| CF₃ | SO₂NHMe | O | Het-1 | H | H | H | H |
| CF₃ | SO₂NMe₂ | O | Het-1 | H | H | H | H |
| CF₃ | H | O | Het-2 | H | H | H | H |
| CF₃ | Me | O | Het-2 | H | H | H | H |
| CF₃ | Et | O | Het-2 | H | H | H | H |
| CF₃ | CH₂CF₃ | O | Het-2 | H | H | H | H |
| CF₃ | CH₂CH=CH₂ | O | Het-2 | H | H | H | H |
| CF₃ | CH₂C≡CH | O | Het-2 | H | H | H | H |
| CF₃ | CH₂OMe | O | Het-2 | H | H | H | H |
| CF₃ | CH₂OCH₂CF₃ | O | Het-2 | H | H | H | H |
| CF₃ | CH₂OCH₂CH₂OMe | O | Het-2 | H | H | H | H |
| CF₃ | CH₂SMe | O | Het-2 | H | H | H | H |
| CF₃ | CH₂COMe | O | Het-2 | H | H | H | H |
| CF₃ | CH₂Ph | O | Het-2 | H | H | H | H |
| CF₃ | CH₂ (4-ClPh) | O | Het-2 | H | H | H | H |
| CF₃ | CH₂ (4-MePh) | O | Het-2 | H | H | H | H |
| CF₃ | CH₂ (4-MeOPh) | O | Het-2 | H | H | H | H |
| CF₃ | CH₂CH₂OPh | O | Het-2 | H | H | H | H |
| CF₃ | COMe | O | Het-2 | H | H | H | H |
| CF₃ | COEt | O | Het-2 | H | H | H | H |
| CF₃ | COn-Pr | O | Het-2 | H | H | H | H |
| CF₃ | COi-Pr | O | Het-2 | H | H | H | H |
| CF₃ | COn-Bu | O | Het-2 | H | H | H | H |
| CF₃ | COs-Bu | O | Het-2 | H | H | H | H |
| CF₃ | COi-Bu | O | Het-2 | H | H | H | H |
| CF₃ | COt-Bu | O | Het-2 | H | H | H | H |
| CF₃ | COn-Pen | O | Het-2 | H | H | H | H |

**[Table 69]**

| R¹ | R² | W | Het-No. | Y¹ | Y² | Y³ | Y⁴ |
|---|---|---|---|---|---|---|---|
| CF₃ | COn-Hex | O | Het-2 | H | H | H | H |
| CF₃ | COCF₃ | O | Het-2 | H | H | H | H |
| CF₃ | COCH₂CF₃ | O | Het-2 | H | H | H | H |
| CF₃ | COCH=CH₂ | O | Het-2 | H | H | H | H |
| CF₃ | COC≡CH | O | Het-2 | H | H | H | H |
| CF₃ | COc-Pr | O | Het-2 | H | H | H | H |
| CF₃ | COc-Bu | O | Het-2 | H | H | H | H |
| CF₃ | COc-Pen | O | Het-2 | H | H | H | H |
| CF₃ | COc-Hex | O | Het-2 | H | H | H | H |
| CF₃ | COCH₂c-Pr | O | Het-2 | H | H | H | H |
| CF₃ | COCH₂OMe | O | Het-2 | H | H | H | H |
| CF₃ | COCH₂OCH₂CF₃ | O | Het-2 | H | H | H | H |
| CF₃ | COCH₂OCH₂CH₂CF₃ | O | Het-2 | H | H | H | H |
| CF₃ | COCH₂SMe | O | Het-2 | H | H | H | H |
| CF₃ | COCH₂SCH₂CF₃ | O | Het-2 | H | H | H | H |
| CF₃ | Bz | O | Het-2 | H | H | H | H |
| CF₃ | 4-ClBz | O | Het-2 | H | H | H | H |
| CF₃ | 4-MeBz | O | Het-2 | H | H | H | H |
| CF₃ | COCH₂Ph | O | Het-2 | H | H | H | H |
| CF₃ | COCH₂(4-ClPh) | O | Het-2 | H | H | H | H |
| CF₃ | COCH₂(4-MePh) | O | Het-2 | H | H | H | H |
| CF₃ | CO (2-tetrahydrofuryl) | O | Het-2 | H | H | H | H |
| CF₃ | CO (3-tetrahydrofuryl) | O | Het-2 | H | H | H | H |
| CF₃ | CO (2-pyridyl) | O | Het-2 | H | H | H | H |
| CF₃ | CO (3-pyridyl) | O | Het-2 | H | H | H | H |
| CF₃ | CO (4-pyridyl) | O | Het-2 | H | H | H | H |
| CF₃ | CO (2-thienyl) | O | Het-2 | H | H | H | H |
| CF₃ | CO (3-thienyl) | O | Het-2 | H | H | H | H |
| CF₃ | CO (2-tetrahydrofurfuryl) | O | Het-2 | H | H | H | H |
| CF₃ | COCH₂(2-pyridyl) | O | Het-2 | H | H | H | H |

**[Table 70]**

| R¹ | R² | W | Het-No. | Y¹ | Y² | Y³ | Y⁴ |
|---|---|---|---|---|---|---|---|
| CF₃ | COCH₂(2-thienyl) | O | Het-2 | H | H | H | H |
| CF₃ | CO₂Me | O | Het-2 | H | H | H | H |
| CF₃ | CO₂Et | O | Het-2 | H | H | H | H |
| CF₃ | CO₂n-Pr | O | Het-2 | H | H | H | H |
| CF₃ | CO₂i-Pr | O | Het-2 | H | H | H | H |
| CF₃ | CO₂n-Bu | O | Het-2 | H | H | H | H |
| CF₃ | CO₂s-Bu | O | Het-2 | H | H | H | H |
| CF₃ | CO₂i-Bu | O | Het-2 | H | H | H | H |
| CF₃ | CO₂t-Bu | O | Het-2 | H | H | H | H |
| CF₃ | CO₂n-Pen | O | Het-2 | H | H | H | H |
| CF₃ | CO₂n-Hex | O | Het-2 | H | H | H | H |
| CF₃ | CO₂CH₂CH₂F | O | Het-2 | H | H | H | H |
| CF₃ | CO₂CH₂CHF₂ | O | Het-2 | H | H | H | H |
| CF₃ | CO₂CH₂CF₃ | O | Het-2 | H | H | H | H |
| CF₃ | CO₂CH=CH₂ | O | Het-2 | H | H | H | H |
| CF₃ | CO₂C≡CH | O | Het-2 | H | H | H | H |
| CF₃ | CO₂c-Pr | O | Het-2 | H | H | H | H |
| CF₃ | CO₂CH₂c-Pr | O | Het-2 | H | H | H | H |
| CF₃ | CO₁CH₂CH₂OMe | O | Het-2 | H | H | H | H |
| CF₃ | CO₂CH₂OH₂OCH₂CF₃ | O | Het-2 | H | H | H | H |
| CF₃ | CO₂CH₂CH₂OCH₂CH₂OMe | O | Het-2 | H | H | H | H |
| CF₃ | CO₂CH₂CH₂SMe | O | Het-2 | H | H | H | H |
| CF₃ | CO₂CH₂CH₂SCH₂CF₃ | O | Het-2 | H | H | H | H |
| CF₃ | CO₂Ph | O | Het-2 | H | H | H | H |
| CF₃ | CO₂ (4-ClPh) | O | Het-2 | H | H | H | H |
| CF₃ | CO₂ (4-MePh) | O | Het-2 | H | H | H | H |
| CF₃ | CO₂CH₂Ph | O | Het-2 | H | H | H | H |
| CF₃ | CO₂CH₂ (4-ClPh) | O | Het-2 | H | H | H | H |
| CF₃ | CO₂CH₂ (4-MePh) | O | Het-2 | H | H | H | H |
| CF₃ | CO₂ (3-oxetanyl) | O | Het-2 | H | H | H | H |

**[Table 71]**

| R¹ | R² | W | et-No. | Y¹ | Y² | | Y⁴ |
|---|---|---|---|---|---|---|---|
| CF₃ | CO₂(2-tetrahydrofuryl) | O | Het-2 | H | H | H | H |
| CF₃ | CO₂(3-tetrahydrofuryl) | O | Het-2 | H | H | H | H |
| CF₃ | CO₂(2-pyridyl) | O | Het-2 | H | H | H | H |
| CF₃ | CO₂(2-thienyl) | O | Het-2 | H | H | H | H |
| CF₃ | CO₂(2-tetrahydrofurfuryl) | O | Het-2 | H | H | H | H |
| CF₃ | CO₂CH₂(2-pyridyl) | O | Het-2 | H | H | H | H |
| CF₃ | CO₂CH₂(2-thienyl) | O | Het-2 | H | H | H | H |
| CF₃ | CO(SMe) | O | Het-2 | H | H | H | H |
| CF₃ | CO(SEt) | O | Het-2 | H | H | H | H |
| CF₃ | CO(SCF₃) | O | Het-2 | H | H | H | H |
| CF₃ | CO(SCH₂CF₃) | O | Het-2 | H | H | H | H |
| CF₃ | CONHEt | O | Het-2 | H | H | H | H |
| CF₃ | CONHCH₂CF₃ | O | Het-2 | H | H | H | H |
| CF₃ | CONEt₂ | O | Het-2 | H | H | H | H |
| CF₃ | SO₂Me | O | Het-2 | H | H | Y³ H | H |
| CF₃ | SO₂Et | O | Het-2 | H | H | H | H |
| CF₃ | SO₂n-Pr | O | Het-2 | H | H | H | H |
| CF₃ | SO₂i-Pr | O | Het-2 | H | H | H | H |
| CF₃ | SO₂n-Bu | O | Het-2 | H | H | H | H |
| CF₃ | SO₂i-Bu | O | Het-2 | H | H | H | H |
| CF₃ | SO₂CH₂Cl | O | Het-2 | H | H | H | H |
| CF₃ | SO₂CCl₃ | O | Het-2 | H | H | H | H |
| CF₃ | SO₂CHF₂ | O | Het-2 | H | H | H | H |
| CF₃ | SO₂CF₃ | O | Het-2 | H | H | H | H |
| CF₃ | SO₂CH₂CF₃ | O | Het-2 | H | H | H | H |
| CF₃ | SO₂CH=CH₂ | O | Het-2 | H | H | H | H |
| CF₃ | SO₂CH₂CH=CH₂ | O | Het-2 | H | H | H | H |
| CF₃ | SO₂CH₂C≡CH | O | Het-2 | H | H | H | H |
| CF₃ | SO₂c-Pr | O | Het-2 | H | H | H | H |
| CF₃ | SO₂c-Hex | O | Het-2 | H | H | H | H |

**[Table 72]**

| R¹ | R² | W | et-No. | Y¹ | Y² | Y³ | Y⁴ |
|---|---|---|---|---|---|---|---|
| CF₃ | SO₂CH₂c-Pr | O | Het-2 | H | H | H | H |
| CF₃ | SO₂CH₂CH₂OMe | O | Het-2 | H | H | H | H |
| CF₃ | SO₂Ph | O | Het-2 | H | H | H | H |
| CF₃ | SO₂(4-ClPh) | O | Het-2 | H | H | H | H |
| CF₃ | SO₂(4-MePh) | O | Het-2 | H | H | H | H |
| CF₃ | SO₂CH₂Ph | O | Het-2 | H | H | H | H |
| CF₃ | SO₂CH₂(4-ClPh) | O | Het-2 | H | H | H | H |
| CF₃ | SO₂CH₂(4-MePh) | O | Het-2 | H | H | H | H |
| CF₃ | SO₂NHMe | O | Het-2 | H | H | H | H |
| CF₃ | SO₂NMe₂ | O | Het-2 | H | H | H | H |
| CF₃ | H | O | Het-1 | F | H | H | H |
| CF₃ | H | O | Het-1 | H | F | H | H |
| CF₃ | H | O | Het-1 | H | H | F | H |
| CF₃ | H | O | Het-1 | H | H | H | F |
| CF₃ | H | O | Het-1 | H | F | F | H |
| CF₃ | H | O | Het-1 | Cl | H | H | H |
| CF₃ | H | O | Het-1 | H | CI | H | H |
| CF₃ | H | O | Het-1 | H | H | Cl | H |
| CF₃ | H | O | Het-1 | H | H | H | Cl |
| CF₃ | H | O | Het-1 | H | Cl | Cl | H |
| CF₃ | H | O | Het-1 | H | H | Br | H |
| CF₃ | H | O | Het-1 | H | H | Me | H |
| CF₃ | H | O | Het-1 | H | H | OMe | H |
| CF₃ | H | O | Het-1 | H | H | CF₃ | H |
| CF₃ | H | O | Het-2 | F | H | H | H |
| CF₃ | H | O | Het-2 | H | F | H | H |
| CF₃ | H | O | Het-2 | H | H | F | H |
| CF₃ | H | O | Het-2 | H | H | H | F |
| CF₃ | H | O | Het-2 | H | F | F | H |
| CF₃ | H | O | Het-2 | Cl | H | H | H |

**[Table 73]**

| R¹ | R² | W | et-No. | Y¹ | Y² | Y³ | |
|---|---|---|---|---|---|---|---|
| CF₃ | H | O | Het-2 | H | Cl | H | H |
| CF₃ | H | O | Het-2 | H | H | CI | H |
| CF₃ | H | O | Het-2 | H | H | H | Cl |
| CF₃ | H | O | Het-2 | H | CI | Cl | H |
| CF₃ | H | O | Het-2 | H | H | Br | H |
| CF₃ | H | O | Het-2 | H | H | Me | H |
| CF₃ | H | O | Het-2 | H | H | OMe | H |
| CF₃ | H | O | Het-2 | H | H | CF₃ | H |
| CF₃ | H | O | Het-3 | H | H | H | H |
| CF₃ | H | O | Het-4 | H | H | H | H |
| CF₃ | H | O | Het-5 | H | H | H | H |
| CF₃ | H | O | Het-6 | H | H | H | H |
| CF₃ | H | O | Het-7 | H | H | H | H |
| CF₃ | H | O | Het-8 | H | H | H | H |
| CF₃ | H | O | Het-9 | H | H | H | Y⁴ H |
| CF₃ | H | O | Het-10 | H | H | H | H |
| CF₃ | H | O | Het-11 | H | H | H | H |
| CF₃ | H | O | Het-12 | H | H | H | H |
| CF₃ | H | O | Het-13 | H | H | H | H |
| CF₃ | H | O | Het-14 | H | H | H | H |
| CF₃ | H | O | Het-15 | H | H | H | H |
| CF₃ | H | O | Het-16 | H | H | H | H |
| CF₃ | H | O | Het-17 | H | H | H | H |
| CF₃ | H | O | Het-18 | H | H | H | H |
| CF₃ | H | O | Het-19 | H | H | H | H |
| CF₃ | COc-Pr | O | Het-3 | H | H | H | H |
| CF₃ | COc-Pr | O | Het-4 | H | H | H | H |
| CF₃ | COc-Pr | O | Het-5 | H | H | H | H |
| CF₃ | COc-Pr | O | Het-6 | H | H | H | H |
| CF₃ | COc-Pr | O | Het-7 | H | H | H | H |

**[Table 74]**

| R¹ | R² | W | Het-No. | Y¹ | Y² | Y³ | Y⁴ |
|---|---|---|---|---|---|---|---|
| CF₃ | COc-Pr | O | Het-8 | H | H | H | H |
| CF₃ | COc-Pr | O | Het-9 | H | H | H | H |
| CF₃ | COc-Pr | O | Het-10 | H | H | H | H |
| CF₃ | COc-Pr | O | Het-11 | H | H | H | H |
| CF₃ | COc-Pr | O | Het-12 | H | H | H | H |
| CF₃ | COc-Pr | O | Het-13 | H | H | H | H |
| CF₃ | COc-Pr | O | Het-14 | H | H | H | H |
| CF₃ | COc-Pr | O | Het-15 | H | H | H | H |
| CF₃ | COc-Pr | O | Het-16 | H | H | H | H |
| CF₃ | COc-Pr | O | Het-17 | H | H | H | H |
| CF₃ | COc-Pr | O | Het-18 | H | H | H | H |
| CF₃ | COc-Pr | O | Het-19 | H | H | H | H |
| CF₃ | CO₂Et | O | Het-3 | H | H | H | H |
| CF₃ | CO₂Et | O | Het-4 | H | H | H | H |
| CF₃ | CO₂Et | O | Het-5 | H | H | H | H |
| CF₃ | CO₂Et | O | Het-6 | H | H | H | H |
| CF₃ | CO₂Et | O | Het-7 | H | H | H | H |
| CF₃ | CO₂Et | O | Het-8 | H | H | H | H |
| CF₃ | CO₂Et | O | Het-9 | H | H | H | H |
| CF₃ | CO₂Et | O | Het-10 | H | H | H | H |
| CF₃ | CO₂Et | O | Het-11 | H | H | H | H |
| CF₃ | CO₂Et | O | Het-12 | H | H | H | H |
| CF₃ | CO₂Et | O | Het-13 | H | H | H | H |
| CF₃ | CO₂Et | O | Het-14 | H | H | H | H |
| CF₃ | CO₂Et | O | Het-15 | H | H | H | H |
| CF₃ | CO₂Et | O | Het-16 | H | H | H | H |
| CF₃ | CO₂Et | O | Het-17 | H | H | H | H |
| CF₃ | CO₂Et | O | Het-18 | H | H | H | H |
| CF₃ | CO₂Et | O | Het-19 | H | H | H | H |
| CF₃ | CO₂CH₂CH₂F | O | Het-3 | H | H | H | H |

**[Table 75]**

| R¹ | R² | W | Het-No. | Y¹ | Y² | Y³ | Y⁴ |
|---|---|---|---|---|---|---|---|
| CF₃ | CO₂CH₂CH₂F | O | Het-4 | H | H | H | H |
| CF₃ | CO₂CH₂CH₂F | O | Het-5 | H | H | H | H |
| CF₃ | CO₂CH₂CH₂F | O | Het-6 | H | H | H | H |
| CF₃ | CO₂CH₂CH₂F | O | Het-7 | H | H | H | H |
| CF₃ | CO₂CH₂CH₂F | O | Het-8 | H | H | H | H |
| CF₃ | CO₂CH₂CH₂F | O | Het-9 | H | H | H | H |
| CF₃ | CO₂CH₂CH₂F | O | Het-10 | H | H | H | H |
| CF₃ | CO₂CH₂CH₂F | O | Het-11 | H | H | H | H |
| CF₃ | CO₂CH₂CH₂F | O | Het-12 | H | H | H | H |
| CF₃ | CO₂CH₂CH₂F | O | Het-13 | H | H | H | H |
| CF₃ | CO₂CH₂CH₂F | O | Het-14 | H | H | H | H |
| CF₃ | CO₂CH₂CH₂F | O | Het-15 | H | H | H | H |
| CF₃ | CO₂CH₂CH₂F | O | Het-16 | H | H | H | H |
| CF₃ | CO₂CH₂CH₂F | O | Het-17 | H | H | H | H |
| CF₃ | CO₂CH₂CH₂F | O | Het-18 | H | H | H | H |
| CF₃ | CO₂CH₂CH₂F | O | Het-19 | H | H | H | H |
| CF₃ | CO₂CH₂CHF₂ | O | Het-3 | H | H | H | H |
| CF₃ | CO₂CH₂CHF₂ | O | Het-4 | H | H | H | H |
| CF₃ | CO₂CH₂CHF₂ | O | Het-5 | H | H | H | H |
| CF₃ | CO₂CH₂CHF₂ | O | Het-6 | H | H | H | H |
| CF₃ | CO₂CH₂CHF₂ | O | Het-7 | H | H | H | H |
| CF₃ | CO₂CH₂CHF₂ | O | Het-8 | H | H | H | H |
| CF₃ | CO₂CH₂CHF₂ | O | Het-9 | H | H | H | H |
| CF₃ | CO₂CH₂CHF₂ | O | Het-10 | H | H | H | H |
| CF₃ | CO₂CH₂CHF₂ | O | Het-11 | H | H | H | H |
| CF₃ | CO₂CH₂CHF₂ | O | Het-12 | H | H | H | H |
| CF₃ | CO₂CH₂CHF₂ | O | Het-13 | H | H | H | H |
| CF₃ | CO₂CH₂CHF₂ | O | Het-14 | H | H | H | H |
| CF₃ | CO₂CH₂CHF₂ | O | Het-15 | H | H | H | H |
| CF₃ | CO₂CH₂CHF₂ | O | Het-16 | H | H | H | H |

**[Table 76]**

| R¹ | R² | W | et-No. | Y¹ | Y² | Y³ | Y⁴ |
|---|---|---|---|---|---|---|---|
| CF₃ | CO₂CH₂CHF₂ | O | Het-17 | H | H | H | H |
| CF₃ | CO₂CH₂CHF₂ | O | Het-18 | H | H | H | H |
| CF₃ | CO₂CH₂CHF₂ | O | Het-19 | H | H | H | H |
| CF₃ | CO₂(3-oxetanyl) | O | Het-3 | H | H | H | H |
| CF₃ | CO₂(3-oxetanyl) | O | Het-4 | H | H | H | H |
| CF₃ | CO₂(3-oxetanyl) | O | Het-5 | H | H | H | H |
| CF₃ | CO₂(3-oxetanyl) | O | Het-6 | H | H | H | H |
| CF₃ | CO₂(3-oxetanyl) | O | Het-7 | H | H | H | H |
| CF₃ | CO₂(3-oxetanyl) | O | Het-8 | H | H | H | H |
| CF₃ | CO₂(3-oxetanyl) | O | Het-9 | H | H | H | H |
| CF₃ | CO₂(3-oxetanyl) | O | Het-10 | H | H | H | H |
| CF₃ | CO₂(3-oxetanyl) | O | Het-11 | H | H | H | H |
| CF₃ | CO₂(3-oxetanyl) | O | Het-12 | H | H | H | H |
| CF₃ | CO₂(3-oxetanyl) | O | Het-13 | H | H | H | H |
| CF₃ | CO₂(3-oxetanyl) | O | Het-14 | H | H | H | H |
| CF₃ | CO₂(3-oxetanyl) | O | Het-15 | H | H | H | H |
| CF₃ | CO₂(3-oxetanyl) | O | Het-16 | H | H | H | H |
| CF₃ | CO₂(3-oxetanyl) | O | Het-17 | H | H | H | H |
| CF₃ | CO₂(3-oxetanyl) | O | Het-18 | H | H | H | H |
| CF₃ | CO₂(3-oxetanyl) | O | Het-19 | H | H | H | H |
| CF₃ | CO₂(3-tetrahydrofuryl) | O | Het-3 | H | H | H | H |
| CF₃ | CO₂(3-tetrahydrofuryl) | O | Het-4 | H | H | H | H |
| CF₃ | CO₂(3-tetrahydrofuryl) | O | Het-5 | H | H | H | H |
| CF₃ | CO₂(3-tetrahydrofuryl) | O | Het-6 | H | H | H | H |
| CF₃ | CO₂(3-tetrahydrofuryl) | O | Het-7 | H | H | H | H |
| CF₃ | CO₂(3-tetrahydrofuryl) | O | Het-8 | H | H | H | H |
| CF₃ | CO₂(3-tetrahydrofuryl) | O | Het-9 | H | H | H | H |
| CF₃ | CO₂(3-tetrahydrofuryl) | O | Het-10 | H | H | H | H |
| CF₃ | CO₂(3-tetrahydrofuryl) | O | Het-11 | H | H | H | H |
| CF₃ | CO₂(3-tetrahydrofuryl) | O | Het-12 | H | H | H | H |

**[Table 77]**

| R¹ | R² | W | Het-No. | Y¹ | Y² | Y³ | Y⁴ |
|---|---|---|---|---|---|---|---|
| CF₃ | CO₂(3-tetrahydrofuryl) | O | Het-13 | H | H | H | H |
| CF₃ | CO₂(3-tetrahydrofuryl) | O | Het-14 | H | H | H | H |
| CF₃ | CO₂(3-tetrahydrofuryl) | O | Het-15 | H | H | H | H |
| CF₃ | CO₂(3-tetrahydrofuryl) | O | Het-16 | H | H | H | H |
| CF₃ | CO₂(3-tetrahydrofuryl) | O | Het-17 | H | H | H | H |
| CF₃ | CO₂(3-tetrahydrofuryl) | O | Het-18 | H | H | H | H |
| CF₃ | CO₂(3-tetrahydrofuryl) | O | Het-19 | H | H | H | H |
| CF₃ | SO₂Me | O | Het-3 | H | H | H | H |
| CF₃ | SO₂Me | O | Het-4 | H | H | H | H |
| CF₃ | SO₂Me | O | Het-5 | H | H | H | H |
| CF₃ | SO₂Me | O | Het-6 | H | H | H | H |
| CF₃ | SO₂Me | O | Het-7 | H | H | H | H |
| CF₃ | SO₂Me | O | Het-8 | H | H | H | H |
| CF₃ | SO₂Me | O | Het-9 | H | H | H | H |
| CF₃ | SO₂Me | O | Het-10 | H | H | H | H |
| CF₃ | SO₂Me | O | Het-11 | H | H | H | H |
| CF₃ | SO₂Me | O | Het-12 | H | H | H | H |
| CF₃ | SO₂Me | O | Het-13 | H | H | H | H |
| CF₃ | SO₂Me | O | Het-14 | H | H | H | H |
| CF₃ | SO₂Me | O | Het-15 | H | H | H | H |
| CF₃ | SO₂Me | O | Het-16 | H | H | H | H |
| CF₃ | SO₂Me | O | Het-17 | H | H | H | H |
| CF₃ | SO₂Me | O | Het-18 | H | H | H | H |
| CF₃ | SO₂Me | O | Het-19 | H | H | H | H |
| CF₃ | H | S | Het-1 | H | H | H | H |
| CF₃ | COc-Pr | S | Het-1 | H | H | H | H |
| CF₃ | CO₂Et | S | Het-1 | H | H | H | H |
| CF₃ | CO₂CH₂CH₂F | S | Het-1 | H | H | H | H |
| CF₃ | CO₂CH₂CHF₂ | S | Het-1 | H | H | H | H |
| CF₃ | CO₂(3-oxetanyl) | S | Het-1 | H | H | H | H |

**[Table 78]**

| R¹ | R² | W | Het-No. | Y¹ | Y² | Y³ | Y⁴ |
|---|---|---|---|---|---|---|---|
| CF₃ | CO₂(3-tetrahydrofuryl) | S | Het-1 | H | H | H | H |
| CF₃ | SO₂Me | S | Het-1 | H | H | H | H |
| CF₃ | H | S | Het-2 | H | H | H | H |
| CF₃ | COc-Pr | S | Het-2 | H | H | H | H |
| CF₃ | CO₂Et | S | Het-2 | H | H | H | H |
| CF₃ | CO₂CH₂CH₂F | S | Het-2 | H | H | H | H |
| CF₃ | CO₂CH₂CHF₂ | S | Het-2 | H | H | H | H |
| CF₃ | CO₂(3-oxetanyl) | S | Het-2 | H | H | H | H |
| CF₃ | CO₂(3-tetrahydrofuryl) | S | Het-2 | H | H | H | H |
| CF₃ | SO₂Me | S | Het-2 | H | H | H | H |

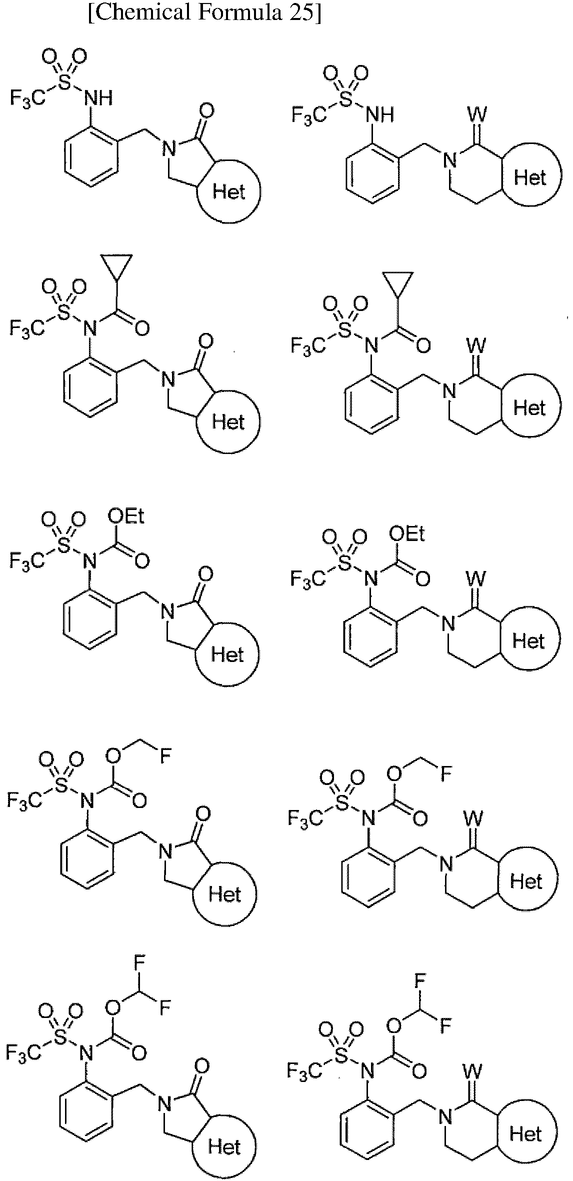

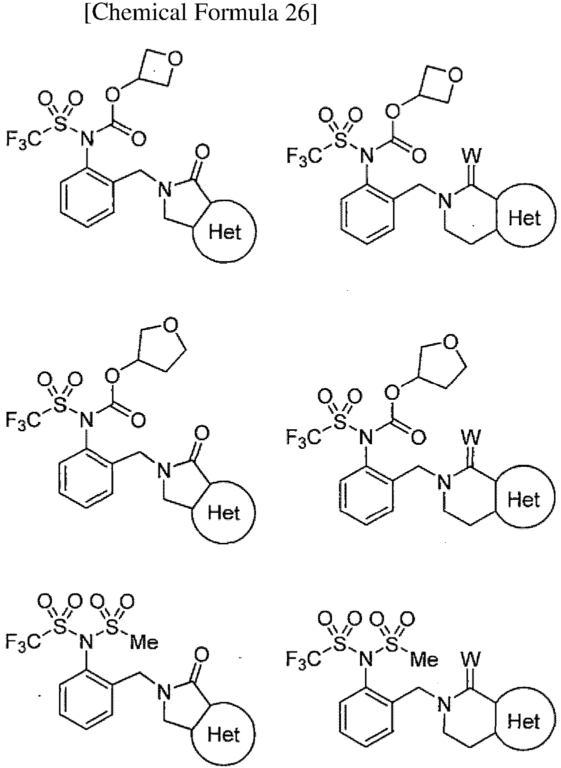

**[Table 79]**

| Het | | | | Het | | | |
|---|---|---|---|---|---|---|---|
| Het-No. | X⁵ | X⁶ | X⁷ | Het-No. | X⁵ | X⁶ | X⁷ |
| Het-19 | Me | H | - | Het-20 | F | Me | - |
| Het-19 | F | H | - | Het-20 | F | F | - |
| Het-19 | Cl | H | - | Het-20 | F | Cl | - |
| Het-19 | OSO₂Me | H | - | Het-20 | F | F | - |
| Het-19 | CN | H | - | Het-21 | Me | H | - |
| Het-19 | NO₂ | H | - | Het-21 | F | H | - |
| Het-19 | H | Me | - | Het-21 | Cl | H | - |
| Het-19 | H | F | - | Het-21 | OSO₂Me | H | - |
| Het-19 | H | Cl | - | Het-21 | CN | H | - |
| Het-19 | H | OSO₂Me | - | Het-21 | NO₂ | H | - |
| Het-19 | H | CN | - | Het-21 | H | Me | - |
| Het-19 | H | NO₂ | - | Het-21 | | F | - |
| Het-19 | H | Ph | - | Het-21 | H | Cl | - |
| Het-19 | F | Me | - | Het-21 | H | OSO₂Me | - |
| Het-19 | F | F | - | Het-21 | H | CN | - |
| Het-19 | F | Cl | - | Het-21 | H | NO₂ | - |
| Het-19 | Me | F | - | Het-21 | H | Ph | - |
| Het-19 | F | OSO₂Me | - | Het-21 | F | Me | - |
| Het-19 | Cl | F | - | Het-21 | F | F | - |
| Het-19 | Cl | Cl | - | Het-21 | F | Cl | - |
| Het-20 | Me | H | - | Het-21 | Me | F | - |
| Het-20 | F | H | - | Het-21 | Cl | F | - |
| Het-20 | Cl | H | - | Het-21 | Cl | CI | - |
| Het-20 | OSO₂Me | H | - | Het-21 | Me | Me | - |
| Het-20 | CN | H | - | Het-22 | Me | H | - |
| Het-20 | NO₂ | H | - | Het-22 | F | H | - |
| Het-20 | Ph | H | - | Het-22 | Cl | H | - |

**[Table 80]**

| Het | | | | Het | | | |
|---|---|---|---|---|---|---|---|
| Het-No. | X⁵ | X⁶ | X⁷ | Het-No. | X⁵ | X⁶ | X⁷ |
| Het-22 | OSO₂Me | H | - | Het-23 | F | F | - |
| Het-22 | CN | H | - | Het-24 | Me | H | - |
| Het-22 | NO₂ | H | - | Het-24 | F | H | - |
| Het-22 | Ph | H | - | Het-24 | Cl | H | - |
| Het-22 | F | Me | - | Het-24 | OSO₂Me | H | - |
| Het-22 | F | F | - | Het-24 | CN | H | - |
| Het-22 | F | Cl | - | Het-24 | NO₂ | H | - |
| Het-22 | F | F | - | Het-24 | Ph | H | - |
| Het-23 | Me | H | - | Het-24 | F | Me | - |
| Het-23 | F | H | - | Het-24 | F | F | - |
| Het-23 | Cl | H | - | Het-24 | F | Cl | - |
| Het-23 | OSO₂Me | H | - | Het-24 | Cl | Cl | - |
| Het-23 | CN | H | - | Het-25 | Me | - | - |
| Het-23 | NO₂ | H | - | Het-25 | F | - | - |
| Het-23 | H | Me | - | Het-25 | Cl | - | - |
| Het-23 | H | F | - | Het-25 | OSO₂Me | - | - |
| Het-23 | H | Cl | - | Het-25 | CN | - | - |
| Het-23 | H | OSO₂Me | - | Het-25 | NO₂ | - | - |
| Het-23 | H | CN | - | Het-25 | Ph | - | - |
| Het-23 | H | NO₂ | - | Het-26 | Me | - | - |
| Het-23 | H | Ph | - | Het-26 | F | - | - |
| Het-23 | F | Me | - | Het-26 | Cl | - | - |
| Het-23 | F | F | - | Het-26 | OSO₂Me | - | - |
| Het-23 | F | Cl | - | Het-26 | CN | - | - |
| Het-23 | Me | F | - | Het-26 | NO₂ | - | - |
| Het-23 | Cl | Cl | - | Het-27 | - | - | - |
| Het-23 | Cl | F | - | Het-28 | Me | - | - |

**[Table 81]**

| Het | | | | Het | | | |
|---|---|---|---|---|---|---|---|
| Het-No. | X⁵ | X⁶ | X⁷ | Het-No. | X⁵ | X⁶ | X⁷ |
| Het-28 | F | - | - | Het-32 | Cl | - | - |
| Het-28 | Cl | - | - | Het-32 | OSO₂Me | - | - |
| Het-28 | OSO₂Me | - | - | Het-32 | CN | - | - |
| Het-28 | CN | - | - | Het-32 | NO₂ | - | - |
| Het-28 | NO₂ | - | - | Het-32 | Ph | - | - |
| Het-29 | Me | - | - | Het-33 | Me | - | - |
| Het-29 | F | - | - | Het-33 | F | - | - |
| Het-29 | Cl | - | - | Het-33 | Cl | - | - |
| Het-29 | OSO₂Me | - | - | Het-33 | OSO₂Me | - | - |
| Het-29 | CN | - | - | Het-33 | CN | - | - |
| Het-29 | NO₂ | - | - | Het-33 | NO₂ | - | - |
| Het-29 | Ph | - | - | Het-33 | Ph | - | - |
| Het-30 | Me | - | - | Het-34 | - | - | - |
| Het-30 | F | - | - | Het-35 | - | - | - |
| Het-30 | Cl | - | - | Het-36 | - | - | - |
| Het-30 | OSO₂Me | - | - | Het-37 | - | - | - |
| Het-30 | CN | - | - | Het-38 | Me | H | H |
| Het-30 | NO₂ | - | - | Het-38 | F | H | H |
| Het-30 | Ph | - | - | Het-38 | Cl | H | H |
| Het-31 | Me | - | - | Het-38 | OSO₂Me | H | H |
| Het-31 | F | - | - | Het-38 | CN | H | H |
| Het-31 | Cl | - | - | Het-38 | NO₂ | H | H |
| Het-31 | OSO₂Me | - | - | Het-38 | H | Me | H |
| Het-31 | CN | - | - | Het-38 | H | F | H |
| Het-31 | NO₂ | - | - | Het-38 | H | Cl | H |
| Het-32 | Me | - | - | Het-38 | H | OSO₂Me | H |
| Het-32 | F | - | - | Het-38 | H | CN | H |

**[Table 82]**

| Het | | | | Het | | | |
|---|---|---|---|---|---|---|---|
| Het-No. | X⁵ | X⁶ | X⁷ | Het-No. | X⁵ | X⁶ | X⁷ |
| Het-38 | H | | H | Het-38 | H | H | SH |
| Het-38 | H | H | Me | Het-38 | H | H | SMe |
| Het-38 | H | H | Et | Het-38 | H | H | SO₂Me |
| Het-38 | H | H | t-Bu | Het-38 | H | H | SO₂CF₃ |
| Het-38 | H | H | c-Pr | Het-38 | H | H | CH₂OMe |
| Het-38 | H | H | F | Het-38 | H | H | CH₂NMe₂ |
| Het-38 | H | H | Cl | Het-38 | H | H | CH=CH₂ |
| Het-38 | H | H | Br | Het-38 | H | H | C≡CH |
| Het-38 | H | H | CF₃ | Het-38 | F | Me | H |
| Het-38 | H | H | C₂F₅ | Het-38 | F | F | H |
| Het-38 | H | H | OH | Het-38 | F | Cl | H |
| Het-38 | H | H | OMe | Het-38 | Me | F | H |
| Het-38 | H | H | OSO₂Me | Het-38 | Cl | F | H |
| Het-38 | H | NO₂ H | OCF₃ | Het-38 | Cl | Cl | H |
| Het-38 | H | H | OCHF₂ | Het-38 | F | F | H |
| Het-38 | H | H | OSiMe₃ | Het-38 | F | H | Me |
| Het-38 | H | H | OCH₂C≡CH | Het-38 | F | H | F |
| Het-38 | H | H | CHO | Het-38 | F | H | Cl |
| Het-38 | H | H | C (=NOMe) Me | Het-38 | Me | H | F |
| Het-38 | H | H | CN | Het-38 | F | H | F |
| Het-38 | H | H | NO₂ | Het-38 | Cl | H | F |
| Het-38 | H | H | CO₂H | Het-38 | F | H | F |
| Het-38 | H | H | CO₂Me | Het-38 | H | F | Me |
| Het-38 | H | H | OCOMe | Het-38 | H | F | F |
| Het-38 | H | H | NHCO₂Me | Het-38 | H | F | Cl |
| Het-38 | H | H | Ph | Het-38 | H | Me | F |
| Het-38 | H | H | NMe₂ | Het-38 | H | Cl | F |
| Het-38 | H | H | NHSO₂Me | Het-38 | H | C l | Cl |

**[Table 83]**

| Het | | | | Het | | | |
|---|---|---|---|---|---|---|---|
| Het-No. | X⁵ | X⁶ | X⁷ | Het-No. | X⁵ | X⁶ | X⁷ |
| Het-38 | H | Me | Me | Het-39 | Ph | H | H |
| Het-38 | Me | Me | Me | Het-39 | NMe₂ | H | H |
| Het-38 | F | F | F | Het-39 | NHSO₂Me | H | H |
| Het-38 | Cl | Cl | Cl | Het-39 | SH | H | H |
| Het-39 | Me | H | H | Het-39 | SMe | H | H |
| Het-39 | Et | H | H | Het-39 | SO₂Me | H | H |
| Het-39 | t-Bu | H | H | Het-39 | SO₂CF₃ | H | H |
| Het-39 | c-Pr | H | H | Het-39 | CH₂OMe | H | H |
| Het-39 | F | H | H | Het-39 | CH₂NMe₂ | H | H |
| Het-39 | Cl | H | H | Het-39 | CH=CHz | H | H |
| Het-39 | Br | H | H | Het-39 | C≡CH | H | H |
| Het-39 | CF₃ | H | H | Het-39 | H | Me | H |
| Het-39 | C₂F₅ | H | H | Het-39 | H | F | H |
| Het-39 | OH | H | H | Het-39 | H | Cl | H |
| Het-39 | OMe | H | H | Het-39 | H | OSO₂Me | H |
| Het-39 | OSO₂Me | H | H | Het-39 | H | CN | H |
| Het-39 | OCF₃ | H | H | Het-39 | H | NO₂ | H |
| Het-39 | OCHF₂ | H | H | Het-39 | H | H | Me |
| Het-39 | OSiMe₃ | H | H | Het-39 | H | H | Et |
| Het-39 | OCH₂C≡CH | H | H | Het-39 | H | H | t-Bu |
| Het-39 | CHO | H | H | Het-39 | H | H | c-Pr |
| Het-39 | C(=NOMe)Me | H | H | Het-39 | H | H | F |
| Het-39 | CN | H | H | Het-39 | H | H | Cl |
| Het-39 | NO₂ | H | H | Het-39 | H | H | Br |
| Het-39 | CO₂H | H | H | Het-39 | H | | CF₃ |
| Het-39 | CO₂Me | H | H | Het-39 | H | H | C₂F₅ |
| Het-39 | OCOMe | H | H | Het-39 | H | H | OH |
| Het-39 | NHCO₂Me | H | H | Het-39 | H | H | OMe |

**[Table 84]**

| Het | | | | Het | | | |
|---|---|---|---|---|---|---|---|
| Het-No. | X⁵ | X⁶ | X⁷ | Het-No. | X⁵ | X⁶ | X⁷ |
| Het-39 | H | H | OSO₂Me | Het-39 | Cl | F | H |
| Het-39 | H | H | OCF₃ | Het-39 | Cl | Cl | H |
| Het-39 | H | H | OCHF₂ | Het-39 | Me | Me | H |
| Het-39 | H | H | OSiMe₃ | Het-39 | F | H | Me |
| Het-39 | H | H | OCH₂C≡CH | Het-39 | F | H | F |
| Het-39 | H | H | CHO | Het-39 | F | H | Cl |
| Het-39 | H | H | C (=NOMe) Me | Het-39 | Me | H | F |
| Het-39 | H | H | CN | Het-39 | Cl | H | F |
| Het-39 | H | H | NO₂ | Het-39 | Cl | H | Cl |
| Het-39 | H | H | CO₂H | Het-39 | Me | H | Me |
| Het-39 | H | H | CO₂Me | Het-39 | H | F | Me |
| Het-39 | H | H | OCOMe | Het-39 | H | F | F |
| Het-39 | H | H | NHCO₂Me | Het-39 | H | F | Cl |
| Het-39 | H | H | Ph | Het-39 | H | Me | F |
| Het-39 | H | H | NMe₂ | Het-39 | H | Cl | F |
| Het-39 | H | H | NHSO₂Me | Het-39 | H | Cl | Cl |
| Het-39 | H | H | SH | Het-39 | H | Me | Me |
| Het-39 | H | H | SMe | Het-39 | Me | Me | Me |
| Het-39 | H | H | SO₂Me | Het-39 | F | F | F |
| Het-39 | H | H | SO₂CF₃ | Het-39 | Cl | Cl | Cl |
| Het-39 | H | H | CH₂OMe | Het-40 | Me | H | - |
| Het-39 | H | H | CH₂NMe₂ | Het-40 | F | H | - |
| Het-39 | H | H | CH=CH₂ | Het-40 | Cl | H | - |
| Het-39 | H | H | C≡CH | Het-40 | OSO₂Me | H | - |
| Het-39 | F | Me | H | Het-40 | CN | H | - |
| Het-39 | F | F | H | Het-40 | NO₂ | H | - |
| Het-39 | F | Cl | H | Het-40 | H | Me | - |
| Het-39 | Me | F | H | Het-40 | H | F | - |

**[Table 85]**

| Het | | | | Het | | | |
|---|---|---|---|---|---|---|---|
| Het-No. | X⁵ | X⁶ | X⁷ | Het-No. | X⁵ | X⁶ | X⁷ |
| Het-40 | H | Cl | - | Het-41 | F | Me | - |
| Het-40 | H | Br | - | Het-41 | F | F | - |
| Het-40 | H | CF₃ | - | Het-41 | F | Cl | - |
| Het-40 | H | OMe | - | Het-41 | Cl | Cl | - |
| Het-40 | H | OSO₂Me | - | Het-42 | Me | H | - |
| Het-40 | H | OCF₃ | - | Het-42 | F | H | - |
| Het-40 | H | OCHF₂ | - | Het-42 | Cl | H | - |
| Het-40 | H | CN | - | Het-42 | OSO₂Me | H | - |
| Het-40 | H | NO₂ | - | Het-42 | CN | H | - |
| Het-40 | F | Me | - | Het-42 | NO₂ | H | - |
| Het-40 | F | F | - | Het-42 | H | Me | - |
| Het-40 | F | Cl | - | Het-42 | H | F | - |
| Het-40 | Me | F | - | Het-42 | H | Cl | - |
| Het-40 | Cl | F | - | Het-42 | H | Br | - |
| Het-40 | Cl | Cl | - | Het-42 | H | CF₃ | - |
| Het-40 | Me | Me | - | Het-42 | H | OMe | - |
| Het-41 | Me | H | - | Het-42 | H | OSO₂Me | - |
| Het-41 | F | H | - | Het-42 | H | OCF₃ | - |
| Het-41 | Cl | H | - | Het-42 | H | OCHF₂ | - |
| Het-41 | Br | H | - | Het-42 | H | CN | - |
| Het-41 | CF₃ | H | - | Het-42 | H | NO₂ | - |
| Het-41 | OMe | H | - | Het-42 | F | Me | - |
| Het-41 | OSO₂Me | H | - | Het-42 | F | F | - |
| Het-41 | OCF₃ | H | - | Het-42 | F | Cl | - |
| Het-41 | OCHF₂ | H | - | Het-42 | Me | F | - |
| Het-41 | CN | H | - | Het-42 | Cl | F | - |
| Het-41 | NO₂ | H | - | Het-42 | Cl | Cl | - |

**[Table 86]**

| Het | | | | Het | | | |
|---|---|---|---|---|---|---|---|
| Het-No. | X⁵ | X⁶ | X⁷ | Het-No. | X⁵ | X⁶ | X⁷ |
| Het-42 | Me | Me | - | Het-47 | F | F | - |
| Het-43 | Me | H | - | Het-47 | Cl | Cl | - |
| Het-43 | F | H | - | Het-48 | F | F | - |
| Het-43 | Cl | H | - | Het-48 | Cl | Cl | - |
| Het-43 | OSO₂Me | H | - | Het-49 | F | F | - |
| Het-43 | CN | H | - | Het-49 | Cl | Cl | - |
| Het-43 | NO₂ | H | - | Het-50 | F | F | - |
| Het-43 | F | Me | - | Het-50 | Cl | Cl | - |
| Het-43 | F | F | - | Het-51 | F | F | - |
| Het-43 | F | Cl | - | Het-51 | Cl | Cl | - |
| Het-43 | Cl | Cl | - | Het-52 | F | F | - |
| Het-44 | Me | - | - | Het-52 | Cl | Cl | - |
| Het-44 | F | - | - | Het-53 | F | F | - |
| Het-44 | Cl | - | - | Het-53 | Cl | Cl | - |
| Het-44 | OSO₂Me | - | - | Het-54 | F | F | - |
| Het-44 | CN | - | - | Het-54 | H | F | - |
| Het-44 | NO₂ | - | - | Het-55 | F | F | - |
| Het-45 | Me | - | - | Het-55 | Cl | Cl | - |
| Het-45 | F | - | - | Het-56 | F | F | - |
| Het-45 | Cl | - | - | Het-56 | Cl | Cl | - |
| Het-45 | OSO₂Me | - | - | Het-57 | F | F | - |
| Het-45 | CN | - | - | Het-57 | Cl | Cl | - |
| Het-45 | NO₂ | - | - | Het-58 | F | F | - |
| Het-46 | F | F | - | Het-58 | Cl | Cl | - |
| Het-46 | Cl | Cl | - | | | | |

More specific examples include:

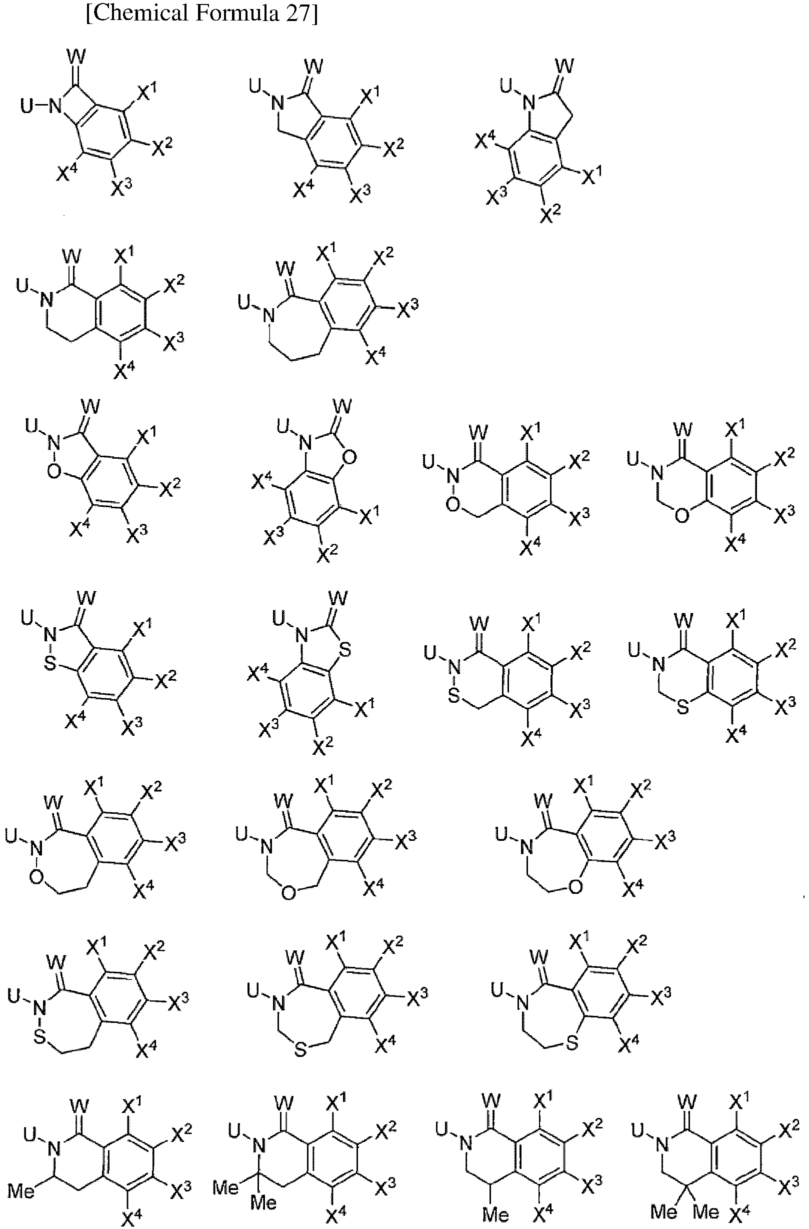

U is represented by the following general formula (23):

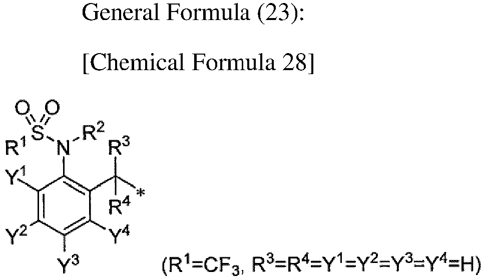

**[Table 87]**

| R² | W | X¹ | X² | X³ | X⁴ |
|---|---|---|---|---|---|
| H | O | H | H | H | H |
| CH₂CH=CH₂ | O | H | H | H | H |
| CH₂C≡CH | O | H | H | H | H |
| CH₂OMe | O | H | H | H | H |
| CH₂OCH₂CF₃ | O | H | H | H | H |
| CH₂OCH₂CH₂OMe | O | H | H | H | H |
| CH₂SMe | O | H | H | H | H |
| CH₂COMe | O | H | H | H | H |
| CH₂CH₂OPh | O | H | H | H | H |
| COMe | O | H | H | H | H |
| COEt | O | H | H | H | H |
| COn-Pr | O | H | H | H | H |
| COi-Pr | O | H | H | H | H |
| COn-Bu | O | H | H | H | H |
| COs-Bu | O | H | H | H | H |
| COi-Bu | O | H | H | H | H |
| COt-Bu | O | H | H | H | H |
| COn-Pen | O | H | H | H | H |
| COn-Hex | O | H | H | H | H |
| COCF₃ | O | H | H | H | H |
| COCH₂CF₃ | O | H | H | H | H |
| COCH=CH₂ | O | H | H | H | H |
| COC≡CH | O | H | H | H | H |
| COc-Pr | O | H | H | H | H |
| COc-Bu | O | H | H | H | H |
| COc-Pen | O | H | H | H | H |
| COc-Hex | O | H | H | H | H |

**[Table 88]**

| R² | W | X¹ | X² | X³ | X⁴ |
|---|---|---|---|---|---|
| COCH₂c-Pr | O | H | H | H | H |
| COCH₂OMe | O | H | H | H | H |
| COCH₂OCH₂CF₃ | O | H | H | H | H |
| COCH₂OCH₂CH₂CF₃ | O | H | H | H | H |
| COCH₂SMe | O | H | H | H | H |
| COCH₂SCH₂CF₃ | O | H | H | H | H |
| Bz | O | H | H | H | H |
| 4-ClBz | O | H | H | H | H |
| 4-MeBz | O | H | H | H | H |
| COCH₂Ph | O | H | H | H | H |
| COCH₂(4-ClPh) | O | H | H | H | H |
| COCH₂(4-MePh) | O | H | H | H | H |
| CO(2-tetrahydrofuryl) | O | H | H | H | H |
| CO(3-tetrahydrofuryl) | O | H | H | H | H |
| CO(2-pyridyl) | O | H | H | H | H |
| CO(3-pyridyl) | O | H | H | H | H |
| CO(4-pyridyl) | O | H | H | H | H |
| CO(2-thienyl) | O | H | H | H | H |
| CO(3-thienyl) | O | H | H | H | H |
| CO (2-tetrahydrofurfuryl) | O | H | H | H | H |
| COCH₂(2-pyridyl) | O | H | H | H | H |
| COCH₂(2-thienyl) | O | H | H | H | H |
| CO₂Me | O | H | H | H | H |
| CO₂Et | O | H | H | H | H |
| CO₂n-Pr | O | H | H | H | H |
| CO₂i-Pr | O | H | H | H | H |
| CO₂n-Bu | O | H | H | H | H |

**[Table 89]**

| R² | W | X¹ | X² | X³ | X⁴ |
|---|---|---|---|---|---|
| CO₂s-Bu | O | H | H | H | H |
| CO₂i -Bu | O | H | H | H | H |
| CO₂t-Bu | O | H | H | H | H |
| CO₂n-Pen | O | H | H | H | H |
| CO₂n-Hex | O | H | H | H | H |
| CO₂CH₂CH₂F | O | H | H | H | H |
| CO₂CH₂CHF₂ | O | H | H | H | H |
| CO₂CH₂CF₃ | O | H | H | H | H |
| CO₂CH=CH₂ | O | H | H | H | H |
| CO₂C≡CH | O | H | H | H | H |
| CO₂c-Pr | O | H | H | H | H |
| CO₂CH₂c-Pr | O | H | H | H | H |
| CO₂CH₂CH₂OMe | O | H | H | H | H |
| CO₂CH₂CH₂OCH₂CF₃ | O | H | H | H | H |
| CO₂CH₂CH₂OCH₂CH₂OMe | O | H | H | H | H |
| CO₂CH₂CH₂SMe | O | H | H | H | H |
| CO₂CH₂CH₂SCH₂CF₃ | O | H | H | H | H |
| CO₂Ph | O | H | H | H | H |
| CO₂(4-ClPh) | O | H | H | H | H |
| CO₂(4-MePh) | O | H | H | H | H |
| CO₂CH₂Ph | O | H | H | H | H |
| CO₂CH₂(4-ClPh) | O | H | H | H | H |
| CO₂CH₂(4-MePh) | O | H | H | H | H |
| CO₂(3-oxetanyl) | O | H | H | H | H |
| CO₂(2-tetrahydrofuryl) | O | H | H | H | H |
| CO₂(3-tetrahydrofuryl) | O | H | H | H | H |
| CO₂(2-pyridyl) | O | H | H | H | H |

**[Table 90]**

| R² | W | X¹ | X² | X³ | X⁴ |
|---|---|---|---|---|---|
| CO₂(2-thienyl) | O | H | H | H | H |
| CO₂(2-tetrahydrofurfuryl) | O | H | H | H | H |
| CO₂CH₂(2-pyr i dyl) | O | H | H | H | H |
| CO₂CH₂(2-th i enyl) | O | H | H | H | H |
| CO(SMe) | O | H | H | H | H |
| CO(SEt) | O | H | H | H | H |
| CO(SCF₃) | O | H | H | H | H |
| CO(SCH₂CF₃) | O | H | H | H | H |
| CONHEt | O | H | H | H | H |
| CONHOH₂CF₃ | O | H | H | H | H |
| CONEt₂ | O | H | H | H | H |
| SO₂Me | O | H | H | H | H |
| SO₂Et | O | H | H | H | H |
| SO₂n-Pr | O | H | H | H | H |
| SO₂i-Pr | O | H | H | H | H |
| SO₂n-Bu | O | H | H | H | H |
| SO₂i-Bu | O | H | H | H | H |
| SO₂CH₂Cl | O | H | H | H | H |
| SO₂CCl₃ | O | H | H | H | H |
| SO₂CHF₂ | O | H | H | H | H |
| SO₂CF₃ | O | H | H | H | H |
| SO₂CH₂CF₃ | O | H | H | H | H |
| SO₂CH=CH₂ | O | H | H | H | H |
| SO₂CH₂CH=CH₂ | O | H | H | H | H |
| SO₂CH₂C≡CH | O | H | H | H | H |
| SO₂c-Pr | O | H | H | H | H |
| SO₂c-Hex | O | H | H | H | H |

**[Table 91]**

| R² | W | | X² | X³ | X⁴ |
|---|---|---|---|---|---|
| SO₂CH₂c-Pr | O | H | H | H | H |
| SO₂CH₂CH₂OMe | O | H | H | H | H |
| SO₂Ph | O | H | H | H | H |
| SO₂(4-ClPh) | O | H | H | H | H |
| SO₂(4-MePh) | O | H | H | H | H |
| SO₂CH₂Ph | O | H | H | H | H |
| SO₂CH₂(4-C IPh) | O | H | H | H | H |
| SO₂CH₂(4-MePh) | O | H | H | H | H |
| SO₂NHMe | O | H | H | H | H |
| SO₂NMe₂ | O | H | H | H | H |
| H | O | F | H | H | H |
| CH₂CF₃ | O | F | H | H | H |
| CH₂CH=CH₂ | O | F | H | H | H |
| CH₂C≡CH | O | F | H | H | H |
| CH₂OMe | O | F | H | H | H |
| CH₂OCH₂CF₃ | O | F | H⁻ | H | H |
| CH₂OCH₂CH₂OMe | O | F | H | H | H |
| CH₂SMe | O | F | H | H | H |
| CH₂COMe | O | F | H | H | H |
| CH₂CH₂OPh | O | F | H | H | H |
| COMe | O | F | H | H | H |
| COEt | O | F | H | H | H |
| COn-Pr | O | F | H | H | H |
| COi-Pr | O | F | H | H | H |
| COn-Bu | O | F | H | H | H |
| COs-Bu | O | F | H | H | H |
| COi-Bu | O | F | H | H | H |

**[Table 92]**

| R² | W | | X² | X³ | X⁴ |
|---|---|---|---|---|---|
| COt-Bu | O | F | H | H | H |
| COn-Pen | O | F | H | H | H |
| COn-Hex | O | F | H | H | H |
| COCF₃ | O | F | H | H | H |
| COCH₂CF₃ | O | F | H | H | H |
| COCH=CH₂ | O | F | H | H | H |
| COC≡CH | O | F | H | H | H |
| COc-Pr | O | F | H | H | H |
| COc-Bu | O | F | H | H | H |
| COc-Pen | O | F | H | H | H |
| COc-Hex | O | F | H | H | H |
| COCH₂c-Pr | O | F | H | H | H |
| COCH₂OMe | O | F | H | H | H |
| COCH₂OCH₂CF₃ | O | F | H | H | H |
| COCH₂OCH₂CH₂CF₃ | O | F | H | H | H |
| COCH₂SMe | O | F | H | H | H |
| COCH₂SCH₂CF₃ | O | F | H | H | H |
| Bz | O | F | H | H | H |
| 4-ClBz | O | F | H | H | H |
| 4-MeBz | O | F | H | H | H |
| COCH₂Ph | O | F | H | H | H |
| COCH₂(4-ClPh) | O | F | H | H | H |
| COCH₂(4-MePh) | O | F | H | H | H |
| CO(2-tetrahydrofuryl) | O | F | H | H | H |
| CO(3-tetrahydrofuryl) | O | F | H | H | H |
| CO(2-pyridyl) | O | F | H | H | H |
| CO(3-pyridyl) | O | F | H | H | H |

**[Table 93]**

| R² | W | | X² | X³ | X⁴ |
|---|---|---|---|---|---|
| CO(4-pyridyl) | O | F | H | H | H |
| CO(2-thienyl) | O | F | H | H | H |
| CO(3-thienyl) | O | F | H | H | H |
| CO(2-tetrahydrofurfuryl) | O | F | H | H | H |
| COCH₂(2-pyridyl) | O | F | H | H | H |
| COCH₂(2-thienyl) | O | F | H | H | H |
| CO₂Me | O | F | H | H | H |
| CO₂Et | O | F | H | H | H |
| CO₂n-Pr | O | F | H | H | H |
| CO₂i-Pr | O | F | H | H | H |
| CO₂n-Bu | O | F | H | H | H |
| CO₂s-Bu | O | F | H | H | H |
| CO₂-i-Bu | O | F | H | H | H |
| CO₂t-Bu | O | F | H | H | H |
| CO₂n-Pen | O | F | H | H | H |
| CO₂n-Hex | O | F | H | H | H |
| CO₂CH₂CH₂F | O | F | H | H | H |
| CO₂CH₂CHF₂ | O | F | H | H | H |
| CO₂CH₂CF₃ | O | F | H | H | H |
| CO₂CH=CH₂ | O | F | H | H | H |
| CO₂C≡CH | O | F | H | H | H |
| CO₂c-Pr | O | F | H | H | H |
| CO₂CH₂c-Pr | O | F | H | H | H |
| CO₂CH₂CH₂OMe | O | F | H | H | H |
| CO₂CH₂CH₂OCH₂CF₃ | O | F | H | H | H |
| CO₂CH₂CH₂OCH₂CH₂OMe | O | F | H | H | H |
| CO₂CH₂CH₂SMe | O | F | H | H | H |

**[Table 94]**

| R² | W | | X² | X³ | X⁴ |
|---|---|---|---|---|---|
| CO₂CH₂CH₂SCH₂CF₃ | O | F | H | H | H |
| CO₂Ph | O | F | H | H | H |
| CO₂(4-ClPh) | O | F | H | H | H |
| CO₂(4-MePh) | O | F | H | H | H |
| CO₂CH₂Ph | O | F | H | H | H |
| CO₂CH₂(4-ClPh) | O | F | H | H | H |
| CO₂CH₂(4-MePh) | O | F | H | H | H |
| CO₂(3-oxetanyl) | O | F | H | H | H |
| CO₂(2-tetrahydrofuryl) | O | F | H | H | H |
| CO₂(3-tetrahydrofuryl) | O | F | H | H | H |
| CO₂(2-pyr idyl) | O | F | H | H | H |
| CO₂(2-thienyl) | O | F | H | H | H |
| CO₂(2-tetrahydrofurfuryl) | O | F | H | H | H |
| CO₂CH₂(2-pyridyl) | O | F | H | H | H |
| CO₂CH₂(2-thienyl) | O | F | H | H | H |
| CO(SMe) | O | F | H | H | H |
| CO(SEt) | O | F | H | H | H |
| CO(SCF₃) | O | F | H | H | H |
| CO(SCH₂CF₃) | O | F | H | H | H |
| CONHEt | O | F | H | H | H |
| CONHCH₂CF₃ | O | F | H | H | H |
| CONEt₂ | O | F | H | H | H |
| SO₂Me | O | F | H | H | H |
| SO₂Et | O | F | H | H | H |
| SO₂n-Pr | O | F | H | H | H |
| SO₂i-Pr | O | F | H | H | H |
| SO₂n-Bu | O | F | H | H | H |

**[Table 95]**

| R² | W | ¹ | X² | X³ | X⁴ |
|---|---|---|---|---|---|
| SO₂i-Bu | O | F | H | H | H |
| SO₂CH₂Cl | O | F | H | H | H |
| SO₂OCl₃ | O | F | H | H | H |
| SO₂CHF₂ | O | F | H | H | H |
| SO₂CF₃ | O | F | H | H | H |
| SO₂CH₂CF₃ | O | F | H | H | H |
| SO₂CH=CH₂ | O | F | H | H | H |
| SO₂CH₂CH=CH₂ | O | F | H | H | H |
| SO₂CH₂C≡CH | O | F | H | H | H |
| SO₂c-Pr | O | F | H | H | H |
| SO₂c-Hex | O | F | H | H | H |
| SO₂CH₂c-Pr | O | F | H | H | H |
| SO₂CH₂CH₂OMe | O | F | H | H | H |
| SO₂Ph | O | F | H | H | H |
| SO₂(4-ClPh) | O | F | H | H | H |
| SO₂(4-MePh) | O | F | H | H | H |
| SO₂CH₂Ph | O | F | H | H | H |
| SO₂CH₂(4-ClPh) | O | F | H | H | H |
| SO₂CH₂(4-MePh) | O | F | H | H | H |
| SO₂NHMe | O | F | H | H | H |
| SO₂NMe₂ | O | F | H | H | H |
| H | O | F | H | H | F |
| CH₂CF₃ | O | F | H | H | F |
| CH₂CH=CH₂ | O | F | H | H | F |
| CH₂C≡CH | O | F | H | H | F |
| CH₂OMe | O | F | H | H | F |
| CH₂OCH₂CF₃ | O | F | H | H | F |

**[Table 96]**

| R² | W | | X² | X³ | X⁴ |
|---|---|---|---|---|---|
| CH₂OCH₂CH₂OMe | O | F | H | H | F |
| CH₂SMe | O | F | H | H | F |
| CH₂COMe | O | F | H | H | F |
| CH₂CH₂OPh | O | F | H | H | F |
| COMe | O | F | H | H | F |
| COEt | O | F | H | H | F |
| COn-Pr | O | F | H | H | F |
| COi-Pr | O | F | H | H | F |
| COn-Bu | O | F | H | H | F |
| COs-Bu | O | F | H | H | F |
| COi-Bu | O | F | H | H | F |
| COt-Bu | O | F | H | H | F |
| COn-Pen | O | F | H | H | F |
| COn-Hex | O | F | H | H | F |
| COCF₃ | O | F | H | H | F |
| COCH₂CF₃ | O | F | H | H | F |
| COCH=CH₂ | O | F | H | H | F |
| COC≡CH | O | F | H | H | F |
| COc-Pr | O | F | H | H | F |
| COc--Bu | O | F | H | H | F |
| COc-Pen | O | F | H | H | F |
| COc-Hex | O | F | H | H | F |
| COCH₂c-Pr | O | F | H | H | F |
| COCH₂OMe | O | F | H | H | F |
| COCH₂OCH₂CF₃ | O | F | H | H | F |
| COCH₂OCH₂CH₂CF₃ | O | F | H | H | F |
| COCH₂SMe | O | F | H | H | F |

**[Table 97]**

| R² | W | | X² | X³ | X⁴ |
|---|---|---|---|---|---|
| COCH₂SCH₂CF₃ | O | F | H | H | F |
| Bz | O | F | H | H | F |
| 4-ClBz | O | F | H | H | F |
| 4-MeBz | O | F | H | H | F |
| COCH₂Ph | O | F | H | H | F |
| COCH₂(4-ClPh) | O | F | H | H | F |
| COCH₂(4-MePh) | O | F | H | H | F |
| CO(2-tetrahydrofuryl) | O | F | H | H | F |
| CO(3-tetrahydrofuryl) | O | F | H | H | F |
| CO(2-pyridyl) | O | F | H | H | F |
| CO(3-pyridyl) | O | F | H | H | F |
| CO(4-pyr i dy l) | O | F | H | H | F |
| CO(2-thienyl) | O | F | H | H | F |
| CO(3-thienyl) | O | F | H | H | F |
| CO(2-tetrahydrofurfuryl) | O | F | H | H | F |
| COCH₂(2-pyridyl) | O | F | H | H | F |
| COCH₂(2-thienyl) | O | F | H | H | F |
| CO₂Me | O | F | H | H | F |
| CO₂Et | O | F | H | H | F |
| CO₂n-Pr | O | F | H | H | F |
| CO₂i-Pr | O | F | H | H | F |
| CO₂n-Bu | O | F | H | H | F |
| CO₂s-Bu | O | F | H | H | F |
| CO₂i-Bu | O | F | H | H | F |
| CO₂t-Bu | O | F | H | H | F |
| CO₂n-Pen | O | F | H | H | F |
| CO₂n-Hex | O | F | H | H | F |

**[Table 98]**

| R² | W | | X² | X³ | X⁴ |
|---|---|---|---|---|---|
| CO₂CH₂CH₂F | O | F | H | H | F |
| CO₂CH₂CHF₂ | O | F | H | H | F |
| CO₂CH₂CF₃ | O | F | H | H | F |
| CO₂CH=CH₂ | O | F | H | H | F |
| CO₂C≡CH | O | F | H | H | F |
| CO₂c-Pr | O | F | H | H | F |
| CO₂CH₂c-Pr | O | F | H | H | F |
| CO₂CH₂CH₂OMe | O | F | H | H | F |
| CO₂CH₂CH₂OCH₂CF₃ | O | F | H | H | F |
| CO₂CH₂CH₂OCH₂CH₂OMe | O | F | H | H | F |
| CO₂CH₂CH₂SMe | O | F | H | H | F |
| CO₂CH₂CH₂SCH₂CF₃ | O | F | H | H | F |
| CO₂Ph | O | F | H | H | F |
| CO₂(4-ClPh) | O | F | H | H | F |
| CO₂(4-MePh) | O | F | H | H | F |
| CO₂CH₂Ph | O | F | H | H | F |
| CO₂CH₂(4-ClPh) | O | F | H | H | F |
| CO₂CH₂(4-MePh) | O | F | H | H | F |
| CO₂(3-oxetanyl) | O | F | H | H | F |
| CO₂(2-tetrahydrofuryl) | O | F | H | H | F |
| CO₂(3-tetrahydrofuryl) | O | F | H | H | F |
| CO₂(2-pyr idyl) | O | F | H | H | F |
| CO₂(2-thienyl) | O | F | H | H | F |
| CO₂(2-tetrahydrofurfuryl) | O | F | H | H | F |
| CO₂CH₂(2-pyridyl) | O | F | H | H | F |
| CO₂CH₂(2-thienyl) | O | F | H | H | F |
| CO(SMe) | O | F | H | H | F |

**[Table 99]**

| R² | W | X¹ | X² | X³ | X⁴ |
|---|---|---|---|---|---|
| CO(SEt) | O | F | H | H | F |
| CO(SCF₃) | O | F | H | H | F |
| CO (SCH₂CF₃) | O | F | H | H | F |
| CONHEt | O | F | H | H | F |
| CONHCH₂CF₃ | O | F | H | H | F |
| CONEt₂ | O | F | H | H | F |
| SO₂Me | O | F | H | H | F |
| SO₂Et | O | F | H | H | F |
| SO₂n-Pr | O | F | H | H | F |
| SO₂i-Pr | O | F | H | H | F |
| SO₂n-Bu | O | F | H | H | F |
| SO₂i-Bu | O | F | H | H | F |
| SO₂CH₂CI | O | F | H | H | F |
| SO₂CCl₃ | O | F | H | H | F |
| SO₂CHF₂ | O | F | H | H | F |
| SO₂CF₃ | O | F | H | H | F |
| SO₂CH₂CF₃ | O | F | H | H | F |
| SO₂CH=CH₂ | O | F | H | H | F |
| SO₂CH₂CH=CH₂ | O | F | H | H | F |
| SO₂CH₂C≡CH | O | F | H | H | F |
| SO₂c-Pr | O | F | H | H | F |
| SO₂c-Hex | O | F | H | H | F |
| SO₂CH₂c-Pr | O | F | H | H | F |
| SO₂CH₂CH₂OMe | O | F | H | H | F |
| SO₂Ph | O | F | H | H | F |
| SO₂(4-ClPh) | O | F | H | H | F |
| SO₂(4-MePh) | O | F | H | H | F |

**[Table 100]**

| R² | W | ¹ | X² | X³ | X⁴ |
|---|---|---|---|---|---|
| SO₂CH₂Ph | O | F | H | H | F |
| SO₂CH₂(4-ClPh) | O | F | H | H | F |
| SO₂CH₂(4-MePh) | O | F | H | H | F |
| SO₂NHMe | O | F | H | H | F |
| SO₂NMe₂ | O | F | H | H | F |
| COc-Pr | O | CI | H | H | H |
| COc-Pr | O | Br | H | H | H |
| COc-Pr | O | I | H | H | H |
| COc-Pr | O | Me | H | H | H |
| COc-Pr | O | Et | H | H | H |
| COc-Pr | O | n-Pr | H | H | H |
| COc-Pr | O | i-Pr | H | H | H |
| COc-Pr | O | n-Bu | H | H | H |
| COc-Pr | O | s-Bu | H | H | H |
| COc-Pr | O | i -Bu | H | H | H |
| COc-Pr | O | t-Bu | H | H | H |
| COc-Pr | O | c-Pr | H | H | H |
| COc-Pr | O | c-Bu | H | H | H |
| COc-Pr | O | OMe | H | H | H |
| COc-Pr | O | OEt | H | H | H |
| COc-Pr | O | OCHF₂ | H | H | H |
| COc-Pr | O | OCF₃ | H | H | H |
| COc-Pr | O | OCH₂CH=CH₂ | H | H | H |
| COc-Pr | O | OCH₂C≡CH | H | H | H |
| COc-Pr | O | OCH₂c-Pr | H | H | H |
| COc-Pr | O | Bn | H | H | H |
| COc-Pr | O | 4-ClBn | H | H | H |

**[Table 101]**

| R² | W | X¹ | X² | X³ | X⁴ |
|---|---|---|---|---|---|
| COc-Pr | O | 4-MeBn | H | H | H |
| COc-Pr | O | 4-0MeBn | H | H | H |
| COc-Pr | O | OAc | H | H | H |
| COc-Pr | O | OBz | H | H | H |
| COc-Pr | O | O(4-ClBz) | H | H | H |
| COc-Pr | O | OCO₂Et | H | H | H |
| COc-Pr | O | OCO (SMe) | H | H | H |
| COc-Pr | O | OSO₂Me | H | H | H |
| COc-Pr | O | OSO₂CF₃ | H | H | H |
| COc-Pr | O | OSO₂Ph | H | H | H |
| COc-Pr | O | OPO (OEt)₂ | H | H | H |
| COc-Pr | O | OCH₂OMe | H | H | H |
| COc-Pr | O | SMe | H | H | H |
| COc-Pr | 0 | SEt | H | H | H |
| COc-Pr | O | SCF₃ | H | H | H |
| COc-Pr | O | SO₂Me | H | H | H |
| COc-Pr | 0 | SO₂CF₃ | H | H | H |
| COc-Pr | O | Ph | H | H | H |
| COc-Pr | O | 4-ClPh | H | H | H |
| COc-Pr | O | 4-MePh | H | H | H |
| COc-Pr | O | NMe₂ | H | H | H |
| COc-Pr | O | NHAc | H | H | H |
| COc-Pr | O | NAc₂ | H | H | H |
| COc-Pr | O | NHSO₂CF₃ | H | H | H |
| COc-Pr | O | N-morpholinyl | H | H | H |
| COc-Pr | O | CO₂Me | H | H | H |
| COc-Pr | O | CO₂Et | H | H | H |

**[Table 102]**

| R² | W | X¹ | X² | X³ | X⁴ |
|---|---|---|---|---|---|
| COc-Pr | O | OH | H | H | H |
| COc-Pr | O | CN | H | H | H |
| COc-Pr | O | NO₂ | H | H | H |
| COc-Pr | O | H | F | H | H |
| COc-Pr | O | H | Cl | H | H |
| COc-Pr | O | H | Br | H | H |
| COc-Pr | O | H | I | H | H |
| COc-Pr | O | H | Me | H | H |
| COc-Pr | O | H | c-Pr | H | H |
| COc-Pr | O | H | OMe | H | H |
| COc-Pr | O | H | OCF₃ | H | H |
| COc-Pr | O | H | OCH₂C≡CH | H | H |
| COc-Pr | O | H | Bn | H | H |
| COc-Pr | O | H | OAc | H | H |
| COc-Pr | O | H | OSO₂Me | H | H |
| COc-Pr | O | H | SMe | H | H |
| COc-Pr | O | H | SO₂Me | H | H |
| COc-Pr | O | H | Ph | H | H |
| COc-Pr | O | H | NMe₂ | H | H |
| COc-Pr | O | H | CN | H | H |
| COc-Pr | O | H | NO₂ | H | H |
| COc-Pr | O | H | H | F | H |
| COc-Pr | O | H | H | C l | H |
| COc-Pr | O | H | H | Br | H |
| COc-Pr | O | H | H | I | H |
| COc-Pr | O | H | H | Me | H |
| COc-Pr | O | H | H | c-Pr | H |

**[Table 103]**

| R² | W | X¹ | X² | X³ | X⁴ |
|---|---|---|---|---|---|
| COc-Pr | O | H | H | OMe | H |
| COc-Pr | O | H | H | OCF₃ | H |
| COc-Pr | O | H | H | OCH₂C≡CH | H |
| COc-Pr | O | H | H | Bn | H |
| COc-Pr | O | H | H | OAc | H |
| COc-Pr | O | H | H | OSO₂Me | H |
| COc-Pr | O | H | H | SMe | H |
| COc-Pr | O | H | H | SO₂Me | H |
| COc-Pr | O | H | H | Ph | H |
| COc-Pr | O | H | H | NMe₂ | H |
| COc-Pr | O | H | H | CN | H |
| COc-Pr | O | H | H | NO₂ | H |
| COc-Pr | O | H | H | H | F |
| COc-Pr | O | H | H | H | Cl |
| COc-Pr | O | H | H | H | Br |
| COc-Pr | O | H | H | H | I |
| COc-Pr | O | H | H | H | Me |
| COc-Pr | O | H | H | H | c-Pr |
| COc-Pr | O | H | H | H | OMe |
| COc-Pr | O | H | H | H | OCF₃ |
| COc-Pr | O | H | H | H | OCH₂C≡CH |
| COc-Pr | O | H | H | H | Bn |
| COc-Pr | O | H | H | H | OAc |
| COc-Pr | O | H | H | H | OSO₂Me |
| COc-Pr | O | H | H | H | SMe |
| COc-Pr | O | H | H | H | SO₂Me |
| COc-Pr | O | H | H | H | Ph |

**[Table 104]**

| R² | W | X¹ | X² | X³ | X⁴ |
|---|---|---|---|---|---|
| COc-Pr | O | H | H | H | NMe₂ |
| COc-Pr | O | H | H | H | CN |
| COc-Pr | O | H | H | H | NO₂ |
| COc-Pr | O | F | F | H | H |
| COc-Pr | O | F | H | F | H |
| COc-Pr | O | F | Cl | H | H |
| COc-Pr | O | F | H | Cl | H |
| COc-Pr | O | F | H | H | Cl |
| COc-Pr | O | F | H | H | Br |
| COc-Pr | O | F | H | H | I |
| COc-Pr | O | F | H | H | Me |
| COc-Pr | O | F | H | H | c-Pr |
| COc-Pr | O | F | H | H | OMe |
| COc-Pr | O | F | H | H | OCF₃ |
| COc-Pr | O | F | H | H | OCH₂C≡CH |
| COc-Pr | O | F | H | H | OSO₂Me |
| COc-Pr | O | F | H | H | SMe |
| COc-Pr | O | F | H | H | SO₂Me |
| COc-Pr | O | F | H | H | NMe₂ |
| COc-Pr | O | F | H | H | CN |
| COc-Pr | O | F | H | H | NO₂ |
| COc-Pr | O | F | F | F | H |
| COc-Pr | O | F | F | H | F |
| COc-Pr | O | F | H | F | F |
| COc-Pr | O | F | F | F | F |
| COc-Pr | O | Cl | F | H | H |
| COc-Pr | O | Cl | H | F | H |

**[Table 105]**

| R² | W | X¹ | X² | X³ | X⁴ |
|---|---|---|---|---|---|
| COc-Pr | O | Cl | H | H | F |
| COc-Pr | O | Cl | Cl | H | H |
| COc-Pr | O | Cl | H | Cl | H |
| COc-Pr | O | Cl | H | H | Cl |
| COc-Pr | O | Cl | H | H | Br |
| COc-Pr | O | Cl | H | H | I |
| COc-Pr | O | Cl | H | H | Me |
| COc-Pr | O | Cl | H | H | c-Pr |
| COc-Pr | O | Cl | H | H | OMe |
| COc-Pr | O | Cl | H | H | OCF₃ |
| COc-Pr | O | Cl | H | H | OCH₂C≡CH |
| COc-Pr | O | Cl | H | H | OSO₂Me |
| COc-Pr | O | Cl | H | H | SMe |
| COc-Pr | O | Cl | H | H | SO₂Me |
| COc-Pr | O | Cl | H | H | NMe₂ |
| COc-Pr | O | Cl | H | H | CN |
| COc-Pr | O | Cl | H | H | NO₂ |
| COc-Pr | O | Br | H | H | F |
| COc-Pr | O | Br | H | H | Cl |
| COc-Pr | O | Br | H | H | Me |
| COc-Pr | O | Br | H | H | OMe |
| COc-Pr | O | Me | H | H | F |
| COc-Pr | O | Me | H | H | Cl |
| COc-Pr | O | Me | H | H | Me |
| COc-Pr | O | Me | H | H | OMe |
| COc-Pr | O | OMe | H | H | F |
| COc-Pr | O | OMe | H | H | Cl |

**[Table 106]**

| R² | W | X¹ | X² | X³ | X⁴ |
|---|---|---|---|---|---|
| COc-Pr | O | OMe | H | H | Me |
| COc-Pr | O | OMe | H | H | OMe |
| COc-Pr | O | H | F | F | H |
| COc-Pr | O | H | F | Cl | H |
| COc-Pr | O | H | F | H | F |
| COc-Pr | O | H | F | H | Cl |
| COc-Pr | O | H | Cl | Cl | H |
| COc-Pr | O | H | Cl | F | H |
| COc-Pr | O | H | Cl | H | F |
| COc-Pr | O | H | Cl | H | Cl |
| COc-Pr | O | H | H | F | F |
| COc-Pr | O | H | H | F | Cl |
| COc-Pr | O | H | H | Cl | Cl |
| COc-Pr | O | F | F | F | F |
| COc-Pr | O | Cl | Cl | Cl | Cl |
| COc-Pr | O | -CH=CH-CH=CH - | | H | H |
| COc-Pr | O | -CH=N-CH=CH- | | H | H |
| COc-Pr | O | -S-CH=N- | | H | H |
| COc-Pr | O | -N=CH-S- | | H | H |
| COc-Pr | O | H | -CH=CH-CH=CH- | | H |
| COc-Pr | O | H | -CH=N-CH=CH- | | H |
| COc-Pr | O | H | -S-CH=N- | | H |
| COc-Pr | O | H | -N=CH-S- | | H |
| COc-Pr | O | H | H | -CH=CH-CH=CH- | |
| COc-Pr | O | H | H | -CH=N-CH=CH- | |
| COc-Pr | O | H | H | -S-CH=N- | |
| COc-Pr | O | H | H | -N=CH-S- | |

**[Table 107]**

| R² | W | X¹ | X² | X³ | X⁴ |
|---|---|---|---|---|---|
| CO₂Et | O | Cl | H | H | H |
| CO₂Et | O | Br | H | H | H |
| CO₂Et | O | I | H | H | H |
| CO₂Et | O | Me | H | H | H |
| CO₂Et | O | Et | H | H | H |
| CO₂Et | O | n-Pr | H | H | H |
| CO₂Et | O | i-Pr | H | H | H |
| CO₂Et | O | n-Bu | H | H | H |
| CO₂Et | O | s-Bu | H | H | H |
| CO₂Et | O | i-Bu | H | H | H |
| CO₂Et | O | t-Bu | H | H | H |
| CO₂Et | O | c-Pr | H | H | H |
| CO₂Et | O | c-Bu | H | H | H |
| CO₂Et | O | OMe | H | H | H |
| CO₂Et | O | OEt | H | H | H |
| CO₂Et | O | OCHF₂ | H | H | H |
| CO₂Et | O | OCF₃ | H | H | H |
| CO₂Et | O | OCH₂CH=CH₂ | H | H | H |
| CO₂Et | O | OCH₂C≡CH | H | H | H |
| CO₂Et | O | OCH₂c-Pr | H | H | H |
| CO₂Et | O | Bn | H | H | H |
| CO₂Et | O | 4-Cl Bn | H | H | H |
| CO₂Et | O | 4-MeBn | H | H | H |
| CO₂Et | O | 4-OMeBn | H | H | H |
| CO₂Et | O | OAc | H | H | H |
| CO₂Et | O | OBz | H | H | H |
| CO₂Et | O | O(4-Cl Bz) | H | H | H |

**[Table 108]**

| R² | W | X¹ | X² | X³ | X⁴ |
|---|---|---|---|---|---|
| CO₂Et | O | OCO₂Et | H | H | H |
| CO₂Et | O | OCO (SMe) | H | H | H |
| CO₂Et | O | OSO₂Me | H | H | H |
| CO₂Et | O | OSO₂CF₃ | H | H | H |
| CO₂Et | O | OSO₂Ph | H | H | H |
| CO₂Et | O | OPO(OEt)₂ | H | H | H |
| CO₂Et | O | OCH₂OMe | H | H | H |
| CO₂Et | O | SMe | H | H | H |
| CO₂Et | O | SEt | H | H | H |
| CO₂Et | O | SCF₃ | H | H | H |
| CO₂Et | O | SO₂Me | H | H | H |
| CO₂Et | O | SO₂CF₃ | H | H | H |
| CO₂Et | O | Ph | H | H | H |
| CO₂Et | O | 4-ClPh | H | H | H |
| CO₂Et | O | 4-MePh | H | H | H |
| CO₂Et | O | NMe₂ | H | H | H |
| CO₂Et | O | NHAc | H | H | H |
| CO₂Et | O | NAc₂ | H | H | H |
| CO₂Et | O | NHSO₂CF₃ | H | H | H |
| CO₂Et | O | N-morpholinyl | H | H | H |
| CO₂Et | O | CO₂Me | H | H | H |
| CO₂Et | O | CO₂Et | H | H | H |
| CO₂Et | O | OH | H | H | H |
| CO₂Et | O | CN | H | H | H |
| CO₂Et | O | NO₂ | H | H | H |
| CO₂Et | O | H | F | H | H |
| CO₂Et | O | H | Cl | H | H |

**[Table 109]**

| R² | W | X¹ | X² | X³ | X⁴ |
|---|---|---|---|---|---|
| CO₂Et | O | H | Br | H | H |
| CO₂Et | O | H | I | H | H |
| CO₂Et | O | H | Me | H | H |
| CO₂Et | O | H | c-Pr | H | H |
| CO₂Et | O | H | OMe | H | H |
| CO₂Et | O | H | OCF₃ | H | H |
| CO₂Et | O | H | OCH₂C≡ CH | H | H |
| CO₂Et | O | H | Bn | H | H |
| CO₂Et | O | H | OAc | H | H |
| CO₂Et | O | H | OSO₂Me | H | H |
| CO₂Et | O | H | SMe | H | H |
| CO₂Et | O | H | SO₂Me | H | H |
| CO₂Et | O | H | Ph | H | H |
| CO₂Et | O | H | NMe₂ | H | H |
| CO₂Et | O | H | CN | H | H |
| CO₂Et | O | H | NO₂ | H | H |
| CO₂Et | O | H | H | F | H |
| CO₂Et | O | H | H | Cl | H |
| CO₂Et | O | H | H | Br | H |
| CO₂Et | O | H | H | I | H |
| CO₂Et | O | H | H | Me | H |
| CO₂Et | O | H | H | c-Pr | H |
| CO₂Et | O | H | H | OMe | H |
| CO₂Et | O | H | H | OCF₃ | H |
| CO₂Et | O | H | H | OCH₂C≡CH | H |
| CO₂Et | O | H | H | Bn | H |
| CO₂Et | O | H | H | OAc | H |

**[Table 110]**

| R² | W | X¹ | X² | X³ | X⁴ |
|---|---|---|---|---|---|
| CO₂Et | O | H | H | OSO₂Me | H |
| CO₂Et | O | H | H | SMe | H |
| CO₂Et | O | H | H | SO₂Me | H |
| CO₂Et | O | H | H | Ph | H |
| CO₂Et | O | H | H | NMe₂ | H |
| CO₂Et | O | H | H | CN | H |
| CO₂Et | O | H | H | NO₂ | H |
| CO₂Et | O | H | H | H | F |
| CO₂Et | O | H | H | H | Br |
| CO₂Et | O | H | H | H | Cl |
| CO₂Et | O | H | H | H | I |
| CO₂Et | O | H | H | H | Me |
| CO₂Et | O | H | H | H | c-Pr |
| CO₂Et | O | H | H | H | OMe |
| CO₂Et | O | H | H | H | OCF₃ |
| CO₂Et | O | H | H | H | OCH₂C≡CH |
| CO₂Et | O | H | H | H | Bn |
| CO₂Et | O | H | H | H | OAc |
| CO₂Et | O | H | H | H | OSO₂Me |
| CO₂Et | O | H | H | H | SMe |
| CO₂Et | O | H | H | H | SO₂Me |
| CO₂Et | O | H | H | H | Ph |
| CO₂Et | O | H | H | H | NMe₂ |
| CO₂Et | O | H | H | H | CN |
| CO₂Et | O | H | H | H | NO₂ |
| CO₂Et | O | F | F | H | H |
| CO₂Et | O | F | H | F | H |

**[Table 111]**

| R² | W | X¹ | X² | X³ | X⁴ |
|---|---|---|---|---|---|
| CO₂Et | O | F | Cl | H | H |
| CO₂Et | O | F | H | Cl | H |
| CO₂Et | O | F | H | H | Cl |
| CO₂Et | O | F | H | H | Br |
| CO₂Et | O | F | H | H | I |
| CO₂Et | O | F | H | H | Me |
| CO₂Et | O | F | H | H | c-Pr |
| CO₂Et | O | F | H | H | OMe |
| CO₂Et | O | F | H | H | OCF₃ |
| CO₂Et | O | F | H | H | OCH₂C≡CH |
| CO₂Et | O | F | H | H | OSO₂Me |
| CO₂Et | O | F | H | H | SMe |
| CO₂Et | O | F | H | H | SO₂Me |
| CO₂Et | O | F | H | H | NMe₂ |
| CO₂Et | O | F | H | H | CN |
| CO₂Et | O | F | H | H | NO₂ |
| CO₂Et | O | F | F | F | H |
| CO₂Et | O | F | F | H | F |
| CO₂Et | O | F | H | F | F |
| CO₂Et | O | F | F | F | F |
| CO₂Et | O | Cl | F | H | H |
| CO₂Et | O | Cl | H | F | H |
| CO₂Et | O | Cl | H | H | F |
| CO₂Et | O | Cl | Cl | H | H |
| CO₂Et | O | Cl | H | Cl | H |
| CO₂Et | O | Cl | H | H | Cl |
| CO₂Et | O | Cl | H | H | Br |

**[Table 112]**

| R² | W | X¹ | X² | X³ | X⁴ |
|---|---|---|---|---|---|
| CO₂Et | O | Cl | H | H | I |
| CO₂Et | O | Cl | H | H | Me |
| CO₂Et | O | Cl | H | H | c-Pr |
| CO₂Et | O | Cl | H | H | OMe |
| CO₂Et | O | Cl | H | H | OCF₃ |
| CO₂Et | O | Cl | H | H | OCH₂C≡CH |
| CO₂Et | O | Cl | H | H | OSO₂Me |
| CO₂Et | O | Cl | H | H | SMe |
| CO₂Et | O | Cl | H | H | SO₂Me |
| CO₂Et | O | Cl | H | H | NMe₂ |
| CO₂Et | O | Cl | H | H | CN |
| CO₂Et | O | Cl | H | H | NO₂ |
| CO₂Et | O | Br | H | H | F |
| CO₂Et | O | Br | H | H | Cl |
| CO₂Et | O | Br | H | H | Me |
| CO₂Et | O | Br | H | H | OMe |
| CO₂Et | O | Me | H | H | F |
| CO₂Et | O | Me | H | H | CI |
| CO₂Et | O | Me | H | H | Me |
| CO₂Et | O | Me | H | H | OMe |
| CO₂Et | O | OMe | H | H | F |
| CO₂Et | O | OMe | H | H | Cl |
| CO₂Et | O | OMe | H | H | Me |
| CO₂Et | O | OMe | H | H | OMe |
| CO₂Et | O | H | F | F | H |
| CO₂Et | O | H | F | Cl | H |
| CO₂Et | O | H | F | H | F |

**[Table 113]**

| R² | W | X¹ | X² | X³ | X⁴ |
|---|---|---|---|---|---|
| CO₂Et | O | H | F | H | Cl |
| CO₂Et | O | H | Cl | Cl | H |
| CO₂Et | O | H | Cl | F | H |
| CO₂Et | O | H | Cl | H | F |
| CO₂Et | O | H | Cl | H | Cl |
| CO₂Et | O | H | H | F | F |
| CO₂Et | O | H | H | F | Cl |
| CO₂Et | O | H | H | Cl | Cl |
| CO₂Et | O | F | F | F | F |
| CO₂Et | O | Cl | Cl | Cl | Cl |
| CO₂Et | O | -CH=CH-CH=CH- | | H | H |
| CO₂Et | O | -CH=N-CH=CH- | | H | H |
| CO₂Et | O | -S-CH=N-H | | H | H |
| CO₂Et | O | -N=CH-S-H | | H | H |
| CO₂Et | O | H | -CH=CH-CH=CH- | | H |
| CO₂Et | O | H | -CH=N-CH=CH- | | H |
| CO₂Et | O | H | -S-CH=N- H | | H |
| CO₂Et | O | H | -N=CH-S- H | | H |
| CO₂Et | O | H | H | -CH=CH-CH=CH- | |
| CO₂Et | O | H | H | -CH=N-CH=CH- | |
| CO₂Et | O | H | H | -S-CH=N- | |
| CO₂Et | O | H | H | -N=CH-S- | |
| CO₂CH₂CH₂F | O | Cl | H | H | H |
| CO₂CH₂CH₂F | O | Br | H | H | H |
| CO₂CH₂CH₂F | O | I | H | H | H |
| CO₂CH₂CH₂F | O | Me | H | H | H |
| CO₂CH₂CH₂F | O | Et | H | H | H |

**[Table 114]**

| R² | W | X¹ | X² | X³ | X⁴ |
|---|---|---|---|---|---|
| CO₂CH₂CH₂F | O | n-Pr | H | H | H |
| CO₂H₂CH₂F | O | i-Pr | H | H | H |
| CO₂CH₂CH₂F | O | n-Bu | H | H | H |
| CO₂H₂CH₂F | O | s-Bu | H | H | H |
| CO₂H₂CH₂F | O | i-Bu | H | H | H |
| CO₂H₂CH₂F | O | t-Bu | H | H | H |
| CO₂CH₂CH₂F | O | c-Pr | H | H | H |
| CO₂CH₂CH₂F | O | c-Bu | H | H | H |
| CO₂CH₂CH₂F | O | OMe | H | H | H |
| CO₂CH₂CH₂F | O | OEt | H | H | H |
| COCH₂CH₂F | O | OCHF₂ | H | H | H |
| COCN₂CH₂F | O | OCF₃ | H | H | H |
| CO₂CH₂CH₂F | O | OCH₂CH=CH₂ | H | H | H |
| CO₂CH₂CH₂F | O | OCH₂C≡CH | H | H | H |
| CO₂CH₂CH₂F | O | OCH₂c-Pr | H | H | H |
| CO₂CH₂CH₂F | O | Bn | H | H | H |
| CO₂CH₂CH₂F | O | 4-ClBn | H | H | H |
| COCH₂CH₂F | O | 4-MeBn | H | H | H |
| CO₂CH₂CH₂F | O | 4-OMeBn | H | H | H |
| CO₂CH₂CH₂F | O | OAc | H | H | H |
| CO₂CH₂CH₂F | O | OBz | H | H | H |
| CO₂CH₂CH₂F | O | O(4-ClBz) | H | H | H |
| CO₂CH₂CH₂F | O | OCO₂Et | H | H | H |
| CO₂CH₂CH₂F | O | OCO (SMe) | H | H | H |
| CO₂CH₂CH₂F | O | OSO₂Me | H | H | H |
| CO₂CH₂CH₂F | O | OSO₂CF₃ | H | H | H |
| CO₂CH₂CH₂F | O | OSO₂Ph | H | H | H |

**[Table 115]**

| R² | W | X¹ | X² | X³ | X⁴ |
|---|---|---|---|---|---|
| CO₂CH₂CH₂F | O | OPO(OEt)₂ | H | H | H |
| CO₂CH₂CH₂F | O | OCH₂OMe | H | H | H |
| CO₂CH₂CH₂F | O | SMe | H | H | H |
| CO₂CH₂CH₂F | O | SEt | H | H | H |
| CO₂CH₂CH₂F | O | SCF₃ | H | H | H |
| CO₂CH₂CH₂F | O | SO₂Me | H | H | H |
| CO₂CH₂CH₂F | O | SO₂CF₃ | H | H | H |
| CO₂CH₂CH₂F | O | Ph | H | H | H |
| CO₂CH₂CH₂F | O | 4-ClPh | H | H | H |
| CO₂CH₂CH₂F | O | 4-MePh | H | H | H |
| CO₂CH₂CH₂F | O | NMe₂ | H | H | H |
| CO₂CH₂CH₂F | O | NHAc | H | H | H |
| CO₂CH₂CH₂F | O | NAc₂ | H | H | H |
| CO₂CH₂CH₂F | O | NHSO₂CF₃ | H | H | H |
| CO₂CH₂CH₂F | O | N-morpholinyl | H | H | H |
| CO₂CH₂CH₂F | O | CO₂Me | H | H | H |
| CO₂CH₂CH₂F | O | CO₂Et | H | H | H |
| CO₂CH₂CH₂F | O | OH | H | H | H |
| CO₂CH₂CH₂F | O | CN | H | H | H |
| CO₂CH₂CH₂F | O | NO₂ | H | H | H |
| CO₂CH₂CH₂ F | O | H | F | H | H |
| CO₂CH₂CH₂F | O | H | Cl | H | H |
| CO₂CH₂CH₂F | O | H | Br | H | H |
| CO₂CH₂CH₂F | O | H | I | H | H |
| CO₂CH₂CH₂F | O | H | Me | H | H |
| CO₂CH₂CH₂F | O | H | c-Pr | H | H |
| CO₂CH₂CH₂F | O | H | OMe | H | H |

**[Table 116]**

| R² | W | X¹ | X² | X³ | X⁴ |
|---|---|---|---|---|---|
| COCH₂CH₂F | O | H | OCF₃ | H | H |
| CO₂CH₂CH₂F | O | H | OCH₂C≡CH | H | H |
| CO₂CH₂CH₂F | O | H | Bn | H | H |
| CO₂CH₂CH₂F | O | H | OAc | H | H |
| CO₂CH₂CH₂F | O | H | OSO₂Me | H | H |
| CO₂CH₂CH₂F | O | H | SMe | H | H |
| COCH₂CH₂F | O | H | SO₂Me | H | H |
| CO₂CH₂CH₂F | O | H | Ph | H | H |
| CO₂CH₂CH₂F | O | H | NMe₂ | H | H |
| CO₂H₂CH₂F | O | H | CN | H | H |
| CO₂CH₂CH₂F | O | H | NO₂ | H | H |
| CO₂CH₂CH₂F | O | H | H | F | H |
| CO₂CH₂CH₂F | O | H | H | Cl | H |
| COCH₂CH₂F | O | H | H | Br | H |
| CO₂CH₂CH₂F | O | H | H | I | H |
| COCH₂CH₂F | O | H | H | Me | H |
| CO₂CH₂CH₂F | O | H | H | c-Pr | H |
| CO₂CH₂CH₂F | O | H | H | OMe | H |
| CO₂CH₂CH₂F | O | H | H | OCF₃ | H |
| CO₂CH₂CH₂F | O | H | H | OCH₂C≡CH | H |
| COCH₂CH₂F | O | H | H | Bn | H |
| CO₂CH₂CH₂F | O | H | H | OAc | H |
| CO₂CH₂CH₂F | O | H | H | OSO₂Me | H |
| CO₂CH₂CH₂F | O | H | H | SMe | H |
| CO₂CH₂CH₂F | O | H | H | SO₂Me | H |
| COCH₂CH₂F | O | H | H | Ph | H |
| CO₂CH₂CH₂F | O | H | H | NMe₂ | H |

**[Table 117]**

| R² | W | X¹ | X² | X³ | X⁴ |
|---|---|---|---|---|---|
| CO₂CH₂CH₂F | O | H | H | CN | H |
| CO₂CH₂CH₂F | O | H | H | NO₂ | H |
| CO₂CH₂CH₂F | O | H | H | H | F |
| CO₂CH₂CH₂F | O | H | H | H | Cl |
| CO₂CH₂CH₂F | O | H | H | H | Br |
| CO₂CH₂CH₂F | O | H | H | H | I |
| CO₂CH₂CH₂F | O | H | H | H | Me |
| CO₂CH₂CH₂F | O | H | H | H | c-Pr |
| CO₂CH₂CH₂F | O | H | H | H | OMe |
| CO₂CH₂CH₂F | O | H | H | H | OCF₃ |
| CO₂CH₂CH₂F | O | H | H | H | OCH₂C≡CH |
| CO₂CH₂CH₂F | O | H | H | H | Bn |
| CO₂CH₂CH₂F | O | H | H | H | OAc |
| CO₂CH₂CH₂F | O | H | H | H | OSO₂Me |
| CO₂CH₂CH₂F | O | H | H | H | SMe |
| CO₂CH₂CH₂F | O | H | H | H | S0₂Me |
| CO₂CH₂CH₂F | O | H | H | H | Ph |
| CO₂CH₂CH₂F | O | H | H | H | NMe₂ |
| CO₂CH₂CH₂F | O | H | H | H | CN |
| CO₂CH₂CH₂F | O | H | H | H | NO₂ |
| CO₂CH₂CH₂F | O | F | F | H | H |
| CO₂CH₂CH₂F | O | F | H | F | H |
| CO₂CH₂CH₂F | O | F | Cl | H | H |
| CO₂CH₂CH₂F | O | F | H | Cl | H |
| CO₂CH₂CH₂F | O | F | H | H | Cl |
| CO₂CH₂CH₂F | O | F | H | H | Br |
| CO₂CH₂CH₂F | O | F | H | H | I |

**[Table 118]**

| R² | W | X¹ | X² | X³ | X⁴ |
|---|---|---|---|---|---|
| CO₂CH₂CH₂F | O | F | H | H | Me |
| CO₂CH₂CH₂F | O | F | H | H | c-Pr |
| CO₂CH₂CH₂F | O | F | H | H | OMe |
| CO₂CH₂CH₂F | O | F | H | H | OCF₃ |
| CO₂CH₂CH₂F | O | F | H | H | OCH₂C≡CH |
| CO₂CH₂CH₂F | O | F | H | H | OSO₂Me |
| CO₂CH₂CH₂F | O | F | H | H | SMe |
| CO₂CH₂CH₂F | O | F | H | H | SO₂Me |
| CO₂CH₂CH₂F | O | F | H | H | NMe₂ |
| CO₂CH₂CH₂F | O | F | H | H | CN |
| CO₂CH₂CH₂F | O | F | H | H | NO₂ |
| CO₂CH₂CH₂F | O | F | F | F | H |
| CO₂CH₂CH₂F | O | F | F | H | F |
| CO₂CH₂CH₂F | O | F | H | F | F |
| CO₂CH₂CH₂F | O | F | F | F | F |
| CO₂CH₂CH₂F | O | Cl | F | H | H |
| CO₂CH₂CH₂F | O | Cl | H | F | H |
| CO₂CH₂CH₂F | O | Cl | H | H | F |
| CO₂CH₂CH₂F | O | Cl | Cl | H | H |
| CO₂CH₂CH₂F | O | Cl | H | Cl | H |
| CO₂CH₂CH₂F | O | Cl | H | H | Cl |
| CO₂CH₂CH₂F | O | Cl | H | H | Br |
| CO₂CH₂CH₂F | O | Cl | H | H | I |
| CO₂CH₂CH₂F | O | Cl | H | H | Me |
| CO₂CH₂CH₂F | O | Cl | H | H | c-Pr |
| CO₂CH₂CH₂F | O | Cl | H | H | OMe |
| CO₂CH₂CH₂F | O | Cl | H | H | OCF₃ |

**[Table 119]**

| R² | W | X¹ | X² | X³ | X⁴ |
|---|---|---|---|---|---|
| CO₂CH₂CH₂F | O | Cl | H | H | OCH₂C≡CH |
| CO₂CH₂CH₂F | O | Cl | H | H | OSO₂Me |
| CO₂CH₂CH₂F | O | Cl | H | H | SMe |
| CO₂CH₂CH₂F | O | Cl | H | H | SO₂Me |
| CO₂CH₂CH₂F | O | Cl | H | H | NMe₂ |
| CO₂CH₂CH₂F | O | Cl | H | H | CN |
| CO₂CH₂CH₂F | O | Cl | H | H | NO₂ |
| CO₂CH₂CH₂F | O | Br | H | H | F |
| CO₂CH₂CH₂F | O | Br | H | H | Cl |
| CO₂CH₂CH₂F | O | Br | H | H | Me |
| CO₂CH₂CH₂F | O | Br | H | H | OMe |
| CO₂CH₂CH₂F | O | Me | H | H | F |
| CO₂CH₂CH₂F | O | Me | H | H | Cl |
| CO₂CH₂CH₂F | O | Me | H | H | Me |
| CO₂CH₂CH₂F | O | Me | H | H | OMe |
| CO₂CH₂CH₂F | O | OMe | H | H | F |
| CO₂CH₂CH₂F | O | OMe | H | H | Cl |
| CO₂CH₂CH₂F | O | OMe | H | H | Me |
| CO₂CH₂CH₂F | O | OMe | H | H | OMe |
| CO₂CH₂CH₂F | O | H | F | F | H |
| CO₂CH₂CH₂F | O | H | F | Cl | H |
| CO₂CH₂CH₂F | O | H | F | H | F |
| CO₂CH₂CH₂F | O | H | F | H | Cl |
| CO₂CH₂CH₂F | O | H | Cl | Cl | H |
| CO₂CH₂CH₂F | O | H | Cl | F | H |
| CO₂CH₂CH₂F | O | H | Cl | H | F |
| CO₂CH₂CH₂F | O | H | Cl | H | Cl |

**[Table 120]**

| R² | W | X¹ | X² | X³ | X⁴ |
|---|---|---|---|---|---|
| CO₂CH₂CH₂F | O | H | H | F | F |
| CO₂CH₂CH₂F | O | H | H | F | Cl |
| CO₂CH₂CH₂F | O | H | H | Cl | Cl |
| CO₂CH₂CH₂F | O | F | F | F | F |
| CO₂CH₂CH₂F | O | Cl | Cl | Cl | Cl |
| CO₂CH₂CH₂F | O | -CH=CH-CH=CH- | | H | H |
| CO₂CH₂CH₂F | O | -CH=N-CH=CH- | | H | H |
| CO₂CH₂CH₂F | O | -S-CH=N | | H | H |
| CO₂CH₂CH₂F | O | -N=CH-S- | | H | H |
| CO₂CH₂CH₂F | O | H | -CH=CH-CH=CH- | | H |
| CO₂CH₂CH₂F | O | H | -CH=N-CH=CH- | | H |
| CO₂CH₂CH₂F | O | H | -S-CH=N- | | H |
| CO₂CH₂CH₂F | O | H | -N=CH-S- | | H |
| CO₂CH₂CH₂F | O | H | H | -CH=CH-CH=CH- | |
| CO₂CH₂CH₂F | O | H | H | -CH=N-CH=CH- | |
| CO₂CH₂CH₂F | O | H | H | -S-CH=N- | |
| CO₂CH₂CH₂F | O | H | H | -N=CH-S- | |
| CO₂CH₂CHF₂ | O | Cl | H | H | H |
| CO₂CH₂CHF₂ | O | Br | H | H | H |
| CO₂CH₂CHF₂ | O | I | H | H | H |
| CO₂CH₂CHF₂ | O | Me | H | H | H |
| CO₂CH₂CHF₂ | O | Et | H | H | H |
| CO₂CH₂CHF₂ | O | n-Pr | H | H | H |
| CO₂CH₂CHF₂ | O | i-Pr | H | H | H |
| CO₂CH₂CHF₂ | O | n-Bu | H | H | H |
| CO₂CH₂CHF₂ | O | s-Bu | H | H | H |
| CO₂CH₂CHF₂ | O | i-Bu | H | H | H |

**[Table 121]**

| R² | W | X¹ | X² | X³ | X⁴ |
|---|---|---|---|---|---|
| CO₂CH₂CHF₂ | O | t-Bu | H | H | H |
| CO₂CH₂CHF₂ | O | c-Pr | H | H | H |
| CO₂CH₂CHF₂ | O | c-Bu | H | H | H |
| CO₂CH₂CHF₂ | O | OMe | H | H | H |
| CO₂CH₂CHF₂ | O | OEt | H | H | H |
| CO₂CH₂CHF₂ | O | OCHF₂ | H | H | H |
| CO₂CH₂CHF₂ | O | OCF₃ | H | H | H |
| CO₂CH₂CHF₂ | O | OCH₂CH=CH₂ | H | H | H |
| CO₂CH₂CHF₂ | O | OCH₂C≡CH | H | H | H |
| CO₂CH₂CHF₂ | O | OCH₂c-Pr | H | H | H |
| CO₂CH₂CHF₂ | O | Bn | H | H | H |
| CO₂CH₂CHF₂ | O | 4-ClBn | H | H | H |
| CO₂CH₂CHF₂ | O | 4-MeBn | H | H | H |
| CO₂CH₂CHF₂ | O | 4-OMeBn | H | H | H |
| CO₂CH₂CHF₂ | O | OAc | H | H | H |
| CO₂CH₂CHF₂ | O | OBz | H | H | H |
| CO₂CH₂CHF₂ | O | O(4-ClBz) | H | H | H |
| CO₂CH₂CHF₂ | O | OCO₂Et | H | H | H |
| CO₂CH₂CHF₂ | O | OCO(SMe) | H | H | H |
| CO₂CH₂CHF₂ | O | OSO₂Me | H | H | H |
| CO₂CH₂CHF₂ | O | OSO₂CF₃ | H | H | H |
| CO₂CH₂CHF₂ | O | OSO₂Ph | H | H | H |
| CO₂CH₂CHF₂ | O | OPO(OEt)₂ | H | H | H |
| CO₂CH₂CHF₂ | O | OCH₂OMe | H | H | H |
| CO₂CH₂CHF₂ | O | SMe | H | H | H |
| CO₂CH₂CHF₂ | O | SEt | H | H | H |
| CO₂CH₂CHF₂ | O | SCF₃ | H | H | H |

**[Table 122]**

| R² | W | X¹ | X² | X³ | X⁴ |
|---|---|---|---|---|---|
| CO₂CH₂CHF₂ | O | SO₂Me | H | H | H |
| CO₂CH₂CHF₂ | O | SO₂CF₃ | H | H | H |
| CO₂CH₂CHF₂ | O | Ph | H | H | H |
| CO₂CH₂CHF₂ | O | 4-ClPh | H | H | H |
| CO₂CH₂CHF₂ | O | 4-MePh | H | H | H |
| CO₂CH₂CHF₂ | O | NMe₂ | H | H | H |
| CO₂CH₂CHF₂ | O | NHAc | H | H | H |
| CO₂CH₂CHF₂ | O | NAc₂ | H | H | H |
| CO₂CH₂CHF₂ | O | NHSO₂CF₃ | H | H | H |
| CO₂CH₂CHF₂ | O | N-morpholinyl | H | H | H |
| CO₂CH₂CHF₂ | O | CO₂Me | H | H | H |
| CO₂CH₂CHF₂ | O | CO₂Et | H | H | H |
| CO₂CH₂CHF₂ | O | OH | H | H | H |
| CO₂CH₂CHF₂ | O | CN | H | H | H |
| CO₂CH₂CHF₂ | O | NO₂ | H | H | H |
| CO₂CH₂CHF₂ | O | H | F | H | H |
| CO₂CH₂CHF₂ | O | H | Cl | H | H |
| CO₂CH₂CHF₂ | O | H | Br | H | H |
| CO₂CH₂CHF₂ | O | H | I | H | H |
| CO₂CH₂CHF₂ | O | H | Me | H | H |
| CO₂CH₂CHF₂ | O | H | c-Pr | H | H |
| CO₂CH₂CHF₂ | O | H | OMe | H | H |
| CO₂CH₂CHF₂ | O | H | OCF₃ | H | H |
| CO₂CH₂CHF₂ | O | H | OCH₂C≡CH | H | H |
| CO₂CH₂CHF₂ | O | H | Bn | H | H |
| CO₂CH₂CHF₂ | O | H | OAc | H | H |
| CO₂CH₂CHF₂ | O | H | OSO₂Me | H | H |

**[Table 123]**

| R² | W | X¹ | X² | X³ | X⁴ |
|---|---|---|---|---|---|
| CO₂CH₂CHF₂ | O | H | SMe | H | H |
| CO₂CH₂CHF₂ | O | H | SO₂Me | H | H |
| CO₂CH₂CHF₂ | O | H | Ph | H | H |
| CO₂CH₂CHF₂ | O | H | NMe₂ | H | H |
| CO₂CH₂CHF₂ | O | H | CN | H | H |
| CO₂CH₂CHF₂ | O | H | NO₂ | H | H |
| CO₂CH₂CHF₂ | O | H | H | F | H |
| CO₂CH₂CHF₂ | O | H | H | Cl | H |
| CO₂CH₂CHF₂ | O | H | H | Br | H |
| CO₂CH₂CHF₂ | O | H | H | I | H |
| CO₂CH₂CHF₂ | O | H | H | Me | H |
| CO₂CH₂CHF₂ | O | H | H | c-Pr | H |
| CO₂CH₂CHF₂ | O | H | H | OMe | H |
| CO₂CH₂CHF₂ | O | H | H | OCF₃ | H |
| CO₂CH₂CHF₂ | O | H | H | OCH₂C≡CH | H |
| CO₂CH₂CHF₂ | O | H | H | Bn | H |
| CO₂CH₂CHF₂ | O | H | H | OAc | H |
| CO₂CH₂CHF₂ | O | H | H | OSO₂Me | H |
| CO₂CH₂CHF₂ | O | H | H | SMe | H |
| CO₂CH₂CHF₂ | O | H | H | SO₂Me | H |
| CO₂CH₂CHF₂ | O | H | H | Ph | H |
| CO₂CH₂CHF₂ | O | H | H | NMe₂ | H |
| CO₂CH₂CHF₂ | O | H | H | CN | H |
| CO₂CH₂CHF₂ | O | H | H | NO₂ | H |
| CO₂CH₂CHF₂ | O | H | H | H | F |
| CO₂CH₂CHF₂ | O | H | H | H | Cl |
| CO₂CH₂CHF₂ | O | H | H | H | Br |

**[Table 124]**

| R² | W | ¹ | X² | X³ | X⁴ |
|---|---|---|---|---|---|
| CO₂CH₂CHF₂ | O | H | H | H | I |
| CO₂CH₂CHF₂ | O | H | H | H | Me |
| CO₂CH₂CHF₂ | O | H | H | H | c-Pr |
| CO₂CH₂CHF₂ | O | H | H | H | OMe |
| CO₂CH₂CHF₂ | O | H | H | H | OCF₃ |
| CO₂CH₂CHF₂ | O | H | H | H | OCH₂C≡CH |
| CO₂CH₂CHF₂ | O | H | H | H | Bn |
| CO₂CH₂CHF₂ | O | H | H | H | OAc |
| CO₂CH₂CHF₂ | O | H | H | H | OSO₂Me |
| CO₂CH₂CHF₂ | O | H | H | H | SMe |
| CO₂CH₂CHF₂ | O | H | H | H | SO₂Me |
| CO₂CH₂CHF₂ | O | H | H | H | Ph |
| CO₂CH₂CHF₂ | O | H | H | H | NMe₂ |
| CO₂CH₂CHF₂ | O | H | H | H | CN |
| CO₂CH₂CHF₂ | O | H | H | H | NO₂ |
| CO₂CH₂CHF₂ | O | F | F | H | H |
| CO₂CH₂CHF₂ | O | F | H | F | H |
| CO₂CH₂CHF₂ | O | F | Cl | H | H |
| CO₂CH₂CHF₂ | O | F | H | Cl | H |
| CO₂CH₂CHF₂ | O | F | H | H | Cl |
| CO₂CH₂CHF₂ | O | F | H | H | Br |
| CO₂CH₂CHF₂ | O | F | H | H | I |
| CO₂CH₂CHF₂ | O | F | H | H | Me |
| CO₂CH₂CHF₂ | O | F | H | H | c-Pr |
| CO₂CH₂CHF₂ | O | F | H | H | OMe |
| CO₂CH₂CHF₂ | O | F | H | H | OCF₃ |
| CO₂CH₂CHF₂ | O | F | H | H | OCH₂C≡CH |

**[Table 125]**

| R² | W | X¹ | X² | X³ | X⁴ |
|---|---|---|---|---|---|
| CO₂CH₂CHF₂ | O | F | H | H | OSO₂Me |
| CO₂CH₂CHF₂ | O | F | H | H | SMe |
| CO₂CH₂CHF₂ | O | F | H | H | SO₂Me |
| CO₂CH₂CHF₂ | O | F | H | H | NMe₂ |
| CO₂CH₂CHF₂ | O | F | H | H | CN |
| CO₂CH₂CHF₂ | O | F | H | H | NO₂ |
| CO₂CH₂CHF₂ | O | F | F | F | H |
| CO₂CH₂CHF₂ | O | F | F | H | F |
| CO₂CH₂CHF₂ | O | F | H | F | F |
| CO₂CH₂CHF₂ | O | F | F | F | F |
| CO₂CH₂CHF₂ | O | Cl | F | H | H |
| CO₂CH₂CHF₂ | O | Cl | H | F | H |
| CO₂CH₂CHF₂ | O | Cl | H | H | F |
| CO₂CH₂CHF₂ | O | Cl | Cl | H | H |
| CO₂CH₂CHF₂ | O | Cl | H | Cl | H |
| CO₂CH₂CHF₂ | O | Cl | H | H | Cl |
| CO₂CH₂CHF₂ | O | Cl | H | H | Br |
| CO₂CH₂CHF₂ | O | Cl | H | H | I |
| CO₂CH₂CHF₂ | O | Cl | H | H | Me |
| CO₂CH₂CHF₂ | O | Cl | H | H | c-Pr |
| CO₂CH₂CHF₂ | O | Cl | H | H | OMe |
| CO₂CH₂CHF₂ | O | Cl | H | H | OCF₃ |
| CO₂CH₂CHF₂ | O | Cl | H | H | OCH₂C≡CH |
| CO₂CH₂CHF₂ | O | Cl | H | H | OSO₂Me |
| CO₂CH₂CHF₂ | O | Cl | H | H | SMe |
| CO₂CH₂CHF₂ | O | Cl | H | H | S0₂Me |
| CO₂CH₂CHF₂ | O | Cl | H | H | NMe₂ |

**[Table 126]**

| R² | W | X¹ | X² | X³ | X⁴ |
|---|---|---|---|---|---|
| CO₂CH₂CHF₂ | O | Cl | H | H | CN |
| CO₂CH₂CHF₂ | O | Cl | H | H | NO₂ |
| CO₂CH₂CHF₂ | O | Br | H | H | F |
| CO₂CH₂CHF₂ | O | Br | H | H | Cl |
| CO₂CH₂CHF₂ | O | Br | H | H | Me |
| CO₂CH₂CHF₂ | O | Br | H | H | OMe |
| CO₂CH₂CHF₂ | O | Me | H | H | F |
| CO₂CH₂CHF₂ | O | Me | H | H | Cl |
| CO₂CH₂CHF₂ | O | Me | H | H | Me |
| CO₂CH₂CHF₂ | O | Me | H | H | 0Me |
| CO₂CH₂CHF₂ | O | OMe | H | H | F |
| CO₂CH₂CHF₂ | O | OMe | H | H | Cl |
| CO₂CH₂CHF₂ | O | OMe | H | H | Me |
| CO₂CH₂CHF₂ | O | OMe | H | H | OMe |
| CO₂CH₂CHF₂ | O | H | F | F | H |
| CO₂CH₂CHF₂ | O | H | F | Cl | H |
| CO₂CH₂CHF₂ | O | H | F | H | F |
| CO₂CH₂CHF₂ | O | H | F | H | Cl |
| CO₂CH₂CHF₂ | O | H | Cl | Cl | H |
| CO₂CH₂CHF₂ | O | H | Cl | F | H |
| CO₂CH₂CHF₂ | O | H | Cl | H | F |
| CO₂CH₂CHF₂ | O | H | Cl | H | Cl |
| CO₂CH₂CHF₂ | O | H | H | F | F |
| CO₂CH₂CHF₂ | O | H | H | F | Cl |
| CO₂CH₂CHF₂ | O | H | H | Cl | Cl |
| CO₂CH₂CHF₂ | O | F | F | F | F |
| CO₂CH₂CHF₂ | O | Cl | Cl | Cl | Cl |

**[Table 127]**

| R² | W | X¹ | X² | X³ | X⁴ |
|---|---|---|---|---|---|
| CO₂CH₂CHF₂ | O | -CH=CH-CH=CH- | | H | H |
| CO₂CH₂CHF₂ | O | -CH=N-CH=CH- | | H | H |
| CO₂CH₂CHF₂ | O | -S-CH=N- | | H | H |
| CO₂CH₂CHF₂ | O | -N=CH-S- | | H | H |
| CO₂CH₂CHF₂ | O | H | -CH=CH-CH=CH- | | H |
| CO₂CH₂CHF₂ | O | H | -CH=N-CH=CH- | | H |
| CO₂CH₂CHF₂ | O | H | -S-CH=N- | | H |
| CO₂CH₂CHF₂ | O | H | -N=CH-S- | | H |
| CO₂CH₂CHF₂ | O | H | H | H-CH=CH-CH=CH- | |
| CO₂CH₂CHF₂ | O | H | H | -CH=N-CH=CH- | |
| CO₂CH₂CHF₂ | O | H | H | -S-CH=N- | |
| CO₂CH₂CHF₂ | O | H | H | -N=CH-S- | |
| CO₂(3-oxetanyl) | O | Cl | H | H | H |
| CO₂(3-oxetanyl) | O | Br | H | H | H |
| CO₂(3-oxetanyl) | O | I | H | H | H |
| CO₂(3-oxetanyl) | O | Me | H | H | H |
| CO₂(3-oxetanyl) | O | Et | H | H | H |
| CO₂(3-oxetanyl) | O | n-Pr | H | H | H |
| CO₂(3-oxetanyl) | O | i-Pr | H | H | H |
| CO₂(3-oxetanyl) | O | n-Bu | H | H | H |
| CO₂(3-oxetanyl) | O | s-Bu | H | H | H |
| CO₂(3-oxetanyl) | O | i-Bu | H | H | H |
| CO₂(3-oxetanyl) | O | t-Bu | H | H | H |
| CO₂(3-oxetanyl) | O | c-Pr | H | H | H |
| CO₂(3-oxetanyl) | O | c-Bu | H | H | H |
| CO₂(3-oxetanyl) | O | OMe | H | H | H |
| CO₂(3-oxetany l) | O | OEt | H | H | H |

**[Table 128]**

| R² | W | X¹ | X² | X³ | X⁴ |
|---|---|---|---|---|---|
| CO₂(3-oxetanyl) | O | OCHF₂ | H | H | H |
| CO₂(3-oxetanyl) | O | OCF₃ | H | H | H |
| CO₂(3-oxetanyl) | O | OCH₂CH=CH₂ | H | H | H |
| CO₂(3-oxetanyl) | O | OCH₂C≡CH | H | H | H |
| CO₂(3-oxetanyl) | O | OCH₂c-Pr | H | H | H |
| CO₂(3-oxetanyl) | O | Bn | H | H | H |
| CO₂(3-oxetanyl) | O | 4-ClBn | H | H | H |
| CO₂(3-oxetanyl) | O | 4-MeBn | H | H | H |
| CO₂(3-oxetanyl) | O | 4-OMeBn | H | H | H |
| CO₂(3-oxetanyl) | O | OAc | H | H | H |
| CO₂(3-oxetanyl) | O | OBz | H | H | H |
| CO₂(3-oxetanyl) | O | O(4-ClBz) | H | H | H |
| CO₂(3-oxetanyl) | O | OCO₂Et | H | H | H |
| CO₂(3-oxetanyl) | O | OCO(SMe) | H | H | H |
| CO₂(3-oxetanyl) | O | OSO₂Me | H | H | H |
| CO₂(3-oxetanyl) | O | OSO₂CF₃ | H | H | H |
| CO₂(3-oxetanyl) | O | OSO₂Ph | H | H | H |
| CO₂(3-oxetanyl) | O | OPO(OEt)₂ | H | H | H |
| CO₂(3-oxetanyl) | O | OCH₂OMe | H | H | H |
| CO₂(3-oxetanyl) | O | SMe | H | H | H |
| CO₂(3-oxetanyl) | O | SEt | H | H | H |
| CO₂(3-oxetanyl) | O | SCF₃ | H | H | H |
| CO₂(3-oxetanyl) | O | SO₂Me | H | H | H |
| CO₂(3-oxetanyl) | O | SO₂CF₃ | H | H | H |
| CO₂(3-oxetanyl) | O | Ph | H | H | H |
| CO₂(3-oxetanyl) | O | 4-ClPh | H | H | H |
| CO₂(3-oxetanyl) | O | 4-MePh | H | H | H |

**[Table 129]**

| R² | W | X¹ | X² | X³ | X⁴ |
|---|---|---|---|---|---|
| CO₂ (3-oxetanyl) | O | NMe₂ | H | H | H |
| CO₂ (3-oxetanyl) | O | NHAc | H | H | H |
| CO₂ (3-oxetanyl) | O | NAc₂ | H | H | H |
| CO₂ (3-oxetanyl) | O | NHSO₂CF₃ | H | H | H |
| CO₂ (3-oxetanyl) | O | N-morpholinyl | H | H | H |
| CO₂ (3-oxetanyl) | O | CO₂Me | H | H | H |
| CO₂ (3-oxetanyl) | O | CO₂Et | H | H | H |
| CO₂ (3-oxetanyl) | O | OH | H | H | H |
| CO₂ (3-oxetanyl) | O | CN | H | H | H |
| CO₂ (3-oxetanyl) | O | NO₂ | H | H | H |
| CO₂ (3-oxetanyl) | O | H | F | H | H |
| CO₂ (3-oxetanyl) | O | H | Cl | H | H |
| CO₂ (3-oxetanyl) | O | H | Br | H | H |
| CO₂ (3-oxetanyl) | O | H | I | H | H |
| CO₂ (3-oxetanyl) | O | H | Me | H | H |
| CO₂ (3-oxetanyl) | O | H | c-Pr | H | H |
| CO₂ (3-oxetanyl) | O | H | OMe | H | H |
| CO₂ (3-oxetanyl) | O | H | OCF₃ | H | H |
| CO₂ (3-oxetanyl) | O | H | OCH₂C≡CH | H | H |
| CO₂ (3-oxetanyl) | O | H | Bn | H | H |
| CO₂ (3-oxetanyl) | O | H | OAc | H | H |
| CO₂ (3-oxetanyl) | O | H | OSO₂Me | H | H |
| CO₂ (3-oxetanyl) | O | H | SMe | H | H |
| CO₂ (3-oxetanyl) | O | H | SO₂Me | H | H |
| CO₂ (3-oxetanyl) | O | H | Ph | H | H |
| CO₂ (3-oxetanyl) | O | H | NMe₂ | H | H |
| CO₂ (3-oxetanyl) | O | H | CN | H | H |

**[Table 130]**

| R² | W | X¹ | X² | X³ | X⁴ |
|---|---|---|---|---|---|
| CO₂ (3-oxetanyl) | O | H | NO₂ | H | H |
| CO₂ (3-oxetanyl) | O | H | H | F | H |
| CO₂ (3-oxetanyl) | O | H | H | Cl | H |
| CO₂ (3-oxetanyl) | O | H | H | Br | H |
| CO₂ (3-oxetanyl) | O | H | H | I | H |
| CO₂ (3-oxetanyl) | O | H | H | Me | H |
| CO₂ (3-oxetanyl) | O | H | H | c-Pr | H |
| CO₂ (3-oxetanyl) | O | H | H | OMe | H |
| CO₂ (3-oxetanyl) | O | H | H | OCF₃ | H |
| CO₂ (3-oxetanyl) | O | H | H | OCH₂C≡CH | H |
| CO₂ (3-oxetanyl) | O | H | H | Bn | H |
| CO₂ (3-oxetanyl) | O | H | H | OAc | H |
| CO₂ (3-oxetanyl) | O | H | H | OSO₂Me | H |
| CO₂ (3-oxetanyl) | O | H | H | SMe | H |
| CO₂ (3-oxetanyl) | O | H | H | SO₂Me | H |
| CO₂ (3-oxetanyl) | O | H | H | Ph | H |
| CO₂ (3-oxetanyl) | O | H | H | NMe₂ | H |
| CO₂ (3-oxetanyl) | O | H | H | CN | H |
| CO₂ (3-oxetanyl) | O | H | H | NO₂ | H |
| CO₂ (3-oxetanyl) | O | H | H | H | F |
| CO₂ (3-oxetanyl) | O | H | H | H | Cl |
| CO₂ (3-oxetanyl) | O | H | H | H | Br |
| CO₂ (3-oxetanyl) | O | H | H | H | I |
| CO₂ (3-oxetanyl) | O | H | H | H | Me |
| CO₂ (3-oxetanyl) | O | H | H | H | c-Pr |
| CO₂ (3-oxetanyl) | O | H | H | H | OMe |
| CO₂ (3-oxetanyl) | O | H | H | H | OCF₃ |

**[Table 131]**

| R² | W | X¹ | X² | X³ | X⁴ |
|---|---|---|---|---|---|
| CO₂ (3-oxetanyl) | O | H | H | H | OCH₂C≡CH |
| CO₂ (3-oxetanyl) | O | H | H | H | Bn |
| CO₂ (3-oxetanyl) | O | H | H | H | OAc |
| CO₂ (3-oxetanyl) | O | H | H | H | OSO₂Me |
| CO₂ (3-oxetanyl) | O | H | H | H | SMe |
| CO₂ (3-oxetanyl) | O | H | H | H | SO₂Me |
| CO₂ (3-oxetanyl) | O | H | H | H | Ph |
| CO₂ (3-oxetanyl) | O | H | H | H | NMe₂ |
| CO₂ (3-oxetanyl) | O | H | H | H | CN |
| CO₂ (3-oxetanyl) | O | H | H | H | NO₂ |
| CO₂ (3-oxetanyl) | O | F | F | H | H |
| CO₂ (3-oxetanyl) | O | F | H | F | H |
| CO₂ (3-oxetanyl) | O | F | Cl | H | H |
| CO₂ (3-oxetanyl) | O | F | H | Cl | H |
| CO₂ (3-oxetanyl) | O | F | H | H | Cl |
| CO₂ (3-oxetanyl) | O | F | H | H | Br |
| CO₂ (3-oxetanyl) | O | F | H | H | I |
| CO₂ (3-oxetanyl) | O | F | H | H | Me |
| CO₂ (3-oxetanyl) | O | F | H | H | c-Pr |
| CO₂ (3-oxetanyl) | O | F | H | H | OMe |
| CO₂ (3-oxetanyl) | O | F | H | H | OCF₃ |
| CO₂ (3-oxetanyl) | O | F | H | H | OCH₂C= CH |
| CO₂ (3-oxetanyl) | O | F | H | H | OSO₂Me |
| CO₂ (3-oxetanyl) | O | F | H | H | SMe |
| CO₂ (3-oxetanyl) | O | F | H | H | SO₂Me |
| CO₂ (3-oxetanyl) | O | F | H | H | NMe₂ |
| CO₂ (3-oxetanyl) | O | F | H | H | CN |

**[Table 132]**

| R² | W | X¹ | X² | X³ | X⁴ |
|---|---|---|---|---|---|
| CO₂ (3-oxetanyl) | O | F | H | H | NO₂ |
| CO₂ (3-oxetanyl) | O | F | F | F | H |
| CO₂ (3-oxetanyl) | O | F | F | H | F |
| CO₂ (3-oxetanyl) | O | F | H | F | F |
| CO₂ (3-oxetanyl) | O | F | F | F | F |
| CO₂ (3-oxetanyl) | O | Cl | F | H | H |
| CO₂ (3-oxetanyl) | O | Cl | H | F | H |
| CO₂ (3-oxetanyl) | O | Cl | H | H | F |
| CO₂ (3-oxetanyl) | O | Cl | Cl | H | H |
| CO₂ (3-oxetanyl) | O | Cl | H | Cl | H |
| CO₂ (3-oxetanyl) | O | Cl | H | H | Cl |
| CO₂ (3-oxetanyl) | O | Cl | H | H | Br |
| CO₂ (3-oxetanyl) | O | Cl | H | H | I |
| CO₂ (3-oxetanyl) | O | Cl | H | H | Me |
| CO₂ (3-oxetanyl) | O | Cl | H | H | c-Pr |
| CO₂ (3-oxetanyl) | O | Cl | H | H | OMe |
| CO₂ (3-oxetanyl) | O | Cl | H | H | OCF₃ |
| CO₂ (3-oxetanyl) | O | Cl | H | H | OCH₂C≡CH |
| CO₂ (3-oxetanyl) | O | Cl | H | H | OSO₂Me |
| CO₂ (3-oxetanyl) | O | Cl | H | H | SMe |
| CO₂ (3-oxetanyl) | O | Cl | H | H | SO₂Me |
| CO₂ (3-oxetanyl) | O | Cl | H | H | NMe₂ |
| CO₂ (3-oxetanyl) | O | Cl | H | H | CN |
| CO₂ (3-oxetanyl) | O | Cl | H | H | NO₂ |
| CO₂ (3-oxetanyl) | O | Br | H | H | F |
| CO₂ (3-oxetanyl) | O | Br | H | H | Cl |
| CO₂ (3-oxetanyl) | O | Br | H | H | Me |

**[Table 133]**

| R² | W | X¹ | X² | X³ | X⁴ |
|---|---|---|---|---|---|
| CO₂(3-oxetanyl) | O | Br | H | H | OMe |
| CO₂ (3-oxetanyl) | O | Me | H | H | F |
| CO₂ (3-oxetanyl) | O | Me | H | H | Cl |
| CO₂ (3-oxetanyl) | O | Me | H | H | Me |
| CO₂ (3-oxetanyl) | O | Me | H | H | OMe |
| CO₂ (3-oxetanyl) | O | OMe | H | H | F |
| CO₂ (3-oxetanyl) | O | OMe | H | H | Cl |
| CO₂ (3-oxetanyl) | O | OMe | H | H | Me |
| CO₂ (3-oxetanyl) | O | OMe | H | H | OMe |
| CO₂ (3-oxetanyl) | O | H | F | F | H |
| CO₂ (3-oxetanyl) | O | H | F | Cl | H |
| CO₂ (3-oxetanyl) | O | H | F | H | F |
| CO₂ (3-oxetanyl) | O | H | F | H | Cl |
| CO₂ (3-oxetanyl) | O | H | Cl | Cl | H |
| CO₂ (3-oxetanyl) | O | H | Cl | F | H |
| CO₂ (3-oxetanyl) | O | H | Cl | H | F |
| CO₂ (3-oxetanyl) | O | H | Cl | H | Cl |
| CO₂ (3-oxetanyl) | O | H | H | F | F |
| CO₂ (3-oxetanyl) | O | H | H | F | Cl |
| CO₂ (3-oxetanyl) | O | H | H | Cl | Cl |
| CO₂ (3-oxetanyl) | O | F | F | F | F |
| CO₂ (3-oxetanyl) | O | Cl | Cl | Cl | Cl |
| CO₂ (3-oxetanyl) | O | -CH=CH-CH=CH- | | H | H |
| CO₂ (3-oxetanyl) | O | -CH=N-CH=CH- | | H | H |
| CO₂ (3-oxetanyl) | O | -S-CH=N- | | H | H |
| CO₂ (3-oxetanyl) | O | -N=CH-S- | | H | H |
| CO₂ (3-oxetanyl) | O | H | -CH=CH-CH=CH- | | H |

**[Table 134]**

| R² | W | X¹ | X² | X³ | X⁴ |
|---|---|---|---|---|---|
| CO₂ (3-oxetanyl) | O | H | -CH=N-CH=CH- | | H |
| CO₂ (3-oxetany l) | O | H | -S-CH=N- | | H |
| CO₂ (3-oxetanyl) | O | H | -N=CH-S- | | H |
| CO₂ (3-oxetany l) | O | H | H | -CH=CH-CH=CH- | |
| CO₂ (3-oxetanyl) | O | H | H | -CH=N-CH=CH- | |
| CO₂ (3-oxetanyl) | O | H | H | -S-CH=N- | |
| CO₂ (3-oxetanyl) | O | H | H | -N=CH-S- | |
| CO₂ (3-tetrahydrofuryl) | O | Cl | H | H | H |
| CO₂ (3-tetrahydrofury l) | O | Br | H | H | H |
| CO₂ (3-tetrahydrofuryl) | O | I | H | H | H |
| CO₂ (3-tetrahydrofuryl) | O | Me | H | H | H |
| CO₂ (3-tetrahydrofuryl) | O | Et | H | H | H |
| CO₂ (3-tetrahydrofuryl) | O | n-Pr | H | H | H |
| CO₂ (3-tetrahydrofury l) | O | i-Pr | H | H | H |
| CO₂ (3-tetrahydrofuryl) | O | n-Bu | H | H | H |
| CO₂ (3-tetrahydrofuryl) | O | s-Bu | H | H | H |
| CO₂ (3-tetrahydrofuryl) | O | i-Bu | H | H | H |
| CO₂ (3-tetrahydrofuryl) | O | t-Bu | H | H | H |
| CO₂ (3-tetrahydrofuryl) | O | c-Pr | H | H | H |
| CO₂ (3-tetrahydrofuryl) | O | c-Bu | H | H | H |
| CO₂ (3-tetrahydrofuryl) | O | OMe | H | H | H |
| CO₂ (3-tetrahydrofuryl) | O | OEt | H | H | H |
| CO₂ (3-tetrahydrofuryl) | O | OCHF₂ | H | H | H |
| CO₂ (3-tetrahydrofuryl) | O | OCF₃ | H | H | H |
| CO₂ (3-tetrahydrofuryl) | O | OCH₂CH=CH₂ | H | H | H |
| CO₂ (3-tetrahydrofuryl) | O | OCH₂C≡CH | H | H | H |
| CO₂ (3-tetrahydrofuryl) | O | OCH₂c-Pr | H | H | H |

**[Table 135]**

| R² | W | X¹ | X² | X³ | X⁴ |
|---|---|---|---|---|---|
| CO₂ (3-tetrahydrofuryl) | O | Bn | H | H | H |
| CO₂ (3-tetrahydrofuryl) | O | 4-ClBn | H | H | H |
| CO₂ (3-tetrahydrofuryl) | O | 4-MeBn | H | H | H |
| CO₂ (3-tetrahydrofuryl) | O | 4-OMeBn | H | H | H |
| CO₂ (3-tetrahydrofuryl) | O | OAc | H | H | H |
| CO₂ (3-tetrahydrofuryl) | O | OBz | H | H | H |
| CO₂ (3-tetrahydrofuryl) | O | O(4-ClBz) | H | H | H |
| CO₂ (3-tetrahydrofuryl) | O | OCO₂Et | H | H | H |
| CO₂ (3-tetrahydrofuryl) | O | OCO(SMe) | H | H | H |
| CO₂ (3-tetrahydrofuryl) | O | OSO₂Me | H | H | H |
| CO₂ (3-tetrahydrofuryl) | O | OSO₂CF₃ | H | H | H |
| CO₂ (3-tetrahydrofuryl) | O | OSO₂Ph | H | H | H |
| CO₂ (3-tetrahydrofuryl) | O | OPO(OEt)₂ | H | H | H |
| CO₂ (3-tetrahydrofuryl) | O | OCH₂OMe | H | H | H |
| CO₂ (3-tetrahydrofuryl) | O | SMe | H | H | H |
| CO₂ (3-tetrahydrofuryl) | O | SEt | H | H | H |
| CO₂ (3-tetrahydrofuryl) | O | SCF₃ | H | H | H |
| CO₂ (3-tetrahydrofuryl) | O | SO₂Me | H | H | H |
| CO₂ (3-tetrahydrofuryl) | O | SO₂CF₃ | H | H | H |
| CO₂ (3-tetrahydrofuryl) | O | Ph | H | H | H |
| CO₂ (3-tetrahydrofuryl) | O | 4-ClPh | H | H | H |
| CO₂ (3-tetrahydrofuryl) | O | 4-MePh | H | H | H |
| CO₂ (3-tetrahydrofuryl) | O | NMe₂ | H | H | H |
| CO₂ (3-tetrahydrofuryl) | O | NHAc | H | H | H |
| CO₂ (3-tetrahydrofuryl) | O | NAc₂ | H | H | H |
| CO₂ (3-tetrahydrofuryl) | O | NHSO₂CF₃ | H | H | H |
| CO₂ (3-tetrahydrofuryl) | O | N-morpholinyl | H | H | H |

**[Table 136]**

| R² | W | X¹ | X² | X³ | X⁴ |
|---|---|---|---|---|---|
| CO₂ (3-tetrahydrofuryl) | O | CO₂Me | H | H | H |
| CO₂ (3-tetrahydrofuryl) | O | CO₂Et | H | H | H |
| CO₂ (3-tetrahydrofuryl) | O | OH | H | H | H |
| CO₂ (3-tetrahydrofuryl) | O | CN | H | H | H |
| CO₂ (3-tetrahydrofuryl) | O | NO₂ | H | H | H |
| CO₂ (3-tetrahydrofuryl) | O | H | F | H | H |
| CO₂ (3-tetrahydrofuryl) | O | H | Cl | H | H |
| CO₂ (3-tetrahydrofuryl) | O | H | Br | H | H |
| CO₂ (3-tetrahydrofuryl) | O | H | I | H | H |
| CO₂ (3-tetrahydrofuryl) | O | H | Me | H | H |
| CO₂ (3-tetrahydrofuryl) | O | H | c-Pr | H | H |
| CO₂ (3-tetrahydrofuryl) | O | H | OMe | H | H |
| CO₂ (3-tetrahydrofuryl) | O | H | OCF₃ | H | H |
| CO₂ (3-tetrahydrofuryl) | O | H | OCH₂C≡CH | H | H |
| CO₂ (3-tetrahydrofuryl) | O | H | Bn | H | H |
| CO₂ (3-tetrahydrofuryl) | O | H | OAc | H | H |
| CO₂ (3-tetrahydrofuryl) | O | H | OSO₂Me | H | H |
| CO₂ (3-tetrahydrofuryl) | O | H | SMe | H | H |
| CO₂ (3-tetrahydrofuryl) | O | H | SO₂Me | H | H |
| CO₂ (3-tetrahydrofuryl) | O | H | Ph | H | H |
| CO₂ (3-tetrahydrofuryl) | O | H | NMe₂ | H | H |
| CO₂ (3-tetrahydrofuryl) | O | H | CN | H | H |
| CO₂ (3-tetrahydrofuryl) | O | H | NO₂ | H | H |
| CO₂ (3-tetrahydrofuryl) | O | H | H | F | H |
| CO₂ (3-tetrahydrofuryl) | O | H | H | Cl | H |
| CO₂ (3-tetrahydrofuryl) | O | H | H | Br | H |
| CO₂ (3-tetrahydrofuryl) | O | H | H | I | H |

**[Table 137]**

| R² | W | X¹ | X² | X³ | X⁴ |
|---|---|---|---|---|---|
| CO₂ (3-tetrahydrofuryl) | O | H | H | Me | H |
| CO₂ (3-tetrahydrofuryl) | O | H | H | c-Pr | H |
| CO₂ (3-tetrahydrofuryl) | O | H | H | OMe | H |
| CO₂ (3-tetrahydrofuryl) | O | H | H | OCF₃ | H |
| CO₂ (3-tetrahydrofuryl) | O | H | H | OCH₂C≡CH | H |
| CO₂ (3-tetrahydrofuryl) | O | H | H | Bn | H |
| CO₂ (3-tetrahydrofuryl) | O | H | H | OAc | H |
| CO₂ (3-tetrahydrofuryl) | O | H | H | OSO₂Me | H |
| CO₂ (3-tetrahydrofuryl) | O | H | H | SMe | H |
| CO₂ (3-tetrahydrofuryl) | O | H | H | SO₂Me | H |
| CO₂ (3-tetrahydrofuryl) | O | H | H | Ph | H |
| CO₂ (3-tetrahydrofuryl) | O | H | H | NMe₂ | H |
| CO₂ (3-tetrahydrofuryl) | O | H | H | CN | H |
| CO₂ (3-tetrahydrofuryl) | O | H | H | NO₂ | H |
| CO₂ (3-tetrahydrofuryl) | O | H | H | H | F |
| CO₂ (3-tetrahydrofuryl) | O | H | H | H | Cl |
| CO₂ (3-tetrahydrofuryl) | O | H | H | H | Br |
| CO₂ (3-tetrahydrofuryl) | O | H | H | H | I |
| CO₂ (3-tetrahydrofuryl) | O | H | H | H | Me |
| CO₂ (3-tetrahydrofuryl) | O | H | H | H | c-Pr |
| CO₂ (3-tetrahydrofuryl) | O | H | H | H | OMe |
| CO₂ (3-tetrahydrofuryl) | O | H | H | H | OCF₃ |
| CO₂ (3-tetrahydrofuryl) | O | H | H | H | OCH₂C≡CH |
| CO₂ (3-tetrahydrofuryl) | O | H | H | H | Bn |
| CO₂ (3-tetrahydrofuryl) | O | H | H | H | OAc |
| CO₂ (3-tetrahydrofuryl) | O | H | H | H | OSO₂Me |
| CO₂ (3-tetrahydrofuryl) | O | H | H | H | SMe |

**[Table 138]**

| R² | W | X¹ | X² | X³ | X⁴ |
|---|---|---|---|---|---|
| CO₂ (3-tetrahydrofuryl) | O | H | H | H | SO₂Me |
| CO₂ (3-tetrahydrofuryl) | O | H | H | H | Ph |
| CO₂ (3-tetrahydrofury l) | O | H | H | H | NMe₂ |
| CO₂ (3-tetrahydrofuryl) | O | H | H | H | CN |
| CO₂ (3-tetrahydrofuryl) | O | H | H | H | NO₂ |
| CO₂ (3-tetrahydrofuryl) | O | F | F | H | H |
| CO₂ (3-tetrahydrofuryl) | O | F | H | F | H |
| CO₂ (3-tetrahydrofuryl) | O | F | Cl | H | H |
| CO₂ (3-tetrahydrofuryl) | O | F | H | Cl | H |
| CO₂ (3-tetrahydrofuryl) | O | F | H | H | Cl |
| CO₂ (3-tetrahydrofuryl) | O | F | H | H | Br |
| CO₂ (3-tetrahydrofuryl) | O | F | H | H | I |
| CO₂ (3-tetrahydrofuryl) | O | F | H | H | Me |
| CO₂ (3-tetrahydrofuryl) | O | F | H | H | c-Pr |
| CO₂ (3-tetrahydrofuryl) | O | F | H | H | OMe |
| CO₂ (3-tetrahydrofuryl) | O | F | H | H | OCF₃ |
| CO₂ (3-tetrahydrofuryl) | O | F | H | H | OCH₂C≡CH |
| CO₂ (3-tetrahydrofuryl) | O | F | H | H | OSO₂Me |
| CO₂ (3-tetrahydrofuryl) | O | F | H | H | SMe |
| CO₂ (3-tetrahydrofuryl) | O | F | H | H | SO₂Me |
| CO₂ (3-tetrahydrofuryl) | O | F | H | H | NMe₂ |
| CO₂ (3-tetrahydrofuryl) | O | F | H | H | CN |
| CO₂ (3-tetrahydrofuryl) | O | F | H | H | NO₂ |
| CO₂ (3-tetrahydrofuryl) | O | F | F | F | H |
| CO₂ (3-tetrahydrofuryl) | O | F | F | H | F |
| CO₂ (3-tetrahydrofuryl) | O | F | H | F | F |
| CO₂ (3-tetrahydrofuryl) | O | F | F | F | F |

**[Table 139]**

| R² | W | ¹ | X² | X³ | X⁴ |
|---|---|---|---|---|---|
| CO₂ (3-tetrahydrofuryl) | O | Cl | F | H | H |
| CO₂ (3-tetrahydrofuryl) | O | Cl | H | F | H |
| CO₂ (3-tetrahydrofuryl) | O | Cl | H | H | F |
| CO₂ (3-tetrahydrofuryl) | O | Cl | Cl | H | H |
| CO₂ (3-tetrahydrofuryl) | O | Cl | H | Cl | H |
| CO₂ (3-tetrahydrofuryl) | O | Cl | H | H | Cl |
| CO₂ (3-tetrahydrofuryl) | O | Cl | H | H | Br |
| CO₂ (3-tetrahydrofuryl) | O | Cl | H | H | I |
| CO₂ (3-tetrahydrofuryl) | O | Cl | H | H | Me |
| CO₂ (3-tetrahydrofuryl) | O | Cl | H | H | c-Pr |
| CO₂ (3-tetrahydrofuryl) | O | Cl | H | H | OMe |
| CO₂ (3-tetrahydrofuryl) | O | Cl | H | H | OCF₃ |
| CO₂ (3-tetrahydrofuryl) | O | Cl | H | H | OCH₂C≡CH |
| CO₂ (3-tetrahydrofuryl) | O | Cl | H | H | OSO₂Me |
| CO₂ (3-tetrahydrofuryl) | O | Cl | H | H | SMe |
| CO₂ (3-tetrahydrofuryl) | O | Cl | H | H | SO₂Me |
| CO₂ (3-tetrahydrofuryl) | O | Cl | H | H | NMe₂ |
| CO₂ (3-tetrahydrofuryl) | O | Cl | H | H | CN |
| CO₂ (3-tetrahydrofuryl) | O | Cl | H | H | NO₂ |
| CO₂ (3-tetrahydrofuryl) | O | Br | H | H | F |
| CO₂ (3-tetrahydrofuryl) | O | Br | H | H | Cl |
| CO₂ (3-tetrahydrofuryl) | O | Br | H | H | Me |
| CO₂ (3-tetrahydrofuryl) | O | Br | H | H | OMe |
| CO₂ (3-tetrahydrofuryl) | O | Me | H | H | F |
| CO₂ (3-tetrahydrofuryl) | O | Me | H | H | Cl |
| CO₂ (3-tetrahydrofuryl) | O | Me | H | H | Me |
| CO₂ (3-tetrahydrofuryl) | O | Me | H | H | OMe |

**[Table 140]**

| R² | W | X¹ | X² | X³ | X⁴ |
|---|---|---|---|---|---|
| CO₂(3-tetrahydrofuryl) | O | OMe | H | H | F |
| CO₂(3-tetrahydrofuryl) | O | OMe | H | H | Cl |
| CO₂(3-tetrahydrofuryl) | O | OMe | H | H | Me |
| CO₂(3-tetrahydrofuryl) | O | OMe | H | H | OMe |
| CO₂(3-tetrahydrofuryl) | O | H | F | F | H |
| CO₂(3-tetrahydrofuryl) | O | H | F | Cl | H |
| CO₂(3-tetrahydrofuryl) | O | H | F | H | F |
| CO₂(3-tetrahydrofuryl) | O | H | F | H | Cl |
| CO₂(3-tetrahydrofuryl) | O | H | Cl | Cl | H |
| CO₂(3-tetrahydrofuryl) | O | H | Cl | F | H |
| CO₂(3-tetrahydrofuryl) | O | H | Cl | H | F |
| CO₂(3-tetrahydrofuryl) | O | H | Cl | H | Cl |
| CO₂(3-tetrahydrofuryl) | O | H | H | F | F |
| CO₂(3-tetrahydrofuryl) | O | H | H | F | Cl |
| CO₂(3-tetrahydrofuryl) | O | H | H | Cl | Cl |
| CO₂(3-tetrahydrofuryl) | O | F | F | F | F |
| CO₂(3-tetrahydrofuryl) | O | Cl | Cl | Cl | Cl |
| CO₂(3-tetrahydrofuryl) | O | -CH=CH-CH=CH- | | H | H |
| CO₂(3-tetrahydrofuryl) | O | -CH=N-CH=CH- | | H | H |
| CO₂(3-tetrahydrofuryl) | O | -S-CH=N | | H | H |
| CO₂(3-tetrahydrofuryl) | O | -N=CH-S | | H | H |
| CO₂(3-tetrahydrofuryl) | O | H | -CH=CH-CH=CH- | | H |
| CO₂(3-tetrahydrofuryl) | O | H | -CH=N-CH=CH- | | H |
| CO₂(3-tetrahydrafuryl) | O | H | -S-CH=N- | | H |
| CO₂(3-tetrahydrofuryl) | O | H | -N=CH-S- | | H |
| CO₂(3-tetrahydrofuryl) | O | H | H | -CH=CH-CH=CH- | |
| CO₂(3-tetrahydrofuryl) | O | H | H | -CH=N-CH=CH- | |

**[Table 141]**

| R² | W | X¹ | X² | X³ | X⁴ |
|---|---|---|---|---|---|
| CO₂(3-tetrahydrofuryl) | O | H | H | -S-CH=N- | |
| CO₂(3-tetrahydrofuryl) | O | H | H | -N=CH-S- | |
| SO₂Me | O | Cl | H | H | H |
| SO₂Me | O | Br | H | H | H |
| SO₂Me | O | I | H | H | H |
| SO₂Me | O | Me | H | H | H |
| SO₂Me | O | Et | H | H | H |
| SO₂Me | O | n-Pr | H | H | H |
| SO₂Me | O | j-Pr | H | H | H |
| SO₂Me | O | n-Bu | H | H | H |
| SO₂Me | O | s-Bu | H | H | H |
| SO₂Me | O | i-Bu | H | H | H |
| SO₂Me | O | t-Bu | H | H | H |
| SO₂Me | O | c-Pr | H | H | H |
| SO₂Me | O | c-Bu | H | H | H |
| SO₂Me | O | OMe | H | H | H |
| SO₂Me | O | OEt | H | H | H |
| SO₂Me | O | OCHF₂ | H | H | H |
| SO₂Me | O | OCF₃ | H | H | H |
| SO₂Me | O | OCH₂CH=CH₂ | H | H | H |
| SO₂Me | O | OCH₂C≡CH | H | H | H |
| SO₂Me | O | OCH₂c-Pr | H | H | H |
| SO₂Me | O | Bn | H | H | H |
| SO₂Me | O | 4-ClBn | H | H | H |
| SO₂Me | O | 4-MeBn | H | H | H |
| SO₂Me | O | 4-0MeBn | H | H | H |
| SO₂Me | O | OAc | H | H | H |

**[Table 142]**

| R² | W | X ¹ | X² | X³ | X⁴ |
|---|---|---|---|---|---|
| SO₂Me | O | OBz | H | H | H |
| SO₂Me | O | O(4-ClBz) | H | H | H |
| SO₂Me | O | OCO₂Et | H | H | H |
| SO₂Me | O | OCO(SMe) | H | H | H |
| SO₂Me | O | OSO₂Me | H | H | H |
| SO₂Me | O | OSO₂CF₃ | H | H | H |
| SO₂Me | O | OSO₂Ph | H | H | H |
| SO₂Me | O | OPO(OEt)₂ | H | H | H |
| SO₂Me | O | OCH₂OMe | H | H | H |
| SO₂Me | O | SMe | H | H | H |
| SO₂Me | O | SEt | H | H | H |
| SO₂Me | O | SCF₃ | H | H | H |
| SO₂Me | O | SO₂Me | H | H | H |
| SO₂Me | O | SO₂CF₃ | H | H | H |
| SO₂Me | O | Ph | H | H | H |
| SO₂Me | O | 4-ClPh | H | H | H |
| SO₂Me | O | 4-MePh | H | H | H |
| SO₂Me | O | NMe₂ | H | H | H |
| SO₂Me | O | NHAc | H | H | H |
| SO₂Me | O | NAc₂ | H | H | H |
| SO₂Me | O | NHSO₂CF₃ | H | H | H |
| SO₂Me | O | N-morpholinyl | H | H | H |
| SO₂Me | O | CO₂Me | H | H | H |
| SO₂Me | O | CO₂Et | H | H | H |
| SO₂Me | O | OH | H | H | H |
| SO₂Me | O | CN | H | H | H |
| SO₂Me | O | NO₂ | H | H | H |

**[Table 143]**

| R² | W | X ¹ | X² | X³ | X⁴ |
|---|---|---|---|---|---|
| SO₂Me | O | H | F | H | H |
| SO₂Me | O | H | Cl | H | H |
| SO₂Me | O | H | Br | H | H |
| SO₂Me | O | H | I | H | H |
| SO₂Me | O | H | Me | H | H |
| SO₂Me | O | H | c-Pr | H | H |
| SO₂Me | O | H | OMe | H | H |
| SO₂Me | O | H | OCF₃ | H | H |
| SO₂Me | O | H | OCH₂C≡CH | H | H |
| SO₂Me | O | H | Bn | H | H |
| SO₂Me | O | H | OAc | H | H |
| SO₂Me | O | H | OSO₂Me | H | H |
| SO₂Me | O | H | SMe | H | H |
| SO₂Me | O | H | SO₂Me | H | H |
| SO₂Me | O | H | Ph | H | H |
| SO₂Me | O | H | NMe₂ | H | H |
| SO₂Me | O | H | CN | H | H |
| SO₂Me | O | H | NO₂ | H | H |
| SO₂Me | O | H | H | F | H |
| SO₂Me | O | H | H | Cl | H |
| SO₂Me | O | H | H | Br | H |
| SO₂Me | O | H | H | I | H |
| SO₂Me | O | H | H | Me | H |
| SO₂Me | O | H | H | c-Pr | H |
| SO₂Me | O | H | H | OMe | H |
| SO₂Me | O | H | H | OCF₃ | H |
| SO₂Me | O | H | H | OCH₂C= CH | H |

**[Table 144]**

| R² | W | X¹ | X² | X³ | X⁴ |
|---|---|---|---|---|---|
| SO₂Me | O | H | H | Bn | H |
| SO₂Me | O | H | H | OAc | H |
| SO₂Me | O | H | H | OSO₂Me | H |
| SO₂Me | O | H | H | SMe | H |
| SO₂Me | O | H | H | SO₂Me | H |
| SO₂Me | O | H | H | Ph | H |
| SO₂Me | O | H | H | NMe₂ | H |
| SO₂Me | O | H | H | CN | H |
| SO₂Me | O | H | H | NO₂ | H |
| SO₂Me | O | H | H | H | F |
| SO₂Me | O | H | H | H | Cl |
| SO₂Me | O | H | H | H | Br |
| SO₂Me | O | H | H | H | I |
| SO₂Me | O | H | H | H | Me |
| SO₂Me | O | H | H | H | c-Pr |
| SO₂Me | O | H | H | H | OMe |
| SO₂Me | O | H | H | H | OCF₃ |
| SO₂Me | O | H | H | H | OCH₂C≡CH |
| SO₂Me | O | H | H | H | Bn |
| SO₂Me | O | H | H | H | OAc |
| SO₂Me | O | H | H | H | OSO₂Me |
| SO₂Me | O | H | H | H | SMe |
| SO₂Me | O | H | H | H | SO₂Me |
| SO₂Me | O | H | H | H | Ph |
| SO₂Me | O | H | H | H | NMe₂ |
| SO₂Me | O | H | H | H | CN |
| SO₂Me | O | H | H | H | NO₂ |

**[Table 145]**

| R² | W | X ¹ | X² | X³ | X⁴ |
|---|---|---|---|---|---|
| SO₂Me | O | F | F | H | H |
| SO₂Me | O | F | H | F | H |
| SO₂Me | O | F | Cl | H | H |
| SO₂Me | O | F | H | Cl | H |
| SO₂Me | O | F | H | H | Cl |
| SO₂Me | O | F | H | H | Br |
| SO₂Me | O | F | H | H | I |
| SO₂Me | O | F | H | H | Me |
| SO₂Me | O | F | H | H | c-Pr |
| SO₂Me | O | F | H | H | OMe |
| SO₂Me | O | F | H | H | OCF₃ |
| SO₂Me | O | F | H | H | OCH₂C≡CH |
| SO₂Me | O | F | H | H | OSO₂Me |
| SO₂Me | O | F | H | H | SMe |
| SO₂Me | O | F | H | H | SO₂Me |
| SO₂Me | O | F | H | H | NMe₂ |
| SO₂Me | O | F | H | H | CN |
| SO₂Me | O | F | H | H | NO₂ |
| SO₂Me | O | F | F | F | H |
| SO₂Me | O | F | F | H | F |
| SO₂Me | O | F | H | F | F |
| SO₂Me | O | F | F | F | F |
| SO₂Me | O | Cl | F | H | H |
| SO₂Me | O | Cl | H | F | H |
| SO₂Me | O | Cl | H | H | F |
| SO₂Me | O | Cl | Cl | H | H |
| SO₂Me | O | Cl | H | Cl | H |

**[Table 146]**

| R² | W | X¹ | X² | X³ | X⁴ |
|---|---|---|---|---|---|
| SO₂Me | O | Cl | H | H | Cl |
| SO₂Me | O | Cl | H | H | Br |
| SO₂Me | O | Cl | H | H | I |
| SO₂Me | O | Cl | H | H | Me |
| SO₂Me | O | Cl | H | H | c-Pr |
| SO₂Me | O | Cl | H | H | OMe |
| SO₂Me | O | Cl | H | H | OCF₃ |
| SO₂Me | O | Cl | H | H | OCH₂C≡CH |
| SO₂Me | O | Cl | H | H | OSO₂Me |
| SO₂Me | O | Cl | H | H | SMe |
| SO₂Me | O | Cl | H | H | SO₂Me |
| SO₂Me | O | Cl | H | H | NMe₂ |
| SO₂Me | O | Cl | H | H | CN |
| SO₂Me | O | Cl | H | H | NO₂ |
| SO₂Me | O | Br | H | H | F |
| SO₂Me | O | Br | H | H | C l |
| SO₂Me | O | Br | H | H | Me |
| SO₂Me | O | Br | H | H | OMe |
| SO₂Me | O | Me | H | H | F |
| SO₂Me | O | Me | H | H | Cl |
| SO₂Me | O | Me | H | H | Me |
| SO₂Me | O | Me | H | H | OMe |
| SO₂Me | O | OMe | H | H | F |
| SO₂Me | O | OMe | H | H | Cl |
| SO₂Me | O | OMe | H | H | Me |
| SO₂Me | O | OMe | H | H | OMe |
| SO₂Me | O | H | F | F | H |

**[Table 147]**

| R² | W | X¹ | X² | X³ | X⁴ |
|---|---|---|---|---|---|
| SO₂Me | O | H | F | Cl | H |
| SO₂Me | O | H | F | H | F |
| SO₂Me | O | H | F | H | Cl |
| SO₂Me | O | H | Cl | Cl | H |
| SO₂Me | O | H | Cl | F | H |
| SO₂Me | O | H | Cl | H | F |
| SO₂Me | O | H | Cl | H | Cl |
| SO₂Me | O | H | H | F | F |
| SO₂Me | O | H | H | F | Cl |
| SO₂Me | O | H | H | Cl | Cl |
| SO₂Me | O | F | F | F | F |
| SO₂Me | O | Cl | Cl | Cl | Cl |
| SO₂Me | O | -CH=CH-CH=CH- | | H | H |
| SO₂Me | O | -CH=N-CH=CH- | | H | H |
| SO₂Me | O | -S-CH=N- | | H | H |
| SO₂Me | O | -N=CH-S- | | H | H |
| SO₂Me | O | H | -CH=CH-CH=CH- | | H |
| SO₂Me | O | H | -CH=N-CH=CH- | | H |
| SO₂Me | O | H | -S-CH=N- | | H |
| SO₂Me | O | H | -N=CH-S- | | H |
| SO₂Me | O | H | H | -CH=CH-CH=CH- | |
| SO₂Me | O | H | H | -CH=N-CH=CH- | |
| SO₂Me | O | H | H | -S-CH=N- | |
| SO₂Me | O | H | H | -N=CH-S- | |
| H | S | H | H | H | H |
| COc-Pr | S | H | H | H | H |
| CO₂Et | S | H | H | H | H |

**[Table 148]**

| R² | W | X¹ | X² | X³ | X⁴ |
|---|---|---|---|---|---|
| CO₂CH₂CH₂F | S | H | H | H | H |
| CO₂CH₂CHF₂ | S | H | H | H | H |
| CO₂(3-oxetanyl) | S | H | H | H | H |
| CO₂(3-tetrahydrofuryl) | S | H | H | H | H |
| SO₂Me | S | H | H | H | H |
| H | S | F | H | H | H |
| COc-Pr | S | F | H | H | H |
| CO₂Et | S | F | H | H | H |
| CO₂CH₂CH₂F | S | F | H | H | H |
| CO₂CH₂CHF₂ | S | F | H | H | H |
| CO₂(3-oxetanyl) | S | F | H | H | H |
| CO₂(3-tetrahydrofuryl) | S | F | H | H | H |
| SO₂Me | S | F | H | H | H |
| H | S | F | H | H | F |
| CO_{c}-Pr | S | F | H | H | F |
| CO₂Et | S | F | H | H | F |
| CO₂CH₂CH₂F | S | F | H | H | F |
| CO₂CH₂CHF₂ | S | F | H | H | F |
| CO₂(3-oxetanyl) | S | F | H | H | F |
| CO₂(3-tetrahydrofuryl) | S | F | H | H | F |
| SO₂Me | S | F | H | H | F |

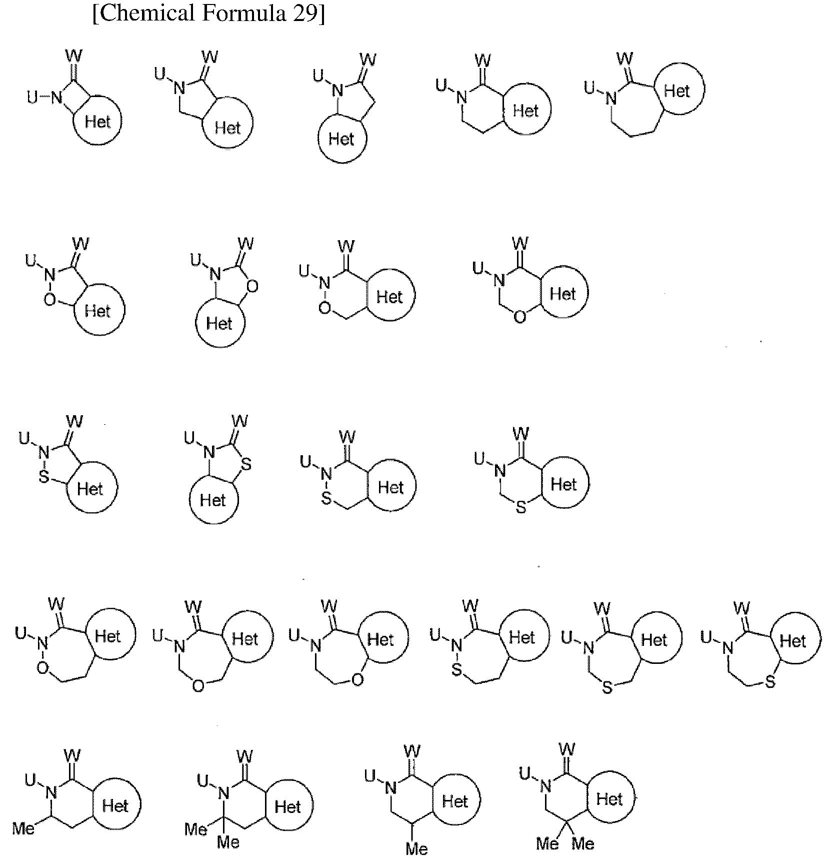

U is the same as general formula (23) above.

**[Table 149]**

| R² | W | Het-No. | R² | W | Het-No. |
|---|---|---|---|---|---|
| H | O | Het-1 | COc-Pr | O | Het-1 |
| Me | O | Het-1 | COc-Bu | O | Het-1 |
| Et | O | Het-1 | COc-Pen | O | Het-1 |
| CH₂CF₃ | O | Het-1 | COc-Hex | O | Het-1 |
| CH₂CH=CH₂ | O | Het-1 | COCH₂c-Pr | O | Het-1 |
| CH₂C≡CH | O | Het-1 | COCH₂OMe | O | Het-1 |
| CH₂OMe | O | Het-1 | COCH₂OCH₂CF₃ | O | Het-1 |
| CH₂OCH₂CF₃ | O | Het-1 | COCH₂OCH₂CH₂CF₃ | O | Het-1 |
| CH₂OCH₂CH₂OMe | O | Het-1 | COCH₂SMe | O | Het-1 |
| CH₂SMe | O | Het-1 | COCH₂SCH₂CF₃ | O | Het-1 |
| CH₂COMe | O | Het-1 | Bz | O | Het-1 |
| CH₂Ph | O | Het-1 | 4-ClBz | O | Het-1 |
| CH₂(4-ClPh) | O | Het-1 | 4-MeBz | O | Het-1 |
| CH₂(4-MePh) | O | Het-1 | COCH₂Ph | O | Het-1 |
| CH₂(4-MeOPh) | O | Het-1 | COCH₂(4-ClPh) | O | Het-1 |
| CH₂CH₂OPh | O | Het-1 | COCH₂(4-MePh) | O | Het-1 |
| COMe | O | Het-1 | CO(2-tetrahydrofuryl) | O | Het-1 |
| COEt | O | Het-1 | CO(3-tetrahydrofuryl) | O | Het-1 |
| COn-Pr | O | Het-1 | CO(2-pyridyl) | O | Het-1 |
| COi-Pr | O | Het-1 | CO(3-pyridyl) | O | Het-1 |
| COn-Bu | O | Het-1 | CO(4-pyridyl) | O | Het-1 |
| COs-Bu | O | Het-1 | CO(2-thienyl) | O | Het-1 |
| C0i-Bu | O | Het-1 | CO(3-thianyl) | O | Het-1 |
| COt-Bu | O | Het-1 | CO(2-tetrahydrofurfuryl) | O | Het-1 |
| COn-Pen | O | Het-1 | COCH₂(2-pyridyl) | O | Het-1 |
| COn-Hex | O | Het-1 | COCH₂(2-thienyl) | O | Het-1 |
| COCF₃ | O | Het-1 | CO₂Me | O | Het-1 |
| COCH₂CF₃ | O | Het-1 | CO₂Et | O | Het-1 |
| COCH=CH₂ | O | Het-1 | CO₂n-Pr | O | Het-1 |
| COC≡CH | O | Het-1 | CO₂i-Pr | O | Het-1 |

**[Table 150]**

| R² | W | Het-No. | R² | W | Het-No. |
|---|---|---|---|---|---|
| CO₂n-Bu | O | Het-1 | CO₂CH₂(2-pyridyl) | O | Het-1 |
| CO₂s-Bu | O | Het-1 | CO₂CH₂(2-thienyl) | O | Het-1 |
| CO₂i-Bu | O | Het-1 | CO(SMe) | O | Het-1 |
| CO₂t-Bu | O | Het-1 | CO(SEt) | O | Het-1 |
| CO₂n-Pen | O | Het-1 | CO(SCF₃) | O | Het-1 |
| CO₂n-Hex | O | Het-1 | CO(SCH₂CF₃) | O | Het-1 |
| CO₂CH₂CH₂F | O | Het-1 | CONHEt | O | Het-1 |
| CO₂CH₂CHF₂ | O | Het-1 | CONHCH₂CF₃ | O | Het-1 |
| CO₂CH₂CF₃ | O | Het-1 | CONEt₂ | O | Het-1 |
| CO₂CH=CH₂ | O | Het-1 | SO₂Me | O | Het-1 |
| CO₂C≡CH | O | Het-1 | SO₂Et | O | Het-1 |
| CO₂c-Pr | O | Het-1 | SO₂n-Pr | O | Het-1 |
| CO₂CH₂c-Pr | O | Het-1 | SO₂i-Pr | O | Het-1 |
| CO₂CH₂CH₂OMe | O | Het-1 | SO₂n-Bu | O | Het-1 |
| CO₂CH₂CH₂OCH₂CF₃ | O | Het-1 | SO₂i-Bu | O | Het-1 |
| CO₂CH₂CH₂OCH₂CH₂OMe | O | Het-1 | SO₂CH₂Cl | O | Het-1 |
| CO₂CH₂CH₂SMe | O | Het-1 | SO₂CCl₃ | O | Het-1 |
| CO₂CH₂CH₂SCH₂CF₃ | O | Het-1 | SO₂CHF₂ | O | Het-1 |
| CO₂Ph | O | Het-1 | SO₂CF₃ | O | Het-1 |
| CO₂(4-ClPh) | O | Het-1 | SO₂CH₂CF₃ | O | Het-1 |
| CO₂(4-MePh) | O | Het-1 | SO₂CH=CH₂ | O | Het-1 |
| CO₂CH₂Ph | O | Het-1 | SO₂CH₂CN=CH₂ | O | Het-1 |
| CO₂CH₂(4-ClPh) | O | Het-1 | SO₂CH₂C≡CH | O | Het-1 |
| CO₂CH₂(4-MePh) | O | Het-1 | SO₂c-Pr | O | Het-1 |
| CO₂(3-oxetanyl) | O | Het-1 | SO₂c-Hex | O | Het-1 |
| CO₂(2-tetrahydrofuryl) | O | Het-1 | SO₂CH₂c-Pr | O | Het-1 |
| CO₂(3-tetrahydrofuryl) | O | Het-1 | SO₂CH₂CH₂OMe | O | Het-1 |
| CO₂(2-pyridyl) | O | Het-1 | SO₂Ph | O | Het-1 |
| CO₂(2-thienyl) | O | Het-1 | SO₂(4-ClPh) | O | Het-1 |
| CO₂(2-tetrahydrofurfuryl) | O | Het-1 | SO₂(4-MePh) | O | Het-1 |

**[Table 151]**

| R² | W | Het-No. | R² | W | Het-No. |
|---|---|---|---|---|---|
| SO₂CH₂Ph | O | Het-1 | COn-Hex | O | Het-2 |
| SO₂CH₂(4-ClPh) | O | Het-1 | COCF₃ | O | Het-2 |
| SO₂CH₂(4-MePh) | O | Het-1 | COCH₂CF₃ | O | Het-2 |
| SO₂NHMe | O | Het-1 | COCH=CH₂ | O | Het-2 |
| SO₂NMe₂ | O | Het-1 | COC≡CH | O | Het-2 |
| H | O | Het-2 | COc-Pr | O | Het-2 |
| Me | O | Het-2 | COc-Bu | O | Het-2 |
| Et | O | Het-2 | COc-Pen | O | Het-2 |
| CH₂CF₃ | O | Het-2 | COc-Hex | O | Het-2 |
| CH₂CH=CH₂ | O | Het-2 | COCH₂c-Pr | O | Het-2 |
| CH₂C≡CH | O | Het-2 | COCH₂OMe | O | Het-2 |
| CH₂OMe | O | Het-2 | COCH₂OCH₂CF₃ | O | Het-2 |
| CH₂OCH₂CF₃ | O | Het-2 | COCH₂OCH₂CH₂CF₃ | O | Het-2 |
| CH₂OCH₂CH₂OMe | O | Het-2 | COCH₂SMe | O | Het-2 |
| CH₂SMe | O | Het-2 | COCH₂SCH₂CF₃ | O | Het-2 |
| CH₂COMe | O | Het-2 | Bz | O | Het-2 |
| CH₂Ph | O | Het-2 | 4-ClBz | O | Het-2 |
| CH₂(4-ClPh) | O | Het-2 | 4-MeBz | O | Het-2 |
| CH₂(4-MePh) | O | Het-2 | COCH₂Ph | O | Het-2 |
| CH₂(4-MeOPh) | O | Het-2 | COCH₂(4-ClPh) | O | Het-2 |
| CH₂CH₂OPh | O | Het-2 | COCH₂(4-MePh) | O | Het-2 |
| COMe | O | Het-2 | CO(2-tetrahydrofuryl) | O | Het-2 |
| COEt | O | Het-2 | CO(3-tetrahydrofuryl) | O | Het-2 |
| COn-Pr | O | Het-2 | CO(2-pyridyl) | O | Het-2 |
| COi-Pr | O | Het-2 | CO(3-pyridyl) | O | Het-2 |
| COn-Bu | O | Het-2 | CO(4-pyridyl) | O | Het-2 |
| COs-Bu | O | Het-2 | CO(2-thienyl) | O | Het-2 |
| COi-Bu | O | Het-2 | CO(3-thienyl) | O | Het-2 |
| COt-Bu | O | Het-2 | CO(2-tetrahydrofurfuryl) | O | Het-2 |
| COn-Pen | O | Het-2 | COCH₂(2-pyr i dyl) | O | Het-2 |

**[Table 152]**

| R² | W | Het-No. | R² | W | Het-No. |
|---|---|---|---|---|---|
| COCH₂(2-thienyl) | O | Het-2 | CO₂(2-tetrahydrofuryl) | O | Het-2 |
| CO₂Me | O | Het-2 | CO₂(3-tetrahydrofuryl) | O | Het-2 |
| CO₂Et | O | Het-2 | CO₂(2-pyridyl) | O | Het-2 |
| CO₂n-Pr | O | Het-2 | CO₂(2-thienyl) | O | Het-2 |
| CO₂i-Pr | O | Het-2 | CO₂(2-tetrahydrofurfuryl) | O | Het-2 |
| CO₂n-Bu | O | Het-2 | CO₂CH₂(2-pyridyl) | O | Het-2 |
| CO₂s-Bu | O | Het-2 | CO₂CH₂(2-thienyl) | O | Het-2 |
| CO₂i-Bu | O | Het-2 | CO(SMe) | O | Het-2 |
| CO₂t-Bu | O | Het-2 | CO(SEt) | O | Het-2 |
| CO₂n-Pen | O | Het-2 | CO(SCF₃) | O | Het-2 |
| CO₂n-Hex | O | Het-2 | CO(SCH₂CF₃) | O | Het-2 |
| CO₂CH₂CN₂F | O | Het-2 | CONHEt | O | Het-2 |
| CO₂CH₂CHF₂ | O | Het-2 | CONHCH₂CF₃ | O | Het-2 |
| CO₂CH₂CF₃ | O | Het-2 | CONEt₂ | O | Het-2 |
| CO₂CH=CH₂ | O | Het-2 | SO₂Me | O | Het-2 |
| CO₂C≡CH | O | Het-2 | SO₂Et | O | Het-2 |
| CO₂c-Pr | O | Het-2 | SO₂n-Pr | O | Het-2 |
| CO₂CH₂c-Pr | O | Het-2 | SO₂i-Pr | O | Het-2 |
| CO₂CH₂CH₂OMe | O | Het-2 | SO₂n-Bu | O | Het-2 |
| CO₂CH₂CH₂OCH₂CF₃ | O | Het-2 | SO₂i-Bu | O | Het-2 |
| CO₂CH₂CH₂OCH₂CH₂OMe | O | Het-2 | SO₂CH₂Cl | O | Het-2 |
| CO₂CH₂CH₂SMe | O | Het-2 | SO₂CCl₃ | O | Het-2 |
| CO₂CH₂CH₂SCH₂CF₃ | O | Het-2 | SO₂CHF₂ | O | Het-2 |
| CO₂Ph | O | Het-2 | SO₂CF₃ | O | Het-2 |
| CO₂(4-ClPh) | O | Het-2 | SO₂CH₂CF₃ | O | Het-2 |
| CO₂(4-MePh) | O | Het-2 | SO₂CH=CH₂ | O | Het-2 |
| CO₂CH₂Ph | O | Het-2 | SO₂CH₂CH=CH₂ | O | Het-2 |
| CO₂CH₂(4-ClPh) | O | Het-2 | SO₂CH₂C≡CH | O | Het-2 |
| CO₂CH₂(4-MePh) | O | Het-2 | SO₂c-Pr | O | Het-2 |
| CO₂(3-oxetanyl) | O | Het-2 | SO₂c-Hex | O | Het-2 |

**[Table 153]**

| R² | W | et-No. | R² | W | et-No. |
|---|---|---|---|---|---|
| SO₂CH₂c-Pr | O | Het-2 | COc-Pr | O | Het-6 |
| SO₂CH₂CH₂OMe | O | Het-2 | COc-Pr | O | Het-7 |
| SO₂Ph | O | Het-2 | COc-Pr | O | Het-8 |
| SO₂(4-ClPh) | O | Het-2 | COc-Pr | O | Het-9 |
| SO₂(4-MePh) | O | Het-2 | COc-Pr | O | Het-10 |
| SO₂CH₂Ph | O | Het-2 | COc-Pr | O | Het-11 |
| SO₂CH₂(4-C I Ph) | O | Het-2 | COc-Pr | O | Het-12 |
| SO₂CH₂(4-MePh) | O | Het-2 | COc-Pr | O | Het-13 |
| SO₂NHMe | O | Het-2 | COc-Pr | O | Het-14 |
| SO₂NMe₂ | O | Het-2 | COc-Pr | O | Het-15 |
| H | O | Het-3 | COc-Pr | O | Het-16 |
| H | O | Het-4 | COc-Pr | O | Het-17 |
| H | O | Het-5 | COc-Pr | O | Het-18 |
| H | O | Het-6 | COc-Pr | O | Het-19 |
| H | O | Het-7 | CO₂Et | O | Het-3 |
| H | O | Het-8 | CO₂Et | O | Het-4 |
| H | O | Het-9 | CO₂Et | O | Het-5 |
| H | O | Het-10 | CO₂Et | O | Het-6 |
| H | O | Het-11 | CO₂Et | O | Het-7 |
| H | O | Het-12 | CO₂Et | O | Het-8 |
| H | O | Het-13 | CO₂Et | O | Het-9 |
| H | O | Het-14 | CO₂Et | O | Het-10 |
| H | O | Het-15 | CO₂Et | O | Het-11 |
| H | O | Het-16 | CO₂Et | O | Het-12 |
| H | O | Het-17 | CO₂Et | O | Het-13 |
| H | O | Het-18 | CO₂Et | O | Het-14 |
| H | O | Het-19 | CO₂Et | O | Het-15 |
| COc-Pr | O | Het-3 | CO₂Et | O | Het-16 |
| COc-Pr | O | Het-4 | CO₂Et | O | Het-17 |
| COc-Pr | O | Het-5 | CO₂Et | O | Het-18 |

**[Table 154]**

| R² | W | et-No. | R² | W | et-No. |
|---|---|---|---|---|---|
| CO₂Et | O | Het-19 | CO₂CH₂CHF₂ | O | Het-15 |
| CO₂CH₂CH₂F | O | Het-3 | CO₂CH₂CHF₂ | O | Het-16 |
| CO₂CH₂CH₂F | O | Het-4 | CO₂CH₂CHF₂ | O | Het-17 |
| CO₂CH₂CH₂F | O | Het-5 | CO₂CH₂CHF₂ | O | Het-18 |
| CO₂CH₂CH₂F | O | Het-6 | CO₂CH₂CHF₂ | O | Het-19 |
| CO₂CH₂CH₂F | O | Het-7 | CO₂(3-oxetanyl) | O | Het-3 |
| CO₂CH₂CH₂F | O | Het-8 | CO₂(3-oxetanyl) | O | Het-4 |
| CO₂CH₂CH₂F | O | Het-9 | CO₂(3-oxetanyl) | O | Het-5 |
| CO₂CH₂CH₂F | O | Het-10 | CO₂(3-oxetanyl) | O | Het-6 |
| CO₂CH₂CH₂F | O | Het-11 | CO₂(3-oxetanyl) | O | Het-7 |
| CO₂CH₂CH₂F | O | Het-12 | CO₂(3-oxetanyl) | O | Het-8 |
| CO₂CH₂CH₂F | O | Het-13 | CO₂(3-oxetanyl) | O | Het-9 |
| CO₂CH₂CH₂F | O | Het-14 | CO₂(3-oxetanyl) | O | Het-10 |
| CO₂CH₂CH₂F | O | Het-15 | CO₂(3-oxetanyl) | O | Het-11 |
| CO₂CH₂CH₂F | O | Het-16 | CO₂(3-oxetanyl) | O | Het-12 |
| CO₂CH₂CH₂F | O | Het-17 | CO₂(3-oxetanyl) | O | Het-13 |
| CO₂CH₂CH₂F | O | Het-18 | CO₂(3-oxetanyl) | O | Het-14 |
| CO₂CH₂CH₂F | O | Het-19 | CO₂(3-oxetanyl) | O | Het-15 |
| CO₂CH₂CHF₂ | O | Het-3 | CO₂(3-oxetanyl) | O | Het-16 |
| CO₂OH₂CHF₂ | O | Het-4 | CO₂(3-oxetanyl) | O | Het-17 |
| CO₂CH₂CHF₂ | O | Het-5 | CO₂(3-oxetanyl) | O | Het-18 |
| CO₂CH₂CHF₂ | O | Het-6 | CO₂(3-oxetanyl) | O | Het-19 |
| CO₂CH₂CHF₂ | O | Het-7 | CO₂(3-tetrahydrofuryl) | O | Het-3 |
| CO₂CH₂CHF₂ | O | Het-8 | CO₂(3-tetrahydrofuryl) | O | Het-4 |
| CO₂CH₂CHF₂ | O | Het-9 | CO₂(3-tetrahydrofuryl) | O | Het-5 |
| CO₂CH₂CHF₂ | O | Het-10 | CO₂(3-tetrahydrofuryl) | O | Het-6 |
| CO₂CH₂CHF₂ | O | Het-11 | CO₂(3-tetrahydrofuryl) | O | Het-7 |
| CO₂CH₂CHF₂ | O | Het-12 | CO₂(3-tetrahydrofuryl) | O | Het-8 |
| CO₂CH₂CHF₂ | O | Het-13 | CO₂(3-tetrahydrofuryl) | O | Het-9 |
| CO₂CH₂CHF₂ | O | Het-14 | CO₂(3-tetrahydrofuryl) | O | Het-10 |

**[Table 155]**

| R² | W | et-No. | R² | W | Het-No. |
|---|---|---|---|---|---|
| CO₂(3-tetrahydrofuryl) | O | Het-11 | SO₂Me | O | Het-15 |
| CO₂(3-tetrahydrofuryl) | O | Het-12 | SO₂Me | O | Het-16 |
| CO₂(3-tetrahydrofuryl) | O | Het-13 | SO₂Me | O | Het-17 |
| CO₂(3-tetrahydrofuryl) | O | Het-14 | SO₂Me | O | Het-18 |
| CO₂(3-tetrahydrofuryl) | O | Het-15 | SO₂Me | O | Het-19 |
| CO₂(3-tetrahydrofuryl) | O | Het-16 | H | S | Het-1 |
| CO₂(3-tetrahydrofuryl) | O | Het-17 | COc-Pr | S | Het-1 |
| CO₂(3-tetrahydrofuryl) | O | Het-18 | CO₂Et | S | Het-1 |
| CO₂(3-tetrahydrofuryl) | O | Het-19 | CO₂CH₂CH₂F | S | Het-1 |
| SO₂Me | O | Het-3 | CO₂CH₂CHF₂ | S | Het-1 |
| SO₂Me | O | Het-4 | CO₂(3-oxetanyl) | S | Het-1 |
| SO₂Me | O | Het-5 | CO₂(3-tetrahydrofuryl) | S | Het-1 |
| SO₂Me | O | Het-6 | SO₂Me | S | Het-1 |
| SO₂Me | O | Het-7 | H | S | Het-2 |
| SO₂Me | O | Het-8 | COc-Pr | S | Het-2 |
| SO₂Me | O | Het-9 | CO₂Et | S | Het-2 |
| SO₂Me | O | Het-10 | CO₂CH₂CH₂F | S | Het-2 |
| SO₂Me | O | Het-11 | CO₂CH₂CHF₂ | S | Het-2 |
| SO₂Me | O | Het-12 | CO₂(3-oxetanyl) | S | Het-2 |
| SO₂Me | O | Het-13 | CO₂(3-tetrahydrofuryl) | S | Het-2 |
| SO₂Me | O | Het-14 | SO₂Me | S | Het-2 |

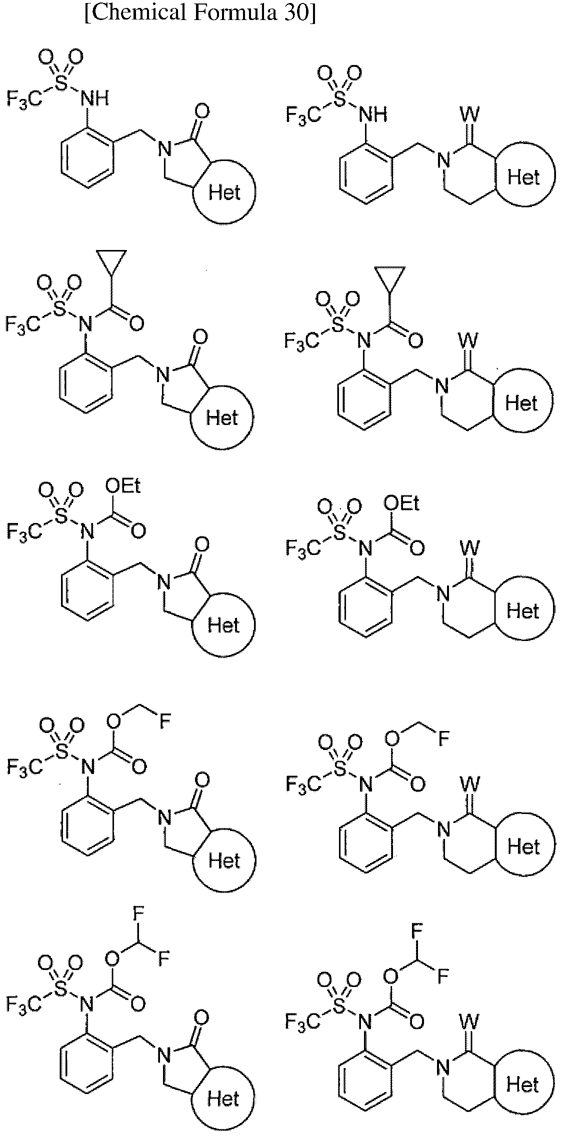

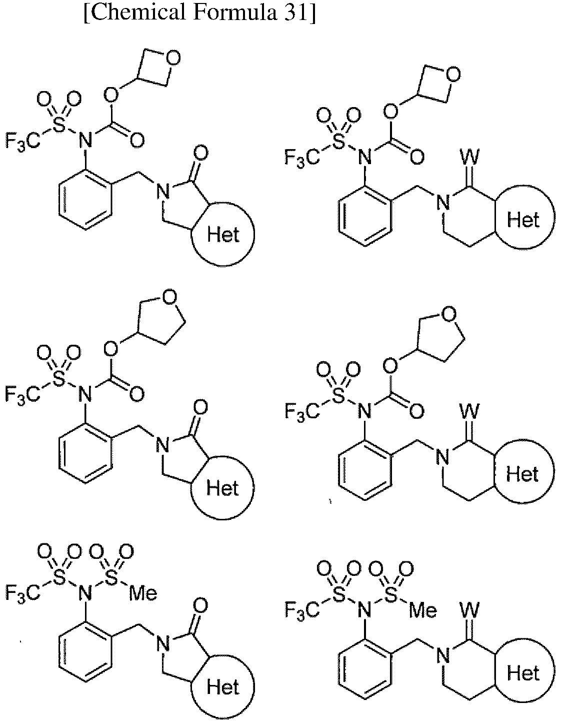

**[Table 156]**

| Het-No. | Het X⁵ | X⁶ | Het-No. | Het X⁵ | X⁶ |
|---|---|---|---|---|---|
| Het-19 | Me | H | Het-20 | F | Me |
| Het-19 | F | H | Het-20 | F | F |
| Het-19 | Cl | H | Het-20 | F | Cl |
| Het-19 | OSO₂Me | H | Het-20 | F | F |
| Het-19 | CN | H | Het-21 | Me | H |
| Het-19 | NO₂ | H | Het-21 | F | H |
| Het-19 | H | Me | Het-21 | Cl | H |
| Het-19 | H | F | Het-21 | OSO₂Me | H |
| Het-19 | H | Cl | Het-21 | CN | H |
| Het-19 | H | OSO₂Me | Het-21 | NO₂ | H |
| Het-19 | H | CN | Het-21 | H | Me |
| Het-19 | H | NO₂ | Het-21 | H | F |
| Het-19 | H | Ph | Het-21 | H | Cl |
| Het-19 | F | Me | Het-21 | H | OSO₂Me |
| Het-19 | F | F | Het-21 | H | CN |
| Het-19 | F | Cl | Het-21 | H | NO₂ |
| Het-19 | Me | F | Het-21 | H | Ph |
| Het-19 | F | OSO₂Me | Het-21 | F | Me |
| Het-19 | Cl | F | Het-21 | F | F |
| Het-19 | Cl | Cl | Het-21 | F | Cl |
| Het-20 | Me | H | Het-21 | Me | F |
| Het-20 | F | H | Het-21 | Cl | F |
| Het-20 | Cl | H | Het-21 | Cl | Cl |
| Het-20 | OSO₂Me | H | Het-21 | Me | Me |
| Het-20 | CN | H | Het-22 | Me | H |
| Het-20 | NO₂ | H | Het-22 | F | H |
| Het-20 | Ph | H | Het-22 | Cl | H |

**[Table 157]**

| Het-No. | Het X⁵ | X⁶ | Het-No. | Het X⁵ | X⁶ |
|---|---|---|---|---|---|
| Het-22 | OSO₂Me | H | Het-23 | F | F |
| Het-22 | CN | H | Het-24 | Me | H |
| Het-22 | NO₂ | H | Het-24 | F | H |
| Het-22 | Ph | H | Het-24 | Cl | H |
| Het-22 | F | Me | Het-24 | OSO₂Me | H |
| Het-22 | F | F | Het-24 | CN | H |
| Het-22 | F | Cl | Het-24 | NO₂ | H |
| Het-22 | F | F | Het-24 | Ph | H |
| Het-23 | Me | H | Het-24 | F | Me |
| Het-23 | F | H | Het-24 | F | F |
| Het-23 | Cl | H | Het-24 | F | Cl |
| Het-23 | OSO₂Me | H | Het-24 | Cl | Cl |
| Het-23 | CN | H | Het-25 | Me | - |
| Het-23 | NO₂ | H | Het-25 | F | - |
| Het-23 | H | Me | Het-25 | Cl | - |
| Het-23 | H | F | Het-25 | OSO₂Me | - |
| Het-23 | H | Cl | Het-25 | CN | - |
| Het-23 | H | OSO₂Me | Het-25 | NO₂ | - |
| Het-23 | H | CN | Het-25 | Ph | - |
| Het-23 | H | NO₂ | Het-26 | Me | - |
| Het-23 | H | Ph | Het-26 | F | - |
| Het-23 | F | Me | Het-26 | Cl | - |
| Het-23 | F | F | Het-26 | OSO₂Me | - |
| Het-23 | F | Cl | Het-26 | CN | - |
| Het-23 | Me | F | Het-26 | NO₂ | - |
| Het-23 | Cl | Cl | Het-27 | - | - |
| Het-23 | Cl | F | Het-28 | Me | - |

**[Table 158]**

| Het-No. | Het X⁵ | X⁶ | Het-No. | Het X⁵ | X⁶ |
|---|---|---|---|---|---|
| Het-28 | F | - | Het-31 | OSO₂Me | - |
| Het-28 | Cl | - | Het-31 | CN | - |
| Het-28 | OSO₂Me | - | Het-31 | NO₂ | - |
| Het-28 | CN | - | Het-32 | Me | - |
| Het-28 | NO₂ | - | Het-32 | F | - |
| Het-29 | Me | - | Het-32 | Cl | - |
| Het-29 | F | - | Het-32 | OSO₂Me | - |
| Het-29 | Cl | - | Het-32 | CN | - |
| Het-29 | OSO₂Me | - | Het-32 | NO₂ | - |
| Het-29 | CN | - | Het-32 | Ph | - |
| Het-29 | NO₂ | - | Het-33 | Me | - |
| Het-29 | Ph | - | Het-33 | F | - |
| Het-30 | Me | - | Het-33 | Cl | - |
| Het-30 | F | - | Het-33 | OSO₂Me | - |
| Het-30 | Cl | - | Het-33 | CN | - |
| Het-30 | OSO₂Me | - | Het-33 | NO₂ | - |
| Het-30 | CN | - | Het-33 | Ph | - |
| Het-30 | NO₂ | - | Het-34 | - | - |
| Het-30 | Ph | - | Het-35 | - | - |
| Het-31 | Me | - | Het-36 | - | - |
| Het-31 | F | - | Het-37 | - | - |
| Het-31 | Cl | - | | | |

The herbicides and agrochemical compositions of the present invention are characterized in that they contain a compound represented by Formula [1] of the present invention or an agrochemically acceptable salt thereof as an active ingredient. In addition, the present invention also relates to an agrochemical composition, more particularly a herbicidal composition, comprising one or two or more compounds represented by Formula [1] of the present invention or an agrochemically acceptable salt thereof, and a carrier acceptable for agrochemical formulations.

The compound represented by Formula [1] according to the present invention or the herbicidal composition according to the present invention can be used in fields, paddy fields, or orchards by means of a foliar spray, soil application, or water surface application, etc. The herbicide of the present invention can also be used for the control of general weeds in fallow fields, ridges, farm roads, waterways, pasture land, cemeteries, parks, roads, playgrounds, vacant land around buildings, cultivated land, railroad track edges, forests, etc. Namely, the present invention also relates to a method of using a compound represented by Formula [1] according to the present invention or a herbicidal composition according to the present invention for controlling weeds in useful plants and useful crops.

The compound represented by formula [1] according to the present invention and the herbicidal composition according to the present invention have extremely potent herbicidal properties.

The following are examples of weeds that can be treated with the compound represented by Formula [1] according to the present invention or a herbicidal composition according to the present invention, but are not necessarily limited thereto.

Excellent herbicidal effects are exerted over a wide range of pre-germination and growing seasons on various problematic weeds in the fields, for example, polygonaceous plants such as *Persicaria lapathifolia* (L.) Delarbre, *Persicaria longiseta,* and *Rumex japonicus, Amaranthaceae* such as *Amaranthus viridis, Amaranthus palmeri* S. Wats., and *Amaranthus retroflexus;* broadleaf weeds such as *Solanum carolinense, Solanum nigrum, Chenopodium album, Abutilon theophrasti, Sida spinosa, Sesbania exaltata, Ambrosia artemisiifolia, Papaver rhoeas, Ipomoea nil, Xanthium strumarium, Stellaria media Stellaria, Matricaria chamomilla L., Galium spurium, Viola mandshurica, Veronica persica, Veronica hederifolia, Lamium amplexicaule, Vicia sativa, Senecio vulgaris, Capsella bursa-pastoris;* perennial and annual *Cyperaceae* weeds such as *Cyperus rotundus, Cyperus esculentus L., Kyllinga brevifolia* Rottb. var. *leiolepis, Cyperus microiria Cyperus,* and *Cyperus iria; Poaceae* weeds such as *Echinochloa esculenta, Digitaria ciliaris, Setaria viridis, Poa annua, Alopecurus aequalis, Sorghum halepense, Alopecurus myosuroides, Lolium multiflorum,* and wild *Avena sativa.* It is also possible to control paddy field annual weeds such as *Echinochloa crus-galli* var. *oryzicola, Echinochloa crus-galli* var. *caudata, Cyperus difformis, Leptochloa chinensis, Monochoria vaginalis, Lindernia dubia Pennell, Lindernia procumbens, Rotala indica, Vandellia micrantha, Limnophila sessiliflora, Ammannia multiflora, Elatine triandra, Monochoria korsakowii, Ludwigia epilobioides, Eclipta prostrata, Bidens frondosa, Aeschynomene indica,* and *Murdannia keisak;* and perennial weeds such as *Sagittaria pygmaea, Sagittaria trifolia, Cyperus serotinus, Eleocharis kuroguwai Ohwi, Schoenoplectiella hotarui, Alisma canaliculatum, Schoenoplectus nipponicus, Bolboschoenus maritimus* (L.) Palla, *Potamogeton distinctus, Leersia japonica, Paspalum distichum, Leersia oryzoides* (L.) Sw., and *Eleocharis acicularis.*

Further, the compound represented by Formula [1] according to the present invention or the herbicidal composition according to the present invention is highly safe for useful plants and useful crops, and is highly safe for, for example, crops such as rice, wheat, barley, oats, rye, millet, common millet, corn, and grain sorghum, soybean, cotton, sugar beet, sugarcane, onion, sunflower, rape seed, peanut, linseed, tobacco, coffee, sweet potato, potato, tomato, and other vegetable crops or grasses.

The useful plants and useful crops herein include so-called genetically modified crops such as corn, soybean, cotton, rapeseed, and sugarcane, which have been transformed by genetic technology to have resistance to herbicides, pests, diseases, etc., and plants having resistance to herbicides, pests, diseases, etc., through breeding and selection. Examples of the genetically modified plants are given here, but are not necessarily limited to them.

Examples of the plants transformed to show resistance to herbicides include glyphosate-resistant plants, bialaphos-resistant plants, bromoxynil-resistant plants, sulfonylurea-based herbicide-resistant plants, imidazolinone-based herbicide-resistant plants, 2,4-D-resistant plants, dicamba-resistant plants, isoxaflutole-resistant plants, and mesotrione-resistant plants.

Examples of the plants transformed to show resistance to pests include plants transformed to produce Bt toxin (insecticidal toxin of *Bacillus thuringiensis*) and plants transformed to produce natural enemy attractants.

Examples of the plants transformed to show resistance to plant diseases include virus-resistant plants and plants transformed to produce defensins. Examples of the plants transformed to increase fruit harvest period and shelf life include plants transformed to inhibit polygalacturonase production and plants transformed to inhibit ethylene biosynthetic enzymes.

Examples of the plants transformed to increase the safety of the harvest include mycotoxin-degrading enzyme-producing plants.

Examples of the plants transformed to be useful for breeding include plants transformed to exhibit a male sterility trait.

Examples of the transgenic plants with traits useful as feedstock for bioethanol include heat-resistant α-amylase-producing plants.

Examples of the plants transformed to show tolerance to environmental stresses include plants tolerant to drought while using RNA chaperones, plants accumulating glycine betaine, a compatible solute abundant in low-temperature resistant plants, plants accumulating proline, a compatible solute, plants accumulating trehalose with high water retention capacity and tolerant to drought, plants overproducing ROS scavenger enzymes, plants tolerant to iron deficiency in alkaline soil by producing mugineic acids, and plants tolerant to iron deficiency by producing mugineic acids.

Examples of the plants transformed to produce specific functional nutrients include plants overproducing oleic acid, plants overproducing stearidonic acid, plants overproducing lysine, provitamin A-fortified crops, vitamin E-fortified plants, plants overproducing anthocyanins, and plants producing cedar allergens, thereby exerting an effect of relieving cedar pollinosis.

To control weeds, the herbicidal composition according to the present invention can be used by adjusting the amount of the compound represented by Formula [1] of the present invention to be effective against weeds but not toxic to useful plants and useful crops. Here, the amount that is effective but not toxic to useful plants and useful crops is the amount that can sufficiently control weeds and does not harm useful plants and useful crops. This amount can vary over a relatively wide range depending on the weeds to be controlled, the plant to be applied, the natural environment in which the compound is used, and the ingredients of the composition in the present invention.

The compound represented by Formula [1] according to the present invention or the herbicidal composition according to the present invention can be applied to all plants or parts of plants, as well as to the soil surrounding plants, or to soil in which seeds are sown, paddy fields, hydroponic water, and cultivation materials by, for instance, spraying, dispersing, powdering, spray, diffusing, impregnating, irrigating, injecting, sprinkling (soaking), foaming, applying, dust coating, coating, powdering, fumigating, smothering, smoking, and painting. All plants herein refer to plants or a plant group such as wild plants, bred plants, naturally occurring plants, and cultivated plants. Examples also include plants produced by breeding such as introduction breeding, segregation breeding, crossbreeding, heterosis breeding, mutation breeding, ploidy breeding, genetic recombinant (transgenic) breeding, or marker-assisted selection.

When the compound represented by Formula [1] according to the present invention or the herbicidal composition according to the present invention is used for treatment to control weeds, the treatment can be carried out, before and/or after weed emergence, throughout the growth period and before growth of useful plants and useful crops. The part of the plant herein means, for instance, the leaf, stem, trunk, branch, flower, fruit body, fruit, seed, root, tuber, or rhizome of the plant or all the parts constituting the plant or any combination thereof.

The herbicidal composition according to the present invention may contain additives normally used in agrochemical formulations if necessary. Examples of the additives include carriers such as solid carriers and liquid carriers, surfactants, binders, adhesive agents, thickeners, coloring agents, spreaders, anti-freezing agents, anti-caking agents, disintegrants, and anti-degradation agents. Other additive ingredients such as preservatives and plant fragments may be used as necessary.

One kind of the additives may be used singly, or two or more kinds thereof may be used in combination.

The above additive components will be described below.

Examples of the solid carriers include: natural minerals such as quartz, clay, kaolinite, pyrophyllite, sericite, talc, chalk, bentonite, attapulgite, montmorillonite, acid leucite, attapulgite, zeolite, natural rock, diatomaceous earth, calcite, marble, pumice, seafoam stone, and dolomite; inorganic salts such as calcium carbonate, ammonium sulfate or other ammonium salts, sodium sulfate, calcium chloride, and potassium chloride; organic solid carriers such as synthetic silicic acid, synthetic silicates, alumina, micronized silica, silicate, starch, cellulose, and plant powders; and plastic carriers such as polyethylene, polypropylene, and polyvinylidene chloride. They may be used singly, or two or more kinds thereof may be used in combination.

Examples of the liquid carriers include: alcohols broadly classified into monovalent alcohols such as methanol, ethanol, propanol, and i-propanol, butanol, or polyvalent alcohols such as ethylene glycol, diethylene glycol, propylene glycol, hexylene glycol, polyethylene glycol, polypropylene glycol, and glycerin; polyhydric alcohol derivatives such as propylene-based glycol ethers; ketones such as acetone, methyl ethyl ketone, methyl isobutyl ketone, diisobutyl ketone, cyclohexanone, and isophorone; ethers such as ethyl ether, dioxane, cellosolve, dipropyl ether, and tetrahydrofuran; aliphatic hydrocarbons such as normal paraffin, naphthene, isoparaffin, kerosene, and mineral oil; aromatic hydrocarbons such as benzene, toluene, xylene, solvent naphtha, and alkyl naphthalene; halogenated hydrocarbons such as dichloroethane, chloroform, and carbon tetrachloride; esters such as ethyl acetate, diisopropyl phthalate, dibutyl phthalate, dioctyl phthalate, and dimethyl adipate; lactones such as γ-butyrolactone; amides such as dimethyl formamide, diethyl formamide, dimethylacetamide, and N-alkyl pyrrolidine; nitriles such as acetonitrile; sulfur compounds such as dimethyl sulfoxide; vegetable oils such as soybean oil, rapeseed oil, cottonseed oil, and castor oil; and water. They may be used singly, or two or more kinds thereof may be used in combination.

Examples of the surfactants include, but are not limited to, nonionic surfactants such as sorbitan fatty acid ester, polyoxyethylene sorbitan fatty acid ester, sucrose fatty acid ester, polyoxyethylene fatty acid ester, polyoxyethylene resin acid ester, polyoxyethylene fatty acid diester, polyoxyethylene alkyl ether, polyoxyethylene alkyl phenol ether, polyoxyethylene dialkyl phenyl ether, polyoxyethylene alkyl phenol formalin condensate, polyoxyethylene polyoxypropylene block polymer, alkyl polyoxyethylene polypropylene block polymer ether, polyoxyethylene alkylamine, polyoxyethylene fatty acid amide, polyoxyethylene fatty acid bisphenyl ether, polyalkylene benzyl phenyl ether, polyoxyalkylene styrene phenyl ether, acetylenediol, polyoxyalkylene-added acetylenediol, polyoxyethylene ether-type silicone, ester-type silicone, fluorosurfactant, polyoxyethylene castor oil, and polyoxyethylene hardened castor oil; anionic surfactants such as an alkyl sulfate, polyoxyethylene alkyl ether sulfate, polyoxyethylene alkyl phenyl ether sulfate, alkyl benzene sulfonate, lignin sulfonate, alkylsulfosuccinate, naphthalene sulfonate, alkyl naphthalene sulfonate, formalin condensed salt of naphthalene sulfonic acid, formalin condensed salt of alkyl naphthalene sulfonic acid, fatty acid salt, polycarboxylate, N-methyl-fatty acid sarcosinate, resin acid salt, polyoxyethylene alkyl ether phosphate, and polyoxyethylene alkyl phenyl ether phosphate; cationic surfactants such as laurylamine hydrochloride, stearylamine hydrochloride, oleylamine hydrochloride, stearylamine acetate, stearylaminopropylamine hydrochloride, and alkyl amine salts such as alkyl trimethylammonium chloride and alkyl dimethyl benzalkonium chloride; and amphoteric surfactants such as an amino acid-type or betaine-type one. One kind of the surfactants may be used singly, or two or more kinds thereof may be used in combination.

In addition, examples of the binders or adhesive agents include carboxymethylcellulose and its salt, dextrin, water-soluble starch, xanthan gum, guar gum, sucrose, polyvinylpyrrolidone, gum Arabic, polyvinyl alcohol, polyvinyl acetate, sodium polyacrylate, polyethylene glycol with an average molecular weight of 6,000 to 20,000, polyethylene oxide with an average molecular weight of 100,000 to 5,000,000, and natural phospholipid (e.g., cephalic acid, lecithinic acid). One kind of the binder or adhesive agent may be used singly, or two or more kinds thereof may be used in combination. Examples of the thickeners include water-soluble polymers such as xanthan gum, guar gum, carboxymethylcellulose, polyvinylpyrrolidone, a carboxyvinyl polymer, an acrylic polymer, a starch derivative, and polysaccharides; or inorganic micropowder such as high-purity bentonite and white carbon. One kind of the thickeners may be used singly, or two or more kinds thereof may be used in combination. Examples of the coloring agents include: inorganic pigments such as iron oxide, titanium oxide, and prussian blue; and organic dyes such as alizarin dye, azo dye, and metal phthalocyanine dye. One kind of the coloring agents may be used singly, or two or more kinds thereof may be used in combination.

Examples of the spreading agents include silicone-based surfactants, cellulose powder, dextrin, processed starch, polyaminocarboxylic acid chelators, cross-linked polyvinylpyrrolidone, copolymers of maleic acid, styrenic acid, and methacrylic acid, half esters of polymers of polyhydric alcohols and dicarboxylic anhydrides, and water-soluble salts of polystyrene sulfonic acid. One kind of the spreading agents may be used singly, or two or more kinds thereof may be used in combination. Examples of the spreaders include various surfactants such as sodium dialkyl sulfosuccinate, polyoxyethylene alkyl ether, polyoxyethylene alkyl phenyl ether, and polyoxyethylene fatty acid ester; and paraffin, terpene, polyamide resin, polyacrylate, polyoxyethylene, wax, polyvinyl alkyl ether, alkyl phenol formalin condensation product, and synthetic resin emulsion. One kind of the spreaders may be used singly, or two or more kinds thereof may be used in combination.

Examples of the anti-freezing agents include polyhydric alcohols such as ethylene glycol, diethylene glycol, propylene glycol, and glycerin. One kind of the anti-freezing agents may be used singly, or two or more kinds thereof may be used in combination.

Examples of the anti-caking agents include polysaccharides such as starch, alginate, mannose, and galactose, polyvinylpyrrolidone, white carbon, ester gum, and petroleum resin. One kind of the anti-caking agents may be used singly, or two or more kinds thereof may be used in combination. Examples of the disintegrants include sodium tripolyphosphate, sodium hexanemetaphosphate, metallic stearate, cellulose powder, dextrin, copolymers of methacrylates, polyvinylpyrrolidone, polyaminocarboxylic acid chelators, sulfonated styrene-isobutylene-maleic anhydride copolymers, and starch-polyacrylonitrile graft copolymers. One kind of the disintegrants may be used singly, or two or more kinds thereof may be used in combination.

Examples of the anti-degradation agents include desiccants such as zeolite, quicklime, and magnesium oxide; antioxidants such as a phenol-based, amine-based, sulfur-based, and phosphoric acid-based antioxidant; and UV absorbers such as a salicylic acid-based UV absorber and a benzophenone-based UV absorber. One kind of the anti-degradation agents may be used singly, or two or more kinds thereof may be used in combination.

Examples of the preservatives include potassium sorbate and 1,2-benzthiazolin-3-one. One kind of the preservatives may be used singly, or two or more kinds thereof may be used in combination.

Examples of the plant fragments include sawdust, palm kernels, corn cobs, and tobacco stalks.

When the above additive components is contained in the microbicide and agrochemical composition of the present invention, the content ratio is usually selected in the range of 5 to 95% and preferably 20 to 90% for the carrier, 0.1 to 30% and preferably 0.5 to 10% for the surfactant, and 0.1 to 30% and preferably 0.5 to 10% for the other additives, on a mass basis.

The herbicide of the present invention can be used as an agent suitable for herbicides such as liquid formulation, emulsifiable concentrate, wettable powder, dusting powder, oil solution, water dispersible granule, flowable, suspension concentrate, granule, jumbo agent, suspoemulsion, and "Mametubu" (trademark registered) agent.

Such a form can be obtained by the conventional method of mixing at least one of the compounds of the present invention with suitable solid or liquid carriers and, optionally, with suitable auxiliary agents (e.g., surfactants, solvents, stabilizers) to improve dispersion of the active ingredient or other properties.

When used, it should be diluted to an appropriate concentration and sprayed or applied directly.

The herbicidal composition of the present invention may contain one or two or more of the compound represented by Formula [1] or salt thereof according to the present invention.

The blending ratio of the present compound represented by Formula [1] in the herbicidal composition of the present invention may be optionally selected, and may be selected, if appropriate, from the range of 0.001 to 50% (by weight) and preferably 0.005 to 30% (by weight) in the case of dusting powder or granule. In the case of emulsifiable concentrate or wettable powder, for example, the blending ratio may be selected, if appropriate, from the range of 1 to 50% (by weight) and preferably 5 to 30% (by weight). Also, in the case of flowable, for example, the blending ratio may be selected, if appropriate, from the range of 1 to 50% (by weight) and preferably 2 to 30% (by weight).

The amount of the present herbicidal composition applied varies depending on the type of compound used, the target crop, the target disease, the tendency of occurrence, environmental conditions, and the dosage form used. However, when used as it is as dusting powder or granule, the active ingredient application amount should be selected from 1 g to 50 kg and preferably from 10 g to 10 kg per hectare. When used in liquid form, such as in emulsifiable concentrate, wettable powder, or flowable, the application amount should be selected from 0.1 to 50,000 ppm and preferably from 10 to 10,000 ppm.

### EXAMPLES

Those skilled in the art will recognize that various electrophilic, nucleophilic, radical, organometallic, oxidative, and/or reductive reactions can be performed on the compounds represented by Formula [1] and intermediates described herein, and substituents may be added or existing substituents may be modified.

It is believed that those skilled in the art using the above description can make full use of the present invention without further detailed explanation. Therefore, the following Examples are to be construed as merely illustrative and do not limit the present disclosure in any way. The steps in the following Examples illustrate the procedure of each step in the overall synthesis scheme. The starting material(s) for each step does not necessarily have to be prepared by the implementation of a specific preparation described in the procedure of other example(s) or step(s).

Note that in the following descriptions, "%" indicates percentage by weight and "parts" indicates parts by weight.

### [Example 1] Preparation of N-[2-(N-trifluoromethanesulfonyl)aminobenzyl]-7-azabicyclo[4.2.0]octa-1(6),2,4-trien-8-one (Compound 1-1)

### Step 1: Preparation of t-butyl[2-[(2-aminobenzamido)methyl]phenyl]carbamate

Isatoic anhydride (326 mg) and benzylamine (450 mg) were dissolved in tetrahydrofuran (20 mL) and the mixture was stirred at 60°C for 8 hours. The reaction solution was concentrated and the residue was then purified by silica gel flash chromatography (ethyl acetate-hexane: 15%-60% elution) using an automated flash purification system (Biotage AB/Isolera^{™}) to afford the title compound as a colorless resinous solid (378 mg, yield: 55%).

### Step 2: Preparation of N-[2-[N-(1,1-dimethyl-ethyl)oxycarbonyl]aminobenzyl]-7-azabicyclo[4.2.0]octa-1(6),2,4-trien-8-one

The compound (370 mg) obtained in step 1 of Example 1 above was dissolved in acetonitrile (15 mL), t-butyl nitrite (189 mg) and acetic acid (165 mg) were added, and the mixture was stirred at room temperature for 3 hours. Water was added to the reaction solution, and after stirring, the precipitated solid was filtered, washed with water, and then air-dried to afford the title compound as a colorless resinous solid (320 mg, yield: 90%).

### Step 3: Preparation of N-(2-aminobenzyl)-7-azabicyclo[4.2.0]octa-1(6),2,4-triene-8-one

The compound (320 mg) obtained in step 2 of Example 1 above was dissolved in dioxane (20 mL), 4-N hydrochloric acid dioxane solution (5 mL) was added, and the mixture was then stirred at room temperature for 20 hours. The precipitated solid was filtered, washed with ethyl acetate, and then air-dried to afford the title compound as a colorless resinous solid (200 mg, yield: 77%).

### Step 4: Preparation of N-[2-(N-trifluoromethanesulfonyl)aminobenzyl]-7-azabicyclo[4.2.0]octa-1(6),2,4-trien-8-one

The compound (200 mg) obtained in step 3 of Example 1 above was dissolved in dichloromethane (20 mL), triethylamine (162 mg) and trifluoromethanesulfonic anhydride (226 mg) were added on an ice water bath, and the mixture was then stirred at room temperature for 20 hours. The reaction solution was concentrated, dissolved in ethyl acetate, and then washed with 1-N aqueous hydrochloric acid solution. The organic layer was dried over anhydrous sodium sulfate, the inorganic material was filtered off, and the solvent was then distilled away under reduced pressure. The residue was then purified by silica gel flash chromatography (ethyl acetate-hexane: 10%-30% elution) using an automated flash purification system (Biotage AB/Isolera^{™}) to afford the title compound as a colorless resinous solid (148 mg, yield: 52%).

### [Example 2] Preparation of N-[2-(N-trifluoromethanesulfonyl)aminobenzyl]-7-chloro-1,3-dihydroisoindol-1-one (compound 2-3)

### Step 1: Preparation of methyl 2-bromomethyl-6-chlorobenzoate

Methyl 2-chloro-6-methylbenzoate (1.4 g) (compound described in WO2010/108651), N-bromosuccinimide (1.42 g), and benzoyl peroxide (122 mg, 75%) were dissolved in carbon tetrachloride (25 mL) and heated to reflux for 16 hours. The reaction solution was cooled and then filtered through Celite, and the solvent was distilled away under reduced pressure. The residue was then purified by silica gel flash chromatography (ethyl acetate-hexane: 5%-15% elution) using an automated flash purification system (Biotage AB/Isolera^{™}) to afford the title compound as a colorless oily substance (1.49 g, yield: 75%).

### Step 2: Preparation of 7-chloro-1,3-dihydroisoindol-1-one

The compound (1.47 g) obtained in step 1 of Example 2 above was dissolved in tetrahydrofuran (40 mL), ammonia water (20 mL) was added, and the mixture was stirred overnight at room temperature. The reaction solution was admixed with water and then extracted with dichloromethane. The organic layer was dried over anhydrous sodium sulfate, the inorganic material was filtered off, and the solvent was then distilled away under reduced pressure. Dichloromethane and hexane were added to the residue, and the precipitated solid was filtered and air-dried to afford the title compound as a pale yellow solid (820 mg, yield: 88%).

### Step 3: Preparation of N-[2-(N-trifluoromethanesulfonyl)aminobenzyl]-7-chloro-1,3-dihydroisoindol-1-one

The compound (90 mg) obtained in step 2 of Example 2, 1,1,1-trifluoro-N-[2-(hydroxymethyl)phenyl]methanesulfonamide (115 mg) (compound listed in WO2010/026989), and paratoluenesulfonic acid monohydrate (49 mg) were dissolved in ortho-dichlorobenzene (5 mL). The mixture was heated and stirred at 250°C for 5 minutes by using a microwave synthesizer (Biotage AB/Initiator+^{™}). After the reaction solution was cooled to room temperature, the reaction solution was subjected to purification by silica gel flash chromatography (ethyl acetate-hexane: 15%-60% elution) using an automated flash purification system (Biotage AB/Isolera^{™}) to afford the title compound as a pale yellow resinous solid (158 mg, yield: 77%).

### [Example 3] Preparation of N-[2-(N-trifluoromethanesulfonyl)aminobenzyl]-5-chloro-8-fluoro-3,4-dihydroisoquinolin-1(2H)-one (compound 3-64)

### Step 1: Preparation of 5-chloro-8-fluoro-3,4-dihydroisoquinolin-1(2H)-one

2-Chloro-5-fluorophenethylamine (900 mg) (compound described in WO2006/009054) and triethylamine (787 mg) were dissolved in dichloromethane (20 mL), and methyl chloroformate (712 mg) was added dropwise on an ice water bath. The mixture was stirred at room temperature overnight. The reaction solution was admixed with water and then extracted with dichloromethane. The organic layer was dried over anhydrous sodium sulfate, the inorganic material was filtered off, and the solvent was then distilled away under reduced pressure. The residue was dissolved in trifluoromethanesulfonic acid (5 mL) and heated and stirred at 160°C for 5 minutes by using a microwave synthesizer (Biotage AB/Initiator+^{™}). The reaction solution was admixed with ice water and then extracted with dichloromethane. The organic layer was dried over anhydrous sodium sulfate, the inorganic material was filtered off, and the solvent was then distilled away under reduced pressure. The residue was then purified by silica gel flash chromatography (ethyl acetate-hexane: 30%-60% elution) using an automated flash purification system (Biotage AB/Isolera^{™}) to afford the title compound as a brown oily substance (290 mg, yield: 28%).

### Step 2: Preparation of N-[2-(N-trifluoromethanesulfonyl)aminobenzyl]-5-chloro-8-fluoro-3,4-dihydroisoquinolin-1(2H)-one

The compound (290 mg) obtained in step 1 of Example 3, 1,1,1-trifluoro-N-[2-(hydroxymethyl)phenyl]methanesulfonamide (327 mg) (compound listed in WO2010/026989), and paratoluenesulfonic acid monohydrate (138 mg) were dissolved in ortho-dichlorobenzene (5 mL). The mixture was heated and stirred at 250°C for 5 minutes by using a microwave synthesizer (Biotage AB/Initiator+^{™}). After the reaction solution was cooled to room temperature, the reaction solution was subjected to purification by silica gel flash chromatography (ethyl acetate-hexane: 20%-70% elution) using an automated flash purification system (Biotage AB/Isolera^{™}) to afford the title compound as a brown resinous solid (91 mg, yield: 14%).

### [Example 4] Preparation of N-[2-(N-ethoxycarbonyl-N-trifluoromethanesulfonyl)aminobenzyl]-5,8-difluoro-3,4-dihydroisoquinolin-1(2H)-one (compound 3-149)

### Step 1: Preparation of dimethyl 2,2'-[carbonyl bis(oxy)]dibenzoate

Methyl salicylate (38.16 g) and triethylamine (64 mL) were dissolved in tetrahydrofuran (120 mL), and a solution of triphosgene (12.0 g) in dichloromethane (120 mL) was added dropwise on an ice water bath. The mixture was then stirred overnight at room temperature. The reaction solution was admixed with 0.5-N aqueous hydrochloric acid solution, and the mixture was extracted with dichloromethane. The extracted solution was admixed with sodium sulfate, dried, and filtered. The organic solvent was then distilled away under reduced pressure. Dichloromethane and hexane were added to the residue, and the precipitated solid was filtered and air-dried to afford the title compound as a colorless solid (33.9 g, yield: 85%).

### Step 2: Preparation of methyl 2-[[(2,5-difluorophenethyl)carbamoyl]oxy]benzoate

The compound (20.9 g) obtained in step 1 of Example 4 and 2,5-difluorophenethylamine (9.9 g) (compound described in WO2000/073283) were dissolved in tetrahydrofuran (210 mL), and the mixture was stirred at room temperature overnight. The reaction solution was concentrated and the residue was then purified by silica gel flash chromatography (ethyl acetate-hexane: 5%-30% elution) using an automated flash purification system (Biotage AB/Isolera^{™}) to afford the title compound as a colorless solid (14.4 g, yield: 68%).

### Step 3: Preparation of 5,8-difluoro-3,4-dihydroisoquinolin-1(2H)-one

The compound (14.4 g) obtained in step 2 of Example 4 above was dissolved in dichloromethane (287 mL), trifluoromethanesulfonic acid (50.6 mL) was added dropwise on an ice water bath, and the mixture was then stirred at room temperature for 2 hours. The reaction solution was gradually added to an ice water bath, and the aqueous layer was then extracted with dichloromethane. The organic layer was dried over magnesium sulfate, the inorganic material was filtered off, and the solvent was then distilled away under reduced pressure. The residue was washed with diethyl ether, filtered, and air-dried to afford the title compound as a colorless solid (3.41 g, yield: 43%).

### Step 4: Preparation of N-(2-nitrobenzyl)-5,8-difluoro-3,4-dihydroisoquinolin-1(2H)-one

The compound (2.18 g) obtained in step 3 of Example 4 above was dissolved in N,N-dimethylformamide (15 mL), and sodium hydride (499.8 mg, 60%) was added on an ice water bath. The mixture was then stirred at room temperature for 15 minutes. The mixture was re-cooled in an ice water bath, a solution of 2-nitrobenzylbromide (3.86 g) in N,N-dimethylformamide (10 mL) was added dropwise, and the mixture was then stirred overnight at room temperature. The reaction solution was admixed with water and 1-N aqueous hydrochloric acid solution, and was extracted with ethyl acetate. The extracted solution was washed with saturated brine and dried over sodium sulfate. The inorganic material was filtered off, and the solvent was then distilled away under reduced pressure. Dichloromethane and hexane were added to the residue, and the precipitated solid was filtered and air-dried to afford the title compound as a pale yellow solid (3.08 g, yield: 81%).

### Step 5: Preparation of N-(2-aminobenzyl)-5,8-difluoro-3,4-dihydroisoquinolin-1(2H)-one

The compound (3.08 g) obtained in step 4 of Example 4 and palladium carbon (308 mg, 10%) were dissolved in ethyl acetate (39 mL). The mixture was stirred overnight at room temperature under a hydrogen atmosphere. The reaction solution was filtered through Celite and washed with ethyl acetate. The filtrate was distilled away under reduced pressure to afford the title compound as a colorless solid (2.77 g, yield: 99%).

### Step 6: Preparation of N-[2-(N-trifluoromethanesulfonyl)aminobenzyl]-5,8-difluoro-3,4-dihydroisoquinolin-1(2H)-one

The compound (250 mg) obtained in step 5 of Example 4 and triethylamine (132 mg) were dissolved in dichloromethane (8.7 mL), trifluoromethanesulfonic anhydride (265 mg) was added dropwise at -78°C. The temperature was gradually raised to room temperature, and the mixture was then stirred at room temperature overnight. The reaction solution was admixed with water and 1-N aqueous hydrochloric acid solution, and the mixture was extracted with dichloromethane. The organic layer was dried over anhydrous sodium sulfate, the inorganic material was filtered off, and the solvent was then distilled away under reduced pressure. The residue was then purified by silica gel flash chromatography (ethyl acetate-hexane: 5%-30% elution) using an automated flash purification system (Biotage AB/Isolera^{™}) to afford the title compound as a colorless resinous solid (256 m g, yield: 70%).

### Step 7: Preparation of N-[2-(N-ethoxycarbonyl-N-trifluoromethanesulfonyl)aminobenzyl]-5,8-difluoro-3,4-dihydroisoquinolin-1(2H)-one

The compound (173 mg) obtained in step 6 of Example 4, ethyl chloroformate (49 mg), and sodium bicarbonate (38 mg) were dissolved in acetonitrile (5 mL), and the mixture was heated to reflux for 8 hours. The reaction solution was admixed with water, and was extracted with ethyl acetate. The organic layer was dried over anhydrous sodium sulfate, the inorganic material was filtered off, and the solvent was then distilled away under reduced pressure. The residue was then purified by silica gel flash chromatography (ethyl acetate-hexane: 10%-30% elution) using an automated flash purification system (Biotage AB/Isolera^{™}) to afford the title compound as a colorless resinous solid (182 m g, yield: 90%).

### [Example 5] Preparation of N-[2-(N-ethoxycarbonyl-N-trifluoromethanesulfonyl)aminobenzyl]-8-fluoro-5-methyl-3,4-dihydroisoquinolin-1(2H)-one (compound 3-151)

### Step 1: Preparation of methyl (5-fluoro-2-methylphenethyl)carbamate

5-Fluoro-2-methylphenethylamine (1.62 g) (compound described in WO2005/090287) and triethylamine (1.6 g) were dissolved in dichloromethane (30 mL), methyl chloroformate (1.2 g) was added dropwise on an ice water bath, and the mixture was stirred at room temperature overnight. The reaction solution was admixed with water and then extracted with dichloromethane. The organic layer was dried over anhydrous sodium sulfate, the inorganic material was filtered off, and the solvent was then distilled away under reduced pressure. The residue was then purified by silica gel flash chromatography (ethyl acetate-hexane: 5%-20% elution) using an automated flash purification system (Biotage AB/Isolera^{™}) to afford the title compound as a brown oily substance (1.49 g, yield: 67%).

### Step 2: Preparation of 8-fluoro-5-methyl-3,4-dihydroisoquinolin-1(2H)-one

The compound (1.0 g) obtained in step 1 of Example 5 was dissolved in trifluoromethanesulfonic acid (5 mL), and the mixture was heated and stirred at 160°C for 8 minutes by using a microwave synthesizer (Biotage AB/Initiator+^{™}). The reaction solution was admixed with ice water and then extracted with dichloromethane. The organic layer was dried over anhydrous sodium sulfate, the inorganic material was filtered off, and the solvent was then distilled away under reduced pressure. The residue was then purified by silica gel flash chromatography (ethyl acetate-hexane: 30%-60% elution) using an automated flash purification system (Biotage AB/Isolera^{™}) to afford the title compound as a brown oily substance (800 mg, yield: 94%).

### Step 3: Preparation of N-[2-(N-trifluoromethanesulfonyl)aminobenzyl]-8-fluoro-5-methyl-3,4-dihydroisoquinolin-1(2H)-one

The compound (1.04 g) obtained in step 2 of Example 5, 1,1,1-trifluoro-N-[2-(hydroxymethyl)phenyl]methanesulfonamide (1.07 g) (compound listed in WO2010/026989), and paratoluenesulfonic acid monohydrate (90 mg) were dissolved in ortho-dichlorobenzene (5 mL). The mixture was heated and stirred at 250°C for 8 minutes by using a microwave synthesizer (Biotage AB/Initiator+^{™}). After the reaction solution was cooled to room temperature, the reaction solution was subjected to purification by silica gel flash chromatography (ethyl acetate-hexane: 15%-60% elution) using an automated flash purification system (Biotage AB/Isolera^{™}) to afford the title compound as a brown resinous solid (1.27 g, yield: 64%).

### Step 4: Preparation of N-[2-(N-ethoxycarbonyl-N-trifluoromethanesulfonyl)aminobenzyl]-8-fluoro-5-methyl-3,4-dihydroisoquinolin-1(2H)-one

The compound (724 mg) obtained in step 3 of Example 5, ethyl chloroformate (283 mg), and sodium bicarbonate (219 mg) were dissolved in acetonitrile (15 mL), and the mixture was heated to reflux for 8 hours. The reaction solution was admixed with water, and was extracted with ethyl acetate. The organic layer was dried over anhydrous sodium sulfate, the inorganic material was filtered off, and the solvent was then distilled away under reduced pressure. The residue was then purified by silica gel flash chromatography (ethyl acetate-hexane: 10%-30% elution) using an automated flash purification system (Biotage AB/Isolera^{™}) to afford the title compound as a pale yellow resinous solid (822 mg, yield: 97%).

### [Example 6] Preparation of N-[2-(N-ethoxycarbonyl-N-trifluoromethanesulfonyl)aminobenzyl]-8-fluoro-5-trifluoromethyl-3,4-dihydroisoquinolin-1(2H)-one (compound 3-152)

### Step 1: Preparation of 5-fluoro-2-trifluoromethylphenethylamine

First, 5-fluoro-2-trifluoromethylbenzeneacetonitrile (2.6 g) (compound described in WO2002/010143) and cobalt(II) chloride (2.49 g) were dissolved in methanol (65 mL). Sodium borohydride (3.63 g) was added gradually on an ice water bath, and the mixture was stirred at room temperature overnight. The reaction solution was filtered through Celite, and the solvent was removed under reduced pressure. The reaction solution was admixed with 1-N aqueous hydrochloric acid solution, and the mixture was washed with dichloromethane. The aqueous solution washed was admixed with aqueous sodium hydroxide, and the mixture was extracted with dichloromethane. The organic layer was dried over anhydrous sodium sulfate, the inorganic material was filtered off, and the solvent was distilled away under reduced pressure to afford the title compound as a brown oily substance (1.31 g, yield: 49%).

### Step 2: Preparation of methyl(5-fluoro-2-trifluoromethylphenethyl)carbamate

The compound (1.31 g) obtained in step 1 of Example 6 and triethylamine (1.03 g) were dissolved in dichloromethane (30 mL). Methyl chloroformate (769 mg) was added dropwise on an ice water bath, and the mixture was stirred at room temperature overnight. The reaction solution was admixed with water and then extracted with dichloromethane. The organic layer was dried over anhydrous sodium sulfate, the inorganic material was filtered off, and the solvent was then distilled away under reduced pressure. The residue was then purified by silica gel flash chromatography (ethyl acetate-hexane: 5%-20% elution) using an automated flash purification system (Biotage AB/Isolera^{™}) to afford the title compound as a yellow oily substance (1.17 g, yield: 65%).

### Step 3: Preparation of 8-fluoro-5-trifluoromethyl-3,4-dihydroisoquinolin-1(2H)-one

The compound (1.17 g) obtained in step 2 of Example 6 was dissolved in trifluoromethanesulfonic acid (5 mL), and the mixture was heated and stirred at 160°C for 8 minutes by using a microwave synthesizer (Biotage AB/Initiator+^{™}). The reaction solution was admixed with ice water and then extracted with dichloromethane. The organic layer was dried over anhydrous sodium sulfate, the inorganic material was filtered off, and the solvent was then distilled away under reduced pressure. The residue was then purified by silica gel flash chromatography (ethyl acetate-hexane: 30%-60% elution) using an automated flash purification system (Biotage AB/Isolera^{™}) to afford the title compound as a brown oily substance (918 mg, yield: 89%).

### Step 4: Preparation of N-[2-(N-trifluoromethanesulfonyl)aminobenzyl]-8-fluoro-5-trifluoromethyl-3,4-dihydroisoquinolin-1(2H)-one

The compound (918 mg) obtained in step 3 of Example 6, 1,1,1-trifluoro-N-[2-(hydroxymethyl)phenyl]methanesulfonamide (887 mg) (compound listed in WO2010/026989), and paratoluenesulfonic acid monohydrate (75 mg) were dissolved in ortho-dichlorobenzene (5 mL). The mixture was heated and stirred at 250°C for 8 minutes by using a microwave synthesizer (Biotage AB/Initiator+^{™}). After the reaction solution was cooled to room temperature, the reaction solution was subjected to purification by silica gel flash chromatography (ethyl acetate-hexane: 15%-50% elution) using an automated flash purification system (Biotage AB/Isolera^{™}) to afford the title compound as a brown resinous solid (213 mg, yield: 30%).

### Step 5: Preparation of N-[2-(N-ethoxycarbonyl-N-trifluoromethanesulfonyl)aminobenzyl]-8-fluoro-5-trifluoromethyl-3,4-dihydroisoquinolin-1(2H)-one

The compound (336 mg) obtained in step 4 of Example 6, ethyl chloroformate (93 mg), and sodium bicarbonate (72 mg) were dissolved in acetonitrile (20 mL), and the mixture was heated to reflux for 8 hours. The reaction solution was admixed with water, and was extracted with ethyl acetate. The organic layer was dried over anhydrous sodium sulfate, the inorganic material was filtered off, and the solvent was then distilled away under reduced pressure. The residue was then purified by silica gel flash chromatography (ethyl acetate-hexane: 15%-50% elution) using an automated flash purification system (Biotage AB/Isolera^{™}) to afford the title compound as a pale yellow resinous solid (367 mg, yield: 95%).

### [Example 7] Preparation of N-[2-(N-ethoxycarbonyl-N-trifluoromethanesulfonyl)aminobenzyl]-8-fluoro-5-methoxy-3,4-dihydroisoquinolin-1(2H)-one (compound 3-153)

### Step 1: Preparation of 5-fluoro-2-methoxyphenethylamine

First, 5-fluoro-2-methoxybenzeneacetonitrile (3.13 g) (compound described in WO2019/184955) and cobalt(II) chloride (3.69 g) were dissolved in methanol (95 mL). Sodium borohydride (5.38 g) was added gradually on an ice water bath, and the mixture was then stirred at room temperature overnight. The reaction solution was filtered through Celite, and the solvent was distilled away under reduced pressure. The reaction solution was admixed with 1-N aqueous hydrochloric acid solution, and the mixture was washed with dichloromethane. The aqueous solution washed was admixed with aqueous sodium hydroxide, and the mixture was extracted with dichloromethane. The organic layer was dried over anhydrous sodium sulfate, the inorganic material was filtered off, and the solvent was distilled away under reduced pressure to afford the title compound as a brown oily substance (1.82 g, yield: 57%).

### Step 2: Preparation of methyl(5-fluoro-2-methoxyphenethyl)carbamate

The compound (1.82 g) obtained in step 1 of Example 7 and triethylamine (1.22 g) were dissolved in dichloromethane (40 mL). Methyl chloroformate (1.22 g) was added dropwise on an ice water bath, and the mixture was then stirred overnight at room temperature. The reaction solution was admixed with water and then extracted with dichloromethane. The organic layer was dried over anhydrous sodium sulfate, the inorganic material was filtered off, and the solvent was then distilled away under reduced pressure. The residue was then purified by silica gel flash chromatography (ethyl acetate-hexane: 5%-20% elution) using an automated flash purification system (Biotage AB/Isolera^{™}) to afford the title compound as a brown oily substance (1.65 g, yield: 67%).

### Step 3: Preparation of 8-fluoro-5-methoxy-3,4-dihydroisoquinolin-1(2H)-one

The compound (700 mg) obtained in step 2 of Example 7 was dissolved in trifluoromethanesulfonic acid (5 mL), and the mixture was heated and stirred at 160°C for 8 minutes by using a microwave synthesizer (Biotage AB/Initiator+^{™}). The reaction solution was admixed with ice water and then extracted with dichloromethane. The organic layer was dried over anhydrous sodium sulfate, the inorganic material was filtered off, and the solvent was then distilled away under reduced pressure. The residue was then purified by silica gel flash chromatography (ethyl acetate-hexane: 30%-60% elution) using an automated flash purification system (Biotage AB/Isolera^{™}) to afford the title compound as a brown oily substance (115 mg, yield: 19%).

### Step 4: Preparation of N-[2-(N-trifluoromethanesulfonyl)aminobenzyl]-8-fluoro-5-methoxy-3,4-dihydroisoquinolin-1(2H)-one

The compound (115 mg) obtained in step 3 of Example 7, 1,1,1-trifluoro-N-[2-(hydroxymethyl)phenyl]methanesulfonamide (133 mg) (compound listed in WO2010/026989), and paratoluenesulfonic acid monohydrate (11 mg) were dissolved in ortho-dichlorobenzene (5 mL). The mixture was heated and stirred at 250°C for 8 minutes by using a microwave synthesizer (Biotage AB/Initiator+^{™}). After the reaction solution was cooled to room temperature, the reaction solution was subjected to purification by silica gel flash chromatography (ethyl acetate-hexane: 15%-50% elution) using an automated flash purification system (Biotage AB/Isolera^{™}) to afford the title compound as a pale yellow resinous solid (155 mg, yield: 61%).

### Step 5: Preparation of N-[2-(N-ethoxycarbonyl-N-trifluoromethanesulfonyl)aminobenzyl]-8-fluoro-5-methoxy-3,4-dihydroisoquinolin-1(2H)-one

The compound (110 mg) obtained in step 4 of Example 7, ethyl chloroformate (83 mg), and sodium bicarbonate (63 mg) were dissolved in acetonitrile (10 mL), and the mixture was heated to reflux for 8 hours. The reaction solution was admixed with water, and was extracted with ethyl acetate. The organic layer was dried over anhydrous sodium sulfate, the inorganic material was filtered off, and the solvent was then distilled away under reduced pressure. The residue was then purified by silica gel flash chromatography (ethyl acetate-hexane: 5%-35% elution) using an automated flash purification system (Biotage AB/Isolera^{™}) to afford the title compound as a colorless resinous solid (72 mg, yield: 57%).

### [Example 8] Preparation of N-[2-(N-ethoxycarbonyl-N-trifluoromethanesulfonyl)aminobenzyl]-8-chloro-5-fluoro-3,4-dihydroisoquinolin-1(2H)-one (compound 3-154)

### Step 1: Preparation of 8-chloro-5-fluoro-3,4-dihydroisoquinolin-1(2H)-one

First, 5-chloro-2-fluorophenethylamine (1.2 g) (compound described in WO2005/090287) and triethylamine (1.05 g) were dissolved in dichloromethane (20 mL). Methyl chloroformate (784 mg) was added dropwise on an ice water bath, and the mixture was then stirred at room temperature overnight. The reaction solution was admixed with water and then extracted with dichloromethane. The organic layer was dried over anhydrous sodium sulfate, the inorganic material was filtered off, and the solvent was then distilled away under reduced pressure. The residue was dissolved in trifluoromethanesulfonic acid (5 mL) and heated and stirred at 160°C for 5 minutes by using a microwave synthesizer (Biotage AB/Initiator+^{™}). The reaction solution was admixed with ice water and then extracted with dichloromethane. The organic layer was dried over anhydrous sodium sulfate, the inorganic material was filtered off, and the solvent was then distilled away under reduced pressure. The residue was then purified by silica gel flash chromatography (ethyl acetate-hexane: 30%-60% elution) using an automated flash purification system (Biotage AB/Isolera^{™}) to afford the title compound as a brown oily substance (413 mg, yield: 30%).

### Step 2: Preparation of N-[2-(N-trifluoromethanesulfonyl)aminobenzyl]-8-chloro-5-fluoro-3,4-dihydroisoquinolin-1(2H)-one

The compound (413 mg) obtained in step 1 of Example 8, 1,1,1-trifluoro-N-[2-(hydroxymethyl)phenyl]methanesulfonamide (466 mg) (compound listed in WO2010/026989), and paratoluenesulfonic acid monohydrate (39 mg) were dissolved in ortho-dichlorobenzene (5 mL). The mixture was heated and stirred at 250°C for 8 minutes by using a microwave synthesizer (Biotage AB/Initiator+^{™}). After the reaction solution was cooled to room temperature, the reaction solution was subjected to purification by silica gel flash chromatography (ethyl acetate-hexane: 15%-50% elution) using an automated flash purification system (Biotage AB/Isolera^{™}) to afford the title compound as a yellow oily substance (124 mg, yield: 14%).

### Step 3: Preparation of N-[2-(N-ethoxycarbonyl-N-trifluoromethanesulfonyl)aminobenzyl]-8-chloro-5-fluoro-3,4-dihydroisoquinolin-1(2H)-one

The compound (83 mg) obtained in step 2 of Example 8, ethyl chloroformate (41 mg), and sodium bicarbonate (32 mg) were dissolved in acetonitrile (10 mL), and the mixture was heated to reflux for 8 hours. The reaction solution was admixed with water, and was extracted with ethyl acetate. The organic layer was dried over anhydrous sodium sulfate, the inorganic material was filtered off, and the solvent was then distilled away under reduced pressure. The residue was then purified by silica gel flash chromatography (ethyl acetate-hexane: 5%-35% elution) using an automated flash purification system (Biotage AB/Isolera^{™}) to afford the title compound as a yellow oily substance (30 mg, yield: 31%).

### [Example 9] Preparation of N-[2-[N-(2,2-difluoroethyl)oxycarbonyl-N-trifluoromethanesulfonyl]aminobenzyl]-5,8-difluoro-3,4-dihydroisoquinolin-1(2H)-one (compound 3-178)

### Step 1: Preparation of 2,2-difluoroethyloxycarbonyl chloride

First, 2,2-difluoroethanol (421 mg) and diisopropylethylamine (1.1 mL) were dissolved in dichloromethane (2.6 mL). A solution of triphosgene (543 mg) in dichloromethane (2.6 mL) was added dropwise on an ice water bath, and the mixture was stirred overnight at room temperature. The reaction solution was admixed with water and then extracted with dichloromethane. The organic layer was dried over anhydrous sodium sulfate, the inorganic material was filtered off, and the solvent was distilled away under reduced pressure to afford the title compound as a colorless liquid (503 mg, yield: 68%).

### Step 2: Preparation of N-[2-[N-(2,2-difluoroethyl)oxycarbonyl-N-trifluoromethanesulfonyl]aminobenzyl]-5,8-difluoro-3,4-dihydroisoquinolin-1(2H)-one

The compound (132 mg) obtained in step 6 of Example 4 and sodium bicarbonate (265 mg) were dissolved in acetonitrile (3.2 mL), and the compound (503 mg) obtained in step 1 of Example 9 was added on an ice water bath. The mixture was then heated to reflux for 8 hours. The reaction solution was admixed with water, and was extracted with ethyl acetate. The resulting organic layer was washed with saturated brine and dried over anhydrous sodium sulfate. The inorganic material was filtered off, and the solvent was then distilled away under reduced pressure. The residue was then purified by silica gel flash chromatography (ethyl acetate-hexane: 10%-30% elution) using an automated flash purification system (Biotage AB/Isolera^{™}) to afford the title compound as a colorless resinous solid (75 mg, yield: 45%).

### [Example 10] Preparation of N-[2-[N-(tetrahydrofuran-3-yl)oxycarbonyl-N-trifluoromethanesulfonyl]aminobenzyl]-5,8-difluoro-3,4-dihydroisoquinolin-1(2H)-one (compound 3-200)

### Step 1: Preparation of tetrahydrofuran-3-yl-oxycarbonyl chloride

First, 3-hydroxytetrahydrofuran (370 mg) and diisopropylethylamine (868 µL) were dissolved in dichloromethane (2.2 mL). A solution of triphosgene (445 mg) in dichloromethane (2.0 mL) was added dropwise on an ice water bath, and the mixture was then stirred overnight at room temperature. The reaction solution was admixed with water and then extracted with dichloromethane. The organic layer was dried over anhydrous sodium sulfate, the inorganic material was filtered off, and the solvent was distilled away under reduced pressure to afford the title compound as a colorless liquid (620 mg, yield: 98%).

### Step 2: Preparation of N-[2-[N-(tetrahydrofuran-3-yl)oxycarbonyl]aminobenzyl]-5,8-difluoro-3,4-dihydroisoquinolin-1(2H)-one

The compound (309 mg) obtained in step 5 of Example 4 and diisopropylethylamine (812 µL) were dissolved in dichloromethane (7.8 mL). A solution of the compound (620 mg) obtained in step 1 of Example 10 in dichloromethane (3 mL) was added dropwise on an ice water bath, and the mixture was then stirred overnight at room temperature. The reaction solution was admixed with saturated aqueous ammonium chloride, and the mixture was extracted with dichloromethane. The extracted solution was washed with saturated brine and dried over sodium sulfate. The inorganic material was filtered off, and the solvent was then distilled away under reduced pressure. The residue was purified by silica gel flash chromatography (ethyl acetate-hexane: 30%-60% elution) using an automated flash purification system (Biotage AB/Isolera^{™}) to afford the title compound as a colorless resinous solid (360 mg, yield: 84%).

### Step 3: Preparation of N-[2-[N-(tetrahydrofuran-3-yl)oxycarbonyl-N trifluoromethanesulfonyl]aminobenzyl]-5,8-difluoro-3,4-dihydroisoquinolin-1(2H)-one

The compound (543 mg) obtained in step 2 of Example 10 was dissolved in tetrahydrofuran (13.5 mL), and sodium hydride (108 mg, 60%) was added on an ice water bath. The mixture was then stirred at room temperature for 5 minutes. The mixture was re-cooled on an ice water bath, and trifluoromethanesulfonic acid chloride (455 mg) was added dropwise. The mixture was then stirred at room temperature overnight. The reaction solution was admixed with saturated aqueous ammonium chloride and was extracted with ethyl acetate. The extracted solution was washed with saturated brine and dried over sodium sulfate. The inorganic material was filtered off, and the solvent was then distilled away under reduced pressure. The residue was purified by silica gel flash chromatography (ethyl acetate-hexane: 30%-100% elution) using an automated flash purification system (Biotage AB/Isolera^{™}) to afford the title compound as a colorless resinous solid (573 mg, yield: 79%).

### [Example 11] Preparation of N-[2-(N-methanesulfonyl-N-trifluoromethanesulfonyl)aminobenzyl]-5,8-difluoro-3,4-dihydroisoquinolin-1(2H)-one (compound 3-247)

The compound (800 mg) obtained in step 6 of Example 4 was dissolved in tetrahydrofuran (9.5 mL), and sodium hydride (380 mg, 60%) was added on an ice water bath. The mixture was then stirred at room temperature for 15 minutes. The mixture was re-cooled on an ice water bath, and methanesulfonyl chloride (1.09 g) was added dropwise. The mixture was then heated to reflux for 8 hours. The reaction solution was admixed with saturated aqueous ammonium chloride and was extracted with ethyl acetate. The organic layer was dried over anhydrous sodium sulfate, the inorganic material was filtered off, and the solvent was then distilled away under reduced pressure. The residue was then purified by silica gel flash chromatography (ethyl acetate-hexane: 5%-20% elution) using an automated flash purification system (Biotage AB/Isolera^{™}) to afford the title compound as a colorless solid (630 mg, yield: 67%).

### [Example 12] Preparation of N-[2-(N-ethoxycarbonyl-N-trifluoromethanesulfonyl)amino-5-fluoro-benzyl]-5,8-difluoro-3,4-dihydroisoquinolin-1(2H)-one (compound 4-30)

### Step 1: Preparation of N-[2-(N-trifluoromethanesulfonyl)amino-5-fluoro-benzyl]-5,8-difluoro-3,4-dihydroisoquinolin-1(2H)-one

The compound (340 mg) obtained in step 3 of Example 4, 1,1,1-trifluoro-N-[4-fluoro-2-(hydroxymethyl)phenyl]methanesulfonamide (507 mg) (compound described in WO2016/207081), and paratoluenesulfonic acid monohydrate (35 mg) were dissolved in ortho-dichlorobenzene (5 mL). The mixture was heated and stirred at 250°C for 8 minutes by using a microwave synthesis system (Biotage AB/ Initiator+^{™}). After the reaction solution was cooled to room temperature, the reaction solution was subjected to purification by silica gel flash chromatography (ethyl acetate-hexane: 15%-50% elution) using an automated flash purification system (Biotage AB/Isolera^{™}) to afford the title compound as a colorless resinous solid (171 mg, yield: 21%).

### Step 2: Preparation of N-[2-(N-ethoxycarbonyl-N-trifluoromethanesulfonyl)aminobenzyl]-5,6-dihydrothieno[2,3-c]pyridin-7(4H)-one

The compound (79 mg) obtained in step 1 of Example 12, ethyl chloroformate (29 mg), and sodium bicarbonate (23 mg) were dissolved in acetonitrile (5 mL), and the mixture was heated to reflux for 8 hours. The reaction solution was admixed with water, and was extracted with ethyl acetate. The organic layer was dried over anhydrous sodium sulfate, the inorganic material was filtered off, and the solvent was then distilled away under reduced pressure. The residue was then purified by silica gel flash chromatography (ethyl acetate-hexane: 5%-20% elution) using an automated flash purification system (Biotage AB/Isolera^{™}) to afford the title compound as a yellow oily substance (46 mg, yield: 50%).

### [Example 13] Preparation of N-[2-(N-trifluoromethanesulfonyl)aminobenzyl]-9-chloro-2,3,4,5-tetrahydro-1H-2-benzazepin-1-one (compound 5-2)

### Step 1: Preparation of methyl[3-(2-chlorophenyl)propyl]carbamate

First, 2-chlorobenzene propanamine (2.32 g) (compound described in WO2017/177004) and triethylamine (3.51 mL) were dissolved in N,N-dimethylformamide (50 mL). Methyl chloroformate (1.94 mL) was added dropwise on an ice water bath, and the mixture was then stirred at room temperature overnight. The reaction solution was admixed with water, and was extracted with ethyl acetate. The organic layer was dried over anhydrous sodium sulfate, the inorganic material was filtered off, and the solvent was then distilled away under reduced pressure. The residue was then purified by silica gel flash chromatography (ethyl acetate-hexane: 7%-60% elution) using an automated flash purification system (Biotage AB/Isolera^{™}) to afford the title compound as a colorless oily substance (2.28 g, yield: 73%).

### Step 2: Preparation of 9-chloro-2,3,4,5-tetrahydro-1H-2-benzazepin-1-one

The compound (751 mg) obtained in step 1 of Example 13 was dissolved in trifluoromethanesulfonic acid (5 mL), and the mixture was heated and stirred at 160°C for 5 minutes by using a microwave synthesizer (Biotage AB/Initiator+^{™}). The reaction solution was admixed with ice water and then extracted with dichloromethane. The organic layer was dried over anhydrous sodium sulfate to afford a mixture of the title compound and 7-chloro-2,3,4,5-tetrahydro-1H-2-benzazepin-1-one as a colorless resinous solid (461 mg, yield: 72%).

### Step 3: Preparation of N-[2-(N-trifluoromethanesulfonyl)aminobenzyl]-9-chloro-2,3,4,5-tetrahydro-1H-2-benzazepin-1-one

The mixture (154 mg) obtained in step 2 of Example 13, 1,1,1-trifluoro-N-[2-(hydroxymethyl)phenyl]methanesulfonamide (177 mg) (compound listed in WO2010/026989), and paratoluenesulfonic acid monohydrate (75 mg) were dissolved in ortho-dichlorobenzene (5 mL). The mixture was heated and stirred at 250°C for 5 minutes by using a microwave synthesizer (Biotage AB/Initiator+^{™}). After the reaction solution was cooled to room temperature, the reaction solution was subjected to purification by silica gel flash chromatography (ethyl acetate-hexane: 15%-60% elution) using an automated flash purification system (Biotage AB/Isolera^{™}) to afford the title compound as a yellow resinous solid (62 mg, yield: 18%) and N-[2-(N-trifluoromethanesulfonyl)aminobenzyl]-7-chloro-2,3,4,5-tetrahydro-1H-2-benzazepin-1-one (compound 5-5) as a yellow oily substance (158 mg, yield: 46%).

### [Example 14] Preparation of N-[2-(N-trifluoromethanesulfonyl)aminobenzyl]-4,5-dihydro-6H-thieno[2,3-c]pyrrol-6-one (compound 6-1)

### Step 1: Preparation of methyl 3-bromomethyl-2-thiophene carboxylate

Methyl 3-methyl-2-thiophene carboxylate (2.87 g) (compound described in WO2012/031004), N-bromosuccinimide (3.27 g), and benzoyl peroxide (297 mg, 75%) were dissolved in carbon tetrachloride (30 mL), and the mixture was heated to reflux for 8 hours. The reaction solution was cooled and then filtered through Celite, and the solvent was then distilled away under reduced pressure. The residue was then purified by silica gel flash chromatography (ethyl acetate-hexane: 5%-15% elution) using an automated flash purification system (Biotage AB/Isolera^{™}) to afford the title compound as a yellow oily substance (1.87 g, yield: 43%).

### Step 2: Preparation of 4,5-dihydro-6H-thieno[2,3-c]pyrrol-6-one

The compound (1.87 g) obtained in step 1 of Example 14 above was dissolved in tetrahydrofuran (40 mL), ammonia water (20 mL) was added, and the mixture was then stirred overnight at room temperature. The reaction solution was admixed with water and then extracted with dichloromethane. The organic layer was dried over anhydrous sodium sulfate, the inorganic material was filtered off, and the solvent was then distilled away under reduced pressure. The residue was dissolved in a mixed solvent of methanol (20 mL) and ethanol (20 mL), potassium carbonate (1.11 g) was added, and the mixture was then heated to reflux for 5 hours. The reaction solution was admixed with water and then extracted with dichloromethane. The organic layer was dried over anhydrous sodium sulfate, the inorganic material was filtered off, and the solvent was then distilled away under reduced pressure. The residue was then purified by silica gel flash chromatography (chloroform-methanol: 3%-6% elution) using an automated flash purification system (Biotage AB/Isolera^{™}) to afford the title compound as a pale yellow resinous solid (450 mg, yield: 40%).

### Step 3: Preparation of N-[2-(N-trifluoromethanesulfonyl)aminobenzyl]-4,5-dihydro-6H-thieno[2,3-c]pyrrol-6-one

The compound (65 mg) obtained in step 2 of Example 14, 1,1,1-trifluoro-N-[2-(hydroxymethyl)phenyl]methanesulfonamide (100 mg) (compound listed in WO2010/026989), and paratoluenesulfonic acid monohydrate (42 mg) were dissolved in ortho-dichlorobenzene (5 mL). The mixture was heated and stirred at 250°C for 5 minutes by using a microwave synthesizer (Biotage AB/Initiator+^{™}). After the reaction solution was cooled to room temperature, the reaction solution was subjected to purification by silica gel flash chromatography (ethyl acetate-hexane: 15%-60% elution) using an automated flash purification system (Biotage AB/Isolera^{™}) to afford the title compound as a pale yellow resinous solid (140 mg, yield: 84%).

### [Example 15] Preparation of N-[2-(N-trifluoromethanesulfonyl)aminobenzyl]-5,6-dihydrofurano[2,3-c]pyrimidin-7(4H)-one (compound 6-4)

### Step 1: Preparation of methyl 2-[[[2-(furan-3-yl)ethyl]carbamoyl]oxy]benzoate

The compound (2.02 g) obtained in step 1 of Example 4 and 3-furanethylamine (680 mg) (compound described in US2004/0087601) were dissolved in tetrahydrofuran (20 mL), and the mixture was then stirred at room temperature overnight. The reaction solution was concentrated and the residue was then purified by silica gel flash chromatography (ethyl acetate-hexane: 5%-30% elution) using an automated flash purification system (Biotage AB/Isolera^{™}) to afford the title compound as a colorless oily substance (716 mg, yield: 40%).

### Step 2: Preparation of 5,6-dihydrofurano[2,3-c]pyrimidin-7(4H)-one

The compound (236 mg) obtained in step 1 of Example 15 was dissolved in dichloromethane (4 mL), trifluoromethanesulfonic acid (1.23 g) was added dropwise on an ice water bath, and the mixture was then stirred at room temperature for 2 hours. The reaction solution was gradually added to an ice water bath, and the aqueous layer was then extracted with dichloromethane. The organic layer was dried over anhydrous sodium sulfate, the inorganic material was filtered off, and the solvent was then distilled away under reduced pressure. The residue was then purified by silica gel flash chromatography (ethyl acetate-hexane: 15%-80% elution) using an automated flash purification system (Biotage AB/Isolera^{™}) to afford the title compound as a pale yellow resinous solid (29 mg, yield: 26%).

### Step 3: Preparation of N-[2-(N-trifluoromethanesulfonyl)aminobenzyl]-5,6-dihydrofurano[2,3-c]pyrimidin-7(4H)-one

The compound (19 mg) obtained in step 2 of Example 15, 1,1,1-trifluoro-N-[2-(hydroxymethyl)phenyl]methanesulfonamide (37 mg) (compound listed in WO2010/026989), and paratoluenesulfonic acid monohydrate (3 mg) were dissolved in ortho-dichlorobenzene (1 mL). The mixture was heated and stirred at 250°C for 5 minutes by using a microwave synthesizer (Biotage AB/Initiator+^{™}). After the reaction solution was cooled to room temperature, the reaction solution was subjected to purification by silica gel flash chromatography (ethyl acetate-hexane: 15%-60% elution) using an automated flash purification system (Biotage AB/Isolera^{™}) to afford the title compound as a pale yellow resinous solid (21 mg, yield: 41%).

### [Example 16] Preparation of N-[2-(N-ethoxycarbonyl-N-trifluoromethanesulfonyl)aminobenzyl]-5,6-dihydrothieno[2,3-c]pyridin-7(4H)-one (compound 6-22)

### Step 1: Preparation of N-[2-(N-trifluoromethanesulfonyl)aminobenzyl]-5,6-dihydrothieno[2,3-c]pyridin-7(4H)-one

First, 5,6-dihydrothieno[2,3-c]pyridin-7(4H)-one (91 mg) (compound listed in WO2007/146759), 1,1,1-trifluoro-N-[2-(hydroxymethyl)phenyl]methanesulfonamide (127 mg) (compound listed in WO2010/026989), and paratoluenesulfonic acid monohydrate (54 mg) were dissolved in ortho-dichlorobenzene (5 mL). The mixture was heated and stirred at 250°C for 5 min by using a microwave synthesizer (Biotage AB/Initiator+^{™}). After the reaction solution was cooled to room temperature, the reaction solution was subjected to purification by silica gel flash chromatography (ethyl acetate-hexane: 15%-50% elution) using an automated flash purification system (Biotage AB/Isolera^{™}) to afford the title compound as a colorless resinous solid (90 mg, yield: 40%).

### Step 2: Preparation of N-[2-(N-ethoxycarbonyl-N-trifluoromethanesulfonyl)aminobenzyl]-5,6-dihydrothieno[2,3-c]pyridin-7(4H)-one

The compound (90 mg) obtained in step 1 of Example 16, ethyl chloroformate (63 mg), and sodium bicarbonate (48 mg) were dissolved in acetonitrile (5 mL), and the mixture was heated to reflux for 8 hours. The reaction solution was admixed with water, and was extracted with ethyl acetate. The organic layer was dried over anhydrous sodium sulfate, the inorganic material was filtered off, and the solvent was then distilled away under reduced pressure. The residue was then purified by silica gel flash chromatography (ethyl acetate-hexane: 5%-30% elution) using an automated flash purification system (Biotage AB/Isolera^{™}) to afford the title compound as a yellow oily substance (103 mg, yield: 96%).

### [Example 17] Preparation of N-[2-(N-trifluoromethanesulfonyl)aminobenzyl]-5,8-difluoro-3,4-dihydroisoquinoline-1(2H)-thione (compound 7-2)

The compound (390 mg) obtained in step 6 of Example 4 and Lawesson's reagent (2.6 g) were dissolved in toluene (9.5 mL). The mixture was then heated to reflux for 4 days while stirring. The reaction solution was concentrated and the residue was then purified by silica gel flash chromatography (chloroform-hexane: 20%-100% elution) using an automated flash purification system (Biotage AB/Isolera^{™}) to afford the title compound as a yellow resinous solid (129 mg, yield: 32%).

### [Example 18] Preparation of N-[2-(N-ethoxycarbonyl-N-trifluoromethylsulfonyl)aminobenzyl]-4-methyl-3,4-dihydroisoquinolin-1(2H)-one (compound 7-15)

### Step 1: Preparation of 2-(2,5-difluorophenyl)propanenitrile

First, 2,5-difluorophenylacetonitrile (2 g) and iodomethane (1.85 g) were dissolved in tetrahydrofuran (30 mL). A solution of potassium t-butoxide (2.2 g) in tetrahydrofuran (10 mL) was added dropwise at -78°C. After the temperature was raised gradually to room temperature, the mixture was stirred at room temperature overnight. The reaction solution was admixed with saturated aqueous ammonium chloride and was extracted with ethyl acetate. The organic layer was dried over anhydrous sodium sulfate, the inorganic material was filtered off, and the solvent was then distilled away under reduced pressure. The residue was then purified by silica gel flash chromatography (ethyl acetate-hexane: 5%-20% elution) using an automated flash purification system (Biotage AB/Isolera^{™}) to afford the title compound as a yellow oily substance (1.76 g, yield: 81%).

### Step 2: Preparation of 2-(2,5-difluorophenyl)propan-1-amine

The compound (1.76 g) obtained in step 1 of Example 18 was dissolved in tetrahydrofuran (20 mL), and dimethylsulfideborane (5 mL) was added. The mixture was then heated to reflux for 8 hours. Next, 1-N aqueous hydrochloric acid solution was added dropwise to the reaction solution, and the mixture was heated to reflux for 2 hours. The reaction solution was admixed with water and then washed with dichloromethane. The aqueous layer was admixed with sodium hydroxide, so that the aqueous solution was made basic. The solution was then extracted with dichloromethane. The organic layer was dried over anhydrous sodium sulfate, the inorganic material was filtered off, and the solvent was distilled away under reduced pressure to afford the title compound as a colorless oily substance (1.37 g, yield: 76%).

### Step 3: Preparation of methyl 2-[[[2-(2,5-difluorophenyl)propyl]carbamoyl]oxy]benzoate

The compound (2.4 g) obtained in step 1 of Example 4 and the compound (1.37 g) obtained in step 2 of Example 18 were dissolved in tetrahydrofuran (25 mL), and the mixture was then stirred at room temperature overnight. The reaction solution was concentrated and the residue was then purified by silica gel flash chromatography (ethyl acetate-hexane: 5%-30% elution) using an automated flash purification system (Biotage AB/Isolera^{™}) to afford the title compound as a colorless oily substance (2.27 g, yield: 89%).

### Step 4: Preparation of 4-methyl-3,4-dihydroisoquinolin-1(2H)-one

The compound (2.27 g) obtained in step 3 of Example 18 was dissolved in dichloromethane (33 mL), trifluoromethanesulfonic acid (9.76 g) was added dropwise on an ice water bath, and the mixture was then stirred at room temperature for 3 hours. The reaction solution was gradually added to an ice water bath, and the aqueous layer was then extracted with dichloromethane. The organic layer was dried over anhydrous sodium sulfate, the inorganic material was filtered off, and the solvent was then distilled away under reduced pressure. The residue was then purified by silica gel flash chromatography (ethyl acetate-hexane: 20%-80% elution) using an automated flash purification system (Biotage AB/Isolera^{™}) to afford the title compound as a colorless oily substance (1.07 g, yield: 83%).

### Step 5: Preparation of N-[2-(N-trifluoromethylsulfonyl)aminobenzyl]-4-methyl-3,4-dihydroisoquinolin-1(2H)-one

The compound (360 mg) obtained in step 4 of Example 18, 1,1,1-trifluoro-N-[2-(hydroxymethyl)phenyl]methanesulfonamide (411 mg) (compound listed in WO2010/026989), and paratoluenesulfonic acid monohydrate (35 mg) were dissolved in ortho-dichlorobenzene (5 mL). The mixture was heated and stirred at 250°C for 8 minutes by using a microwave synthesizer (Biotage AB/Initiator+^{™}). After the reaction solution was cooled to room temperature, the reaction solution was subjected to purification by silica gel flash chromatography (ethyl acetate-hexane: 15%-60% elution) using an automated flash purification system (Biotage AB/Isolera^{™}) to afford the title compound as a colorless resinous solid (636 mg, yield: 80%).

### Step 6: Preparation of N-[2-(N-ethoxycarbonyl-N-trifluoromethylsulfonyl)aminobenzyl]-4-methyl-3,4-dihydroisoquinolin-1(2H)-one

The compound (456 mg) obtained in step 5 of Example 18, ethyl chloroformate (285 mg), and sodium bicarbonate (221 mg) were dissolved in acetonitrile (15 mL), and the mixture was heated to reflux for 8 hours. The reaction solution was admixed with water, and was extracted with ethyl acetate. The organic layer was dried over anhydrous sodium sulfate, the inorganic material was filtered off, and the solvent was then distilled away under reduced pressure. The residue was then purified by silica gel flash chromatography (ethyl acetate-hexane: 5%-20% elution) using an automated flash purification system (Biotage AB/Isolera^{™}) to afford the title compound as a yellow oily substance (472 mg, yield: 89%).

### [Example 19] Preparation of N-[2-(N-ethoxycarbonyl-N-trifluoromethanesulfonyl)aminobenzyl]-1,3-dihydroindol-2-one (compound 8-7)

### Step 1: Preparation of N-[2-(N-trifluoromethanesulfonyl)aminobenzyl]-1,3-dihydroindol-2-one

Oxyindoline (250 mg), 1,1,1-trifluoro-N-[2-(hydroxymethyl)phenyl]methanesulfonamide (140 mg) (compound listed in WO2010/026989), and paratoluenesulfonic acid monohydrate (95 mg) were dissolved in ortho-dichlorobenzene (3 mL). The mixture was heated and stirred at 250°C for 5 minutes by using a microwave synthesizer (Biotage AB/Initiator+^{™}). After the reaction solution was cooled to room temperature, the reaction solution was subjected to purification by silica gel flash chromatography (ethyl acetate-hexane: 10%-40% elution) using an automated flash purification system (Biotage AB/Isolera^{™}) to afford the title compound as a colorless resinous solid (87 mg, yield: 23%).

### Step 2: Preparation of N-[2-(N-ethoxycarbonyl-N-trifluoromethanesulfonyl)aminobenzyl]-1,3-dihydroindol-2-one

The compound (76 mg) obtained in step 1 of Example 19, ethyl chloroformate (56 mg), and sodium bicarbonate (44 mg) were dissolved in acetonitrile (10 mL), and the mixture was heated to reflux for 3 hours. The reaction solution was admixed with water, and was extracted with ethyl acetate. The organic layer was dried over anhydrous sodium sulfate, the inorganic material was filtered off, and the solvent was then distilled away under reduced pressure. The residue was then purified by silica gel flash chromatography (ethyl acetate-hexane: 10%-50% elution) using an automated flash purification system (Biotage AB/Isolera^{™}) to afford the title compound as a yellow oily substance (75 mg, yield: 80%).

### [Example 20] Preparation of N-[2-(N-ethoxycarbonyl-N-trifluoromethanesulfonyl)aminobenzyl]-1,4-dihydroisoquinolin-3(2H)-one (compound 9-5)

### Step 1: Preparation of N-[2-(N-trifluoromethanesulfonyl)aminobenzyl]-1,4-dihydroisoquinolin-3(2H)-one

First, 1,4-dihydroisoquinolin-3(2H)-one (570 mg) (compound listed in WO2003/082265), 1,1,1-trifluoro-N-[2-(hydroxymethyl)phenyl]methanesulfonamide (824 mg) (compound listed in WO2010/026989), and paratoluenesulfonic acid monohydrate (307 mg) were dissolved in ortho-dichlorobenzene (5 mL). The mixture was heated and stirred at 250°C for 5 min by using a microwave synthesizer (Biotage AB/Initiator+^{™}). After the reaction solution was cooled to room temperature, the reaction solution was subjected to purification by silica gel flash chromatography (ethyl acetate-hexane: 20%-80% elution) using an automated flash purification system (Biotage AB/Isolera^{™}) to afford the title compound as a colorless resinous solid (700 mg, yield: 57%).

### Step 2: Preparation of N-[2-(N-ethoxycarbonyl-N-trifluoromethanesulfonyl)aminobenzyl]-1,4-dihydroisoquinolin-3(2H)-one

The compound (673 mg) obtained in step 1 of Example 20, ethyl chloroformate (285 mg), and sodium bicarbonate (221 mg) were dissolved in acetonitrile (30 mL), and the mixture was heated to reflux for 4 hours. The reaction solution was admixed with water, and was extracted with ethyl acetate. The organic layer was dried over anhydrous sodium sulfate, the inorganic material was filtered off, and the solvent was then distilled away under reduced pressure. The residue was then purified by silica gel flash chromatography (ethyl acetate-hexane: 10%-30% elution) using an automated flash purification system (Biotage AB/Isolera^{™}) to afford the title compound as a yellow oily substance (623 mg, yield: 78%).

The compounds shown in [Table 159] to [Table 187] were synthesized by substantially the same production methods.

Note that when X¹ and X² are bonded together to represent -S-CH=N- as in No. 3-88, S is bonded to the bond point of X¹ and N is bonded to the bond point of X². In addition, when E¹ and E² are bonded together to represent -CH=C(OMe)- as in No. 7-11, CH is bonded to the bond point of E¹ and C(OMe) is bonded to the bond point of E².

**[Table 159]**

| | | | | | |
|---|---|---|---|---|---|
| 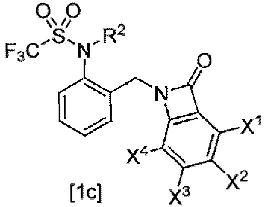 | | | | | |

| No. | R² | X¹ | X² | X³ | X⁴ |
|---|---|---|---|---|---|
| 1-1 | H | H | H | H | H |
| 1-2 | H | F | H | H | H |
| 1-3 | H | H | F | H | H |
| 1-4 | H | H | H | F | H |
| 1-5 | H | H | H | H | F |
| 1-6 | H | H | H | H | CF₃ |
| 1-7 | H | Cl | H | H | Cl |
| 1-8 | H | H | F | F | H |
| 1-9 | CO₂Et | F | H | H | H |
| 1-10 | CO₂Et | H | F | H | H |
| 1-11 | CO₂Et | H | H | F | H |

**[Table 160]**

| | | | | | | | | | |
|---|---|---|---|---|---|---|---|---|---|
| 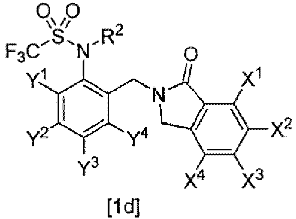 | | | | | | | | | |

| No. | R² | X¹ | X² | X³ | X⁴ | Y¹ | Y² | Y³ | Y⁴ |
|---|---|---|---|---|---|---|---|---|---|
| 2-1 | H | H | H | H | H | H | H | H | H |
| 2-2 | H | F | H | H | H | H | H | H | H |
| 2-3 | H | Cl | H | H | H | H | H | H | H |
| 2-4 | H | Br | H | H | H | H | H | H | H |
| 2-5 | H | Me | H | H | H | H | H | H | H |
| 2-6 | H | Ph | H | H | H | H | H | H | H |
| 2-7 | H | CN | H | H | H | H | H | H | H |
| 2-8 | H | OH | H | H | H | H | H | H | H |
| 2-9 | H | OMe | H | H | H | H | H | H | H |
| 2-10 | H | OAc | H | H | H | H | H | H | H |
| 2-11 | H | OBz | H | H | H | H | H | H | H |
| 2-12 | H | 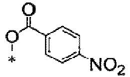 | H | H | H | H | H | H | H |
| 2-13 | H | OCO₂Me | H | H | H | H | H | H | H |
| 2-14 | H | OC(O)S-i-Pr | H | H | H | H | H | H | H |
| 2-15 | H | 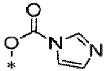 | H | H | H | H | H | H | H |
| 2-16 | H | 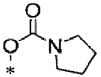 | H | H | H | H | H | H | H |
| 2-17 | H | OMs | H | H | H | H | H | H | H |

**[Table 161]**

| No. | R² | X¹ | X² | X³ | X⁴ | Y¹ | Y² | Y³ | Y⁴ |
|---|---|---|---|---|---|---|---|---|---|
| 2-18 | H | OTf | H | H | H | H | H | H | H |
| 2-19 | H | OSO₂Ph | H | H | H | H | H | H | H |
| 2-20 | H | OPO(OEt)₂ | H | H | H | H | H | H | H |
| 2-21 | H | O-TBDMS | H | H | H | H | H | H | H |
| 2-22 | H | O-Propargyl | H | H | H | H | H | H | H |
| 2-23 | H | O-allyl | H | H | H | H | H | H | H |
| 2-24 | H | OBn | H | H | H | H | H | H | H |
| 2-25 | H | OCH₂c-Pr | H | H | H | H | H | H | H |
| 2-26 | H | NH₂ | H | H | H | H | H | H | H |
| 2-27 | H | NHAc | H | H | H | H | H | H | H |
| 2-28 | H | N(Ac)₂ | H | H | H | H | H | H | H |
| 2-29 | H | NHTf | H | H | H | H | H | H | H |
| 2-30 | H | 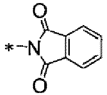 | H | H | H | H | H | H | H |
| 2-31 | H | H | F | H | H | H | H | H | H |
| 2-32 | H | H | Me | H | H | H | H | H | H |
| 2-33 | H | H | OH | H | H | H | H | H | H |
| 2-34 | H | H | OMe | H | H | H | H | H | H |
| 2-35 | H | H | OAc | H | H | H | H | H | H |
| 2-36 | H | H | OTf | H | H | H | H | H | H |
| 2-37 | H | H | O-Propargyl | H | H | H | H | H | H |
| 2-38 | H | H | NO₂ | H | H | H | H | H | H |
| 2-39 | H | H | 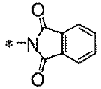 | H | H | H | H | H | H |

**[Table 162]**

| No. | R² | X¹ | X² | X³ | X⁴ | Y¹ | Y² | Y³ | Y⁴ |
|---|---|---|---|---|---|---|---|---|---|
| 2-40 | H | H | H | Br | H | H | H | H | H |
| 2-41 | H | H | H | Me | H | H | H | H | H |
| 2-42 | H | H | H | OH | H | H | H | H | H |
| 2-43 | H | H | H | OMe | H | H | H | H | H |
| 2-44 | H | H | H | OAc | H | H | H | H | H |
| 2-45 | H | H | H | O-Propargyl | H | H | H | H | H |
| 2-46 | H | H | H | H | OH | H | H | H | H |
| 2-47 | H | H | H | H | OMe | H | H | H | H |
| 2-48 | H | H | H | H | OAc | H | H | H | H |
| 2-49 | H | H | H | H | OTf | H | H | H | H |
| 2-50 | H | H | H | H | O-Propargyl | H | H | H | H |
| 2-51 | H | H | H | H | NO₂ | H | H | H | H |
| 2-52 | H | H | Br | Br | OMe | H | H | H | H |
| 2-53 | H | H | H | H | H | H | CF₃ | H | H |
| 2-54 | H | H | H | Br | H | H | CF₃ | H | H |
| 2-55 | CO₂Et | H | H | H | H | H | H | H | H |
| 2-56 | CO₂Et | NH₂ | H | H | H | H | H | H | H |
| 2-57 | CO₂Et | H | H | Br | H | H | H | H | H |
| 2-58 | CO₂Et | H | H | H | H | H | CF₃ | H | H |
| 2-59 | CO₂Et | H | H | Br | H | H | CF₃ | H | H |
| 2-60 | O-MOM | O-MOM | H | H | H | H | H | H | H |

**[Table 163]**

| 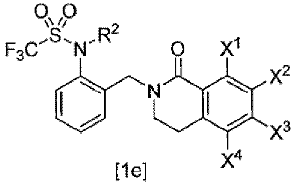 | | | | | |
|---|---|---|---|---|---|
| No. | R² | X¹ | X² | X³ | X⁴ |
| 3-1 | H | H | H | H | H |
| 3-2 | H | F | H | H | H |
| 3-3 | H | Cl | H | H | H |
| 3-4 | H | Br | H | H | H |
| 3-5 | H | I | H | H | H |
| 3-6 | H | Me | H | H | H |
| 3-7 | H | c-Pr | H | H | H |
| 3-8 | H | Ph | H | H | H |
| 3-9 | H | CF₃ | H | H | H |
| 3-10 | H | CN | H | H | H |
| 3-11 | H | OH | H | H | H |
| 3-12 | H | OMe | H | H | H |
| 3-13 | H | OAc | H | H | H |
| 3-14 | H | OCHF₂ | H | H | H |
| 3-15 | H | OTf | H | H | H |
| 3-16 | H | O-Propargyl | H | H | H |
| 3-17 | H | NMe₂ | H | H | H |
| 3-18 | H | CO₂Me | H | H | H |
| 3-19 | H | H | F | H | H |
| 3-20 | H | H | Cl | H | H |

**[Table 164]**

| No. | R² | X¹ | X² | X³ | X⁴ |
|---|---|---|---|---|---|
| 3-21 | H | H | Br | H | H |
| 3-22 | H | H | I | H | H |
| 3-23 | H | H | Me | H | H |
| 3-24 | H | H | Et | H | H |
| 3-25 | H | H | t-Bu | H | H |
| 3-26 | H | H | Ph | H | H |
| 3-27 | H | H | CF₃ | H | H |
| 3-28 | H | H | NO₂ | H | H |
| 3-29 | H | H | OH | H | H |
| 3-30 | H | H | OMe | H | H |
| 3-31 | H | H | O-CO₂Et | H | H |
| 3-32 | H | H | O-Bz | H | H |
| 3-33 | H | H | O-Propargyl | H | H |
| 3-34 | H | H | OTf | H | H |
| 3-35 | H | H | H | F | H |
| 3-36 | H | H | H | Cl | H |
| 3-37 | H | H | H | Br | H |
| 3-38 | H | H | H | I | H |
| 3-39 | H | H | H | Me | H |
| 3-40 | H | H | H | CF₃ | H |
| 3-41 | H | H | H | OMe | H |
| 3-42 | H | H | H | H | F |
| 3-43 | H | H | H | H | Cl |
| 3-44 | H | H | H | H | Me |

**[Table 165]**

| No. | R² | X¹ | X² | X³ | X⁴ |
|---|---|---|---|---|---|
| 3-45 | H | H | H | H | NO₂ |
| 3-46 | H | H | H | H | OMe |
| 3-47 | H | H | H | H | OAc |
| 3-48 | H | H | H | H | OMs |
| 3-49 | H | H | H | H | OTf |
| 3-50 | H | H | H | H | O-CO₂Et |
| 3-51 | H | H | H | H | O-Bz |
| 3-52 | H | H | H | H | O-Propargyl |
| 3-53 | H | F | Me | H | H |
| 3-54 | H | F | NO₂ | H | H |
| 3-55 | H | Cl | Cl | H | H |
| 3-56 | H | Cl | Me | H | H |
| 3-57 | H | F | H | F | H |
| 3-58 | H | F | H | Me | H |
| 3-59 | H | F | H | c-Pr | H |
| 3-60 | H | F | H | CN | H |
| 3-61 | H | Cl | H | Cl | H |
| 3-62 | H | Me | H | Me | H |
| 3-63 | H | F | H | H | F |
| 3-64 | H | F | H | H | Cl |
| 3-65 | H | F | H | H | Br |
| 3-66 | H | F | H | H | Me |
| 3-67 | H | F | H | H | CF₃ |
| 3-68 | H | F | H | H | OMe |

**[Table 166]**

| No. | R² | X¹ | X² | X³ | X⁴ |
|---|---|---|---|---|---|
| 3-69 | H | CI | H | H | F |
| 3-70 | H | CI | H | H | CI |
| 3-71 | H | OH | H | H | F |
| 3-72 | H | OMe | H | H | F |
| 3-73 | H | OMs | H | H | F |
| 3-74 | H | NMe₂ | H | H | F |
| 3-75 | H | 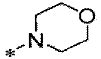 | H | H | F |
| 3-76 | H | SMe | H | H | F |
| 3-77 | H | SO₂Me | H | H | F |
| 3-78 | H | SMe | H | H | SMe |
| 3-79 | H | H | Cl | Cl | H |
| 3-80 | H | H | Me | F | H |
| 3-81 | H | H | Me | Cl | H |
| 3-82 | H | H | NO₂ | F | H |
| 3-83 | H | H | NO₂ | Cl | H |
| 3-84 | H | H | H | F | F |
| 3-85 | H | F | NO₂ | F | H |
| 3-86 | H | Cl | NO₂ | Cl | H |
| 3-87 | H | -CH=CH-CH=CH- | | H | H |
| 3-88 | H | -S-CH=N- | | H | H |
| 3-89 | H | H | -CH=CH-CH=CH- | | H |
| 3-90 | Me | F | H | H | F |
| 3-91 | Propargyl | F | H | H | F |

**[Table 167]**

| No. | R² | X¹ | X² | X³ | X⁴ |
|---|---|---|---|---|---|
| 3-92 | PMB | F | H | H | F |
| 3-93 | 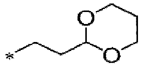 | F | H | H | F |
| 3-94 | CH₂CN | F | H | H | F |
| 3-95 | CH₂OEt | F | H | H | F |
| 3-96 | 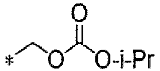 | F | H | H | F |
| 3-97 | Ac | F | H | H | F |
| 3-98 | 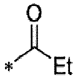 | F | H | H | F |
| 3-99 | 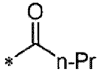 | F | H | H | F |
| 3-100 | 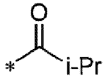 | F | H | H | F |
| 3-101 | 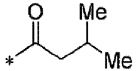 | F | H | H | F |
| 3-102 | 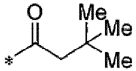 | F | H | H | F |
| 3-103 | 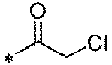 | F | H | H | F |
| 3-104 | 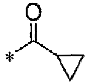 | F | H | H | H |
| 3-105 | 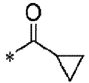 | Cl | H | H | H |

**[Table 168]**

| No. | R² | X¹ | X² | x³ | X⁴ |
|---|---|---|---|---|---|
| 3-106 | 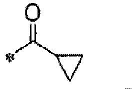 | H | H | Cl | H |
| 3-107 | 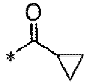 | F | H | H | F |
| 3-108 | 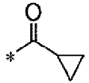 | F | H | H | Me |
| 3-109 | 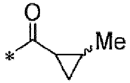 | F . | H | H | F |
| 3-110 | 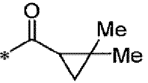 | F | H | H | F |
| 3-111 | 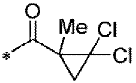 | F | H | H | F |
| 3-112 | 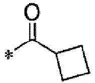 | F | H | H | F |
| 3-113 | 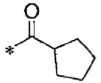 | F | H | H | F |
| 3-114 | 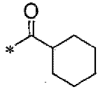 | F | H | H | F |
| 3-115 | 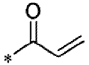 | F | H | H | F |
| 3-116 | 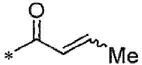 | F | H | H | F |
| 3-117 | 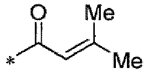 | F | H | H | F |

**[Table 169]**

| No. | R² | X¹ | X² | X³ | X⁴ |
|---|---|---|---|---|---|
| 3-118 | 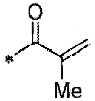 | F | H | H | F |
| 3-119 | 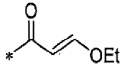 | F | H | H | F |
| 3-120 | Bz | F | H | H | F |
| 3-121 | 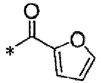 | F | H | H | F |
| 3-122 | 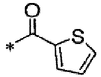 | F | H | H | F |
| 3-123 | 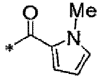 | F | H | H | F |
| 3-124 | 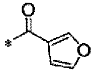 | F | H | H | F |
| 3-125 | 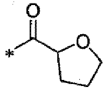 | F | H | H | F |
| 3-126 | 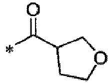 | F | H | H | F |
| 3-127 | 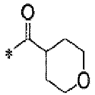 | F | H | H | F |
| 3-128 | 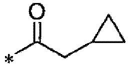 | F | H | H | F |
| 3-129 | 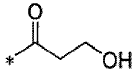 | F | H | H | F |

**[Table 170]**

| No. | R² | X¹ | X² | X³ | X⁴ |
|---|---|---|---|---|---|
| 3-130 | 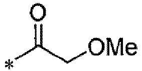 | F | H | H | F |
| 3-131 | 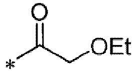 | F | H | H | F |
| 3-132 | 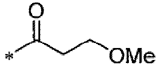 | F | H | H | F |
| 3-133 | 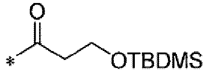 | F | H | H | F |
| 3-134 | 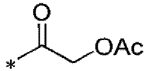 | F | H | H | F |
| 3-135 | 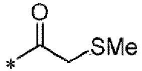 | F | H | H | F |
| 3-136 | 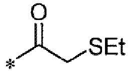 | F | H | H | F |
| 3-137 | 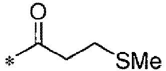 | F | H | H | F |
| 3-138 | CO₂Me | F | H | H | F |
| 3-139 | CO₂Et | H | H | H | H |
| 3-140 | CO₂Et | F | H | H | H |
| 3-141 | CO₂Et | c-Pr | H | H | H |
| 3-142 | CO₂Et | CN | H | H | H |
| 3-143 | CO₂Et | OH | H | H | H |
| 3-144 | CO₂Et | Ph | H | H | H |
| 3-145 | CO₂Et | H | H | H | Me |

**[Table 171]**

| No. | R² | X¹ | X² | X³ | X⁴ |
|---|---|---|---|---|---|
| 3-146 | CO₂Et | F | H | Me | H |
| 3-147 | CO₂Et | F | H | c-Pr | H |
| 3-148 | CO₂Et | F | H | CN | H |
| 3-149 | CO₂Et | F | H | H | F |
| 3-150 | CO₂ Et | F | H | H | Br |
| 3-151 | CO₂Et | F | H | H | Me |
| 3-152 | CO₂Et | F | H | H | CF₃ |
| 3-153 | CO₂Et | F | H | H | OMe |
| 3-154 | CO₂Et | Cl | H | H | F |
| 3-155 | CO₂Et | NMe₂ | H | H | F |
| 3-156 | CO₂Et | 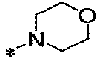 | H | H | F |
| 3-157 | CO₂Et | SMe | H | H | F |
| 3-158 | CO₂Et | SMe | H | H | SMe |
| 3-159 | CO₂Et | SO₂Me | H | H | F |
| 3-160 | CO₂Et | H | H | F | F |
| 3-161 | CO₂n-Pr | F | H | H | F |
| 3-162 | CO₂i-Pr | F | H | H | F |
| 3-163 | CO₂n-Bu | F | H | H | F |
| 3-164 | CO₂i-Bu | F | H | H | F |
| 3-165 | CO₂s-Bu | F | H | H | F |
| 3-166 | CO₂t-Bu | F | H | H | F |
| 3-167 | CO₂n-Pen | F | H | H | F |
| 3-168 | CO₂n-Hex | F | H | H | F |

**[Table 172]**

| No. | R² | X¹ | X² | X³ | X⁴ |
|---|---|---|---|---|---|
| 3-169 | CO₂n-Hep | F | H | H | F |
| 3-170 | C0₂CH₂CMe₃ | F | H | H | F |
| 3-171 | CO₂CH₂Cl | F | H | H | F |
| 3-172 | CO₂CH₂CCl₃ | F | H | H | F |
| 3-173 | CO₂CHClMe | F | H | H | F |
| 3-174 | CO₂CH₂CH₂F | F | H | H | H |
| 3-175 | CO₂CH₂CH₂F | F | H | H | F |
| 3-176 | CO₂CH₂CHF₂ | H | H | H | H |
| 3-177 | CO₂CH₂CHF₂ | F | H | H | H |
| 3-178 | CO₂CH₂CHF₂ | F | H | H | F |
| 3-179 | CO₂CH₂CF₃ | F | H | H | F |
| 3-180 | CO₂CH₂CH₂Cl | F | H | H | F |
| 3-181 | CO₂CHMeCHF₂ | F | H | H | F |
| 3-182 | CO₂CHMeCF₃ | F | H | H | F |
| 3-183 | 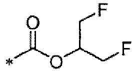 | F | H | H | F |
| 3-184 | CO₂c-Pr | F | H | H | F |
| 3-185 | CO₂c-Bu | F | H | H | F |
| 3-186 | CO₂c-Pen | F | H | H | F |
| 3-187 | CO₂c-Hex | F | H | H | F |
| 3-188 | 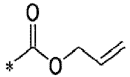 | F | H | H | F |
| 3-189 | 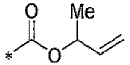 | F | H | H | F |

**[Table 173]**

| No. | R² | X¹ | X² | X³ | X⁴ |
|---|---|---|---|---|---|
| 3-190 | 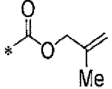 | F | H | H | F |
| 3-191 | 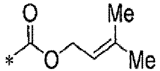 | F | H | H | F |
| 3-192 | 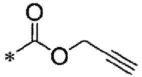 | F | H | H | F |
| 3-193 | 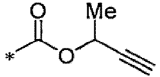 | F | H | H | F |
| 3-194 | CO₂Ph | F | H | H | F |
| 3-195 | CO₂(p-NO₂Ph) | F | H | H | F |
| 3-196 | 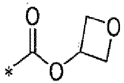 | H | H | H | H |
| 3-197 | 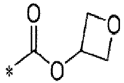 | F | H | H | H |
| 3-198 | 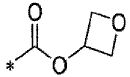 | F | H | H | F |
| 3-199 | 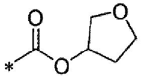 | H | H | H | H |
| 3-200 | 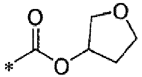 | F | H | H | F |
| 3-201 | 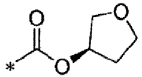 | F | H | H | F |
| 3-202 | 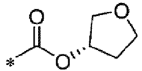 | F | H | H | F |

**[Table 174]**

| No. | R² | X¹ | X² | X³ | X⁴ |
|---|---|---|---|---|---|
| 3-203 | 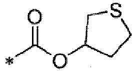 | F | H | H | F |
| 3-204 | 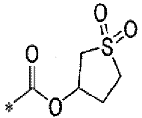 | F | H | H | F |
| 3-205 | 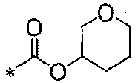 | F | H | H | F |
| 3-206 | 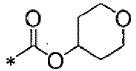 | F | H | H | F |
| 3-207 | 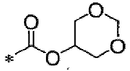 | F | H | H | F |
| 3-208 | 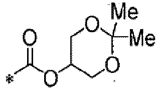 | F | H | H | F |
| 3-209 | 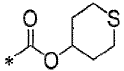 | F | H | H | F |
| 3-210 | 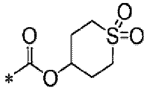 | F | H | H | F |
| 3-211 | 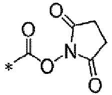 | F | H | H | F |
| 3-212 | CO₂CH₂c-Pr | F | H | H | F |
| 3-213 | CO₂Bn | F | H | H | F |
| 3-214 | 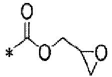 | F | H | H | F |

**[Table 175]**

| No. | R² | X¹ | X² | X³ | X⁴ |
|---|---|---|---|---|---|
| 3-215 | 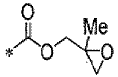 | F | H | H | F |
| 3-216 | 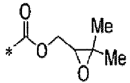 | F | H | H | F |
| 3-217 | 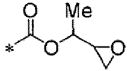 | F | H | H | F |
| 3-218 | 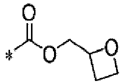 | F | H | H | F |
| 3-219 | 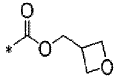 | F | H | H | F |
| 3-220 | 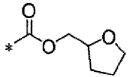 | F | H | H | F |
| 3-221 | 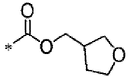 | F | H | H | F |
| 3-222 | 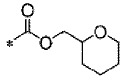 | F | H | H | F |
| 3-223 | 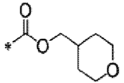 | F | H | H | F |
| 3-224 | 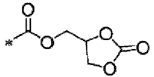 | F | H | H | F |
| 3-225 | 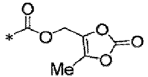 | F | H | H | F |
| 3-226 | CO₂CH₂CH₂CN | F | H | H | F |
| 3-227 | CO₂CH₂CH₂OH | F | H | H | F |

**[Table 176]**

| No. | R² | X¹ | X² | X³ | X⁴ |
|---|---|---|---|---|---|
| 3-228 | CO₂CH₂CH₂OMe | F | H | H | F |
| 3-229 | CO₂CHMeCH₂OMe | F | H | H | F |
| 3-230 | 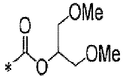 | F | H | H | F |
| 3-231 | CO₂CH₂CH₂OTBDPS | F | H | H | F |
| 3-232 | CO₂CH₂CH₂OAc | F | H | H | F |
| 3-233 | CO₂CH₂CH₂OSO₂Me | F | H | H | F |
| 3-234 | 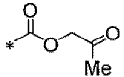 | F | H | H | F |
| 3-235 | CO₂CH₂CO₂Me | F | H | H | F |
| 3-236 | CO₂CH₂CH₂SMe | F | H | H | F |
| 3-237 | CO₂CH₂CH₂SO₂Me | F | H | H | F |
| 3-238 | C(O)S-i-Pr | H | H | H | H |
| 3-239 | C(O)S-i-Pr | F | H | H | H |
| 3-240 | C(O)SEt | F | H | H | F |
| 3-241 | C(O)S-i-Pr | F | H | H | F |
| 3-242 | C(O)S-s-Bu | F | H | H | F |
| 3-243 | 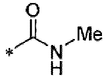 | F | H | H | F |
| 3-244 | 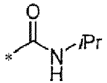 | F | H | H | F |
| 3-245 | 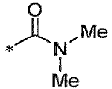 | F | H | H | F |

**[Table 177]**

| No. | R² | X¹ | X² | X³ | X⁴ |
|---|---|---|---|---|---|
| 3-246 | Ms | H | H | H | H |
| 3-247 | Ms | F | H | H | F |

| No. | R² | X¹ | X² | X³ | X⁴ |
|---|---|---|---|---|---|
| 3-248 | Ms | OH | H | H | F |
| 3-249 | SO₂Et | F | H | H | F |
| 3-250 | Tf | OH | H | H | H |
| 3-251 | Tf | O-Propargyl | H | H | H |
| 3-252 | Tf | F | H | H | F |
| 3-253 | SO₂c-Pr | F | H | H | F |
| 3-254 | Na | F | H | H | F |
| 3-255 | K | F | H | H | F |

**[Table 178]**

| 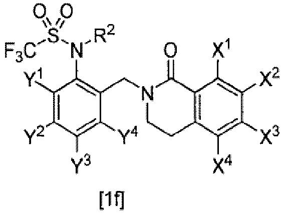 | | | | | | | | | |
|---|---|---|---|---|---|---|---|---|---|
| No. | R² | X¹ | X² | X³ | X⁴ | Y¹ | Y² | Y³ | Y⁴ |
| 4-1 | H | H | H | H | H | Cl | H | H | H |
| 4-2 | H | H | H | H | H | H | Me | H | H |
| 4-3 | H | H | H | H | H | H | OMe | H | H |
| 4-4 | H | H | H | H | H | H | H | F | H |
| 4-5 | H | H | H | H | H | H | H | Cl | H |
| 4-6 | H | H | H | H | H | H | H | Br | H |
| 4-7 | H | H | H | H | H | H | H | Me | H |
| 4-8 | H | H | H | H | H | H | H | H | Cl |
| 4-9 | H | H | H | H | H | Cl | H | Cl | H |
| 4-10 | H | Cl | H | H | H | H | H | Cl | H |
| 4-11 | H | H | H | Cl | H | H | H | Cl | H |
| 4-12 | H | F | H | H | F | F | H | H | H |
| 4-13 | H | F | H | H | F | Cl | H | H | H |
| 4-14 | H | F | H | H | F | Me | H | H | H |
| 4-15 | H | F | H | H | F | H | F | H | H |
| 4-16 | H | F | H | H | F | H | H | F | H |
| 4-17 | H | F | H | H | F | H | H | Cl | H |
| 4-18 | H | F | H | H | F | H | H | Me | H |
| 4-19 | H | F | H | H | F | H | H | OH | H |
| 4-20 | H | F | H | H | F | H | H | OMe | H |
| 4-21 | H | F | H | H | F | H | H | H | F |
| 4-22 | H | F | H | H | F | H | H | H | Cl |
| 4-23 | H | F | H | H | F | H | H | H | Me |
| 4-24 | H | F | H | H | F | H | -CH=CHCH=CH- | | H |
| 4-25 | CO₂Et | Cl | H | H | H | H | H | Cl | H |

**[Table 179]**

| No. | R² | X¹ | X² | X³ | X⁴ | Y¹ | Y² | Y³ | Y⁴ |
|---|---|---|---|---|---|---|---|---|---|
| 4-26 | CO₂Et | F | H | H | F | F | H | H | H |
| 4-27 | CO₂Et | F | H | H | F | Cl | H | H | H |
| 4-28 | CO₂Et | F | H | H | F | Me | H | H | H |
| 4-29 | CO₂Et | F | H | H | F | H | F | H | H |
| 4-30 | CO₂Et | F | H | H | F | H | H | F | H |
| 4-31 | CO₂Et | F | H | H | F | H | H | Cl | H |
| 4-32 | CO₂Et | F | H | H | F | H | H | Me | H |
| 4-33 | CO₂Et | F | H | H | F | H | H | CF₃ | H |
| 4-34 | CO₂Et | F | H | H | F | H | H | OH | H |
| 4-35 | CO₂Et | F | H | H | F | H | H | OMe | H |
| 4-36 | CO₂Et | F | H | H | F | H | H | H | F |
| 4-37 | CO₂Et | F | H | H | F | H | H | H | Cl |
| 4-38 | CO₂Et | F | H | H | F | H | H | H | Me |
| 4-39 | CO₂Et | F | H | H | F | H | -CH=CHCH=CH- | | H |
| 4-40 | CO₂n-Pen | F | H | H | F | H | F | H | H |
| 4-41 | CO₂n-Pen | F | H | H | F | H | H | F | H |
| 4-42 | CO₂n-Pen | F | H | H | F | H | H | H | F |

**[Table 180]**

| 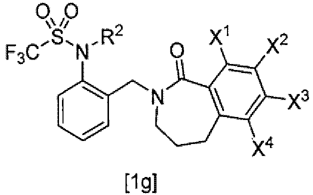 | | | | | |
|---|---|---|---|---|---|
| No. | R² | X¹ | X² | X³ | X⁴ |
| 5-1 | H | H | H | H | H |
| 5-2 | H | Cl | H | H | H |
| 5-3 | H | Me | H | H | H |
| 5-4 | H | H | H | F | H |
| 5-5 | H | H | H | Cl | H |
| 5-6 | H | H | H | Me | H |

**[Table 181]**

| | | | |
|---|---|---|---|
| 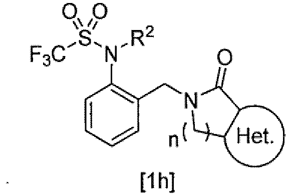 | | | |

| No. | n | R² | Het. |
|---|---|---|---|
| 6-1 | 1 | H | 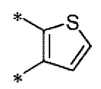 |
| 6-2 | 1 | H | 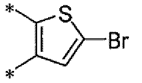 |
| 6-3 | 1 | H | 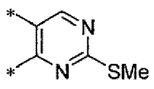 |
| 6-4 | 2 | H | 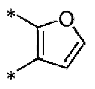 |
| 6-5 | 2 | H | 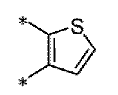 |
| 6-6 | 2 | H | 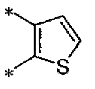 |
| 6-7 | 2 | H | 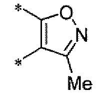 |
| 6-8 | 2 | H | 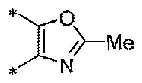 |
| 6-9 | 2 | H | 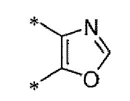 |

**[Table 182]**

| No. | n | R² | Het. |
|---|---|---|---|
| 6-10 | 2 | H | 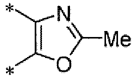 |
| 6-11 | 2 | H | 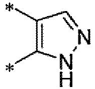 |
| 6-12 | 2 | H | 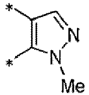 |
| 6-13 | 2 | H | 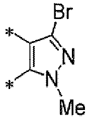 |
| 6-14 | 2 | H | 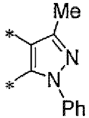 |
| 6-15 | 2 | H | 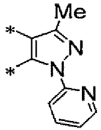 |
| 6-16 | 2 | H | 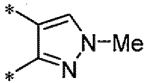 |
| 6-17 | 2 | H | 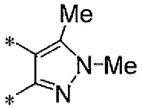 |
| 6-18 | 2 | H | 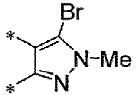 |
| 6-19 | 2 | H | 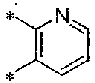 |

**[Table 183]**

| No. | n | R² | Het. |
|---|---|---|---|
| 6-20 | 2 | H | 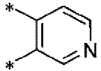 |
| 6-21 | 2 | H | 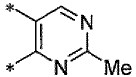 |
| 6-22 | 2 | CO₂Et | 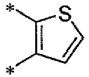 |
| 6-23 | 2 | CO₂Et | 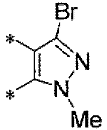 |
| 6-24 | 2 | CO₂Et | 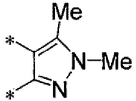 |
| 6-25 | 2 | CO₂Et | 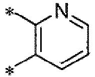 |
| 6-26 | 2 | CO₂Et | 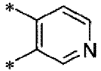 |
| 6-27 | 2 | CO₂Et | 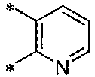 |

**[Table 184]**

| | | | | | | | | | | |
|---|---|---|---|---|---|---|---|---|---|---|
| 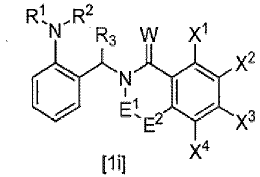 | | | | | | | | | | |

| No. | R¹ | R² | R³ | W | E¹ | E² | X¹ | X² | X³ | X⁴ |
|---|---|---|---|---|---|---|---|---|---|---|
| 7-1 | Tf | H | H | S | -CH₂- | -CH₂- | H | H | H | H |
| 7-2 | Tf | H | H | S | -CH₂- | -CH₂- | F | H | H | F |
| 7-3 | Tf | H | H | O | -CHMe- | -CH₂- | H | H | H | H |
| 7-4 | Tf | H | H | O | -CMe₂- | -CH₂- | H | H | H | H |
| 7-5 | Tf | H | H | O | -CMe₂- | -O- | H | H | H | H |
| 7-6 | Tf | H | H | O | -O- | -CH₂- | H | H | H | H |
| 7-7 | Tf | H | H | O | -CH₂- | -S- | H | H | H | H |
| 7-8 | Tf | H | H | O | -CH₂- | -CHMe- | H | H | H | H |
| 7-9 | Tf | H | H | O | -CH₂- | -CHMe- | F | H | H | F |
| 7-10 | Tf | H | H | O | -CH₂- | -CMe₂- | F | H | H | F |
| 7-11 | Tf | H | H | O | -CH=C(OMe)- | | F | H | H | F |

**[Table 185]**

| No. | R¹ | R² | R³ | W | E¹ | E² | X¹ | X² | X³ | X⁴ |
|---|---|---|---|---|---|---|---|---|---|---|
| 7-12 | Tf | CO₂Et | H | S | -CH₂- | -CH₂- | F | H | H | F |
| 7-13 | Tf | CO₂Et | H | O | -CHMe- | -CH₂- | H | H | H | H |
| 7-14 | Tf | CO₂Et | H | O | -O- | -CH₂- | H | H | H | H |
| 7-15 | Tf | CO₂Et | H | O | -CH₂- | -CHMe- | F | H | H | F |
| 7-16 | Tf | CO₂Et | H | O | -CH₂- | -CMe₂- | H | H | H | H |
| 7-17 | Tf | CO₂Et | H | O | -CH=CH- | | H | H | H | H |
| 7-18 | Tf | H | Me | O | -CH₂- | -CH₂- | F | H | H | F |
| 7-19 | SO₂CHF₂ | H | H | O | -CH₂- | -CH₂- | F | H | H | F |

**[Table 186]**

| | | | | | | | | | |
|---|---|---|---|---|---|---|---|---|---|
| 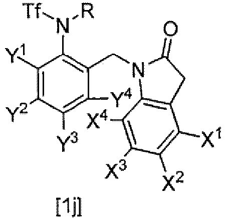 | | | | | | | | | |

| No. | R | X¹ | X² | X³ | X⁴ | Y¹ | Y² | Y³ | Y⁴ |
|---|---|---|---|---|---|---|---|---|---|
| 8-1 | H | H | H | H | H | H | H | H | H |
| 8-2 | H | H | F | H | H | H | H | H | H |
| 8-3 | H | H | Cl | H | H | H | H | H | H |
| 8-4 | H | H | Br | H | H | H | H | H | H |
| 8-5 | H | H | H | Cl | H | H | H | H | H |
| 8-6 | H | H | H | H | H | H | CF₃ | H | H |
| 8-7 | CO₂Et | H | H | H | H | H | H | H | H |
| 8-8 | CO₂Et | H | F | H | H | H | H | H | H |
| 8-9 | CO₂Et | H | Cl | H | H | H | H | H | H |
| 8-10 | CO₂Et | H | Br | H | H | H | H | H | H |
| 8-11 | CO₂Et | H | H | Cl | H | H | H | H | H |
| 8-12 | CO₂Et | H | H | H | H | H | CF₃ | H | H |

**[Table 187]**

| | | | | | |
|---|---|---|---|---|---|
| 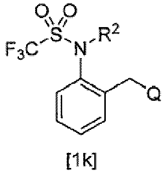 | | | | | |

| No. | R² | Q | No. | R² | Q |
|---|---|---|---|---|---|
| 9-1 | H | 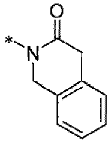 | 9-5 | CO₂Et | 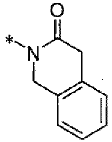 |
| 9-2 | H | 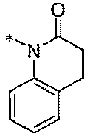 | 9-6 | CO₂Et | 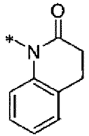 |
| 9-3 | H | 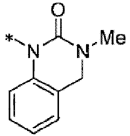 | 9-7 | CO₂Et | 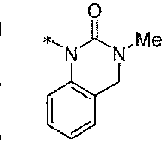 |
| 9-4 | H | 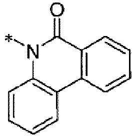 | 9-8 | CO₂Et | 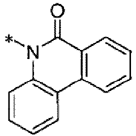 |

The following tables show ¹H-NMR data (CDCl₃/TMS δ(ppm) values) for the compounds obtained in the above Examples and for the present invention compounds produced by substantially the same methods. Note that some of the compounds of the present invention are diastereomeric mixtures, and rotational isomers are observed by NMR.

In the following tables, the "lp" means a low-polarity compound in the diastereomeric mixture. The "mp" means a high-polarity compound in the diastereomeric mixture. The "trans" means to indicate data of the trans form, which is the main component of the geometrical isomer mixture. The "DMSO-d6" means that dimethyl sulfoxide-d6 was used as the measurement solvent. The "CD3OD" means that methanol (d4) was used as the measurement solvent.

**[Table 188]**

| No. | CDCI₃/TMS *δ* (ppm) |
|---|---|
| 1-1 | 5.66 (s, 2H), 7.30 (dt, 1H), 7.40 (dt, 1H), 7.57 (d, 1H), 7.72 (dd, 1H), 7.86 (dt, 1H), 8.00 (dt, 1H), 8.21 (d, 1H), 8.38 (dd, 1H), 10.39 (brs, 1H). |
| 1-2 | 5.67 (s, 2H), 7.31 (dt, 1H), 7.42 (dt, 1H), 7.57 (dd, 1H), 7.66-7.77 (m, 2H), 7.83 (dt, 1H), 8.16 (d, 1H), 10.10 (brs, 1H). |
| 1-3 | 5.66 (s, 2H), 7.31 (dt, 1H), 7.41 (dt, 1H), 7.54-7.62 (m, 2H), 7.72 (dd, 1H), 7.84 (dd, 1H), 8.41 (dd, 1H), 10.19 (brs, 1H). |
| 1-4 | 5.65 (s, 2H), 7.31 (dt, 1H), 7.41 (dt, 1H), 7.58 (dd, 1H), 7.66-7.75 (m, 2H), 8.00 (dd, 1H), 8.26 (dd, 1H), 10.19 (brs, 1H). |
| 1-5 | 5.63 (s, 2H), 7.31 (dt, 1H), 7.41 (dt, 1H), 7.50 (dt, 1H), 7.56 (dd, 1H), 7.69 (dd, 1H), 7.96 (dt, 1H), 8.03 (d, 1H), 9.65 (brs, 1H). |
| 1-6 | 5.68 (s, 2H), 7.31 (dt, 1H), 7.41 (dt, 1H), 7.57 (dd, 1H), 7.72 (dd, 1H), 8.08 (t, 1H), 8.23 (d, 1H), 8.42 (d, 1H), 9.78 (brs, 1H). |
| 1-7 | 5.63 (s, 2H), 7.32 (dt, 1H), 7.42 (dt, 1H), 7.57 (dd, 1H), 7.69 (dd, 1H), 7.73 (d, 1H), 7.90 (d, 1H), 9.61 (brs, 1H). |
| 1-8 | 5.65 (s, 2H), 7.31 (dt, 1H), 7.42 (dt, 1H), 7.57 (dd, 1H), 7.70 (dd, 1H), 8.00 (dd, 1H), 8.13 (dd, 1H), 9.80 (brs, 1H). |
| 1-9 | 1.29 (t, 3H), 4.23-4.42 (m, 2H), 5.67 (d, 1H), 5.80 (d, 1H), 7.23-7.29 (m, 1H), 7.42 (dt, 1H), 7.47 (dt, 1H), 7.59-7.68 (m, 2H), 7.75 (dt, 1H), 8.11 (d, 1H). |
| 1-10 | 1.27 (t, 3H), 4.21-4.40 (m, 2H), 5.66 (d, 1H), 5.78 (d, 1H), 7.26 (d, 1H), 7.38-7.53 (m, 3H), 7.64 (dd, 1H), 7.78 (dd, 1H), 8.35 (dd, 1H). |
| 1-11 | 1.27 (t, 3H), 4.22-4.41 (m, 2H), 5.65 (d, 1H), 5.78 (d, 1H), 7.26 (d, 1H), 7.42 (dt, 1H), 7.47 (dt, 1H), 7.60-7.67 (m, 2H), 7.94 (dd, 1H), 8.19 (dd, 1H). |
| 2-1 | 4.54 (s, 2H), 4.75 (s, 2H), 7.22-7.26 (m, 1H), 7.35-7.39 (m, 2H), 7.43-7.50 (m, 2H), 7.54-7.62 (m, 2H), 7.86 (d, 1H), 11.54 (brs, 1H) |
| 2-2 | 4.57 (s, 2H), 4.72 (s, 2H), 7.08 (t, 1H), 7.22-7.28 (m, 2H), 7.34-7.39 (m, 2H), 7.51 (dt, 1H), 7.58 (dd, 1H), 11.11 (brs, 1H) |
| 2-3 | 4.51 (s, 2H), 4.73 (s, 2H), 7.24-7.29 (m, 1H), 7.31-7.48 (m, 5H), 7.61 (d, 1H), 11.14 (brs, 1H) |
| 2-4 | 4.49 (s, 2H), 4.73 (s, 2H), 7.24-7.28 (m, 1H), 7.33-7.40 (m, 4H), 7.60-7.62 (m, 2H), 11.13 (brs, 1H) |

**[Table 189]**

| No. | CDCI₃/TMS *δ* (ppm) |
|---|---|
| 2-5 | 2.70 (s, 3H), 4.46 (s, 2H), 4.71 (s, 2H), 7.18-7.26 (m, 3H), 7.33-7.42 (m, 3H), 7.61 (d, 1H), 11.66 (brs, 1H) |
| 2-6 | 4.53 (s, 2H), 4.70 (s, 2H), 7.24 (t, 1H), 7.34-7.46 (m, 7H), 7.49-7.53 (m, 2H), 7.55-7.61 (m, 2H), 7.86 (d, 1H), 11.33 (brs, 1H) |
| 2-7 | 4.26 (s, 2H), 4.85 (s, 2H), 7.02 (t, 1H), 7.16-7.21 (m, 2H), 7.35 (d, 1H), 7.52-7.57 (m, 2H), 7.66 (d, 1H) |
| 2-8 | 4.51 (s, 2H), 4.70 (s, 2H), 6.85 (d, 1H), 6.91 (d, 1H), 7.23-7.28 (m, 1H), 7.32-7.43 (m, 3H), 7.5 (d, 1H), 8.14 (brs, 1H), 11.04 (brs, 1H) |
| 2-9 | 3.95 (s, 3H), 4.50 (s, 2H), 4.68 (s, 2H), 6.89 (d, 1H), 6.98 (d, 1H), 7.22-7.26 (m, 1H), 7.32-7.37 (m, 2H), 7.48 (t, 1H), 7.58 (d, 1H), 11.37 (brs, 1H) |
| 2-10 | 2.41 (s, 3H), 4.50 (s, 2H), 4.66 (s, 2H), 7.09 (d, 1H), 7.21-7.37 (m, 4H), 7.53-7.60 (m, 2H), 11.32 (brs, 1H) |
| 2-11 | 4.52 (s, 2H), 4.66 (s, 2H), 7.21 (t, 1H), 7.30-7.37 (m, 4H), 7.50-7.67 (m, 5H), 8.27 (d, 2H), 11.12 (brs, 1H) |
| 2-12 | 4.56 (s, 2H), 4.68 (s, 2H), 7.20-7.29 (m, 1H), 7.32-7.40 (m, 4H), 7.58 (d, 1H), 7.64 (t, 1H), 8.38 (d, 2H), 8.44 (d, 2H), 11.07 (brs, 1H) |
| 2-13 | 3.98 (s, 3H), 4.51 (s, 2H), 4.67 (s, 2H), 7.16 (d, 1H), 7.25 (d, 1H), 7.33-7.38 (m, 3H), 7.53-7.60 (m, 2H), 11.22 (brs, 1H) |
| 2-14 | 1.46 (d, 6H), 3.65 (dq, 1H), 4.51 (s, 2H), 4.66 (s, 2H), 7.13 (d, 1H), 7.22-7.26 (m, 1H), 7.29-7.37 (m, 3H), 7.52-7.58 (m, 2H), 11.25 (brs, 1H) |
| 2-15 | 4.56 (s, 2H), 4.69 (s, 2H), 7.17 (s, 1H), 7.22-7.43 (m, 5H), 7.57 (d, 1H), 7.63-7.67 (m, 2H), 8.33 (s, 1H), 11.54 (brs, 1H) |
| 2-16 | 1.91-2.01 (m, 4H), 3.48 (t, 2H), 3.66 (t, 3H), 4.39 (s, 2H), 4.65 (s, 2H), 7.12-7.16 (m, 2H), 7.21 (d, 1H), 7.26-7.30 (m, 2H), 7.50 (t, 1H), 7.57 (d, 1H) |
| 2-17 | 3.49 (s, 3H), 4.54 (s, 2H), 4.72 (s, 2H), 7.23-7.26 (m, 1H), 7.33-7.40 (m, 4H), 7.58-7.62 (m, 2H), 11.19 (brs, 1H) |
| 2-18 | 4.57 (s, 2H), 4.75 (s, 2H), 7.24-7.27 (m, 1H), 7.31-7.39 (m, 3H), 7.47 (d, 1H), 7.59-7.66 (m, 2H), 10.89 (brs, 1H) |
| 2-19 | 4.52 (s, 2H), 4.65 (s, 2H), 7.22-7.26 (m, 1H), 7.32 (dd, 1H), 7.37-7.41 (m, 2H), 7.52 (d,1H), 7.57-7.70 (m, 5H), 8.22 (d, 2H), 11.40 (brs, 1H) |

**[Table 190]**

| No. | CDCI₃/TMS *δ* (ppm) |
|---|---|
| 2-20 | 1.38 (dt, 6H), 4.37 (dq, 4H), 4.43 (s, 2H), 4.67 (s, 2H), 7.17-7.22 (m, 2H), 7.30-7.36 (m, 3H), 7.49 (t, 1H), 7.57 (d, 1H) |
| 2-21 | 0.23 (s, 6H), 1.05 (s, 9H), 4.43 (s, 2H), 4.67 (s, 2H), 6.81 (d, 1H), 6.98 (d, 1H), 7.20 (dt, 1H), 7.31-7.40 (m, 3H), 7.58 (d, 1H), 11.86 (brs, 1H) |
| 2-22 | 2.50 (s, 1H), 4.50 (s, 2H), 4.68 (s, 2H), 4.90 (s, 2H), 7.03-7.10 (m, 2H), 7.23-7.27 (m, 1H), 7.32-7.38 (m, 2H), 7.49 (t, 1H), 7.58 (d, 1H), 11.31 (brs, 1H) |
| 2-23 | 4.48 (s, 2H), 4.68 (s, 2H), 4.71 (d, 2H), 5.31 (d, 1H), 5.50 (d, 1H), 6.06 (m, 1H), 6.88 (d, 1H), 6.98 (d, 1H), 7.22-7.26 (m, 1H), 7.24-7.36 (m, 2H), 7.44 (t, 1H), 7.58 (d, 1H), 11.50 (brs, 1H) |
| 2-24 | 4.47 (s, 2H), 4.69 (s, 2H), 5.29 (s, 2H), 6.87 (d, 1H), 6.97 (d, 1H), 7.20-7.42 (m, 7H), 7.50 (d, 21H), 7.60 (d, 1H), 11.56 (brs, 1H) |
| 2-25 | 0.40 (m, 2H), 0.65 (m, 2H), 1.35 (m, 1H), 3.98 (d, 2H), 4.47 (s, 2H), 4.67 (s, 2H), 6.88 (d, 1H), 6.96 (d, 1H), 7.23 (dd, 1H), 7.32-7.37 (m, 2H), 7.43 (t, 1H), 7.58 (d, 1H), 11.54 (brs, 1H) |
| 2-26 | 4.42 (s, 2H), 4.65 (s, 2H), 6.54 (brs, 2H), 6.55 (d, 1H), 6.65 (d, 1H), 7.20-7.26 (m, 2H), 7.31-7.38 (m, 2H), 7.60 (d, 1H), 11.63 (brs, 1H) |
| 2-27 | 2.26 (s, 3H), 4.52 (s, 2H), 4.71 (s, 2H), 7.07 (d, 1H), 7.24-7.28 (m, 2H), 7.34-7.42 (m, 2H), 7.50 (t, 1H), 7.61 (d, 1H), 8.49 (d, 1H), 9.93 (brs, 1H), 11.09 (brs, 1H) |
| 2-28 | 2.26 (s, 3H), 2.34 (s,3H), 4.30 (s, 2H), 4.63 (d, 1H), 5.02 (d, 1H), 7.06 (d, 1H), 7.23-7.27 (m, 1H), 7.37-7.53 (m, 4H), 8.49 (d, 1H), 10.28 (brs, 1H) |
| 2-29 | 4.54 (s, 2H), 4.73 (s, 2H), 7.18 (d, 1H), 7.26-7.29 (m, 1H), 7.35-7.43 (m, 2H), 7.49 (t, 1H), 7.59-7.62 (m, 2H) |
| 2-30 | 4.54 (s, 2H), 4.66 (s, 2H), 7.21-7.36 (m, 3H), 7.42 (d, 2H), 7.52-7.55 (m, 2H), 7.70 (t, 1H), 7.82 (m, 2H), 7.99 (m, 2H), 10.95 (brs, 1H) |
| 2-31 | 4.53 (s, 2H), 4.74 (s, 2H), 7.23-7.29 (m, 2H), 7.35-7.44 (m, 3H), 7.49 (m, 1H), 7.60 (d, 1H), 11.34 (brs, 1H) |
| 2-32 | 2.42 (s, 3H), 4.49 (s, 2H), 4.73 (s, 2H), 7.23 (dt, 1H), 7.29-7.39 (m, 4H), 7.59 (t, 1H), 7.65 (s, 1H), 11.58 (brs, 1H) |
| 2-33 | 4.47 (s, 2H), 4.72 (s, 2H), 5.13 (brs, 1H), 7.05 (dd, 1H), 7.22-7.38 (m, 5H), 7.61 (d, 1H), 11.49 (brs, 1H) |
| 2-34 | 3.84 (s, 3H), 4.48 (s, 2H), 4.73 (s, 2H), 7.11 (dd, 1H), 7.23-7.27 (m, 1H), 7.31-7.39 (m, 4H), 7.61 (d, 1H), 11.57 (brs, 1H) |

**[Table 191]**

| No. | CDCl₃/TMS *δ* (ppm) |
|---|---|
| 2-35 | 2.31 (s, 3H), 4.52 (s, 2H), 4.73 (s, 2H), 7.21-7.29 (m, 2H), 7.34-7.39 (m, 2H), 7.44 (d, 1H), 7.55-7.61 (m, 2H), 11.39 (brs, 1H) |
| 2-36 | 4.60 (s, 2H), 4.76 (s, 2H), 7.24-7.28 (m, 1H), 7.35-7.42 (m, 2H), 7.48 (m, 1H), 7.55-7.62 (m, 2H), 7.77 (s, 1H), 11.10 (brs, 1H) |
| 2-37 | 2.51 (s, 1H), 4.48 (s, 2H), 4.72 (m, 4H), 7.18 (dd, 1H), 7.23 (t, 1H), 7.32-7.44 (m, 4H), 7.60 (d, 1H), 11.52 (brs, 1H) |
| 2-38 | 4.67 (s, 2H), 4.79 (s, 2H), 7.25-7.29 (m, 1H), 7.37-7.42 (m, 2H), 7.60-7.66 (m, 2H), 8.45 (dd, 1H), 8.70 (s, 1H), 10.95 (brs, 1H) |
| 2-39 | 4.59 (s, 2H), 4.76 (s, 2H), 7.22-7.26 (m, 1H), 7.35-7.40 (m, 2H), 7.55-7.67 (m, 3H), 7.82 (m, 2H), 7.94-7.97 (m, 3H) |
| 2-40 | 4.52 (s, 2H), 4.73 (s, 2H), 7.23-7.26 (m, 1H), 7.33-7.41 (m, 2H), 7.60-7.63 (m, 3H), 7.72 (d, 2H), 11.32 (brs, 1H) |
| 2-41 | 2.44 (s, 3H), 4.48 (s, 2H), 4.72 (s, 2H), 7.21-7.28 (m, 3H), 7.33-7.39 (m, 2H), 7.60 (dd, 1H), 7.72 (d, 1H), 11.56 (brs, 1H) |
| 2-42 | 4.46 (s, 2H), 4.70 (s, 2H), 5.31 (brs, 1H), 6.88-6.91 (m, 2H), 7.23-7.26 (m, 1H), 7.33-7.38 (m, 2H), 7.61 (d, 1H), 7.73 (d, 1H), 11.60 (brs, 1H) |
| 2-43 | 3.86 (s, 3H), 4.47 (s, 2H), 4.70 (s, 2H), 6.91 (s, 1H), 6.98 (d, 1H), 7.28-7.37 (m, 3H), 7.61 (d, 1H), 7.77 (d, 1H), 11.66 (brs, 1H) |
| 2-44 | 2.32 (s, 3H), 4.53 (s, 2H), 4.73 (s, 2H), 7.17 (dd, 1H), 7.23-7.26 (m, 2H), 7.34-7.38 (m, 2H), 7.59 (d, 1H), 7.84 (d, 1H), 11.41 (brs, 1H) |
| 2-45 | 2.54 (s, 1H), 4.49 (s, 2H), 4.70 (s, 2H), 4.74 (s, 2H), 6.98-7.07 (m, 2H), 7.23-7.26 (m, 1H), 7.33-7.39 (m, 2H), 7.60 (d, 1H), 7.77 (d, 1H), 11.61 (brs, 1H) |
| 2-46 | 4.52 (s, 2H), 4.75 (s, 2H), 6.93 (d, 1H), 7.24-7.26 (m, 1H), 7.31-7.39 (m, 3H), 7.45 (d, 1H), 7.61 (d, 1H), 11.48 (brs, 1H) |
| 2-47 | 3.89 (s, 3H), 4.47 (s, 2H), 4.73 (s, 2H), 7.01 (m, 1H), 7.22-7.30 (m, 1H), 7.34-7.39 (m, 2H), 7.72-7.45 (m, 2H), 7.60 (d, 1H), 11.53 (brs, 1H) |
| 2-48 | 2.36 (s, 3H), 4.38 (s, 2H), 4.71 (s, 2H), 7.16 (d, 1H), 7.22-7.27 (m, 2H), 7.33-7.40 (m, 2H), 7.59-7.61 (m, 2H) |
| 2-49 | 4.59 (s, 2H), 4.76 (s, 2H), 7.26-7.33 (m, 2H), 7.37-7.41 (m, 2H), 7.61 (d, 1H), 7.71 (d, 1H) |

**[Table 192]**

| No. | CDCl₃/TMS *δ* (ppm) |
|---|---|
| 2-50 | 2.55 (s, 1H), 4.43 (s, 2H), 4.72 (s, 2H), 4.76 (s, 2H), 7.00 (d, 1H), 7.24-7.27 (m, 1H), 7.34-7.39 (m, 3H), 7.54 (d, 1H), 7.60 (d, 1H) |
| 2-51 | 4.81 (s, 2H), 5.03 (s, 2H), 7.26-7.29 (m, 1H), 7.31-7.47 (m, 2H), 7.62 (d, 1H), 7.74 (t, 1H), 8.21 (d, 1H), 8.43 (d, 1H), 11.09 (brs, 1H) |
| 2-52 | 3.93 (s, 3H), 4.51 (s, 2H), 4.72 (s, 2H), 7.20-7.31 (m, 2H), 7.34-7.41 (m, 2H), 7.61 (d, 1H), 7.80 (s, 1H), 10.80 (brs, 1H) |
| 2-53 | 4.55 (s, 2H), 4.79 (s, 2H), 7.44-7.52 (m, 4H), 7.59 (dt, 1H), 7.86-7.89 (m, 2H), 11.81 (brs, 1H) |
| 2-54 | 4.53 (s, 2H), 4.77 (s, 2H), 7.49-7.51 (m, 2H), 7.63-7.65 (m, 2H), 7.73 (d, 1H), 7.89 (s, 1H), 11.58 (brs, 1H) |
| 2-55 | 1.33 (t, 3H), 4.28 (s, 2H), 4.30-4.40 (m, 2H), 4.71 (d, 1H), 5.06 (d, 2H), 7.23-7.26 (m, 1H), 7.36-7.56 (m, 6H), 7.91 (d, 2H) |
| 2-56 | 3.45 (s, 3H), 4.29 (s, 2H), 4.64 (d, 1H), 5.12 (d, 1H), 5.23 (brs, 2H), 6.59 (d, 1H), 6.67 (d, 1H), 7.23-7.28 (m, 3H), 7.30-7.39 (m, 4H) |
| 2-57 | 1.33 (t, 3H), 4.24 (ab-q, 2H), 4.30-4.40 (m, 2H), 4.72 (d, 1H), 5.00 (d, 2H), 7.22-7.26 (m, 2H), 7.36-7.47 (m, 2H), 7.56 (s, 1H), 7.62 (d, 1H), 7.76 (d, 1H) |
| 2-58 | 1.35 (t, 3H), 4.30 (s, 2H), 4.35-4.43 (m, 2H), 4.77 (d, 1H), 5.07 (d, 1H), 7.42 (d, 1H), 7.47-7.58 (m, 4H), 7.69 (d, 1H), 7.91 (d, 1H) |
| 2-59 | 1.36 (t, 3H), 4.27 (s, 2H), 4.35-4.43 (m, 2H), 4.78 (d, 1H), 5.02 (d, 1H), 7.47-7.79 (m, 6H) |
| 2-60 | 3.50 (s, 3H), 3.57 (s, 3H), 4.29 (s, 2H), 4.71 (d, 1H), 5.02 (d, 1H), 5.12-2.17 (m, 2H), 5.40 (ab-q, 2H), 7.03 (d, 1H), 7.19 (d, 1H), 7.32-7.39 (m, 4H), 7.45 (t, 1H) |
| 3-1 | 3.03 (t, 2H), 3.73 (t, 2H), 4.70 (s, 2H), 7.10-7.28 (m, 3H), 7.30-7.48 (m, 3H), 7.62 (d, 1H), 8.11 (d, 1H), 11.80 (brs, 1H). |
| 3-2 | 2.98 (t, 2H), 3.71 (t, 2H), 4.68 (s, 2H), 6.82-7.12 (m, 2H), 7.20-7.45 (m, 4H), 7.63 (d, 1H), 11.30 (brs, 1H). |
| 3-3 | 2.95 (t, 2H), 3.68 (t, 2H), 4.69 (s, 2H), 7.06 (d, 1H), 7.18-7.50 (m, 5H), 7.63 (d, 1H), 11.40 (brs, 1H). |
| 3-4 | 2.96 (t, 2H), 3.68 (t, 2H), 4.70 (s, 2H), 7.11 (d, 1H), 7.18-7.55 (m, 5H), 7.58-7.78 (m, 1H), 11.30 (brs, 1H). |

**[Table 193]**

| No. | CDCl₃/TMS *δ* (ppm) |
|---|---|
| 3-5 | 2.98 (t, 2H), 3.72 (t, 2H), 4.67 (s, 2H), 7.06 (d, 1H), 7.12-7.48 (m, 3H), 7.52-7.81 (m, 4H), 11.40 (brs, 1H). |
| 3-6 | 2.68 (s, 3H), 2.94 (t, 2H), 3.65 (t, 2H), 4.68 (s, 2H), 6.99 (d, 1H), 7.08-7.48 (m, 5H), 7.63 (d, 1H), 11.87 (brs, 1H). |
| 3-7 | 0.58-0.68 (m, 2H), 0.98-1.12 (m, 2H), 2.91 (t, 2H), 3.06-3.20 (m, 1H), 3.66 (t, 2H), 4.71 (s, 2H), 6.82-7.00 (m, 2H), 7.16-7.42 (m, 4H), 7.63 (d, 1H), 11.80 (brs, 1H). |
| 3-8 | 3.03 (t, 2H), 3.75 (t, 2H), 4.61 (s, 2H), 7.08-7.47 (m, 11H), 7.59 (d, 1H), 11.06 (brs, 1H). |
| 3-9 | 3.00 (t, 2H), 3.70 (t, 2H), 4.71 (s, 2H), 7.08-7.61 (m, 6H), 7.71 (d, 1H), 11.15 (brs, 1H). |
| 3-10 | 3.07 (t, 2H), 3.75 (t, 2H), 4.74 (s, 2H), 7.17-7.47 (m, 4H), 7.39 (t, 1H), 7.54 (d, 1H), 11.40 (brs, 1H). |
| 3-11 | 3.00 (t, 2H), 3.72 (t, 2H), 4.67 (s, 2H), 6.61 (d, 1H), 6.83 (d, 1H), 7.18-7.48 (m, 54H), 7.62 (d, 1H), 10.84 (brs, 1H), 11.59 (brs, 1H). |
| 3-12 | 2.88 (t, 2H), 3.64 (t, 2H), 3.90 (s, 3H), 4.66 (s, 2H), 6.73 (d, 1H), 6.87 (d, 1H), 7.10-7.52 (m, 4H), 7.64 (d, 1H), 11.59 (brs, 1H). |
| 3-13 | 2.40 (s, 3H), 3.00 (t, 2H), 3.68 (t, 2H), 4.62 (s, 2H), 6.98 (d, 1H), 7.06 (d, 1H), 7.13-7.53 (m, 4H), 7.61 (d, 1H), 11.71 (brs, 1H). |
| 3-14 | 2.96 (t, 2H), 3.68 (t, 2H), 4.68 (s, 2H), 6.72 (t, 1H), 7.09 (d, 1H), 7.17-7.48 (m, 5H), 7.63 (d, 1H), 11.40 (brs, 1H). |
| 3-15 | 3.06 (t, 2H), 3.77 (t, 2H), 4.70 (s, 2H), 7.12-7.55 (m, 6H), 7.58 (d, 1H), 11.40 (brs, 1H). |
| 3-16 | 2.49 (t, 1H), 2.89 (t, 2H), 3.64 (t, 2H), 4.66 (s, 2H), 4.83(d, 2H), 6.81 (d, 1H), 7.07 (d, 1H), 7.14-7.45 (m, 4H), 7.62 (d, 1H), 11.38 (brs, 1H). |
| 3-17 | 2.85 (s, 6H), 2.87 (t, 2H), 3.62 (t, 2H), 4.68 (s, 2H), 6.58 (d, 1H), 6.83 (d, 1H), 7.10-7.39 (m, 4H), 7.61 (d, 1H), 11.02 (brs, 1H). |
| 3-18 | 3.04 (t, 2H), 3.73 (t, 2H), 3.95 (s, 3H), 4.65 (s, 2H), 7.15-7.50 (m, 6H), 7.60 (d, 1H), 11.45 (brs, 1H). |
| 3-19 | 3.00 (t, 2H), 3.73 (t, 2H), 4.69 (s, 2H), 7.02-7.43 (m, 5H), 7.61 (d, 1H), 7.76 (dd, 1H), 11.61 (brs, 1H). |

**[Table 194]**

| No. | CDCl₃/TMS *δ* (ppm) |
|---|---|
| 3-20 | 3.00 (t, 2H), 3.73 (t, 2H), 4.68 (s, 2H), 7.12 (d, 1H), 7.13-7.43 (m, 4H), 7.62 (d, 1H), 8.07 (d, 1H), 11.57 (brs, 1H). |
| 3-21 | 2.98 (t, 2H), 3.73 (t, 2H), 4.68 (s, 2H), 7.06 (d, 1H), 7.11-7.59 (m, 4H), 7.61 (d, 1H), 8.22 (d, 1H), 11.57 (brs, 1H). |
| 3-22 | 2.98 (t, 2H), 3.72 (t, 2H), 4.67 (s, 2H), 7.08-7.46 (m, 4H), 7.50-7.80 (m, 3H), 11.70 (brs, 1H). |
| 3-23 | 2.35 (s, 3H), 2.98 (t, 2H), 3.70 (t, 2H), 4.69 (s, 2H), 7.05 (d, 1H), 7.11-7.48 (m, 4H), 7.62 (d, 1H), 7.93 (s, 1H), 11.86 (brs, 1H). |
| 3-24 | 1.21 (t, 3H), 2.64 (q, 2H), 2.98 (t, 2H), 3.71 (t, 2H), 4.68 (s, 2H), 7.07 (d, 1H), 7.12-7.40 (m, 4H), 7.62 (d, 1H), 7.94 (s, 1H), 11.91 (brs, 1H). |
| 3-25 | 1.32 (s, 9H), 2.99 (t, 2H), 3.71 (t, 2H), 4.69 (s, 2H), 7.10 (d, 1H), 7.12-7.52 (m, 4H), 7.62 (d, 1H), 8.12 (d, 1H), 11.86 (brs, 1H). |
| 3-26 | 3.08 (t, 2H), 3.76 (t, 2H), 4.71 (s, 2H), 7.06 (d, 1H), 7.10-7.70 (m, 11H), 8.15 (d, 1H), 11.87 (brs, 1H). |
| 3-27 | 3.11 (t, 2H), 3.78 (t, 2H), 4.71 (s, 2H), 7.14-7.44 (m, 4H), 7.62 (d, 1H), 7.68 (dd, 1H), 8.38 (s, 1H), 11.50 (brs, 1H). |
| 3-28 | 3.16 (t, 2H), 3.81 (t, 2H), 4.73 (s, 2H), 7.17-7.48 (m, 4H), 7.63 (d, 1H), 8.30 (dd, 1H), 8.94 (d, 1H), 11.27 (brs, 1H). |
| 3-29 | 2.95 (t, 2H), 3.70 (t, 2H), 4.69 (s, 2H), 5.38 (brs, 1H), 6.95 (dd, 1H), 7.05 (d, 1H), 7.14-7.48 (m, 3H), 7.53 (d, 1H), 7.63 (d, 1H), 11.69 (brs, 1H). |
| 3-30 | 2.96 (t, 2H), 3.71 (t, 2H), 3.84 (s, 3H), 4.69 (s, 2H), 6.99 (dd, 1H), 7.07 (d, 1H), 7.13-7.45 (m, 3H), 7.55-7.70 (m, 2H), 11.78 (brs, 1H). |
| 3-31 | 1.39 (t, 3H), 3.03 (t, 2H), 3.74 (t, 2H), 4.31 (q, 2H), 4.69 (s, 2H), 7.11-7.42 (m, 5H), 7.62 (d, 1H), 7.91 (d, 1H), 11.43 (brs, 1H). |
| 3-32 | 3.05 (t, 2H), 3.76 (t, 2H), 4.70 (s, 2H), 7.11-7.78 (m, 9H), 7.95 (d, 1H), 8.10-8.28 (m, 2H), 11.67 (brs, 1H). |
| 3-33 | 2.51 (t, 1H), 2.96 (t, 2H), 3.71 (t, 2H), 4.70 (d, 2H), 4.72 (s, 2H), 7.00-7.48 (m, 5H), 7.52-7.75 (m, 2H), 11.72 (brs, 1H). |
| 3-34 | 3.07 (t, 2H), 3.77 (t, 2H), 4.70 (s, 2H), 7.12-7.42 (m, 5H), 7.63 (d, 1H), 8.01 (d, 1H), 11.29 (brs, 1H). |

**[Table 195]**

| No. | CDCl₃/TMS *δ* (ppm) |
|---|---|
| 3-35 | 3.03 (t, 2H), 3.73 (t, 2H), 4.68 (s, 2H), 6.86 (dd, 1H), 6.91-7.08 (m, 1H), 7.11-7.42 (m, 3H), 7.62 (d, 1H), 8.11 (dd, 1H), 11.69 (brs, 1H). |
| 3-36 | 3.01 (t, 2H), 3.73 (t, 2H), 4.68 (s, 2H), 7.16 (d, 1H), 7.19 (t, 1H), 7.26-7.40 (m, 3H), 7.62 (d, 1H), 8.01 (d, 1H), 11.63 (brs, 1H). |
| 3-37 | 3.01 (t, 2H), 3.72 (t, 2H), 4.68 (s, 2H), 7.12-7.53 (m, 5H), 7.63 (d, 1H), 7.97 (d, 1H), 11.59 (brs, 1H). |
| 3-38 | 2.96 (t, 2H), 3.67 (t, 2H), 4.70 (s, 2H), 6.99 (t, 1H), 7.08-7.46 (m, 4H), 7.62 (d, 1H), 7.97 (d, 1H), 11.35 (brs, 1H). |
| 3-39 | 2.36 (s, 3H), 2.98 (t, 2H), 3.70 (t, 2H), 4.68 (s, 2H), 6.97 (s, 1H), 7.10-7.42 (m, 4H), 7.62 (d, 1H), 7.98 (d, 1H), 11.91 (brs, 1H). |
| 3-40 | 3.10 (t, 2H), 3.79 (t, 2H), 4.71 (s, 2H), 7.12-7.30 (m, 1H), 7.32-7.40 (m, 1H), 7.42 (s, 1H), 7.51-7.65 (m, 1H), 8.19 (d, 1H), 11.50 (brs, 1H). |
| 3-41 | 2.99 (t, 2H), 3.69 (t, 2H), 3.83 (s, 3H), 4.67 (s, 2H), 6.64 (d, 1H), 6.62 (dd, 1H), 7.14-7.46 (m, 3H), 7.62 (d, 1H), 8.04 (d, 1H), 12.00 (brs, 1H). |
| 3-42 | 3.06 (t, 2H), 3.74 (t, 2H), 4.70 (s, 2H), 7.11-7.42 (m, 5H), 7.63 (d, 1H), 7.92 (d, 1H), 11.56 (brs, 1H). |
| 3-43 | 3.13 (t, 2H), 3.74 (t, 2H), 4.69 (s, 2H), 7.15-7.43 (m, 4H), 7.50 (d, 1H), 7.62 (d, 1H), 8.03 (d, 1H), 11.59 (brs, 1H). |
| 3-44 | 2.28 (s, 3H), 2.95 (t, 2H), 3.71 (t, 2H), 4.69 (s, 2H), 7.11-7.44 (m, 5H), 7.62 (d, 1H), 7.97 (d, 1H), 11.88 (brs, 1H). |
| 3-45 | 3.40 (t, 2H), 3.75 (t, 2H), 4.73 (s, 2H), 7.18-7.47 (m, 3H), 7.53 (t, 1H), 7.64 (d, 1H), 8.45 (d, 1H), 11.30 (brs, 1H). |
| 3-46 | 2.99 (t, 2H), 3.68 (t, 2H), 3.84 (s, 3H), 4.68 (s, 2H), 6.99 (d, 1H), 7.12-7.42 (m, 4H), 7.62 (d, 1H), 7.72 (d, 2H), 11.81 (brs, 1H). |
| 3-47 | 2.32 (s, 3H), 2.87 (t, 2H), 3.70 (t, 2H), 4.68 (s, 2H), 7.06 (d, 1H), 7.10-7.47 (m, 5H), 7.62 (d, 1H), 8.02 (d, 1H), 11.59 (brs, 1H). |
| 3-48 | 3.14 (t, 2H), 3.23 (s, 3H), 3.73 (t, 2H), 4.69 (s, 2H), 7.15-7.49 (m, 5H), 7.61 (d, 1H), 8.08 (d, 1H), 11.57 (brs, 1H). |
| 3-49 | 3.13 (t, 2H), 3.77 (t, 2H), 4.71 (s, 2H), 7.17-7.52 (m, 5H), 7.63 (d, 1H), 8.17 (dd, 1H), 11.39 (brs, 1H). |

**[Table 196]**

| No. | CDCl₃/TMS *δ* (ppm) |
|---|---|
| 3-50 | 1.39 (t, 3H), 2.96 (t, 2H), 3.72 (t, 2H), 4.32 (q, 2H), 4.69 (s, 2H), 7.12-7.49 (m, 5H), 7.62 (d, 1H), 8.03 (d, 1H), 11.63 (brs, 1H). |
| 3-51 | 2.94 (t, 2H), 3.71 (t, 2H), 4.69 (s, 2H), 7.11-7.48 (m, 5H), 7.49-7.78 (m, 4H), 8.00-8.29 (m, 3H), 11.59 (brs, 1H). |
| 3-52 | 2.51 (t, 1H), 3.02 (t, 2H), 3.69 (t, 2H), 4.68 (s, 2H), 4.72 (d, 2H), 7.12 (d, 1H), 7.14-7.43 (m, 4H), 7.61 (d, 1H), 7.76 (d, 1H), 11.81 (brs, 1H). |
| 3-53 | 2.24 (d, 3H), 2.93 (t, 2H), 3.68 (t, 2H), 4.67 (s, 2H), 6.84 (d, 1H), 7.10-7.42 (m, 4H), 7.62 (d, 1H), 11.42 (brs, 1H). |
| 3-54 | 3.12 (t, 2H), 3.78 (t, 2H), 4.71 (s, 2H), 7.06 (d, 1H), 7.12-7.53 (m, 4H), 7.62 (d, 1H), 8.34 (dd, 1H), 11.10 (brs, 1H). |
| 3-55 | 2.91 (t, 2H), 3.69 (t, 2H), 4.69 (s, 2H), 7.03 (d, 1H), 7.22 (dt, 1H), 7.31-7.43 (m, 2H), 7.49 (d, 1H), 7.63 (d, 1H), 11.72 (brs, 1H). |
| 3-56 | 2.38 (s, 3H), 2.87 (t, 2H), 3.66 (t, 2H), 4.69 (s, 2H), 6.97 (d, 1H), 7.16-7.48 (m, 4H), 7.63 (d, 1H), 11.45 (brs, 1H). |
| 3-57 | 2.98 (t, 2H), 3.70 (t, 2H), 4.67 (s, 2H), 6.68-6.88 (m, 1H), 7.17-7.48 (m, 4H), 7.63 (d, 1H), 11.27 (brs, 1H). |
| 3-58 | 2.33 (s, 3H), 2.93 (t, 2H), 3.67 (t, 2H), 4.66 (s, 2H), 6.76 (s, 1H), 6.84 (d, 1H), 7.18-7.43 (m, 3H), 7.63 (d, 1H), 11.41 (brs, 1H). |
| 3-59 | 0.78-0.88 (m, 2H), 1.00-1.11 (m, 2H), 1.80-1.91 (m, 1H), 2.91 (t, 2H), 3.66 (t, 2H), 4.65 (s, 2H), 6.64 (s, 1H), 6.66 (d, 1H), 7.18-7.40 (m, 3H), 7.62 (dd, 1H), 11.30 (brs. 1H). |
| 3-60 | 3.03 (t, 2H), 3.75 (t, 2H), 4.69 (s, 2H), 7.18-7.47 (m, 5H), 7.63 (d, 1H), 11.40 (brs, 1H). |
| 3-61 | 2.93 (t, 2H), 3.68 (t, 2H), 4.68 (s, 2H), 7.08 (s, 1H), 7.13-7.47 (m, 4H), 7.63 (d, 1H), 11.18 (brs, 1H). |
| 3-62 | 2.29 (s, 3H), 2.64 (s, 3H), 2.89 (t, 2H), 3.62 (t, 2H), 4.66 (s, 2H), 6.80 (s, 1H), 6.93 (s, 1H), 7.11-7.23 (m, 1H), 7.24-7.41 (m, 2H), 7.63 (d, 1H), 11.97 (brs, 1H). |
| 3-63 | 2.99 (t, 2H), 3.73 (t, 2H), 4.68 (s, 2H), 6.92-7.03 (m, 1H), 7.11-7.50 (m, 4H), 7.63 (d, 1H), 11.15 (brs, 1H). |
| 3-64 | 3.06 (t, 2H), 3.73 (t, 2H), 4.68 (s, 2H), 6.99 (t, 1H), 7.12-7.48 (m, 4H), 7.61 (d, 1H), 11.17 (brs, 1H). |

**[Table 197]**

| No. | CDCl₃/TMS *δ* (ppm) |
|---|---|
| 3-65 | 3.03 (t, 2H), 3.73 (t, 2H), 4.68 (s, 2H), 6.76-7.10 (m, 1H), 7.12-7.38 (m, 2H), 7.63 (d, 1H), 8.04-8.22 (m, 1H), 11.68 (brs, 1H). |
| 3-66 | 2.23 (s, 3H), 2.87 (t, 2H), 3.69 (t, 2H), 4.68 (s, 2H), 6.81-6.95 (m, 1H), 7.15-7.46 (m, 4H), 7.63 (d, 1H), 11.41 (brs, 1H). |
| 3-67 | 3.03 (t, 2H), 3.73 (t, 2H), 4.68 (s, 2H), 6.87 (dd, 1H), 7.12 (dt, 1H), 7.16-7.48 (m, 2H), 7.63 (d, 1H), 8.12 (t, 1H), 11.68 (brs, 1H). |
| 3-68 | 2.93 (t, 2H), 3.67 (t, 2H), 3.81 (s, 3H), 4.67 (s, 2H), 6.88-7.04 (m, 2H), 7.12-7.45 (m, 3H), 7.62 (d, 1H), 11.40 (brs, 1H). |
| 3-69 | 3.06 (t, 2H), 3.74 (t, 2H), 4.70 (s, 2H), 7.11-7.41 (m, 4H), 7.63 (d, 1H), 7.92 (d, 1H), 11.57 (brs, 1H). |
| 3-70 | 3.05 (t, 2H), 3.69 (t, 2H), 4.70 (s, 2H), 7.12-7.43 (m, 5H), 7.63 (d, 1H), 11.13 (brs, 1H). |
| 3-71 | 3.03 (t, 2H), 3.74 (t, 2H), 4.68 (s, 2H), 6.80 (dd, 1H), 7.11 (t, 1H), 7.20-7.43 (m, 3H), 7.62 (d, 1H), 10.67 (brs, 1H), 11.33 (brs, 1H). |
| 3-72 | 2.91 (t, 2H), 3.66 (t, 2H), 3.89 (s, 3H), 4.67 (s, 2H), 6.83 (dd, 1H), 7.14 (t, 1H), 7.18-7.43 (m, 3H), 7.64 (d, 1H), 11.40 (brs, 1H). |
| 3-73 | 3.00 (t, 2H), 3.45 (s, 3H), 3.71 (t, 2H), 4.68 (s, 2H), 7.12-7.48 (m, 5H), 7.63 (d, 1H), 11.34 (brs, 1H). |
| 3-74 | 2.84 (s, 6H), 2.91 (t, 2H), 3.67 (t, 2H), 4.69 (s, 2H), 6.72-6.88 (m, 1H), 7.04 (t, 1H), 7.18-7.42 (m, 3H), 7.61 (d, 1H), 11.40 (brs, 1H). |
| 3-75 | 2.88-3.10 (m, 6H), 3.69 (t, 2H), 3.81-4.00 (m, 4H), 4.67 (s, 2H), 6.81-6.98 (m, 1H), 7.12 (t, 1H), 7.17-7.43 (m, 3H), 7.62 (d, 1H), 11.78 (brs, 1H). |
| 3-76 | 2.39 (s, 3H), 3.00 (t, 2H), 3.67 (t, 2H), 4.69 (s, 2H), 7.04-7.44 (m, 5H), 7.63 (d, 1H), 11.40 (brs, 1H). |
| 3-77 | 2.95 (t, 2H), 3.65 (s, 3H), 3.72 (t, 2H), 4.75 (s, 2H), 7.18-7.42 (m, 4H), 7.67 (d, 1H), 8.17-8.29 (m, 1H), 11.89 (brs, 1H). |
| 3-78 | 2.38 (s, 3H), 2.41 (s, 3H), 3.07 (t, 2H), 3.64 (t, 2H), 4.68 (s, 2H), 7.15 (d, 1H), 7.16-7.44 (m, 4H), 7.62 (d, 1H), 11.43 (brs, 1H). |
| 3-79 | 3.00 (t, 2H), 3.73 (t, 2H), 4.68 (s, 2H), 7.12-7.46 (m, 4H), 7.63 (d, 1H), 8.19 (s, 1H), 11.40 (brs, 1H). |

**[Table 198]**

| No. | CDCl₃/TMS *δ* (ppm) |
|---|---|
| 3-80 | 2.26 (d, 3H), 2.98 (t, 2H), 3.70 (t, 2H), 4.67 (s, 2H), 6.80 (d, 1H), 7.12-7.42 (m, 3H), 7.63 (d, 1H), 7.98 (d, 1H), 11.76 (brs, 1H). |
| 3-81 | 2.36 (s, 3H), 2.97 (t, 2H), 3.70 (t, 2H), 4.67 (s, 2H), 7.16 (s, 1H), 7.16-7.50 (m, 3H), 7.63 (d, 1H), 7396 (s, 1H), 11.69 (brs, 1H). |
| 3-82 | 3.14 (t, 2H), 3.81 (t, 2H), 4.71 (s, 2H), 7.13 (d, 1H), 7.17-7.48 (m, 3H), 7.63 (d, 1H), 8.81 (d, 1H), 11.13 (brs, 1H). |
| 3-83 | 3.12 (t, 2H), 3.80 (t, 2H), 4.70 (s, 2H), 7.18-7.46 (m, 4H), 7.61 (d, 1H), 8.57 (d, 1H), 11.13 (brs, 1H). |
| 3-84 | 3.09 (t, 2H), 3.75 (t, 2H), 4.69 (s, 2H), 7.03-7.52 (m, 4H), 7.62 (d, 1H), 7.81-7.96 (m, 1H), 11.47 (brs, 1H). |
| 3-85 | 3.07 (t, 2H), 3.73 (t, 2H), 4.69 (s, 2H), 7.03 (t, 1H), 7.12-7.51 (m, 3H), 7.63 (d, 1H), 10.88 (brs, 1H). |
| 3-86 | 3.01 (t, 2H), 3.74 (t, 2H), 4.70 (s, 2H), 7.06 (d, 1H), 7.12-7.43 (m, 4H), 7.62 (d, 1H), 10.84 (brs, 1H). |
| 3-87 | 3.10 (t, 2H), 3.76 (t, 2H), 4.78 (s, 2H), 7.17-7.29 (m, 2H), 7.32-7.45 (m, 2H), 7.62 (t, 1H), 7.66 (dt, 1H), 7.80 (d, 1H), 7.91 (d, 1H), 9.35 (d, 1H), 11.84 (brs, 1H). |
| 3-88 | 3.22 (t, 2H), 3.81 (t, 2H), 4.76 (s, 2H), 7.21 (t, 1H), 7.28-7.42 (m, 2H), 7.62 (d, 1H), 8.17 (d, 1H), 9.05 (s, 1H), 11.53 (brs, 1H). |
| 3-89 | 3.20 (t, 2H), 3.80 (t, 2H), 4.76 (s, 2H), 7.17-7.42 (m, 3H), 7.42-7.62 (m, 3H), 7.65 (d, 1H), 7.78 (d, 1H), 7.95 (d, 1H), 8.70 (s, 1H), 11.88 (brs, 1H). |
| 3-90 | 2.92-3.13 (m, 2H), 3.49 (s, 3H), 3.67 (t, 2H), 4.51 (d, 1H), 5.38 (d, 1H), 7.01-7.25 (m, 2H), 7.30-7.48 (m, 4H). |
| 3-91 | 2.44 (t, 1H), 2.88-3.10 (m, 2H), 3.62 (t, 2H), 4.43-4.80 (m, 3H), 5.39 (d, 1H), 6.96-7.21 (m, 2H), 7.30-7.49 (m, 4H). |
| 3-92 | 2.62-2.83 (m, 2H), 2.83-3.05 (m, 2H), 3.79 (3,2H), 4.57 (d, 1H), 4.50-4.63 (m, 2H), 5.13 (d, 1H), 6.81 (d, 1H), 6.91-7.20 (m, 4H), 7.21-7.40 (m, 3H). |
| 3-93 | 1.21-1.36 (m, 2H), 1.81-1.96 (m, 2H), 2.87-3.10 (m, 2H), 3.50-3.76 (m, 4H), 3.81-4.12 (m, 4H), 4.53 (d, 1H), 4.55 (t, 1H), 5.37 (d, 1H), 6.97-7.22 (m, 2H), 7.23-7.46 (m, 4H). |
| 3-94 | 3.10 (t, 2H), 3.67-3.88 (m, 2H), 4.38 (d, 1H), 4.84-5.06 (d, 2H), 5.16 (d, 1H), 7.02 (dt, 1H), 7.18 (dt, 1H), 7.35-7.58 (m, 4H). |

**[Table 199]**

| No. | CDCl₃/TMS *δ* (ppm) |
|---|---|
| 3-95 | 1.23 (t, 3H), 2.82-3.08 (m, 2H), 3.48-3.89 (m, 4H), 4.68 (d, 1H), 5.02-5.18 (m, 2H), 5.23 (d, 1H), 6.97-7.21 (m, 2H), 7.27-7.49 (m, 4H). |
| 3-96 | 1.29 (dd, 6H), 2.89-3.09 (m, 2H), 3.50-3.69 (m, 2H), 4.68 (d, 1H), 4.85 (sept, 1H), 5.17 (d, 1H), 5.67 (d, 1H), 5.82 (d, 1H), 6.94-7.21 (m, 2H), 7.30-7.50 (m, 4H). |
| 3-97 | 2.30 (s, 3H), 2.90-3.12 (m, 2H), 3.51-3.79 (m, 2H), 4.12 (d, 1H), 5.23 (d, 1H), 7.06 (dt, 1H), 7.12-7.28 (m, 2H), 7.38-7.54 (m, 3H). |
| 3-98 | 1.11 (t, 3H), 2.30-2.51 (m, 2H), 2.92-3.11 (m, 2H), 3.51-3.70 (m, 2H), 4.37 (d, 1H), 5.31 (d, 1H), 7.04 (dt, 1H), 7.10-7.29 (m, 2H), 7.38-7.55 (m, 3H). |
| 3-99 | 0.90 (t, 3H), 1.57-1.71 (m, 2H), 2.28-2.40 (m, 2H), 2.91-3.12 (m, 2H), 3.50-3.69 (m, 2H), 4.38 (d, 1H), 5.31 (d, 1H), 7.06 (dt, 1H), 7.12-7.28 (m, 2H), 7.39-7.56 (m, 3H). |
| 3-100 | 1.11 (d, 3H), 1.23 (d, 3H), 2.50-2.67 (m, 2H), 2.90-3.11 (m, 2H), 3.59 (t, 2H), 4.42 (d, 1H), 5.32 (d, 1H), 7.07 (dt, 1H), 7.13-7.29 (m, 2H), 7.37-7.56 (m, 3H). |
| 3-101 | 0.92 (dd, 6H), 2.08-2.28 (m, 3H), 2.89-3.11 (m, 2H), 3.49-3.68 (m, 2H), 4.39 (d, 1H), 5.31 (d, 1H), 7.04 (dt, 1H), 7.11-7.27 (m, 2H), 7.34-7.58 (m, 3H). |
| 3-102 | 1.03 (s, 9H), 2.14-2.38 (m, 2H), 2.90-3.12 (m, 2H), 3.50-3.67 (m, 2H), 4.38 (d, 1H), 5.32 (d, 1H), 7.04 (dt, 1H), 7.12-7.24 (m, 2H), 7.34-7.53 (m, 3H). |
| 3-103 | 2.92-3.12 (m, 2H), 3.53-3.78 (m, 2H), 4.29 (d, 2H), 4.47 (d, 1H), 5.30 (d, 1H), 7.02 (dt, 1H), 7.11-7.31 (m, 2H), 7.40-7.58 (m, 3H). |
| 3-104 | 0.82-1.06 (m, 2H), 1.01-1.31 (m, 2H), 1.44-1.58 (m, 1H), 3.04 (t, 2H), 3.63 (t, 2H), 4.47 (d, 1H), 5.38 (d, 1H), 6.93-7.10 (m, 2H), 7.30 (d, 1H), 7.33-7.54 (m, 4H). |
| 3-105 | 0.81-1.08 (m, 2H), 1.10-1.31 (m, 2H), 1.42-1.59 (m, 1H), 2.91-3.11 (m, 2H), 3.51-3.70 (m, 2H), 4.49 (d, 1H), 5.40 (d, 1H), 7.13 (d, 1H), 7.26-7.58 (m, 6H). |
| 3-106 | 0.81-1.06 (m, 2H), 1.07-1.31 (m, 2H), 1.45-1.62 (m, 2H), 3.02 (t, 2H), 3.52-3.71 (m, 2H), 4.52 (d, 1H), 5.31 (d, 1H), 7.22 (s, 1H), 7.26-7.55 (m, 5H), 8.04 (d, 1H). |
| 3-107 | 0.78-1.36 (m, 5H), 2.92-3.1.4 (m, 2H), 3.63 (t, 2H), 4.49 (d, 1H), 5.35 (d, 1H), 7.00-7.22 (m, 2H), 7.30 (d, 1H), 7.39-7.58 (m, 3H). |
| 3-108 | 0.80-1.04 (m, 2H), 1.05-1.31 (m, 2H), 1.42-1.58 (m, 1H), 2.27 (s, 3H), 2.85-3.01 (m, 2H), 3.62 (t, 2H), 4.46 (d, 1H), 5.37 (d, 1H), 6.98 (dt, 1H), 7.20-7.58 (m, 5H). |
| 3-109 | 0.75-1.72 (m, 7H), 2.91-3.11 (m, 2H), 3.52-3.69 (m, 2H), 4.37-4.55 (m, 1H), 5.26-5.49 (m, 1H), 7.03-7.08 (m, 1H), 7.17-7.20 (m, 1H), 7.28-7.30 (m, 1H), 7.43-7.52 (m, 3H) |

**[Table 200]**

| No. | CDCl₃/TMS *δ* (ppm) |
|---|---|
| 3-110 | 0.84-0.91 (m, 3H), 1.15-1.50 (m, 6H), 2.87-3.10 (m, 2H), 3.47-3.52 (m, 1H), 3.61-3.70 (m, 1H), 4.35(d, 0.5H), 4.53 (d, 0.5H), 5.31 (d, 0.5H), 5.45 (d, 0.5H), 7.03-7.05 (m, 1H), 7.17-7.35(m, 2H), 7.43-7.51 (m ,3H) |
| 3-111 | 1.10 (s, 3H), 1.39 (d, 1H), 2.49 (d, 1H), 2.90-3.18 (m, 2H), 3.58-3.73 (m, 2H), 4.42 (d, 1H), 5.55 (d, 1H), 7.06 (dt, 1H), 7.20 (dt, 1H), 7.40-7.64 (m, 3H), 7.70 (d, 1H). |
| 3-112 | 1.82-2.05 (m, 4H), 2.20-2.57 (m, 2H), 2.90-3.20 (m, 3H), 3.52-3.58 (m, 2H), 4.33 (d, 1H), 5.35 (d, 1H), 7.04 (dt, 1H), 7.11-7.23 (m, 2H), 7.35-7.55 (m, 3H). |
| 3-113 | 1.38-2.04 (m, 8H), 2.52-2.70 (m, 1H), 2.90-3.12 (m, 2H), 3.60 (t, 2H), 4.39 (d, 1H), 5.37 (d, 1H), 7.04 (dt, 1H), 7.11-7.30 (m, 2H), 7.36-7.57 (m, 3H). |
| 3-114 | 1.10-1.30 (m, 2H), 1.32-2.92 (m, 8H), 2.25-2.40 (m, 1H), 2.90-3.10 (m, 2H), 3.46-3.61 (m, 2H), 4.46 (d, 1H), 5.26 (d, 1H), 7.04 (dt, 1H), 7.11-7.29 (m, 2H), 7.38-7.58 (m, 3H). |
| 3-115 | 2.91-3.10 (m, 2H), 3.51-3.67 (m, 2H), 4.50 (d, 1H), 5.19 (d, 1H), 5.89 (d, 1H), 6.07-6.19 (d, 1H), 6.65 (d, 1H), 7.06 (dt, 1H), 7.12-7.29 (m, 2H), 7.38-7.58 (m, 3H). |
| 3-116 | trans : 1.83 (dd, 3H), 2.89-3.12 (m, 2H), 3.50-3.69 (m, 2H), 4.48 (d, 1H), 5.21 (d, 1H), 5.77 (dd, 1H), 7.05 (dt, 1H), 7.11-7.31 (m, 2H), 7.36-7.59 (m, 3H). |
| 3-117 | 1.82 (d, 3H), 2.20 (d, 3H), 2.89-3.10 (m, 2H), 3.40-3.58 (m, 2H), 4.47 (d, 1H), 5.25 (d, 1H), 7.02 (dt, 1H), 7.12-7.28 (m, 2H), 7.33-7.53 (m, 3H). |
| 3-118 | 1.85 (s, 3H), 2.88-3.13 (m, 2H), 3.52-3.68 (m, 2H), 4.47 (d, 1H), 5.43 (d, 1H), 5.46 (s, 1H), 5.50 (s, 1H), 7.05 (dt, 1H), 7.11-7.28 (m, 2H), 7.32-7.50 (m, 3H). |
| 3-119 | 1.26 (t, 3H), 2.90-3.13 (m, 2H), 3.51-3.70 (m, 2H), 3.72-3.99 (m, 2H), 4.48 (d, 1H), 5.10-5.29 (m, 2H), 7.02 (dt, 1H), 7.10-7.30 (m, 2H), 7.32-7.55 (m, 3H), 7.75 (d, 1H). |
| 3-120 | 2.89-3.12 (m, 2H), 3.48-3.65 (m, 2H), 4.57 (d, 1H), 5.41 (d, 1H), 6.97-7.09 (m, 1H), 7.12-7.48 (m, 9H), 7.55 (d, 1H). |
| 3-121 | 2.91-3.10 (m, 2H), 3.62 (t, 2H), 4.61 (d, 1H), 5.42 (d, 1H), 6.38 (dd, 1H), 6.51 (d, 1H), 6.97-7.21 (m, 2H), 7.21-7.41 (m, 2H), 7.47 (d, 1H), 7.54 (d, 2H). |
| 3-122 | 2.90-3.09 (m, 2H), 3.56 (t, 2H), 4.61 (d, 1H), 5.29 (d, 1H), 6.94 (t, 1H), 7.02 (dt, 1H), 7.11-7.23 (m, 2H), 7.30-7.48 (m, 2H), 7.49-7.53 (m, 3H). |
| 3-123 | 2.88-3.08 (m, 2H), 3.52 (t, 2H), 3.96 (s, 3H), 4.57-4.78 (m, 1H), 5.19-5.38 (m, 1H), 5.80 (dd, 1H), 5.92 (dd, 1H), 6.83 (s, 1H), 7.03 (dt, 1H), 7.17 (dt, 1H), 7.30-7.57 (m, 4H). |
| 3-124 | 2.92-3.08 (m, 2H), 3.51-3.69 (m, 2H), 4.54 (d, 1H), 5.24 (d, 1H), 6.36 (d, 1H), 6.81 (s,1H), 7.04 (dt, 1H), 7.17 (dt, 1H), 7.25 (s, 1H), 7.31 (d,1H), 7.37-7.63 (m, 3H). |

**[Table 201]**

| No. | CDCI₃/TMS *δ* (ppm) |
|---|---|
| 3-125 | 1.79-2.29 (m, 4H), 2.89-3.10 (m, 2H), 3.52-3.60 (m, 1H), 3.64 (t, 1H), 3.80-3.99 (m, 2H), 4.28-4.49 (m, 1.5H), 4.63 (d, 0.5H), 5.22 (d, 1H), 5.38 (d, 1H), 7.04 (dt, 1H). 7.11-7.55 (m, 5H). |
| 3-126 | 1.90-2.40 (m, 2H), 2.94-3.26 (m, 3H), 3.59-4.08 (m, 6H), 4.38 (dd, 1H), 5.28 (dd, 1H), 6.98-7.10 (m, 1H), 7.14-7.28 (m, 2H), 7.40-7.57 (m, 3H). |
| 3-127 | 1.54-1.89 (m, 3H), 1.90-2.09 (m, 1H), 2.63-2.79 (m, 1H), 2.94-3.12 (m, 2H), 3.11-2.27 (m, 1H), 3.30-3.42 (m, 1H), 3.57-3.72 (m, 2H), 3.81-4.02 (m, 2H), 4.38 (d, 1H). 5.27 (d, 1H), 7.17 (dt, 1H, 7.13-7.30 (m, 2H), 7.37-7.59 (m, 3H). |
| 3-128 | 0.02-0.18 (m, 2H), 0.47-0.62 (m, 2H), 0.95-1.10 (m, 2H), 2.27-2.42 (m, 2H), 2.90-3.15 (m, 2H), 3.50-3.70 (m, 2H), 4.37 (d, 1H), 5.28 (d, 1H), 7.06 (dt, 1H), 7.11-7.28 (m, 2H), 7.32-7.52 (m, 3H). |
| 3-129 | 2.56-2.86 (m, 3H), 3.02-3.18 (m, 2H), 3.72 (t, 2H), 3.86-3.98 (m, 2H), 4.35 (d, 1H), 5.29 (d, 1H), 7.06 (dt, 1H), 7.16-7.31 (m, 2H), 7.38-7.56 (m, 3H). |
| 3-130 | 2.91-3.09 (m, 2H), 3.39 (s, 3H), 3.52-3.69 (m, 2H), 4.21 (s, 2H), 4.39 (d, 1H), 5.27 (d, 1H), 7.02 (dt, 1H), 7.11-7.28 (m, 2H), 7.37-7.57 (m, 3H). |
| 3-131 | **1.17** (t, 3H), 2.92-3.09 (m, 2H), 3.41-3.68 (m, 4H), 4.16-4.28 (m, 2H), 4.38 (d, 1H), 5.27 (d, 1H), 7.03 (dt, 1H), 7.11-7.27 (m, 2H), 7.36-7.57 (m, 3H). |
| 3-132 | 2.58-2.71 (m, 2H), 2.92-3.11 (m, 2H), 3.29 (s, 3H), 3.51-3.69 (m, 4H), 4.47 (d, 1H), 5.23 (d, 1H), 7.03 (dt, 1H), 7.12-7.28 (m, 2H), 7.37-7.55 (m, 3H). |
| 3-133 | 1.02 (s, 3H), 2.67 (t, 2H), 2.89-3.08 (m, 2H), 3.47-3.62 (m, 2H), 3.78-4.06 (m, 2H), 4.00 (d, 1H), 5.19 (d, 1H), 7.02 (dt, 1H), 7.12-7.28 (m, 2H), 7.32-7.56 (m, 9H). 7.58-7.69 (m, 4H). |
| 3-134 | 2.12 (s, 3H), 3.02 (t, 2H), 3.60 (t, 2H), 4.51 (d, 1H), 4.75 (d, 1H), 4.83 (d, 1H), 5.19 (d, 1H), 7.03 (m, 1H), 7.17 (dt, 1H), 7.32 (d, 1H), 7.40-7.58 (m, 3H). |
| 3-135 | 2.19 (s, 3H), 2.92-3.12 (m, 2H), 3.25 (d, 1H), 3.31 (d, 1H), 3.64 (t, 2H), 4.43 (d, 1H), 5.33 (d, 1H), 7.03 (dt, 1H), 7.18 (dt, 1H), 7.30-7.57 (m, 4H). |
| 3-136 | 1.24 (t, 3H), 2.58-2.73 (m, 2H), 2.92-3.13 (m, 2H), 3.21-3.41 (m, 2H), 3.63 (t, 2H), 4.44 (d, 1H), 5.34 (d, 1H), 7.03 (dt, 1H), 7.16 (dt, 1H), 7.35 (d, 1H), 7.39-7.57 (m, 3H). |
| 3-137 | 2.71 (s, 3H), 2.67-2.92 (m, 4H), 2.98-3.12 (m, 2H), 3.63 (t, 2H), 4.41 (d, 1H), 5.26 (d, 1H), 7.02 (dt, 1H), 7.18 (dt, 1H), 7.26 (d, 1H), 7.38-7.58 (m, 3H). |
| 3-138 | 2.91-3.11 (m, 2H), 3.47 (s, 3H), 3.64 (t, 2H), 4.48 (d, 1H), 5.35 (d, 1H), 6.97-7.22 (m, 2H), 7.26-7.48 (m, 4H). |
| 3-139 | 1.32 (t, 3H), 2.89-3.09 (m, 2H), 3.50 (t, 2H), 4.26-4.46 (m, 2H), 4.74 (d, 1H), 5.03 (d, 1H), 7.11-7.28 (m, 2H), 7.32-7.51 (m, 5H), 8.16 (d, 1H). |

**[Table 202]**

| No. | CDCI₃/TMS *δ* (ppm) |
|---|---|
| 3-140 | 1.32 (t, 3H), 2.86-3.08 (m, 2H), 3.47 (t, 2H), 4.28-4.48 (m, 2H), 4.71 (d, 1H), 5.03 (d, 1H), 6.98 (d, 1H), 7.05 (dt, 1H), 7.24 (t, 1H), 7.32-7.52 (m, 4H). |
| 3-141 | 0.63-0.78 (m, 2H), 1.00-1.09 (m, 2H), 1.04 (t, 3H), 2.89 (t, 2H), 3.20-3.32 (m, 1H), 3.44 (t, 2H), 4.28-4.47 (m, 2H), 4.76 (d, 1H), 5.05 (d, 1H), 6.90 (d, 1H), 6.96 (d, 1H), 7.18-7.41 (m, 3H), 7.43-7.55 (m, 2H). |
| 3-142 | 1.34 (t, 3H), 2.91-3.09 (m, 2H), 3.51 (t, 2H), 4.31-4.48 (m, 2H), 4.84 (d, 1H), 4.98 (d, 1H), 7.20-7.28 (m, 1H), 7.33-7.58 (m, 5H), 7.76 (d, 1H). |
| 3-143 | 1.34 (t, 3H), 2.81-3.09 (m, 2H), 3.41-3.57 (m, 2H), 4.22-4.41 (m, 2H), 4.76 (d, 1H), 4.92 (d, 1H), 6.61 (d, 1H), 6.84 (d, 1H), 7.18-7.58 (m, 5H), 12.47 (brs, 1H). |
| 3-144 | 1.26 (dt, 3H), 2.96 (t, 2H), 3.45-3.59 (m, 2H), 4.32 (dq, 2H), 4.63 (d, 1H), 4.94 (d, 1H), 7.19 (d, 1H), 7.18-7.48 (m, 11H). |
| 3-145 | 1.32 (t, 3H), 2.29 (s, 3H), 2.81-2.99 (m, 2H), 3.49 (t, 2H), 4.22-4.42 (m, 2H), 4.72 (d, 1H), 5.03 (d, 1H), 7.16-7.53 (m, 6H), 8.04 (d, 1H). |
| 3-146 | 1.32 (t, 3H), 2.34 (s, 3H), 2.80-2.98 (m, 2H), 3.44 (t, 2H), 4.29-4.46 (m, 2H), 4.68 (d, 1H), 5.03 (d, 1H), 6.78 (s, 1H), 6.87 (d, 1H), 7.22 (d, 1H), 7.32-7.50 (m, 3H). |
| 3-147 | 0.70-0.80 (m, 2H), 1.00-1.11 (m, 2H), 1.04 (t, 3H), 1.81-1.92 (m, 1H), 2.82-2.95 (m, 2H), 3.43 (t, 2H), 4.28-4.42 (m, 2H), 4.67 (d, 1H), 5.02 (d, 1H), 6.67 (s, 1H), 6.70 (d, 1H), 7.22 (d, 1H), 7.31-7.48 (m, 3H). |
| 3-148 | 1.34 (t, 3H), 2.88-3.08 (m, 2H), 3.50 (t, 2H), 4.28-4.47 (m, 2H), 4.82 (d, 1H), 4.92 (d, 1H), 7.18-7.32 (m, 2H), 7.36 (d, 1H), 7.36-7.52 (m, 2H). |
| 3-149 | 1.33 (t, 3H), 2.89-3.02 (m, 2H), 3.40-3.51 (m, 2H), 4.28-4.46 (m, 2H), 4.76 (d, 1H), 4.97 (d, 1H), 6.97-7.09 (m, 1H), 7.12-7.20 (m, 1H), 7.25 (t, 1H), 7.32-7.51 (d, 3H). |
| 3-150 | 1.32 (t, 3H), 2.78-3.10 (m, 2H), 4.33 (t, 2H), 4.19-4.47 (m, 2H), 4.76 (d, 1H), 4.97 (d, 1H), 6.88 (dd, 1H), 7.03 (dt, 1H), 7.24 (t, 1H), 7.24-7.52 (m, 2H), 8.04-8.22 (m, 1H). |
| 3-151 | 1.32 (t, 3H), 2.24 (s, 3H), 2.72-2.92 (m, 2H), 3.46 (t, 2H), 4.24-4.47 (m, 2H), 4.69 (d, 1H), 5.05 (d, 1H), 6.97 (dt, 1H), 7.24 (t, 2H), 7.31-7.52 (m, 3H). |
| 3-152 | 1.32 (t, 3H), 2.87-3.04 (m, 2H), 3.50 (t, 2H), 4.23-4.46 (m, 2H), 4.75 (d, 1H), 4.98 (d, 1H), 6.88 (d, 1H), 7.04 (dt, 1H), 7.24 (t, 2H), 7.31-7.50 (m, 2H). |
| 3-153 | 1.32 (t, 3H), 2.80-3.00 (m, 2H), 3.43 (t, 2H), 3.82 (s, 3H), 4.23-4.42 (m, 2H), 4.72 (d, 1H), 5.00 (d, 1H), 6.88-7.09 (m, 2H), 7.22 (d, 1H), 7.38 (dt, 1H), 7.40-7.52 (m, 2H). |
| 3-154 | 1.32 (t, 3H), 2.89-3.09 (m, 2H), 3.51 (t, 2H), 4.23-4.47 (m, 2H), 4.79 (d, 1H), 4.97 (d, 1H), 7.11-7.56 (m, 5H), 7.96 (d, 1H). |

**[Table 203]**

| No. | CDCI₃/TMS *δ* (ppm) |
|---|---|
| 3-155 | 1.33(t, 3H), 2.85 (t, 2H), 2.89 (s, 6H), 3.46 (t, 2H), 4.28-4.45 (m, 2H), 4.78 (d, 1H), 4.99 (d, 1H), 6.83 (dd, 1H), 7.05 (t, 1H), 7.21 (d, 1H), 7.30-7.52 (m, 3H). |
| 3-156 | 1.33 (t, 3H), 2.86 (t, 2H), 3.02-3.18 (m, 4H), 3.45 (t, 2H), 3.90 (t, 4H), 4.29-4.47 (m, 2H), 4.79 (d, 1H), 4.93 (d, 1H), 6.90 (dd, 1H), 7.11 (t, 1H), 7.22 (d, 1H), 7.32-7.51 (m, 3H). |
| 3-157 | 1.32 (t, 3H), 2.43 (s, 3H), 2.88-3.06 (m, 2H), 3.44 (t, 2H), 4.29-4.43 (m, 2H), 4.74 (d, 1H), 4.99 (d, 1H), 7.10-7.28 (m, 3H), 7.32-7.51 (m, 3H). |
| 3-158 | 1.32 (t, 3H), 2.42 (s, 3H), 2.43 (s, 3H), 2.93-3.10 (m, 2H), 3.42 (t, 2H), 4.29-4.43 (m, 2H), 4.73 (d, 1H), 5.00 (d, 1H), 7.21 (t, 2H), 7.30-7.51 (m, 4H). |
| 3-159 | 1.33 (t, 3H), 2.98 (t, 2H), 3.49 (t, 2H), 3.68 (s, 3H), 4.28-4.48 (m, 2H), 4.93 (s, 2H), 7.18-7.36 (m, 2H), 7.36-7.57 (m, 3H), 8.26 (dd, 1H). |
| 3-160 | 1.33 (t, 3H), 2.87-3.12 (m, 2H), 3.51 (t, 2H), 4.24-4.46 (m, 2H), 4.81 (d, 1H), 4.91 (d, 1H), 7.06-7.30 (m, 2H), 7.31-7.55 (m, 3H), 7.85-8.02 (m, 1H). |
| 3-161 | 0.90 (t, 3H), 1.69 (q, 2H), 2.87-3.01 (m, 2H), 3.48 (t, 2H), 4.27 (t, 2H), 4.76 (d, 1H), 4.98 (d, 1H), 6.95-7.32 (m, 3H), 7.35-7.55 (m, 3H). |
| 3-162 | 1.33 (dd, 6H), 2.84-3.02 (m, 2H), 3.47 (m, 2H), 4.79 (d, 1H), 4.94 (d, 1H), 5.12 (sept, 1H), 7.03 (dt, 1H), 7.16 (dt, 1H), 7.22 (d, 1H), 7.32-7.45 (m, 1H), 7.48 (d, 2H). |
| 3-163 | 0.90 (t, 3H), 1.21-1.41 (m, 2H), 1.55-1.72 (m, 2H), 2.87-3.03 (m, 2H), 3.47 (t, 2H), 4.31 (t, 2H), 4.77 (d, 1H), 4.97 (d, 1H), 7.14 (dt, 1H), 7.10-7.21 (m, 1H), 7.18 (d, 1H), 7.32-7.43 (m, 1H), 7.47 (d, 1H). |
| 3-164 | 0.88 (dd, 6H), 1.82-2.06 (m, 2H), 2.81-3.03 (m, 2H), 3.48 (t, 2H), 3.94-4.20 (m, 2H), 4.76 (d, 1H), 4.99 (d, 1H), 7.03 (dt, 1H), 7.08-7.29 (m, 2H), 7.30-7.54 (m, 3H). |
| 3-165 | 0.88 (dt, 3H), 1.31 (dd, 3H), 1.50-1.79 (m, 2H), 2.83-3.08 (m, 2H), 3.47 (t, 2H), 4.70-4.81 (m, 1H), 4.88-5.06 (m, 2H), 7.05 (dt, 1H), 7.10-7.42 (m, 3H), 7.47 (d, 2H). |
| 3-166 | 1.53 (s, 9H), 2.82-3.04 (m, 2H), 3.33-3.54 (m, 2H), 4.79 (d, 1H), 4.95 (d, 1H), 7.03 (dt, 1H), 7.10-7.29 (m, 2H), 7.33-7.51 (m, 3H). |
| 3-167 | 0.87 (t, 3H), 1.10-1.38 (m, 4H), 1.53-1.76 (m, 2H), 2.82-3.08 (m, 2H), 3.48 (t, 2H), 4.30 (t, 2H), 4.78 (d, 1H), 4.96 (d, 1H), 6.97-7.32 (m, 3H), 7.30-7.52 (m, 3H). |
| 3-168 | 0.87 (t, 3H), 1.11-1.38 (m, 6H), 1.53-1.71 (m, 2H), 2.82-3.06 (m, 2H), 3.47 (t, 2H), 4.21-3.39 (m, 2H), 4.77 (d, 1H), 4.96 (d, 1H), 7.06 (dt, 1H), 7.10-7.28 (m, 3H), 7.32-7.43 (m, 1H), 7.47 (d, 1H). |
| 3-169 | 0.88 (t, 3H), 1.10-1.38 (m, 8H), 1.51-1.73 (m, 2H), 2.81-3.06 (m, 2H), 3.47 (t, 2H), 4.20-4.39 (m, 2H), 4.78 (d, 1H), 4.96 (d, 1H), 7.03 (dt, 1H), 7.09-7.31 (m, 2H), 7.32-7.53 (m, 3H). |

**[Table 204]**

| No. | CDCT₃/TMS *δ* (ppm) |
|---|---|
| 3-170 | 0.85 (s, 9H), 2.88-3.02 (m, 2H), 3.42-3.56 (m, 2H), 3.88 (d, 1H), 4.08 (d, 1H), 4.73 (d, 1H), 5.04 (d, 1H), 7.03 (dt, 1H), 7.16 (dt, 1H), 7.21-7.27 (m, 1H), 7.34-7.52 (m, 3H). |
| 3-171 | 2.89-3.08 (m, 2H), 3.50 (t, 2H), 4.76 (d, 1H), 4.94 (d, 1H), 5.78 (d, 1H), 5.83 (d, 1H), 7.02 (dt, 1H), 7.16 (dt, 1H), 7.20-7.29 (m, 1H), 7.36-7.55 (m, 3H). |
| 3-172 | 2.88-3.08 (m, 2H), 3.40-3.58 (m, 2H), 4.76 (d, 1H), 4.81 (d, 1H), 4.96 (d, 1H), 5.06 (d, 1H), 7.03 (dt, 1H), 7.17 (dt, 1H), 7.21-7.32 (m, 1H), 7.38-7.57 (m, 3H). |
| 3-173 | 1.82 (dd, 3H), 2.89-3.03 (m, 2H), 3.40-3.57 (m, 2H), 4.68-4.98 (m, 2H), 6.55 (q, 1H), 6.95-7.28 (m, 3H), 7.35-7.56 (m, 3H). |
| 3-174 | 2.84-3.08 (m, 2H), 3.47 (t, 2H), 4.41-4.73 (m, 4H), 4.82 (d, 1H), 4.92 (d, 1H), 6.97 (d, 1H), 7.05 (t, 1H), 7.21-7.32 (m, 1H), 7.34-7.53 (m, 4H). |
| 3-175 | 2.95 (t, 2H), 3.48 (t, 2H), 4.42-4.70 (m, 4H), 4.76-4.97 (m, 2H), 7.02 (dt, 1H), 7.15 (dt, 1H), 7.21-7.32 (m, 1H), 7.37-7.56 (m, 3H). |
| 3-176 | 2.89-3.10 (m, 2H), 3.51 (t, 2H), 4.45 (dt, 2H), 4.82 (d, 1H), 4.90 (d, 1H), 6.01 (tt, 1H), 7.18 (d, 1H), 7.24 (d, 1H), 7.30-7.55 (m, 5H), 8.13 (d, 1H). |
| 3-177 | 2.88-3.08 (m, 2H), 3.49 (t, 2H), 4.40-4.57 (m, 2H), 4.81 (d, 1H), 4.89 (d, 1H), 6.03 (tt, 1H), 6.97 (d, 1H), 7.04 (dt, 1H), 7.19-7.28 (m, 1H), 7.32-7.55 (m, 4H). |
| 3-178 | 2.90-3.05 (m, 2H), 3.50 (t, 2H), 4.40-4.58 (m, 2H), 4.52 (d, 1H), 4.53 (d, 1H), 6.04 (tt, 1H), 7.03 (dt, 1H), 7.15 (dt, 1H), 7.21-7.30 (m, 1H), 7.40-7.54 (m, 3H). |
| 3-179 | 2.96 (t, 2H), 3.49 (t, 2H), 4.60-4.79 (m, 2H), 4.85 (s, 2H), 7.02 (dt, 1H), 7.16 (dt, 1H), 7.20-7.30 (m, 1H), 7.40-7.58 (m, 3H). |
| 3-180 | 2.88-3.08 (m, 2H), 3.50 (t, 3H), 3.72 (t, 3H), 4.50-4.62 (m, 2H), 4.82 (d, 1H), 4.93 (d, 1H), 7.03 (dt, 1H), 7.16 (dt, 1H), 7.19-7.30 (m, 1H), 7.35-7.55 (m, 3H). |
| 3-181 | 1.42 (d, 3H), 2.85-3.08 (m, 2H), 3.39-3.56 (m, 2H), 4.77 (dd, 1H), 4.92 (dd, 1H), 5.06-5.26 (m, 1H), 5.85 (ddt, 1H), 7.03 (dt, 1H), 7.10-7.30 (m, 2H), 7.34-7.53 (m, 3H). |
| 3-182 | 1.53 (dd, 3H), 2.82-3.05 (m, 2H), 3.39-3.55 (m, 2H), 4.71 (d, 1H), 4.80 (d, 1H), 4.90 (d, 1H), 5.00 (d, 1H), 5.26-5.47 (m, 1H), 6.95-7.32 (m, 4H), 7.32-7.56 (m, 2H). |
| 3-183 | 2.89-3.00 (m, 2H), 3.48 (t, 2H), 4.51-4.80 (m, 5H), 5.00 (d, 1H), 5.22-5.41 (m, 1H), 7.02 (dt, 1H), 7.15 (dt, 1H), 7.21-7.28 (m, 1H), 7.39-7.52 (m, 3H). |
| 3-184 | 0.70-0.90 (m, 4H), 2.82-3.06 (m, 2H), 3.47 (t, 2H), 4.28-4.39 (m, 1H), 4.77 (d, 1H), 4.91 (d, 1H), 7.03 (dt, 1H), 7.11-7.26 (m, 2H), 7.35-7.51 (m, 3H). |

**[Table 205]**

| No. | CDCl₃/TMS *δ* (ppm) |
|---|---|
| 3-185 | 1.51-1.91 (m, 2H), 2.06-2.21 (m, 2H), 2.26-2.47 (m, 2H), 2.82-3.01 (m, 2H), 3.47 (t, 2H), 4.77 (d, 1H), 4.96 (d, 1H), 5.07-5.19 (m, 1H), 7.03 (dt, 1H), 7.16 (dt, 1H), 7.19-7.28 (m, 1H), 7.33-7.49 (m, 3H). |
| 3-186 | 1.50-1.73 (m, 4H), 1.73-1.92 (m, 4H), 2.84-3.00 (m, 2H), 3.47 (t, 2H), 4.80 (d, 1H), 4.93 (d, 1H), 5.27-5.37 (m, 1H), 6.95-7.28 (m, 3H), 7.32-7.50 (m, 3H). |
| 3-187 | 1.15-1.41 (m, 4H), 1.41-1.68 (m, 2H), 1.68-2.00 (m, 4H), 2.88-3.04 (m, 2H), 3.47 (t, 2H), 4.76 (d, 1H), 4.82-4.96 (m, 1H), 4.98 (d, 1H), 7.03 (dt, 1H), 7.15 (dt, 1H), 7.20-7.30 (m, 1H), 7.35-7.51 (m, 3H). |
| 3-188 | 2.82-3.04 (m, 2H), 3.47 (t, 2H), 4.70-4.83 (m, 3H), 4.96 (d, 1H), 5.25-5.39 (m, 2H), 5.80-5.98 (m, 1H), 7.03 (dt, 1H), 7.15 (dt, 1H), 7.21-7.28 (m, 1H), 7.33-7.51 (m, 3H). |
| 3-189 | 1.40 (t, 3H), 2.82-3.00 (m, 2H), 3.46 (t, 2H), 4.70-4.99 (m, 2H), 5.10-5.32 (m, 2H), 5.32-5.49 (m, 1H), 5.71-5.89 (m, 1H), 7.03 (dt, 1H), 7.16 (dt, 1H), 7.19-7.30 (m, 1H), 7.33-7.52 (m, 3H). |
| 3-190 | 1.69 (s, 3H), 2.84-3.03 (m, 2H), 3.47 (t, 2H), 4.51-4.86 (m, 3H), 4.87-5.06 (m, 3H), 7.04 (dt, 1H), 7.15 (dt, 1H), 7.20-7.28 (m, 1H), 7.33-7.55 (m, 3H). |
| 3-191 | 1.35 (s, 3H), 1.71 (s, 3H), 2.82-3.09 (m, 2H), 3.42-3.59 (m, 2H), 4.21-4.31 (m, 1H), 4.37-4.51 (m, 2H), 5.25 (t, 1H), 5.34 (d, 1H), 7.04 (dt, 1H), 7.15 (dt, 1H), 7.21-7.43 (m, 4H). |
| 3-192 | 2.57 (t, 1H, 2.88-3.00 (m, 2H), 3.48 (t, 2H), 4.71-4.88 (m, 2H), 4.88-4.99 (m, 2H), 7.03 (dt, 1H), 7.15 (dt, 1H), 7.20-7.31 (m, 1H), 7.37-7.52 (m, 3H). |
| 3-193 | 1.57 (dd, 3H), 2.58 (dd, 1H), 2.82-3.08 (m, 2H), 3.40-3.57 (m, 2H), 4.70-4.99 (m, 2H), 5.42-5.57 (m, 1H), 6.92-7.28 (m, 3H), 7.32-7.53 (m, 3H). |
| 3-194 | 2.82-3.02 (m, 2H), 3.51 (t, 2H), 4.97 (d, 2H), 7.02 (dt, 1H), 7.15 (dt, 1H), 7.17-7.57 (m, 9H). |
| 3-195 | 2.82-3.09 (m, 2H), 3.56 (t, 2H), 4.93 (s, 2H), 6.90-7.23 (m, 2H), 7.30-7.63 (m, 6H), 8.19-8.30 (m, 2H). |
| 3-196 | 2.89-3.08 (m, 2H), 3.52 (t, 2H), 4.61-4.71 (m, 2H), 4.76 (d, 1H), 4.85 (q, 2H), 4.99 (d, 1H), 5.58 (sept, 1H), 7.18 (d, 1H), 7.25 (d, 1H), 7.33-7.53 (m, 5H), 8.14 (d, 1H). |
| 3-197 | 2.82-3.09 (m, 2H), 3.48 (t, 2H), 4.58-4.71 (m, 2H), 4.76 (d, 1H), 4.81-4.92 (m, 2H), 4.96 (d, 1H), 5.57-5.69 (m, 1H), 6.97 (d, 1H), 7.04 (t, 1H), 7.25 (d, 1H), 7.31-7.53 (m, 4H). |
| 3-198 | 2.86-3.07 (m, 2H), 3.49 (t, 2H), 4.60-4.74 (m, 2H), 4.74-4.95 (m, 4H), 5.58-5.69 (m, 1H), 7.02 (dt, 1H), 7.16 (dt, 1H), 7.21-7.28 (m, 1H), 7.38-7.53 (m, 3H). |
| 3-199 | 2.04-2.26 (m, 2H), 2.89-3.08 (m, 2H), 3.50 (q, 2H), 3.73-3.98 (m, 4H), 4.75 (d, 0.5H), 4.88 (d, 1H), 4.98 (d, 0.5H), 5.38-5.47 (m, 1H), 7.13-7.31 (m, 2H), 7.33-7.53 (m, 5H), 8.15 (d, 1H). |

**[Table 206]**

| No. | CDCl₃/TMS *δ* (ppm) |
|---|---|
| 3-200 | 2.10-2.28 (m, 2H), 2.87-3.06 (m, 2H), 3.40-3.53 (m, 2H), 3.78-4.00 (m, 4H), 4.71-4.84 (m, 1H), 4.86-4.99 (m, 1H), 5.40-5.50 (m, 1H), 6.97-7.08 (m, 1H), 7.11-7.30 (m. 2H), 7.36-7.52 (m, 3H). |
| 3-201 | 2.08-2.28 (m, 2H), 2.87-3.04 (m, 2H), 3.40-3.53 (m, 2H), 3.70-4.00 (m, 4H), 4.71-4.84 (m, 1H), 4.86-4.99 (m, 1H), 5.40-5.50 (m, 1H), 6.97-7.08 (m, 1H), 7.11-7.30 (m, 2H), 7.34-7.52 (m, 3H). |
| 3-202 | 2.04-2.28 (m, 2H), 2.87-3.04 (m, 2H), 3.40-3.56 (m, 2H), 3.70-4.00 (m, 4H), 4.71-4.84 (m, 1H), 4.86-5.00 (m, 1H), 5.40-5.50 (m, 1H), 6.97-7.08 (m, 1H), 7.11-7.30 (m. 2H), 7.34-7.52 (m, 3H). |
| 3-203 | 1.91-2.08 (m, 1H), 2.29-2.48 (m, 1H), 2.63-3.21 (m, 6H), 3.40-3.58 (m, 2H), 3.48-3.65 (m, 2H), 4.63-4.82 (m, 1H), 4.97 (d, 1H), 6.96-7.09 (m, 1H), 7.10-7.30 (m, 2H), 7.38-7.53 (m, 3H). |
| 3-204 | 2.40-2.68 (m, 1.5H), 2.83-3.28 (m, 4H), 3.28-3.65 (m, 4.5H), 4.41 (d, 0.5H), 4.81 (s, 1H), 5.25 (d, 0.5H), 5.60-5.70 (m 0.5H), 5.70-5.78 (m, 0.5H), 6.91-7.08 (m, 1H), 7.11-7.32 (m, 2H), 7.40-7.57 (m, 3H). |
| 3-205 | 1.41-2.06 (m, 4H), 2.82-3.06 (m, 2H), 3.41-3.82 (m, 6H), 4.67-4.81 (m, 1H), 4.82-5.02 (m, 1H), 4.99 (d, 1H), 6.97-7.10 (m, 1H), 7.12-7.32 (m, 2H), 7.36-7.57 (m, 3H). |
| 3-206 | 1.67-1.88 (m, 2H), 1.91-2.07 (m, 2H), 2.81-3.05 (m, 2H), 3.38-3.66 (m, 4H), 3.70-3.88 (m, 2H), 4.80 (d, 1H), 4.91 (d, 1H), 5.06-5.17 (m, 1H), 7.03 (dt, 1H), 7.16 (dt, 1H), 7.20-7.29 (m, 1H), 7.37-7.51 (m, 3H). |
| 3-207 | 2.90-2.97 (m, 2H), 3.49 (t, 2H), 3.93-4.10 (m, $H), 4.73-4.83 (m, 3H), 4.87 (d, 1H), 5.02 (d, 1H), 7.02 (dt, 1H), 7.15 (dt, 1H), 7.20-7.30 (m, 1H), 7.51-7.37 (m, 3H). |
| 3-208 | 1.32 (s, 3H), 1.34 (d, 3H), 2.85-3.03 (m, 2H), 3.48 (q, 2H), 3.73 (dq, 1H), 4.05 (dq, 1H), 4.23 (dd, 0.5H), 4.27-4.38 (m, 2H), 4.45 (dd, 0.5H), 4.76-4.93 (m, 2H), 7.03 (dt, 1H), 7.16 (dt, 1H), 7.22-7.28 (m, 1H), 7.36-7.50 (m, 3H). |
| 3-209 | 1.91-2.23 (m, 4H), 2.47-2.60 (m, 2H), 2.65-2.81 (m, 2H), 2.89-3.05 (m, 2H), 3.40-3.56 (m, 2H), 4.79 (d, 1H), 4.93 (d, 1H), 4.94-5.09 (m, 1H), 7.03 (dt, 1H), 7.11-7.30 (m, 2H), 7.38-7.56 (m, 3H). |
| 3-210 | 2.28-2.51 (m, 3H), 2.71-2.82 (m, 1H), 2.83-3.08 (m, 3.11-3.28 (m, 1H), 3.42-3.58 (m, 3H), 4.54 (d, 1H), 5.09 (d, 1H), 5.25 (brs, 1H), 7.01 (dt, 1H), 7.15 (dt, 1H), 7.22-7.34 (m, 1H), 7.41-7.59 (m, 3H). |
| 3-211 | 2.70-2.90 (brs, 4H), 2.89-3.08 (m, 2H), 3.41-3.64 (m, 2H), 4.78 (d, 1H), 5.19 (d, 1H), 7.04 (dt, 1H), 7.17 (dt, 1H), 7.33-7.58 (m, 4H). |
| 3-212 | 0.26-0.38 (m, 2H), 0.53-0.66 (m, 2H), 1.10-1.25 (m, 1H), 2.87-3.03 (m, 2H), 3.48 (t, 2H), 4.02-4.23 (m, 2H), 4.78 (d, 1H), 4.99 (d, 1H), 7.03 (dt, 1H), 7.14 (dt, 1H), 7.20-7.30 (m, 1H). 7.36-7.52 (m, 3H). |
| 3-213 | 2.79-2.91 (m, 2H), 3.23-3.41 (m, 2H), 4.77 (d, 1H), 4.86 (d, 1H), 5.31 (q, 2H), 7.03 (dt, 1H), 7.10-7.42 (m, 8H), 7.47 (d, 2H). |
| 3-214 | 2.58 (dt, 1H), 2.82 (dt, 1H), 2.96 (t, 2H), 3.19-3.29 (m, 1H), 3.49 (t, 2H), 4.09-4.22 (m, 1H), 4.52-4.70 (m, 1H), 4.87 (s, 1H), 7.03 (dt, 1H), 7.11-7.31 (m, 2H), 7.36-7.54 (m, 3H). |

**[Table 207]**

| No. | CDCl₃/TMS *δ* (ppm) |
|---|---|
| 3-215 | 1.31 (d, 3H), 2.55-2.72 (m, 2H), 2.95 (t, 2H), 3.41-3.52 (m, 2H), 4.09 (d, 0.5H), 4.30 (q, 1H), 4.53 (d, 0.5H), 4.75-4.99 (m, 2H), 7.03 (dt, 1H), 7.16 (dt, 1H), 7.22-7.30 (m, 1H), 7.35-7.53 (m, 3H). |
| 3-216 | 0.94 (s, 1.5H), 1.03 (s, 1.5H), 1.27 (s, 1.5H), 1.34 (s, 1.5H), 2.87-3.10 (m, 2H), 3.51-3.67 (m, 2H), 5.58-3.69 (m, 0.5H), 3.95 (d, 1H), 4.07-4.20 (m, 0.5H), 4.56 (dd, 1H), 5.42 (dd, 1H), 7.03 (dt, 1H), 7.18 (dt, 1H), 7.30-7.48 (m, 4H). |
| 3-217 | lp : 1.36 (d, 1.5H), 1.40 (d, 1.5H), 2.52 (dd, 0.5H), 2.61 (dd, 0.5H), 2.68 (t, 0.5H), 2.79 (t, 0.5H), 2.95 (t, 1H), 3.02-3.10 (m, 1H), 3.48 (t, 2H), 4.79-5.00 (m, 2.5H), 5.10-5.18 (m, 0.5H), 7.03 (dt, 1H), 7.16 (dt, 1H), 7.22-7.29 (m, 1H), 7.35-7.52 (m, 3H). |
| | mp:1.42 (d, 1.5H), 1.44 (d, 1.5H), 2.63 (dq, 1H), 2.80 (t, 0.5H), 2.86 (t, 0.5H), 2.95 (q, 2H), 3.02-3.11 (m, 1H), 3.43-3.53 (m, 2H), 4.70-4.98 (m, 3H), 7.03 (tt, 1H), 7.16 (tt, 1H), 7.22-7.29 (m, 1H), 7.36-7.53 (m, 3H). |
| 3-218 | 2.32-2.56 (m, 1H), 2.54-2.72 (m, 1H), 2.84-3.06 (m, 2H), 3.41-3.58 (m, 2H), 4.06-4.32 (m, 1.5H), 4.40 (d, 1H), 4.43-4.61 (m, 1.5H), 4.71-5.09 (m, 3H), 6.94-7.20 (m, 2H), 7.24-7.53 (m, 4H). |
| 3-219 | 2.86-3.05 (m, 2H), 3.30-3.42 (m, 1H), 3.48 (t, 2H), 4.34-4.44 (m, 2H), 4.45-4.62 (m, 2H), 4.81-4.90 (m, 4H), 7.03 (dt, 1H), 7.15 (dt, 1H), 7.18-7.28 (m, 1H), 7.35-7.52 (m, 3H). |
| 3-220 | 1.48-1.63 (m, 1H), 1.64-2.00 (m, 3H), 2.86-3.03 (m, 2H), 3.42-3.53 (m,2H), 3.60-3.79 (m, 2H), 4.02-4.18 (m, 1H), 4.21-4.35 (m, 2H), 4.72-5.00 (m, 2H), 7.03 (dt, 1H), 7.16 (dt, 1H), 7.20-7.29 (m, 1H), 7.35-7.53 (m, 3H). |
| 3-221 | 1.50-1.68 (m, 1H), 1.92-2.12 (m, 1H), 2.50-2.71 (m, 1H), 2.85-3.06 (m, 2H), 3.40-3.58 (m, 3H), 3.56-3.88 (m, 3H), 4.12-4.41 (m, 2H), 4.80 (dd, 1H), 4.93 (dd, 1H), 7.03 (dt, 1H), 7.15 (dt, 1H), 7.24 (d, 1H), 7.32-7.57 (m, 3H). |
| 3-222 | 1.15-1.29 (m, 1H), 1.42-1.57 (m, 4H), 1.78-1.90 (m, 1H), 2.86-3.01 (m, 2H), 3.30-3.58 (m, 4H), 3.90-4.01 (m, 1H), 4.11-4.32 (m, 2H), 4.70-5.02 (m, 2H), 7.03 (dt, 1H), 7.15 (dt, 1H), 7.20-7.31 (m, 1H), 7.32-7.54 (m, 3H). |
| 3-223 | 1.20-1.41 (m, 2H), 1.48-1.67 (m, 2H), 1.90-2.04 (m, 1H), 2.89-3.06 (m, 2H), 3.31-3.44 (m, 2H), 3.48 (t, 2H), 3.90-4.02 (m, 2H), 4.08-4.27 (m, 2H), 4.76 (d, 1H), 4.97 (d, 1H), 7.03 (dt, 1H), 7.15 (dt, 1H), 7.24 (d, 1H), 7.12-7.51 (m, 3H). |
| 3-224 | 2.89-3.10 (m, 2H), 3.45-3.70 (m, 2H), 4.21-4.52 (m, 2.5H), 4.53-4.70 (m, 2.5H), 4.89-5.21 (m, 2H), 6.91-7.06 (m, 1H), 7.08-7.32 (m, 2H), 7.37-7.58 (m, 3H). |
| 3-225 | 1.82 (s, 3H), 3.11 (t, 2H), 3.70-3.83 (m, 2H), 4.35 (d, 1H), 4.92 (q, 2H), 5.24 (d, 1H), 7.02 (dt, 1H), 7.11-7.22 (m, 2H), 7.29-7.46 (m, 3H). |
| 3-226 | 2.86 (t, 2H), 2.91-3.08 (m, 2H), 3.53 (t, 2H),4.41-4.62 (m, 2H), 4.79 (d, 1H), 4.91 (d, 1H), 7.03 (dt, 1H), 7.15 (dt, 1H), 7.22-7.31 (m, 1H), 7.41-7.55 (m, 3H). |
| 3-227 | 2.92-3.11 (m, 2H), 3.59 (t, 2H), 3.78-4.06 (m, 2H), 4.32-4.53 (m, 4H), 5.18 (d, 1H), 7.04 (dt, 1H), 7.16 (dt, 1H), 7.22-7.30 (m, 1H), 7.37-7.56 (m, 3H). |
| 3-228 | 2.89-3.00 (m, 2H), 3.31 (s, 3H), 3.48 (t, 2H), 3.59 (t, 2H), 4.35-4.49 (m, 1H), 4.82 (d, 1H), 4.93 (d, 1H), 7.03 (dt, 1H), 7.16 (dt, 1H), 7.20-7.28 (m, 1H), 7.32-7.50 (m, 3H). |

**[Table 208]**

| No. | CDCl₃/TMS *δ* (ppm) |
|---|---|
| 3-229 | 1.29 (dd, 3H), 2.85-3.00 (m, 2H), 3.31 (d, 3H), 3.35-3.52 (m, 4H), 4.72-5.00 (m, 2H), 5.10-5.28 (m, 1H), 6.97-7.09 (m, 1H), 7.12-7.29 (m, 2H), 7.37-7.51 (m, 3H). |
| 3-230 | 2.93 (t, 2H), 3.30 (s, 3H), 3.34 (s, 3H), 3.42-3.58 (m, 6H), 4.88 (s, 2H), 5.17-5.28 (m, 1H), 7.03 (dt, 1H), 7.16 (dt, 1H), 7.21-7.30 (m, 1H), 7.32-7.52 (m, 3H). |
| 3-231 | 1.01 (s, 9H), 2.82-2.98 (m, 2H), 3.38-3.50 (m, 2H), 3.85-3.90 (m, 2H), 4.30-4.52 (m, 2H), 4.88 (q, 2H), 7.03 (dt, 1H), 7.16 (dt, 1H), 7.20-7.28 (m, 1H), 7.32-7.51 (m, 9H), 7.54-7.66 (m, 4H). |
| 3-232 | 2.07 (s, 3H), 2.83-3.03 (m, 2H), 3.49 (t, 2H), 4.21-4.40 (m, 2H), 4.45-4.56 (m, 2H), 4.77 (d, 1H), 4.98 (d, 1H), 7.03 (dt, 1H), 7.16 (dt, 1H), 7.21-7.30 (m, 1H), 7.35-7.56 (m, 3H). |
| 3-233 | 2.90-3.08 (m, 2H), 2.99 (s, 3H), 3.52 (t, 2H), 4.40-4.68 (m, 4H), 4.80 (d, 1H), 4.91 (d, 1H), 7.02 (dt, 1H), 7.16 (dt, 1H), 7.21-7.31 (m, 1H), 7.38-7.54 (m, 3H). |
| 3-234 | 2.16 (s, 3H), 2.96 (t, 2H), 3.45-3.59 (m, 2H), 4.75 (d, 1H), 4.83 (q, 2H), 5.05 (d, 1H), 7.03 (dt, 1H), 7.16 (dt, 1H), 7.30-7.53 (m, 4H). |
| 3-235 | 3.00 (t, 2H), 3.50 (t, 2H), 3.72-3.82 (m, 3H), 4.68-4.77 (m, 2H), 4.79 (d, 1H), 5.07 (d, 1H), 7.03 (dt, 1H), 7.17 (dt, 1H), 7.32 (d, 1H), 7.36-7.51 (m, 3H). |
| 3-236 | 2.11 (s, 3H), 2.59-2.78 (m, 2H), 2.90-3.09 (m, 2H), 3.60 (t, 2H), 3.82-4.11 (m, 2H), 4.50 (d, 1H), 5.45 (d, 1H), 7.04 (dt, 1H), 7.17 (dt, 1H), 7.30-7.48 (m, 4H). |
| 3-237 | 2.64 (s, 3H), 2.92-3.10 (m, 2H), 3.38-3.49 (m, 1H), 3.52-3.69 (m, 3H), 4.65 (d, 1H), 4.65-4.84 (m, 2H), 4.96 (d, 1H), 7.02 (dt, 1H), 7.17 (dt, 1H), 7.27 (d, 1H), 7.36-7.51 (m, 3H). |
| 3-238 | 1.29 (dd, 6H), 2.89-3.10 (m, 2H), 3.42-3.68 (m, 2H), 4.61 (d, 1H), 5.27 (d, 1H), 7.20 (d,1H), 7.21-7.63 (m, 6H), 8.18 (d, 1H). |
| 3-239 | 2.89-3.12 (m, 2H), 3.48-3.65 (m, 2H), 4.57 (d, 1H), 5.41 (d, 1H), 6.97-7.09 (m, 1H), 7.12-7.48 (m, 9H), 7.55 (d, 1H). |
| 3-240 | 1.30 (dd, 6H), 2.87-3.04 (m, 2H), 3.42-3.70 (m, 3H), 4.54 (d, 1H), 5.32 (d, 1H), 6.93-7.14 (m, 2H), 7.21-7.60 (m, 5H). |
| 3-241 | 1.26 (t, 3H), 2.82-3.11 (m, 4H), 3.48-3.66 (m, 2H), 4.61 (d, 1H), 5.26 (d, 1H), 7.04 (dt, 1H), 7.17 (dt, 1H), 7.32 (d, 1H), 7.40-7.61 (m, 3H). |
| 3-242 | 0.81-0.98 (m, 3H), 1.29 (d, 3H), 1.48-1.69 (m, 2H), 2.81-3.08 (m, 2H), 3.40-3.53 (m, 2H), 4.61 (dd, 1H), 5.25 (dd, 1H), 7.05 (dt, 1H), 7.17 (dt, 1H), 7.31 (d, 1H). 7.43 (t, 1H), 7.45-7.58 (m, 2H). |
| 3-243 | 2.81 (d, 3H), 3.11 (t, 2H), 3.80 (t, 2H), 4.32 (d, 1H), 5.24 (d, 1H), 7.00 (dt, 1H), 7.10-7.23 (m, 2H), 7.30-7.49 (m, 4H). |

**[Table 209]**

| No. | CDCl₃/TMS *δ* (ppm) |
|---|---|
| 3-244 | 1.06 (d, 3H), 1.21 (d, 3H), 3.02-3.19 (m, 2H), 3.73-3.97 (m, 3H), 4.31 (d, 1H), 5.19 (d, 1H), 6.98 (dt, 1H), 7.17 (dt, 1H), 7.21-7.50 (m, 5H). |
| 3-245 | 0.73-0.97 (m, 6H), 2.87-3.10 (m, 2H), 3.10-3.38 (m, 4H), 3.58 (t, 2H), 4.90 (brd, 2H), 7.04 (dt, 1H), 7.18 (dt, 1H), 7.29-7.49 (m, 4H). |
| 3-246 | 2.92-3.11 (m, 2H), 3.62 (t, 2H), 3.66 (s, 3H), 4.78 (d, 1H), 5.12 (d, 1H), 7.21 (d, 1H), 7.32-7.58 (m, 6H), 8.11 (d, 1H). |
| 3-247 | 3.00 (t, 2H), 3.58 (t, 2H), 3.64 (s, 3H), 4.84 (d, 1H), 5.06 (d, 1H), 7.04 (dt, 1H), 7.18 (dt, 1H), 7.32-7.57 (m, 4H). |
| 3-248 | 3.01 (t, 2H), 3.06 (t, 2H), 3.63 (s, 3H), 4.85 (d, 1H), 5.00 (d, 1H), 6.82 (dd, 1H), 7.13 (t, 1H), 7.35-7.50 (m, 3H), 7.54 (t, 1H), 12.03 (s, 1H). |
| 3-249 | 1.59 (t, 3H), 2.98 (t, 2H), 3.56 (t, 2H), 3.71-3.90 (m, 2H), 4.90 (d, 1H), 5.06 (d, 1H), 7.00-7.22 (m, 2H), 7.34-7.57 (m, 4H). |
| 3-250 | 2.97 (t, 2H), 3.51 (t, 2H), 4.95 (s, 2H), 6.65 (d, 1H), 6.87 (d, 1H), 7.28-7.63 (m, 5H), 12.35 (s, 1H). |
| 3-251 | 2.53 (t, 1H), 2.91 (t, 2H), 3.44 (t, 2H), 4.88 (d, 2H), 4.98 (s, 2H), 6.85 (d, 1H), 7.12 (d, 1H), 7.31-7.63 (m, 5H). |
| 3-252 | 2.97 (t, 2H), 3.50 (t, 2H), 4.98 (s, 2H), 7.06 (dt, 1H), 7.11-7.28 (m, 1H), 7.32-7.68 (m, 4H). |
| 3-253 | 1.20-1.51 (m, 4H), 2.98 (t, 2H), 3.11-3.28 (m, 1H), 3.56 (t, 2H), 4.84 (d, 1H), 5.22 (d, 1H), 7.05 (dt, 1H), 7.18 (dt, 1H), 7.30-7.58 (m, 4H). |
| 3-254 | DMSO-d6 : 2.89 (t, 2H), 3.51 (t, 2H), 4.68 (s, 2H), 6.72 (t, 1H), 6.91-7.07 (m, 2H), 7.13-7.27 (m, 2H), 7.42 (dt, 1H). |
| 3-255 | DMSO-d6 : 2.89 (t, 2H), 3.50 (t, 2H), 4.68 (s, 2H), 6.63-6.79 (m, 1H), 6.92-7.06 (m, 2H), 7.13-7.27 (m, 2H), 7.42 (dt, 1H). |
| 4-1 | 3.05 (t, 2H), 3.76 (t, 2H), 4.76 (s, 2H), 7.17 (d, 1H), 7.21-7.56 (m, 5H), 8.05 (d, 1H), 10.69 (brs, 1H). |
| 4-2 | 2.34 (s, 3H), 3.02 (t, 2H), 3.71 (t, 2H), 4.64 (s, 2H), 7.01 (d, 1H), 7.10-7.51 (m, 5H), 8.08 (d, 1H), 11.60 (brs, 1H). |
| 4-3 | 3.02 (t, 2H), 3.70 (t, 2H), 3.80 (s, 3H), 4.62 (s, 2H), 6.73 (dd, 1H), 7.10-7.50 (m, 5H), 8.09 (d, 1H), 11.90 (brs, 1H). |

**[Table 210]**

| No. | CDCl₃/TMS *δ* (ppm) |
|---|---|
| 4-4 | 3.06 (t, 2H), 3.75 (t, 2H), 4.66 (s, 2H), 7.01-7.12 (m, 2H), 7.18 (d, 1H), 7.30-7.62 (m, 3H), 8.08 (d, 1H), 11.67 (brs, 1H). |
| 4-5 | 3.06 (t, 2H), 3.74 (t, 2H), 4.65 (s, 2H), 7.18 (d, 1H), 7.27-7.51 (m, 4H), 7.57 (d, 1H), 8.09 (d, 1H), 11.82 (brs, 1H). |
| 4-6 | 3.06 (t, 2H), 3.74 (t, 2H), 4.64 (s, 2H), 7.18 (d, 1H), 7.26-7.69 (m, 5H), 8.08 (d, 1H), 11.84 (brs, 1H). |
| 4-7 | 2.33 (s, 3H), 3.03 (t, 2H), 3.73 (t, 2H), 4.64 (s, 2H), 7.05-7.20 (m, 3H), 7.23-7.58 (m, 3H), 8.06 (d, 1H), 11.66 (brs, 1H). |
| 4-8 | 2.91-3.11 (m, 2H), 3.99 (t, 2H), 4.84 (s, 2H), 7.11-7.39 (m, 4H), 7.40-7.61 (m, 2H), 8.08 (d, 1H), 12.36 (brs, 1H). |
| 4-9 | 3.09 (t, 2H), 3.78 (t, 2H), 4.74 (s, 2H), 7.12-7.29 (m, 2H), 7.29-7.52 (m, 3H), 8.05 (d, 1H), 10.69 (brs, 1H). |
| 4-10 | 2.98 (t, 2H), 3.69 (t, 2H), 4.65 (s, 2H), 7.09 (d, 1H), 7.21-7.43 (m, 4H), 7.58 (d, 1H), 11.35 (brs, 1H). |
| 4-11 | 3.04 (t, 2H), 3.74 (t, 2H), 4.63 (s, 2H), 7.20 (s, 1H), 7.23-7.42 (m, 3H), 7.57 (d, 1H), 8.03 (d, 1H), 11.64 (brs, 1H). |
| 4-12 | 3.02 (t, 2H), 3.76 (t, 2H), 4.73 (s, 2H), 7.00 (dt, 1H), 7.10-7.39 (m, 4H), 11.50 (brs, 1H). |
| 4-13 | 3.02 (t, 2H), 3.77 (t, 2H), 4.78 (s, 2H), 7.00 (dt, 1H), 7.16 (dt, 1H), 7.21-7.38 (m, 2H), 7.49 (dd, 1H), 11.04 (brs, 1H). |
| 4-14 | 2.49 (s, 3H), 3.00 (t, 2H), 3.78 (t, 2H), 4.70 (brs, 2H), 6.99 (dt, 1H), 7.14 (dt, 1H), 7.18-7.34 (m, 3H), 10.35 (brs, 1H). |
| 4-15 | 3.00 (t, 2H), 3.70 (t, 2H), 4.64 (s, 2H), 6.82-7.09 (m, 2H), 7.18 (dt, 1H), 7.22-7.49 (m, 2H), 11.50 (brs, 1H). |
| 4-16 | 3.02 (t, 2H), 3.75 (t, 2H), 4.65 (s, 2H), 6.91-7.25 (m, 4H), 7.59 (dd, 1H), 10.97 (brs, 1H). |
| 4-17 | 3.02 (t, 2H), 3.74 (t, 2H), 4.63 (s, 2H), 7.02 (dt, 1H), 7.18 (dt, 1H), 7.20-7.47 (m, 2H), 7.58 (d, 1H), 11.50 (brs, 1H). |
| 4-18 | 2.35 (s, 3H), 2.99 (t, 2H), 3.73 (t, 2H), 4.64 (s, 2H), 6.91-7.26 (m, 4H), 7.50 (d, 1H), 11.02 (brs, 1H). |

**[Table 211]**

| No. | CDCl₃/TMS *δ* (ppm) |
|---|---|
| 4-19 | 2.98 (t, 2H), 3.71 (t, 2H), 4.62 (s, 2H), 6.78 (d, 1H), 6.81-7.08 (m, 2H), 7.15 (dt, 1H), 7.29-7.40 (m, 1H), 11.50 (brs, 1H). |
| 4-20 | 3.00 (t, 2H), 3.74 (t, 2H), 3.81 (s, 3H), 4.63 (s, 2H), 6.80-7.06 (m, 3H), 7.11-7.21 (m, 1H), 7.46-7.55 (m, 1H), 10.70 (brs, 1H). |
| 4-21 | 3.02 (t, 2H), 3.84 (t, 2H), 4.71 (d, 2H), 6.92-7.18 (m, 3H), 7.17 (dt, 1H), 7.35 (q, 1H), 7.42 (d, 1H), 11.49 (brs, 1H). |
| 4-22 | 3.03 (t, 2H), 3.98 (t, 2H), 4.81 (s, 2H), 7.00 (dt, 1H), 7.17 (dt, 1H), 7.20-7.39 (m, 2H), 7.53 (dd, 1H), 11.60 (brs, 1H). |
| 4-23 | 2.51 (s, 3H), 3.01 (t, 2H), 3.76 (t, 2H), 4.72 (s, 2H), 7.00 (dt, 1H), 7.08-7.31 (m, 3H), 7.46 (d, 1H), 10.92 (brs, 1H). |
| 4-24 | 2.97 (t, 2H), 3.75 (t, 2H), 4.85 (brs, 2H), 7.02 (dt, 1H), 7.17 (dt, 1H), 7.42-7.58 (m, 2H), 7.76-7.89 (m, 3H), 8.10 (s, 1H), 11.18 (brs, 1H). |
| 4-25 | 1.33 (t, 3H), 2.96 (t, 2H), 3.47 (t, 2H), 4.29-4.47 (m, 2H), 4.67 (d, 1H), 5.01 (d, 1H), 7.11 (d, 1H), 7.16 (d, 1H), 7.22-7.50 (m, 4H). |
| 4-26 | 1.33 (t, 3H), 2.96 (t, 2H), 3.49 (t, 2H), 4.39 (q, 2H), 4.82 (d, 1H), 4.91 (d, 1H), 6.98-7.11 (m, 1H), 7.11-7.34 (m, 3H), 7.40-7.52 (m, 1H). |
| 4-27 | 1.33 (t, 3H), 2.88-3.06 (m, 2H), 3.39-3.54 (m, 2H), 4.31-4.47 (m, 2H), 4.82 (d, 1H), 4.91 (d, 1H), 7.05 (dt, 1H), 7.17 (dt, 1H), 7.32-7.51 (m, 3H). |
| 4-28 | 1.34 (t, 3H), 2.34 (s, 3H), 2.93 (t, 2H), 3.44 (t, 2H), 4.39 (q, 2H), 4.75 (d, 1H), 4.88 (d, 1H), 7.03 (dt, 1H), 7.16 (dt, 1H), 7.19-7.40 (m, 3H). |
| 4-29 | 1.34 (t, 3H), 2.83-3.06 (m, 2H), 3.47 (t, 2H), 4.30-4.49 (m, 2H), 4.73 (d, 1H), 4.89 (d, 1H), 6.90-7.10 (m, 2H), 7.10-7.26 (m, 2H), 7.42-7.51 (m, 1H). |
| 4-30 | 1.34 (t, 3H), 2.90-3.11 (m, 2H), 3.50 (t, 2H), 4.30-4.48 (m, 2H), 4.70 (d, 1H), 4.95 (d, 1H), 6.92-7.12 (m, 3H), 7.12-7.31 (m, 3H). |
| 4-31 | 1.34 (t, 3H), 2.90-3.09 (m, 2H), 3.50 (t, 2H), 4.30-4.49 (m, 2H), 4.72 (d, 1H), 4.92 (d, 1H), 7.04 (dt, 1H), 7.12-7.26 (m, 2H), 7.32-7.50 (m, 2H). |
| 4-32 | 1.33 (t, 3H), 2.37 (s, 3H), 2.82-3.06 (m, 2H), 3.48 (t, 2H), 4.30-4.48 (m, 2H), 4.73 (d, 1H), 4.91 (d, 1H), 7.03 (dt, 1H), 7.09-7.31 (m, 4H). |
| 4-33 | 1.35 (t, 3H), 2.88-3.07 (m, 2H), 3.42-3.57 (m, 2H), 4.31-4.49 (m, 2H), 4.83 (d, 1H), 4.98 (d, 1H), 7.05 (dt, 1H), 7.19 (dt, 1H), 7.38 (d, 1H), 7.60-7.76 (m, 2H). |

**[Table 212]**

| No. | CDCl₃/TMS *δ* (ppm) |
|---|---|
| 4-34 | 1.33 (t, 3H), 2.72-2.92 (m, 2H), 3.48 (t, 2H), 4.29-4.44 (m, 2H), 4.58 (d, 1H), 4.92 (d, 1H), 6.78-6.92 (m, 2H), 6.93-7.13 (m, 3H), 7.56 (brs, 1H). |
| 4-35 | 1.33 (t, 3H), 2.87-3.05 (m, 2H), 3.48 (t, 2H), 3.80 (s, 3H), 4.28-4.48 (m, 2H), 4.70 (d, 1H), 4.92 (d, 1H), 6.88 (dt, 1H), 6.95 (d, 1H), 7.03 (dt, 1H), 7.10-7.22 (m. 2H). |
| 4-36 | 1.33 (t, 3H), 2.83-3.01 (m, 2H), 3.39-3.54 (m, 2H), 4.28-4.47 (m, 2H), 4.91 (d, 1H), 4.97 (d, 1H), 6.97 (dt, 1H), 7.05-7.20 (m, 2H), 7.22-7.31 (m, 1H), 7.37-7.46 (m, 1H). |
| 4-37 | 1.35 (t, 3H), 2.81-3.01 (m, 2H), 3.29-3.46 (m, 2H), 4.39 (q, 2H), 4.92 (d, 1H), 5.18 (d, 1H), 6.99 (dt, 1H), 7.12 (dt, 1H), 7.22 (d, 1H), 7.40 (t, 1H), 7.59 (d, 1H). |
| 4-38 | 1.33 (t, 3H), 2.39 (s, 3H), 2.73-2.93 (m, 2H), 3.21-3.37 (m, 2H), 4.31-4.47 (m, 2H), 4.86 (d, 1H), 5.00 (d, 1H), 7.01 (dt, 1H), 7.05-7.19 (m, 2H), 7.28-7.40 (m, 2H). |
| 4-39 | 1.33 (t, 3H), 2.90-3.08 (m, 2H), 3.55 (t, 2H), 4.29-4.48 (m, 2H), 4.92 (d, 1H), 5.08 (d, 1H), 7.05 (dt, 1H), 7.18 (dt, 1H), 7.50-7.65 (m, 2H), 7.77 (s, 1H), 7.81-7.92 (m, 3H). |
| 4-40 | 0.88 (t, 3H), 1.18-1.39 (m, 4H), 1.56-1.77 (m, 2H), 2.85-3.03 (m, 2H), 3.47 (t, 2H), 4.23-4.37 (m, 2H), 4.74 (d, 1H), 4.88 (d, 1H), 6.91-7.10 (m, 2H), 7.11-7.28 (m, 2H), 7.42-7.52 (m, 1H). |
| 4-41 | 0.88 (t, 3H), 1.18-1.39 (m, 4H), 1.54-1.78 (m, 2H), 2.86-3.06 (m, 2H), 3.50 (t, 2H), 4.31 (q, 2H), 4.72 (d, 1H), 4.93 (d, 1H), 6.92-7.10 (m, 2H), 7.10-7.28 (m, 3H). |
| 4-42 | 0.87 (t, 3H), 1.18-1.39 (m, 4H), 1.54-1.79 (m, 2H), 2.82-3.00 (m, 2H), 3.40-3.53 (m, 2H), 4.20-4.39 (m, 2H), 4.90 (d, 1H), 4.98 (d, 1H), 6.97 (dt, 1H), 7.03-7.30 (m. 3H), 7.32-7.49 (m, 1H). |
| 5-1 | 2.01-2.23 (m, 2H), 2.74 (t, 2H), 3.31 (t, 2H), 4.69 (s, 2H), 7.15 (d, 1H), 7.22-7.50 (m, 5H), 7.58-7.75 (m, 2H), 11.85 (brs, 1H). |
| 5-2 | 1.62-1.87 (m, 1H), 2.08-2.29 (m, 1H), 2.48-2.80 (m, 2H), 3.12-3.51 (m, 2H), 4.62 (d, 1H), 4.82 (d, 1H), 7.03 (d, 1H), 7.10-7.49 (m, 5H), 7.66 (d, 1H), 11.31 (brs, 1H). |
| 5-3 | 1.58-1.92 (m, 1H), 2.08-2.40 (m, 1H), 2.39 (s, 3H), 2.42-2.80 (m, 2H), 3.10-3.52 (m, 2H), 4.52 (brs, 1H), 4.87 (brs, 1H), 6.94 (d, 1H), 7.11 (d, 1H), 7.12-7.49 (m, 4H), 7.65 (d, 1H), 11.88 (brs, 1H). |
| 5-4 | 2.15 (t, 2H), 2.75 (t, 2H), 3.31 (t, 2H), 4.68 (s, 2H), 6.87 (dd, 1H), 7.01 (dt, 1H), 7.12-7.46 (m, 3H), 7.60-7.78 (m, 2H), 11.73 (brs, 1H). |
| 5-5 | 2.17 (t, 2H), 2.75 (t, 2H), 3.33 (t, 2H), 4.70 (s, 2H), 7.12-7.52 (m, 5H), 7.58-7.78 (m, 2H), 11.69 (brs, 1H). |
| 5-6 | 2.12 (t, 2H), 2.35 (s, 3H), 2.71 (t, 2H), 3.29 (t, 2H), 4.67 (s, 2H), 6.96 (s, 1H), 7.12 (d, 1H), 7.19-7.42 (m, 3H), 7.57 (d, 1H), 7.66 (d, 1H), 11.96 (brs, 1H). |

**[Table 213]**

| No. | CDCl₃/TMS *δ* (ppm) |
|---|---|
| 6-1 | 4.47 (s, 2H), 4.70 (s, 2H), 7.03 (d, 1H), 7.18-7.46 (m, 3H), 7.61 (d, 1H), 7.68 (d, 1H), 11.32 (brs, 1H). |
| 6-2 | 4.44 (s, 2H), 4.68 (s, 2H), 7.06 (s, 1H), 7.18-7.45 (m, 3H), 7.61 (d, 1H), 11.04. |
| 6-3 | 2.60 (s, 3H), 4.52 (s, 2H), 4.74 (s, 2H), 7.20-7.48 (m, 3H), 7.61 (d, 2H), 8.88 (s, 1H), 10.86 (brs, 1H). |
| 6-4 | 2.89 (t, 2H), 3.77 (t, 2H), 4.62 (s, 2H), 6.36 (s, 1H), 7.12-7.42 (m, 4H), 7.43-7.68 (m, 2H), 11.18 (brs, 1H). |
| 6-5 | 2.99 (t, 2H), 3.77 (t, 2H), 4.65 (s, 2H), 6.89 (d, 1H), 7.14-7.42 (m, 3H), 7.52 (d, 1H), 7.61 (d, 1H), 11.52 (brs, 1H). |
| 6-6 | 3.12 (t, 2H), 3.81 (t, 2H), 4.65 (s, 2H), 7.09 (d, 1H), 7.12-7.45 (m, 3H), 7.42 (d, 1H), 7.62 (d, 1H), 11.67 (brs, 1H). |
| 6-7 | 2.41 (s, 3H), 2.81 (t, 2H), 3.79 (t, 2H), 4.70 (s, 2H), 7.18-7.36 (m, 2H), 7.39 (dt, 1H), 7.59 (d, 1H), 11.32 (brs, 1H). |
| 6-8 | 2.52 (s, 3H), 2.97 (t, 2H), 3.83 (t, 2H), 4.63 (s, 2H), 7.23 (t, 1H), 7.30-7.43 (m, 2H), 7.60 (d, 1H), 11.58 (brs, 1H). |
| 6-9 | CD3OD : 3.18 (t, 2H), 3.44 (t, 2H), 4.21 (s, 2H), 6.89 (dt, 1H), 7.13-7.29 (m, 2H), 7.40 (d, 1H), 7.98 (s, 1H), 10.86 (brs, 1H). |
| 6-10 | 2.32 (s, 3H), 2.91 (t, 2H), 3.60 (t, 2H), 4.56 (s, 2H), 6.99 (t, 1H), 7.11 (d, 1H), 7.17-7.30 (m, 1H), 7.53 (d, 1H), 11.25 (brs, 1H). |
| 6-11 | 2.94 (t, 2H), 3.59 (ft, 2H), 5.31 (s, 2H), 7.17-7.68 (m, 5H), 8.29 (s, 1H), 11.35 (brs, 1H). |
| 6-12 | 2.98 (t, 2H), 3.77 (t, 2H), 4.61 (s, 2H), 7.20 (t, 1H), 7.27-7.41 (m, 2H), 7.59 (d, 1H), 7.83 (s, 1H), 10.89 (brs, 1H). |
| 6-13 | 2.97 (t, 2H), 3.71 (t, 2H), 3.85 (s, 3H), 4.63 (s, 2H), 7.20 (dt, 1H), 7.31 (dd, 1H), 7.36 (dt, 1H), 7.61 (d, 1H), 11.28 (brs, 1H). |
| 6-14 | 2.54 (s, 3H), 3.10 (t, 2H), 3.71 (t, 2H), 4.65 (s, 2H), 7.19 (dt, 1H), 7.28-7.52 (m, 7H), 7.63 (d, 1H), 11.79. |
| 6-15 | 2.54 (s, 3H), 3.64 (t, 2H), 3.75 (t, 2H), 4.65 (s, 2H), 7.16-7.24 (m, 2H), 7.29-7.41 (m, 2H), 7.63 (d, 1H), 7.81 (dt, 1H), 7.95 (d, 1H), 8.38 (d, 1H), 11.83 (brs, 1H). |

**[Table 214]**

| No. | CDCl₃/TMS *δ* (ppm) |
|---|---|
| 6-16 | 2.97 (t, 2H), 3.73 (t, 2H), 3.87 (s, 3H), 4.63 (s, 2H), 7.21 (dt, 1H), 7.29-7.41 (m, 2H), 7.61 (dd, 1H), 7.78 (s, 1H), 10.89 (brs, 1H). |
| 6-17 | 2.52 (s, 3H), 2.81-2.98 (m, 2H), 3.67 (t, 2H), 3.73 (s, 3H), 7.20 (dt, 1H), 7.21-7.40 (m, 2H), 7.62 (d, 1H), 11.23 (brs, 1H). |
| 6-18 | 2.97 (t, 2H), 3.71 (t, 2H), 3.85 (s, 3H), 4.63 (s, 2H), 7.20 (dt, 1H), 7.31 (dd, 1H), 7.37 (dt, 1H), 7.62 (d, 1H). |
| 6-19 | 3.06 (t, 2H), 3.76 (t, 2H), 4.73 (s, 2H), 7.18 (t, 1H), 7.21-7.41 (m, 3H), 7.56 (d, 2H), 8.64 (t, 1H), 11.46 (brs, 1H). |
| 6-20 | DMSO-d6 : 3.02 (t, 2H), 3.57 (t, 2H), 4.77 (s, 2H), 7.00-7.32 (m, 4H), 7.78 (d, 1H), 8.63 (t, 1H), 11.23 (brs, 1H). |
| 6-21 | 2.76 (s, 3H), 3.17 (t, 2H), 3.83 (t, 2H), 4.70 (s, 2H), 7.20-7.48 (m, 3H), 7.63 (d, 1H), 9.16 (s, 1H), 11.36 (brs, 1H). |
| 6-22 | 1.32 (t, 3H), 2.80-3.00 (m, 2H), 3.44-3.62 (m, 2H), 4.27-4.42 (m, 2H), 4.68 (d, 1H), 4.95 (d, 1H), 6.91 (d, 1H), 7.22 (d, 1H), 7.38 (dt, 1H), 7.41-7.53 (m, 3H). |
| 6-23 | 1.32 (t, 3H), 2.78-3.02 (m, 2H), 3.55 (t, 2H), 3.78 (s, 3H), 4.28-4.47 (m, 2H), 4.63 (d, 1H), 4.91 (d, 1H), 7.22 (d, 1H), 7.37 (dt, 1H), 7.38-7.52 (m, 2H). |
| 6-24 | 1.36-1.41 (m, 3H), 2.60 (s, 3H), 2.88 (dt, 2H), 3.50 (t, 2H), 3.79 (s, 3H), 4.30-4.47 (m, 2H), 4.72 (d, 1H), 4.88 (d, 1H), 7.23 (d, 1H), 7.31-7.51 (m, 3H). |
| 6-25 | 1.34 (t, 3H), 2.89-3.12 (m, 2H), 3.41-3.59 (m, 2H), 4.26-4.46 (m, 2H), 4.77 (d, 1H), 5.11 (d, 1H), 7.24 (t, 2H), 7.28-7.62 (m, 4H), 8.72 (d, 1H). |
| 6-26 | 1.33 (t, 3H), 2.90-3.10 (m, 2H), 3.55 (t, 2H), 4.30-4.48 (m, 2H), 4.82 (d, 1H), 4.94 (d, 1H), 7.25 (d, 1H), 7.32-7.55 (m, 3H), 7.95 (d, 1H), 8.56 (s, 1H), 8.69 (d. 1H). |
| 6-27 | 1.23 (t, 3H), 4.22 (q, 2H), 5.34 (d, 1H), 5.50 (d, 1H), 5.59 (dd, 1H), 6.50 (dd, 1H), 7.20-7.32 (m, 3H), 7.42-7.71 (m, 4H), 8.21 (dd, 1H), 8.70 (dd, 1H). |
| 7-1 | 2.98 (t, H), 3.81 (t, 2H), 5.46 (s, 2H), 7.13 (d, 1H), 7.21-7.49 (m, 5H), 7.64 (d, 1H), 8.53 (d, 1H), 10.33 (brs, 1H). |
| 7-2 | 2.92 (t, 2H), 3.77 (t, 2H), 5.41 (s, 2H), 7.05 (dt, 1H), 7.15 (dt, 1H), 7.29 (dd, 1H), 7.38-7.44 (m, 2H), 7.63 (dd, 1H), 9.94] (brs, 1H). |
| 7-3 | 1.16 (d, 3H), 2.69 (dd, 1H), 3.29 (dd, 1H), 3.92-4.12 (m, 1H), 4.37 (d, 1H), 4.93 (d, 1H), 7.10-7.29 (m, 2H), 7.29-7.52 (m, 4H), 7.63 (d, 1H), 8.09 (d, 1H), 11.88 (brs, 1H). |

**[Table 215]**

| No. | CDCl₃/TMS *δ* (ppm) |
|---|---|
| 7-4 | 1.41 (s, 6H), 2.98 (s, 2H), 4.78 (s, 2H), 7.09-7.51 (m, 6H), 7.54 (d, 1H), 8.11 (d, 1H), 11.72 (brs, 1H). |
| 7-5 | 1.71 (s, 6H), 4.76 (s, 2H), 6.89 (d, 1H), 7.09 (t, 1H), 7.21 (t, 1H), 7.31-7.42 (m, 2H), 7.47 (dt, 1H), 7.60 (d, 1H), 7.97 (dd, 1H), 11.23 (brs, 1H). |
| 7-6 | 4.93 (s, 2H), 5.15 (s, 2H), 7.15 (d, 1H), 7.25 (t, 1H), 7.34-7.58 (m, 4H), 7.58 (d, 1H), 8.04 (d, 1H), 10.83 (brs, 1H). |
| 7-7 | 4.67 (s, 2H), 4.78 (s, 2H), 7.18-7.47 (m, 6H), 7.64 (d, 1H), 8.14 (dd, 1H), 11.29 (brs, 1H). |
| 7-8 | 1.25 (d, 3H), 3.06-3.24 (m, 1H), 3.44 (dd, 1H), 3.79 (dd, 1H), 4.60-4.79 (m, 2H), 7.08-7.51 (m, 6H), 7.62 (d, 1H), 8.11 (dd, 1H), 11.83 (brs, 1H). |
| 7-9 | 1.07 (d, 3H), 3.22-3.39 (m, 1H), 3.45 (dd, 1H), 3.92 (dd, 1H), 4.59 (d, 1H), 4.77 (d, 1H), 7.00 (dt, 1H), 7.16 (dt, 1H), 7.20-7.47 (m, 3H), 7.64 (d, 1H), 11.17 (brs, 1H). |
| 7-10 | 1.18-1.40 (m, 6H), 3.41 (s, 2H), 4.67 (s, 2H), 7.00 (dt, 1H), 7.14 (dt, 1H), 7.18-7.46 (m, 3H), 7.64 (d, 1H), 11.22 (brs, 1H). |
| 7-11 | 3.92 (s, 3H), 5.14 (s, 2H), 6.74 (s, 1H), 7.39 (dt, 1H), 7.48 (dd, 1H), 7.55-7.64 (m, 2H), 7.73 (d, 1H), 7.93 (d, 1H), 8.48 (d, 1H), 12.19 (brs, 1H). |
| 7-12 | 1.35 (t, 3H), 2.80-3.05 (m, 2H), 3.57 (t, 2H), 4.28-4.48 (m, 2H), 5.28 (d, 1H), 5.83 (d, 1H), 7.06 (dt, 1H), 7.13 (dt, 1H), 7.20-7.32 (m, 1H), 7.36-7.54 (m, 3H). |
| 7-13 | 1.11 (d, 1.5H), 1.18 (d, 1.5H), 1.20-1.38 (m, 3H), 2.64 (dt, 2H), |
| 7-14 | 1.26 (t, 3H), 4.31 (q, 2H), 4.89-5.10 (m, 4H), 7.13 (d, 1H), 7.25 (d, 1H), 7.35-7.53 (m, 4H), 7.62 (dd, 1H), 8.11 (d, 1H). |
| 7-15 | 1.19 (d, 1.5H), 1.23 (d, 1.5H), 1.33 (dt, 3H), 3.09-3.38 (m, 2H), 3.63-3.78 (m, 1H), 4.30-4.48 (m, 2H), 4.65 (dd, 1H), 5.07 (dd, 1H), 7.03 (dt, 1H), 7.16 (dt, 1H), 7.19-7.29 (m, 1H), 7.31-7.59 (m, 3H). |
| 7-16 | 1.23-1.39 (m, 6H), 3.20 (dd, 2H), 4.30-4.48 (m, 2H), 4.62 (d, 1H), 5.10 (d, 1H), 7.02 (dt, 1H), 7.13 (dt, 1H), 7.23 (d, 1H), 7.38 (dt, 1H), 7.46 (dt, 1H), 7.52 (d, 1H). |
| 7-17 | 1.18-1.39 (m, 3H), 4.18-4.39 (m, 2H), 5.22 (d, 1H), 5.41 (d, 1H), 6.51 (d, 1H), 7.11 (d, 1H), 7.18-7.59 (m, 6H), 7.65 (t, 1H), 8.46 (d, 1H). |
| 7-18 | 1.68 (d, 3H), 2.67-2.82 (m, 1H), 2.90-3.05 (m, 1H), 3.37-3.52 (m, 1H), 3.60-3.72 (m, 1H), 6.06 (q, 1H), 6.98 (dt, 1H), 7.14 (dt, 1H), 7.23-7.40 (m, 2H), 7.46 (d, 1H), 7.58 (d, 1H), 10.52 (brs, 1H). |

**[Table 216]**

| No. | CDCl₃/TMS *δ* (ppm) |
|---|---|
| 7-19 | 2.93 (t, 2H), 3.55 (t, 2H), 4.58 (d, 1H), 4.82 (d, 1H), 6.49 (t, 1H), 6.95-7.21 (m, 3H), 7.22-7.41 (m, 3H), 9.28 (brs, 1H). |
| 8-1 | 3.58 (s, 2H), 4.88 (s, 2H), 7.12 (t, 1H), 7.19-7.29 (m, 3H), 7.35-7.40 (m, 2H), 7.49 (dd, 1H), 7.60 (dd, 1H), 10.89 (brs, 1H) |
| 8-2 | 3.59 (s, 2H), 4.87 (s, 2H), 7.03-7.14 (m, 3H), 7.22-7.26 (m, 1H), 7.39 (dt, 1H), 7.45 (dd, 1H), 7.60 (d, 1H), 10.73 (brs, 1H) |
| 8-3 | 3.58 (s, 2H), 4.86 (s, 2H), 7.12 (d, 1H), 7.22-7.26 (m, 2H), 7.33-7.45 (m, 3H), 7.60 (d, 1H), 10.61 (brs, 1H) |
| 8-4 | 3.58 (s, 2H), 4.86 (s, 2H), 7.07 (d, 1H), 7.22-7.26 (m, 1H), 7.36-7.45 (m, 3H), 7.50 (dd, 1H), 7.60 (d, 1H), 10.60 (brs, 1H) |
| 8-5 | 3.56 (s, 2H), 4.85 (s, 2H), 7.10 (dd, 1H), 7.18-7.21 (m, 2H), 7.28 (dt, 1H), 7.40 (dt, 1H), 7.46 (dd, 1H), 7.60 (dd, 1H), 10.59 (brs, 1H) |
| 8-6 | 3.61 (s, 2H), 4.92 (s, 2H), 7.12-7.20 (m, 2H), 7.30 (d, 1H), 7.39 (t, 1H), 7.49 (d, 1H), 7.63 (d, 1H), 7.87 (s, 1H) |
| 8-7 | 1.36 (t, 3H), 3.69 (s, 2H), 4.35-4.47 (m, 2H), 5.02 (ab-q, 2H), 6.60 (d, 1H), 7.03 (dt, 1H), 7.13-7.18 (m, 2H), 7.25-7.30 (m, 2H), 7.35-7.40 (m, 1H) |
| 8-8 | 1.36 (t, 3H), 3.69 (s, 2H), 4.36-4.47 (m, 2H), 4.96 (d, 1H), 5.04 (d, 1H), 6.58 (dd, 1H), 6.85 (dt, 1H), 7.04 (m, 1H), 7.13 (m, 1H), 7.25-7.28 (m, 1H), 7.37-7.41 (m, 2H) |
| 8-9 | 1.36 (t, 3H), 3.68 (s, 2H), 4.35-4.53 (m, 2H), 4.95 (d, 1H), 5.05 (d, 1H), 6.58 (d, 1H), 7.11-7.14 (m, 2H), 7.25-7.27 (m, 2H), 7.37-7.40 (m, 2H) |
| 8-10 | 1.36 (t, 3H), 3.68 (s, 2H), 4.35-4.53 (m, 2H), 5.00 (ab-q, 2H), 6.53 (d, 1H), 7.12 (dd, 1H), 7.25-7.30 (m, 2H), 7.37-7.40 (m, 3H) |
| 8-11 | 1.36 (t, 3H), 3.65 (ab-q, 2H),4.43 (dq, 2H), 4.87 (d, 1H), 5.13 (d, 1H), 6.60(d, 1H), 7.01 (dd, 1H), 7.14-7.20 (m, 2H), 7.26-7.28 (m, 1H), 7.37-7.41 (m, 2H) |
| 8-12 | 1.38 (t, 3H), 3.70 (s, 2H), 4.35-4.53 (m, 2H), 5.06 (ab-q, 2H), 6.63 (d, 1H), 7.06 (t, 1H), 7.18 (t, 1H), 7.28-7.30 (m, 2H), 7.50 (s, 1H), 7.62 (d, 1H) |
| 9-1 | 3.69 (s, 2H), 4.66 (s, 4H), 7.11-7.49 (m, 7H), 7.63 (d, 1H), 11.58 (brs, 1H). |
| 9-2 | 2.61-2.78 (m, 2H), 2.87 (t, 2H), 5.13 (s, 2H), 7.02-7.22 (m, 3H), 7.22-7.46 (m, 4H), 7.54 (d, 1H), 10.38 (brs, 1H). |

**[Table 217]**

| No. | CDCl₃/TMS *δ* (ppm) |
|---|---|
| 9-3 | 3.05 (s, 3H), 4.43 (s, 2H), 5.07 (s, 2H), 6.97-7.09 (m, 2H), 7.13-7.39 (m, 4H), 7.42 (d, 1H), 7.57 (d, 1H), 11.04 (brs, 1H). |
| 9-4 | 5.62(s, 2H), 7.18 (t, 1H), 7.43 (t, 1H), 7.50-7.71 (m, 5H), 7.82 (dt, 1H), 7.92 (d, 1H), 8.31 (d, 1H), 8.39 (d, 1H), 8.57 (d, 1H), 10.98 (brs, 1H). |
| 9-5 | 1.33 (t, 3H), 3.74 (s, 2H), 4.27-4.49 (m, 4H), 4.75 (d, 1H), 4.95 (d, 1H), 7.07 (d, 1H), 7.12-7.32 (m, 5H), 7.32-7.48 (m, 2H). |
| 9-6 | 1.35 (t, 3H), 2.82 (t, 2H), 2.91-3.12 (m, 2H), 4.29-4.52 (m, 2H), 5.11 (d, 1H), 5.37 (d, 1H), 6.76 (d, 1H), 6.92-7.14 (m, 3H), 7.26 (d, 1H), 7.21-7.42 (m, 3H). |
| 9-7 | 1.34 (t, 3H), 3.09 (s, 3H), 4.29-4.51 (m, 2H), 4.49 (q, 2H), 6.61 (d, 1H), 6.96 (t, 1H), 7.02-7.20 (m, 311), 7.20-7.40 (m, 3H). |
| 9-8 | 1.41 (t, 3H), 4.38-4.57 (m, 2H), 6.87 (d, 1H), 7.16-7.46 (m, 6H), 7.63 (t, 1H), 7.83 (dt, 1H), 8.34 (t, 2H), 8.59 (d, 1H). |

Next, Use Examples of the present invention will be described.

### (1) To Implement Formulation

### <Formulation Example 1> Wettable Powder

Ten parts of a compound according to the present invention, 15 parts of sodium lignin sulfonate, 2 parts of sodium lauryl sulfate, 15 parts of hydrated silicon dioxide, and 58 parts of clay were admixed and pulverized to prepare 10% wettable powder.

### <Formulation Example 2> Flowable

Ten parts of a compound according to the present invention, 4 parts of polyoxyethylene allyl phenyl ether sulfate, 5 parts of polyoxyethylene alkyl ether, 5 parts of propylene glycol, 0.2 parts of silicon antifoaming agent, 0.8 parts of sodium montmorillonite, and 50 parts of water were mixed and wet-pulverized using a DYNO-MILL to obtain a pulverized suspension.

Here, 75 parts of the pulverized suspension was admixed with 10 parts of a xanthan gum solution containing 0.2 parts of xanthan gum and 0.1 parts of 2-benzisothiazolin-3-one and 15 parts of water to prepare 10% aqueous suspension-type agrochemical composition.

### <Formulation Example 3> Emulsifiable Concentrate

Ten parts of a compound according to the present invention, 2 parts of calcium dodecylbenzenesulfonate, and 15 parts of castor oil ethoxylate were admixed with and dissolved in 73 parts of aromatic hydrocarbon mixture to prepare homogeneous 10% emulsifiable oily liquid.

### <Formulation Example 4> Water Dispersible Granule

Ten parts of a compound according to the present invention, 20 parts of sodium lignin sulfonate, 10 parts of sodium salt of naphthalene sulfonic acid condensate, 3 parts of sodium alkyl benzene sulfonate, 0.5 parts of silicon-based antifoaming agent, 5 parts of diatomaceous earth, 10 parts of ammonium sulfate, 10 parts of talc, and 31.5 parts of clay were added, stirred, mixed, and pulverized thoroughly to obtain a pulverized material. The pulverized material was optionally admixed with an appropriate amount of water, granulated with a granulator, dried, and then sieved to prepare 10% wettable fine granules.

### <Formulation Example 5> Emulsion

Ten parts of a compound according to the present invention, 15 parts of aromatic hydrocarbon mixture, 2 parts of calcium dodecylbenzenesulfonate, 20 parts of polyoxyethylene castor oil, and 4 parts of propylene glycol were added and dissolved to obtain a mixed solution. The mixed solution was admixed with 49 parts of water by using a homogenizer to prepare homogeneous 10% emulsion liquid.

### <Formulation Example 6> Granule

Ten parts of a compound according to the present invention, 3 parts of polycarboxylic acid type anionic surfactant, 0.2 parts of sodium dioctyl sulfosuccinate, 2 parts of dextrin, 15 parts of sodium bentonite, and 69.8 parts of calcium carbonate were admixed uniformly, kneaded with an appropriate amount of water, extruded and granulated with a basket-type granulator, dried, and then sieved to prepare 10% fine granules.

### <Formulation Example 7> Microemulsion

Ten parts of a compound according to the present invention was mixed with 12 parts of fatty acid dimethylamide, 10 parts of cyclohexanone, and 15 parts of aryl phenol ethoxylate and further admixed with 10 parts of alcohol ethoxylate and 43 parts of water. The mixture was stirred for several minutes while heating to prepare stable 10% watersoluble liquid.

### (2) Test for evaluating herbicidal efficacy of paddy field flooding treatment

### <Test Example 1> Herbicidal effect test using paddy field flooding treatment

After each 40-cm² plastic cup was loaded with puddled paddy field soil, seeds of each of the following species were sown and grown in a greenhouse: *Echinochloa crus-galli* var. *oryzicola* (EC), *Schoenoplectiella juncoides* (SJ), *Monochoria vaginalis* (MV), or *Ammannia multiflora* (AM). After plants were grown for 14 days, water was flooded to a depth of 3 cm, and a 10% wettable powder, which had been adjusted according to Formulation Example 1, was diluted with distilled water and applied dropwise to the water surface. The amount applied was 1000, 500, 250, 125, 63, 31, or 16 g of active ingredient per hectare. The test was conducted in two series. Herbicidal efficacy (degree (%) of suppression of growth of terrestrial part: 100 (complete death) to 0 (no action)) was investigated at 21 days after the treatment. The results of the present invention are shown below.

### Effects on Echinochloa crus-galli var. oryzicola (EC)

Compound 3-188 or 6-17 showed 80% or higher herbicidal efficacy at a dose of 31 g a.i./ha.

Compound 6-24 showed 80% or higher herbicidal efficacy at a dose of 63 g a.i./ha.

Compound 3-69, 3-99, 3-103, 3-109, 3-130, 3-149, 3-164, 3-175, 3-186, 3-189, 3-193, 3-199, 3-218, 3-229, 3-241, 3-243, 3-246, 3-247, 3-255, 4-16, 4-26, or 4-30 showed 80% or higher herbicidal efficacy at a dose of 125 g a.i./ha.

Compound 2-55, 3-9, 3-10, 3-42, 3-58, 3-63, 3-64, 3-97, 3-98, 3-100, 3-101, 3-112, 3-113, 3-117, 3-129, 3-133, 3-140, 3-154, 3-173, 3-177, 3-179, 3-181, 3-182, 3-183, 3-190, 3-192, 3-198, 3-202, 3-205, 3-207, 3-214, 3-215, 3-217, 3-219, 3-222, 3-223, 3-227, 3-228, 3-234, 3-238, 3-244, 5-6, 6-4, 6-22, 8-1, or 9-1 showed 80% or higher herbicidal efficacy at a dose of 250 g a.i./ha.

Compound 2-1, 2-2, 2-22, 2-32, 3-2, 3-3, 3-19, 3-23, 3-27, 3-39, 3-41, 3-53, 3-66, 3-73, 3-80, 3-104, 3-107, 3-108, 3-111, 3-115, 3-121, 3-124, 3-125, 3-127, 3-131, 3-132, 3-134, 3-135, 3-136, 3-139, 3-146, 3-148, 3-151, 3-160, 3-163, 3-165, 3-167, 3-171, 3-172, 3-174, 3-178, 3-180, 3-184, 3-185, 3-187, 3-203, 3-204, 3-206, 3-208, 3-211, 3-212, 3-220, 3-221, 3-224, 3-226, 3-232, 3-233, 3-235, 3-237, 3-239, 3-240, 3-249, 3-252, 3-254, 4-2, 4-5, 4-6, 4-7, 4-36, 5-1, 5-2, 6-7, 6-8, 6-12, 6-16, 6-20, 6-23, 6-26, 7-9, or 8-7 showed 80% or higher herbicidal efficacy at a dose of 500 g a.i./ha.

Compound 8-2 showed 80% or higher herbicidal efficacy at a dose of 1000 g a.i./ha.

### Effects on Schoenoplectiella juncoides (SJ)

Compound 3-116 showed 80% or higher herbicidal efficacy at a dose of 16 g a.i./ha.

Compound 3-124, 3-175, 3-202, 3-226, 3-255, 6-4, 6-8, or 6 -12 showed 80% or higher herbicidal efficacy at a dose of 31 g a.i./ha.

Compound 1-2, 1-6, 2-9, 2-55, 3-3, 3-4, 3-28, 3-39, 3-41, 3-63, 3-69, 3-107, 3-111, 3-112, 3-121, 3-126, 3-129, 3-130, 3-141, 3-146, 3-149, 3-173, 3-179, 3-184, 3-185, 3-189, 3-204, 3-205, 3-206, 3-207, 3-208, 3-217, 3-227, 3-229, 3-241, 3-246, 3-247, 3-254, 6-14, 6-16, 6-17, 6-22, 7-1, 7-7, or 7-8 showed 80% or higher herbicidal efficacy at a dose of 63 g a.i./ha.

Compound 1-1, 1-5, 1-9, 2-1, 2-3, 2-41, 3-12, 3-22, 3-30, 3-37, 3-42, 3-44, 3-48, 3-58, 3-59, 3-64, 3-70, 3-73, 3-77, 3-88, 3-94, 3-98, 3-101, 3-135, 3-142, 3-145, 3-147, 3-148, 3-154, 3-157, 3-164, 3-166, 3-181, 3-182, 3-199, 3-210, 3-218, 3-219, 3-220, 3-223, 3-238, 3-249, 4-1, 4-7, 4-29, 4-30, 4-40, 6-1, 6-21, 6-24, 7-9, 7-10, 7-15, 7-16, or 9-1 showed 80% or higher herbicidal efficacy at a dose of 125 g a.i./ha.

Compound 1-4, 2-4, 2-5, 2-22, 2-34, 2-40, 3-1, 3-2, 3-6, 3-7, 3-9, 3-10, 3-15, 3-16, 3-17, 3-18, 3-19, 3-21, 3-25, 3-33, 3-36, 3-45, 3-46, 3-52, 3-62, 3-65, 3-66, 3-67, 3-92, 3-106, 3-110, 3-117, 3-131, 3-136, 3-138, 3-168, 3-170, 3-190, 3-192, 3-193, 3-209, 3-212, 3-214, 3-237, 3-242, 3-243, 3-251, 3-252, 4-2, 4-5, 4-6, 4-10, 4-13, 4-14, 4-15, 4-16, 4-26, 4-27, 4-28, 4-32, 4-36, 4-37, 4-38, 4-41, 4-42, 5-1, 6-2, 6-5, 7-3, or 8-1 showed 80% or higher herbicidal efficacy at a dose of 250 g a.i./ha.

Compound 1-3, 1-10, 1-11, 2-7, 2-18, 2-32, 2-33, 2-37, 2-57, 3-14, 3-23, 3-27, 3-38, 3-43, 3-53, 3-61, 3-68, 3-76, 3-78, 3-89, 3-90, 3-91, 3-96, 3-100, 3-102, 3-103, 3-104, 3-105, 3-108, 3-119, 3-122, 3-128, 3-137, 3-139, 3-152, 3-153, 3-156, 3-158, 3-159, 3-163, 3-165, 3-167, 3-174, 3-177, 3-178, 3-183, 3-197, 3-198, 3-222, 3-228, 3-230, 3-233, 3-239, 3-244, 3-253, 4-3, 4-8, 4-9, 4-11, 4-12, 4-21, 5-2, 5-6, 6-6, 6-13, 6-15, 6-18, 8-2, 9-2, or 9-5 showed 80% or higher herbicidal efficacy at a dose of 500 g a.i./ha.

Compound 7-17, 8-5, or 9-6 showed 80% or higher herbicidal efficacy at a dose of 1000 g a.i./ha.

### Effects on Monochoria vaginalis (MV)

Compound 3-149, 3-202, 3-211, 3-220, 3-222, or 3-232 showed 80% or higher herbicidal efficacy at a dose of 16 g a.i./ha.

Compound 2-22, 3-63, 3-69, 3-101, 3-107, 3-109, 3-112, 3 -135, 3-136, 3-166, 3-198, 3-227, 3-234, 3-246, 3-247, 4-26, 4-30, or 4-36 showed 80% or higher herbicidal efficacy at a dose of 31 g a.i./ha.

Compound 2-1, 2-55, 3-2, 3-6, 3-19, 3-44, 3-59, 3-76, 3078, 3-97, 3-99, 3-100, 3-102, 3-103, 3-111, 3-113, 3-116, 3-117, 3-121, 3-125, 3-128, 3-129, 3-131, 3-133, 3-137, 3-145, 3-154, 3-156, 3-165, 3-173, 3-174, 3-175, 3-177, 3-178, 3-179, 3-180, 3-181, 3-182, 3-183, 3-184, 3-188, 3-190, 3-197, 3-199, 3-203, 3-204, 3-206, 3-215, 3-217, 3-219, 3-225, 3-230, 3-233, 3-235, 3-237, 3-240, 3-241, 3-242, 3-249, 3-252, 3-254, 4-8, 4-15, 4-16, 4-22, 4-29, 4-37, 5-6, 6-4, 6-5, 6-7, 7-1, 7-7, 7-9, or 7-10 showed 80% or higher herbicidal efficacy at a dose of 63 g a.i./ha.

Compound 1-6, 2-18, 2-31, 2-34, 2-41, 3-1, 3-3, 3-12, 3-21, 3-23, 3-28, 3-36, 3-37, 3-39, 3-41, 3-42, 3-43, 3-46, 3-55, 3-58, 3-60, 3-62, 3-64, 3-66, 3-70, 3-71, 3-73, 3-98, 3-104, 3-105, 3-106, 3-108, 3-115, 3-118, 3-124, 3-130, 3-140, 3-157, 3-171, 3-185, 3-186, 3-187, 3-189, 3-191, 3-192, 3-193, 3-195, 3-201, 3-207, 3-208, 3-218, 3-228, 3-229, 3-238, 3-243, 3-255, 4-7, 4-12, 4-14, 4-21, 4-31, 4-38, 4-40, 4-41, 4-42, 5-1, 5-3, 5-5, 6-1, 6-6, 6-8, 6-13, 6-15, 6-16, 6-17, 7-2, 7-12, 7-13, 7-15, or 8-1 showed 80% or higher herbicidal efficacy at a dose of 125 g a.i./ha.

Compound 1-1, 1-3, 1-4, 2-2, 2-3, 2-32, 2-37, 2-40, 2-57, 3-9, 3-13, 3-15, 3-20, 3-22, 3-24, 3-25, 3-27, 3-30, 3-33, 3-35, 3-45, 3-51, 3-52, 3-53, 3-57, 3-61, 3-72, 3-77, 3-91, 3-110, 3-114, 3-119, 3-126, 3-127, 3-134, 3-139, 3-148, 3-151, 3-153, 3-163, 3-169, 3-170, 3-209, 3-210, 3-212, 3-221, 3-223, 3-244, 4-2, 4-5, 4-6, 4-11, 4-13, 4-17, 4-18, 4-23, 4-27, 5-2, 6-14, 6-19, 6-20, 6-22, 7-8, 7-16, 8-2, 8-7, 8-8, or 9-1 showed 80% or higher herbicidal efficacy at a dose of 250 g a.i./ha.

Compound 1-2, 1-5, 1-9, 2-4, 2-5, 2-7, 2-9, 2-23, 2-26, 2-30, 2-33, 2-36, 2-38, 2-43, 2-47, 2-51, 3-4, 3-11, 3-14, 3-16, 3-38, 3-65, 3-74, 3-80, 3-84, 3-87, 3-88, 3-89, 3-92, 3-94, 3-96, 3-120, 3-122, 3-123, 3-143, 3-147, 3-150, 3-158, 3-159, 3-160, 3-161, 3-167, 3-172, 3-214, 3-224, 3-226, 3-239, 4-1, 4-3, 4-4, 4-9, 4-10, 5-4, 6-10, 6-12, 6-18, 6-21, 6-25, 6-26, 7-3, 8-3, 8-5, 8-9, 8-11, or 9-5 showed 80% or higher herbicidal efficacy at a dose of 500 g a.i./ha.

Compound 8-4 or 9-3 showed 80% or higher herbicidal efficacy at a dose of 1000 g a.i./ha.

### Effects on Ammannia multiflora (AM)

Compounds 1-6, 2-18, 3-2, 3-3, 3-4, 3-24, 3-25, 3-42, 3-57, 3-63, 3-64, 3-69, 3-70, 3-72, 3-91, 3-96, 3-97, 3-98, 3-99, 3-100, 3-101, 3-102, 3-103, 3-104, 3-105, 3-107, 3-108, 1-109, 1-110, 3-111, 3-112, 3-113, 3-114, 3-115, 3-116, 3-117, 3-119, 3-121, 3-124, 3-126, 3-127, 3-128, 3-129, 3-130, 3-131, 3-132, 3-133, 3-134, 3-135, 3-136, 3-137, 3-140, 3-149, 3-154, 3-164, 3-166, 3-170, 3-171, 3-172, 3-173, 3-174, 3-175, 3-177, 3-178, 3-179, 3-180, 3-181, 3-182, 3-183, 3-184, 3-185, 3-186, 3-188, 3-189, 3-190, 3-192, 3-193, 3-195, 3-197, 3-198, 3-201, 3-202, 3-203, 3-204, 3-205, 3-206, 3-207, 3-208, 3-210, 3-211, 3-212, 3-214, 3-215, 3-217, 3-218, 3-219, 3-220, 3-221, 3-222, 3-223, 3-225, 3-226, 3-227, 3-228, 3-229, 3-232, 3-233, 3-234, 3-237, 3-240, 3-241, 3-242, 3-243, 3-244, 3-246, 3-247, 3-249, 3-252, 3-254, 3-255, 4-12, 4-15, 4-16, 4-21, 4-26, 4-30, 4-36, 6-4, 6-8, 6-17, 6-18, 6-22, 7-2, 7-9, or 7-15 showed 80% or higher herbicidal efficacy at a dose of 16 g a.i./ha.

Compound 1-5, 2-9, 2-22, 3-1, 3-10, 3-26, 3-33, 3-44, 3-46, 3-48, 3-51, 3-53, 3-58, 3-59, 3-60, 3-61, 3-76, 3-78, 3-118, 3-125, 3-142, 3-146, 3-148, 3-156, 3-199, 3-209, 3-224, 3-230, 3-235, 3-239, 4-13, 4-22, 4-27, 4-29, 4-37, 4-41, 6-7, 6-12, 6-14, 6-16, or 7-10 showed 80% or higher herbicidal efficacy at a dose of 31 g a.i./ha.

Compound 1-2, 2-3, 2-4, 2-19, 2-55, 3-6, 3-7, 3-15, 3-19, 3-10, 3-21, 3-23, 3-30, 3-31, 3-34, 3-36, 3-39, 3-40, 3-41, 3-43, 3-47, 3-49, 3-50, 3-52, 3-55, 3-56, 3-62, 3-65, 3-66, 3-68, 3-79, 3-80, 3-87, 3-88, 3-89, 3-92, 3-106, 3-120, 3-122, 3-123, 3-139, 3-145, 3-147, 3-151, 3-152, 3-153, 3-161, 3-162, 3-163, 3-165, 3-167, 3-168, 3-169, 3-194, 3-213, 4-1, 4-4, 4-5, 4-6, 4-7, 4-8, 4-10, 4-11, 4-14, 4-25, 4-40, 4-42, 5-6, 6-1, 6-5, 6-6, 6-13, 6-19, 6-20, 6-21, 6-25, 6-26, 7-1, 7-7, 7-8, or 7-12 showed 80% or higher herbicidal efficacy at a dose of 63 g a.i./ha.

Compound 1-1, 1-3, 1-4, 1-9, 1-11, 2-1, 2-2, 2-17, 2-31, 2-34, 2-41, 2-57, 3-9, 3-12, 3-13, 3-14, 3-22, 3-27, 3-28, 3-29, 3-32, 3-35, 3-37, 3-38, 3-45, 3-81, 3-84, 3-86, 3-94, 3-138, 3-141, 3-160, 3-187, 3-191, 4-17, 4-18, 4-23, 4-28, 4-31, 4-38, 5-1, 5-2, 5-5, 6-2, 6-15, 6-23, 6-24, 7-16, 8-1, 8-3, 8-7, 8-8, or 9-1 showed 80% or higher herbicidal efficacy at a dose of 125 g a.i./ha.

Compound 1-10, 2-5, 2-23, 2-25, 2-32, 2-38, 2-40, 2-43, 2-50, 2-52, 3-5, 3-16, 3-18, 3-67, 3-71, 3-73, 3-82, 3-93, 3-95, 3-150, 3-157, 3-158, 3-231, 3-236, 3-238, 3-251, 3-253, 4-2, 4-3, 4-9, 4-32, 4-33, 5-3, 8-2, 8-9, 8-11, or 9-5 showed 80% or higher herbicidal efficacy at a dose of 250 g a.i./ha.

Compound 2-7, 2-24, 2-33, 2-36, 2-47, 2-51, 3-11, 3-77, 3-83, 3-85, 3-159, 6-3, 6-10, 7-3, 7-13, or 8-5 showed 80% or higher herbicidal efficacy at a dose of 500 g a.i./ha.

Compound 2-58, 7-17, 8-4, 8-10, 9-3, or 9-7 showed 80% or higher herbicidal efficacy at a dose of 1000 g a.i./ha.

Although each compound of the present invention is a new compound, the following control compounds a and b are known as structurally similar compounds.

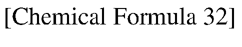

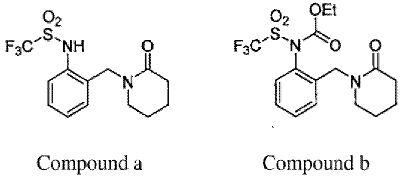

The control compound a is described as Compound No. III-1 on page 245 of PLT 5, and the control compound b is described as Compound No. III-9 on page 245 of PTL 5. The present inventors, however, have found that compounds of the present invention are clearly superior to the control compounds in terms of herbicidal efficacy. As an example, herbicidal efficacy against critical paddy field weeds such as *Monochoria vaginalis* (MV) and *Ammannia multiflora* (AM) as well as *Oryza sativa* (OS) chemical damage were compared and are specifically described below as a Comparative Example.

[Comparative Example] Herbicidal efficacy test of treatment during growth stage of millet under flooding conditions.

After each 40-cm² plastic cup was loaded with puddled paddy field soil, seeds of each of *Monochoria vaginalis* (MV) or *Ammannia multiflora* (AM) were sown and grown in a greenhouse. After plants were grown for 14 days, one rice plant (variety: Nippon-bare) at the 2-leaf stage was transplanted at a transplanting depth of 1 cm. Water was flooded to a depth of 3 cm, and wettable powder containing the present invention compound, which had been adjusted according to Formulation Example 1, or each control compound was diluted with distilled water and applied dropwise to the water surface. The amount applied was 125, 63, or 31 g of active ingredient per hectare. The test was conducted in two series. On day 21 after treatment, the herbicidal efficacy and chemical damage were examined according to the following criteria.

### Criteria

9-10 ... Herbicidal activity "Maximum", 90% or higher herbicidal efficacy or chemical damage
7 to 8 ... Herbicidal activity "high", 70% or higher and less than 90% herbicidal efficacy or chemical damage
5 to 6 ... Herbicidal activity "medium", 50% or higher and less than 70% herbicidal efficacy or chemical damage
0 to 4 ... Herbicidal activity "none to low", 0% or higher and less than 50% herbicidal efficacy or chemical damage

The results are described below.

**[Table 218]**

| No. | Dose (g a.i./ha) | OS | MV | AM |
|---|---|---|---|---|
| Compound a | 125 | 10 | 10 | 10 |
| | 63 | 10 | 10 | 10 |
| | 31 | 6 | 10 | 10 |
| Compound b | 125 | 10 | 10 | 10 |
| | 63 | 8 | 10 | 10 |
| | 31 | 3 | 7 | 10 |
| 3-1 | 125 | 0 | 10 | 10 |
| | 63 | 0 | 10 | 10 |
| | 31 | 0 | 10 | 10 |
| 3-139 | 125 | 0 | 10 | 10 |
| | 63 | 0 | 10 | 10 |
| | 31 | 0 | 7 | 10 |

### INDUSTRIAL APPLICABILITY

This invention provides novel compounds with excellent herbicidal activity, is extremely useful in the fields of agrochemicals and agriculture, and thus has industrial applicability.

## Claims

1. A compound or a salt thereof, the compound represented by General Formula [1]: 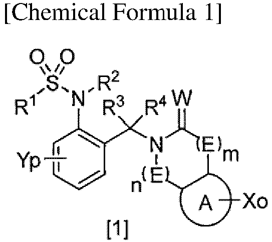
wherein A is an optionally substituted benzene ring or heteroaromatic ring,
E is -C(R⁵)(R⁶)-, an oxygen atom, a sulfur atom, SO, SO₂, or N(R⁷),
W is an oxygen or sulfur atom,
o is from 0 to 4,
p is from 0 to 4,
m is from 0 to 3,
n is from 0 to 3,
R¹ is halo C₁-C₆ alkyl,
R² is a hydrogen atom, C₁-C₆ alkyl optionally substituted by one or more substituents selected from Z¹, C₂-C₆ alkenyl, halo C₂-C₆ alkenyl, C₂-C₆ alkynyl, halo C₂-C₆ alkynyl, (C₁-C₁₂ alkyl)carbonyl, (halo C₁-C₁₂ alkyl)carbonyl, (C₃-C₆ cycloalkyl)carbonyl, (halo C₃-C₆ cycloalkyl)carbonyl, (C₁-C₆ alkyl C₃-C₆ cycloalkyl)carbonyl, C₁-C₆ alkyl(halo C₃-C₆ cycloalkyl)carbonyl, (C₃-C₆ cycloalkoxy)carbonyl, (C₂-C₆ alkenyl)carbonyl, (halo C₂-C₆ alkenyl)carbonyl, phenyl(C₂-C₆ alkenyl)carbonyl optionally substituted by one or more substituents selected from Z², C₁-C₆ alkoxy(C₂-C₆ alkenyl)carbonyl, (C₂-C₆ alkynyl)carbonyl, phenylcarbonyl optionally substituted by one or more substituents selected from Z², heterocyclyl carbonyl optionally substituted by one or more substituents selected from Z², C₃-C₆ cycloalkyl(C₁-C₆ alkyl)carbonyl, (C₁-C₆ alkyl)carbonyl substituted by one substituent selected from Z⁴, (hydroxy C₁-C₆ alkyl)carbonyl, C₁-C₆ alkoxy(C₁-C₆ alkyl)carbonyl, halo C₁-C₆ alkoxy(C₁-C₆ alkyl)carbonyl, (C₁-C₆ alkyl)carbonyl substituted by one substituent selected from Z⁵, phenoxy(halo C₁-C₆ alkyl)carbonyl, (halo C₁-C₆ alkyl)carbonyl substituted by Z⁵, tri(C₁-C₆ alkyl)silyloxy(C₁-C₆ alkyl)carbonyl, C₁-C₆ alkoxy C₁-C₆ alkoxy(C₁-C₆ alkyl)carbonyl, (C₁-C₆ alkyl)carbonyl(C₁-C₆ alkyl)carbonyl, (C₁-C₆ alkyl)carbonyloxy(C₁-C₆ alkyl)carbonyl, (C₁-C₆ alkoxy)carbonyl(C₁-C₆ alkyl)carbonyl, C₁-C₆ alkylthio(C₁-C₆ alkyl)carbonyl, halo C₁-C₆ alkylthio(C₁-C₆ alkyl)carbonyl, (C₁-C₁₂ alkoxy)carbonyl, (halo C₁-C₆ alkoxy)carbonyl, (C₃-C₆ cycloalkyloxy)carbonyl, (C₂-C₆ alkenyloxy)carbonyl, (halo C₂-C₆ alkenyloxy)carbonyl, (C₂-C₆ alkynyloxy)carbonyl, phenoxycarbonyl optionally substituted by one or more substituents selected from Z², heterocyclyloxycarbonyl optionally substituted by one or more substituents selected from Z², C₃-C₆ cycloalkyl(C₁-C₆ alkoxy)carbonyl, (C₁-C₆ alkoxy)carbonyl substituted by one substituent selected from Z⁴, (cyano C₁-C₆ alkoxy)carbonyl, (hydroxy C₁-C₆ alkoxy)carbonyl, C₁-C₆ alkoxy(C₁-C₆ alkoxy)carbonyl, (C₁-C₆ alkoxy)carbonyl substituted by one or more substituents selected from C₁-C₆ alkoxy, halo C₁-C₆ alkoxy(C₁-C₆ alkoxy)carbonyl, tri-C₁-C₆ alkylsilyloxy(C₁-C₆ alkoxy)carbonyl, C₁-C₆ alkoxy C₁-C₆ alkoxy(C₁-C₆ alkoxy)carbonyl, (C₁-C₆ alkyl)carbonyloxy(C₁-C₆ alkoxy)carbonyl, C₁-C₆ alkylsulfonyloxy(C₁-C₆ alkoxy)carbonyl, (C₁-C₆ alkyl)carbonyl(C₁-C₆ alkoxy)carbonyl, (C₁-C₆ alkoxy)carbonyl(C₁-C₆ alkoxy)carbonyl, C₁-C₆ alkylthio(C₁-C₆ alkoxy)carbonyl, halo C₁-C₆ alkylthio(C₁-C₆ alkoxy)carbonyl, C₁-C₆ alkylsulfinyl(C₁-C₆ alkoxy)carbonyl, C₁-C₆ alkylsulfonyl(C₁-C₆ alkoxy)carbonyl, (C₁-C₆ alkylthio)carbonyl, (halo C₁-C₆ alkylthio)carbonyl, mono(C₁-C₆ alkyl)aminocarbonyl, mono(halo C₁-C₆ alkyl)aminocarbonyl, same or different di(C₁-C₆ alkyl)aminocarbonyl, same or different halo di(C₁-C₆ alkyl)aminocarbonyl, C₁-C₆ alkoxyoxalyl, C₁-C₆ alkylsulfonyl, halo C₁-C₆ alkylsulfonyl, C₃-C₆ cycloalkylsulfonyl, C₂-C₆ alkenylsulfonyl, halo C₁-C₆ alkenylsulfonyl, phenylsulfonyl optionally substituted by one or more substituents selected from Z², heterocyclylsulfonyl optionally substituted by one or more substituents selected from Z², C₃-C₆ cycloalkyl C₁-C₆ alkyl sulfonyl, C₁-C₆ alkyl sulfonyl substituted by one substituent selected from Z⁴, C₁-C₆ alkoxy C₁-C₆ alkylsulfonyl, aminosulfonyl, mono C₁-C₆ alkylaminosulfonyl, same or different di C₁-C₆ alkylaminosulfonyl, C₁-C₆ alkylthio, halo C₁-C₆ alkylthio, or cyano,
Z¹ is, each independently, a halogen atom, C₃-C₆ cycloalkyl, phenyl optionally substituted by one or more substituents selected from Z², heterocyclyl optionally substituted by one or more substituents selected from Z², cyano, C₁-C₆ alkoxy optionally substituted by one or more substituents selected from Z³, C₃-C₆ cycloalkyloxy, phenoxy optionally substituted by one or more substituents selected from Z², phenylcarbonyloxy optionally substituted by one or more substituents selected from Z², heterocyclyl carbonyloxy optionally substituted by one or more substituents selected from Z², (C₁-C₆ alkyl)carbonyloxy optionally substituted by one or more halogen atoms, (C₃-C₆ cycloalkyl)carbonyloxy, (C₁-C₆ alkoxy)carbonyloxy, phenoxycarbonyloxy optionally substituted by one or more substituents selected from Z², -CONR¹¹R¹², C₁-C₆ alkylthio optionally substituted by one or more halogen atoms, C₁-C₆ alkylsulfinyl optionally substituted by one or more halogen atoms, C₁-C₆ alkylsulfonyl optionally substituted by one or more halogen atoms, phenylthio optionally substituted by one or more substituents selected from Z², phenylsulfinyl optionally substituted by one or more substituents selected from Z², C₁-C₆ alkylthio optionally substituted by one substituent selected from Z⁴, C₁-C₆ alkylsulfinyl optionally substituted by one substituent selected from Z⁴, C₁-C₆ alkylsulfinyl optionally substituted by one substituent selected from Z⁴, thiocyanato, (C₁-C₆ alkyl)carbonyl optionally substituted by one or more halogen atoms, (C₁-C₆ alkoxy)carbonyl optionally substituted by one or more halogen atoms, or phenylcarbonyl optionally substituted by one or more substituents selected from Z²,
Z² each independently represents halogen, C₁-C₆ alkyl, C₂-C₆ alkenyl, C₂-C₆ alkynyl, C₃-C₆ cycloalkyl, halo C₁-C₆ alkyl, halo C₃-C₆ cycloalkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, phenyl, (C₁-C₆ alkyl)carbonyl, (halo C₁-C₆ alkyl)carbonyl, (C₁-C₆ alkoxy)carbonyl, carboxyl, mono(C₁-C₆ alkyl)aminocarbonyl, phenyl substituted by one or more substituents selected from Z⁶,. heterocyclyl optionally substituted by one or more substituents selected from Z⁷, phenoxy optionally substituted by one or more substituents selected from Z⁷, phenylthio optionally substituted by one or more substituents selected from Z⁷, phenylsulfinyl optionally substituted by one or more substituents selected from Z⁷, phenylsulfonyl optionally substituted by one or more substituents selected from Z⁷, phenylcarbonyl optionally substituted by one or more substituents selected from Z⁷, di(C₁-C₆ alkyl)aminocarbonyl, -NR¹³R¹⁴, hydroxyl, cyano, or nitro,
or two Z² are optionally bonded together with an adjacent carbon or nitrogen atom on a benzene or heterocyclic ring to form a 5- or 6-membered carbocycle or a 5- or 6-membered heterocycle,
provided that the heterocycle contains one or two heteroatoms selected from the group consisting of an oxygen atom, a sulfur atom, and a nitrogen atom where the nitrogen atom is optionally substituted by C₁-C₆ alkyl, C₂-C₆ alkenyl, C₂-C₆ alkynyl, or C₃-C₆ cycloalkyl, and the carbocycle or heterocycle is optionally substituted by one or more halogen atoms,
the heteroaromatic ring is thiophene, furan, pyrrole, oxazole, isoxazoline, thiazole, isothiazole, pyrazole, imidazole, 1,3,4-oxadiazole, 1,2,4-oxadiazole, 1,3,4-thiadiazole, 1,2,4-thiadiazole, 1,2,4-triazole, 1,2,3-thiadiazole, 1,2,3-triazole, 1,2,3,4-tetrazole, pyridine, pyrimidine, pyrazine, pyridazine, 1,3,5-triazine, 1,2,4-triazine, benzothiophene, benzofuran, indole, benzothiazole, benzoimidazole, benzoisoxazole, benzoisothiazole, indazole, benzoxazole, quinoline, isoquinoline, quinoxaline, phthalazine, cinnoline, or quinazoline,
the heterocyclyl is thienyl, furyl, pyrrolyl, oxazolyl, isoxazolyl, isoxazolinyl, thiazolyl, isothiazolyl, pyrazolyl, imidazolyl, 1,3,4-oxadiazolyl, 1,2,4-oxadiazolyl, 1,3,4-thiadiazolyl, 1,2,4-thiadiazolyl, 1,2,4-triazolyl, 1,2,3-thiadiazolyl, 1,2,3-triazolyl, 1,2,3,4-tetrazolyl, pyridyl, pyrimidinyl, pyrazinyl, pyridazinyl, 1,3,5-triazinyl, 1,2,4-triazinyl, benzothienyl, benzofuryl, indolyl, benzothiazolyl, benzoimidazolyl, benzoisoxazolyl, benzoisothiazolyl, indazolyl, benzoxazolyl, quinolyl, isoquinolyl, quinoxalinyl, phthalazinyl, cinnolinyl, quinazolinyl, oxiranyl, oxetanyl, tetrahydrofuranyl, tetrahydrothienyl, 1,1-dioxytetrahydrothienyl, tetrahydropyranyl, tetrahydrothiopyranyl, 1,3-dioxanyl, 2-oxo-1,3-dioxolanyl, 2-oxo-1,3-dioxolyl, or 2,5-dioxopyrrolidinyl,
Z³ is a halogen atom, phenyl optionally substituted by one or more substituents selected from Z², tri-C₁-C₆ alkylsilyl, C₁-C₆ alkyl diphenylsilyl, C₁-C₆ alkoxy optionally substituted by one or more halogen atoms, phenylcarbonyloxy optionally substituted by one or more substituents selected from Z², or phenylsulfonyl optionally substituted by one or more substituents selected from Z²,
Z⁴ is phenyl optionally substituted by one or more substituents selected from Z² or heterocyclyl optionally substituted by one or more substituents selected from Z²,
Z⁵ is phenoxy optionally substituted by one or more substituents selected from Z²,
Z⁶ is selected from the group consisting of di(C₁-C₆ alkyl)aminocarbonyl, hydroxy, amino, cyano, and nitro,
Z⁷ is selected from the group consisting of halogen, C₁-C₆ alkyl, C₂-C₆ alkenyl, C₂-C₆ alkynyl, C₃-C₆ cycloalkyl, halo C₁-C₆ alkyl, halo C₃-C₆ cycloalkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, (C₁-C₆ alkyl)carbonyl, (halo C₁-C₆ alkyl)carbonyl, (C₁-C₆ alkoxy)carbonyl, carboxyl, mono(C₁-C₆ alkyl)aminocarbonyl, same or different di(C₁-C₆ alkyl)aminocarbonyl, hydroxyl, amino, cyano, and nitro,
Z⁸ is selected from the group consisting of phenylthio optionally substituted by one or more substituents selected from Z², phenylsulfinyl optionally substituted by one or more substituents selected from Z², and phenylsulfonyl optionally substituted by one or more substituents selected from Z²,
R¹¹ and R¹² are each independently selected from the group consisting of a hydrogen atom, C₁-C₆ alkyl, and phenyl optionally substituted by one or more substituents selected from Z²,
R¹³ is selected from the group consisting of a hydrogen atom, and C₁-C₆ alkyl,
R¹⁴ is a hydrogen atom or phenyl optionally substituted by one or more substituents selected from Z⁷,
R³ and R⁴ are each independently a hydrogen atom, C₁-C₆ alkyl, halo C₁-C₆ alkyl, C₁-C₆ cycloalkyl, halo C₁-C₆ cycloalkyl, C₁-C₆ alkoxy, halo C₁-C₆ alkoxy, halogen, or cyano,
or R³ and R⁴ are optionally bonded together to form a 3- to 7-membered ring,
R⁵ and R⁶ are each independently a hydrogen atom, halogen, C₁-C₆ alkyl, halo C₁-C₆ alkyl, C₂-C₆ alkenyl, C₂-C₆ alkynyl, C₃-C₆ cycloalkyl, halo C₃-C₆ cycloalkyl, hydroxy C₁-C₆ alkyl, C₁-C₆ alkoxy C₁-C₆ alkyl, halo C₁-C₆ alkoxy C₁-C₆ alkyl, C₁-C₆ alkylthio C₁-C₆ alkyl, halo C₁-C₆ alkylthio C₁-C₆ alkyl, mono(C₁-C₆ alkyl)amino C₁-C₆ alkyl, same or different di(C₁-C₆ alkyl)amino C₁-C₆ alkyl, phenyl optionally substituted by one or more substituents selected from Z², heterocyclyl optionally substituted by one or more substituents selected from Z², C₁-C₆ alkyl substituted by one substituent selected from Z⁴, C₁-C₆ alkyl substituted by one substituent selected from Z⁵, (C₁-C₆ alkyl)carbonyl, (halo C₁-C₆ alkyl)carbonyl, phenylcarbonyl optionally substituted by one or more substituents selected from Z², (C₁-C₆ alkoxy)carbonyl, (halo C₁-C₆ alkoxy)carbonyl, carboxyl, -NR¹¹R¹², C₁-C₆ alkoxy, halo C₁-C₆ alkoxy, phenoxy optionally substituted by one or more substituents selected from Z², C₁-C₆ alkylthio, halo C₁-C₆ alkylthio, phenylthio optionally substituted by one or more substituents selected from Z², C₁-C₆ alkylsulfonyl, halo C₁-C₆ alkylsulfonyl, mono(C₁-C₆ alkyl)amino, di(C₁-C₆ alkyl)amino, (C₁-C₆ alkyl)carbonyloxy, hydroxyl, amino, cyano, or nitro,
or R⁵ and R⁶ on the same carbon are optionally bonded together to form a 3- to 7-membered ring optionally interrupted by one or two heteroatoms selected from the group consisting of an oxygen atom, a sulfur atom, and a nitrogen atom where the nitrogen atom is optionally substituted by C₁-C₆ alkyl, C₂-C₆ alkenyl, C₂-C₆ alkynyl, or C₃-C₆ cycloalkyl or optionally substituted, or to form an olefin,
or R⁵ and R⁶ are optionally bonded to R⁵ or R⁶ on different carbon to form a 3- to 8-membered ring optionally interrupted by one or two heteroatoms selected from the group consisting of an oxygen atom, a sulfur atom, and a nitrogen atom where the nitrogen atom is optionally substituted by C₁-C₆ alkyl, C₂-C₆ alkenyl, C₂-C₆ alkynyl, or C₃-C₆ cycloalkyl or using optionally substituted C₁-C₄ alkylene, halo C₁-C₄ alkylene, C₂-C₄ alkenylene, or halo C₂-C₄ alkenylene,
or R⁵ and R⁶ are optionally bonded together with R⁵ or R⁶ on adjacent carbon to form a bond,
R⁷ is a hydrogen atom, C₁-C₆ alkyl, halo C₁-C₆ alkyl, C₃-C₆ cycloalkyl, halo C₃-C₆ cycloalkyl, C₂-C₆ alkenyl, halo C₂-C₆ alkenyl, C₂-C₆ alkynyl, hydroxy C₁-C₆ alkyl, C₁-C₆ alkoxy C₁-C₆ alkyl, halo C₁-C₆ alkoxy C₁-C₆ alkyl, C₃-C₆ cycloalkyl C₁-C₆ alkyl, C₁-C₆ alkyl substituted by one substituent selected from Z⁴, phenyl optionally substituted by one or more substituents selected from Z², C₁-C₆ alkyl substituted by one substituent selected from Z⁴, (C₁-C₆ alkyl)carbonyl C₁-C₆ alkyl, (halo C₁-C₆ alkyl)carbonyl C₁-C₆ alkyl, (C₁-C₆ alkoxy)carbonyl C₁-C₆ alkyl, (halo C₁-C₆ alkoxy)carbonyl C₁-C₆ alkyl, (C₁-C₆ alkyl)carbonyl, (halo C₁-C₆ alkyl)carbonyl, (C₃-C₆ cycloalkyl)carbonyl, C₃-C₆ cycloalkyl(C₁-C₆ alkyl)carbonyl, (C₂-C₆ alkenyl)carbonyl, phenylcarbonyl optionally substituted by one or more substituents selected from Z², heterocyclyl carbonyl optionally substituted by one or more substituents selected from Z², (C₁-C₆ alkoxy)carbonyl, (halo C₁-C₆ alkoxy)carbonyl, phenoxycarbonyl optionally substituted by one or more substituents selected from Z², aminocarbonyl, mono(C₁-C₆ alkyl)aminocarbonyl, mono halo(C₁-C₆ alkyl)aminocarbonyl, same or different di(C₁-C₆ alkyl)aminocarbonyl, same of different halo di(C₁-C₆ alkyl)aminocarbonyl, (C₁-C₆ alkylthio)carbonyl, (halo C₁-C₆ alkylthio)carbonyl, C₁-C₆ alkylsulfonyl, halo C₁-C₆ alkylsulfonyl, phenylsulfonyl optionally substituted by one or more substituents selected from Z², C₁-C₆ alkylthio C₁-C₆ alkyl, halo C₁-C₆ alkylthio C₁-C₆ alkyl, C₁-C₆ alkyl substituted by one substituent selected from Z⁸, C₁-C₆ alkylsulfinyl C₁-C₆ alkyl, halo C₁-C₆ alkylsulfinyl C₁-C₆ alkyl, C₁-C₆ alkylsulfonyl C₁-C₆ alkyl, halo C₁-C₆ alkylsulfonyl C₁-C₆ alkyl, cyano, amino, or hydroxy,
X is each independently a hydrogen atom, halogen, C₁-C₆ alkyl, C₂-C₆ alkenyl, C₂-C₆ alkynyl, C₃-C₆ cycloalkyl, halo C₁-C₆ alkyl, halo C₂-C₆ alkenyl, halo C₂-C₆ alkynyl, halo C₃-C₆ cycloalkyl, C₁-C₆ alkoxy, halo C₁-C₆ alkoxy, C₂-C₆ alkenyloxy, C₂-C₆ alkynyloxy, C₃-C₆ cycloalkyl C₁-C₆ alkyloxy, phenyl C₁-C₆ alkyloxy, (C₁-C₆ alkyl)carbonyloxy, phenylcarbonyloxy optionally substituted by one or more substituents selected from Z², heterocyclyl carbonyloxy optionally substituted by one or more substituents selected from Z², (C₁-C₆ alkoxy)carbonyloxy, (C₁-C₆ alkylthio)carbonyloxy, C₁-C₆ alkylsulfonyloxy, halo C₁-C₆ alkylsulfonyloxy, phenylsulfonyloxy, di C₁-C₆ alkylphosphorooxy, tri C₁-C₆ alkylsilyloxy, C₁-C₆ alkoxyC₁-C₆ alkyl, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, phenyl optionally substituted by one or more substituents selected from Z², heterocyclyl optionally substituted by one or more substituents selected from Z², phenoxy optionally substituted by one or more substituents selected from Z², phenylthio optionally substituted by one or more substituents selected from Z², phenylsulfinyl optionally substituted by one or more substituents selected from Z², phenylsulfonyl optionally substituted by one or more substituents selected from Z², di(C₁-C₆ alkyl)amino, (C₁-C₆ alkyl)carbonylamino, bis((C₁-C₆ alkyl)carbonyl)amino, halo C₁-C₆ alkylsulfonylamino, morpholinyl, isoindoline-1,3-dione-2-yl, (C₁-C₆ alkyl)carbonyl, (halo C₁-C₆ alkyl)carbonyl, phenylcarbonyl optionally substituted by one or more substituents selected from Z², (C₁-C₆ alkoxy)carbonyl, carboxyl, mono(C₁-C₆ alkyl)aminocarbonyl, same or different di(C₁-C₆ alkyl)aminocarbonyl, phenylaminocarbonyl optionally substituted by one or more substituents selected from Z², phenyl(C₁-C₆ alkylamino)carbonyl, (C₁-C₆ alkylamino)carbonyl substituted by one substituent selected from Z⁴, hydroxy, amino, cyano, or nitro,
or two X attached to an adjacent carbon or nitrogen atom on a benzene or heterocyclic ring are bonded together with a ring atom to which they are attached to form a 4-to 6-membered carbocycle or heterocycle where the heterocycle contains one or two heteroatoms selected from the group consisting of an oxygen atom, a sulfur atom, and a nitrogen atom,
provided that the nitrogen atom is optionally substituted by C₁-C₆ alkyl, C₂-C₆ alkenyl, C₂-C₆ alkynyl, or C₃-C₆ cycloalkyl, and
Y is each independently a hydrogen atom, halogen, C₁-C₆ alkyl, C₂-C₆ alkenyl, C₂-C₆ alkynyl, C₃-C₆ cycloalkyl, halo C₁-C₆ alkyl, halo C₂-C₆ alkenyl, halo C₂-C₆ alkynyl, halo C₃-C₆ cycloalkyl, C₁-C₆ alkoxy, halo C₁-C₆ alkoxy, C₂-C₆ alkenyloxy, C₂-C₆ alkynyloxy, C₃-C₆ cycloalkyl C₁-C₆ alkyloxy, phenyl C₁-C₆ alkyloxy, (C₁-C₆ alkyl)carbonyloxy, phenylcarbonyloxy optionally substituted by one or more substituents selected from Z², heterocyclyl carbonyloxy optionally substituted by one or more substituents selected from Z², (C₁-C₆ alkoxy)carbonyloxy, (C₁-C₆ alkylthio)carbonyloxy, C₁-C₆ alkylsulfonyloxy, halo C₁-C₆ alkylsulfonyloxy, phenylsulfonyloxy, di C₁-C₆ alkylphosphorooxy, tri C₁-C₆ alkylsilyloxy, C₁-C₆ alkoxy C₁-C₆ alkyl, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, phenyl optionally substituted by one or more substituents selected from Z², heterocyclyl optionally substituted by one or more substituents selected from Z², phenoxy optionally substituted by one or more substituents selected from Z², phenylthio optionally substituted by one or more substituents selected from Z², phenylsulfinyl optionally substituted by one or more substituents selected from Z², phenylsulfonyl optionally substituted by one or more substituents selected from Z², di(C₁-C₆ alkyl)amino, (C₁-C₆ alkyl)carbonylamino, bis((C₁-C₆ alkyl)carbonyl)amino, halo C₁-C₆ alkylsulfonylamino, morpholinyl, isoindoline-1,3-dione-2-yl, (C₁-C₆ alkyl)carbonyl, (halo C₁-C₆ alkyl)carbonyl, phenylcarbonyl optionally substituted by one or more substituents selected from Z², (C₁-C₆ alkoxy)carbonyl, carboxyl, -NR¹¹R¹², hydroxy, amino, cyano, or nitro,
or two Y are optionally bonded together with an adjacent carbon or nitrogen atom on a benzene ring to form a 5- or 6-membered carbocycle or a 5- or 6-membered heterocycle,
provided that the heterocycle contains one or two heteroatoms selected from the group consisting of an oxygen atom, a sulfur atom, and a nitrogen atom where the nitrogen atom is optionally substituted by C₁-C₆ alkyl, C₂-C₆ alkenyl, C₂-C₆ alkynyl, or C₃-C₆ cycloalkyl, and the carbocycle or heterocycle is optionally substituted by one or more halogen atoms.

2. The compound or salt thereof according to claim 1, the compound represented by General Formula [1a]: 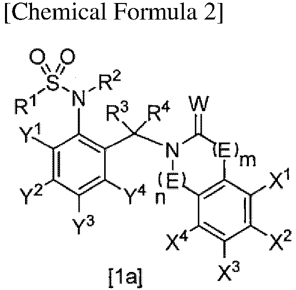
wherein E is -CH₂-,
W is an oxygen or sulfur atom,
m is 0,
n is 0, 1, or 3,
R¹ is fluoro C₁-C₆ alkyl,
R² is a hydrogen atom, C₁-C₆ alkoxy C₁-C₆ alkyl, or (C₁-C₁₂ alkoxy)carbonyl,
R³ and R⁴ are each a hydrogen atom,
X¹, X², X³, and X⁴ are each independently a hydrogen atom, halogen, C₁-C₆ alkyl, halo C₁-C₆ alkyl, C₁-C₆ alkoxy, C₂-C₆ alkenyloxy, C₂-C₆ alkynyloxy, C₃-C₆ cycloalkyl C₁-C₆ alkyloxy, phenyl C₁-C₆ alkyloxy, (C₁-C₆ alkyl)carbonyloxy, phenylcarbonyloxy optionally substituted by one or more substituents selected from Z², heterocyclylcarbonyloxy, (C₁-C₆ alkoxy)carbonyloxy, (C₁-C₆ alkylthio)carbonyloxy, C₁-C₆ alkylsulfonyloxy, halo C₁-C₆ alkylsulfonyloxy, phenylsulfonyloxy, di C₁-C₆ alkylphosphorooxy, tri C₁-C₆ alkylsilyloxy, C₁-C₆ alkoxy C₁-C₆ alkyl, phenyl, hydroxyl, amino, cyano, or nitro,
Y¹, Y², Y³, and Y⁴ are each independently a hydrogen atom or halo C₁-C₆ alkyl, and
Z² is nitro.

3. The compound or salt thereof according to claim 1, the compound represented by General Formula [1a]: 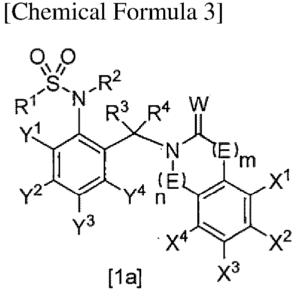
wherein E is -CH₂-,
W is an oxygen or sulfur atom,
m is 0,
n is 2,
R¹ is fluoro C₁-C₆ alkyl,
R² is a hydrogen atom, C₂-C₆ alkynyl, C₁-C₆ alkyl substituted by one substituent selected from Z⁴, cyano C₁-C₆ alkyl, C₁-C₆ alkoxy C₁-C₆ alkyl, (C₁-C₆ alkoxy)carbonyloxy C₁-C₆ alkyl, (C₁-C₁₂ alkyl)carbonyl, (halo C₁-C₁₂ alkyl)carbonyl, (C₃-C₆ cycloalkyl)carbonyl, C₁-C₆ alkyl(C₃-C₆ cycloalkyl)carbonyl, C₁-C₆ alkyl halo(C₃-C₆ cycloalkyl)carbonyl, (C₂-C₆ alkenyl)carbonyl, C₁-C₆ alkoxy(C₂-C₆ alkenyl)carbonyl, phenylcarbonyl, heterocyclyl carbonyl optionally substituted by one or more substituents selected from Z², C₃-C₆ cycloalkyl(C₁-C₆ alkyl)carbonyl, hydroxy(C₁-C₆ alkyl)carbonyl, C₁-C₆ alkoxy(C₁-C₆ alkyl)carbonyl, tri(C₁-C₆ alkyl)silyloxy (C₁-C₆ alkyl)carbonyl, (C₁-C₆ alkyl) carbonyloxy (C₁-C₆ alkyl)carbonyl, C₁-C₆ alkylthio(C₁-C₆ alkyl)carbonyl, (C₁-C₁₂ alkoxy)carbonyl, (halo C₁-C₆ alkoxy)carbonyl, (C₃-C₆ cycloalkyloxy)carbonyl, (C₂-C₆ alkenyloxy)carbonyl, (C₂-C₆ alkynyloxy)carbonyl, phenoxycarbonyl optionally substituted by one or more substituents selected from Z², heterocyclyloxycarbonyl optionally substituted by one or more substituents selected from Z², C₃-C₆ cycloalkyl(C₁-C₆ alkoxy)carbonyl, (C₁-C₆ alkoxy)carbonyl substituted by one substituent selected from Z⁴, (cyano C₁-C₆ alkoxy)carbonyl, (hydroxy C₁-C₆ alkoxy)carbonyl, C₁-C₆ alkoxy(C₁-C₆ alkoxy)carbonyl, (C₁-C₆ alkoxy)carbonyl substituted by one or more substituents selected from C₁-C₆ alkoxy, (C₁-C₆ alkyl)diphenylsilyloxy(C₁-C₆ alkoxy)carbonyl, (C₁-C₆ alkyl)carbonyloxy(C₁-C₆ alkoxy)carbonyl, C₁-C₆ alkylsulfonyloxy(C₁-C₆ alkoxy)carbonyl, (C₁-C₆ alkyl)carbonyl(C₁-C₆ alkoxy)carbonyl, (C₁-C₆ alkoxy)carbonyl(C₁-C₆ alkoxy)carbonyl, C₁-C₆ alkylthio(C₁-C₆ alkoxy)carbonyl, C₁-C₆ alkylsulfonyl(C₁-C₆ alkoxy)carbonyl, (C₁-C₆ alkylthio)carbonyl, mono(C₁-C₆ alkyl)aminocarbonyl, same or different di(C₁-C₆ alkyl)aminocarbonyl, C₁-C₆ alkylsulfonyl, halo C₁-C₆ alkylsulfonyl, or C₃-C₆ cycloalkylsulfonyl,
R³ and R⁴ are each a hydrogen atom,
X¹, X², X³, and X⁴ are each independently a hydrogen atom, halogen, C₁-C₆ alkyl, C₃-C₆ cycloalkyl, halo C₁-C₆ alkyl, C₁-C₆ alkoxy, halo C₁-C₆ alkoxy, C₂-C₆ alkynyloxy, (C₁-C₆ alkyl)carbonyloxy, phenylcarbonyloxy, (C₁-C₆ alkoxy)carbonyloxy, C₁-C₆ alkylsulfonyloxy, halo C₁-C₆ alkylsulfonyloxy, C₁-C₆ alkylthio, C₁-C₆ alkylsulfonyl, phenyl, same or different di(C₁-C₆ alkyl)amino, (C₁-C₆ alkyl)carbonylamino, bis((C₁-C₆ alkyl)carbonyl)amino, halo C₁-C₆ alkylsulfonylamino, morpholinyl, isoindoline-1,3-dione-2-yl, (C₁-C₆ alkoxy)carbonyl, hydroxyl, cyano, or nitro,
or X¹, X², X³, or X⁴ is optionally bonded together with an adjacent carbon atom on a benzene ring to form a 5- or 6-membered carbocycle or a 5- or 6-membered heterocycle, where the heterocycle contains one or two heteroatoms selected from a sulfur atom and a nitrogen atom,
Y¹, Y², Y³, and Y⁴ are each independently a hydrogen atom, halogen, C₁-C₆ alkyl, halo C₁-C₆ alkyl, C₁-C₆ alkoxy, or hydroxyl,
or Y¹ and Y², Y² and Y³, or Y³ and Y⁴ are optionally bonded together with a carbon atom to which they are attached to form a 4- to 6-membered carbocycle,
Z² is C₁-C₆ alkyl, C₁-C₆ alkoxy, or nitro, and
the heterocyclyl is thienyl, furyl, pyrrolyl, oxiranyl, oxetanyl, tetrahydrofuranyl, tetrahydrothienyl, 1,1-dioxytetrahydrothienyl, tetrahydropyranyl, tetrahydrothiopyranyl, 1,3-dioxanyl, 2-oxo-1,3-dioxolanyl, 2-oxo-1,3-dioxolyl, or 2,5-dioxopyrrolidinyl.

4. The compound or salt thereof according to claim 3, wherein n is 2,
R¹ is trifluoromethyl,
R² is a hydrogen atom, (C₃-C₆ cycloalkyl)carbonyl, (C₂-C₄ alkenyl)carbonyl, C₁-C₆ alkoxy(C₁-C₆ alkyl)carbonyl, 2-furylcarbonyl, C₁-C₆ alkylthio(C₁-C₆ alkyl)carbonyl, (C₁-C₅ alkoxy)carbonyl, (halo C₁-C₃ alkoxy)carbonyl, (C₃-C₄ cycloalkyloxy)carbonyl, (C₂-C₃ alkynyloxy)carbonyl, heterocyclyloxycarbonyl, C₃-C₄ cycloalkyl(C₁-C₂ alkoxy)carbonyl, heterocyclyl(C₁-C₂ alkoxy)carbonyl, (cyano C₁-C₆ alkoxy)carbonyl, (hydroxy C₁-C₆ alkoxy)carbonyl, C₁-C₆ alkoxy(C₁-C₆ alkoxy)carbonyl, C₁-C₂ alkylsulfonyl, or (C₁-C₄ alkylthio)carbonyl,
X¹ is a fluorine atom, a chloro atom, or methyl,
X² and X³ are each a hydrogen atom,
X⁴ is a hydrogen atom, a fluorine atom, a chloro atom, methyl, trifluoromethyl, or methoxy,
Y¹, Y², Y³, and Y⁴ are each a hydrogen atom, and
the heterocyclyl is oxiranyl, oxetanyl, tetrahydrofuranyl, or tetrahydropyranyl.

5. The compound or salt thereof according to claim 1, the compound represented by General Formula [1b]: 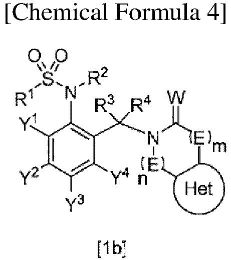
wherein Het is selected from 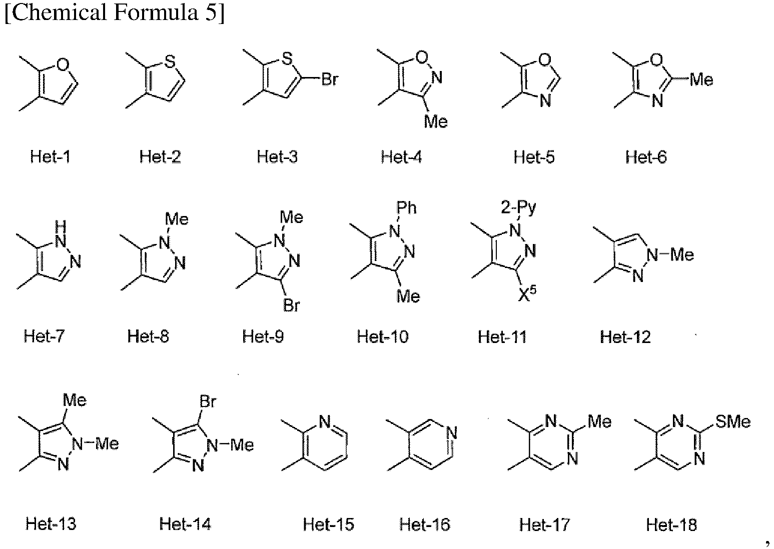
E is -CH₂-,
W is an oxygen atom,
m is 0,
n is from 1 to 2,
R¹ is fluoro C₁-C₆ alkyl,
R² is a hydrogen atom or (C₁-C₁₂ alkoxy)carbonyl,
R³ and R⁴ are each a hydrogen atom, and
Y¹, Y², Y³, and Y⁴ are each a hydrogen atom.

6. The compound or salt thereof according to claim 5, wherein
Het is Het-1 or Het-2,
n is 2,
R¹ is trifluoromethyl, and
R² is a hydrogen atom or (C₂-C₆ alkoxy)carbonyl.

7. The compound or salt thereof according to claim 1, which is selected from the group consisting of
N-[2-(N-trifluoromethanesulfonyl)aminobenzyl]-7-azabicyclo[4.2.0]octa-1(6),2,4-trien-8-one,
N-[2-(N-trifluoromethanesulfonyl)aminobenzyl]-5-fluoro-7-azabicyclo[4.2.0]octa-1(6),2,4-trien-8-one,
N-[2-(N-trifluoromethanesulfonyl)aminobenzyl]-4-fluoro-7-azabicyclo[4.2.0]octa-1(6),2,4-trien-8-one,
N-[2-(N-trifluoromethanesulfonyl)aminobenzyl]-3-fluoro-7-azabicyclo[4.2.0]octa-1(6),2,4-trien-8-one,
N-[2-(N-trifluoromethanesulfonyl)aminobenzyl]-2-fluoro-7-azabicyclo[4.2.0]octa-1(6),2,4-trien-8-one,
N-[2-(N-trifluoromethanesulfonyl)aminobenzyl]-2-trifluoromethyl-7-azabicyclo[4.2.0]octa-1(6),2,4-trien-8-one,
N-[2-(N-trifluoromethanesulfonyl)aminobenzyl]-2,5-dichloro-7-azabicyclo[4.2.0]octa-1(6),2,4-trien-8-one,
N-[2-(N-trifluoromethanesulfonyl)aminobenzyl]-3,4-difluoro-7-azabicyclo[4.2.0]octa-1(6),2,4-trien-8-one,
N-[2-(N-ethoxycarbonyl-N-trifluoromethanesulfonyl)aminobenzyl]-5-fluoro-7-azabicyclo[4.2.0]octa-1(6),2,4-trien-8-one,
N-[2-(N-ethoxycarbonyl-N-trifluoromethanesulfonyl)aminobenzyl]-4-fluoro-7-azabicyclo[4.2.0]octa-1(6),2,4-trien-8-one,
N-[2-(N-ethoxycarbonyl-N-trifluoromethanesulfonyl)aminobenzyl]-3-fluoro-7-azabicyclo[4.2.0]octa-1(6),2,4-trien-8-one,
N-[2-(N-trifluoromethanesulfonyl)aminobenzyl]-1,3-dihydroisoindol-1-one,
N-[2-(N-trifluoromethanesulfonyl)aminobenzyl]-7-fluoro-1,3-dihydroisoindol-1-one,
N-[2-(N-trifluoromethanesulfonyl)aminobenzyl]-7-chloro-1,3-dihydroisoindol-1-one,
N-[2-(N-trifluoromethanesulfonyl)aminobenzyl]-7-bromo-1,3-dihydroisoindol-1-one,
N-[2-(N-trifluoromethanesulfonyl)aminobenzyl]-7-methyl-1,3-dihydroisoindol-1-one,
N-[2-(N-trifluoromethanesulfonyl)aminobenzyl]-7-phenyl-1,3-dihydroisoindol-1-one,
N-[2-(N-trifluoromethanesulfonyl)aminobenzyl]-7-cyano-1,3-dihydroisoindol-1-one,
N-[2-(N-trifluoromethanesulfonyl)aminobenzyl]-7-hydroxy-1,3-dihydroisoindol-1-one,
N-[2-(N-trifluoromethanesulfonyl)aminobenzyl]-7-methoxy-1,3-dihydroisoindol-1-one,
N-[2-(N-trifluoromethanesulfonyl)aminobenzyl]-7-acetoxy-1,3-dihydroisoindol-1-one,
N-[2-(N-trifluoromethanesulfonyl)aminobenzyl]-7-benzoyloxy-1,3-dihydroisoindol-1-one,
N-[2-(N-trifluoromethanesulfonyl)aminobenzyl]-7-(4-nitro-benzoyl)oxy-1,3-dihydroisoindol-1-one,
N-[2-(N-trifluoromethanesulfonyl)aminobenzyl]-7-methoxycarbonyloxy-1,3-dihydroisoindol-1-one,
N-[2-(N-trifluoromethanesulfonyl)aminobenzyl]-7-(2-propylthio)carbonyloxy-1,3-dihydroisoindol-1-one,
N-[2-(N-trifluoromethanesulfonyl)aminobenzyl]-7-(1H-imidazol-1-yl-carbonyloxy)-1,3-dihydroisoindol-1-one,
N-[2-(N-trifluoromethanesulfonyl)aminobenzyl]-7-(pyrrolidin-1-yl-carbonyloxy)-1,3-dihydroisoindol-1-one,
N-[2-(N-trifluoromethanesulfonyl)aminobenzyl]-7-methanesulfonyloxy-1,3-dihydroisoindol-1-one,
N-[2-(N-trifluoromethanesulfonyl)aminobenzyl]-7-trifluoromethanesulfonyloxy-1,3-dihydroisoindol-1-one,
N-[2-(N-trifluoromethanesulfonyl)aminobenzyl]-7-phenylsulfonyloxy-1,3-dihydroisoindol-1-one,
N-[2-(N-trifluoromethanesulfonyl)aminobenzyl]-7-diethylphosphorooxy-1,3-dihydroisoindol-1-one,
N-[2-(N-trifluoromethanesulfonyl)aminobenzyl]-7-tert-butyldimethylsilyloxy-1,3-dihydroisoindol-1-one,
N-[2-(N-trifluoromethanesulfonyl)aminobenzyl]-7-propargyloxy-1,3-dihydroisoindol-1-one,
N-[2-(N-trifluoromethanesulfonyl)aminobenzyl]-7-allyloxy-1,3-dihydroisoindol-1-one,
N-[2-(N-trifluoromethanesulfonyl)aminobenzyl]-7-benzyloxy-1,3-dihydroisoindol-1-one,
N-[2-(N-trifluoromethanesulfonyl)aminobenzyl]-7-cyclopropylmethyloxy-1,3-dihydroisoindol-1-one,
N-[2-(N-trifluoromethanesulfonyl)aminobenzyl]-7-amino-1,3-dihydroisoindol-1-one,
N-[2-(N-trifluoromethanesulfonyl)aminobenzyl]-7-acetylamino-1,3-dihydroisoindol-1-one,
N-[2-(N-trifluoromethanesulfonyl)aminobenzyl]-7-bisacetylamino-1,3-dihydroisoindol-1-one,
N-[2-(N-trifluoromethanesulfonyl)aminobenzyl]-7-trifluoromethanesulfonylamino-1,3-dihydroisoindol-1-one,
N-[2-(N-trifluoromethanesulfonyl)aminobenzyl]-7-(isoindolin-1,3-dione-2-yl)-1,3-dihydroisoindol-1-one,
N-[2-(N-trifluoromethanesulfonyl)aminobenzyl]-6-fluoro-1,3-dihydroisoindol-1-one,
N-[2-(N-trifluoromethanesulfonyl)aminobenzyl]-6-methyl-1,3-dihydroisoindol-1-one,
N-[2-(N-trifluoromethanesulfonyl)aminobenzyl]-6-hydroxy-1,3-dihydroisoindol-1-one,
N-[2-(N-trifluoromethanesulfonyl)aminobenzyl]-6-methoxy-1,3-dihydroisoindol-1-one,
N-[2-(N-trifluoromethanesulfonyl)aminobenzyl]-6-acetoxy-1,3-dihydroisoindol-1-one,
N-[2-(N-trifluoromethanesulfonyl)aminobenzyl]-6-trifluoromethanesulfonyloxy-1,3-dihydroisoindol-1-one,
N-[2-(N-trifluoromethanesulfonyl)aminobenzyl]-6-propargyloxy-1,3-dihydroisoindol-1-one,
N-[2-(N-trifluoromethanesulfonyl)aminobenzyl]-6-nitro-1,3-dihydroisoindol-1-one,
N-[2-(N-trifluoromethanesulfonyl)aminobenzyl]-6-(isoindolin-1,3-dione-2-yl)-1,3-dihydroisoindol-1-one,
N-[2-(N-trifluoromethanesulfonyl)aminobenzyl]-5-bromo-1,3-dihydroisoindol-1-one,
N-[2-(N-trifluoromethanesulfonyl)aminobenzyl]-5-methyl-1,3-dihydroisoindol-1-one,
N-[2-(N-trifluoromethanesulfonyl)aminobenzyl]-5-hydroxy-1,3-dihydroisoindol-1-one,
N-[2-(N-trifluoromethanesulfonyl)aminobenzyl]-5-methoxy-1,3-dihydroisoindol-1-one,
N-[2-(N-trifluoromethanesulfonyl)aminobenzyl]-5-acetoxy-1,3-dihydroisoindol-1-one,
N-[2-(N-trifluoromethanesulfonyl)aminobenzyl]-5-propargyloxy-1,3-dihydroisoindol-1-one,
N-[2-(N-trifluoromethanesulfonyl)aminobenzyl]-4-hydroxy-1,3-dihydroisoindol-1-one,
N-[2-(N-trifluoromethanesulfonyl)aminobenzyl]-4-methoxy-1,3-dihydroisoindol-1-one,
N-[2-(N-trifluoromethanesulfonyl)aminobenzyl]-4-acetoxy-1,3-dihydroisoindol-1-one,
N-[2-(N-trifluoromethanesulfonyl)aminobenzyl]-4-trifluoromethanesulfonyloxy-1,3-dihydroisoindol-1-one,
N-[2-(N-trifluoromethanesulfonyl)aminobenzyl]-4-propargyloxy-1,3-dihydroisoindol-1-one,
N-[2-(N-trifluoromethanesulfonyl)aminobenzyl]-4-nitro-1,3-dihydroisoindol-1-one,
N-[2-(N-trifluoromethanesulfonyl)aminobenzyl]-5-fluoro-6-nitro-1,3-dihydroisoindol-1-one,
N-[2-(N-trifluoromethanesulfonyl)aminobenzyl]-5,6-dibromo-4-methoxy-1,3-dihydroisoindol-1-one,
N-[2-(N-trifluoromethanesulfonyl)amino-5-trifluoromethyl-benzyl]-1,3-dihydroisoindol-1-one,
N-[2-(N-trifluoromethanesulfonyl)amino-5-trifluoromethyl-benzyl]-5-bromo-1,3-dihydroisoindol-1-one,
N-[2-(N-ethoxycarbonyl-N-trifluoromethanesulfonyl)aminobenzyl]-1,3-dihydroisoindol-1-one,
N-[2-(N-ethoxycarbonyl-N-trifluoromethanesulfonyl)aminobenzyl]-7-amino-1,3-dihydroisoindol-1-one,
N-[2-(N-ethoxycarbonyl-N-trifluoromethanesulfonyl)aminobenzyl]-5-bromo-1,3-dihydroisoindol-1-one,
N-[2-(N-ethoxycarbonyl-N-trifluoromethanesulfonyl)amino-5-trifluoromethylbenzyl]-1,3-dihydroisoindol-1-one,
N-[2-(N-ethoxycarbonyl-N-trifluoromethanesulfonyl)amino-5-trifluoromethylbenzyl]-5-bromo-1,3-dihydroisoindol-1-one,
N-[2-(N-trifluoromethanesulfonyl)aminobenzyl]-7-methoxymethyloxy-1,3-dihydroisoindol-1-one,
N-[2-(N-trifluoromethanesulfonyl)aminobenzyl]-3,4-dihydroisoquinolin-1(2H)-one,
N-[2-(N-trifluoromethanesulfonyl)aminobenzyl]-8-fluoro-3,4-dihydroisoquinolin-1(2H)-one,
N-[2-(N-trifluoromethanesulfonyl)aminobenzyl]-8-chloro-3,4-dihydroisoquinolin-1(2H)-one,
N-[2-(N-trifluoromethanesulfonyl)aminobenzyl]-8-bromo-3,4-dihydroisoquinolin-1(2H)-one,
N-[2-(N-trifluoromethanesulfonyl)aminobenzyl]-8-iodo-3,4-dihydroisoquinolin-1(2H)-one,
N-[2-(N-trifluoromethanesulfonyl)aminobenzyl]-8-methyl-3,4-dihydroisoquinolin-1(2H)-one,
N-[2-(N-trifluoromethanesulfonyl)aminobenzyl]-8-cyclopropyl-3,4-dihydroisoquinolin-1(2H)-one,
N-[2-(N-trifluoromethanesulfonyl)aminobenzyl]-8-phenyl-3,4-dihydroisoquinolin-1(2H)-one,
N-[2-(N-trifluoromethanesulfonyl)aminobenzyl]-8-trifluoromethyl-3,4-dihydroisoquinolin-1(2H)-one,
N-[2-(N-trifluoromethanesulfonyl)aminobenzyl]-8-cyano-3,4-dihydroisoquinolin-1(2H)-one,
N-[2-(N-trifluoromethanesulfonyl)aminobenzyl]-8-hydroxy-3,4-dihydroisoquinolin-1(2H)-one,
N-[2-(N-trifluoromethanesulfonyl)aminobenzyl]-8-methoxy-3,4-dihydroisoquinolin-1(2H)-one,
N-[2-(N-trifluoromethanesulfonyl)aminobenzyl]-8-acetoxy-3,4-dihydroisoquinolin-1(2H)-one,
N-[2-(N-trifluoromethanesulfonyl)aminobenzyl]-8-difluoromethoxy-3,4-dihydroisoquinolin-1(2H)-one,
N-[2-(N-trifluoromethanesulfonyl)aminobenzyl]-8-trifluoromethoxy-3,4-dihydroisoquinolin-1(2H)-one,
N-[2-(N-trifluoromethanesulfonyl)aminobenzyl]-8-propargyloxy-3,4-dihydroisoquinolin-1(2H)-one,
N-[2-(N-trifluoromethanesulfonyl)aminobenzyl]-8-dimethylamino-3,4-dihydroisoquinolin-1(2H)-one,
N-[2-(N-trifluoromethanesulfonyl)aminobenzyl]-8-methoxycarbonyl-3,4-dihydroisoquinolin-1(2H)-one,
N-[2-(N-trifluoromethanesulfonyl)aminobenzyl]-7-fluoro-3,4-dihydroisoquinolin-1(2H)-one,
N-[2-(N-trifluoromethanesulfonyl)aminobenzyl]-7-chloro-3,4-dihydroisoquinolin-1(2H)-one,
N-[2-(N-trifluoromethanesulfonyl)aminobenzyl]-7-bromo-3,4-dihydroisoquinolin-1(2H)-one,
N-[2-(N-trifluoromethanesulfonyl)aminobenzyl]-7-iodo-3,4-dihydroisoquinolin-1(2H)-one,
N-[2-(N-trifluoromethanesulfonyl)aminobenzyl]-7-methyl-3,4-dihydroisoquinolin-1(2H)-one,
N-[2-(N-trifluoromethanesulfonyl)aminobenzyl]-7-ethyl-3,4-dihydroisoquinolin-1(2H)-one,
N-[2-(N-trifluoromethanesulfonyl)aminobenzyl]-7-tert-butyl-3,4-dihydroisoquinolin-1(2H)-one,
N-[2-(N-trifluoromethanesulfonyl)aminobenzyl]-7-phenyl-3,4-dihydroisoquinolin-1(2H)-one,
N-[2-(N-trifluoromethanesulfonyl)aminobenzyl]-7-trifluoromethyl-3,4-dihydroisoquinolin-1(2H)-one,
N-[2-(N-trifluoromethanesulfonyl)aminobenzyl]-7-nitro-3,4-dihydroisoquinolin-1(2H)-one,
N-[2-(N-trifluoromethanesulfonyl)aminobenzyl]-7-hydroxy-3,4-dihydroisoquinolin-1(2H)-one,
N-[2-(N-trifluoromethanesulfonyl)aminobenzyl]-7-methoxy-3,4-dihydroisoquinolin-1(2H)-one,
N-[2-(N-trifluoromethanesulfonyl)aminobenzyl]-7-ethoxycarbonyloxy-3,4-dihydroisoquinolin-1(2H)-one,
N-[2-(N-trifluoromethanesulfonyl)aminobenzyl]-7-benzoyloxy-3,4-dihydroisoquinolin-1(2H)-one,
N-[2-(N-trifluoromethanesulfonyl)aminobenzyl]-7-propargyloxy-3,4-dihydroisoquinolin-1(2H)-one,
N-[2-(N-trifluoromethanesulfonyl)aminobenzyl]-7-trifluoromethoxy-3,4-dihydroisoquinolin-1(2H)-one,
N-[2-(N-trifluoromethanesulfonyl)aminobenzyl]-6-fluoro-3,4-dihydroisoquinolin-1(2H)-one,
N-[2-(N-trifluoromethanesulfonyl)aminobenzyl]-6-chloro-3,4-dihydroisoquinolin-1(2H)-one,
N-[2-(N-trifluoromethanesulfonyl)aminobenzyl]-6-bromo-3,4-dihydroisoquinolin-1(2H)-one,
N-[2-(N-trifluoromethanesulfonyl)aminobenzyl]-6-iodo-3,4-dihydroisoquinolin-1(2H)-one,
N-[2-(N-trifluoromethanesulfonyl)aminobenzyl]-6-methyl-3,4-dihydroisoquinolin-1(2H)-one,
N-[2-(N-trifluoromethanesulfonyl)aminobenzyl]-6-trifluoromethyl-3,4-dihydroisoquinolin-1(2H)-one,
N-[2-(N-trifluoromethanesulfonyl)aminobenzyl]-6-methoxy-3,4-dihydroisoquinolin-1(2H)-one,
N-[2-(N-trifluoromethanesulfonyl)aminobenzyl]-5-fluoro-3,4-dihydroisoquinolin-1(2H)-one,
N-[2-(N-trifluoromethanesulfonyl)aminobenzyl]-5-chloro-3,4-dihydroisoquinolin-1(2H)-one,
N-[2-(N-trifluoromethanesulfonyl)aminobenzyl]-5-methyl-3,4-dihydroisoquinolin-1(2H)-one,
N-[2-(N-trifluoromethanesulfonyl)aminobenzyl]-5-nitro-3,4-dihydroisoquinolin-1(2H)-one,
N-[2-(N-trifluoromethanesulfonyl)aminobenzyl]-5-methoxy-3,4-dihydroisoquinolin-1(2H)-one,
N-[2-(N-trifluoromethanesulfonyl)aminobenzyl]-5-acetoxy-3,4-dihydroisoquinolin-1(2H)-one,
N-[2-(N-trifluoromethanesulfonyl)aminobenzyl]-5-methanesulfonyloxy-3,4-dihydroisoquinolin-1(2H)-one,
N-[2-(N-trifluoromethanesulfonyl)aminobenzyl]-5-trifluoromethoxy-3,4-dihydroisoquinolin-1(2H)-one,
N-[2-(N-trifluoromethanesulfonyl)aminobenzyl]-5-ethoxycarbonyloxy-3,4-dihydroisoquinolin-1(2H)-one,
N-[2-(N-trifluoromethanesulfonyl)aminobenzyl]-5-benzoyloxy-3,4-dihydroisoquinolin-1(2H)-one,
N-[2-(N-trifluoromethanesulfonyl)aminobenzyl]-5-propargyloxy-3,4-dihydroisoquinolin-1(2H)-one,
N-[2-(N-trifluoromethanesulfonyl)aminobenzyl]-8-fluoro-7-methyl-3,4-dihydroisoquinolin-1(2H)-one,
N-[2-(N-trifluoromethanesulfonyl)aminobenzyl]-8-fluoro-7-nitro-3,4-dihydroisoquinolin-1(2H)-one,
N-[2-(N-trifluoromethanesulfonyl)aminobenzyl]-7,8-dichloro-3,4-dihydroisoquinolin-1(2H)-one,
N-[2-(N-trifluoromethanesulfonyl)aminobenzyl]-8-chloro-7-methyl-3,4-dihydroisoquinolin-1(2H)-one,
N-[2-(N-trifluoromethanesulfonyl)aminobenzyl]-6,8-difluoro-3,4-dihydroisoquinolin-1(2H)-one,
N-[2-(N-trifluoromethanesulfonyl)aminobenzyl]-8-fluoro-6-methyl-3,4-dihydroisoquinolin-1(2H)-one,
N-[2-(N-trifluoromethanesulfonyl)aminobenzyl]-6-cyclopropyl-8-fluoro-3,4-dihydroisoquinolin-1(2H)-one,
N-[2-(N-trifluoromethanesulfonyl)aminobenzyl]-6-cyano-8-fluoro-3,4-dihydroisoquinolin-1(2H)-one,
N-[2-(N-trifluoromethanesulfonyl)aminobenzyl]-6,8-dichloro-3,4-dihydroisoquinolin-1(2H)-one,
N-[2-(N-trifluoromethanesulfonyl)aminobenzyl]-6,8-dimethyl-3,4-dihydroisoquinolin-1(2H)-one,
N-[2-(N-trifluoromethanesulfonyl)aminobenzyl]-5,8-difluoro-3,4-dihydroisoquinolin-1(2H)-one,
N-[2-(N-trifluoromethanesulfonyl)aminobenzyl]-5-chloro-8-fluoro-3,4-dihydroisoquinolin-1(2H)-one,
N-[2-(N-trifluoromethanesulfonyl)aminobenzyl]-5-bromo-8-fluoro-3,4-dihydroisoquinolin-1(2H)-one,
N-[2-(N-trifluoromethanesulfonyl)aminobenzyl]-8-fluoro-5-methyl-3,4-dihydroisoquinolin-1(2H)-one,
N-[2-(N-trifluoromethanesulfonyl)aminobenzyl]-8-fluoro-5-trifluoromethyl-3,4-dihydroisoquinolin-1(2H)-one,
N-[2-(N-trifluoromethanesulfonyl)aminobenzyl]-8-fluoro-5-methoxy-3,4-dihydroisoquinolin-1(2H)-one,
N-[2-(N-trifluoromethanesulfonyl)aminobenzyl]-5-chloro-8-fluoro-3,4-dihydroisoquinolin-1(2H)-one,
N-[2-(N-trifluoromethanesulfonyl)aminobenzyl]-5,8-dichloro-3,4-dihydroisoquinolin-1(2H)-one,
N-[2-(N-trifluoromethanesulfonyl)aminobenzyl]-5-fluoro-8-hydroxy-3,4-dihydroisoquinolin-1(2H)-one,
N-[2-(N-trifluoromethanesulfonyl)aminobenzyl]-5-fluoro-8-methoxy-3,4-dihydroisoquinolin-1(2H)-one,
N-[2-(N-trifluoromethanesulfonyl)aminobenzyl]-5-fluoro-8-methanesulfonyloxy-3,4-dihydroisoquinolin-1(2H)-one,
N-[2-(N-trifluoromethanesulfonyl)aminobenzyl]-5-fluoro-8-dimethylamino-3,4-dihydroisoquinolin-1(2H)-one,
N-[2-(N-trifluoromethanesulfonyl)aminobenzyl]-5-fluoro-8-(N-morpholinyl)-3,4-dihydroisoquinolin-1(2H)-one,
N-[2-(N-trifluoromethanesulfonyl)aminobenzyl]-5-fluoro-8-methylthio-3,4-dihydroisoquinolin-1(2H)-one,
N-[2-(N-trifluoromethanesulfonyl)aminobenzyl]-5-fluoro-8-methanesulfonyl-3,4-dihydroisoquinolin-1(2H)-one,
N-[2-(N-trifluoromethanesulfonyl)aminobenzyl]-5,8-bis(methanesulfonyl)-3,4-dihydroisoquinolin-1(2H)-one,
N-[2-(N-trifluoromethanesulfonyl)aminobenzyl]-6,7-dichloro-3,4-dihydroisoquinolin-1(2H)-one,
N-[2-(N-trifluoromethanesulfonyl)aminobenzyl]-6-fluoro-7-methyl-3,4-dihydroisoquinolin-1(2H)-one,
N-[2-(N-trifluoromethanesulfonyl)aminobenzyl]-6-chloro-7-methyl-3,4-dihydroisoquinolin-1(2H)-one,
N-[2-(N-trifluoromethanesulfonyl)aminobenzyl]-6-fluoro-7-nitro-3,4-dihydroisoquinolin-1(2H)-one,
N-[2-(N-trifluoromethanesulfonyl)aminobenzyl]-6-chloro-7-nitro-3,4-dihydroisoquinolin-1(2H)-one,
N-[2-(N-trifluoromethanesulfonyl)aminobenzyl]-5,6-difluoro-3,4-dihydroisoquinolin-1(2H)-one,
N-[2-(N-trifluoromethanesulfonyl)aminobenzyl]-6,8-difluoro-7-nitro-3,4-dihydroisoquinolin-1(2H)-one,
N- [2-(N-trifluoromethanesulfonyl)aminobenzyl] - 6,8 -dichloro-7 -nitro- 3,4-dihydroisoquinolin-1(2H)-one,
N-[2-(N-trifluoromethanesulfonyl)aminobenzyl]-3,4-dihydrobenzo[h]isoquinolin-1(2H)-one,
N-[2-(N-trifluoromethanesulfonyl)aminobenzyl]-3,4-dihydrobenzo[g]isoquinolin-1(2H)-one,
N-[2-(N-trifluoromethanesulfonyl)aminobenzyl]-7,8-dihydrothiazolo[4,5-h]isoquinoline-9(6H)-one,
N-[2-(N-methyl-N-trifluoromethanesulfonyl)aminobenzyl]-5,8-difluoro-3,4-dihydroisoquinolin-1(2H)-one,
N-[2-(N-propargyl-N-trifluoromethanesulfonyl)aminobenzyl]-5,8-difluoro-3,4-dihydroisoquinolin-1(2H)-one,
N-[2-(N-(4-methoxybenzyl)-N-trifluoromethanesulfonyl)aminobenzyl]-5,8-difluoro-3,4-dihydroisoquinolin-1(2H)-one,
N-[2-[N-[3-(1,3-dioxan-2-yl)propanyl]-N-trifluoromethanesulfonyl]aminobenzyl]-5,8-difluoro-3,4-dihydroisoquinolin-1(2H)-one,
N-[2-(N-cyanomethyl-N-trifluoromethanesulfonyl)aminobenzyl]-5,8-difluoro-3,4-dihydroisoquinolin-1(2H)-one,
N-[2-(N-ethoxymethyl-N-trifluoromethanesulfonyl)aminobenzyl]-5,8-difluoro-3,4-dihydroisoquinolin-1(2H)-one,
N-[2-[N-(isopropyloxycarbonyloxymethyl)-N-trifluoromethanesulfonyl]aminobenzyl]-5,8-difluoro-3,4-dihydroisoquinolin-1(2H)-one,
N-[2-(N-acethyl-N-trifluoromethanesulfonyl)aminobenzyl]-5,8-difluoro-3,4-dihydroisoquinolin-1(2H)-one,
N-[2-(N-ethylcarbonyl-N-trifluoromethanesulfonyl)aminobenzyl]-5,8-difluoro-3,4-dihydroisoquinolin-1(2H)-one,
N-[2-[N-(1-propylcarbonyl)-N-trifluoromethanesulfonyl]aminobenzyl]-5,8-difluoro-3,4-dihydroisoquinolin-1(2H)-one,
N-[2-[N-(2-propylcarbonyl)-N-trifluoromethanesulfonyl]aminobenzyl]-5,8-difluoro-3,4-dihydroisoquinolin-1(2H)-one,
N-[2-[N-(2-methyl-1-propylcarbonyl)-N-trifluoromethanesulfonyl]aminobenzyl]-5,8-difluoro-3,4-dihydroisoquinolin-1(2H)-one,
N-[2-[N-(2,2-dimethyl-1-propylcarbonyl)-N-trifluoromethanesulfonyl]aminobenzyl]-5,8-difluoro-3,4-dihydroisoquinolin-1(2H)-one,
N-[2-(N-chloromethylcarbonyl-N-trifluoromethanesulfonyl)aminobenzyl]-5,8-difluoro-3,4-dihydroisoquinolin-1(2H)-one,
N-[2-(N-cyclopropylcarbonyl-N-trifluoromethanesulfonyl)aminobenzyl]-8-fluoro-3,4-dihydroisoquinolin-1(2H)-one,
N-[2-(N-cyclopropylcarbonyl-N-trifluoromethanesulfonyl)aminobenzyl]-8-chloro-3,4-dihydroisoquinolin-1(2H)-one,
N-[2-(N-cyclopropylcarbonyl-N-trifluoromethanesulfonyl)aminobenzyl]-6-chloro-3,4-dihydroisoquinolin-1(2H)-one,
N-[2-(N-cyclopropylcarbonyl-N-trifluoromethanesulfonyl)aminobenzyl]-5,8-difluoro-3,4-dihydroisoquinolin-1(2H)-one,
N-[2-(N-cyclopropylcarbonyl-N-trifluoromethanesulfonyl)aminobenzyl]-8-fluoro-5-methyl-3,4-dihydroisoquinolin-1(2H)-one,
N-[2-[N-(2-methyl-cyclopropyl)carbonyl-N-trifluoromethanesulfonyl]aminobenzyl]-5,8-difluoro-3,4-dihydroisoquinolin-1(2H)-one,
N-[2-[N-(2,2-dimethyl-cyclopropyl)carbonyl-N-trifluoromethanesulfonyl]aminobenzyl]-5,8-difluoro-3,4-dihydroisoquinolin-1(2H)-one,
N-[2-[N-(2,2-dichloro-1-methyl-cyclopropyl)carbonyl-N-trifluoromethanesulfonyl]aminobenzyl]-5,8-difluoro-3,4-dihydroisoquinolin-1(2H)-one,
N-[2-(N-cyclobutylcarbonyl-N-trifluoromethanesulfonyl)aminobenzyl]-5,8-difluoro-3,4-dihydroisoquinolin-1(2H)-one,
N-[2-(N-cyclopentylcarbonyl-N-trifluoromethanesulfonyl)aminobenzyl]-5,8-difluoro-3,4-dihydroisoquinolin-1(2H)-one,
N-[2-(N-cyclohexylcarbonyl-N-trifluoromethanesulfonyl)aminobenzyl]-5,8-difluoro-3,4-dihydroisoquinolin-1(2H)-one,
N-[2-(N-acryloyl-N-trifluoromethanesulfonyl)aminobenzyl]-5,8-difluoro-3,4-dihydroisoquinolin-1(2H)-one,
N-[2-[N-(prop-1-en-1-yl)carbonyl-N-trifluoromethanesulfonyl] aminobenzyl]-5,8-difluoro-3,4-dihydroisoquinolin-1(2H)-one,
N-[2-[N-(2-methyl-prop-1-en-1-yl)carbonyl-N-trifluoromethanesulfonyl]aminobenzyl]-5,8-difluoro-3,4-dihydroisoquinolin-1(2H)-one,
N-[2-[N-(prop-1-en-2-yl)carbonyl-N-trifluoromethanesulfonyl)aminobenzyl]-5,8-difluoro-3,4-dihydroisoquinolin-1(2H)-one,
N-[2-[N-(3-ethoxyacryloyl)-N-trifluoromethanesulfonyl]aminobenzyl]-5,8-difluoro-3,4-dihydroisoquinolin-1(2H)-one,
N-[2-(N-benzoyl-N-trifluoromethanesulfonyl)aminobenzyl]-5,8-difluoro-3,4-dihydroisoquinolin-1(2H)-one,
N-[2-[N-(furan-2-yl)carbonyl-N-trifluoromethanesulfonyl] aminobenzyl] -5, 8-difluoro-3,4-dihydroisoquinolin-1(2H)-one,
N-[2-[N-(thiophen-2-yl)carbonyl-N-trifluoromethanesulfonyl]aminobenzyl]-5,8-difluoro-3,4-dihydroisoquinolin-1(2H)-one,
N-[2-[N-(1-methyl-1H-pyrrol-2-yl)carbonyl-N-trifluoromethanesulfonyl]aminobenzyl]-5,8-difluoro-3,4-dihydroisoquinolin-1(2H)-one,
N-[2-[N-(furan-3-yl)carbonyl-N-trifluoromethanesulfonyl]aminobenzyl]-5,8-difluoro-3,4-dihydroisoquinolin-1(2H)-one,
N-[2-[N-(tetrahydrofuran-2-yl)carbonyl-N-trifluoromethanesulfonyl]aminobenzyl]-5,8-difluoro-3,4-dihydroisoquinolin-1(2H)-one,
N-[2-[N-(tetrahydrofuran-3-yl)carbonyl-N-trifluoromethanesulfonyl]aminobenzyl]-5,8-difluoro-3,4-dihydroisoquinolin-1(2H)-one,
N-[2-[N-(tetrahydropyran-4-yl)carbonyl-N-trifluoromethanesulfonyl]aminobenzyl]-5,8-difluoro-3,4-dihydroisoquinolin-1(2H)-one,
N-[2-[N-(2-cyclopropylacetyl)-N-trifluoromethanesulfonyl]aminobenzyl]-5,8-difluoro-3,4-dihydroisoquinolin-1(2H)-one,
N-[2-[N-(2-hydroxyethyl)carbonyl-N-trifluoromethanesulfonyl]aminobenzyl]-5,8-difluoro-3,4-dihydroisoquinolin-1(2H)-one,
N-[2-(N-methoxyacetyl-N-trifluoromethanesulfonyl)aminobenzyl]-5,8-difluoro-3,4-dihydroisoquinolin-1(2H)-one,
N-[2-(N-ethoxyacetyl-N-trifluoromethanesulfonyl)aminobenzyl]-5,8-difluoro-3,4-dihydroisoquinolin-1(2H)-one,
N-[2-[N-(2-methoxyethyl)carbonyl-N-trifluoromethanesulfonyl]aminobenzyl]-5,8-difluoro-3,4-dihydroisoquinolin-1(2H)-one,
N-[2-[N-(2-tert-butyldimethylsilyloxyethyl)carbonyl-N-trifluoromethanesulfonyl]aminobenzyl]-5,8-difluoro-3,4-dihydroisoquinolin-1(2H)-one,
N-[2-(N-acetoxyacetyl-N-trifluoromethanesulfonyl)aminobenzyl]-5,8-difluoro-3,4-dihydroisoquinolin-1(2H)-one,
N-[2-(N-methylsulfylacetyl-N-trifluoromethanesulfonyl)aminobenzyl]-5,8-difluoro-3,4-dihydroisoquinolin-1(2H)-one,
N-[2-(N-ethylsulfylacetyl-N-trifluoromethanesulfonyl)aminobenzyl]-5,8-difluoro-3,4-dihydroisoquinolin-1(2H)-one,
N-[2-[N-(1-methylsulfylethyl)carbonyl-N-trifluoromethanesulfonyl]aminobenzyl]-5,8-difluoro-3,4-dihydroisoquinolin-1(2H)-one,
N-[2-(N-methoxycarbonyl-N-trifluoromethanesulfonyl)aminobenzyl]-5,8-difluoro-3,4-dihydroisoquinolin-1(2H)-one,
N-[2-(N-ethoxycarbonyl-N-trifluoromethanesulfonyl)aminobenzyl]-3,4-dihydroisoquinolin-1(2H)-one,
N-[2-(N-ethoxycarbonyl-N-trifluoromethanesulfonyl)aminobenzyl]-8-fluoro-3,4-dihydroisoquinolin-1(2H)-one,
N-[2-(N-ethoxycarbonyl-N-trifluoromethanesulfonyl)aminobenzyl]-8-cyclopropyl-3,4-dihydroisoquinolin-1(2H)-one,
N-[2-(N-ethoxycarbonyl-N-trifluoromethanesulfonyl)aminobenzyl]-8-cyano-3,4-dihydroisoquinolin-1(2H)-one,
N-[2-(N-ethoxycarbonyl-N-trifluoromethanesulfonyl)aminobenzyl]-8-hydroxy-3,4-dihydroisoquinolin-1(2H)-one,
N-[2-(N-ethoxycarbonyl-N-trifluoromethanesulfonyl)aminobenzyl]-8-phenyl-3,4-dihydroisoquinolin-1(2H)-one,
N-[2-(N-ethoxycarbonyl-N-trifluoromethanesulfonyl)aminobenzyl]-5-methyl-3,4-dihydroisoquinolin-1(2H)-one,
N-[2-(N-ethoxycarbonyl-N-trifluoromethanesulfonyl)aminobenzyl]-8-fluoro-6-methyl-3,4-dihydroisoquinolin-1(2H)-one,
N-[2-(N-ethoxycarbonyl-N-trifluoromethanesulfonyl)aminobenzyl]-6-cyclopropyl-8-fluoro-3,4-dihydroisoquinolin-1(2H)-one,
N-[2-(N-ethoxycarbonyl-N-trifluoromethanesulfonyl)aminobenzyl]-6-cyano-8-fluoro-3,4-dihydroisoquinolin-1(2H)-one,
N-[2-(N-ethoxycarbonyl-N-trifluoromethanesulfonyl)aminobenzyl]-5,8-difluoro-3,4-dihydroisoquinolin-1(2H)-one,
N-[2-(N-ethoxycarbonyl-N-trifluoromethanesulfonyl)aminobenzyl]-5-bromo-8-fluoro-3,4-dihydroisoquinolin-1(2H)-one,
N-[2-(N-ethoxycarbonyl-N-trifluoromethanesulfonyl)aminobenzyl]-8-fluoro-5-methyl-3,4-dihydroisoquinolin-1(2H)-one,
N-[2-(N-ethoxycarbonyl-N-trifluoromethanesulfonyl)aminobenzyl]-8-fluoro-5-trifluoromethyl-3,4-dihydroisoquinolin-1(2H)-one,
N-[2-(N-ethoxycarbonyl-N-trifluoromethanesulfonyl)aminobenzyl]-8-fluoro-5-methoxy-3,4-dihydroisoquinolin-1(2H)-one,
N-[2-(N-ethoxycarbonyl-N-trifluoromethanesulfonyl)aminobenzyl]-8-chloro-5-fluoro-3,4-dihydroisoquinolin-1(2H)-one,
N-[2-(N-ethoxycarbonyl-N-trifluoromethanesulfonyl)aminobenzyl]-8-dimethylamino-5-fluoro-3,4-dihydroisoquinolin-1(2H)-one,
N-[2-(N-ethoxycarbonyl-N-trifluoromethanesulfonyl)aminobenzyl]-5-fluoro-8-(N-morpholinyl)-3,4-dihydroisoquinolin-1(2H)-one,
N-[2-(N-ethoxycarbonyl-N-trifluoromethanesulfonyl)aminobenzyl]-5-fluoro-8-methylthio-3,4-dihydroisoquinolin-1(2H)-one,
N-[2-(N-ethoxycarbonyl-N-trifluoromethanesulfonyl)aminobenzyl]-5,8-bis(methylthio)-3,4-dihydroisoquinolin-1(2H)-one,
N-[2-(N-ethoxycarbonyl-N-trifluoromethanesulfonyl)aminobenzyl]-5-fluoro-8-methanesulfonyl-3,4-dihydroisoquinolin-1(2H)-one,
N-[2-(N-ethoxycarbonyl-N-trifluoromethanesulfonyl)aminobenzyl]-5,6-difluoro-3,4-dihydroisoquinolin-1(2H)-one,
N-[2-[N-(1-propyl)oxycarbonyl-N-trifluoromethanesulfonyl]aminobenzyl]-5,8-difluoro-3,4-dihydroisoquinolin-1(2H)-one,
N-[2-[N-(2-propyl)oxycarbonyl-N-trifluoromethanesulfonyl]aminobenzyl]-5,8-difluoro-3,4-dihydroisoquinolin-1(2H)-one,
N-[2-[N-(1-butyl)oxycarbonyl-N-trifluoromethanesulfonyl]aminobenzyl]-5,8-difluoro-3,4-dihydroisoquinolin-1(2H)-one,
N-[2-[N-(2-methyl-1-propyl)oxycarbonyl-N-trifluoromethanesulfonyl]aminobenzyl]-5,8-difluoro-3,4-dihydroisoquinolin-1(2H)-one,
N-[2-[N-(2-butyl)oxycarbonyl-N-trifluoromethanesulfonyl]aminobenzyl]-5,8-difluoro-3,4-dihydroisoquinolin-1(2H)-one,
N-[2-[N-(1,1-dimethyl-ethyl)oxycarbonyl-N-trifluoromethanesulfonyl]aminobenzyl]-5,8-difluoro-3,4-dihydroisoquinolin-1(2H)-one,
N-[2-[N-(1-pentyl)oxycarbonyl-N-trifluoromethanesulfonyl]aminobenzyl]-5,8-difluoro-3,4-dihydroisoquinolin-1(2H)-one,
N-[2-[N-(1-hexyl)oxycarbonyl-N-trifluoromethanesulfonyl]aminobenzyl]-5,8-difluoro-3,4-dihydroisoquinolin-1(2H)-one,
N-[2-[N-(1-heptyl)oxycarbonyl-N-trifluoromethanesulfonyl]aminobenzyl]-5,8-difluoro-3,4-dihydroisoquinolin-1(2H)-one,
N-[2-[N-(2,2-dimethyl-1-propyl)oxycarbonyl-N-trifluoromethanesulfonyl]aminobenzyl]-5,8-difluoro-3,4-dihydroisoquinolin-1(2H)-one,
N-[2-[N-(chloromethyl)oxycarbonyl-N-trifluoromethanesulfonyl]aminobenzyl]-5,8-difluoro-3,4-dihydroisoquinolin-1(2H)-one,
N-[2-[N-(trichloromethyl)oxycarbonyl-N-trifluoromethanesulfonyl]aminobenzyl]-5,8-difluoro-3,4-dihydroisoquinolin-1(2H)-one,
N-[2-[N-(1-chloroethyl)oxycarbonyl-N-trifluoromethanesulfonyl]aminobenzyl]-5,8-difluoro-3,4-dihydroisoquinolin-1(2H)-one,
N-[2-[N-(2-fluoroethyl)oxycarbonyl-N-trifluoromethanesulfonyl]aminobenzyl]-8-fluoro-3,4-dihydroisoquinolin-1(2H)-one,
N-[2-[N-(2-fluoroethyl)oxycarbonyl-N-trifluoromethanesulfonyl]aminobenzyl]-5,8-difluoro-3,4-dihydroisoquinolin-1(2H)-one,
N-[2-[N-(2,2-difluoroethyl)oxycarbonyl-N-trifluoromethanesulfonyl]aminobenzyl]-3,4-dihydroisoquinolin-1(2H)-one,
N-[2-[N-(2,2-difluoroethyl)oxycarbonyl-N-trifluoromethanesulfonyl]aminobenzyl]-8-fluoro-3,4-dihydroisoquinolin-1(2H)-one,
N-[2-[N-(2,2-difluoroethyl)oxycarbonyl-N-trifluoromethanesulfonyl]aminobenzyl]-5,8-difluoro-3,4-dihydroisoquinolin-1(2H)-one,
N-[2-[N-(2,2,2-trifluoroethyl)oxycarbonyl-N-trifluoromethanesulfonyl]aminobenzyl]-5,8-difluoro-3,4-dihydroisoquinolin-1(2H)-one,
N-[2-[N-(2-chloroethyl)oxycarbonyl-N-trifluoromethanesulfonyl]aminobenzyl]-5,8-difluoro-3,4-dihydroisoquinolin-1(2H)-one,
N-[2-[N-(2,2-difluoro-1-methyl-ethyl)oxycarbonyl-N-trifluoromethanesulfonyl]aminobenzyl]-5,8-difluoro-3,4-dihydroisoquinolin-1(2H)-one,
N-[2-[N-(2,2,2-trifluoro-1-methyl-ethyl)oxycarbonyl-N-trifluoromethanesulfonyl]aminobenzyl]-5,8-difluoro-3,4-dihydroisoquinolin-1(2H)-one,
N-[2-[N-(1,3-difluoropropan-2-yl)oxycarbonyl-N-trifluoromethanesulfonyl]aminobenzyl]-5,8-difluoro-3,4-dihydroisoquinolin-1(2H)-one,
N-[2-(N-cyclopropyloxycarbonyl-N-trifluoromethanesulfonyl)aminobenzyl]-5,8-difluoro-3,4-dihydroisoquinolin-1(2H)-one,
N-[2-(N-cyclobutyloxycarbonyl-N-trifluoromethanesulfonyl)aminobenzyl]-5,8-difluoro-3,4-dihydroisoquinolin-1(2H)-one,
N-[2-(N-cyclopentyloxycarbonyl-N-trifluoromethanesulfonyl)aminobenzyl]-5,8-difluoro-3,4-dihydroisoquinolin-1(2H)-one,
N-[2-(N-cyclohexyloxycarbonyl-N-trifluoromethanesulfonyl)aminobenzyl]-5,8-difluoro-3,4-dihydroisoquinolin-1(2H)-one,
N-[2-(N-allyloxycarbonyl-N-trifluoromethanesulfonyl)aminobenzyl]-5,8-difluoro-3,4-dihydroisoquinolin-1(2H)-one,
N-[2-[N-(but-3-en-2-yl)oxycarbonyl-N-trifluoromethanesulfonyl]aminobenzyl]-5,8-difluoro-3,4-dihydroisoquinolin-1(2H)-one,
N-[2-[N-(2-methylallyl)oxycarbonyl-N-trifluoromethanesulfonyl]aminobenzyl]-5,8-difluoro-3,4-dihydroisoquinolin-1(2H)-one,
N-[2-[N-(3-methylbut-2-en-1-yl)oxycarbonyl-N-trifluoromethanesulfonyl]aminobenzyl]-5,8-difluoro-3,4 dihydroisoquinolin-1(2H)-one,
N-[2-(N-propargyloxycarbonyl-N-trifluoromethanesulfonyl)aminobenzyl]-5,8-difluoro-3,4-dihydroisoquinolin-1(2H)-one,
N-[2-[N-(but-3-yn-2-yl)oxycarbonyl-N-trifluoromethanesulfonyl]aminobenzyl]-5,8-difluoro-3,4-dihydroisoquinolin-1(2H)-one,
N-[2-(N-phenoxycarbonyl-N-trifluoromethanesulfonyl)aminobenzyl]-5,8-difluoro-3,4-dihydroisoquinolin-1(2H)-one,
N-[2-[N-(4-nitro-phenoxy)carbonyl-N-trifluoromethanesulfonyl]aminobenzyl]-5,8-difluoro-3,4-dihydroisoquinolin-1(2H)-one,
N-[2-[N-(oxetan-3-yl)oxycarbonyl-N-trifluoromethanesulfonyl]aminobenzyl]-3,4-dihydroisoquinolin-1(2H)-one,
N-[2-[N-(oxetan-3-yl)oxycarbonyl-N-trifluoromethanesulfonyl]aminobenzyl]-8-fluoro-3,4-dihydroisoquinolin-1(2H)-one,
N-[2-[N-(oxetan-3-yl)oxycarbonyl-N-trifluoromethanesulfonyl]aminobenzyl]-5,8-difluoro-3,4-dihydroisoquinolin-1(2H)-one,
N-[2-[N-(tetrahydrofuran-3-yl)oxycarbonyl-N-trifluoromethanesulfonyl]aminobenzyl]-3,4-dihydroisoquinolin-1(2H)-one,
N-[2-[N-(tetrahydrofuran-3-yl)oxycarbonyl-N-trifluoromethanesulfonyl]aminobenzyl]-5,8-difluoro-3,4-dihydroisoquinolin-1(2H)-one,
N-[2-[N-((S)-tetrahydrofuran-3-yl)oxycarbonyl-N-trifluoromethanesulfonyl]aminobenzyl]-5,8-difluoro-3,4-dihydroisoquinolin-1(2H)-one,
N-[2-[N-((R)-tetrahydrofuran-3-yl)oxycarbonyl-N-trifluoromethanesulfonyl]aminobenzyl]-5,8-difluoro-3,4-dihydroisoquinolin-1(2H)-one,
N-[2-[N-(tetrahydrothiophen-3-yl)oxycarbonyl-N-trifluoromethanesulfonyl]aminobenzyl]-5,8-difluoro-3,4-dihydroisoquinolin-1(2H)-one,
N-[2-[N-(1,1-dioxydotetrahydrothiophen-3-yl)oxycarbonyl-N-trifluoromethanesulfonyl]aminobenzyl]-5,8-difluoro-3,4-dihydroisoquinolin-1(2H)-one,
N-[2-[N-(tetrahydro-2H-pyran-3-yl)oxycarbonyl-N-trifluoromethanesulfonyl]aminobenzyl]-5,8-difluoro-3,4-dihydroisoquinolin-1(2H)-one,
N-[2-[N-(tetrahydro-2H-pyran-4-yl)oxycarbonyl-N-trifluoromethanesulfonyl]aminobenzyl]-5,8-difluoro-3,4-dihydroisoquinolin-1(2H)-one,
N-[2-[N-(1,3-dioxan-5-yl)oxycarbonyl-N-trifluoromethanesulfonyl]aminobenzyl]-5,8-difluoro-3,4-dihydroisoquinolin-1(2H)-one,
N-[2-[N-(2,2-dimethyl-1,3-dioxan-5-yl)oxycarbonyl-N-trifluoromethanesulfonyl]aminobenzyl]-5,8-difluoro-3,4 dihydroisoquinolin-1(2H)-one,
N-[2-[N-(tetrahydro-2H-thiopyran-4-yl)oxycarbonyl-N-trifluoromethanesulfonyl]aminobenzyl]-5,8-difluoro-3,4-dihydroisoquinolin-1(2H)-one,
N-[2-[N-(1,1-dioxydotetrahydro-2H-thiopyran-4-yl)oxycarbonyl-N-trifluoromethanesulfonyl]aminobenzyl]-5,8-difluoro-3,4-dihydroisoquinolin-1(2H)-one,
N-[2-[N-(2,5-dioxopyrrolidin-1-yl)oxycarbonyl-N-trifluoromethanesulfonyl]aminobenzyl]-5,8-difluoro-3,4-dihydroisoquinolin-1(2H)-one,
N-[2-[N-(cyclopropylmethyloxy)carbonyl-N-trifluoromethanesulfonyl]aminobenzyl]-5,8-difluoro-3,4-dihydroisoquinolin-1(2H)-one,
N-[2-(N-benzyloxycarbonyl-N-trifluoromethanesulfonyl)aminobenzyl]-5,8-difluoro-3,4-dihydroisoquinolin-1(2H)-one,
N-[2-[N-(oxirane-2-ylmethyl)oxycarbonyl-N-trifluoromethanesulfonyl]aminobenzyl]-5,8-difluoro-3,4-dihydroisoquinolin-1(2H)-one,
N-[2-[N-[(2-methyloxiran-2-yl)methyl]oxycarbonyl-N-trifluoromethanesulfonyl]aminobenzyl]-5,8-difluoro-3,4-dihydroisoquinolin-1(2H)-one,
N-[2-[N-[(3,3-dimethyloxiran-2-yl)methyl]oxycarbonyl-N-trifluoromethanesulfonyl]aminobenzyl]-5,8-difluoro-3,4-dihydroisoquinolin-1(2H)-one,
N-[2-[N-[1-(oxiran-2-yl)ethyl]oxycarbonyl-N-trifluoromethanesulfonyl]aminobenzyl]-5,8-difluoro-3,4-dihydroisoquinolin-1(2H)-one,
N-[2-[N-(oxetan-2-ylmethyl)oxycarbonyl-N-trifluoromethanesulfonyl]aminobenzyl]-5,8-difluoro-3,4-dihydroisoquinolin-1(2H)-one,
N-[2-[N-(oxetan-3-ylmethyl)oxycarbonyl-N-trifluoromethanesulfonyl]aminobenzyl]-5,8-difluoro-3,4-dihydroisoquinolin-1(2H)-one,
N-[2-[N-[(tetrahydrofuran-2-yl)methyl]oxycarbonyl-N-trifluoromethanesulfonyl]aminobenzyl]-5,8-difluoro-3,4-dihydroisoquinolin-1(2H)-one,
N-[2-[N-[(tetrahydrofuran-3-yl)methyl]oxycarbonyl-N-trifluoromethanesulfonyl]aminobenzyl]-5,8-difluoro-3,4-dihydroisoquinolin-1(2H)-one,
N-[2-[N-[(tetrahydro-2H-pyran-2-yl)methyl]oxycarbonyl-N-trifluoromethanesulfonyl]aminobenzyl]-5,8-difluoro-3,4-dihydroisoquinolin-1(2H)-one,
N-[2-[N-[(tetrahydro-2H-pyran-4-yl)methyl]oxycarbonyl-N-trifluoromethanesulfonyl]aminobenzyl]-5,8-difluoro-3,4-dihydroisoquinolin-1(2H)-one,
N-[2-[N-[(2-oxo-1,3-dioxolan-4-yl)methyl]oxycarbonyl-N-trifluoromethanesulfonyl]aminobenzyl]-5,8-difluoro-3,4-dihydroisoquinolin-1(2H)-one,
N-[2-[N-[(5-methyl-2-oxo-1,3-dioxol-4-yl)methyl]oxycarbonyl-N-trifluoromethanesulfonyl]aminobenzyl]-5,8-difluoro-3,4-dihydroisoquinolin-1(2H)-one,
N-[2-[N-(2-cyano-ethoxy)carbonyl-N-trifluoromethanesulfonyl]aminobenzyl]-5,8-difluoro-3,4-dihydroisoquinolin-1(2H)-one,
N-[2-[N-(2-hydroxy-ethoxy)carbonyl-N-trifluoromethanesulfonyl]aminobenzyl]-5,8-difluoro-3,4-dihydroisoquinolin-1(2H)-one,
N-[2-[N-(2-methoxy-ethoxy)carbonyl-N-trifluoromethanesulfonyl]aminobenzyl]-5,8-difluoro-3,4-dihydroisoquinolin-1(2H)-one,
N-[2-[N-(1-methyl-2-methoxy-ethoxy)carbonyl-N-trifluoromethanesulfonyl]aminobenzyl]-5,8-difluoro-3,4-dihydroisoquinolin-1(2H)-one,
N-[2-[N-(1,3-dimethoxypropan-2-yl)oxycarbonyl-N-trifluoromethanesulfonyl]aminobenzyl]-5,8-difluoro-3,4-dihydroisoquinolin-1(2H)-one,
N-[2-[N-[2-(*tert*-butyldiphenylsilyloxy)ethyl]oxycarbonyl-N-trifluoromethanesulfonyl]aminobenzyl]-5,8-difluoro-3,4-dihydroisoquinolin-1(2H)-one,
N-[2-[N-[2-(acetyloxy)ethoxy]carbonyl-N-trifluoromethanesulfonyl]aminobenzyl]-5,8-difluoro-3,4-dihydroisoquinolin-1(2H)-one,
N-[2-[N-[2-(methanesulfonyloxy)ethoxylcarbonyl-N-trifluoromethanesulfonyl]aminobenzyl]-5,8-difluoro-3,4-dihydroisoquinolin-1(2H)-one,
N-[2-[N-(2-oxopropyl)oxycarbonyl-N-trifluoromethanesulfonyl]aminobenzyl]-5,8-difluoro-3,4-dihydroisoquinolin-1(2H)-one,
N-[2-[N-(methoxycarbonylmethoxycarbonyl)oxycarbonyl-N-trifluoromethanesulfonyl]aminobenzyl]-5,8-difluoro-3,4-dihydroisoquinolin-1(2H)-one,
N-[2-[N-(2-methylthio-ethoxy)carbonyl-N-trifluoromethanesulfonyl]aminobenzyl]-5,8-difluoro-3,4-dihydroisoquinolin-1(2H)-one,
N-[2-[N-(2-methanesulfonyl-ethoxy)carbonyl-N-trifluoromethanesulfonyl]aminobenzyl]-5,8-difluoro-3,4-dihydroisoquinolin-1(2H)-one,
N-[2-[N-(2-propylthio)carbonyl-N-trifluoromethanesulfonyl]aminobenzyl]-3,4-dihydroisoquinolin-1(2H)-one,
N-[2-[N-(2-propylthio)carbonyl-N-trifluoromethanesulfonyl]aminobenzyl]-8-fluoro-3,4-dihydroisoquinolin-1(2H)-one,
N-[2-(N-ethylthio-carbonyl-N-trifluoromethanesulfonyl)aminobenzyl]-5,8-difluoro-3,4-dihydroisoquinolin-1(2H)-one,
N-[2-[N-(2-propylthio)carbonyl-N-trifluoromethanesulfonyl]aminobenzyl]-5,8-difluoro-3,4-dihydroisoquinolin-1(2H)-one,
N-[2-[N-(2-butyl)thio-carbonyl-N-trifluoromethanesulfonyl]aminobenzyl]-5,8-difluoro-3,4-dihydroisoquinolin-1(2H)-one,
N-[2-(N-methylaminocarbonyl-N-trifluoromethanesulfonyl)aminobenzyl]-5,8-difluoro-3,4-dihydroisoquinolin-1(2H)-one,
N-[2-[N-(2-propylamino)carbonyl-N-trifluoromethanesulfonyl]aminobenzyl]-5,8-difluoro-3,4-dihydroisoquinolin-1(2H)-one,
N-[2-(N-dimethylaminocarbonyl-N-trifluoromethanesulfonyl)aminobenzyl]-5,8-difluoro-3,4-dihydroisoquinolin-1(2H)-one,
N-[2-(N-methanesulfonyl-N-trifluoromethanesulfonyl)aminobenzyl]-3,4-dihydroisoquinolin-1(2H)-one,
N-[2-(N-methanesulfonyl-N-trifluoromethanesulfonyl)aminobenzyl]-5,8-difluoro-3,4-dihydroisoquinolin-1(2H)-one,
N-[2-(N-methanesulfonyl-N-trifluoromethanesulfonyl)aminobenzyl]-5-fluoro-8-hydroxy-3,4-dihydroisoquinolin-1(2H)-one,
N-[2-(N-ethanesulfonyl-N-trifluoromethanesulfonyl)aminobenzyl]-5,8-difluoro-3,4-dihydroisoquinolin-1(2H)-one,
N-[2-[N,N-bis(trifluoromethanesulfonyl)]aminobenzyl]-8-hydroxy-3,4-dihydroisoquinolin-1(2H)-one,
N-[2-[N,N-bis(trifluoromethanesulfonyl)]aminobenzyl]-8-propargyloxy-3,4-dihydroisoquinolin-1(2H)-one,
N-[2-[N,N-bis(trifluoromethanesulfonyl)]aminobenzyl]-5,8-difluoro-3,4-dihydroisoquinolin-1(2H)-one,
N-[2-(N-cyclopropanesulfonyl-N-trifluoromethanesulfonyl)aminobenzyl]-5,8-difluoro-3,4-dihydroisoquinolin-1(2H)-one,
sodium[2-[(5,8-difluoro-1-oxo-3,4-dihydroisoquinolin-2(1H)-yl)methyl]phenyl] [(trifluoromethyl) sulfonyl] amide,
potassium[2-[(5,8-difluoro-1-oxo-3,4-dihydroisoquinolin-2(1H)-yl)methyl]phenyl] [(trifluoromethyl) sulfonyl] amide,
N-[2-(N-trifluoromethanesulfonyl)amino-3-chloro-benzyl]-3,4-dihydroisoquinolin-1(2H)-one,
N-[2-(N-trifluoromethanesulfonyl)amino-4-methyl-benzyl]-3,4-dihydroisoquinolin-1(2H)-one,
N-[2-(N-trifluoromethanesulfonyl)amino-4-methoxy-benzyl]-3,4-dihydroisoquinolin-1(2H)-one,
N-[2-(N-trifluoromethanesulfonyl)amino-5-fluoro-benzyl]-3,4-dihydroisoquinolin-1(2H)-one,
N-[2-(N-trifluoromethanesulfonyl)amino-5-chloro-benzyl]-3,4-dihydroisoquinolin-1(2H)-one,
N-[2-(N-trifluoromethanesulfonyl)amino-5-bromo-benzyl]-3,4-dihydroisoquinolin-1(2H)-one,
N-[2-(N-trifluoromethanesulfonyl)amino-5-methyl-benzyl]-3,4-dihydroisoquinolin-1(2H)-one,
N-[2-(N-trifluoromethanesulfonyl)amino-6-chloro-benzyl]-3,4-dihydroisoquinolin-1(2H)-one,
N-[2-(N-trifluoromethanesulfonyl)amino-3,5-dichloro-benzyl]-3,4-dihydroisoquinolin-1(2H)-one,
N-[2-(N-trifluoromethanesulfonyl)amino-5-chloro-benzyl]-8-chloro-3,4-dihydroisoquinolin-1(2H)-one,
N-[2-(N-trifluoromethanesulfonyl)amino-5-chloro-benzyl]-6-chloro-3,4-dihydroisoquinolin-1(2H)-one,
N-[2-(N-trifluoromethanesulfonyl)amino-3-fluoro-benzyl]-5,8-difluoro-3,4-dihydroisoquinolin-1(2H)-one,
N-[2-(N-trifluoromethanesulfonyl)amino-3-chloro-benzyl]-5,8-difluoro-3,4-dihydroisoquinolin-1(2H)-one,
N-[2-(N-trifluoromethanesulfonyl)amino-3-methyl-benzyl]-5,8-difluoro-3,4-dihydroisoquinolin-1(2H)-one,
N-[2-(N-trifluoromethanesulfonyl)amino-4-fluoro-benzyl]-5,8-difluoro-3,4-dihydroisoquinolin-1(2H)-one,
N-[2-(N-trifluoromethanesulfonyl)amino-5-fluoro-benzyl]-5,8-difluoro-3,4-dihydroisoquinolin-1(2H)-one,
N-[2-(N-trifluoromethanesulfonyl)amino-5-chloro-benzyl]-5,8-difluoro-3,4-dihydroisoquinolin-1(2H)-one,
N-[2-(N-trifluoromethanesulfonyl)amino-5-methyl-benzyl]-5,8-difluoro-3,4-dihydroisoquinolin-1(2H)-one,
N-[2-(N-trifluoromethanesulfonyl)amino-5-hydroxy-benzyl]-5,8-difluoro-3,4-dihydroisoquinolin-1(2H)-one,
N-[2-(N-trifluoromethanesulfonyl)amino-5-methoxy-benzyl]-5,8-difluoro-3,4-dihydroisoquinolin-1(2H)-one,
N-[2-(N-trifluoromethanesulfonyl)amino-6-fluoro-benzyl]-5,8-difluoro-3,4-dihydroisoquinolin-1(2H)-one,
N-[2-(N-trifluoromethanesulfonyl)amino-6-chloro-benzyl]-5,8-difluoro-3,4-dihydroisoquinolin-1(2H)-one,
N-[2-(N-trifluoromethanesulfonyl)amino-6-methyl-benzyl]-5,8-difluoro-3,4-dihydroisoquinolin-1(2H)-one,
N-[2-(N-trifluoromethanesulfonyl)amino-naphthalen-3-yl-methyl]-5,8-difluoro-3,4-dihydroisoquinolin-1(2H)-one,
N-[2-(N-ethoxycarbonyl-N-trifluoromethanesulfonyl)amino-5-chloro-benzyl]-8-chloro-3,4-dihydroisoquinolin- 1(2H)-one,
N-[2-(N-ethoxycarbonyl-N-trifluoromethanesulfonyl)amino-3-fluoro-benzyl]-5,8-difluoro-3,4-dihydroisoquinolin-1(2H)-one,
N-[2-(N-ethoxycarbonyl-N-trifluoromethanesulfonyl)amino-3-chloro-benzyl]-5,8-difluoro-3,4-dihydroisoquinolin-1(2H)-one,
N-[2-(N-ethoxycarbonyl-N-trifluoromethanesulfonyl)amino-3-methyl-benzyl]-5,8-difluoro-3,4-dihydroisoquinolin-1(2H)-one,
N-[2-(N-ethoxycarbonyl-N-trifluoromethanesulfonyl)amino-4-fluoro-benzyl]-5,8-difluoro-3,4-dihydroisoquinolin-1(2H)-one,
N-[2-(N-ethoxycarbonyl-N-trifluoromethanesulfonyl)amino-5-fluoro-benzyl]-5,8-difluoro-3,4-dihydroisoquinolin-1(2H)-one,
N-[2-(N-ethoxycarbonyl-N-trifluoromethanesulfonyl)amino-5-chloro-benzyl]-5,8-difluoro-3,4-dihydroisoquinolin-1(2H)-one,
N-[2-(N-ethoxycarbonyl-N-trifluoromethanesulfonyl)amino-5-methyl-benzyl]-5,8-difluoro-3,4-dihydroisoquinolin-1(2H)-one,
N-[2-(N-ethoxycarbonyl-N-trifluoromethanesulfonyl)amino-5-trifluoromethylbenzyl]-5,8-difluoro-3,4-dihydroisoquinolin-1(2H)-one,
N-[2-(N-ethoxycarbonyl-N-trifluoromethanesulfonyl)amino-5-hydroxy-benzyl]-5,8-difluoro-3,4-dihydroisoquinolin-1(2H)-one,
N-[2-(N-ethoxycarbonyl-N-trifluoromethanesulfonyl)amino-5-methoxy-benzyl]-5,8-difluoro-3,4-dihydroisoquinolin-1(2H)-one,
N-[2-(N-ethoxycarbonyl-N-trifluoromethanesulfonyl)amino-6-fluoro-benzyl]-5,8-difluoro-3,4-dihydroisoquinolin-1(2H)-one,
N-[2-(N-ethoxycarbonyl-N-trifluoromethanesulfonyl)amino-6-chloro-benzyl]-5,8-difluoro-3,4-dihydroisoquinolin-1(2H)-one,
N-[2-(N-ethoxycarbonyl-N-trifluoromethanesulfonyl)amino-6-methyl-benzyl]-5,8-difluoro-3,4-dihydroisoquinolin-1(2H)-one,
N-[2-(N-ethoxycarbonyl-N-trifluoromethanesulfonyl)amino-naphthalen-3-yl-methyl]-5,8-difluoro-3,4-dihydroisoquinolin-1(2H)-one,
N-[2-(N-pent-1-yloxycarbonyl-N-trifluoromethanesulfonyl)amino-4-fluoro-benzyl]-5,8-difluoro-3,4-dihydroisoquinolin-1(2H)-one,
N-[2-(N-pent-1-yloxycarbonyl-N-trifluoromethanesulfonyl)amino-5-fluoro-benzyl]-5,8-difluoro-3,4-dihydroisoquinolin-1(2H)-one,
N-[2-(N-pent-1-yloxycarbonyl-N-trifluoromethanesulfonyl)amino-6-fluoro-benzyl]-5,8-difluoro-3,4-dihydroisoquinolin-1(2H)-one,
N-[2-(N-trifluoromethanesulfonyl)aminobenzyl]-2,3,4,5-tetrahydro-1H-2-benzazepin-1-one,
N-[2-(N-trifluoromethanesulfonyl)aminobenzyl]-9-chloro-2,3,4,5-tetrahydro-1H-2-benzazepin-1-one,
N-[2-(N-trifluoromethanesulfonyl)aminobenzyl]-9-methyl-2,3,4,5-tetrahydro-1H-2-benzazepin-1-one,
N-[2-(N-trifluoromethanesulfonyl)aminobenzyl]-7-fluoro-2,3,4,5-tetrahydro- 1H-2-benzazepin-1-one,
N-[2-(N-trifluoromethanesulfonyl)aminobenzyl]-7-chloro-2,3,4,5-tetrahydro-1H-2-benzazepin-1-one,
N-[2-(N-trifluoromethanesulfonyl)aminobenzyl]-7-methyl-2,3,4,5-tetrahydro-1H-2-benzazepin-1-one,
N-[2-(N-trifluoromethanesulfonyl)aminobenzyl]-4,5-dihydro-6H-thieno[2,3-c]pyrrol-6-one,
N-[2-(N-trifluoromethanesulfonyl)aminobenzyl]-2-bromo-4,5-dihydro-6H-thieno[2,3-c]pyrrol-6-one,
N-[2-(N-trifluoromethanesulfonyl)aminobenzyl]-2-(methylthio)-6,7-dihydro-5H-pyrrolo[3,4-d]pyrimidin-5-one,
N-[2-(N-trifluoromethanesulfonyl)aminobenzyl]-5,6-dihydrofurano[2,3-c]pyrimidin-7(4H)-one,
N-[2-(N-trifluoromethanesulfonyl)aminobenzyl]-5,6-dihydrothieno[2,3-c]pyridin-7(4H)-one,
N-[2-(N-trifluoromethanesulfonyl)aminobenzyl]-6,7-dihydrothieno[3,2-c]pyridin-4(5H)-one,
N-[2-(N-trifluoromethanesulfonyl)aminobenzyl]-3-methyl-5,6-dihydroisoxazolo[5,4-c]pyridin-7(4H)-one,
N-[2-(N-trifluoromethanesulfonyl)aminobenzyl]-2-methyl-6,7-dihydrooxazolo[5,4-c]pyridin-4(5H)-one,
N-[2-(N-trifluoromethanesulfonyl)aminobenzyl]-6,7-dihydrooxazolo[4,5-c]pyridin-4(5H)-one,
N-[2-(N-trifluoromethanesulfonyl)aminobenzyl]-2-methyl-6,7-dihydrooxazolo[4,5-c]pyridin-4(5H)-one,
5-[2-(N-trifluoromethanesulfonyl)aminobenzyl]-1,5,6,7-tetrahydro-4H-pyrazolo[4,3-c]pyridin-4-one,
5-[2-(N-trifluoromethanesulfonyl)aminobenzyl]-1-methyl-1,5,6,7-tetrahydro-4H-pyrazolo[4,3-c]pyridin-4-one,
5-[2-(N-trifluoromethanesulfonyl)aminobenzyl]-3-bromo-1-methyl-1,5,6,7-tetrahydro-4H-pyrazolo[4,3-c]pyridin-4-one,
5-[2-(N-trifluoromethanesulfonyl)aminobenzyl]-3-methyl-1-phenyl-1,5,6,7-tetrahydro-4H-pyrazolo[4,3-c]pyridin-4-one,
5-[2-(N-trifluoromethanesulfonyl)aminobenzyl]-3-methyl-1-(pyridin-2-yl)-1,5,6,7-tetrahydro-4H-pyrazolo[4,3-c]pyridin-4-one,
5-[2-(N-trifluoromethanesulfonyl)aminobenzyl]-2-methyl-2,5,6,7-tetrahydro-4H-pyrazolo[4,3-c]pyridin-4-one,
5-[2-(N-trifluoromethanesulfonyl)aminobenzyl]-2,3-dimethyl-2,5,6,7-tetrahydro-4H-pyrazolo[4,3-c]pyridin-4-one,
5-[2-(N-trifluoromethanesulfonyl)aminobenzyl]-3-bromo-2-methyl-2,5,6,7-tetrahydro-4H-pyrazolo[4,3-c]pyridin-4-one,
N-[2-(N-trifluoromethanesulfonyl)aminobenzyl]-6,7-dihydro-1,7-naphthyridin-8(5H)-one,
N-[2-(N-trifluoromethanesulfonyl)aminobenzyl]-3,4-dihydro-2,6-naphthyridin-1(2H)-one,
N-[2-(N-trifluoromethanesulfonyl)aminobenzyl]-2-methyl-7,8-dihydropyrido[4,3-d]pyrimidin-5(6H)-one,
N-[2-(N-ethoxycarbonyl-N-trifluoromethanesulfonyl)aminobenzyl]-5,6-dihydrothieno[2,3-c]pyridin-7(4H)-one,
5-[2-(N-ethoxycarbonyl-N-trifluoromethanesulfonyl)aminobenzyl]-3-bromo-1-methyl-1,5,6,7-tetrahydro-4H-pyrazolo[4,3-c]pyridin-4-one,
5-[2-(N-ethoxycarbonyl-N-trifluoromethanesulfonyl)aminobenzyl]-2,3-dimethyl-2,5,6,7-tetrahydro-4H-pyrazolo[4,3-c]pyridin-4-one,
N-[2-(N-ethoxycarbonyl-N-trifluoromethanesulfonyl)aminobenzyl]-6,7-dihydro-1,7-naphthyridin-8(5H)-one,
N-[2-(N-ethoxycarbonyl-N-trifluoromethanesulfonyl)aminobenzyl]-3,4-dihydro-2,6-naphthyridin-1(2H)-one,
N-[2-(N-ethoxycarbonyl-N-trifluoromethanesulfonyl)aminobenzyl]-7,8-dihydro-1,6-naphthyridin-5(6H)-one,
N-[2-(N-trifluoromethanesulfonyl)aminobenzyl]-3,4-dihydroisoquinolin-1(2H)-thione,
N-[2-(N-trifluoromethanesulfonyl)aminobenzyl]-5,8-difluoro-3,4-dihydroisoquinoline-1(2H)-thione,
N-[2-(N-trifluoromethanesulfonyl)aminobenzyl]-3-methyl-3,4-dihydroisoquinolin-1(2H)-one,
N-[2-(N-trifluoromethanesulfonyl)aminobenzyl]-3,3-dimethyl-3,4-dihydroisoquinolin-1(2H)-one,
N-[2-(N-trifluoromethanesulfonyl)aminobenzyl]-2,2-dimethyl-2,3-dihydro-4H-benzo[e] [1,3]oxazin-4-one,
N-[2-(N-trifluoromethanesulfonyl)aminobenzyl]-1H-benzo[d][1,2]oxazin-4(3H)-one,
N-[2-(N-trifluoromethanesulfonyl)aminobenzyl]-2,3-dihydro-4H-benzo[e] [1,3]thiazin-4-one,
N-[2-(N-trifluoromethanesulfonyl)aminobenzyl]-4-methyl-3,4-dihydroisoquinolin-1(2H)-one,
N-[2-(N-trifluoromethanesulfonyl)aminobenzyl]-4-methyl-3,4-dihydroisoquinolin-1(2H)-one,
N-[2-(N-trifluoromethanesulfonyl)aminobenzyl]-4,4-dimethyl-3,4-dihydroisoquinolin-1(2H)-one,
N-[2-(N-trifluoromethanesulfonyl)aminobenzyl]-4-methoxyisoquinolin-1(2H)-one,
N-[2-(N-ethoxycarbonyl-N-trifluoromethylsulfonyl)aminobenzyl]-5,8-difluoro-3,4-dihydroisoquinolin-1(2H)-thione,
N-[2-(N-ethoxycarbonyl-N-trifluoromethylsulfonyl)aminobenzyl]-3-methyl-3,4-dihydroisoquinolin-1(2H)-one,
N-[2-(N-ethoxycarbonyl-N-trifluoromethylsulfonyl)aminobenzyl]-1H-benzo[d][1,2]oxazin-4(3H)-one,
N-[2-(N-ethoxycarbonyl-N-trifluoromethylsulfonyl)aminobenzyl]-4-methyl-3,4-dihydroisoquinolin-1(2H)-one,
N-[2-(N-ethoxycarbonyl-N-trifluoromethylsulfonyl)aminobenzyl]-4,4-dimethyl-3,4-dihydroisoquinolin-1(2H)-one,
N-[2-(N-ethoxycarbonyl-N-trifluoromethylsulfonyl)aminobenzyl]-isoquinolin-1(2H)-one,
N-[2-(N-trifluoromethylsulfonyl)amino-phenylethan-1-yl]-5,8-difluoro-3,4-dihydroisoquinolin-1(2H)-one,
N-[2-(N-difluoromethanesulfonyl)aminobenzyl]-5,8-difluoro-3,4-dihydroisoquinolin-1(2H)-one,
N-[2-(N-trifluoromethanesulfonyl)aminobenzyl]-1,3-dihydroindol-2-one,
N-[2-(N-trifluoromethanesulfonyl)aminobenzyl]-5-fluoro-1,3-dihydroindol-2-one,
N-[2-(N-trifluoromethanesulfonyl)aminobenzyl]-5-chloro-1,3-dihydroindol-2-one,
N-[2-(N-trifluoromethanesulfonyl)aminobenzyl]-5-bromo-1,3-dihydroindol-2-one,
N-[2-(N-trifluoromethanesulfonyl)aminobenzyl]-6-chloro-1,3-dihydroindol-2-one,
N-[2-(N-trifluoromethanesulfonyl)amino-5-trifluoromethyl-benzyl]-1,3-dihydroindol-2-one,
N-[2-(N-ethoxycarbonyl-N-trifluoromethanesulfonyl)aminobenzyl]-1,3-dihydroindol-2-one,
N-[2-(N-ethoxycarbonyl-N-trifluoromethanesulfonyl)aminobenzyl]-5-fluoro-1,3-dihydroindol-2-one,
N-[2-(N-ethoxycarbonyl-N-trifluoromethanesulfonyl)aminobenzyl]-5-chloro-1,3-dihydroindol-2-one,
N-[2-(N-ethoxycarbonyl-N-trifluoromethanesulfonyl)aminobenzyl]-5-bromo-1,3-dihydroindol-2-one,
N-[2-(N-ethoxycarbonyl-N-trifluoromethanesulfonyl)aminobenzyl]-6-chloro-1,3-dihydroindol-2-one,
N-[2-(N-ethoxycarbonyl-N-trifluoromethanesulfonyl)amino-5-trifluoromethylbenzyl]-1,3-dihydroindol-2-one,
N-[2-(N-trifluoromethanesulfonyl)aminobenzyl]-1,4-dihydroisoquinolin-3(2H)-one,
N-[2-(N-trifluoromethanesulfonyl)aminobenzyl]-3,4-dihydroquinolin-2(1H)-one,
N-[2-(N-trifluoromethanesulfonyl)aminobenzyl]-3-methyl-3,4dihydroquinazolin-2(1H)-one,
N-[2-(N-trifluoromethanesulfonyl)aminobenzyl]-phenanthridin-6(5H)-one,
N-[2-(N-ethoxycarbonyl-N-trifluoromethanesulfonyl)aminobenzyl]-1,4-dihydroisoquinolin-3(2H)-one,
N-[2-(N-ethoxycarbonyl-N-trifluoromethanesulfonyl)aminobenzyl]-3,4-dihydroquinolin-2(1H)-one,
1-[2-(N-ethoxycarbonyl-N-trifluoromethanesulfonyl)aminobenzyl]-3-methyl-3,4-dihydroquinazolin-2(1H)-one, and
N-[2-(N-ethoxycarbonyl-N-trifluoromethanesulfonyl)aminobenzyl]-phenanthridin-6(5H)-one.

8. The compound or salt thereof according to any one of claims 1 to 7, wherein the compound represented by Formula [1] is a mixture of enantiomers or a mixture of diastereomers.

9. A composition having herbicidal activity, comprising the compound or salt thereof represented by Formula [1] according to any one of claims 1 to 8 as an active ingredient.

10. A method of weed control, comprising treating soil and/or a plant with an effective amount of the composition according to claim 9.

11. A method of controlling paddy field weeds, comprising treating paddy field soil with an effective amount of the composition according to claim 9.
